(19) Europäisches Patentamt
European Patent Office
Office européen des brevets

(11) EP 4 578 862 A1

(12) EUROPEAN PATENT APPLICATION
published in accordance with Art. 153(4) EPC

(43) Date of publication:
02.07.2025 Bulletin 2025/27

(21) Application number: 23854360.7

(22) Date of filing: 11.08.2023

(51) International Patent Classification (IPC):
C07D 519/00 (2006.01) C07D 405/12 (2006.01)
A61K 31/437 (2006.01) A61K 31/4709 (2006.01)
A61P 35/00 (2006.01)

(52) Cooperative Patent Classification (CPC):
A61K 31/437; A61K 31/4709; A61P 35/00;
C07D 405/12; C07D 519/00

(86) International application number:
PCT/CN2023/112666

(87) International publication number:
WO 2024/037459 (22.02.2024 Gazette 2024/08)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 18.08.2022 CN 202210995461
01.09.2022 CN 202211067799
22.12.2022 CN 202211659606
24.02.2023 CN 202310167302

(71) Applicant: Medshine Discovery Inc.
Nanjing, Jiangsu 210032 (CN)

(72) Inventors:
- JIANG, Fen
Shanghai 200131 (CN)
- LIU, Xiao
Shanghai 200131 (CN)
- ZHANG, Yang
Shanghai 200131 (CN)
- ZHANG, Li
Shanghai 200131 (CN)
- LI, Jian
Shanghai 200131 (CN)
- CHEN, Shuhui
Shanghai 200131 (CN)

(74) Representative: Dehns
10 Old Bailey
London EC4M 7NG (GB)

# (54) AMIDE-CONTAINING HETEROCYCLIC DERIVATIVES AND USE THEREOF

(57) Provided are a series of amide-containing heterocyclic derivatives and the use thereof, and specifically provided are compounds represented by formula (III) and pharmaceutically acceptable salts thereof.

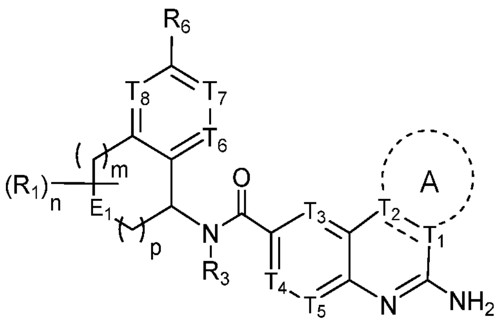

( III )



Processed by Luminess, 75001 PARIS (FR)

## Description

The present application claims the right of the following priorities:

CN20221 09954611, application date: August 18, 2022;
CN2022110677997, application date: September 01, 2022;
CN2022116596067, application date: December 22, 2022;
CN2023101673027, application date: February 24, 2023.

TECHNICAL FIELD

The present disclosure relates to a series of amide-containing heterocyclic derivatives and a use thereof, specifically to a compound of formula (III) and a pharmaceutically acceptable salt thereof.

BACKGROUND

PRMT5 (protein arginine methyltransferase 5) is an important type II protein arginine methyltransferase that regulates gene expression by modulating the methylation level of histone arginine. PRMT5 is highly expressed in various tumors (colorectal cancer, lung cancer, ovarian cancer, prostate cancer, lymphoma, leukemia, and glioblastoma, etc.), and its expression level is closely related to the occurrence, development, and prognosis of the tumor. Therefore, inhibiting PRMT5 is expected to become a novel therapeutic strategy for these tumors. Currently, several inhibitors in this field have entered clinical research (such as GSK3326595, JNJ64619178, PF06939999, PRT543, and PRT811). Among them, GSK-3326595, as the first PRMT5 inhibitor to enter clinical research, can effectively inhibit the function of PRMT5. However, due to its lack of selectivity, GSK-3326595 inhibits PRMT5 in both tumor and normal cells, leading to a high risk of clinical hematologic side effects such as thrombocytopenia, anemia, and neutropenia. Therefore, selectively inhibiting the function of PRMT5 in tumor cells without affecting the activity of PRMT5 in normal cells is expected to improve the therapeutic index of PRMT5 inhibitors and is a new direction for the research of PRMTS inhibitors. MTAP (methylthioadenosine phosphorylase) is the first enzyme in the salvage pathway of methionine and purine synthesis, and can catalyze the metabolism of MTA (methylthioadenosine), a byproduct of polyamine metabolism. The MTAP gene is often co-deleted with the common tumor suppressor gene CDKN2A *in vivo,* and the proportion of such co-deletion in tumors can reach 9% to 15%. The loss of the MTAP gene can lead to the intracellular accumulation of MTA. MTA can compete with SAM for binding to PRMT5 to form a PRMT5·MTA complex and inhibit the catalytic activity of PRMT5 on SAM. Inhibitors developed for the PRMT5·MTA complex can selectively inhibit PRMT5 in MTAP-deficient tumor cells and have little effect on PRMT5 in normal cells, thereby selectively killing MTAP-deficient tumor cells and achieving better safety. Currently, only AMG-193 and MRTX1719, inhibitors targeting the PRMT5·MTA complex, are undergoing or about to undergo clinical trials. Therefore, developing inhibitors targeting the PRMT5 ·MTA complex is clinically important for the treatment of MTAP-deficient tumors.

CONTENT OF THE PRESENT INVENTION

The present disclosure provides a compound of formula (III) or a pharmaceutically acceptable salt thereof,

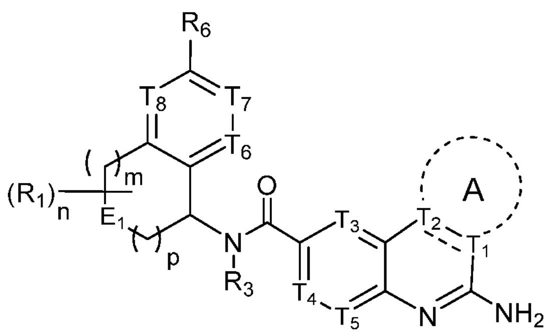

( III ) ,

wherein

$E_1$ is selected from $CH_2$, NH, and O;
the structural moiety

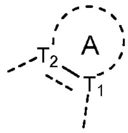

is selected from

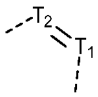 , 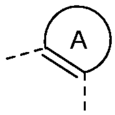 , 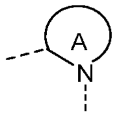 ,

and

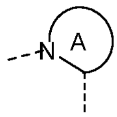 ;

each $R_1$ is independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_a$;
$R_6$ is selected from $C_{2-4}$ alkynyl and $C_{2-4}$ alkenyl, and the $C_{2-4}$ alkynyl and $C_{2-4}$ alkenyl are each independently and optionally substituted by 1, 2, or 3 $R_b$;
alternatively, $R_6$ is selected from halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_b$, in which case the structural moiety

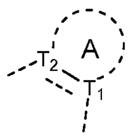

is selected from

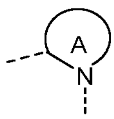 and 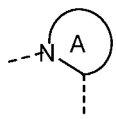 ;

ring A is selected from 5- to 6-membered heteroaryl, $C_{4-6}$ cycloalkenyl, or 5- to 6-membered heterocycloalkenyl, and each 5- to 6-membered heteroaryl, $C_{4-6}$ cycloalkenyl, or 5-to 6-membered heterocycloalkenyl is independently and optionally substituted by 1, 2, or 3 $R_c$;
$T_1$ and $T_2$ are each independently selected from $CR_7$ and N;
$T_3$, $T_4$, and $T_5$ are each independently selected from $CR_4$ and N;
$T_6$, $T_7$, and $T_8$ are each independently selected from $CR_5$ and N;
$R_3$ is selected from H, $C_{1-3}$ alkyl, $C_{3-6}$ cycloalkyl, 4- to 6-membered heterocycloalkyl, -$C_{1-3}$ alkyl-$C_{3-6}$ cycloalkyl, and -$C_{1-3}$ alkyl-4- to 6-membered heterocycloalkyl, and the $C_{1-3}$ alkyl, $C_{3-6}$ cycloalkyl, 4- to 6-membered heterocycloalkyl, -$C_{1-3}$ alkyl-$C_{3-6}$ cycloalkyl, and -$C_{1-3}$ alkyl-4- to 6-membered heterocycloalkyl are each independently and optionally substituted by 1, 2, or 3 $R_e$;
$R_4$, $R_5$, and $R_7$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_d$;
$R_a$, $R_c$, $R_a$, and $R_e$ are each independently selected from H, D, halogen, OH, $NH_2$, $CH_3$, and $CD_3$;
each $R_b$ is independently selected from H, D, halogen, OH, $NH_2$, $C_{1-3}$ alkyl, $C_{1-3}$ alkoxy, $C_{1-3}$ alkylamino, and 4- to 6-membered heterocycloalkyl, and the $C_{1-3}$ alkyl, $C_{1-3}$ alkoxy, and $C_{1-3}$ alkylamino are each independently and optionally substituted by 1, 2, or 3 R;

each R is independently selected from H, D, halogen, OH, $NH_2$, $CH_3$, $CF_3$, and $CD_3$;
n is selected from 0, 1, 2, and 3;
m is selected from 0, 1, and 2;
p is selected from 0, 1, and 2, and m and p are not simultaneously 0;
"hetero" in the 4- to 6-membered heterocycloalkyl, 5- to 6-membered heteroaryl, or 5- to 6-membered heterocycloalkenyl represents 1, 2, 3, or 4 heteroatoms or heteroatom groups independently selected from -O-, -NH-, -S-, and N.

The present disclosure further provides a compound of formula (III) or a pharmaceutically acceptable salt thereof,

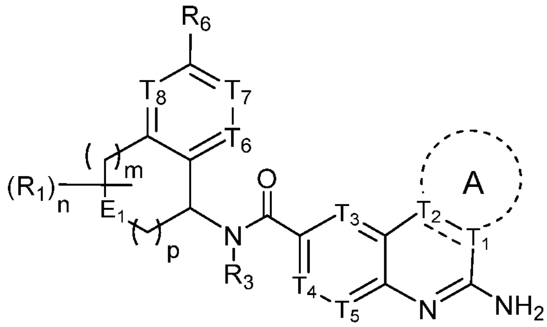

( III )

,

wherein

each $R_1$ is independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_a$;
$E_1$ is selected from $CH_2$, NH, and O;
$R_6$ is selected from --$R_2$, $R_2$ is selected from H, halogen, OH, $NH_2$, and $C_{1-3}$ alkyl, and the $C_{1-3}$ alkyl is optionally substituted by 1, 2, or 3 $R_b$;
the structural moiety

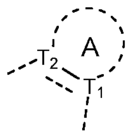

is selected from

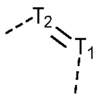
,
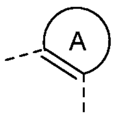
,
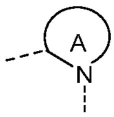
,

and

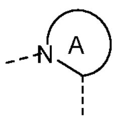
;

alternatively, $R_6$ is selected from halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_b$, in which case the structural moiety

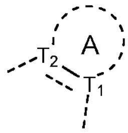

is selected from

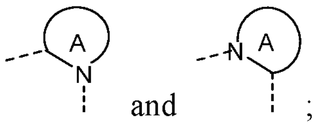

T$_1$ and T$_2$ are each independently selected from $CR_7$ and N;
ring A is selected from 5- to 6-membered heteroaryl, $C_{4-6}$ cycloalkenyl, and 5- to 6-membered heterocycloalkenyl, and the 5- to 6-membered heteroaryl, $C_{4-6}$ cycloalkenyl, and 5-to 6-membered heterocycloalkenyl are each independently and optionally substituted by 1, 2, or 3 $R_c$;
$T_3$, $T_4$, and $T_5$ are each independently selected from $CR_4$ and N;
$T_6$, $T_7$, and $T_8$ are each independently selected from $CR_5$ and N;
$R_3$ is selected from H and $C_{1-3}$ alkyl, and the $C_{1-3}$ alkyl is optionally substituted by 1, 2, or 3 $R_e$;
$R_4$, $R_5$, and $R_7$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_d$;
$R_a$, $R_b$, $R_c$, $R_d$, and $R_e$ are each independently selected from H, D, halogen, OH, $NH_2$, $CH_3$, and $CD_3$;
n is selected from 0, 1, 2, and 3;
m is selected from 0, 1, and 2;
p is selected from 0, 1, and 2, and m and p are not simultaneously 0;
"hetero" in the 5- to 6-membered heteroaryl or 5- to 6-membered heterocycloalkenyl represents 1, 2, 3, or 4 heteroatoms or heteroatom groups independently selected from -O-, - NH-, -S-, and N.

The present disclosure further provides a compound of formula (II) or a pharmaceutically acceptable salt thereof,

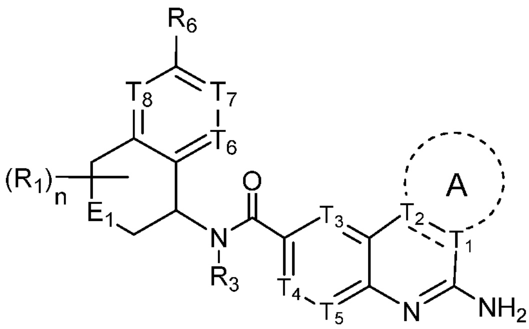

( II )

,

wherein

each $R_1$ is independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_a$;
$R_6$ is selected from --$R_2$, $R_2$ is selected from H, halogen, OH, $NH_2$, and $C_{1-3}$ alkyl, and the $C_{1-3}$ alkyl is optionally substituted by 1, 2, or 3 $R_b$;
the structural moiety

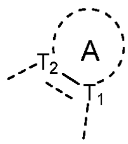

is selected from

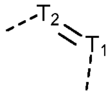,
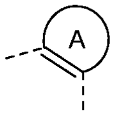,
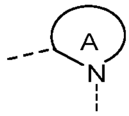,

and

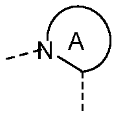;

alternatively, $R_6$ is selected from halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_b$, in which case the structural moiety

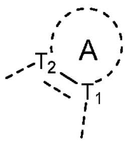

is selected from

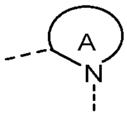
and
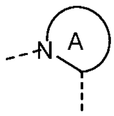;

$T_1$ and $T_2$ are each independently selected from $CR_7$ and N;
ring A is selected from 5- to 6-membered heteroaryl, $C_{4-6}$ cycloalkenyl, and 5- to 6-membered heterocycloalkenyl, and the 5- to 6-membered heteroaryl, $C_{4-6}$ cycloalkenyl, and 5-to 6-membered heterocycloalkenyl are each independently and optionally substituted by 1, 2, or 3 $R_c$;
$T_3$, $T_4$, and $T_5$ are each independently selected from $CR_4$ and N;
$T_6$, $T_7$, and $T_8$ are each independently selected from $CR_5$ and N;
$R_3$ is selected from H and $C_{1-3}$ alkyl, and the $C_{1-3}$ alkyl is optionally substituted by 1, 2, or 3 $R_e$;
$R_4$, $R_5$, and $R_7$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_d$; $R_a$, $R_b$, $R_e$, $R_d$, and $R_e$ are each independently selected from H, D, halogen, OH, $NH_2$, $CH_3$, and $CD_3$;
n is selected from 0, 1, 2, and 3;
"hetero" in the 5- to 6-membered heteroaryl or 5- to 6-membered heterocycloalkenyl represents 1, 2, 3, or 4 heteroatoms or heteroatom groups independently selected from -O-, - NH-, -S-, and N.

The present disclosure further provides a compound of formula (I) or a pharmaceutically acceptable salt thereof,

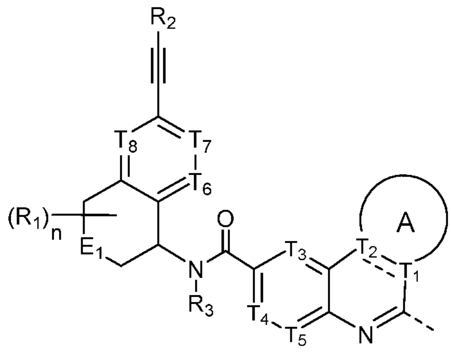

(I)

,

wherein

each $R_1$ is independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_a$;
$R_2$ is selected from H, halogen, OH, $NH_2$, and $C_{1-3}$ alkyl, and the $C_{1-3}$ alkyl is optionally substituted by 1, 2, or 3 $R_b$;
$E_1$ is selected from $CH_2$, NH, and O;
the structural moiety

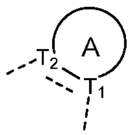

is selected from

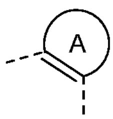

, 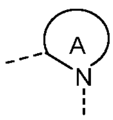

, and 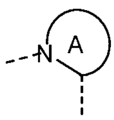

;

ring A is selected from 5- to 6-membered heteroaryl, $C_{4-6}$ cycloalkenyl, and 5- to 6-membered heterocycloalkenyl, and the 5- to 6-membered heteroaryl, $C_{4-6}$ cycloalkenyl, and 5-to 6-membered heterocycloalkenyl are each independently and optionally substituted by 1, 2, or 3 $R_c$;
$T_3$, $T_4$, and $T_5$ are each independently selected from $CR_4$ and N;
$T_6$, $T_7$, and $T_8$ are each independently selected from $CR_5$ and N;
$R_3$ is selected from H and $C_{1-3}$ alkyl, and the $C_{1-3}$ alkyl is optionally substituted by 1, 2, or 3 $R_e$;
$R_4$ and $R_5$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_d$;
$R_a$, $R_b$, $R_c$, $R_d$, and $R_e$ are each independently selected from H, halogen, OH, $NH_2$, $CH_3$, and $CD_3$;
n is selected from 0, 1, 2, and 3;
"hetero" in the 5- to 6-membered heteroaryl or 5- to 6-membered heterocycloalkenyl represents 1, 2, 3, or 4 heteroatoms or heteroatom groups independently selected from -O-, - NH-, -S-, and N.

The present disclosure further provides a compound of formula (I) or a pharmaceutically acceptable salt thereof,

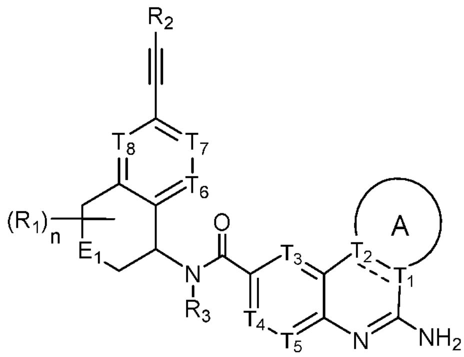

(I)

,

wherein

each $R_1$ is independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_a$;
$R_2$ is selected from H, halogen, OH, $NH_2$, and $C_{1-3}$ alkyl, and the $C_{1-3}$ alkyl is optionally substituted by 1, 2, or 3 $R_b$;
$E_1$ is selected from $CH_2$, NH, and O;
the structural moiety

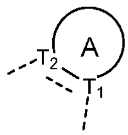

is selected from

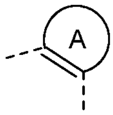

, 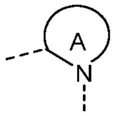

, and 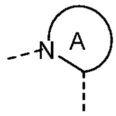

;

ring A is selected from 5- to 6-membered heteroaryl, $C_{4-6}$ cycloalkenyl, and 5- to 6-membered heterocycloalkenyl, and the 5- to 6-membered heteroaryl, $C_{4-6}$ cycloalkenyl, and 5-to 6-membered heterocycloalkenyl are each independently and optionally substituted by 1, 2, or 3 $R_c$;
$T_3$, $T_4$, and $T_5$ are each independently selected from $CR_4$ and N;
$T_6$, $T_7$, and $T_8$ are each independently selected from $CR_5$ and N;
$R_3$ is selected from H and $C_{1-3}$ alkyl, and the $C_{1-3}$ alkyl is optionally substituted by 1, 2, or 3 $R_e$;
$R_4$ and $R_5$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_d$;
$R_a$, $R_b$, $R_c$, $R_d$, and $R_e$ are each independently selected from H, halogen, OH, $NH_2$, $CH_3$, and $CD_3$;
n is selected from 0, 1, 2, and 3;
"hetero" in the 5- to 6-membered heteroaryl or 5- to 6-membered heterocycloalkenyl represents 1, 2, 3, or 4 heteroatoms or heteroatom groups independently selected from -O-, - NH-, -S-, and N.

In some embodiments of the present disclosure, each $R_a$ is independently selected from H, D, F, Cl, OH, $NH_2$, $CH_3$, and $CD_3$, and other variables are as defined in the present disclosure.
In some embodiments of the present disclosure, each $R_b$ is independently selected from H, D, F, Cl, OH, $NH_2$, $CH_3$, $CH_2CH_3$, $OCH_3$, $OCH_2CH_3$, $NHCH_3$, $NHCH_2CH_3$, $N(CH_3)_2$, pyrrolidinyl, piperidinyl, piperazinyl, and morpholinyl, and the $CH_3$, $CH_2CH_3$, $OCH_3$, $OCH_2CH_3$, $NHCH_3$, $NHCH_2CH_3$, $N(CH_3)_2$, pyrrolidinyl, piperidinyl, piperazinyl, and morpholinyl are each independently and optionally substituted by 1, 2, or 3 R, and other variables are as defined in the present disclosure.
In some embodiments of the present disclosure, each $R_b$ is independently selected from H, D, F, Cl, OH, $NH_2$, $CH_3$, $CD_3$, $CF_3$, $-C(CH_3)_2OH$, $N(CH_3)_2$,

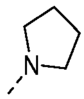, 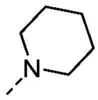,

and

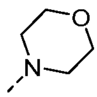,

and other variables are as defined in the present disclosure.

In some embodiments of the present disclosure, each $R_b$ is independently selected from H, D, F, Cl, OH, $NH_2$, $CH_3$, and $CD_3$, and other variables are as defined in the present disclosure.

In some embodiments of the present disclosure, each $R_c$ is independently selected from H, D, F, Cl, OH, $NH_2$, $CH_3$, and $CD_3$, and other variables are as defined in the present disclosure.

In some embodiments of the present disclosure, each $R_d$ is independently selected from H, D, F, Cl, OH, $NH_2$, $CH_3$, and $CD_3$, and other variables are as defined in the present disclosure.

In some embodiments of the present disclosure, each $R_e$ is independently selected from H, D, F, Cl, OH, $NH_2$, $CH_3$, and $CD_3$, and other variables are as defined in the present disclosure.

In some embodiments of the present disclosure, the $R_1$ is selected from H, F, Cl, Br, I, $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$, and the $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$ are each independently and optionally substituted by 1, 2, or 3 $R_a$, and each $R_a$ and other variables are as defined in the present disclosure.

In some embodiments of the present disclosure, the $R_1$ is selected from F and $CH_3$, and other variables are as defined in the present disclosure.

In some embodiments of the present disclosure, the $R_2$ is selected from H, F, Cl, Br, I, OH, $NH_2$, $CH_3$, $CH_2CH_3$, $CH_2CH_2CH_3$, and $CH(CH_3)_2$, and the $CH_3$, $CH_2CH_3$, $CH_2CH_2CH_3$, and $CH(CH_3)_2$ are each independently and optionally substituted by 1, 2, or 3 $R_b$, and each $R_b$ and other variables are as defined in the present disclosure.

In some embodiments of the present disclosure, the $R_2$ is selected from H and $CF_3$, and other variables are as defined in the present disclosure.

In some embodiments of the present disclosure, the $R_3$ is selected from H, $CH_3$, $CH_2CH_3$, $CH_2CH_2CH_3$, $CH(CH_3)_2$, cyclopropyl, cyclobutyl, oxetanyl, azetidinyl, -$CH_2$-cyclopropyl, -$CH_2CH_2$-cyclopropyl, and -$CH_2$-oxetanyl, and the $CH_3$, $CH_2CH_3$, $CH_2CH_2CH_3$, $CH(CH_3)_2$, cyclopropyl, cyclobutyl, oxetanyl, azetidinyl, -$CH_2$-cyclopropyl, -$CH_2CH_2$-cyclopropyl, and -$CH_2$-oxetanyl are each independently and optionally substituted by 1, 2, or 3 $R_e$, and other variables are as defined in the present disclosure.

In some embodiments of the present disclosure, the $R_3$ is selected from $R_3$ is selected from H, $CH_3$, $CF_3$, $CD_3$, $CH_2CH_3$, $CH(CH_3)_2$, cyclopropyl, cyclobutyl,

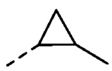, 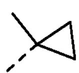,

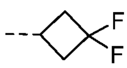, 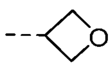, and 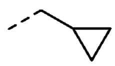,

and other variables are as defined in the present disclosure.

In some embodiments of the present disclosure, the $R_3$ is selected from H, $CH_3$, $CF_3$, $CD_3$, $CH_2CH_3$, and $CH(CH_3)_2$, and other variables are as defined in the present disclosure.

In some embodiments of the present disclosure, the $R_3$ is selected from H and $CH_3$, and other variables are as defined in the present disclosure.

In some embodiments of the present disclosure, the $R_4$ is selected from H, F, Cl, Br, I, $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$, and the $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$ are each independently and optionally substituted by 1, 2, or 3 $R_a$, and each $R_d$ and other variables are as defined in the present disclosure.

In some embodiments of the present disclosure, the $R_4$ is selected from H, F, Cl, Br, and $CH_3$, and other variables are as defined in the present disclosure.

In some embodiments of the present disclosure, the $R_5$ is selected from H, F, Cl, Br, I, $CH_3$, $CH_2CH_3$,

$CH_2CH_2CH_3$, $CH(CH_3)_2$, $OCH_3$, $OCH_2CH_3$, $OCH_2CH_2CH_3$, and $OCH(CH_3)_2$, and the $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$ are each independently and optionally substituted by 1, 2, or 3 $R_d$, and each $R_d$ and other variables are as defined in the present disclosure.

In some embodiments of the present disclosure, the $R_5$ is selected from H, F, Cl, Br, and $CH_3$, and other variables are as defined in the present disclosure.

In some embodiments of the present disclosure, the $R_7$ is selected from H, F, Cl, Br, I, $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$, and the $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$ are each independently and optionally substituted by 1, 2, or 3 $R_d$, and each $R_d$ and other variables are as defined in the present disclosure. In some embodiments of the present disclosure, the $R_7$ is selected from H, F, Cl, Br, $CH_3$, and $CD_3$, and other variables are as defined in the present disclosure.

In some embodiments of the present disclosure, the $R_6$ is selected from ethynyl, propynyl, vinyl, and propenyl, and the ethynyl, propynyl, vinyl, and propenyl are each independently and optionally substituted by 1, 2, or 3 $R_b$, and other variables are as defined in the present disclosure.

In some embodiments of the present disclosure, the $R_6$ is selected from

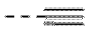,

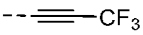, 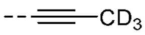, 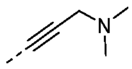, 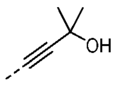, 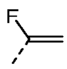, 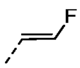, 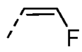, 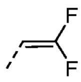, 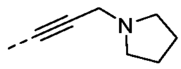,

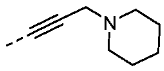, and 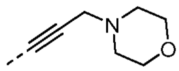,

and other variables are as defined in the present disclosure.

In some embodiments of the present disclosure, the compound or the pharmaceutically acceptable salt thereof, wherein the $R_6$ is selected from

, 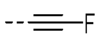, , 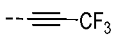,

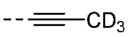, 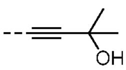, 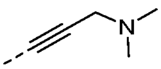, 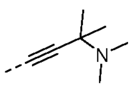, 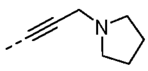, 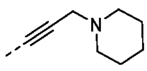, and 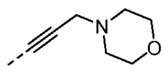,

and other variables are as defined in the present disclosure.

In some embodiments of the present disclosure, the compound or the pharmaceutically acceptable salt thereof, wherein the $R_6$ is selected from

, 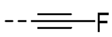, ,

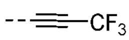, 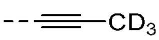, 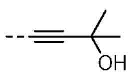, 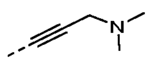, and 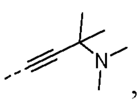,

and other variables are as defined in the present disclosure.

In some embodiments of the present disclosure, the $R_6$ is selected from

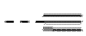,

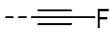, , 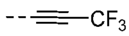, and 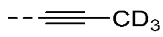,

and other variables are as defined in the present disclosure.

In some embodiments of the present disclosure, the $R_6$ is selected from

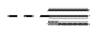,

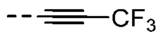,

$CH_3$, and $CF_3$, and other variables are as defined in the present disclosure.

In some embodiments of the present disclosure, the $R_6$ is selected from

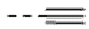

and

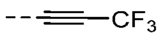,

and other variables are as defined in the present disclosure.

In some embodiments of the present disclosure, the $R_6$ is selected from

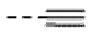,

and other variables are as defined in the present disclosure.

In some embodiments of the present disclosure, the $R_6$ is selected from

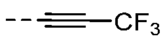,

and other variables are as defined in the present disclosure.

In some embodiments of the present disclosure, the $R_6$ is selected from $CH_3$ and $CF_3$, and other variables are as defined in the present disclosure.

In some embodiments of the present disclosure, the structural moiety

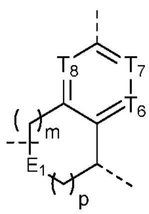

is selected from

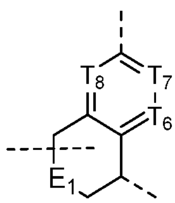,

and other variables are as defined in the present disclosure.

In some embodiments of the present disclosure, the structural moiety

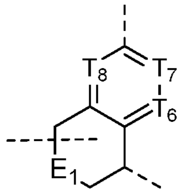

is selected from

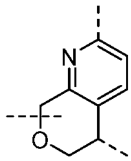,

and other variables are as defined in the present disclosure.

In some embodiments of the present disclosure, the structural moiety

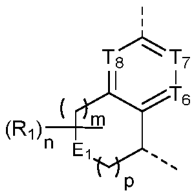

is selected from

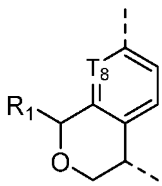 and 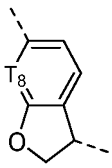,

and other variables are as defined in the present disclosure.

In some embodiments of the present disclosure, the structural moiety

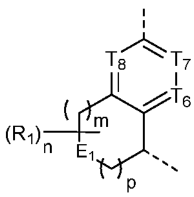

is selected from

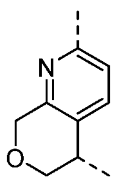, 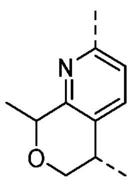, 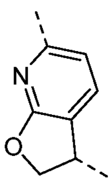, 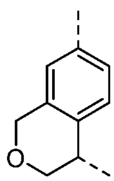, 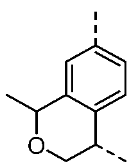, and 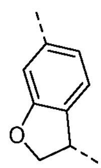,

and other variables are as defined in the present disclosure.

In some embodiments of the present disclosure, the structural moiety

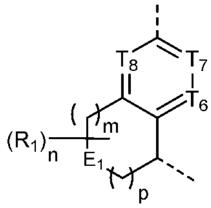

is selected from

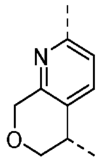, 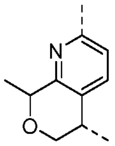, 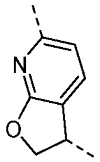, 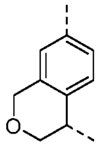, 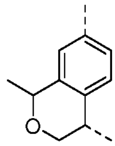, 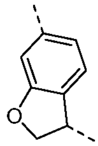, 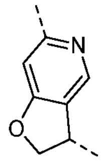,

and

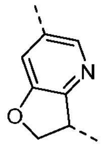,

and other variables are as defined in the present disclosure.

In some embodiments of the present disclosure, the structural moiety

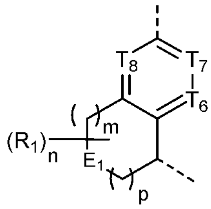

is selected from

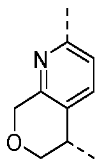, 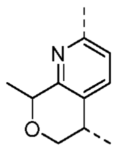, 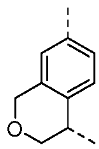, and 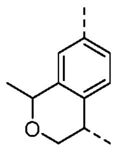,

and other variables are as defined in the present disclosure.

In some embodiments of the present disclosure, the structural moiety

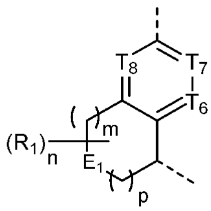

is selected from

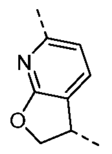 and 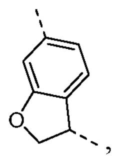,

and other variables are as defined in the present disclosure.

In some embodiments of the present disclosure, the ring A is selected from 5- to 6-membered heteroaryl or 5- to 6-membered heterocycloalkenyl, and each 5- to 6-membered heteroaryl or 5- to 6-membered heterocycloalkenyl is independently and optionally substituted by 1, 2, or 3 $R_c$, and other variables are as defined in the present disclosure.

In some embodiments of the present disclosure, the ring A is selected from 5- to 6-membered heteroaryl, and the 5- to 6-membered heteroaryl is optionally substituted by 1, 2, or 3 $R_c$, and other variables are as defined in the present disclosure.

In some embodiments of the present disclosure, the ring A is selected from 5-membered heteroaryl, and the 5-membered heteroaryl is optionally substituted by 1, 2, or 3 $R_c$, and other variables are as defined in the present disclosure.

In some embodiments of the present disclosure, the ring Ais selected from imidazolyl, pyrazolyl, pyrrolyl, thiazolyl, thienyl, furanyl, and oxazolyl, and the imidazolyl, pyrazolyl, pyrrolyl, thiazolyl, thienyl, furanyl, and oxazolyl are independently and optionally substituted by 1, 2, or 3 $R_c$, and other variables are as defined in the present disclosure.

In some embodiments of the present disclosure, the ring Ais selected from imidazolyl and pyrazolyl, and the imidazolyl and pyrazolyl are each independently and optionally substituted by 1, 2, or 3 $R_c$, and other variables are as defined in the present disclosure.

In some embodiments of the present disclosure, the ring A is selected from pyrazolyl, and the pyrazolyl is optionally substituted by 1, 2, or 3 $R_c$, and other variables are as defined in the present disclosure.

In some embodiments of the present disclosure, the ring Ais selected from imidazolyl, and the imidazolyl is optionally substituted by 1, 2, or 3 $R_c$, and other variables are as defined in the present disclosure.

In some embodiments of the present disclosure, the ring A is selected from pyridyl, pyrimidinyl, pyrazinyl, pyridazinyl, imidazolyl, pyrazolyl, pyrrolyl, thiazolyl, thienyl, furanyl, oxazolyl, cyclobutenyl, cyclopentenyl, cyclohexenyl, oxacyclopentenyl, oxacyclohexenyl, azacyclopentenyl, and azacyclohexenyl, and the pyridyl, pyrimidinyl, pyrazinyl, pyridazinyl, imidazolyl, pyrazolyl, pyrrolyl, thiazolyl, thienyl, furanyl, oxazolyl, cyclobutenyl, cyclopentenyl, cyclohexenyl, oxacyclopentenyl, oxacyclohexenyl, azacyclopentenyl, and azacyclohexenyl are each independently and optionally substituted by 1, 2, or 3 $R_c$, and each $R_c$ and other variables are as defined in the present disclosure.

In some embodiments of the present disclosure, the ring A is selected from pyridyl, pyrimidinyl, pyrazinyl, pyridazinyl, imidazolyl, pyrazolyl, pyrrolyl, thiazolyl, thienyl, furanyl, oxazolyl, triazolyl, cyclobutenyl, cyclopentenyl, cyclohexenyl, oxacyclopentenyl, oxacyclohexenyl, azacyclopentenyl, and azacyclohexenyl, and the pyridyl, pyrimidinyl, pyrazinyl, pyridazinyl, imidazolyl, pyrazolyl, pyrrolyl, thiazolyl, thienyl, furanyl, oxazolyl, triazolyl, cyclobutenyl, cyclopentenyl, cyclohexenyl, oxacyclopentenyl, oxacyclohexenyl, azacyclopentenyl, and azacyclohexenyl are each independently and optionally substituted by 1, 2, or 3 $R_c$, and each $R_c$ and other variables are as defined in the present disclosure.

In some embodiments of the present disclosure, the $T_1$ is CH, and other variables are as defined in the present disclosure.

In some embodiments of the present disclosure, the $T_2$ is CH, and other variables are as defined in the present disclosure.

In some embodiments of the present disclosure, the $T_3$ is CH, and other variables are as defined in the present disclosure.

In some embodiments of the present disclosure, the $T_4$ is CH, CF, CCl, $CCH_3$, and N, and other variables are as defined in the present disclosure.

In some embodiments of the present disclosure, the $T_5$ is CH, and other variables are as defined in the present disclosure.

In some embodiments of the present disclosure, the $T_6$ is CH and N, and other variables are as defined in the present disclosure.

In some embodiments of the present disclosure, the $T_7$ is CH and N, and other variables are as defined in the present disclosure.

In some embodiments of the present disclosure, the Ts is CH and N, and other variables are as defined in the present disclosure.

In some embodiments of the present disclosure, the structural moiety

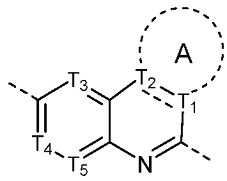

is selected from

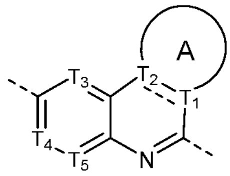

,

and other variables are as defined in the present disclosure.

In some embodiments of the present disclosure, the structural moiety

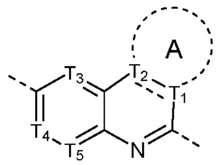

is selected from

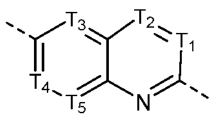

,

and other variables are as defined in the present disclosure.

In some embodiments of the present disclosure, the structural moiety

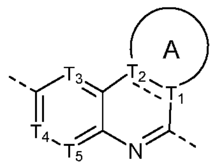

is selected from

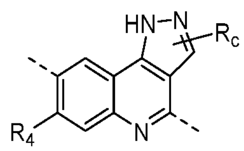

,

and other variables are as defined in the present disclosure.

In some embodiments of the present disclosure, the structural moiety

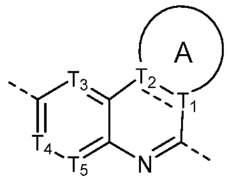

is selected from

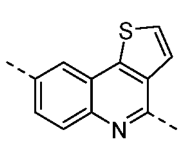, 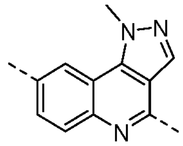, 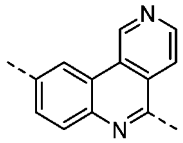,

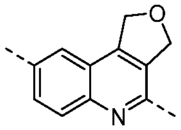, and 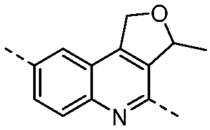,

and other variables are as defined in the present disclosure.

In some embodiments of the present disclosure, the structural moiety

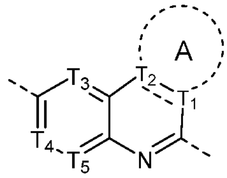

is selected from

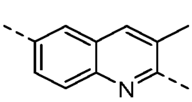, 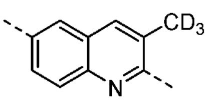, 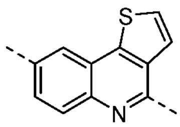

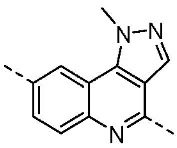, 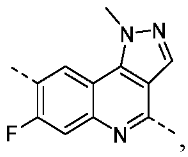, 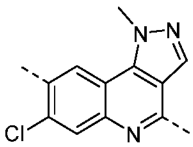, 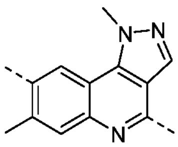, 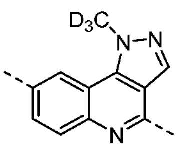, 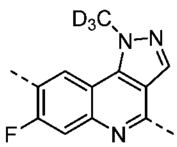,

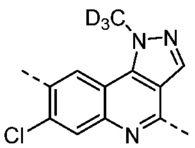, 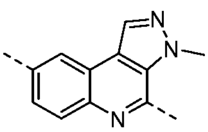, 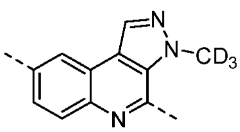, 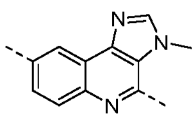, 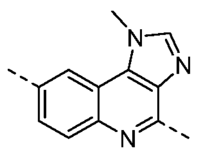,

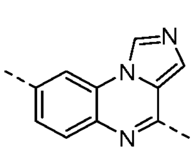, 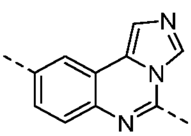, 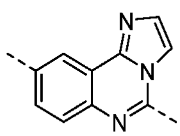, 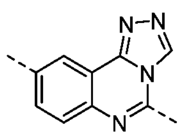, 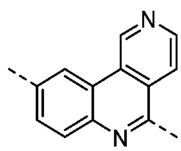,

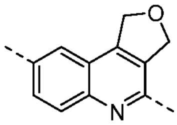, 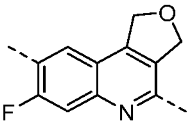, 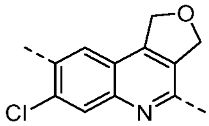, 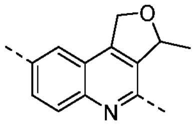, 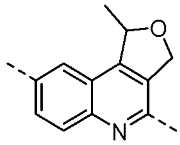,

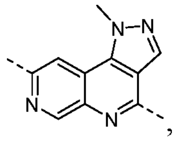, and 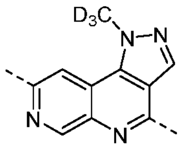,

and other variables are as defined in the present disclosure.

In some embodiments of the present disclosure, the structural moiety

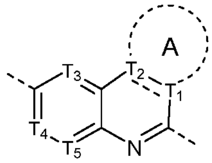

is selected from

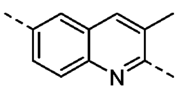, 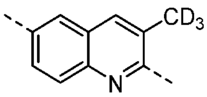, 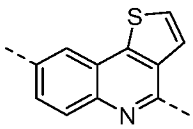 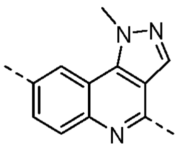,

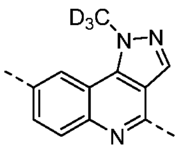, 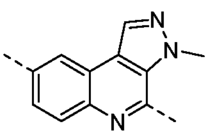, 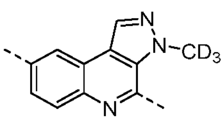, 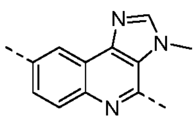, 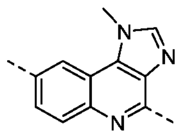,

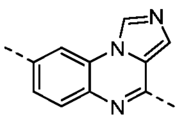, 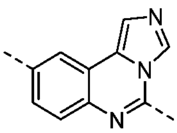, 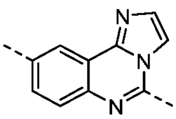, 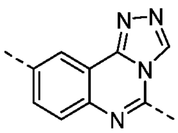, 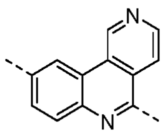, 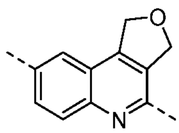,

and

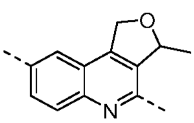,

and other variables are as defined in the present disclosure.

In some embodiments of the present disclosure, the structural moiety

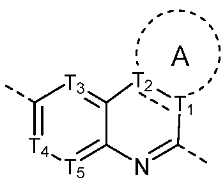

is selected from

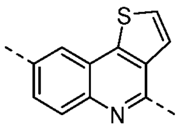 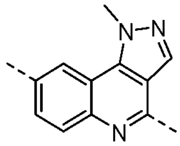, 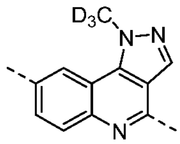,

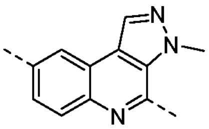, 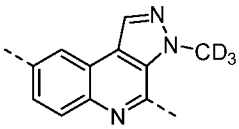, 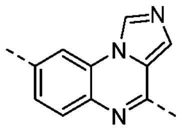, 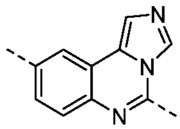, 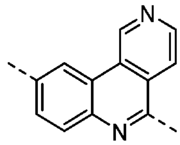,

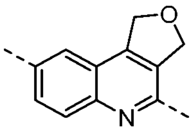, and 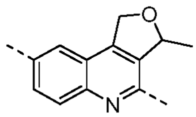,

and other variables are as defined in the present disclosure.

In some embodiments of the present disclosure, the structural moiety

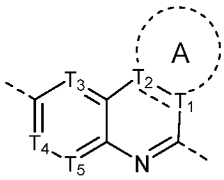

is selected from

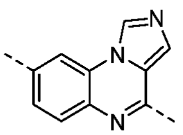, 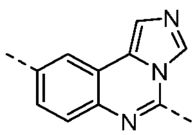, 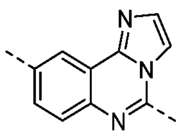,

and

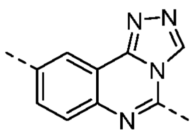,

and other variables are as defined in the present disclosure.

In some embodiments of the present disclosure, the $E_1$ is selected from O, and other variables are as defined in the present disclosure.

In some embodiments of the present disclosure, the compound or the pharmaceutically acceptable salt thereof is selected from:

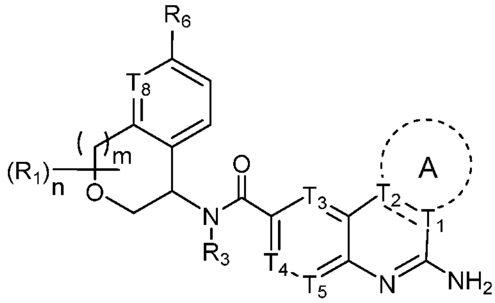

(P)

,

wherein

the structural moiety

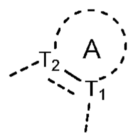

is selected from

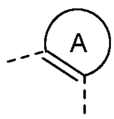 , 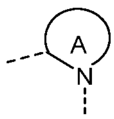 , and 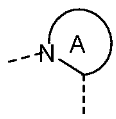 ;

ring A is selected from 5- to 6-membered heteroaryl or 5- to 6-membered heterocycloalkenyl, and each 5- to 6-membered heteroaryl or 5- to 6-membered heterocycloalkenyl is independently and optionally substituted by 1, 2, or 3 $R_c$;
$T_3$, $T_4$, and $T_5$ are each independently selected from $CR_4$;
each $T_8$ is independently selected from CH and N;
each $R_1$ is independently selected from H, halogen, and $C_{1-3}$ alkyl, and the $C_{1-3}$ alkyl is optionally substituted by 1, 2, or 3 $R_a$;
$R_3$ is selected from H and $C_{1-3}$ alkyl, and the $C_{1-3}$ alkyl is optionally substituted by 1, 2, or 3 $R_e$;
$R_4$ is selected from H, halogen, and $C_{1-3}$ alkyl, and the $C_{1-3}$ alkyl is optionally substituted by 1, 2, or 3 $R_d$;
$R_6$ is selected from $C_{2-4}$ alkynyl and $C_{2-4}$ alkenyl, and the $C_{2-4}$ alkynyl and $C_{2-4}$ alkenyl are each independently and optionally substituted by 1, 2, or 3 $R_b$;
$R_a$, $R_c$, $R_a$, and $R_e$ are each independently selected from H, D, halogen, OH, $NH_2$, $CH_3$, and $CD_3$;
each $R_b$ is independently selected from H, D, halogen, $C_{1-3}$ alkyl, $C_{1-3}$ alkylamino, and 4- to 6-membered heterocycloalkyl, and the $C_{1-3}$ alkyl, $C_{1-3}$ alkoxy, and $C_{1-3}$ alkylamino are each independently and optionally substituted by 1, 2, or 3 R;
each R is independently selected from H, D, halogen, OH, $NH_2$, $CH_3$, $CF_3$, and $CD_3$;
n is selected from 0, 1, 2, and 3;
m is selected from 0 and 1.

In some embodiments of the present disclosure, the compound or the pharmaceutically acceptable salt thereof is selected from:

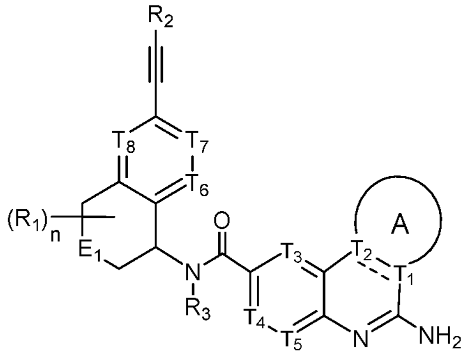

(I)

,

wherein

each $R_1$ is independently selected from H, halogen, and $C_{1-3}$ alkyl, and the $C_{1-3}$ alkyl is optionally substituted by 1, 2, or 3 $R_a$;
$R_2$ is selected from H, halogen, and $C_{1-3}$ alkyl, and the $C_{1-3}$ alkyl is optionally substituted by 1, 2, or 3 $R_b$;
$E_1$ is selected from O;
the structural moiety

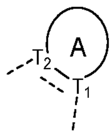

is selected from

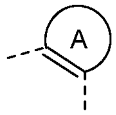 , 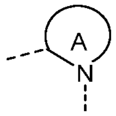 , and 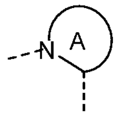 ;

ring A is selected from 5- to 6-membered heteroaryl and 5- to 6-membered heterocycloalkenyl, and the 5- to 6-membered heteroaryl and 5- to 6-membered heterocycloalkenyl are each independently and optionally substituted by 1, 2, or 3 $R_c$;
$T_3$, $T_4$, and $T_5$ are each independently selected from $CR_4$;
$T_6$ and $T_7$ are each independently CH;
$T_8$ is selected from CH and N;
$R_3$ is selected from H and $C_{1-3}$ alkyl, and the $C_{1-3}$ alkyl is optionally substituted by 1, 2, or 3 $R_e$;
$R_4$ is selected from H, halogen, and $C_{1-3}$ alkyl, and the $C_{1-3}$ alkyl is optionally substituted by 1, 2, or 3 $R_a$;
$R_a$, $R_b$, $R_c$, $R_d$, and $R_e$ are each independently selected from H, halogen, OH, $NH_2$, $CH_3$, and $CD_3$;
n is selected from 0, 1, 2, and 3;
"hetero" in the 5- to 6-membered heteroaryl or 5- to 6-membered heterocycloalkenyl represents 1, 2, 3, or 4 heteroatoms or heteroatom groups independently selected from -O-, - NH-, -S-, and N.

In some embodiments of the present disclosure, the compound of formula (P) or formula (I) or the pharmaceutically acceptable salt thereof, wherein ring A is selected from 5-to 6-membered heteroaryl, and the 5- to 6-membered heteroaryl is optionally substituted by 1, 2, or 3 $R_c$, and other variables are as defined in the present disclosure.
In some embodiments of the present disclosure, the compound of formula (P) or formula (I) or the pharmaceutically acceptable salt thereof, wherein ring A is selected from 5-membered heteroaryl, and the 5-membered heteroaryl is optionally substituted by 1, 2, or 3 $R_c$, and other variables are as defined in the present disclosure.
In some embodiments of the present disclosure, the compound of formula (P) or formula (I) or the pharmaceutically acceptable salt thereof, wherein ring A is selected from imidazolyl, pyrazolyl, pyrrolyl, thiazolyl, thienyl, furanyl, and oxazolyl, and the imidazolyl, pyrazolyl, pyrrolyl, thiazolyl, thienyl, furanyl, and oxazolyl are each independently and optionally substituted by 1, 2, or 3 $R_c$, and other variables are as defined in the present disclosure.

In some embodiments of the present disclosure, the compound of formula (P) or formula (I) or the pharmaceutically acceptable salt thereof, wherein ring A is selected from imidazolyl and pyrrolyl, and the imidazolyl and pyrrolyl are each independently and optionally substituted by 1, 2, or 3 $R_c$, and other variables are as defined in the present disclosure.

In some embodiments of the present disclosure, the compound of formula (P) or formula (I) or the pharmaceutically acceptable salt thereof, wherein $T_4$ and $T_5$ are each independently selected from CH, $T_8$ is independently selected from CH and N, and other variables are as defined in the present disclosure.

In some embodiments of the present disclosure, the compound of formula (P) or formula (I) or the pharmaceutically acceptable salt thereof, wherein each $R_1$ is independently selected from F and $CH_3$, and other variables are as defined in the present disclosure.

In some embodiments of the present disclosure, the compound of formula (P) or formula (I) or the pharmaceutically acceptable salt thereof, wherein each $R_1$ is independently selected from $CH_3$, and other variables are as defined in the present disclosure.

In some embodiments of the present disclosure, the compound of formula (P) or formula (I) or the pharmaceutically acceptable salt thereof, wherein each $R_3$ is selected from H, $CH_3$, $CF_3$, $CD_3$, $CH_2CH_3$, and $CH(CH_3)_2$, and other variables are as defined in the present disclosure.

In some embodiments of the present disclosure, the compound of formula (P) or formula (I) or the pharmaceutically acceptable salt thereof, wherein n is selected from 0 and 1, and other variables are as defined in the present disclosure.

In some embodiments of the present disclosure, the compound of formula (P) or the pharmaceutically acceptable salt thereof, wherein $R_6$ is selected from ethynyl and propynyl, and the ethynyl and propynyl are each independently and optionally substituted by 1, 2, or 3 $R_b$, and other variables are as defined in the present disclosure.

In some embodiments of the present disclosure, the compound of formula (P) or the pharmaceutically acceptable salt thereof, wherein $R_6$ is selected from ethynyl and propynyl, and the ethynyl and propynyl are each independently and optionally substituted by 1, 2, or 3 $R_b$, and other variables are as defined in the present disclosure.

In some embodiments of the present disclosure, the compound of formula (P) or the pharmaceutically acceptable salt thereof, wherein $R_6$ is selected from

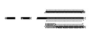 , 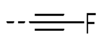 ,

 , 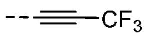 , 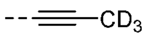 , 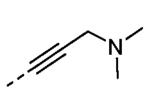 , 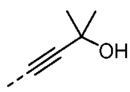 , and 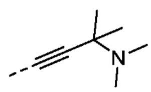 ,

and other variables are as defined in the present disclosure.

In some embodiments of the present disclosure, the compound of formula (P) or the pharmaceutically acceptable salt thereof, wherein $R_6$ is selected from

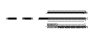 , 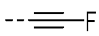 ,

 , 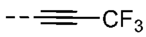 , and 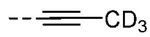 ,

and other variables are as defined in the present disclosure.

In some embodiments of the present disclosure, the compound of formula (P) or the pharmaceutically acceptable salt thereof, wherein the structural moiety

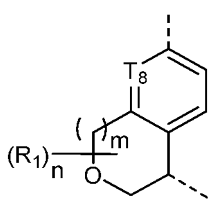

is selected from

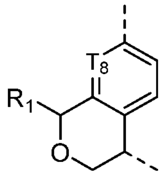 and 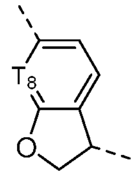,

and other variables are as defined in the present disclosure.

In some embodiments of the present disclosure, the compound of formula (I) or the pharmaceutically acceptable salt thereof, wherein $E_1$ is O, and other variables are as defined in the present disclosure.

There are still some embodiments of the present disclosure which are obtained by any combination of the above variables.

The present disclosure further provides a compound of the following formula or a pharmaceutically acceptable salt thereof,

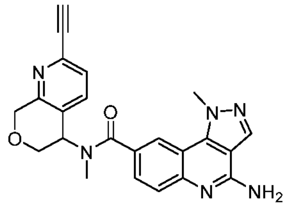
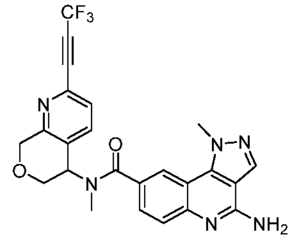
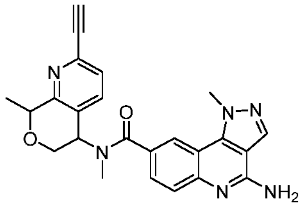
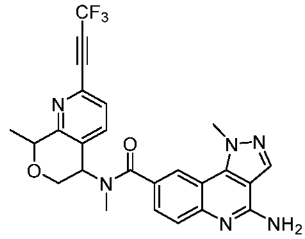

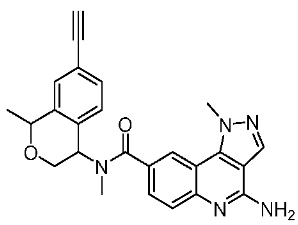
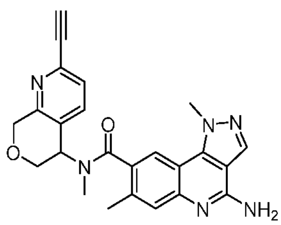
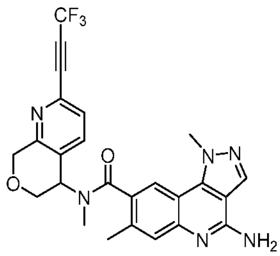
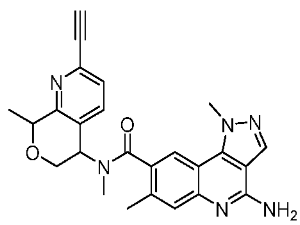

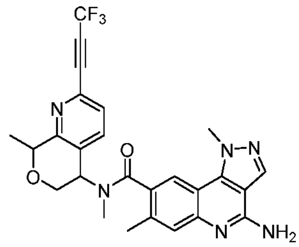
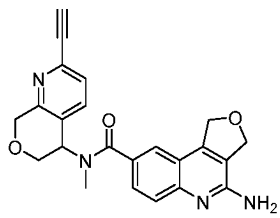
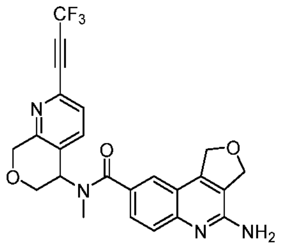
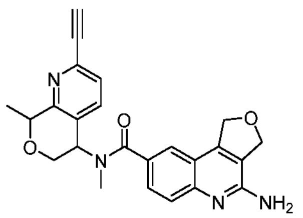

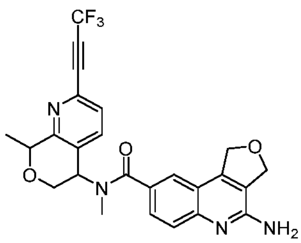
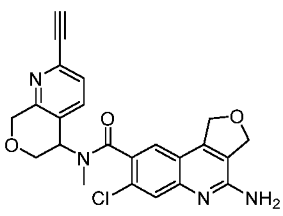
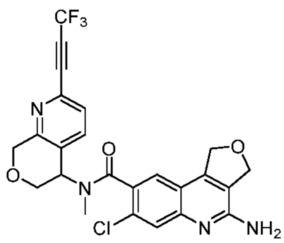
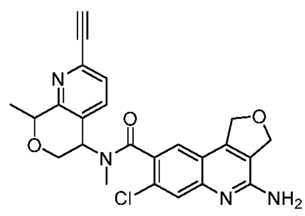

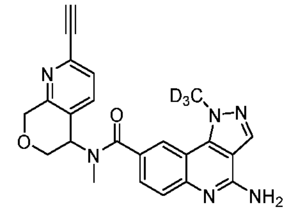
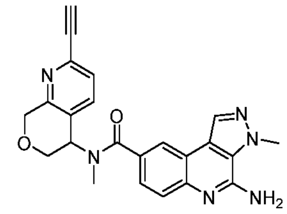
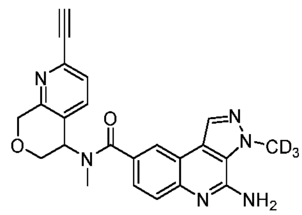
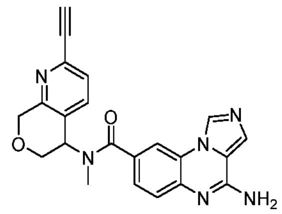

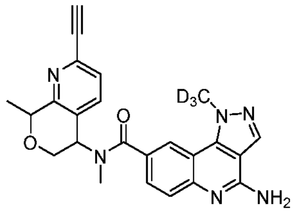
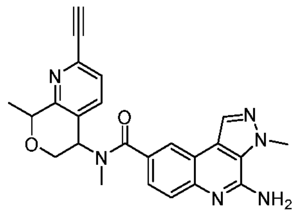
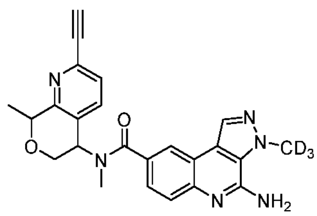
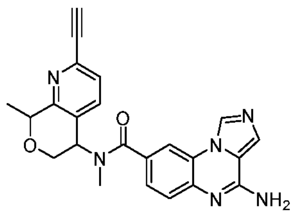

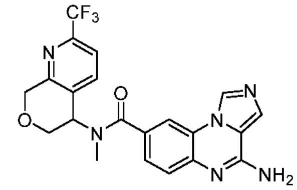
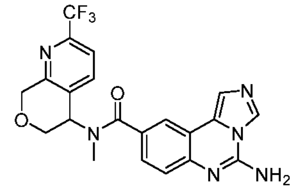
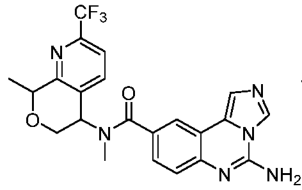
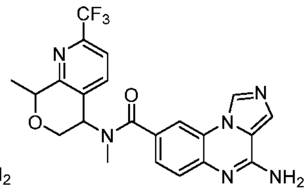

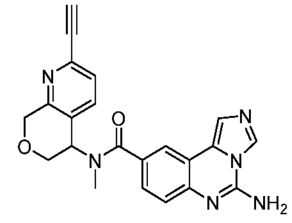
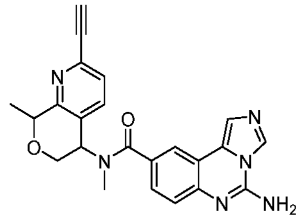
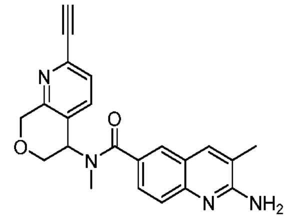
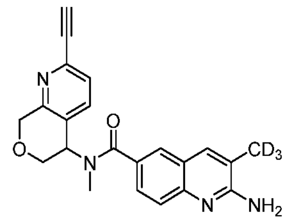

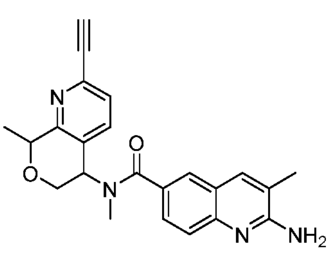
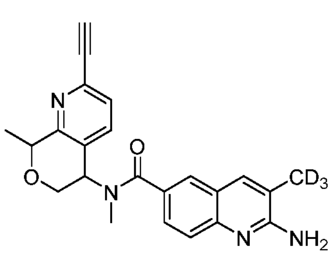
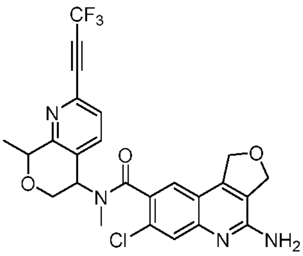

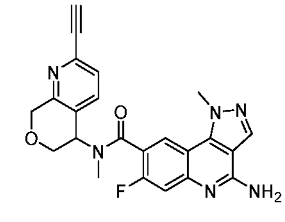
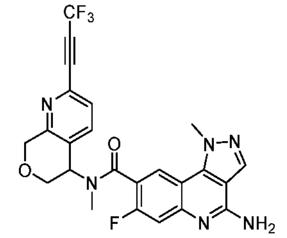
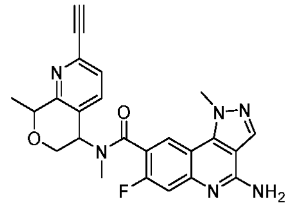
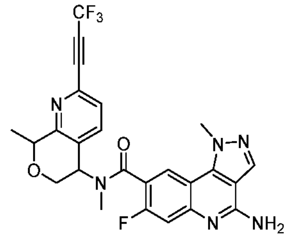

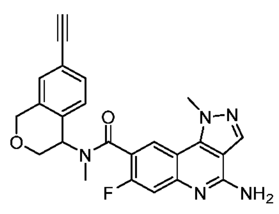
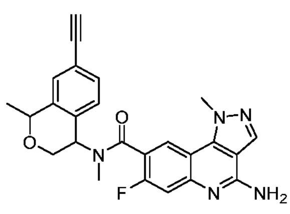
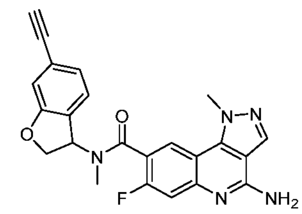
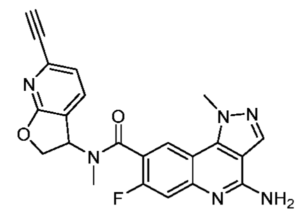

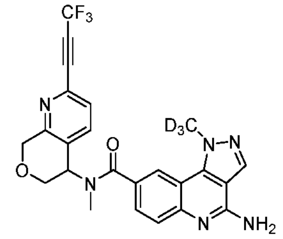
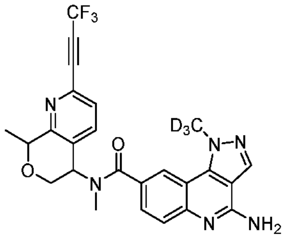
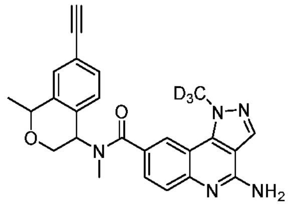
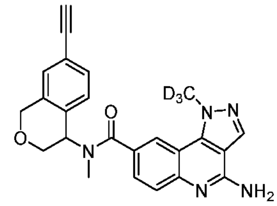

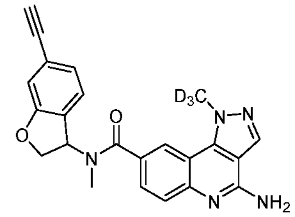
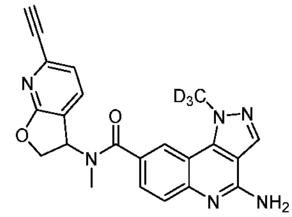
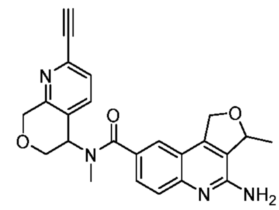
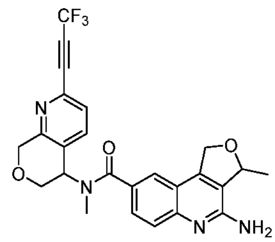

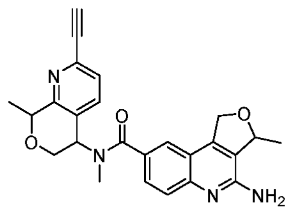
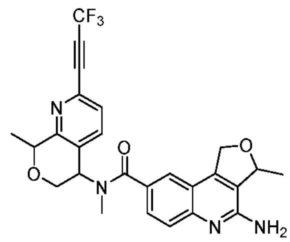
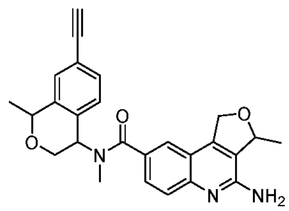
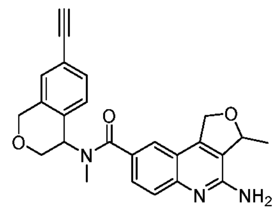

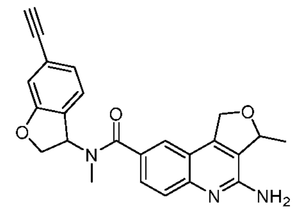
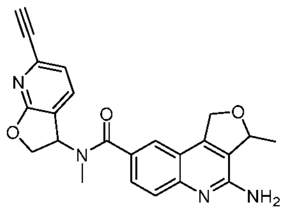
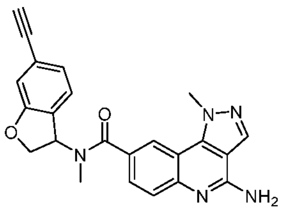
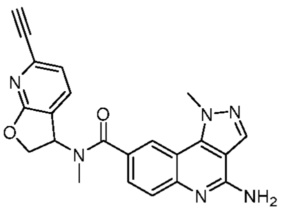

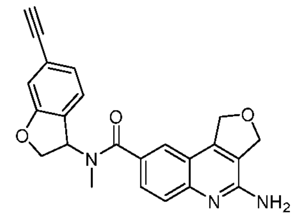
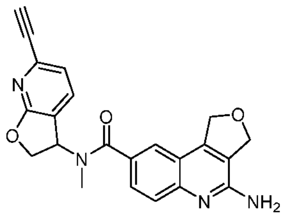
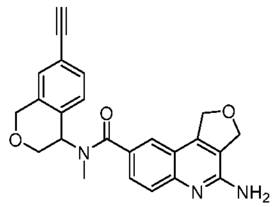
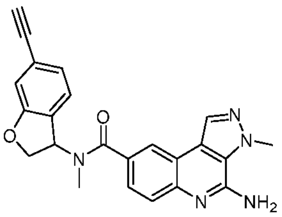

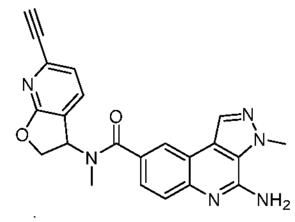
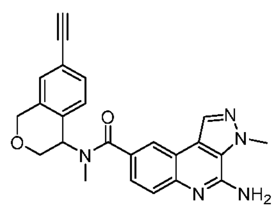
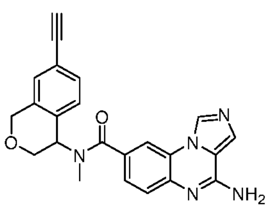
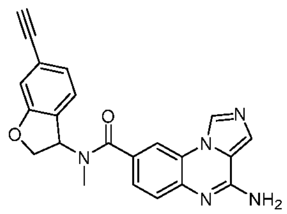

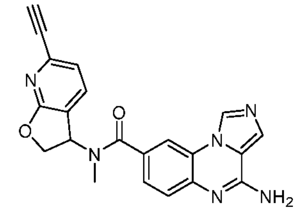 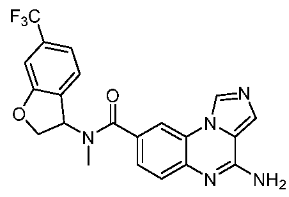 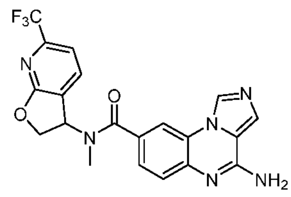 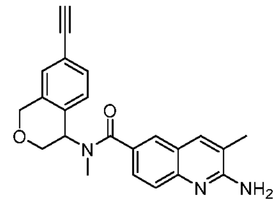

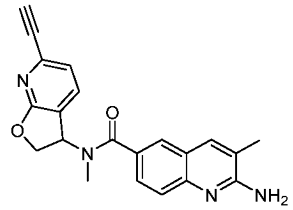 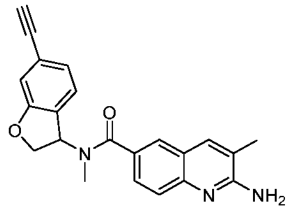 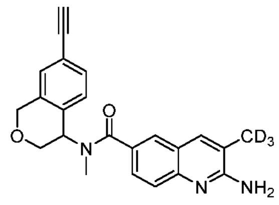 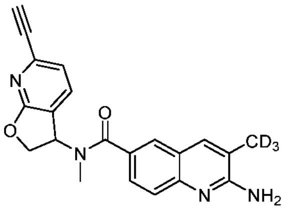

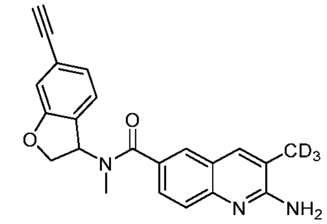

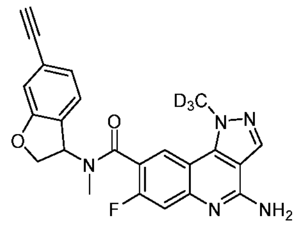 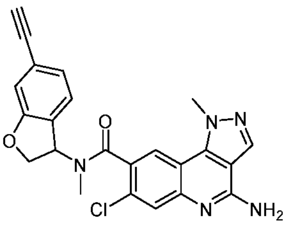 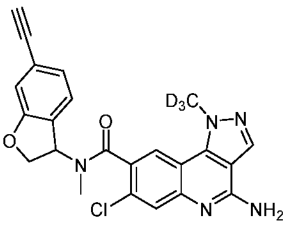 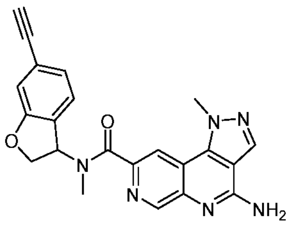

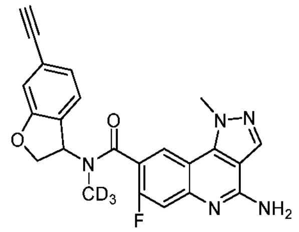 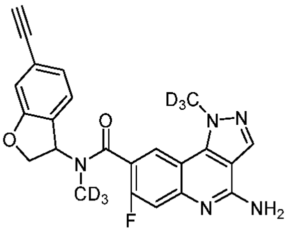 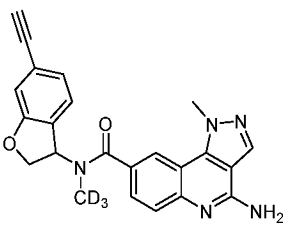 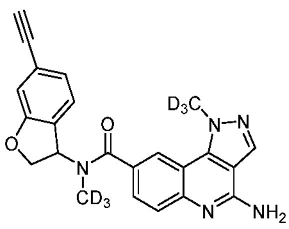

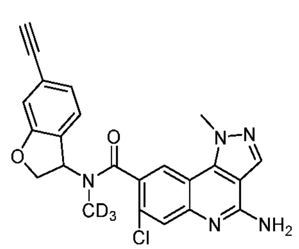 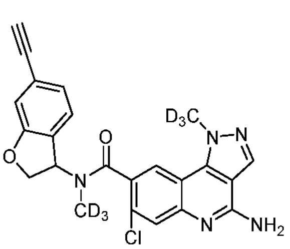 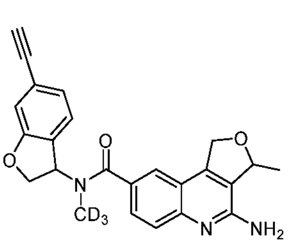 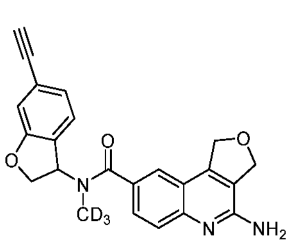

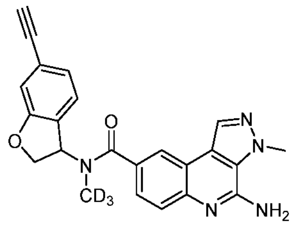
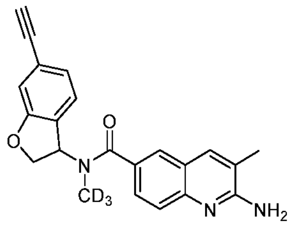
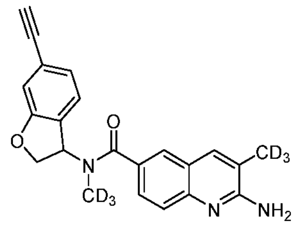
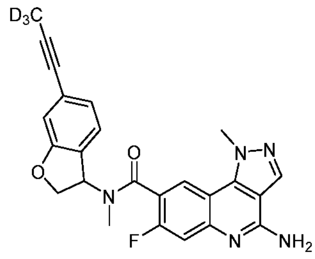

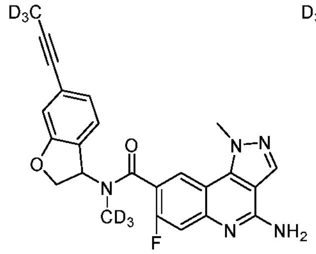
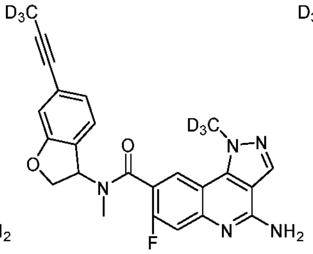
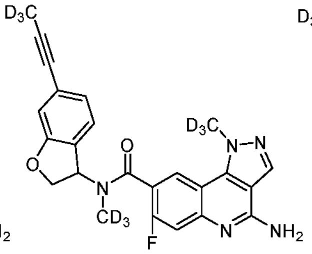
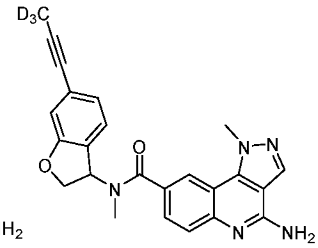

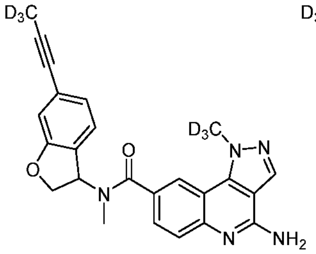
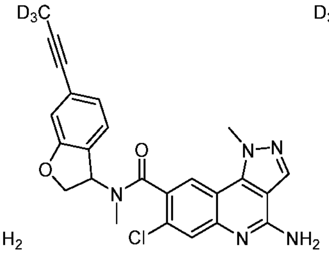
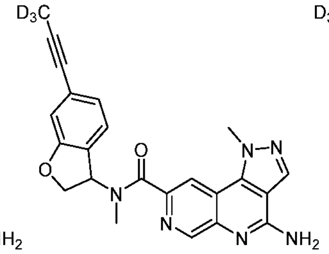
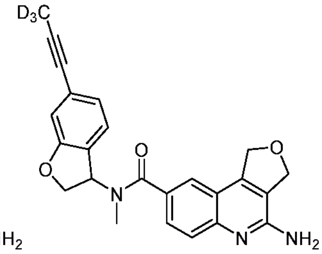

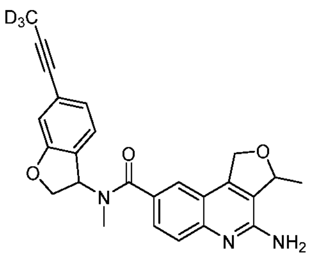
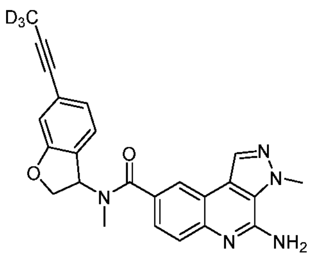
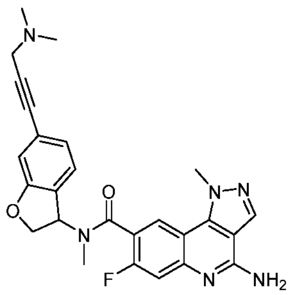
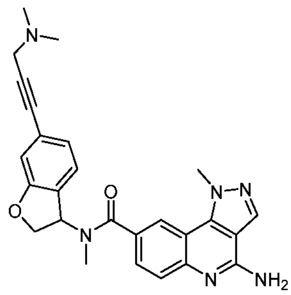

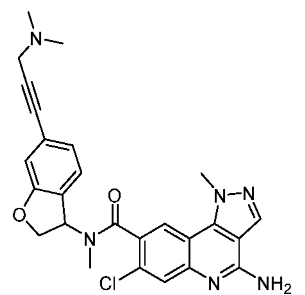
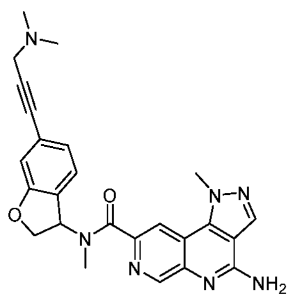
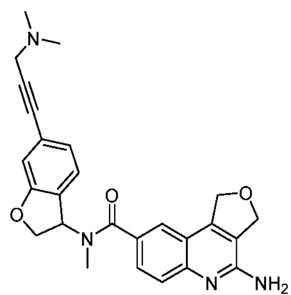
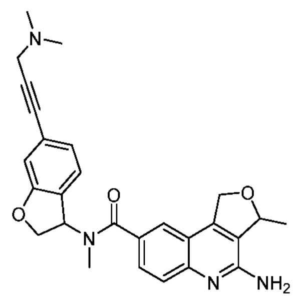

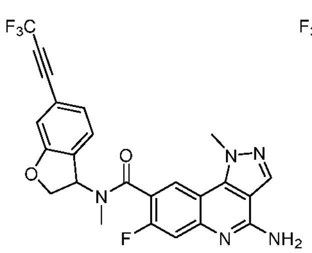
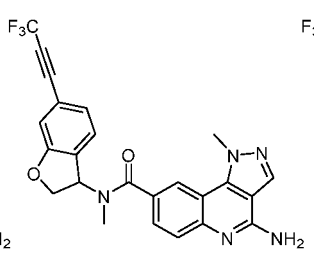
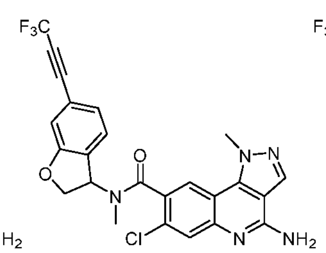
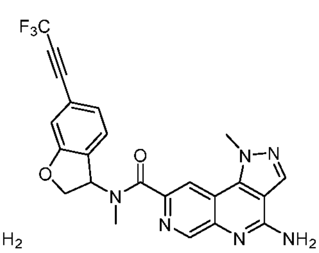

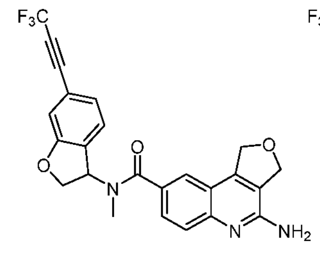
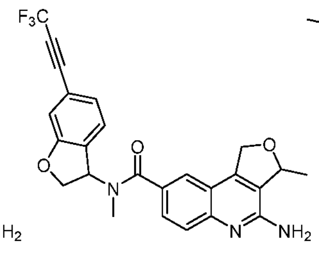
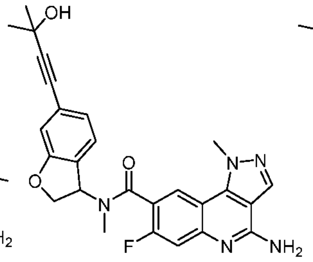
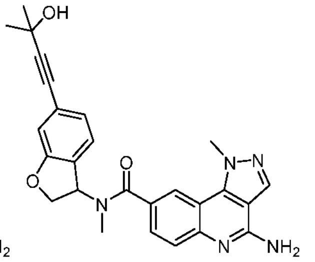
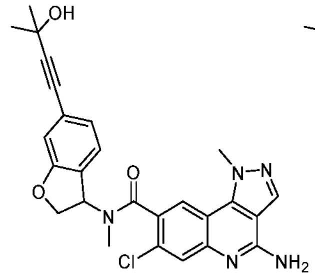
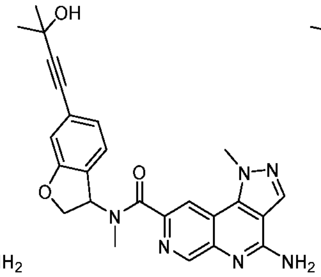
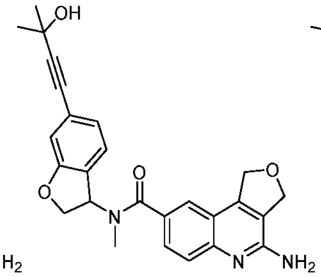
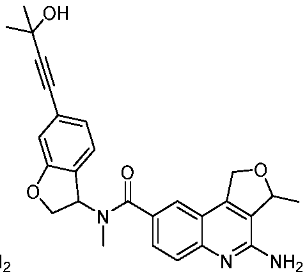
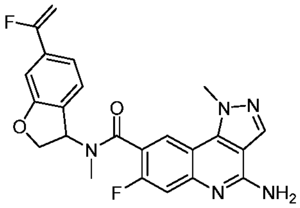
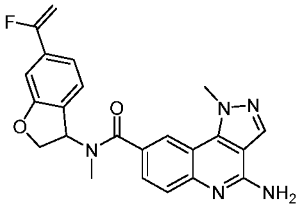
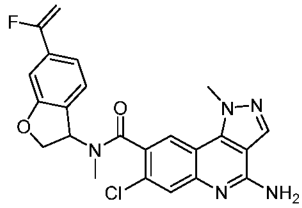
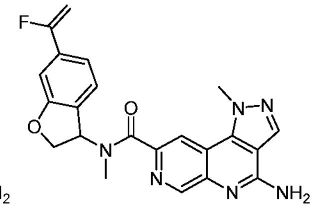
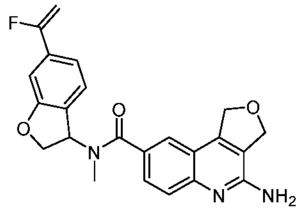
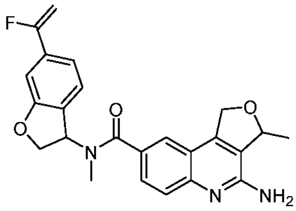
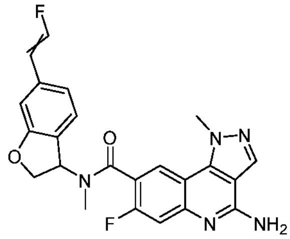
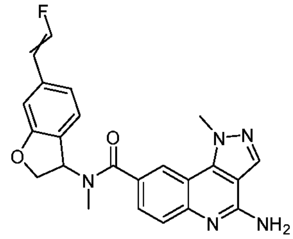
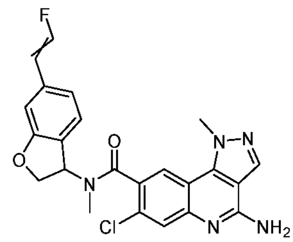
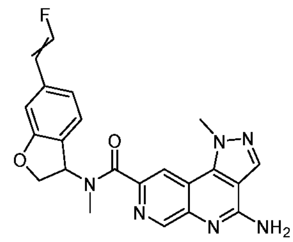
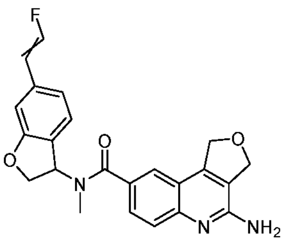
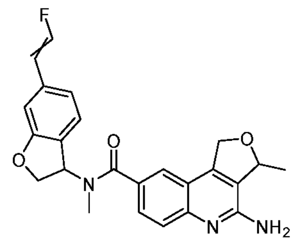
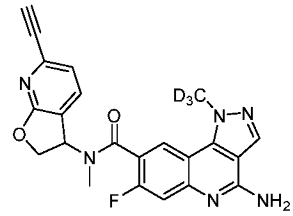
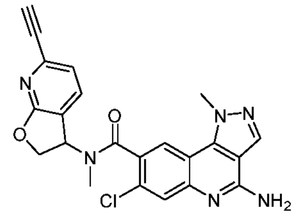
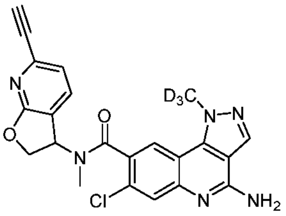
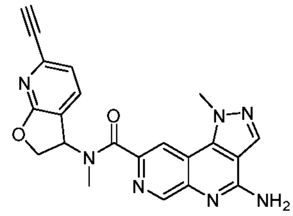

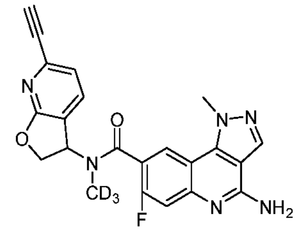
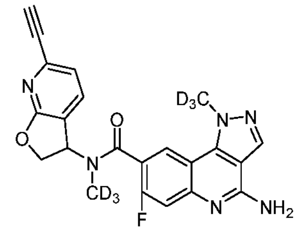
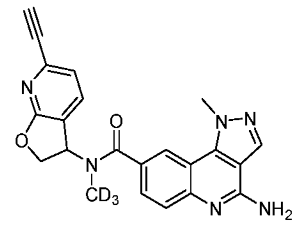
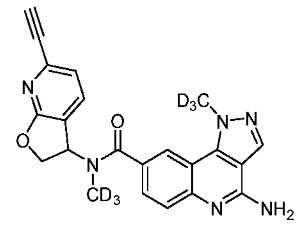
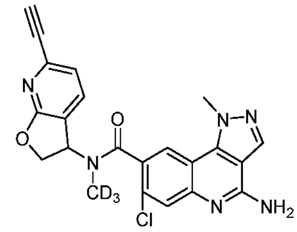
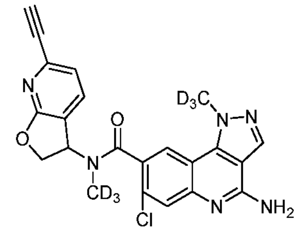
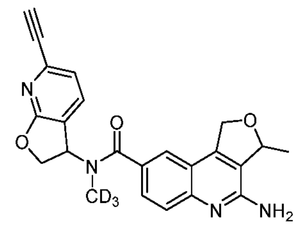
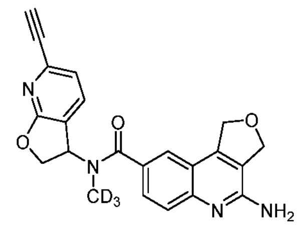
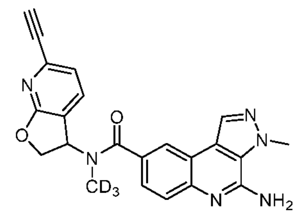
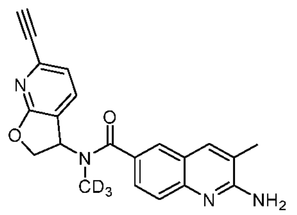
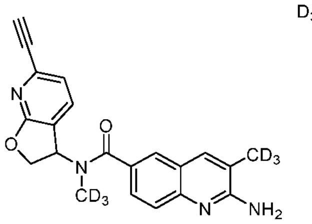
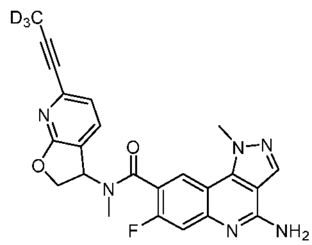
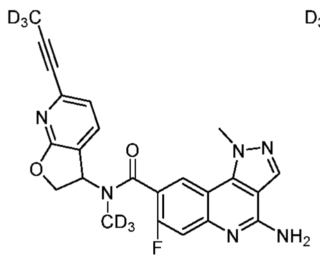
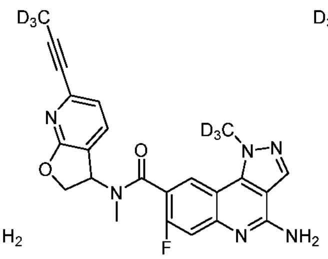
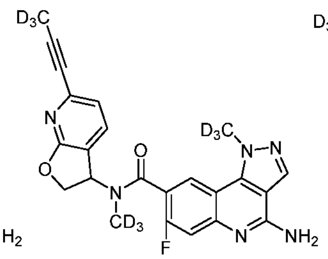
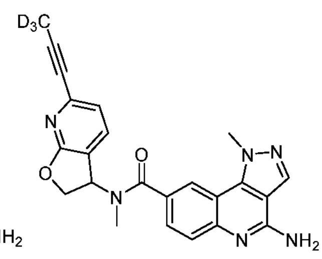
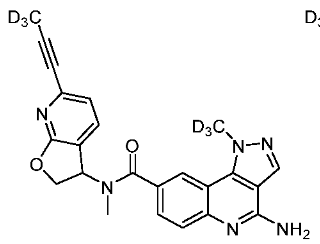
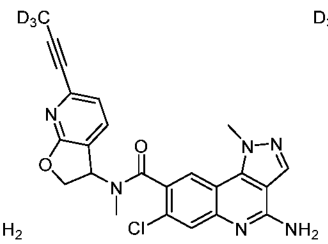
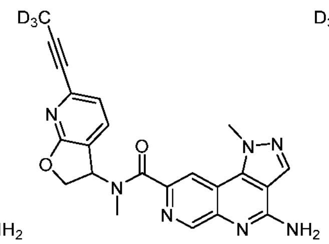
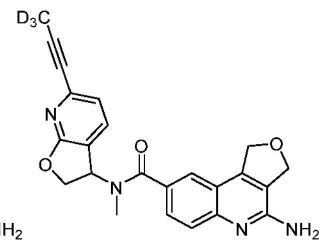
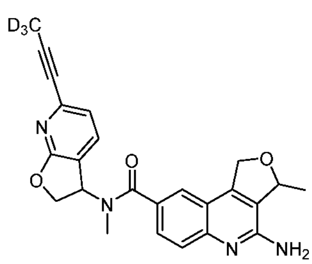
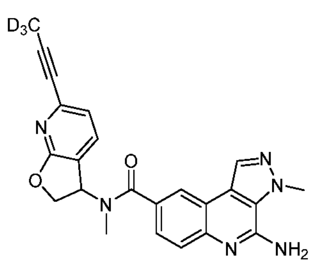
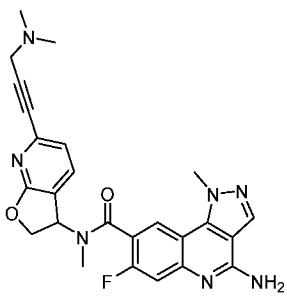
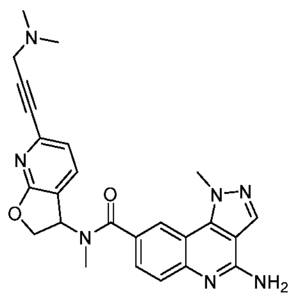

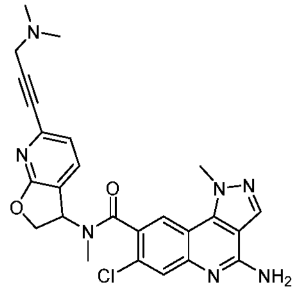
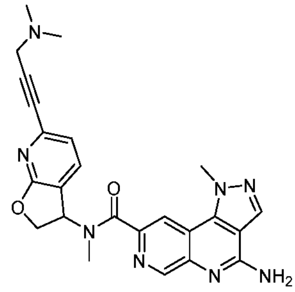
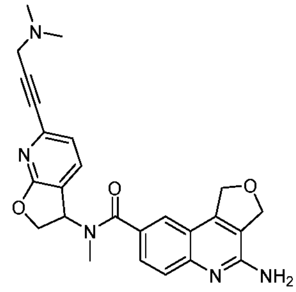
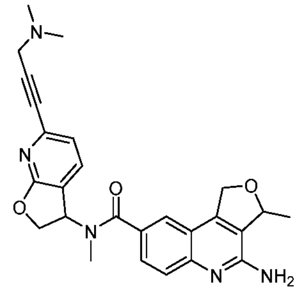

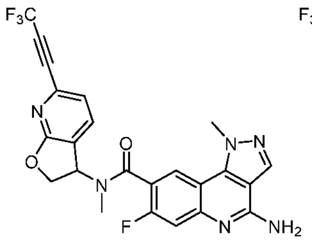
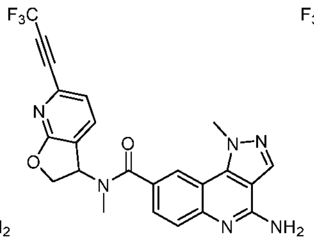
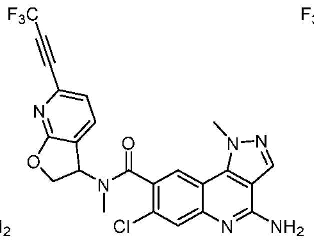
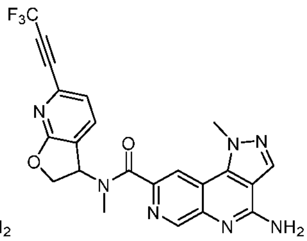

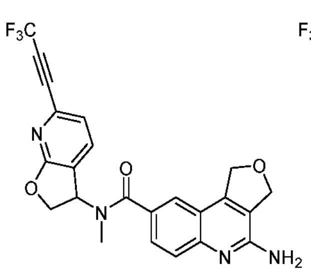
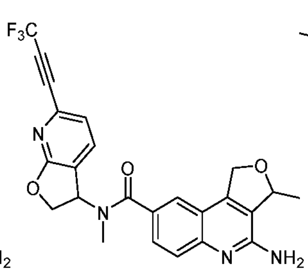
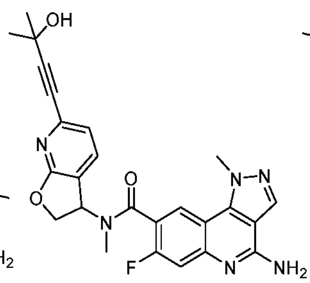
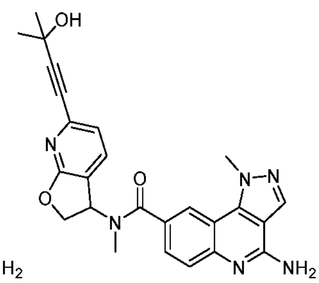

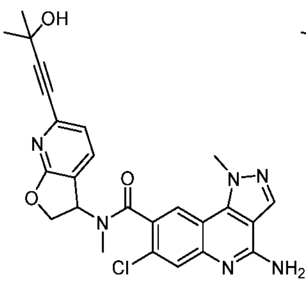
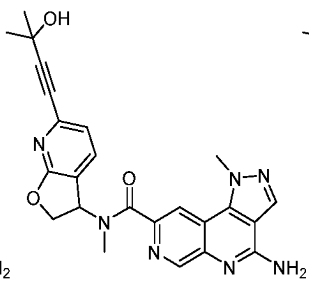
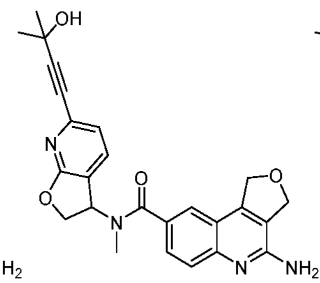
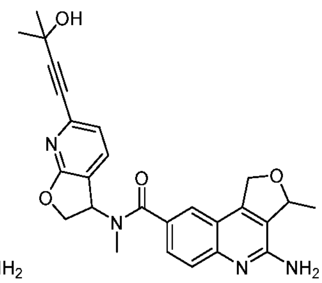

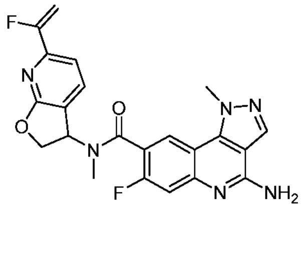
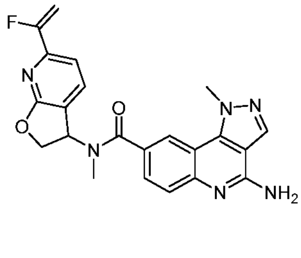
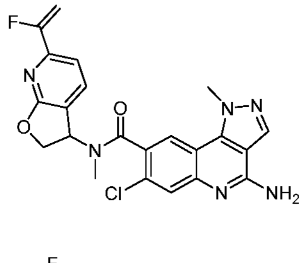
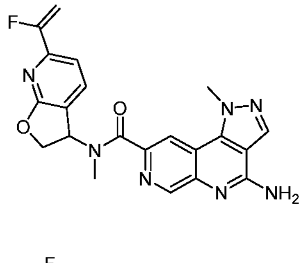

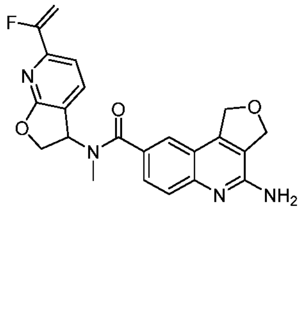
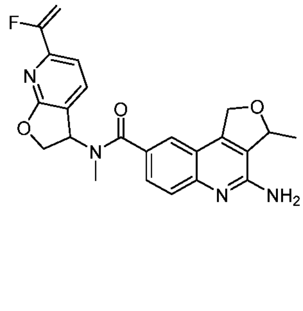
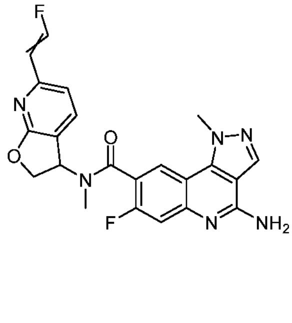
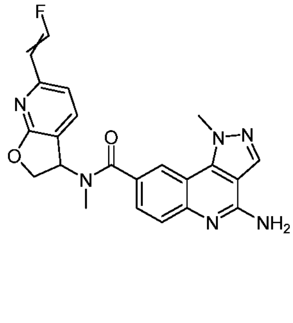

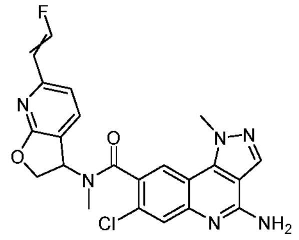 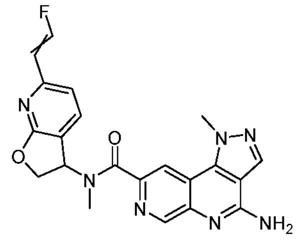 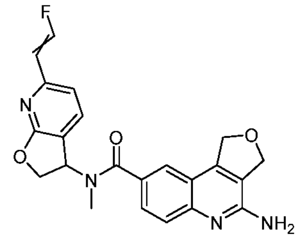 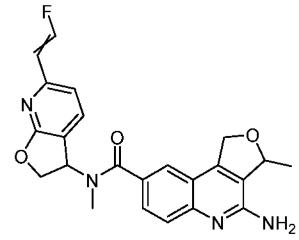

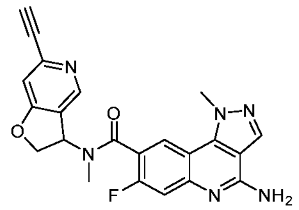 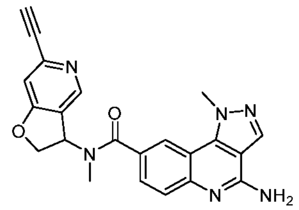 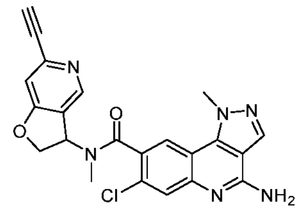 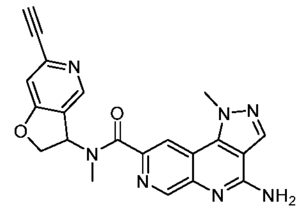

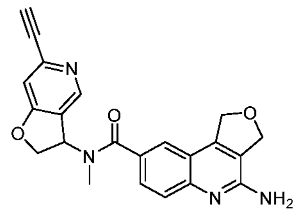 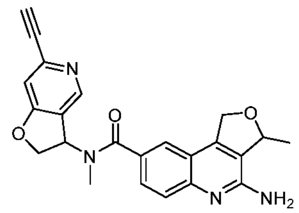 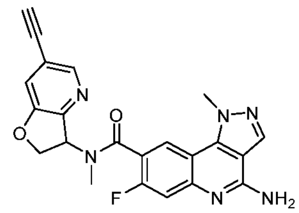 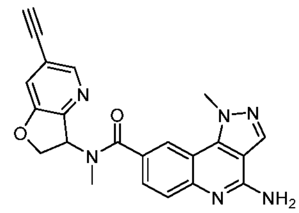

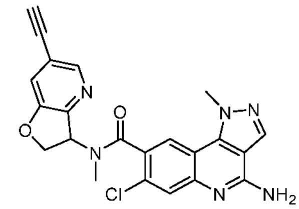 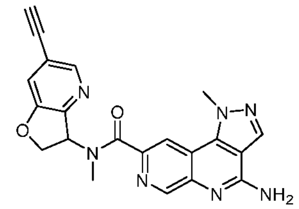 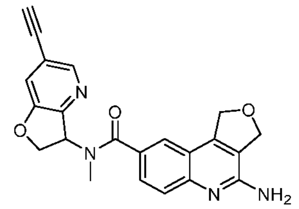 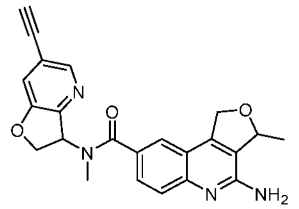

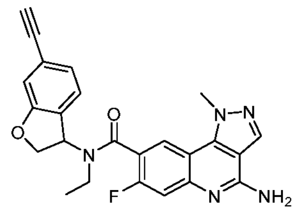 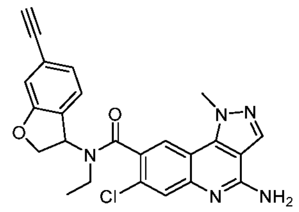 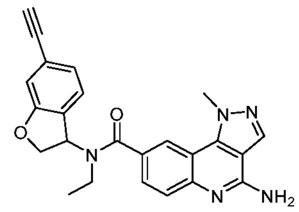 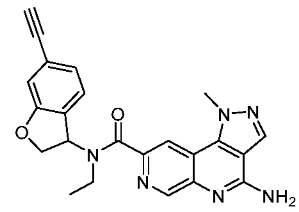

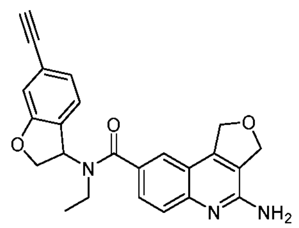 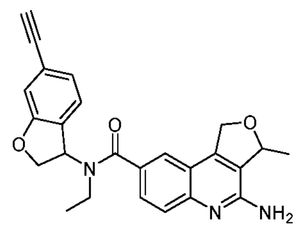 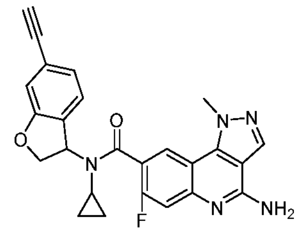 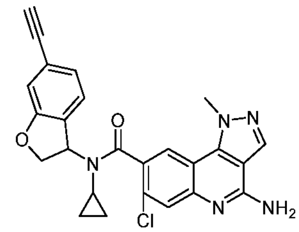

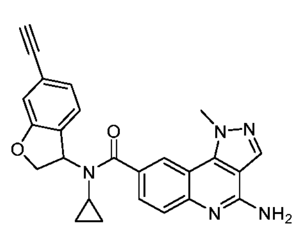 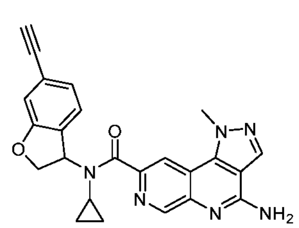 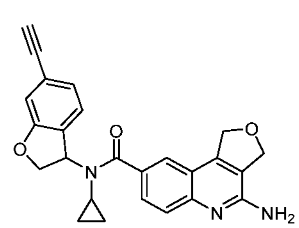 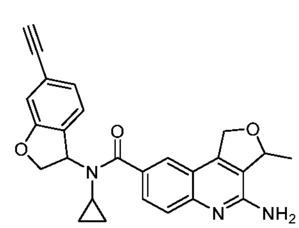

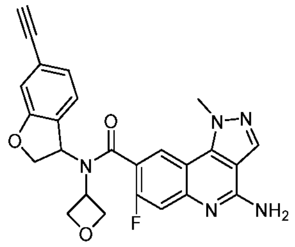
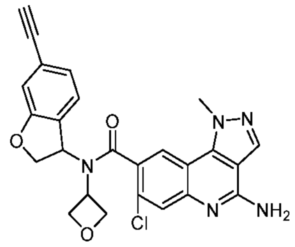
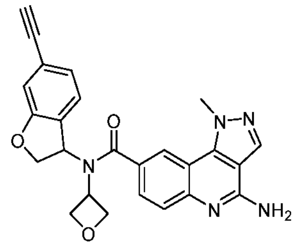
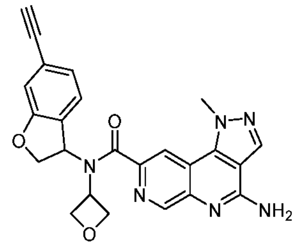
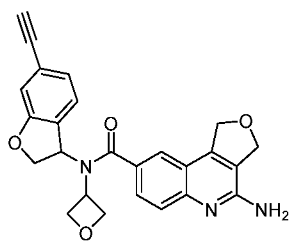
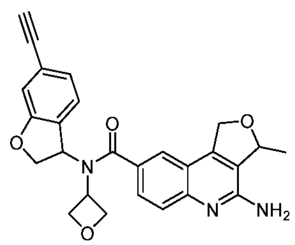
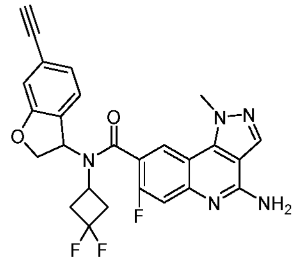
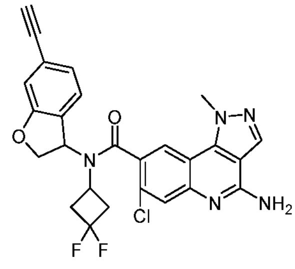
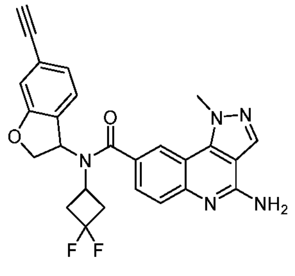
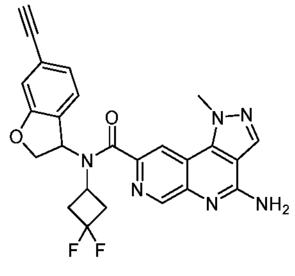
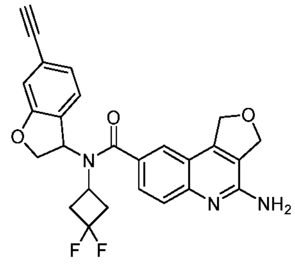
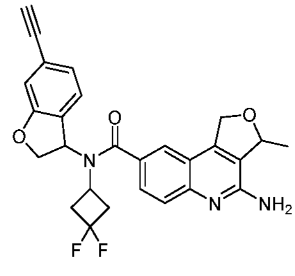
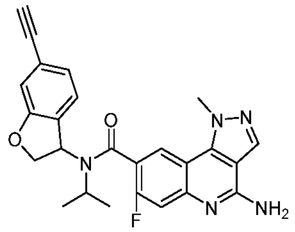
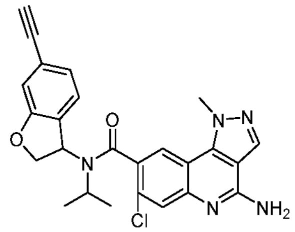
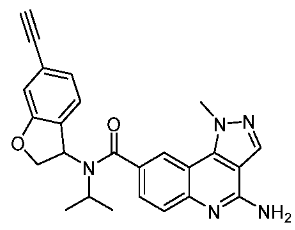
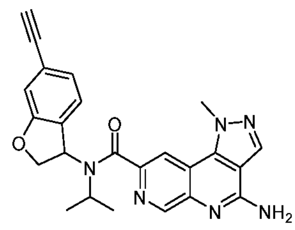
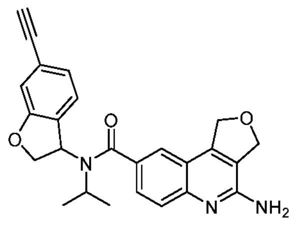
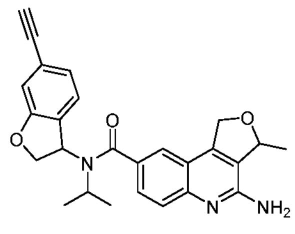
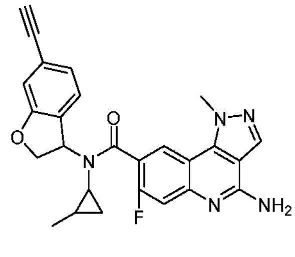
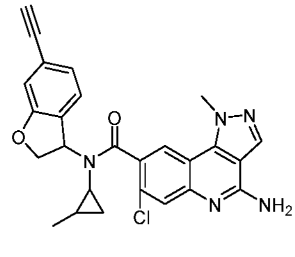
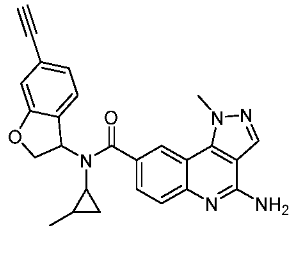
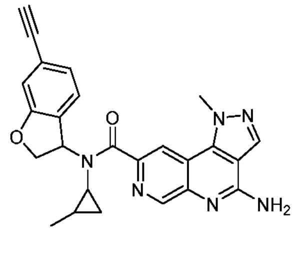
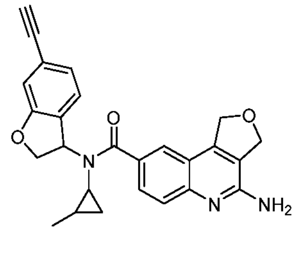
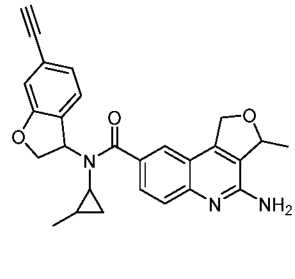

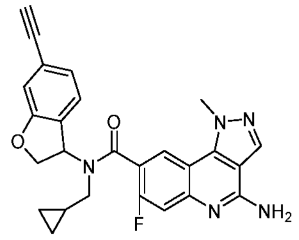
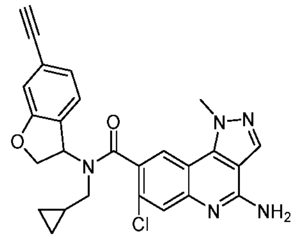
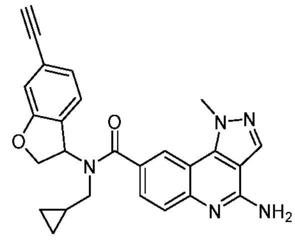
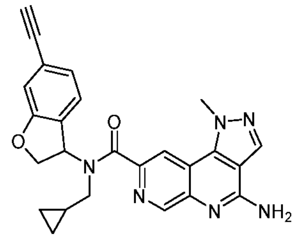

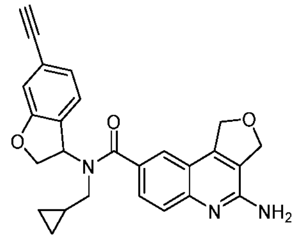
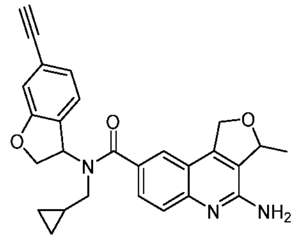
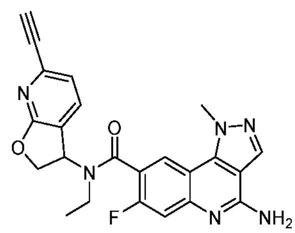
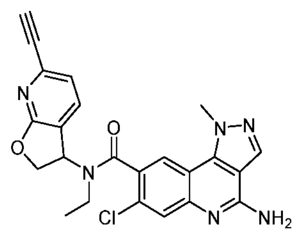

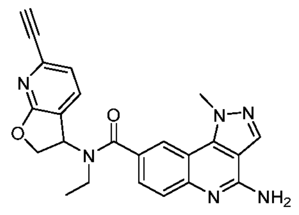
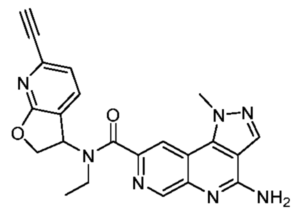
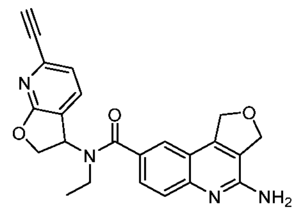
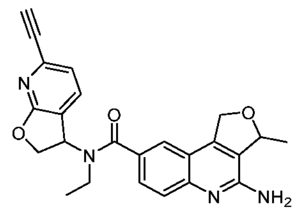

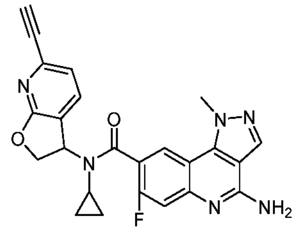
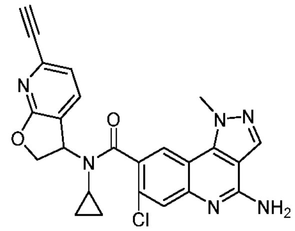
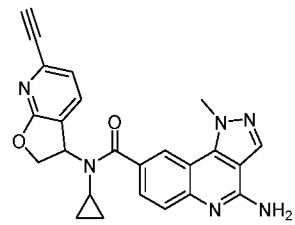
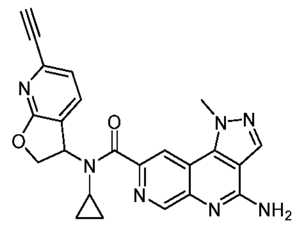

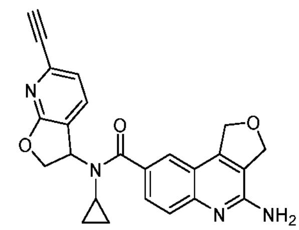
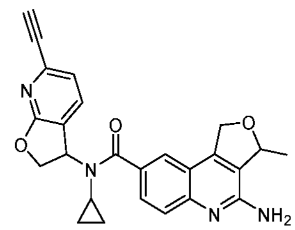
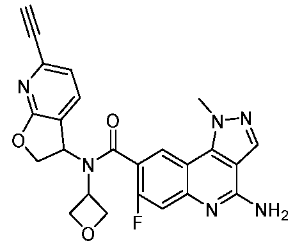
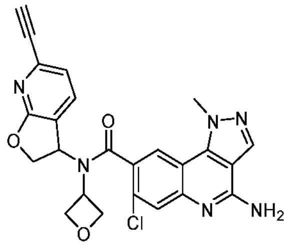

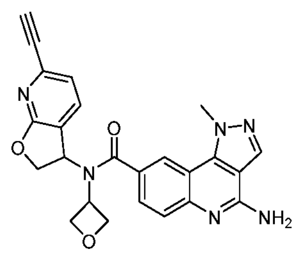
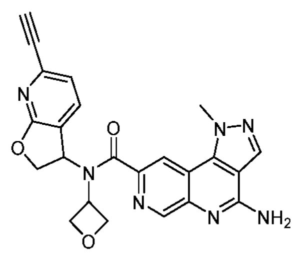
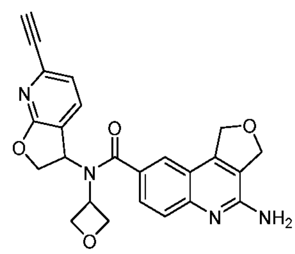
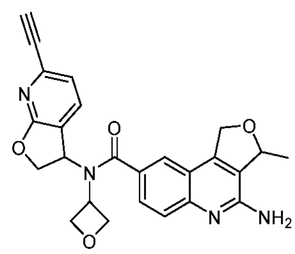

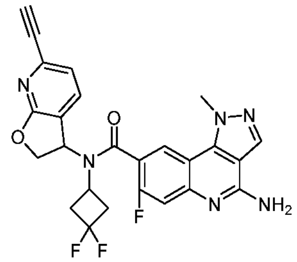
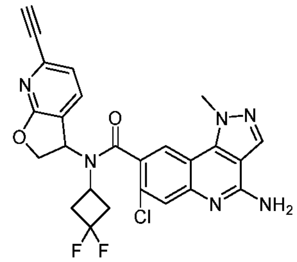
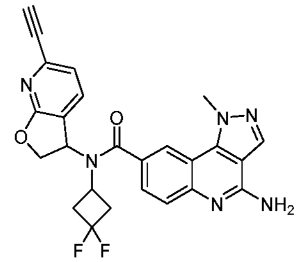
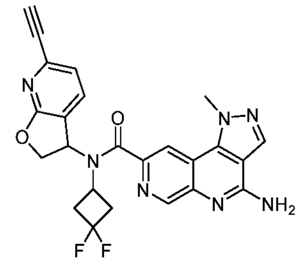

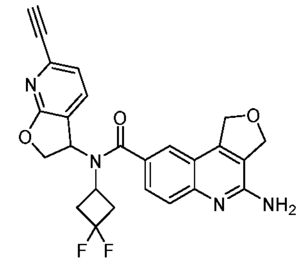
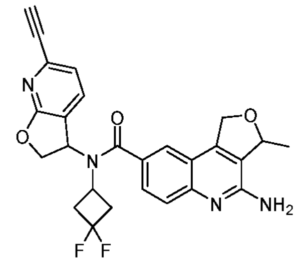
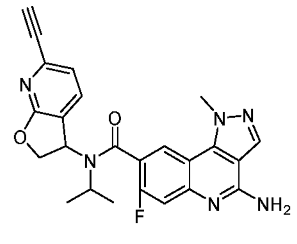
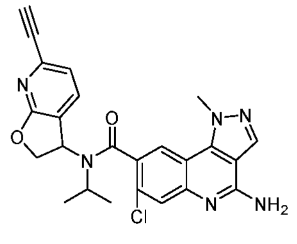

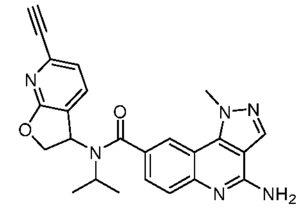
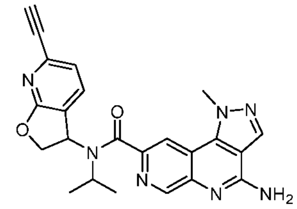
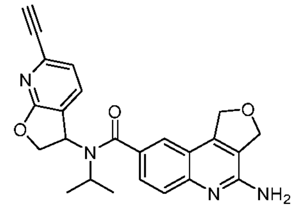
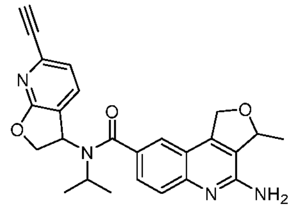

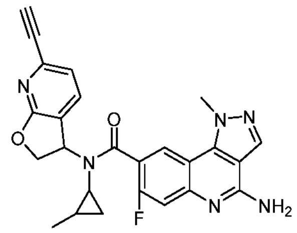
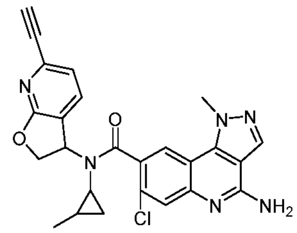
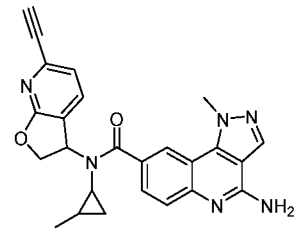
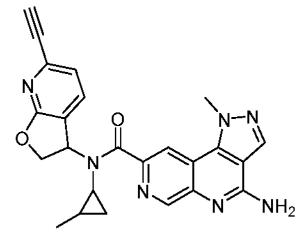

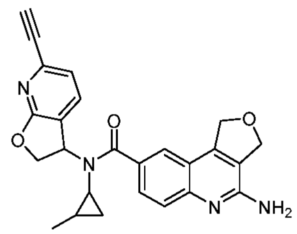
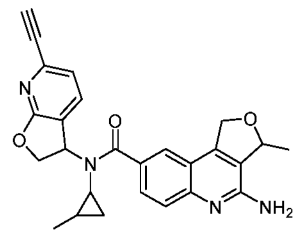
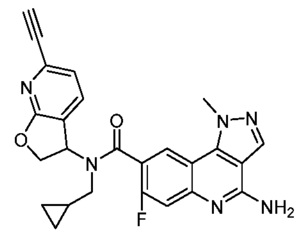
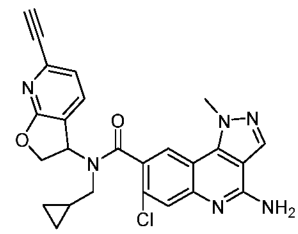

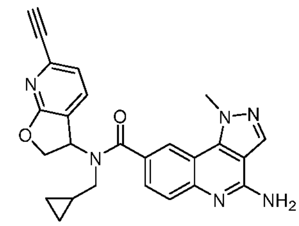
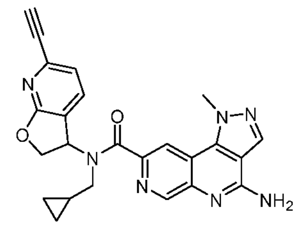
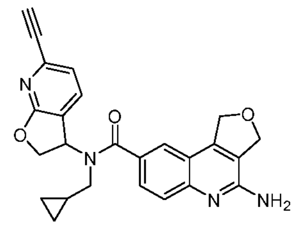
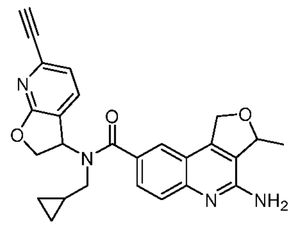

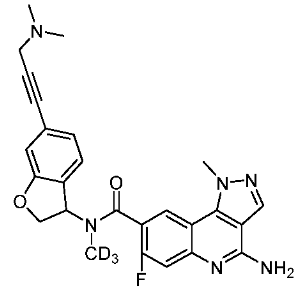
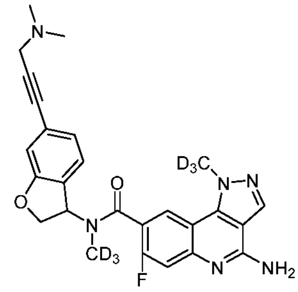
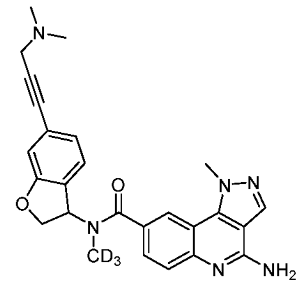
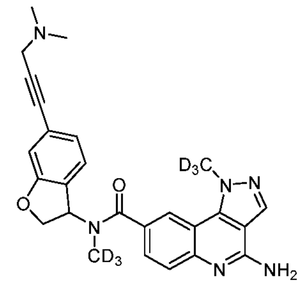

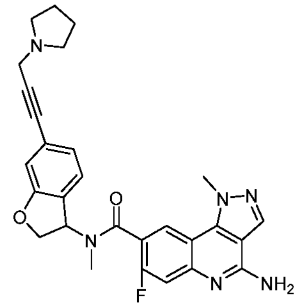
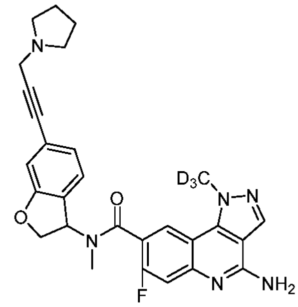
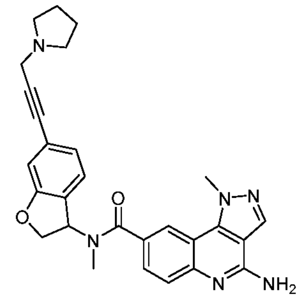
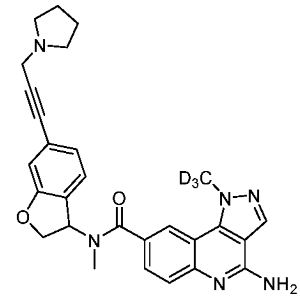

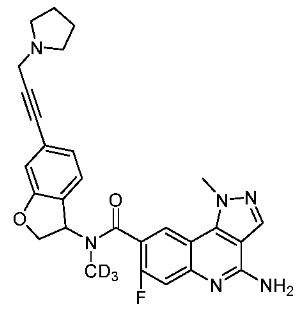
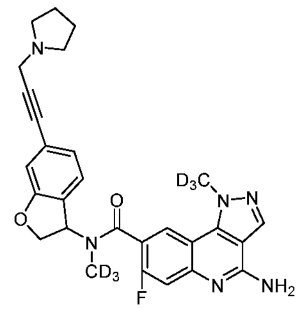
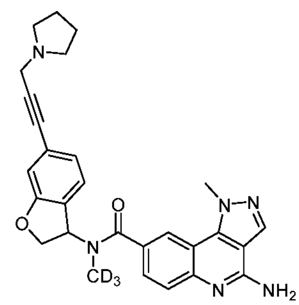
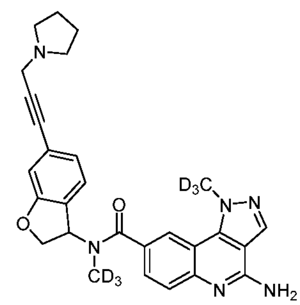

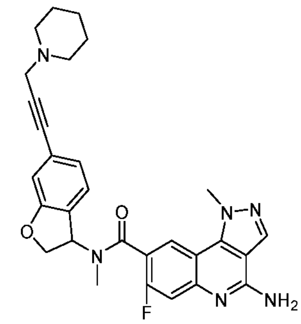
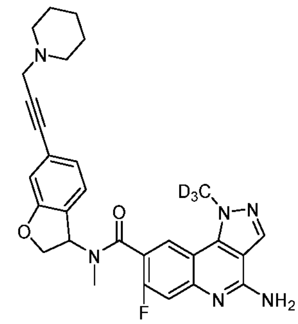
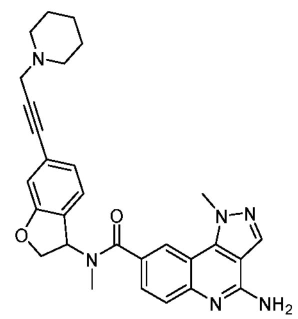
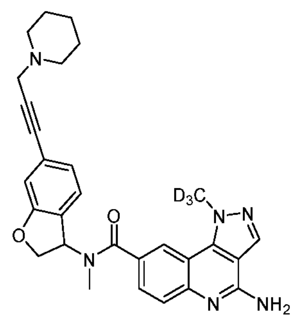

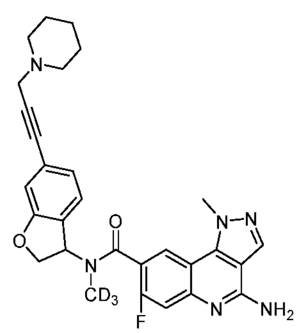
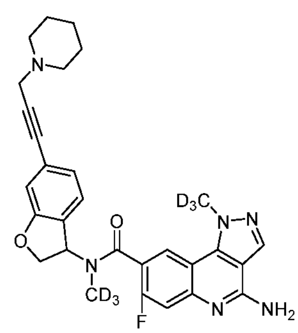
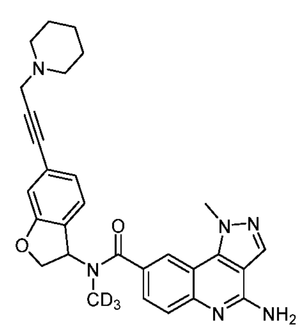
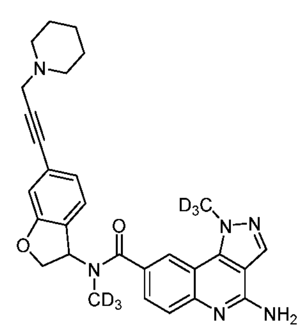

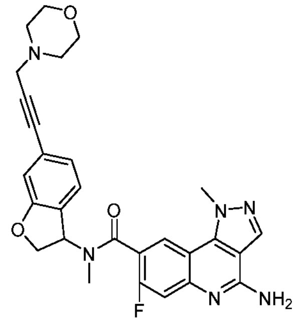
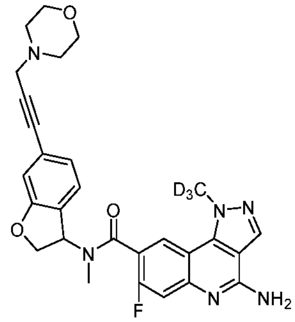
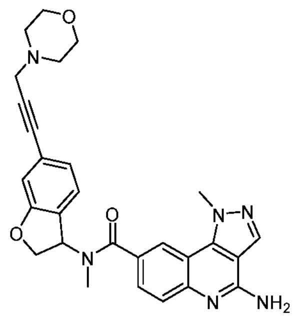
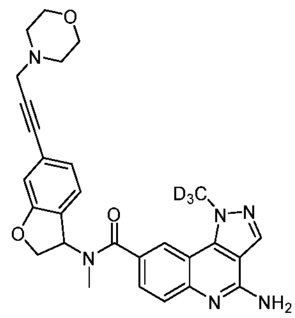
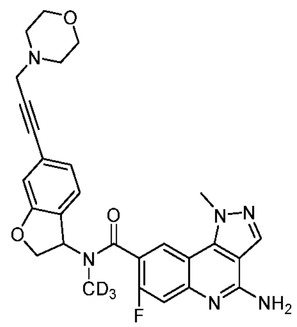
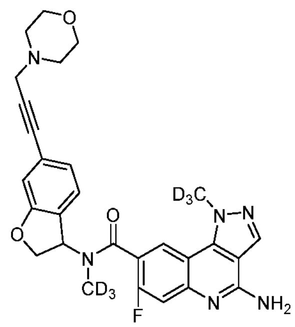
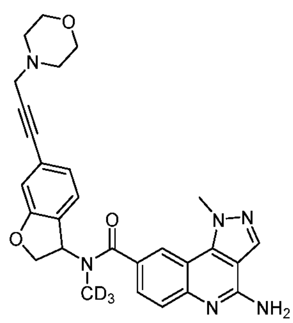
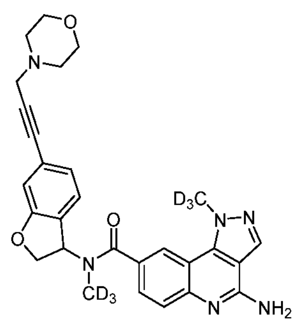
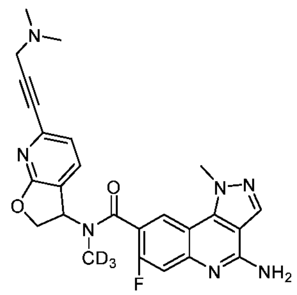
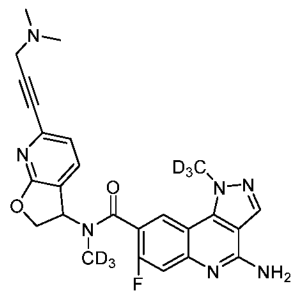
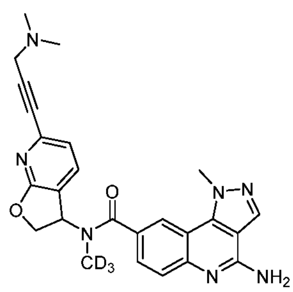
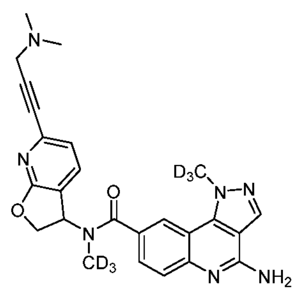
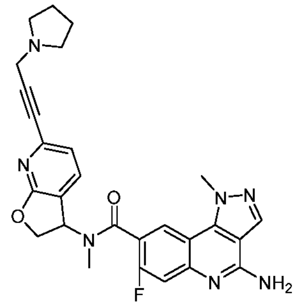
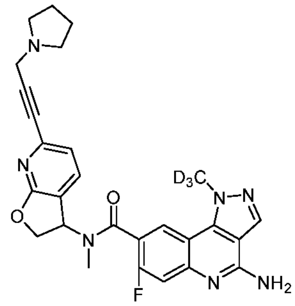
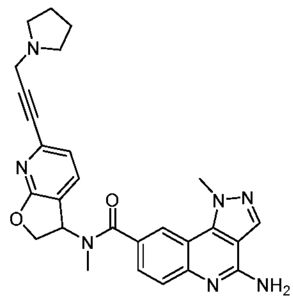
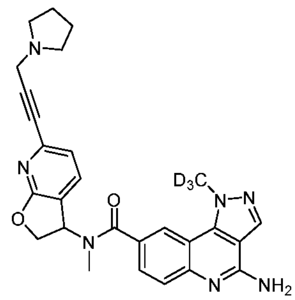
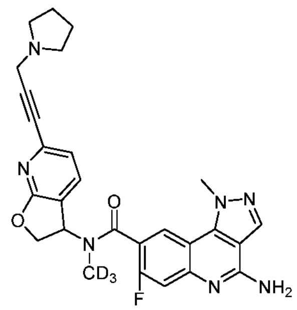
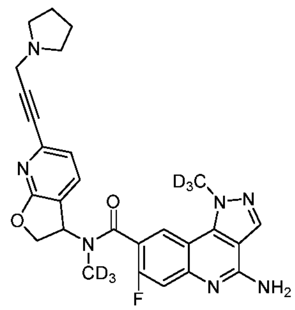
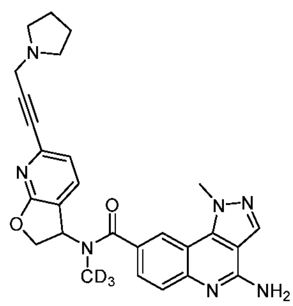
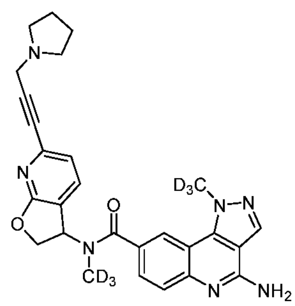

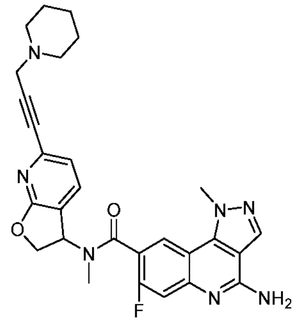
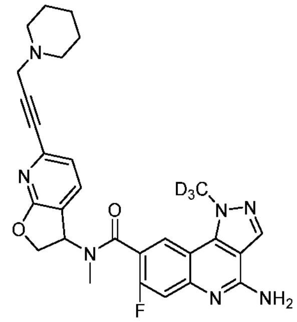
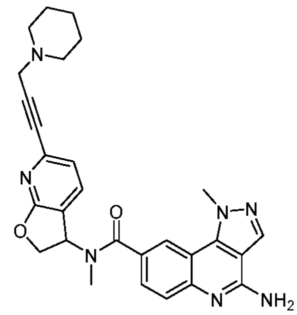
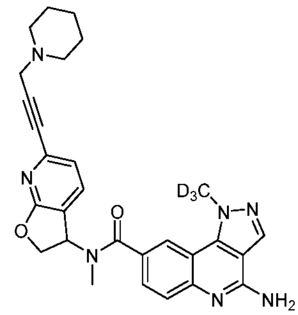
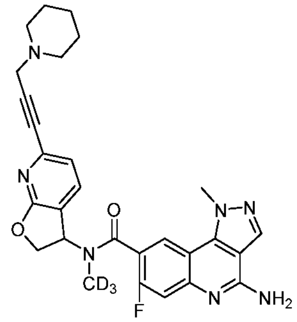
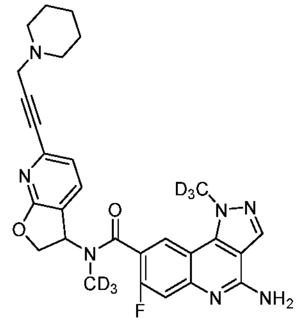
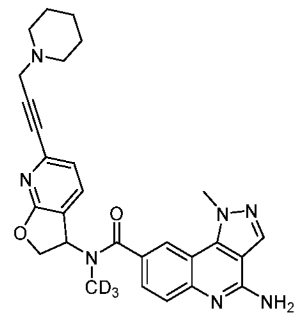
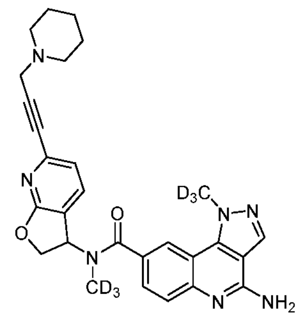
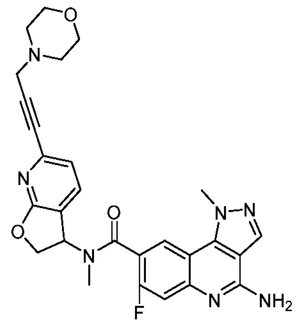
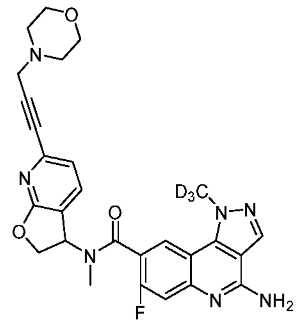
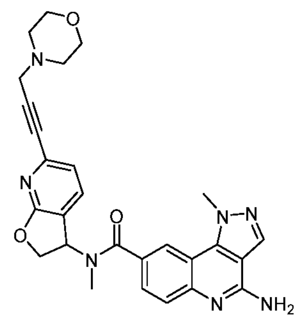
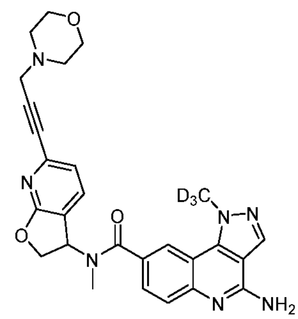
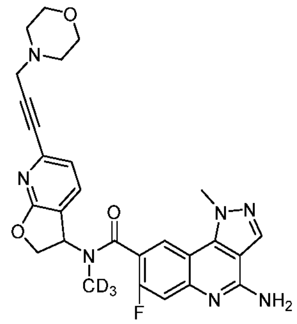
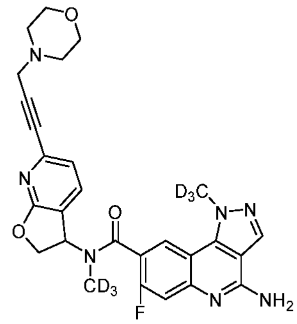
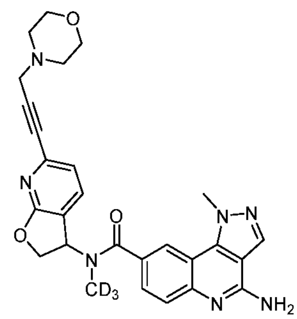
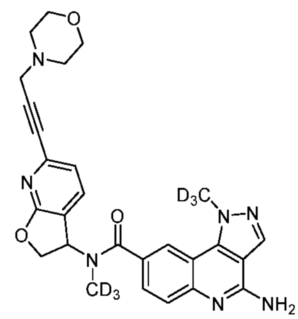
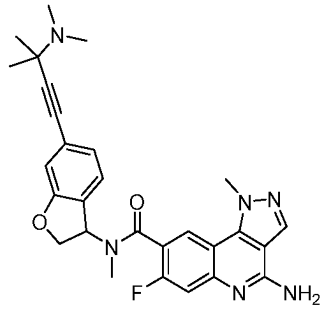
.

In some embodiments of the present disclosure, the compound is selected from:

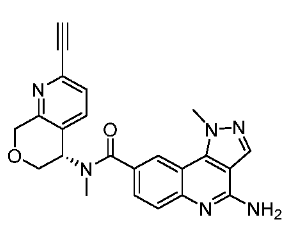
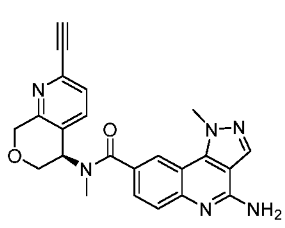
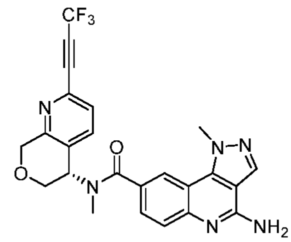
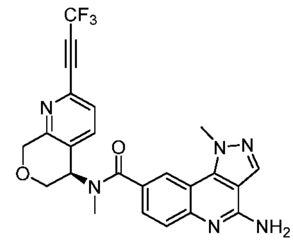
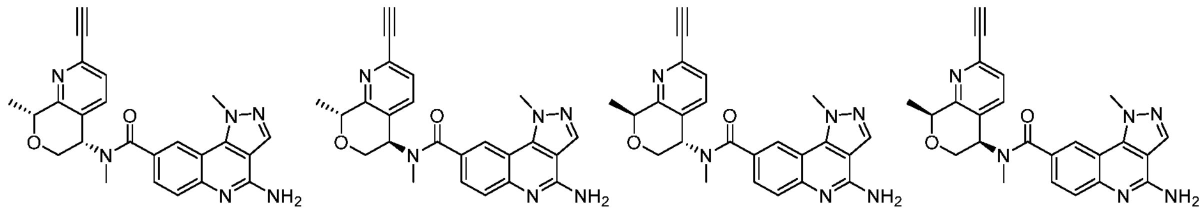
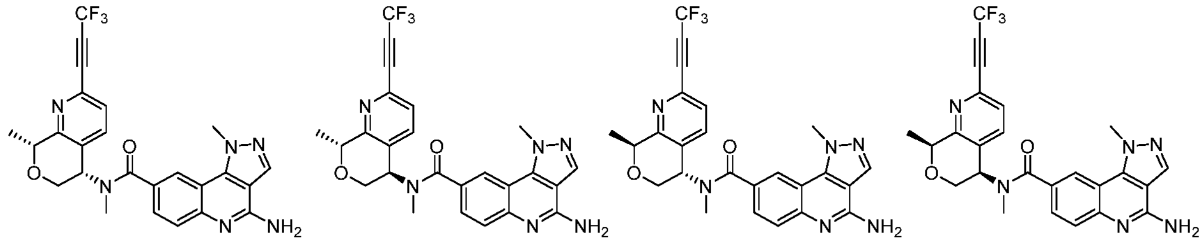
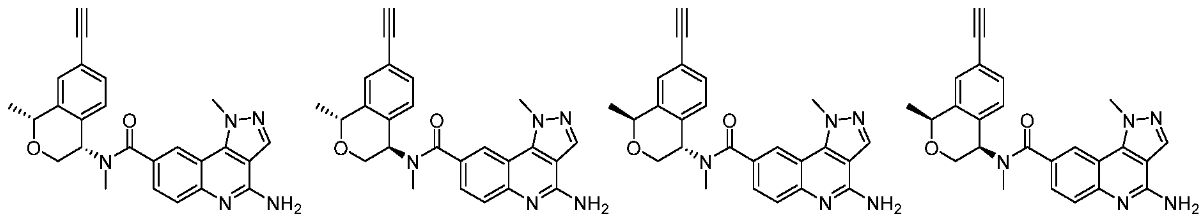
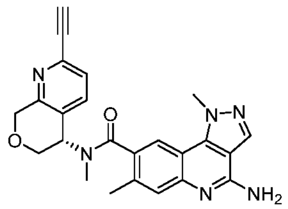
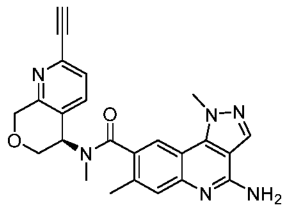
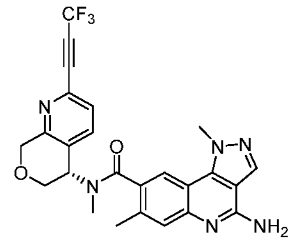
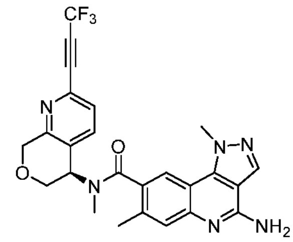
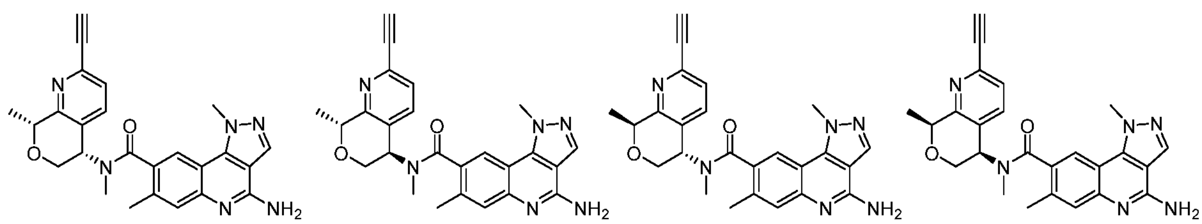

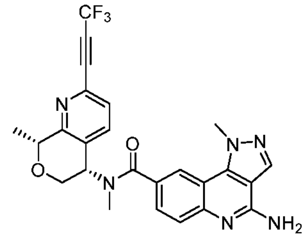 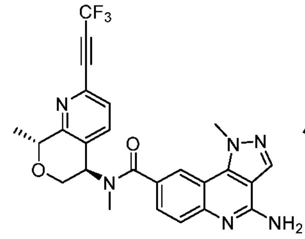 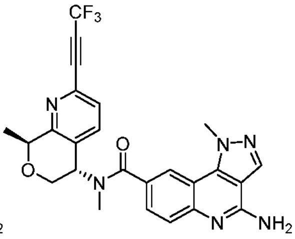 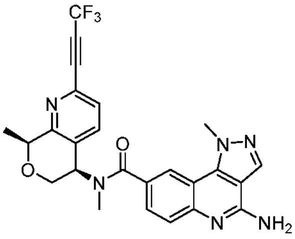

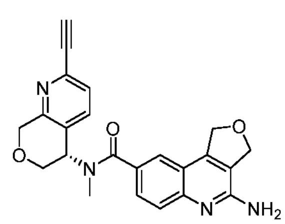 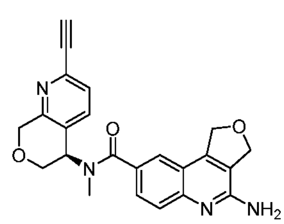 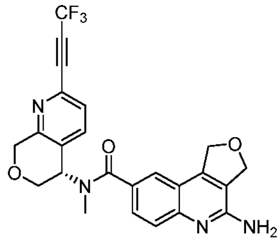 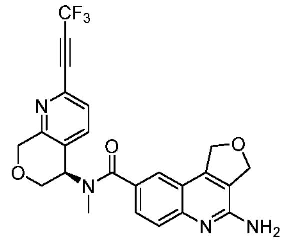

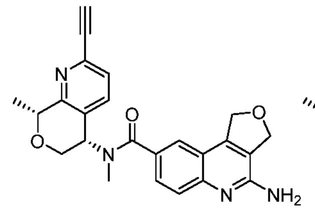 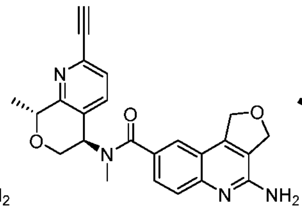 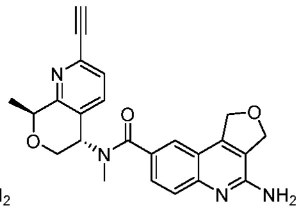 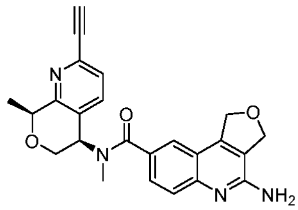

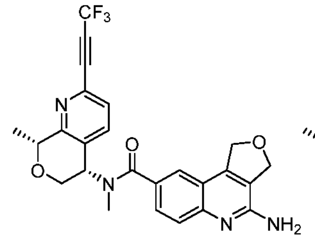 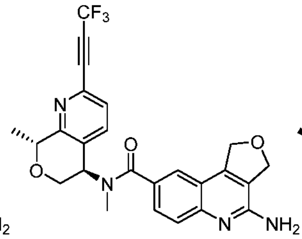 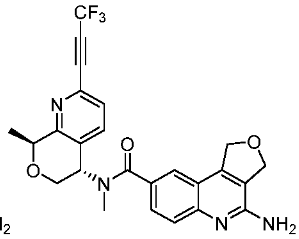 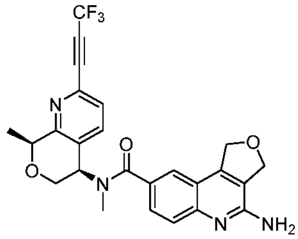

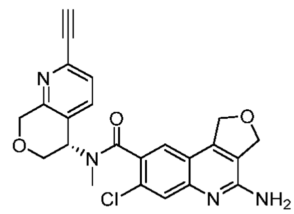 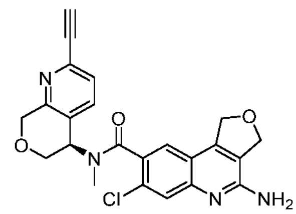 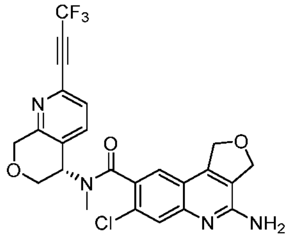 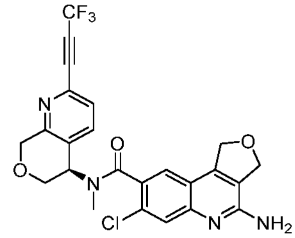

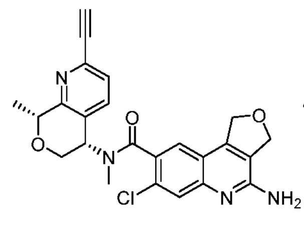 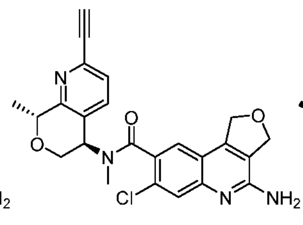 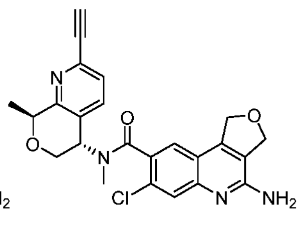 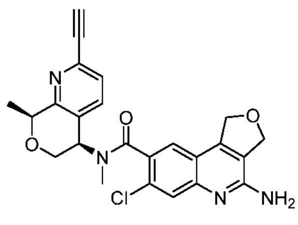

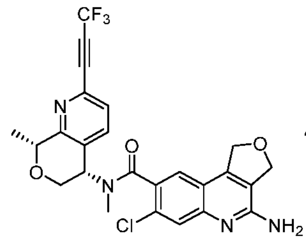 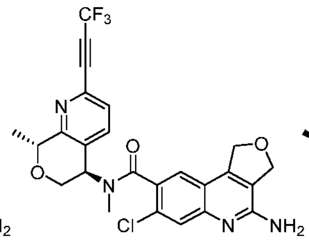 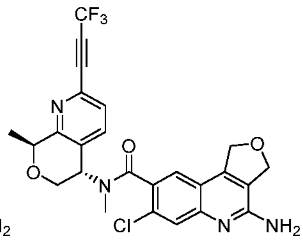 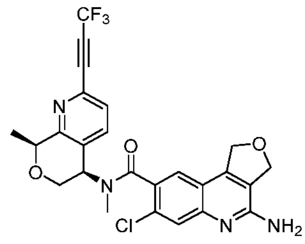

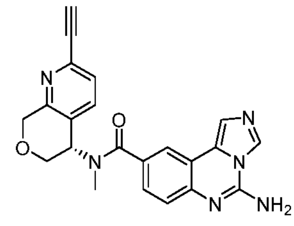 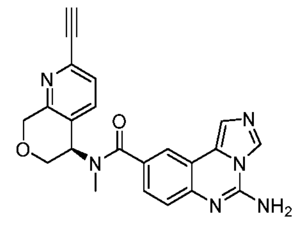 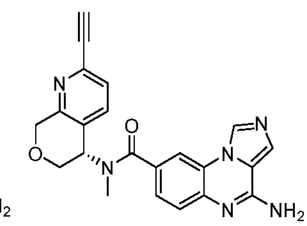 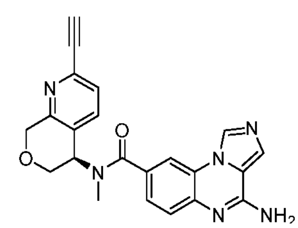

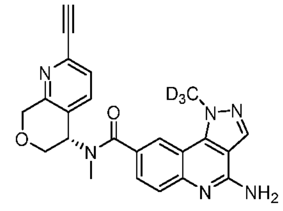 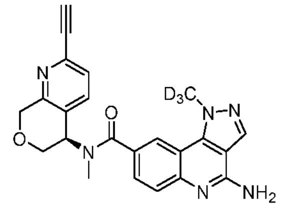 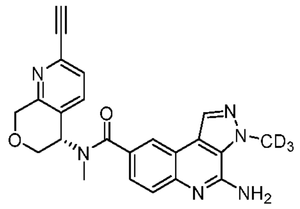 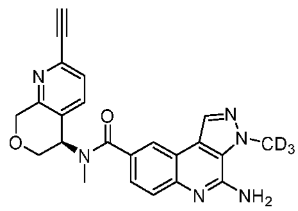

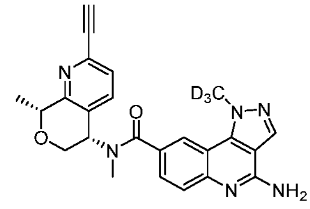 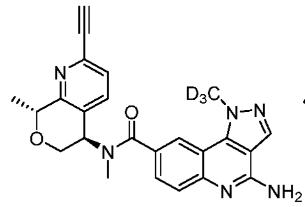 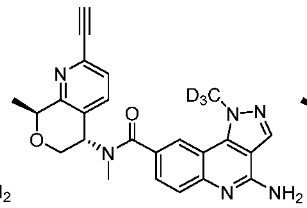 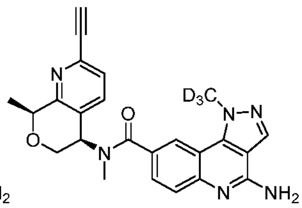

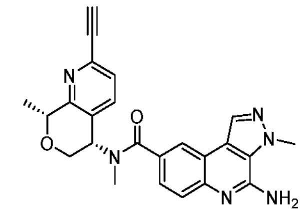 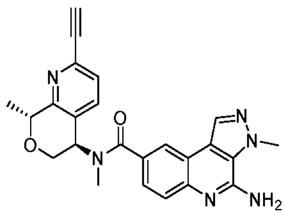 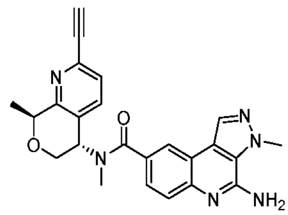 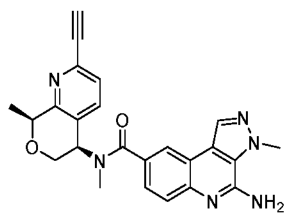

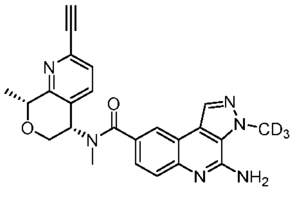 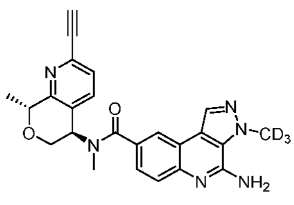 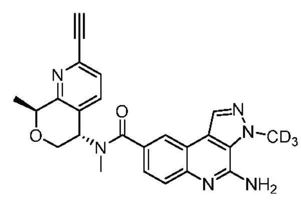 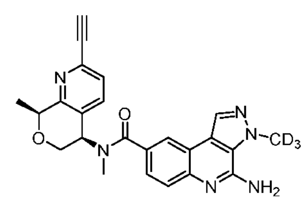

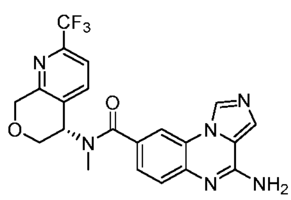 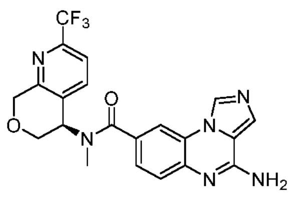 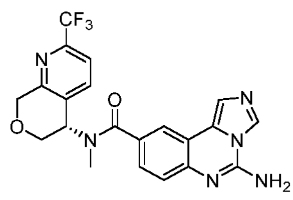 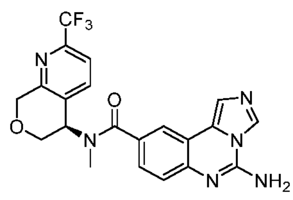

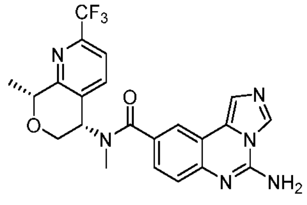 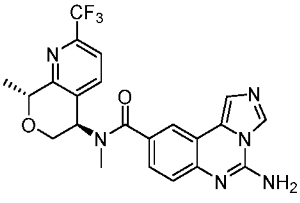 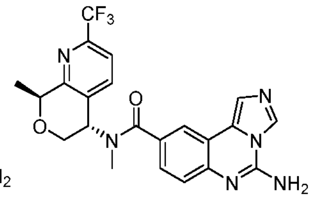 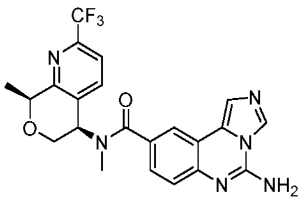

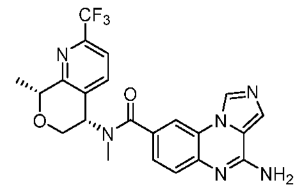 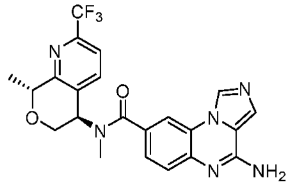 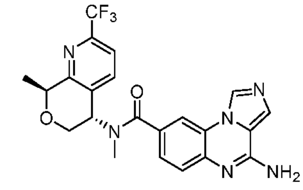 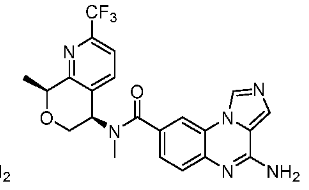

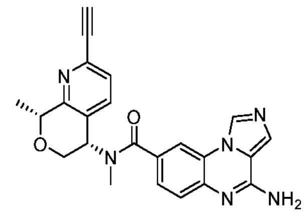 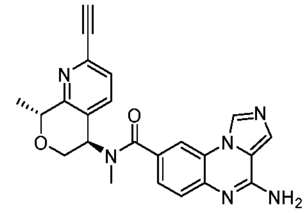 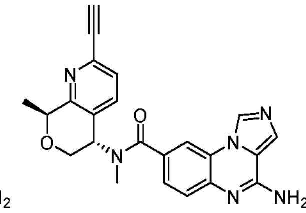 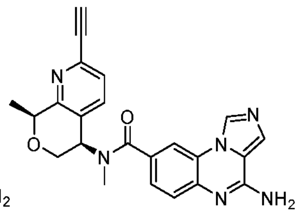

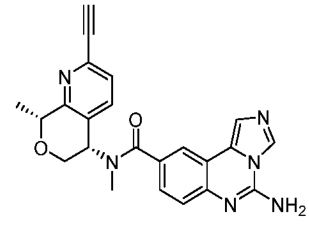 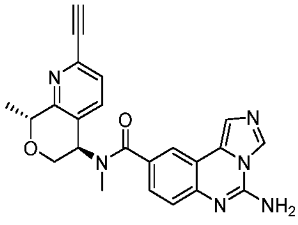 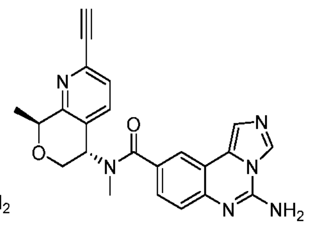 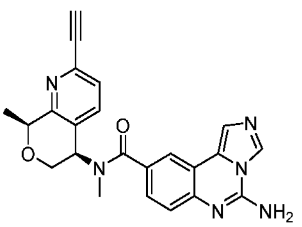

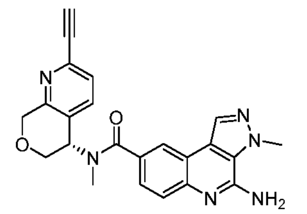 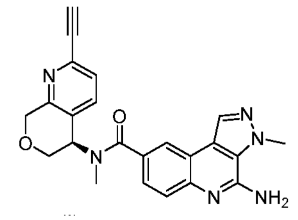 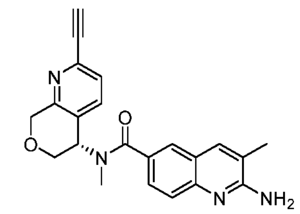 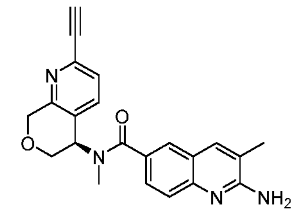

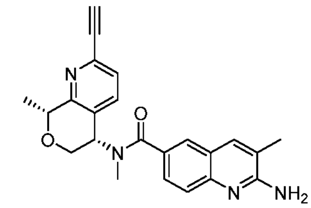 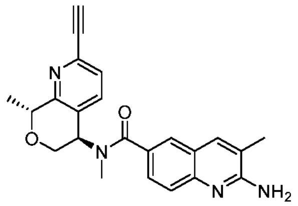 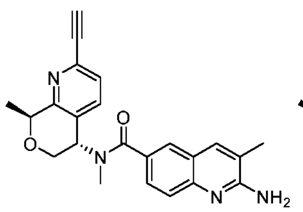 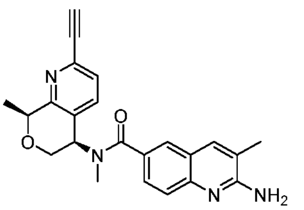

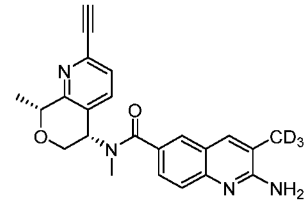 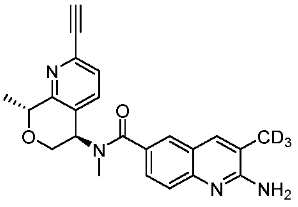 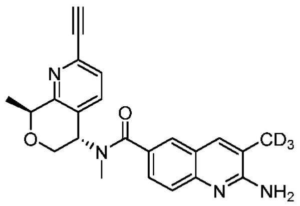 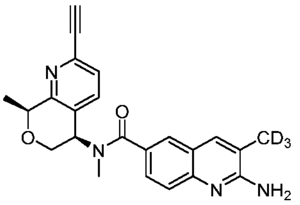

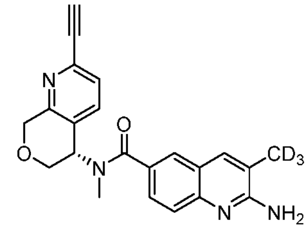
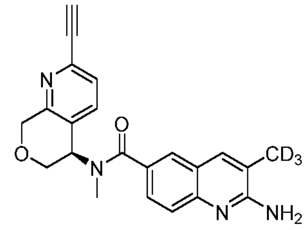

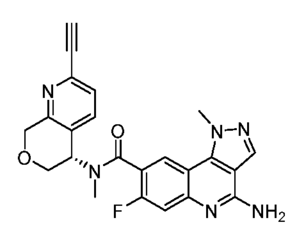
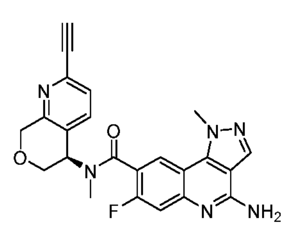
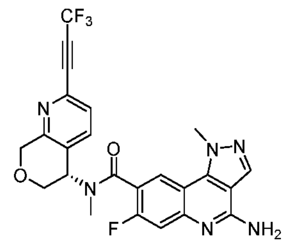
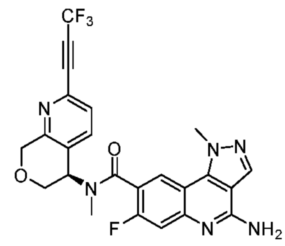

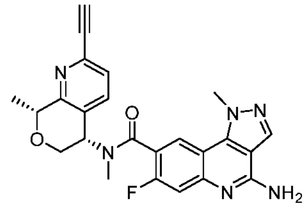
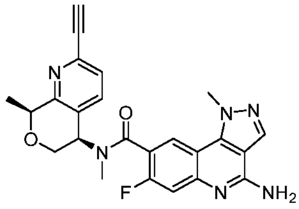
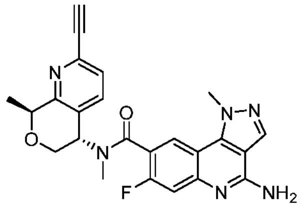
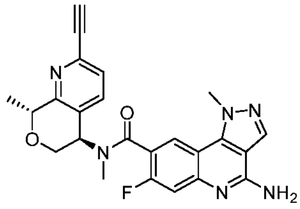

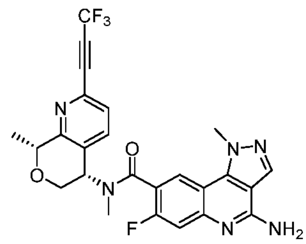
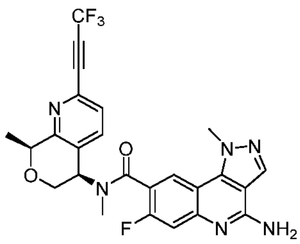
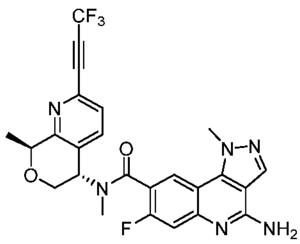
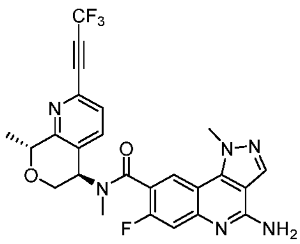

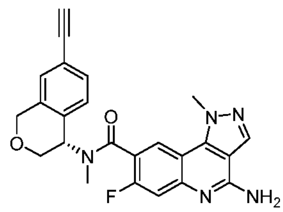
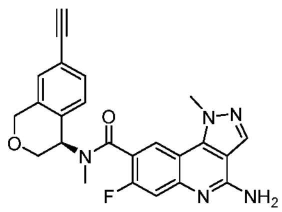
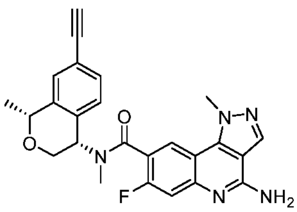
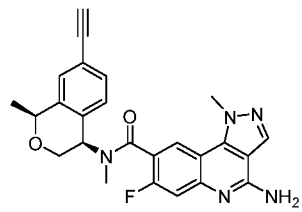

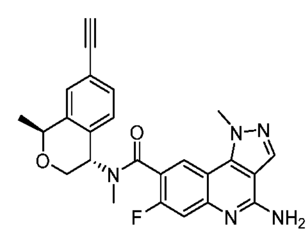
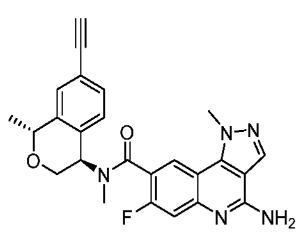
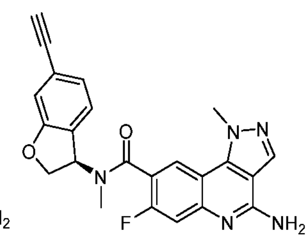
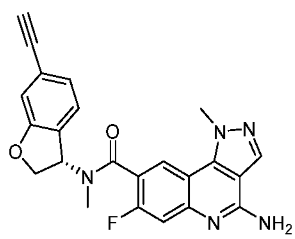

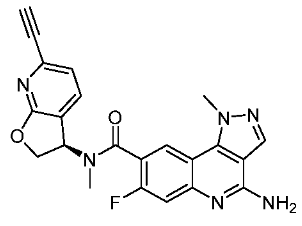 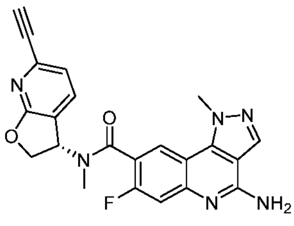 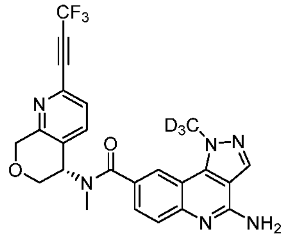 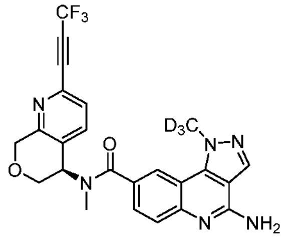

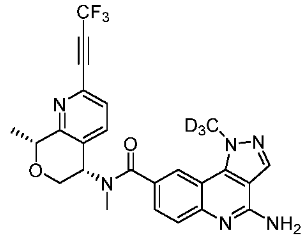 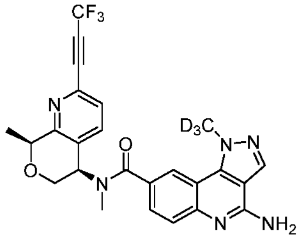 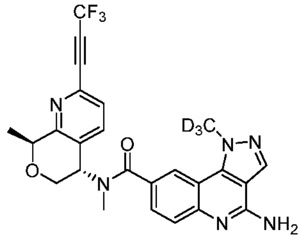 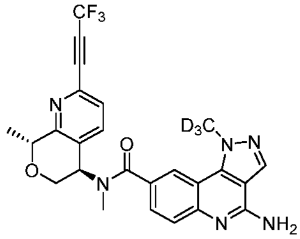

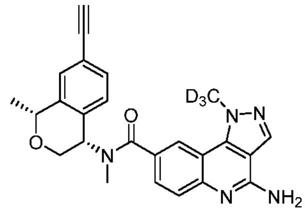 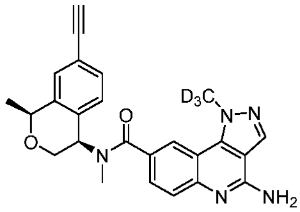 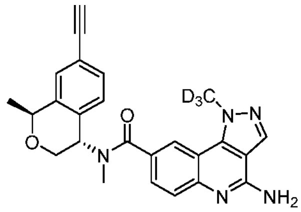 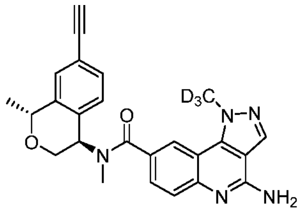

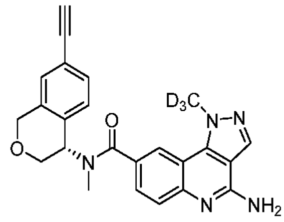 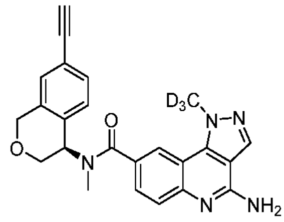 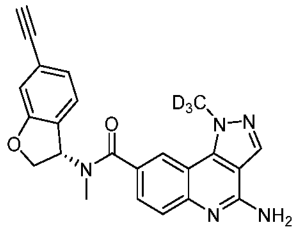 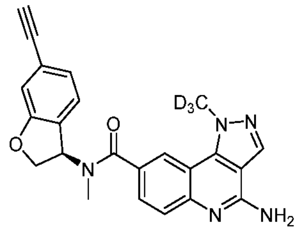

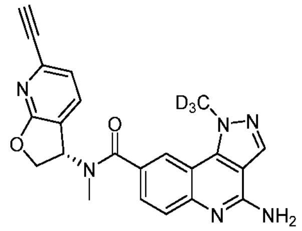 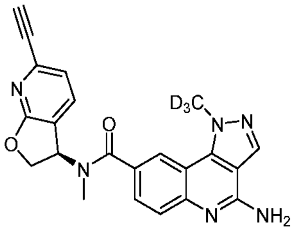 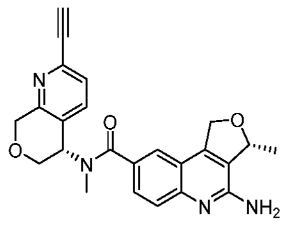 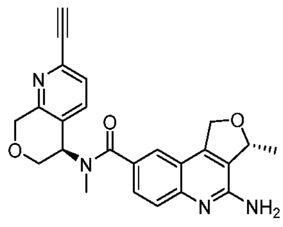

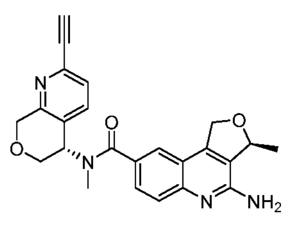 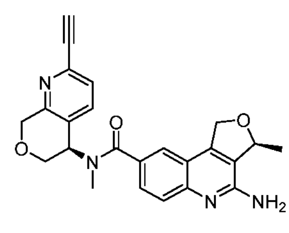 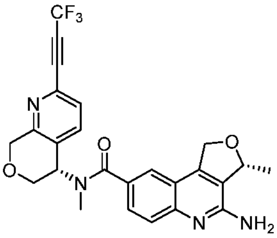 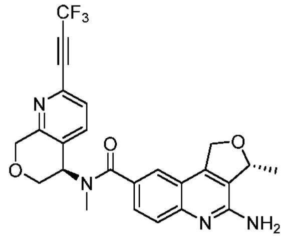

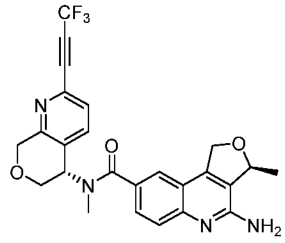
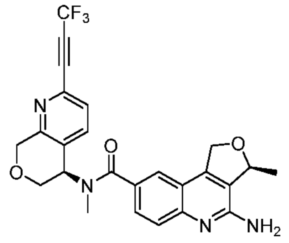
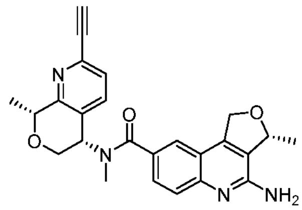
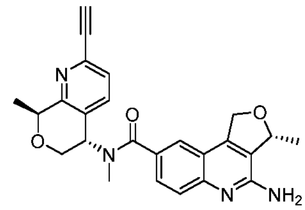

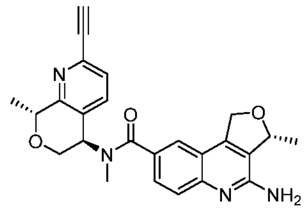
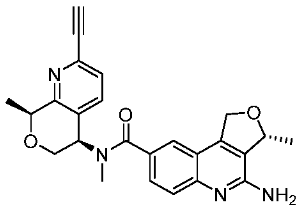
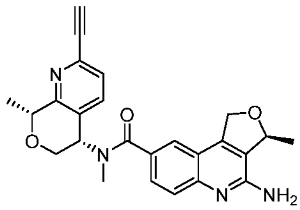
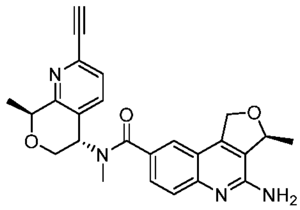

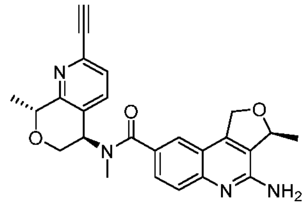
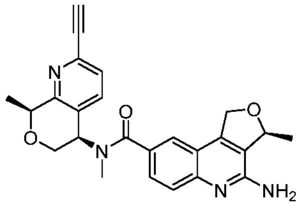
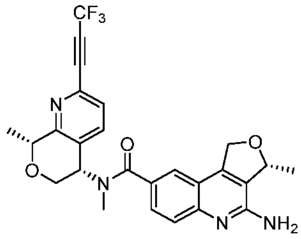
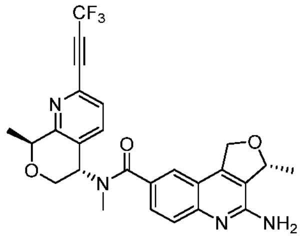

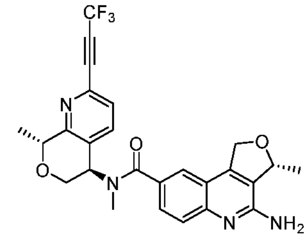
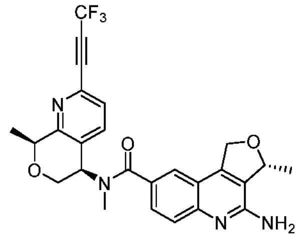
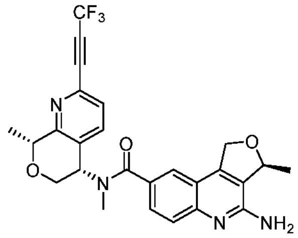
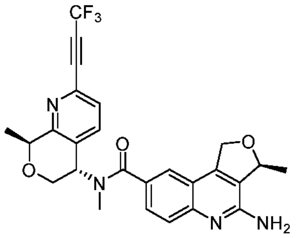

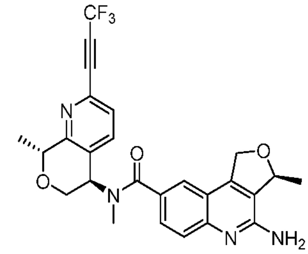
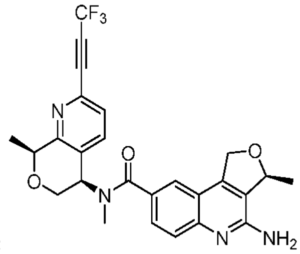
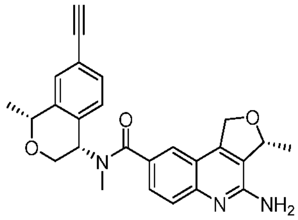
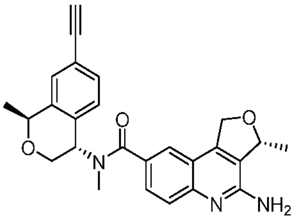

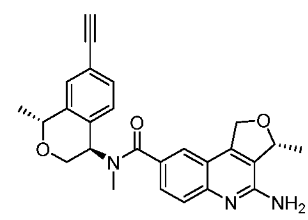
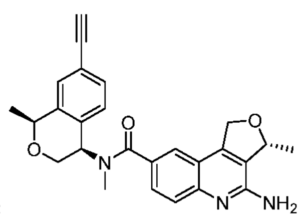
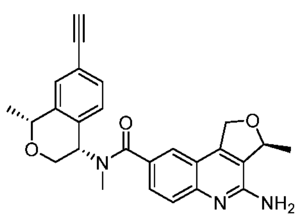
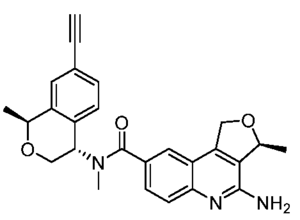

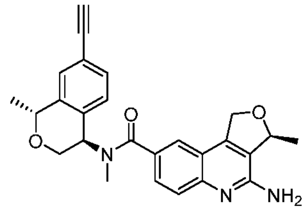
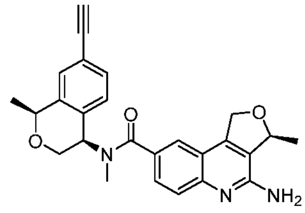
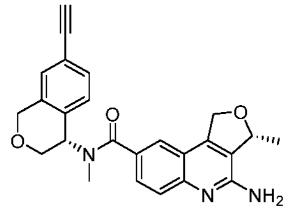
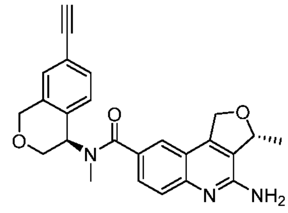

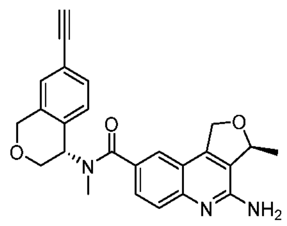
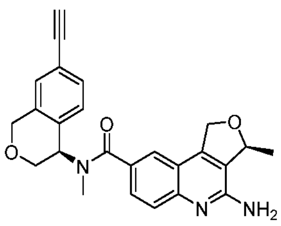
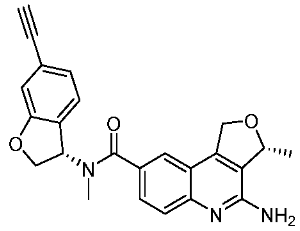
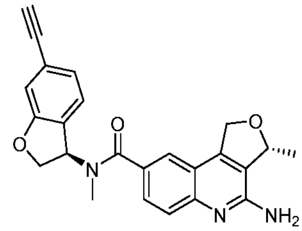

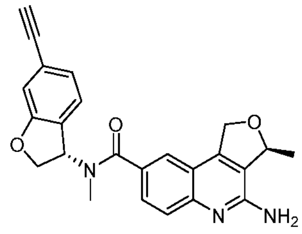
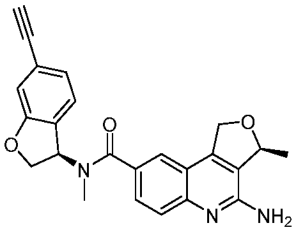
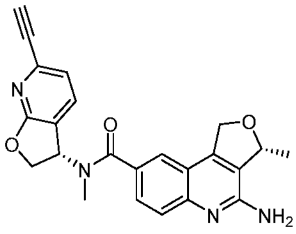
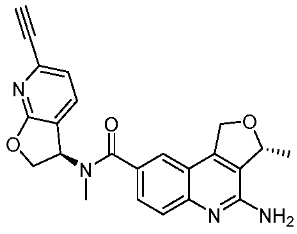

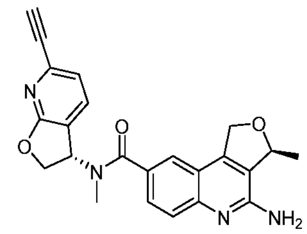
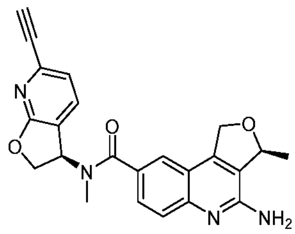
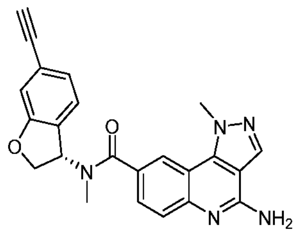
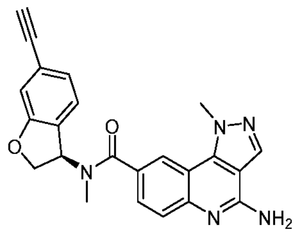

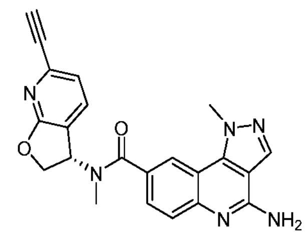
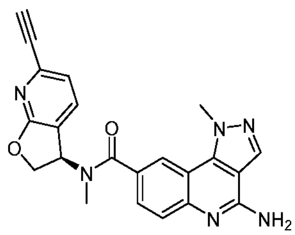
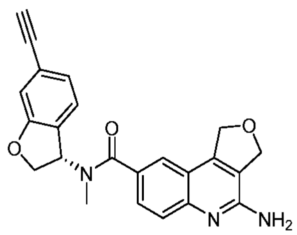
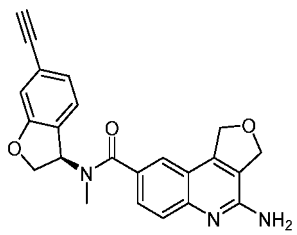

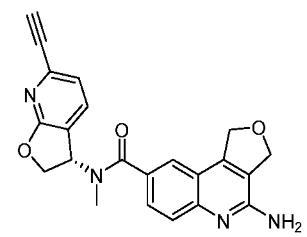
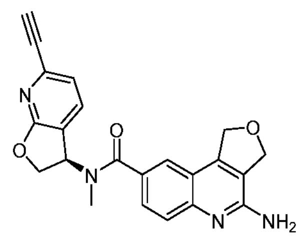
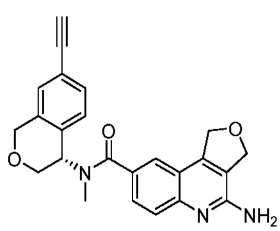
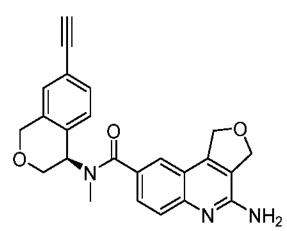

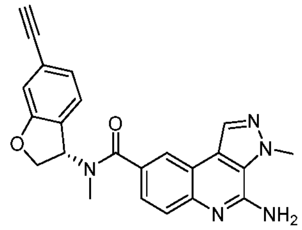
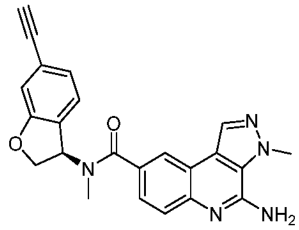
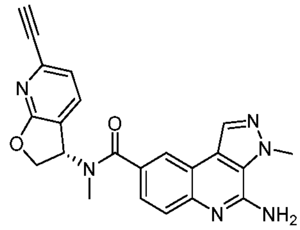
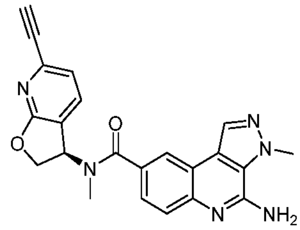

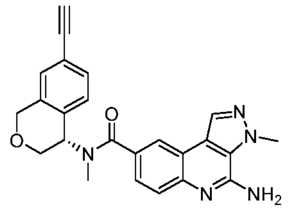
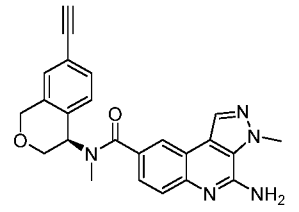
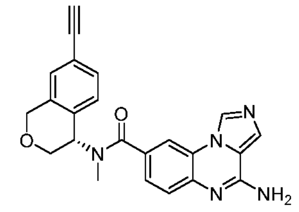
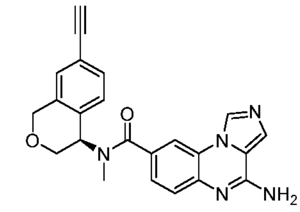

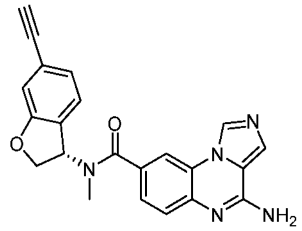
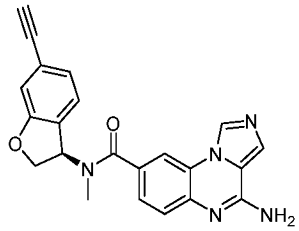
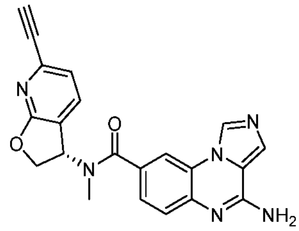
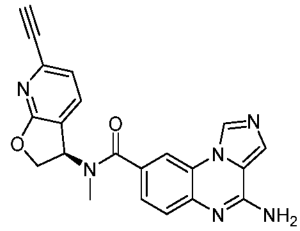

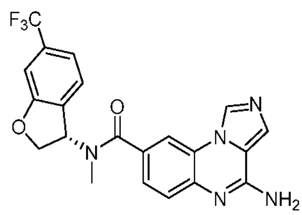
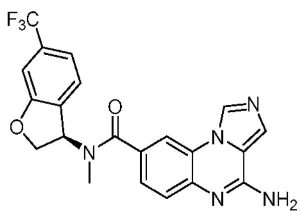
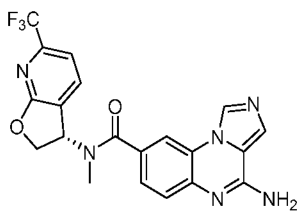
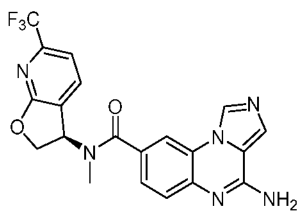

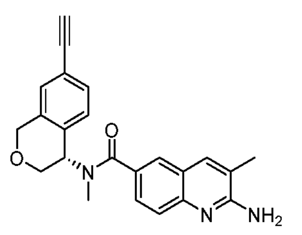
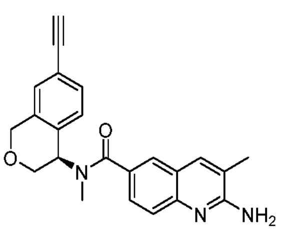
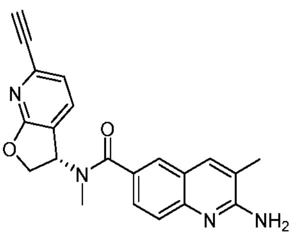
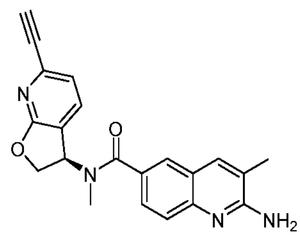

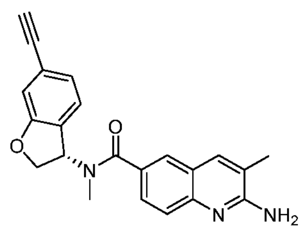
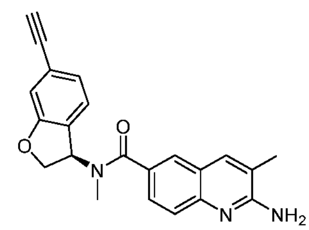
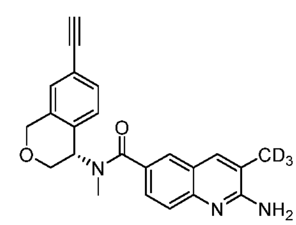
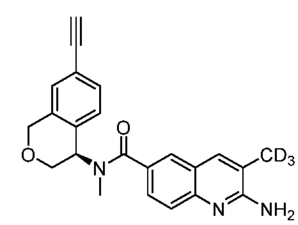

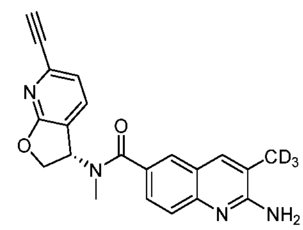
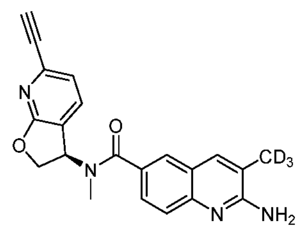
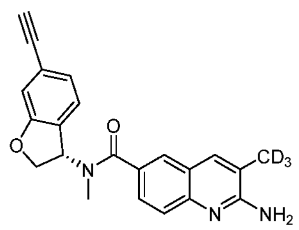
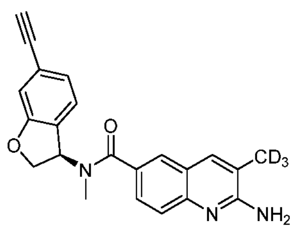

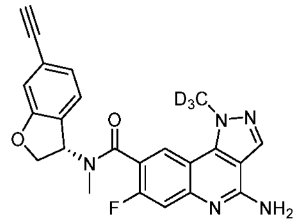 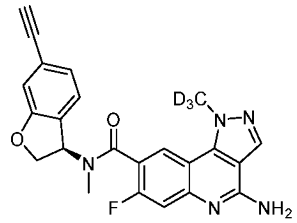 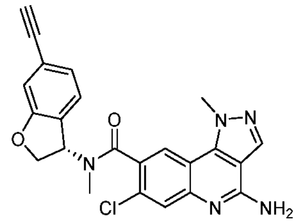 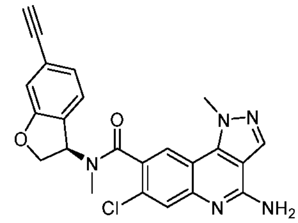

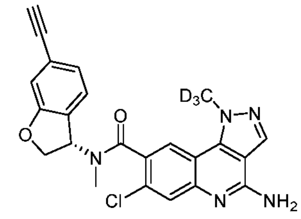 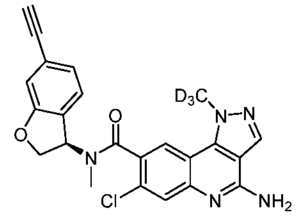 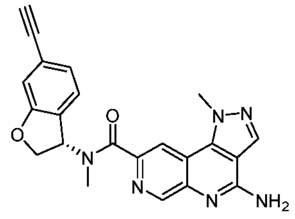 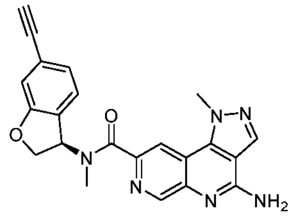

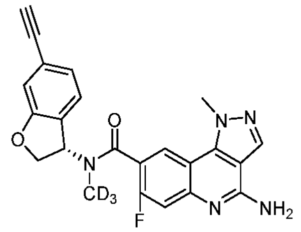 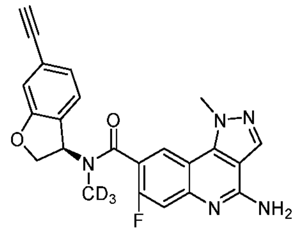 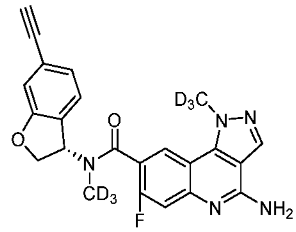 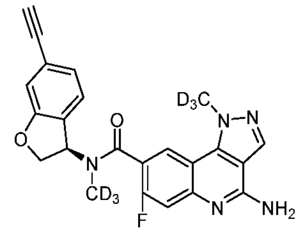

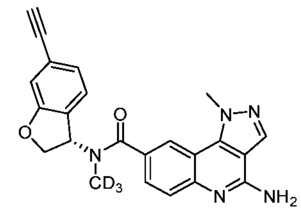 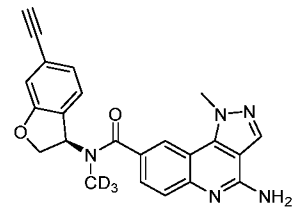 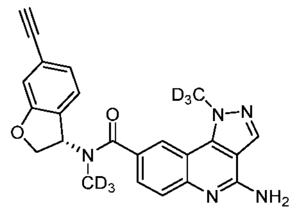 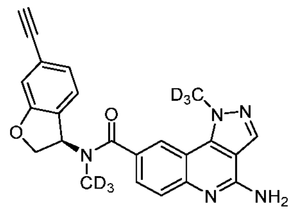

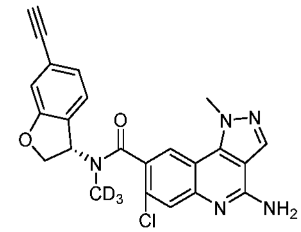 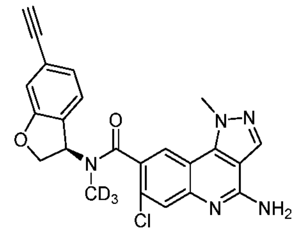 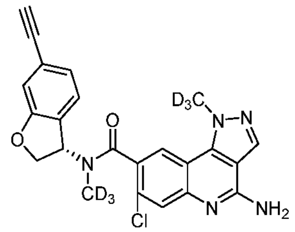 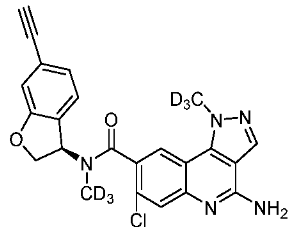

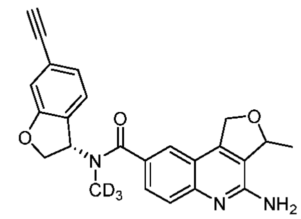 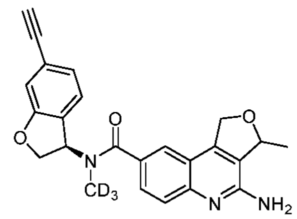 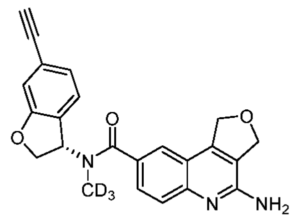 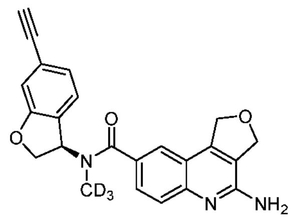

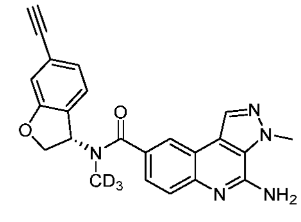 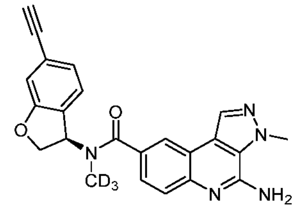 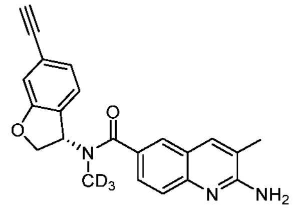 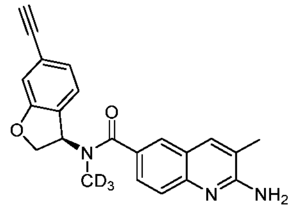

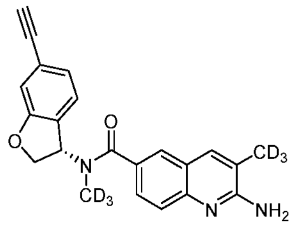
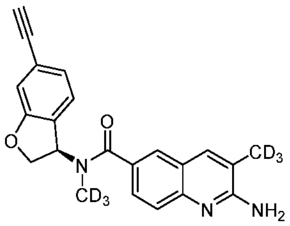
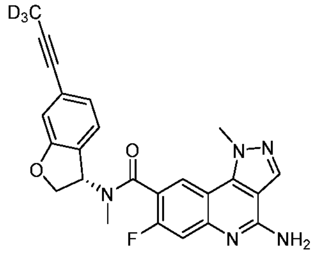
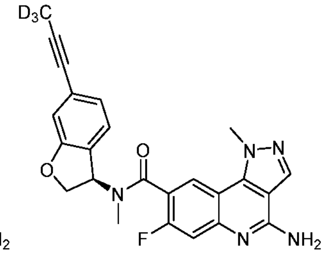
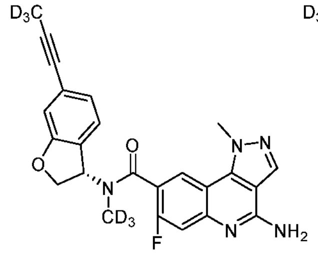
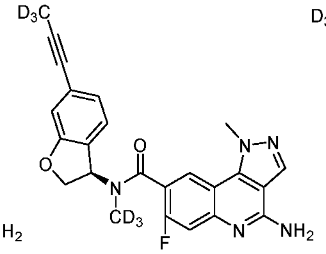
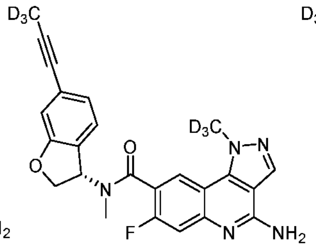
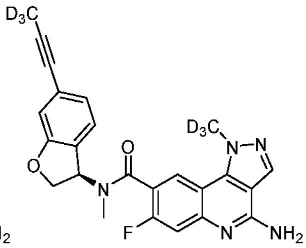
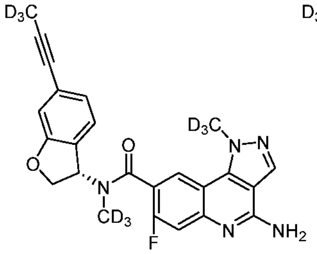
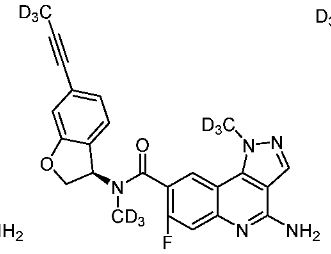
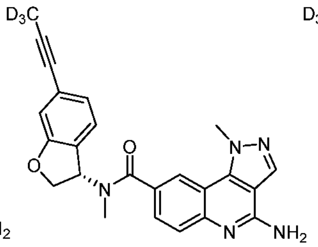
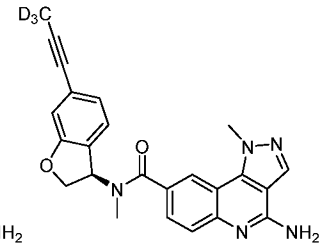
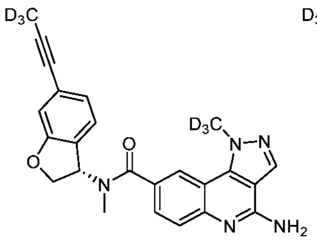
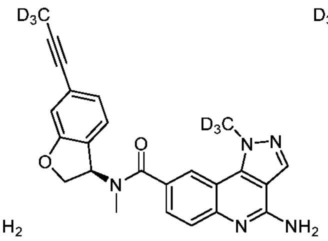
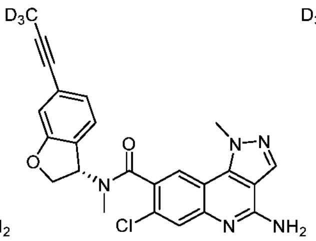
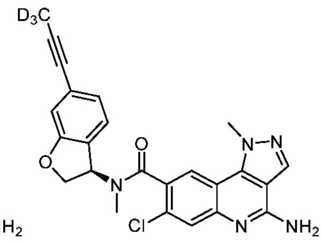
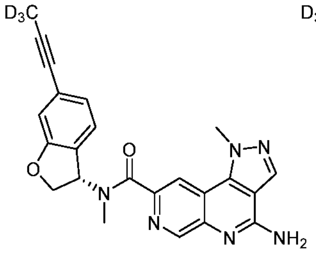
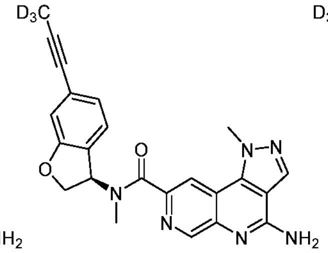
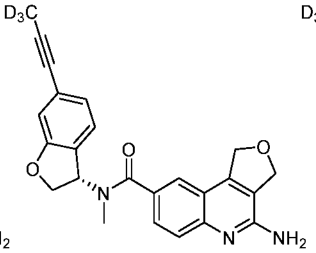
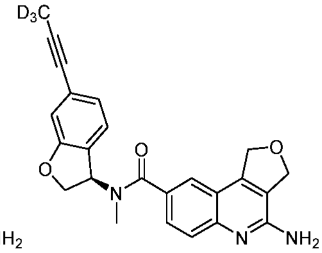
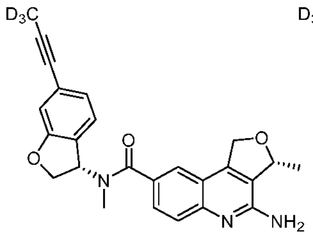
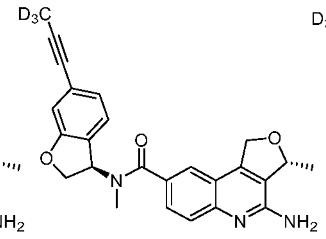
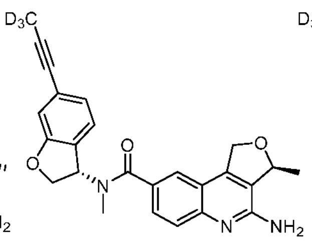
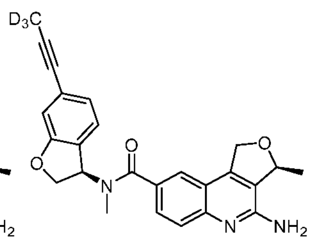

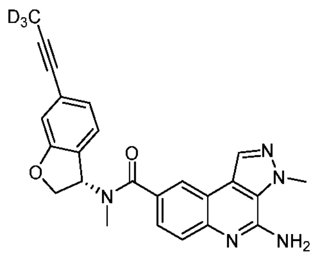
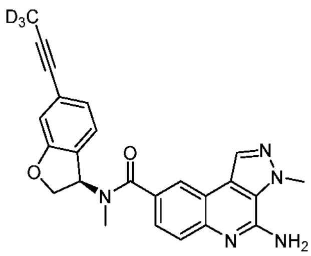
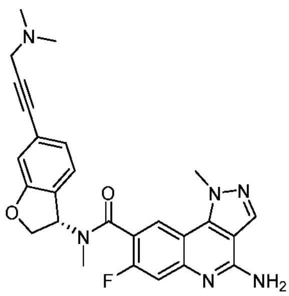
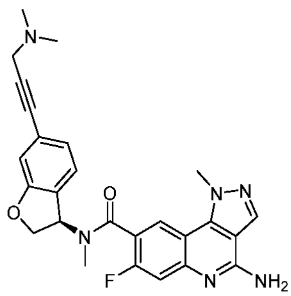

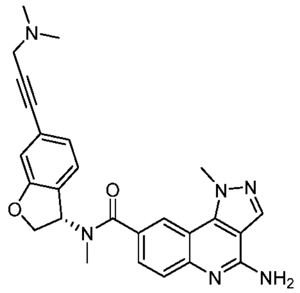
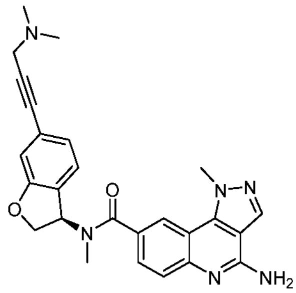
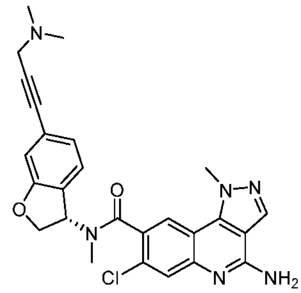
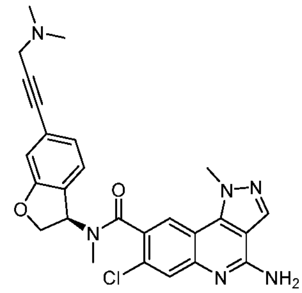

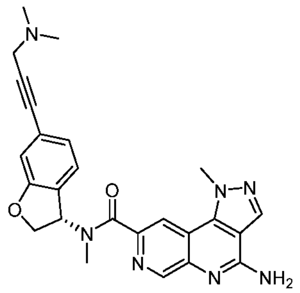
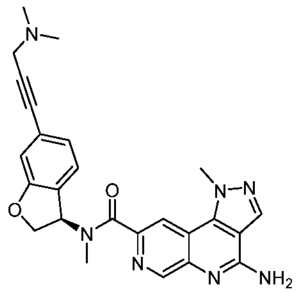
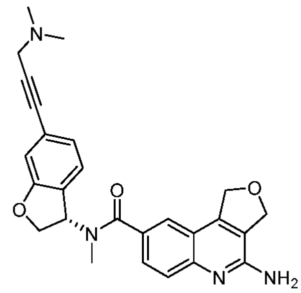
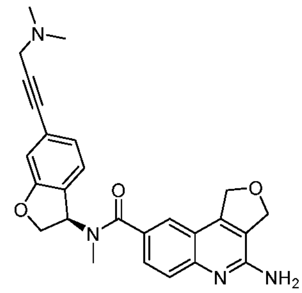

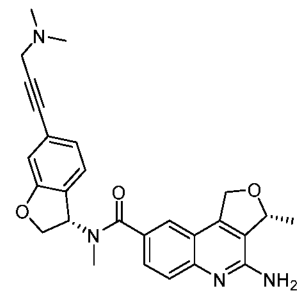
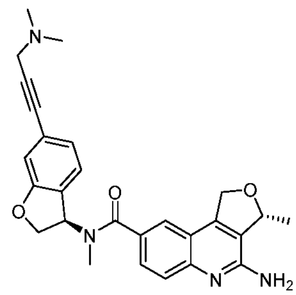
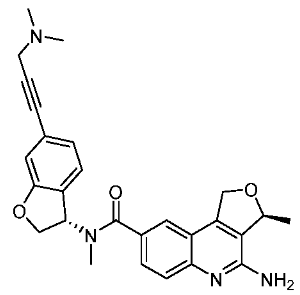
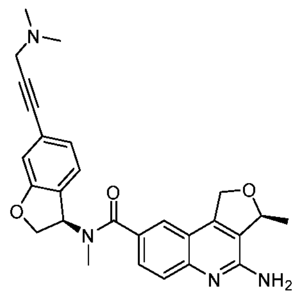

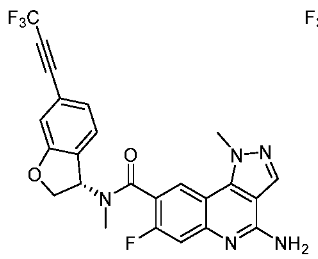
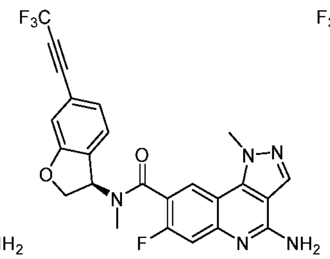
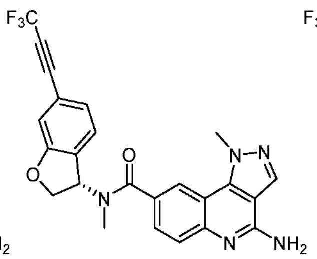
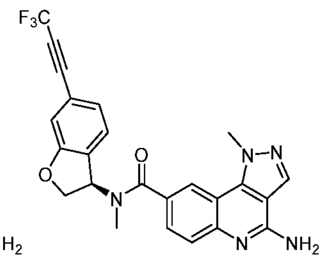

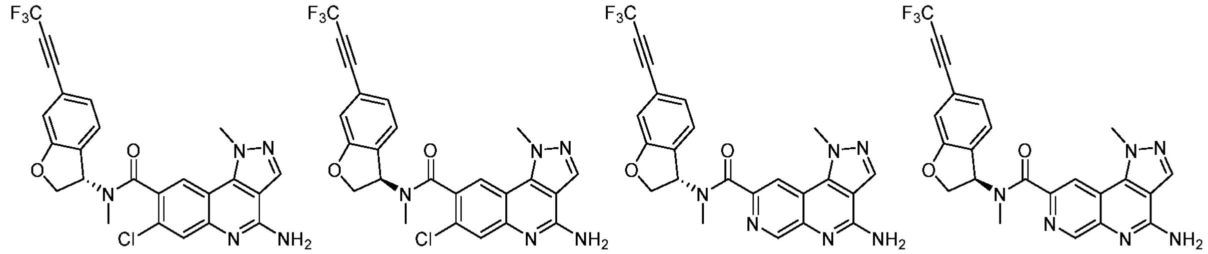

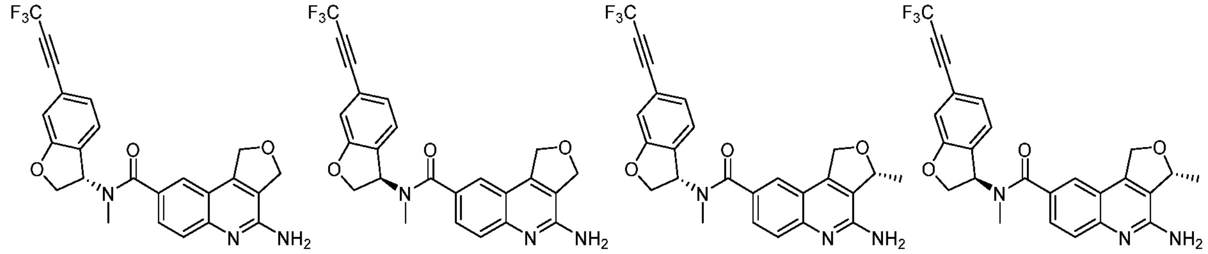

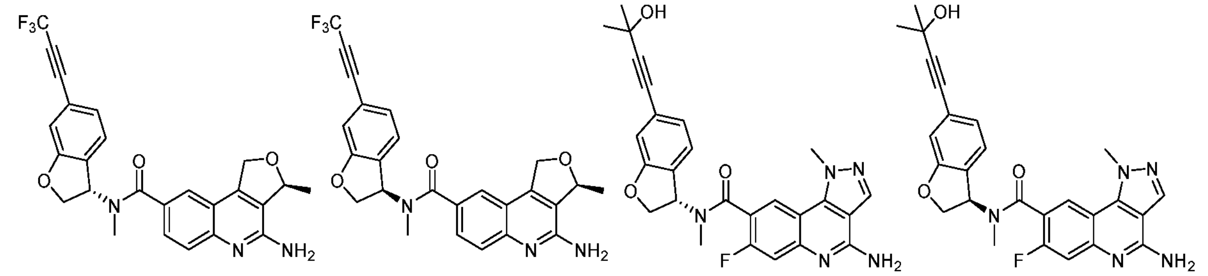

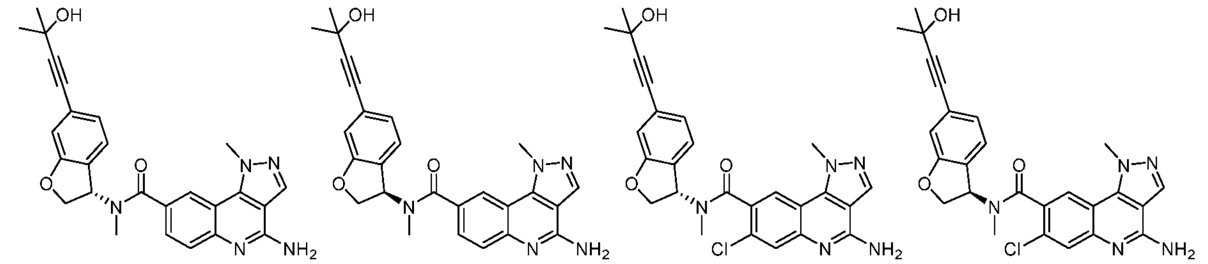

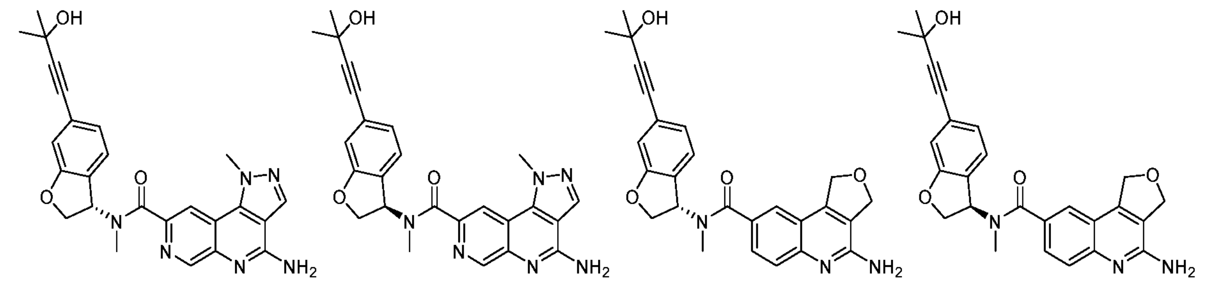

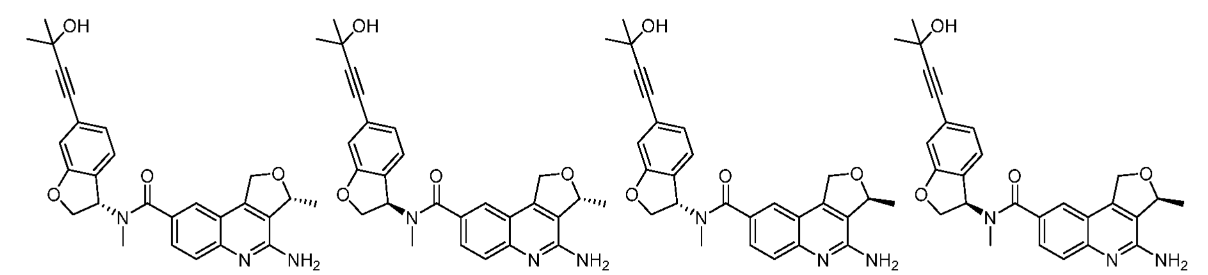

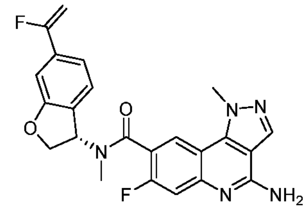
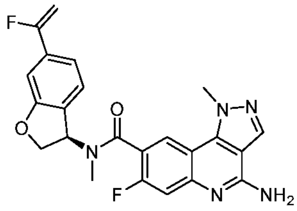
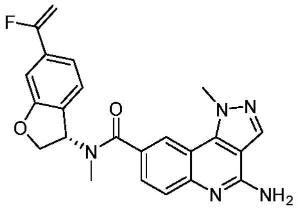
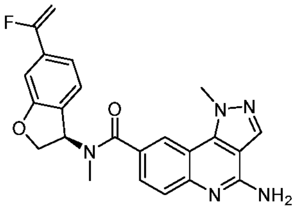
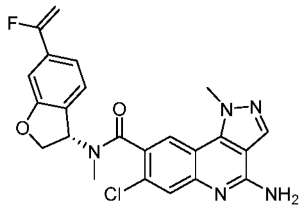
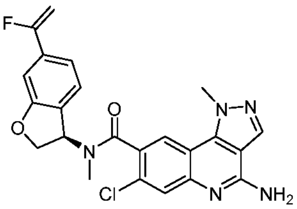
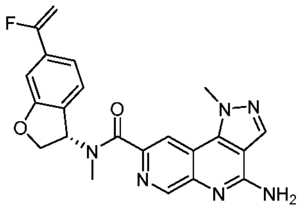
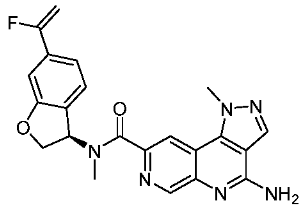
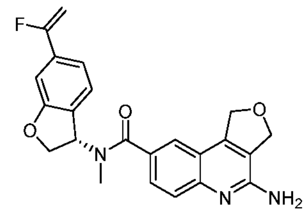
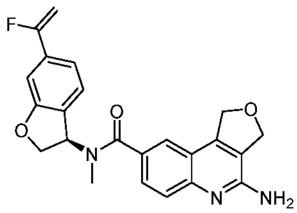
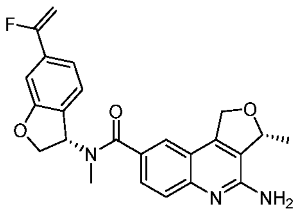
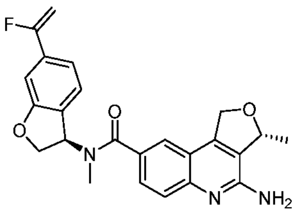
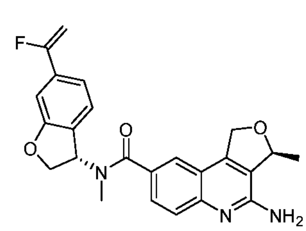
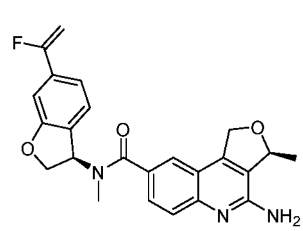
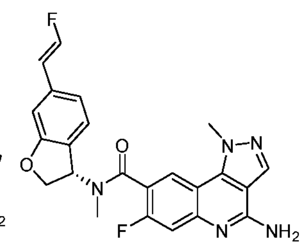
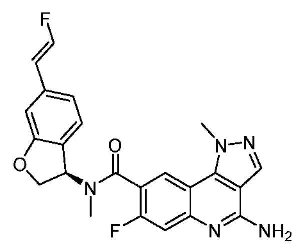
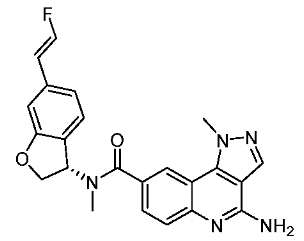
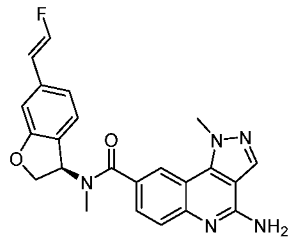
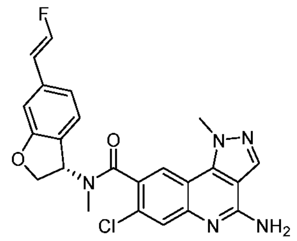
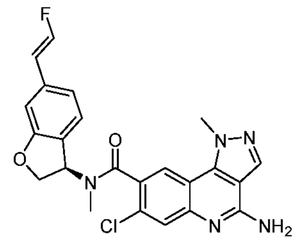
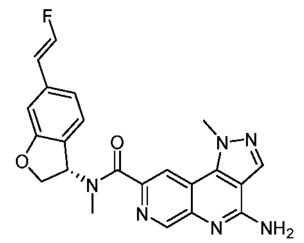
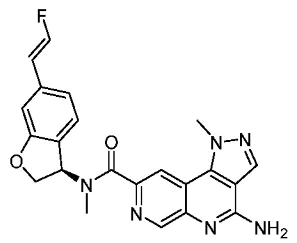
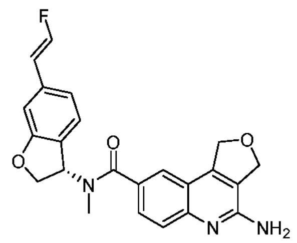
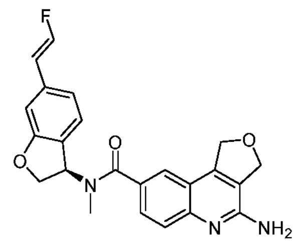

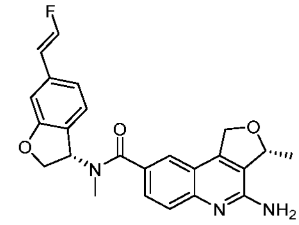
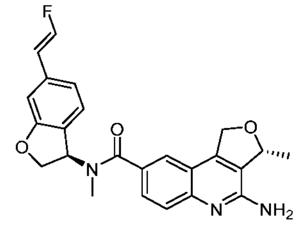
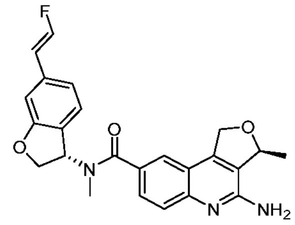
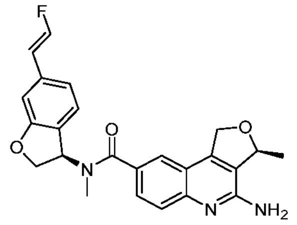

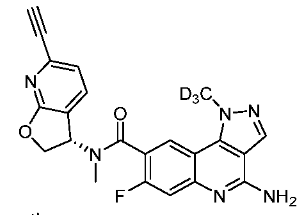
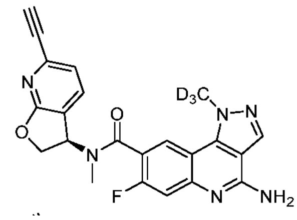
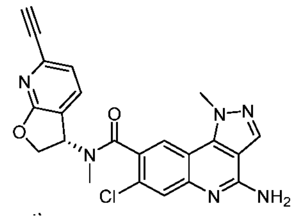
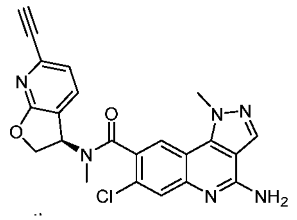

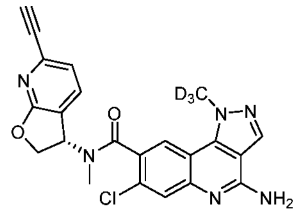
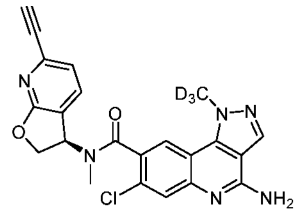
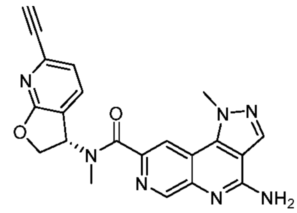
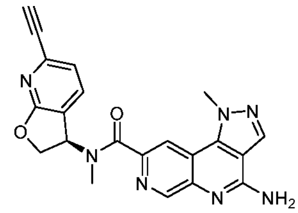

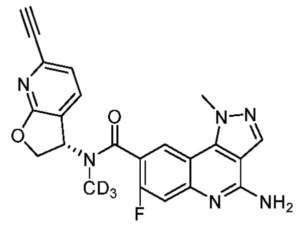
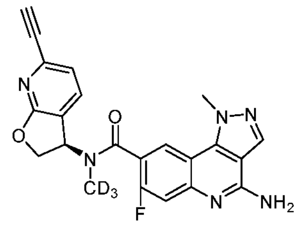
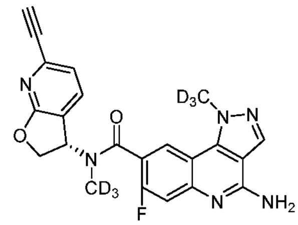
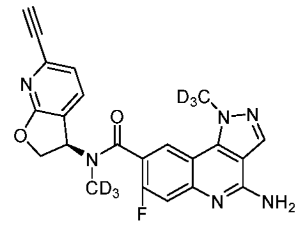

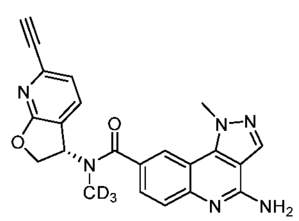
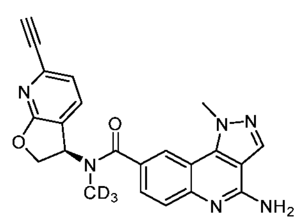
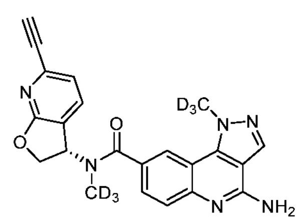
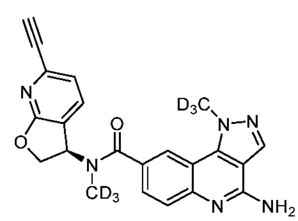

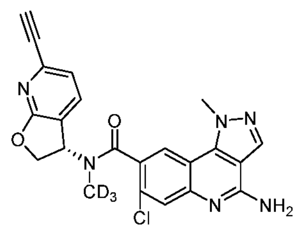
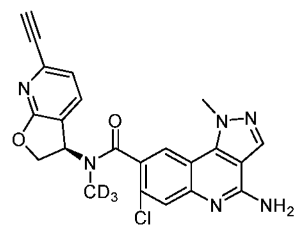
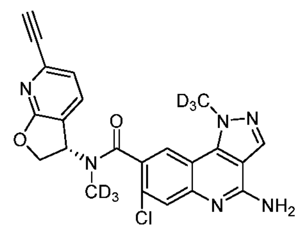
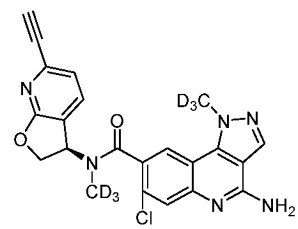

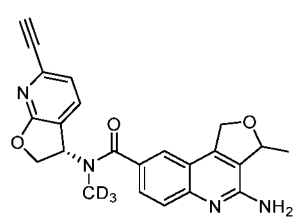
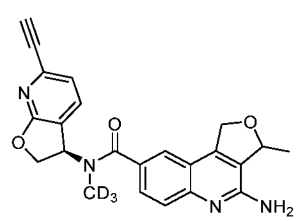
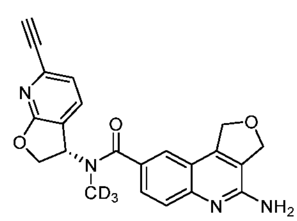
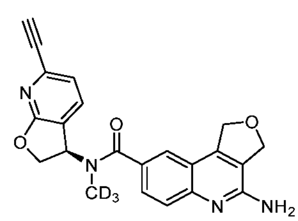

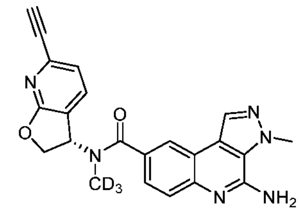
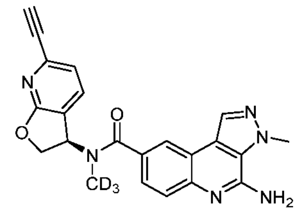
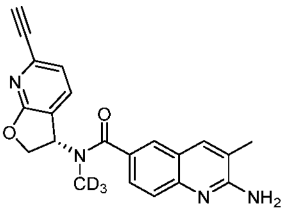
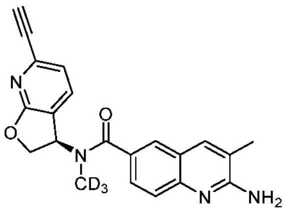
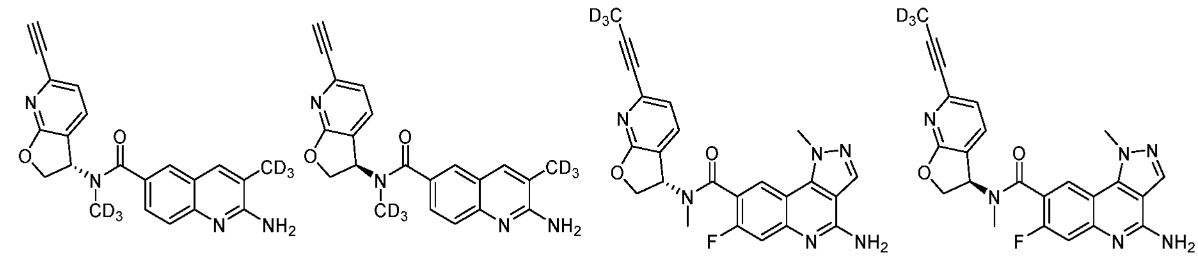
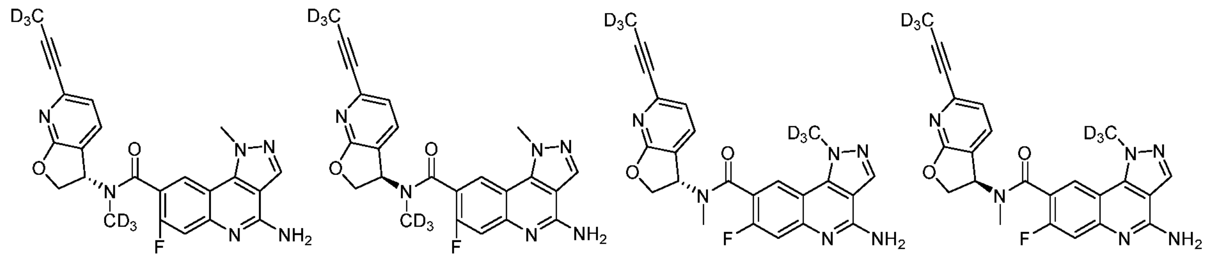
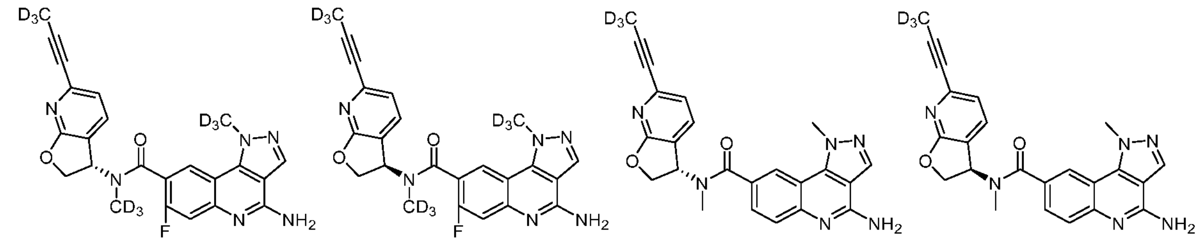
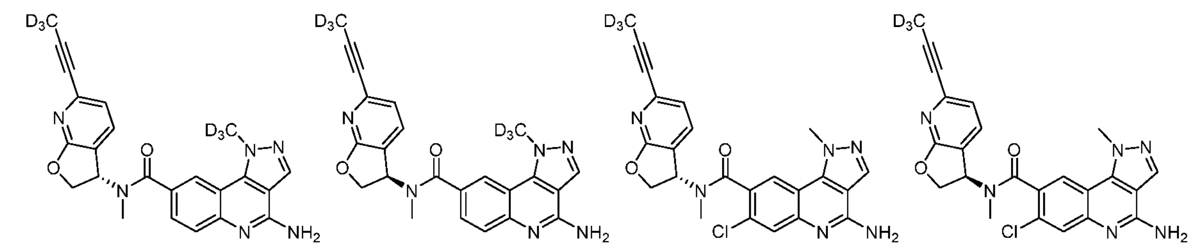
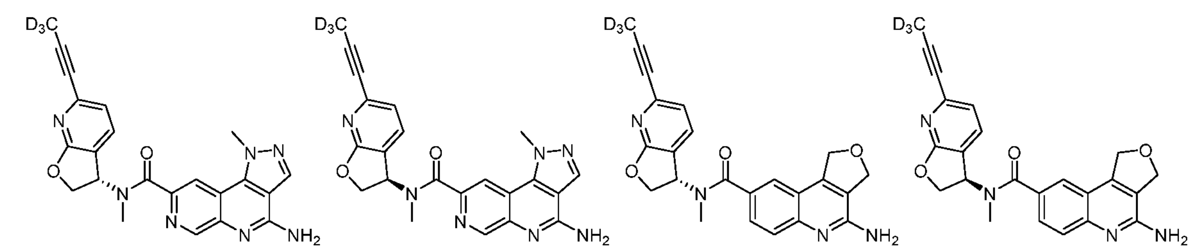

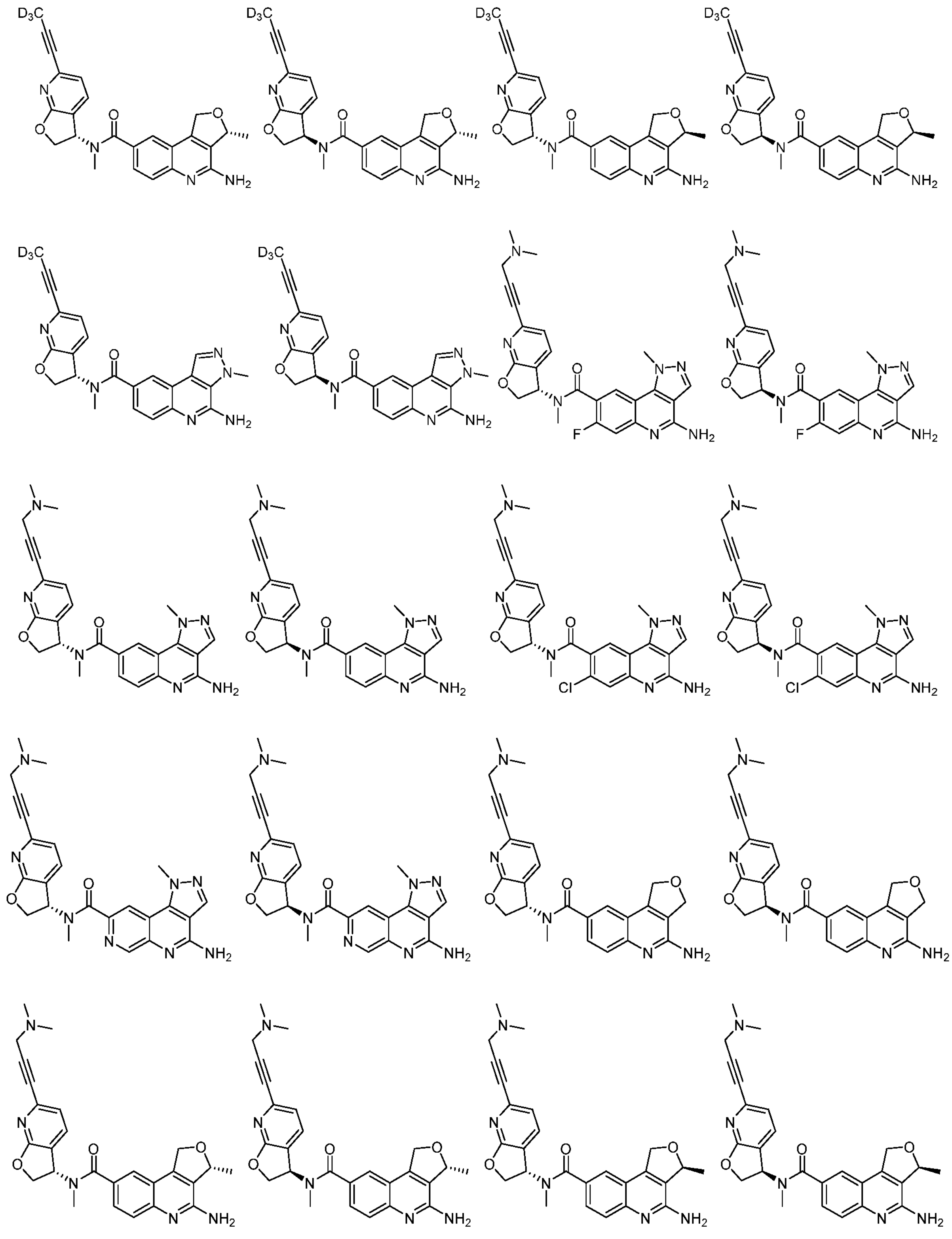

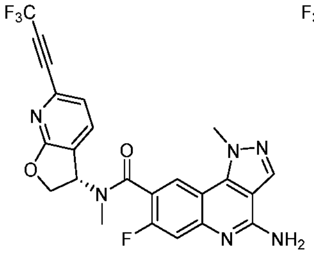
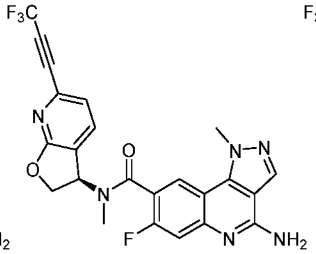
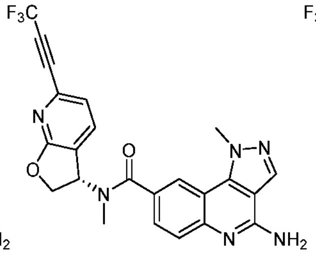
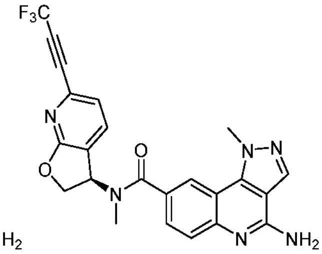

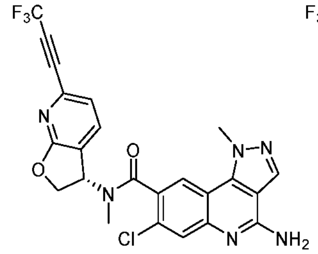
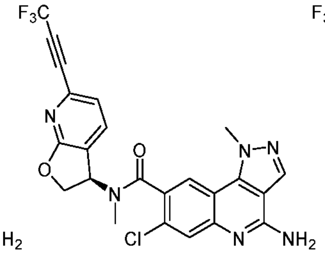
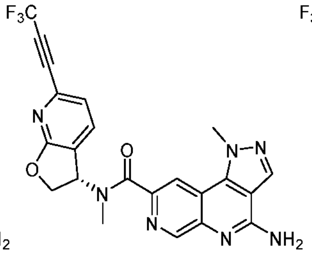
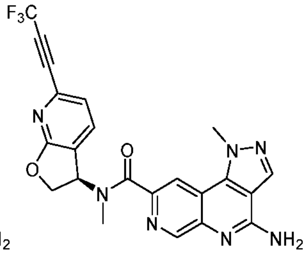

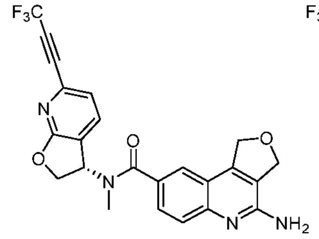
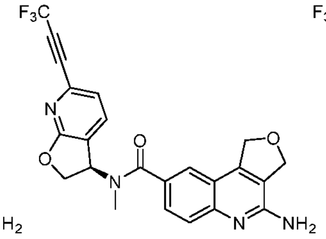
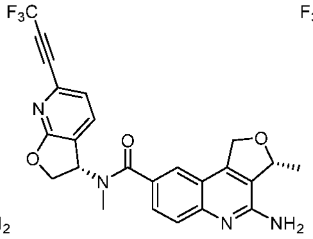
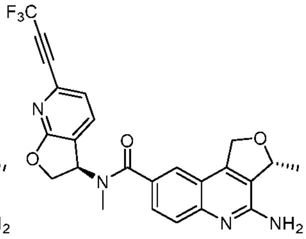

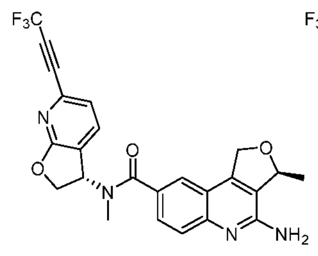
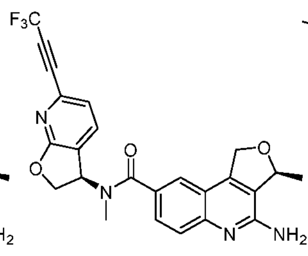
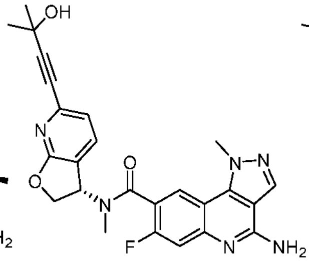
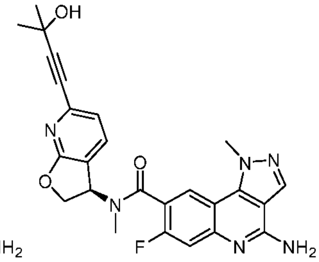

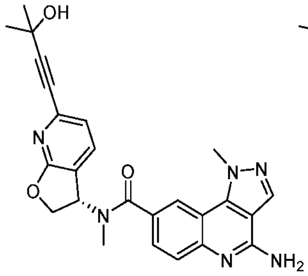
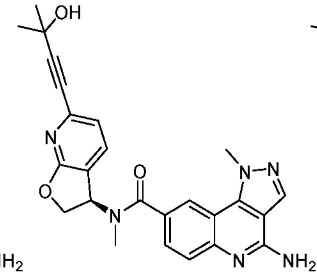
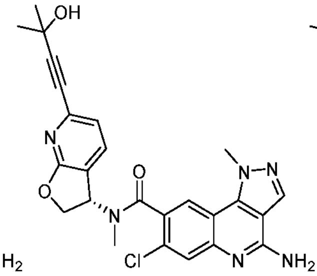
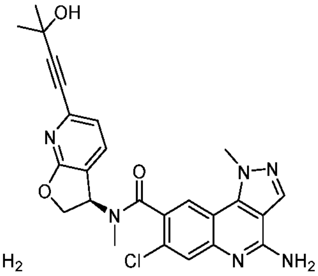

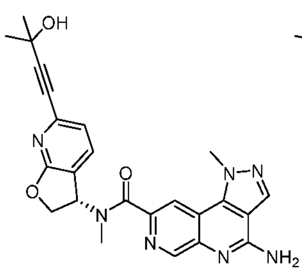
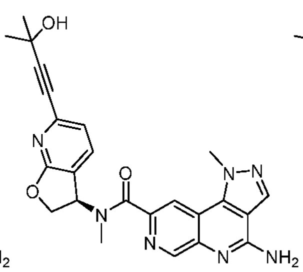
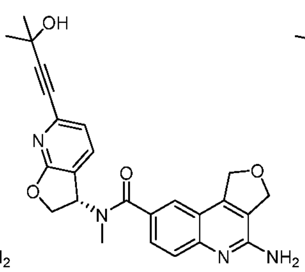
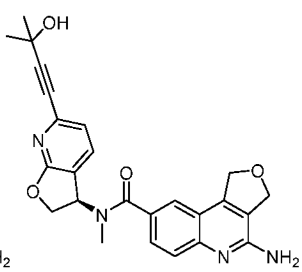

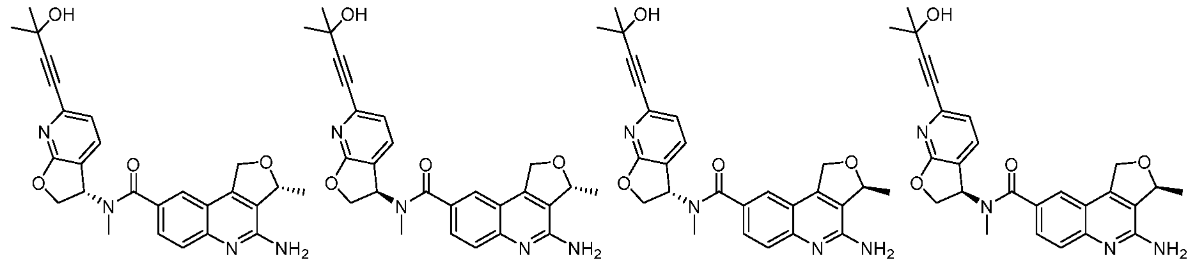

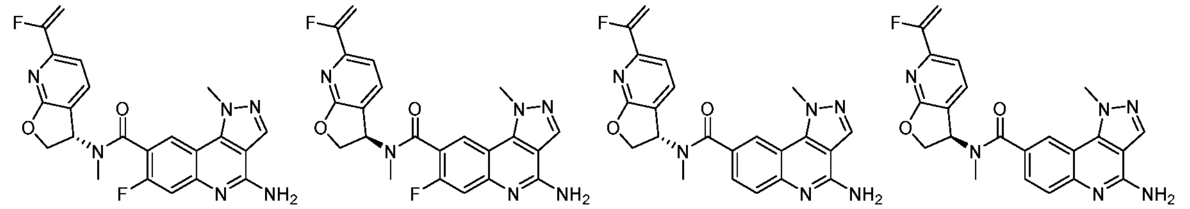

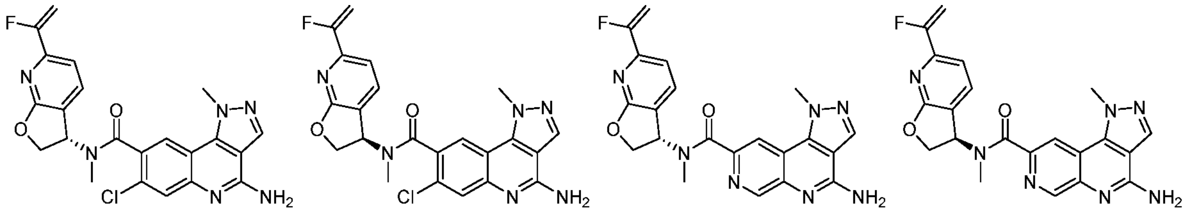

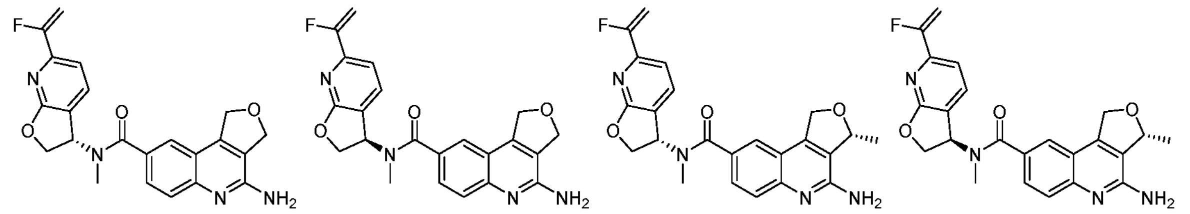

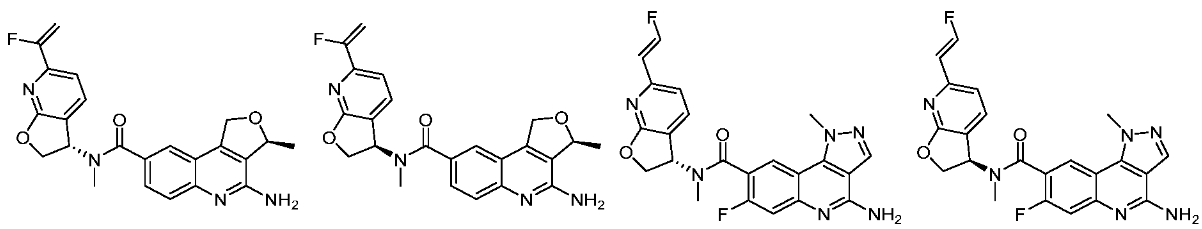

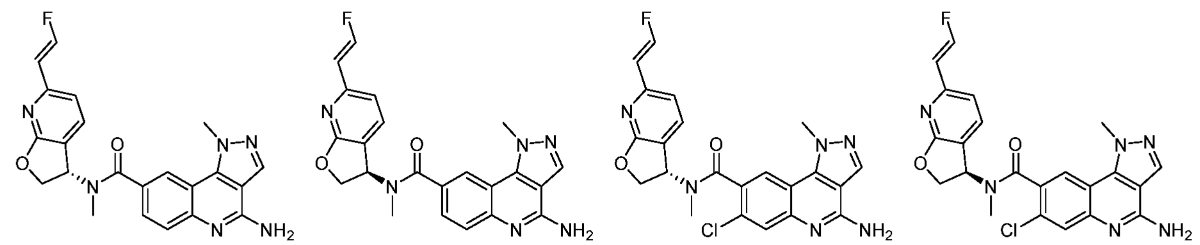

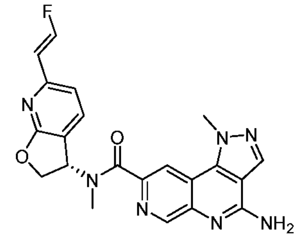
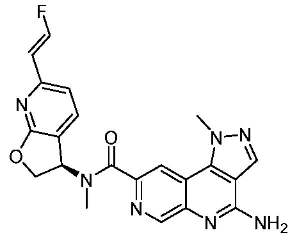
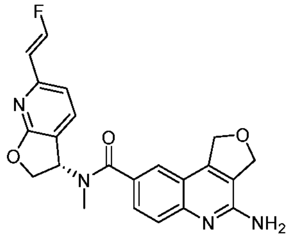
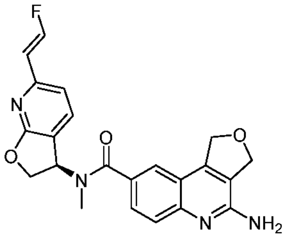
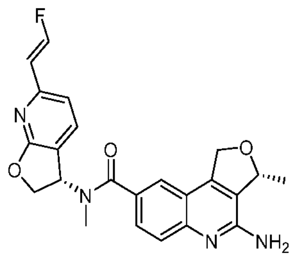
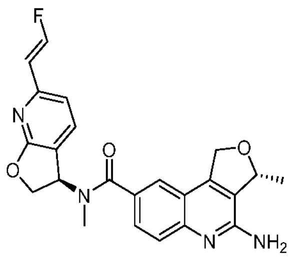
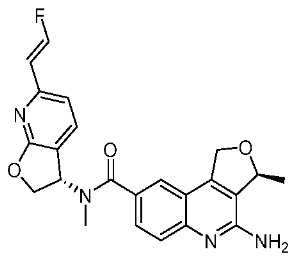
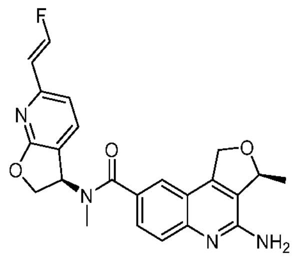
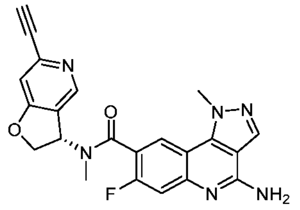
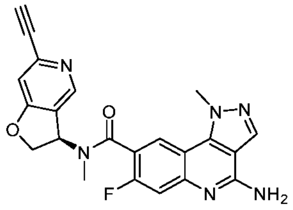
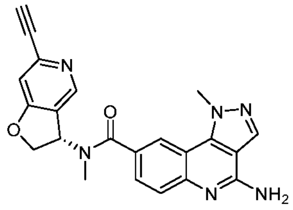
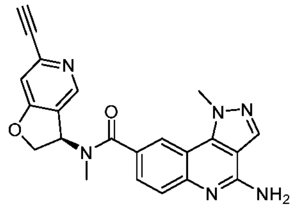
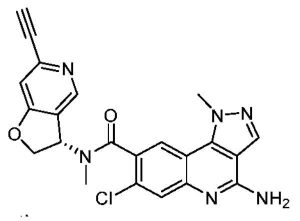
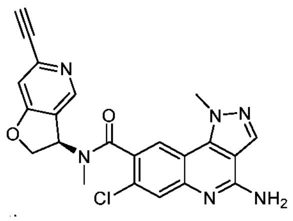
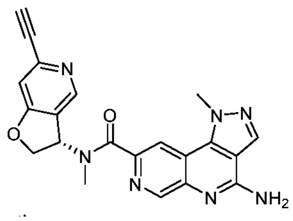
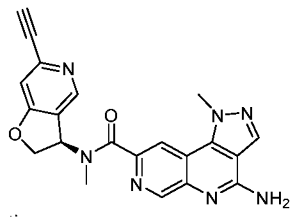
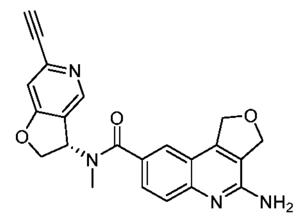
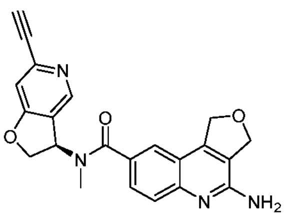
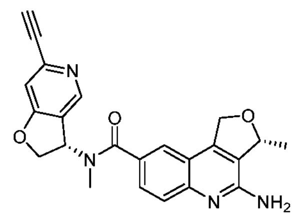
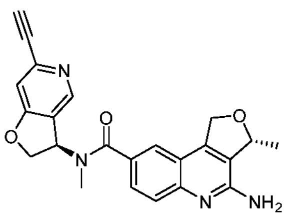
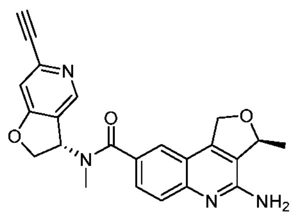
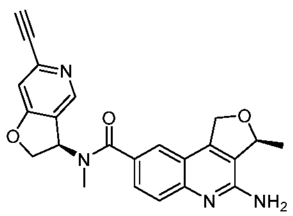
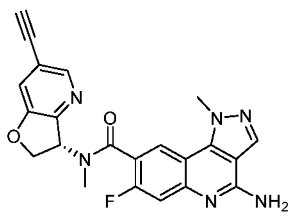
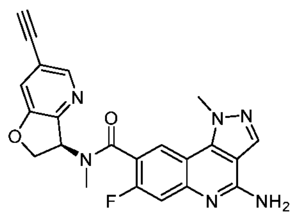
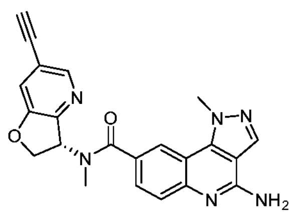
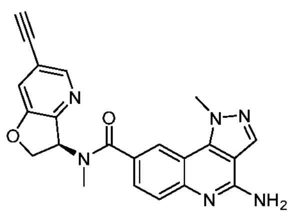
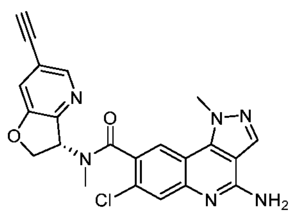
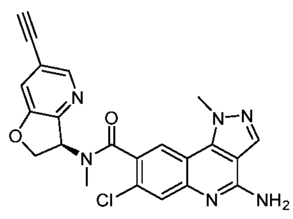

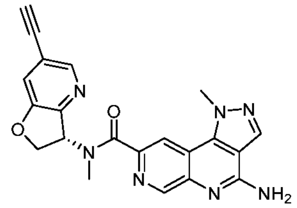
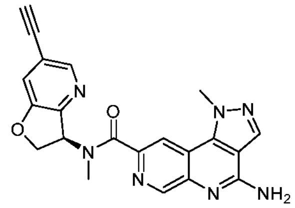
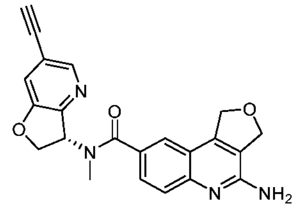
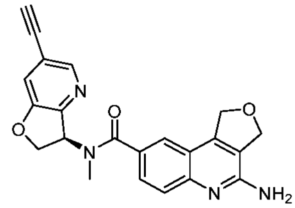

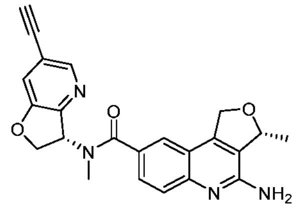
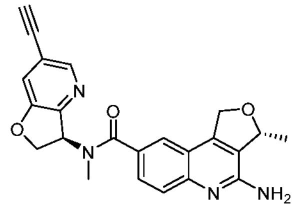
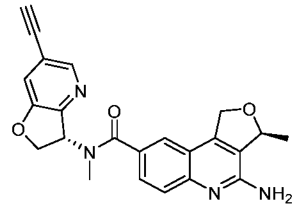
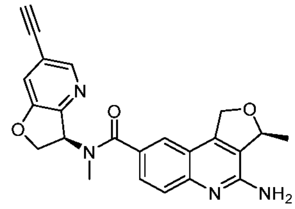

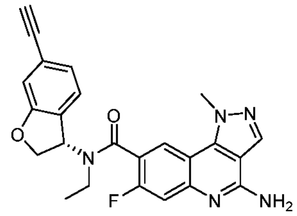
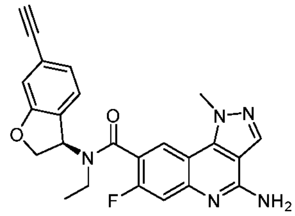
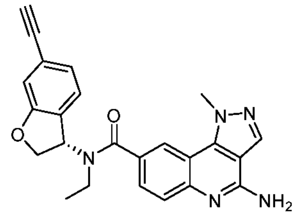
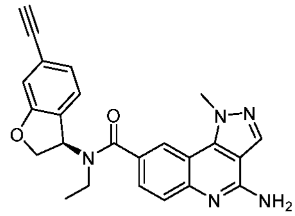

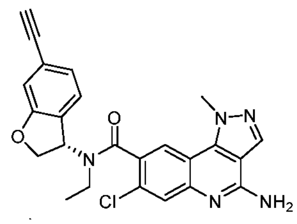
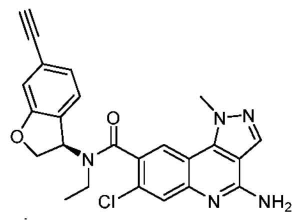
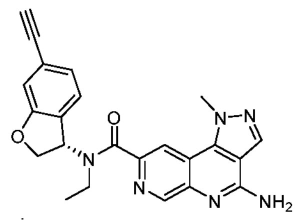
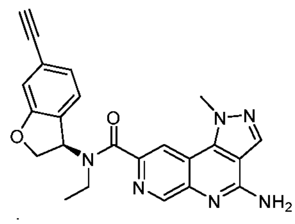

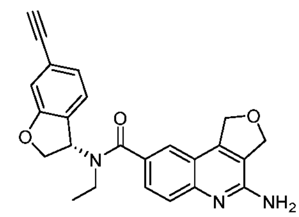
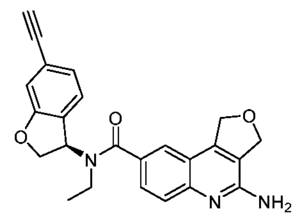
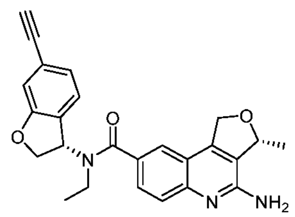
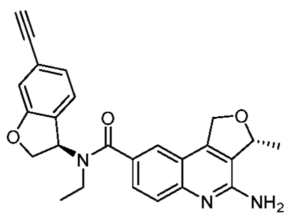

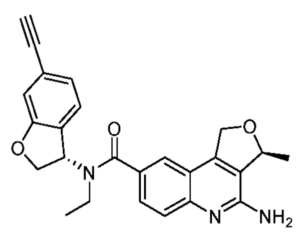
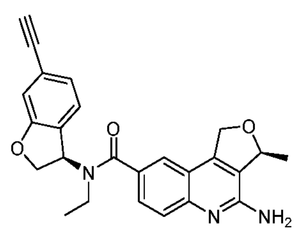
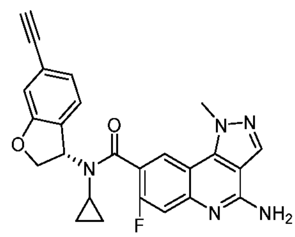
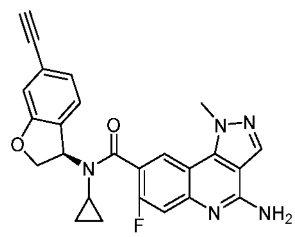

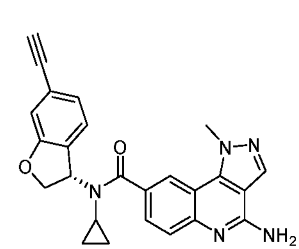
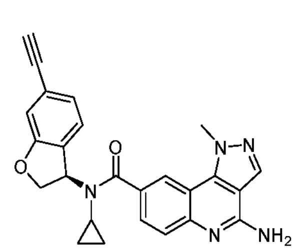
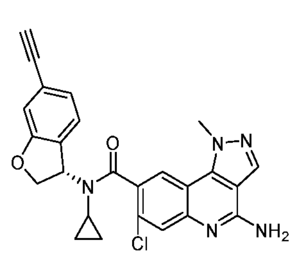
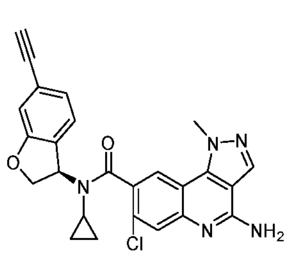

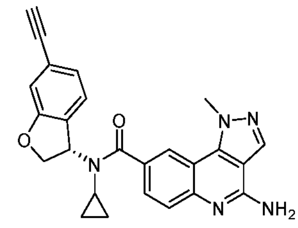 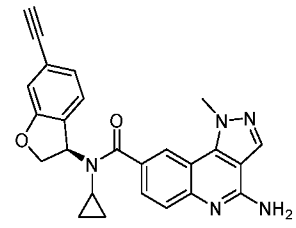 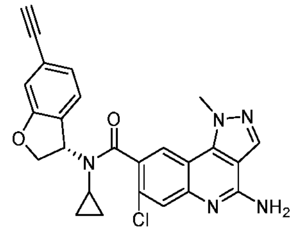 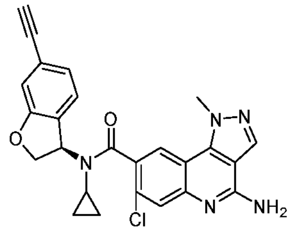

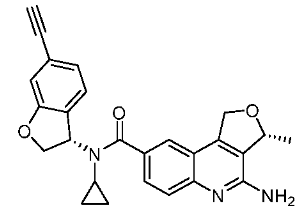 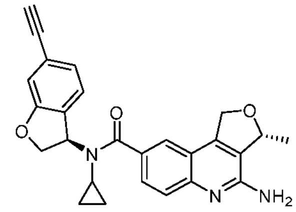 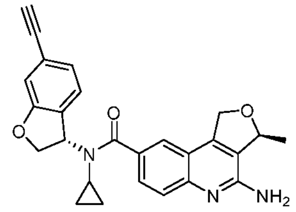 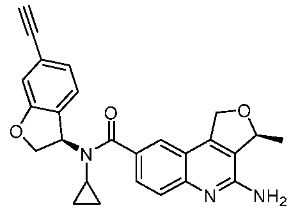

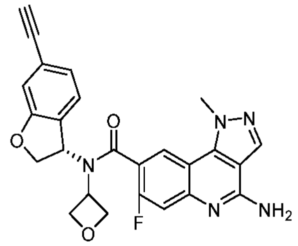 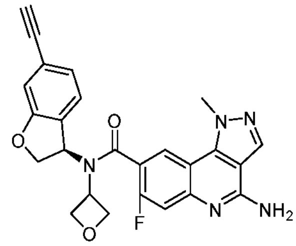 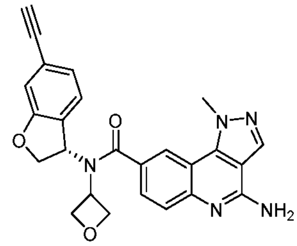 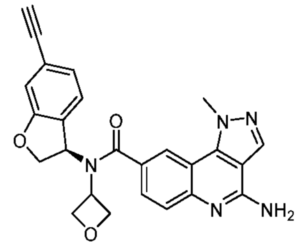

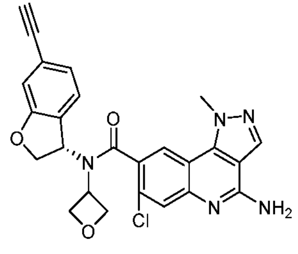 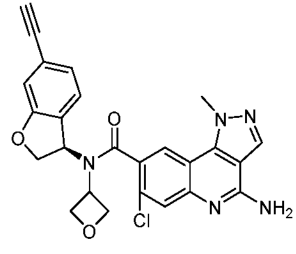 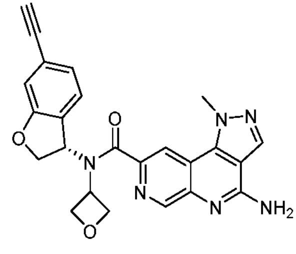 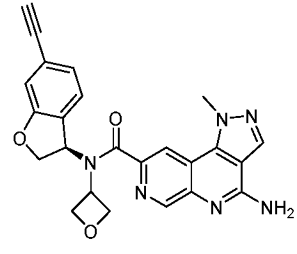

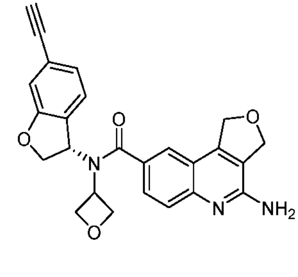 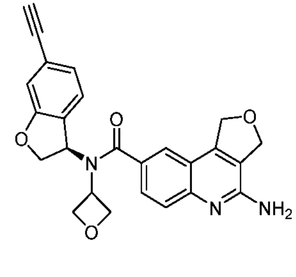 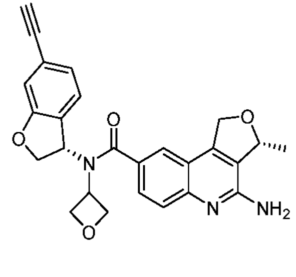 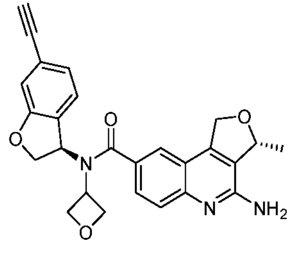

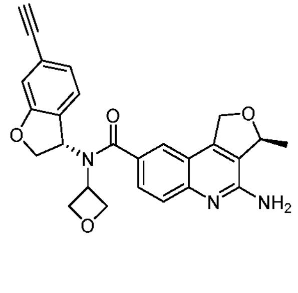 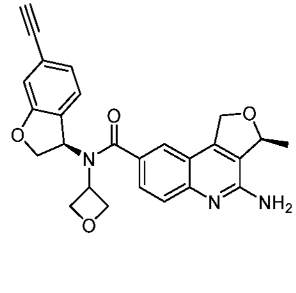 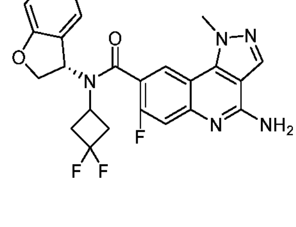 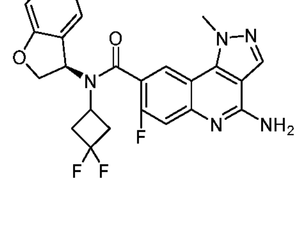

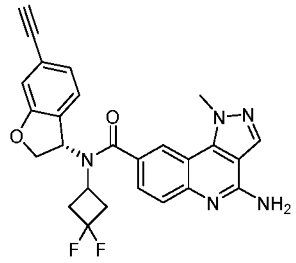 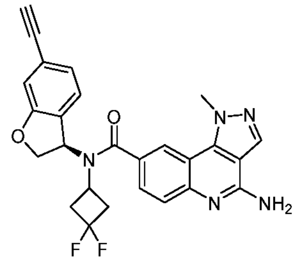 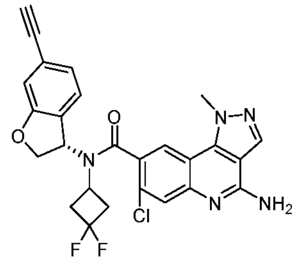 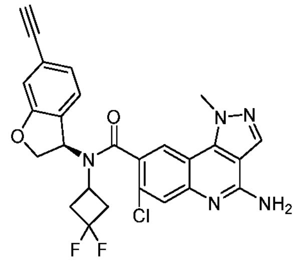

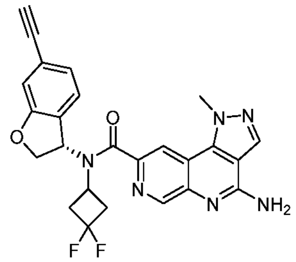 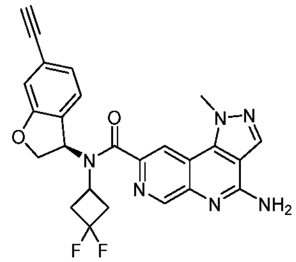 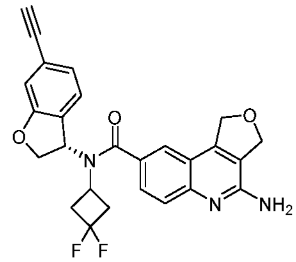 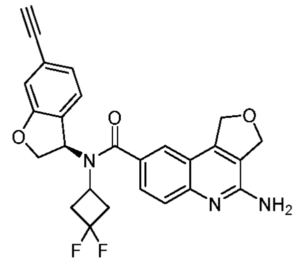

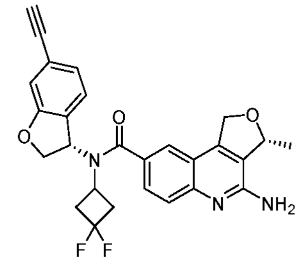 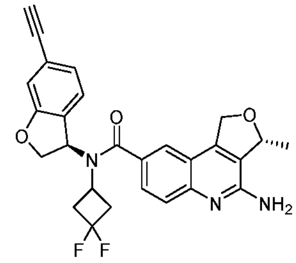 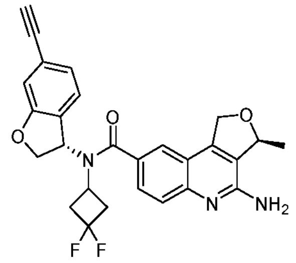 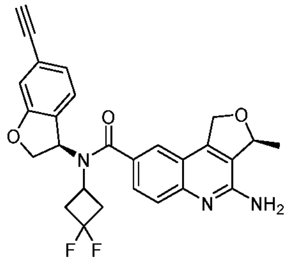

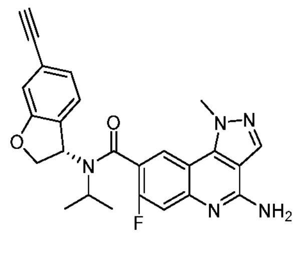 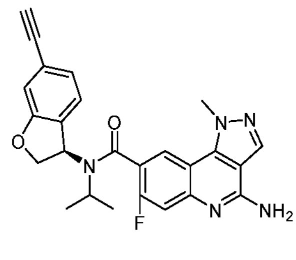 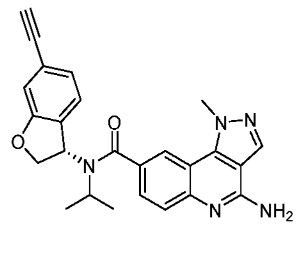 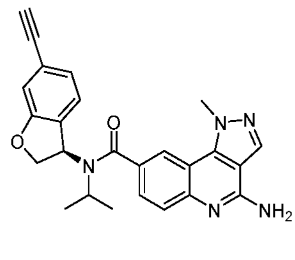

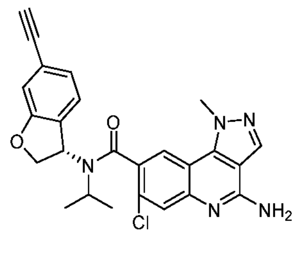 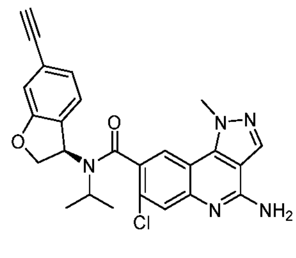 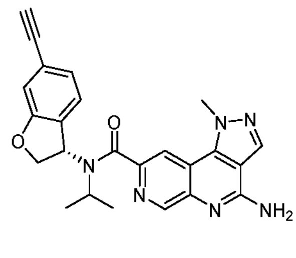 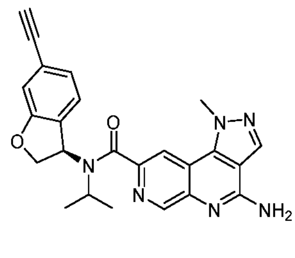

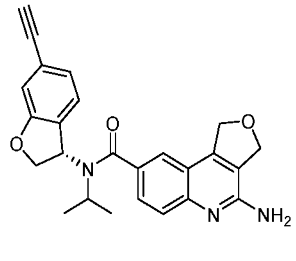 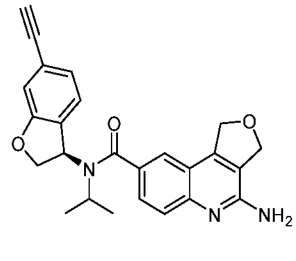 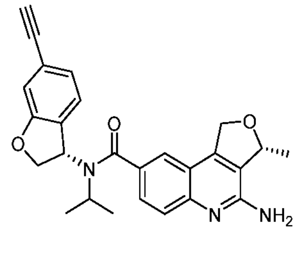 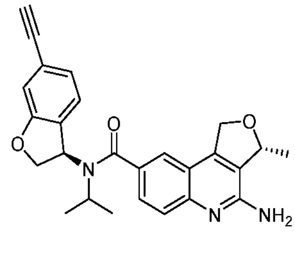

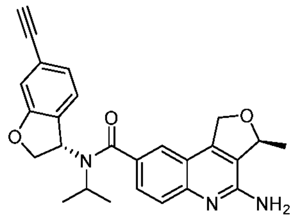 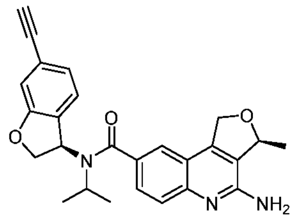 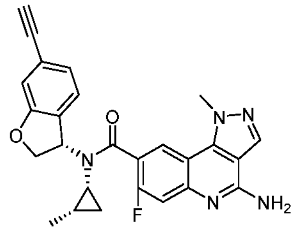 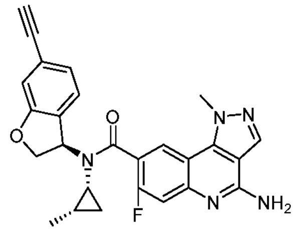

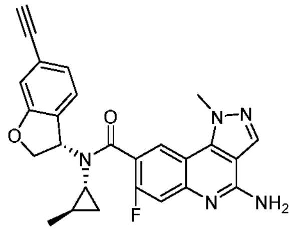 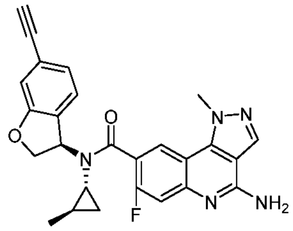 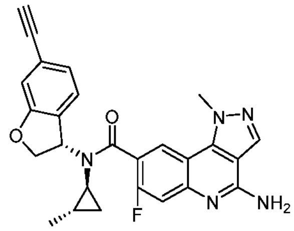 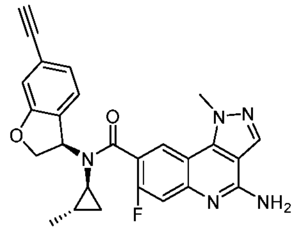

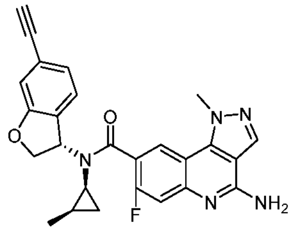 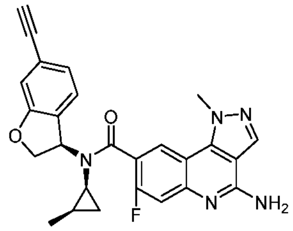 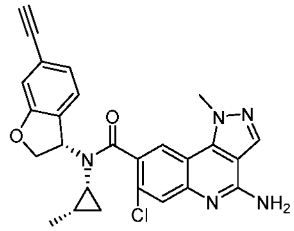 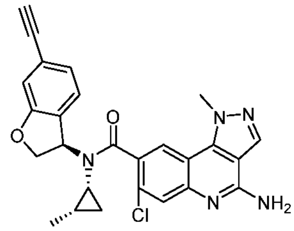

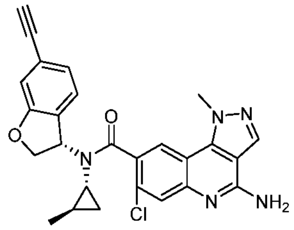 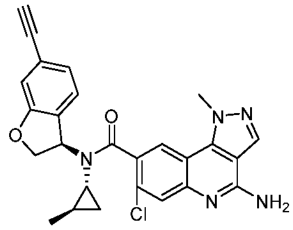 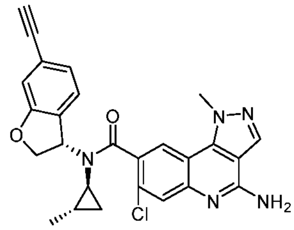 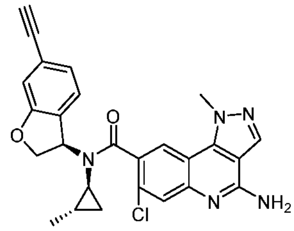

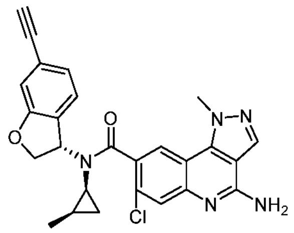 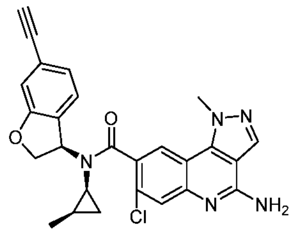 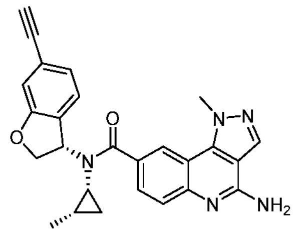 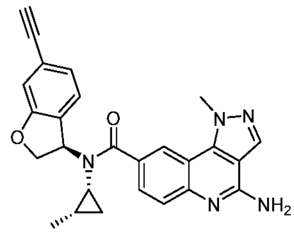

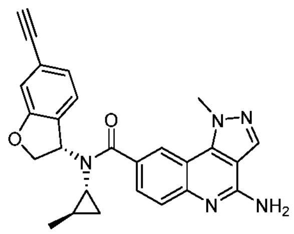 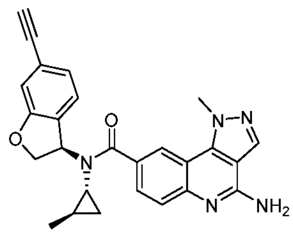 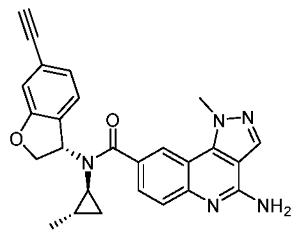 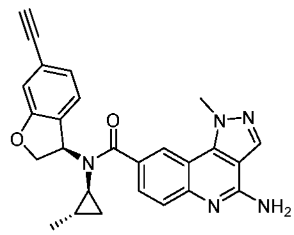

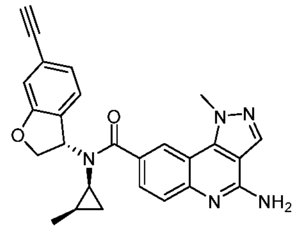 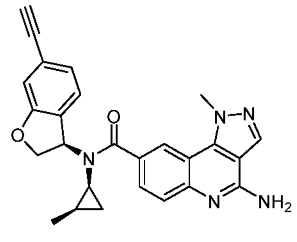 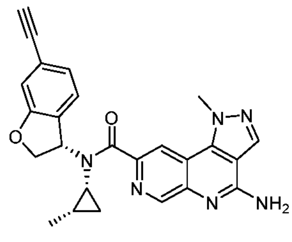 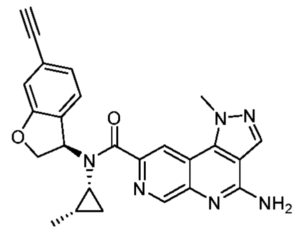

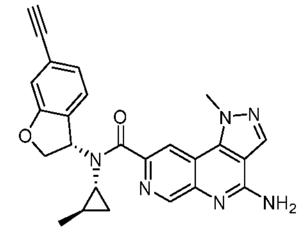 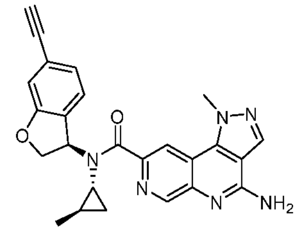 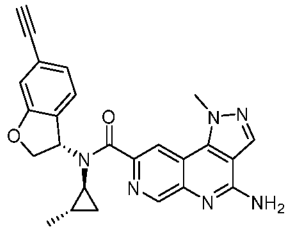 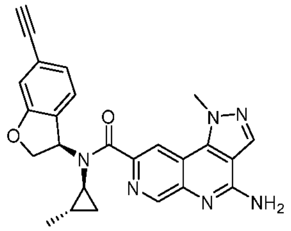

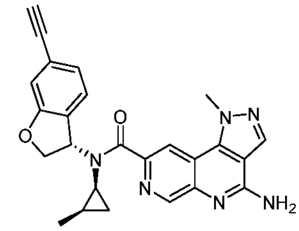 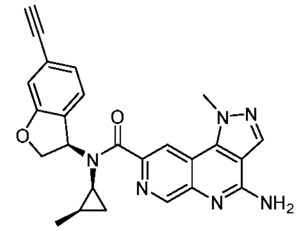 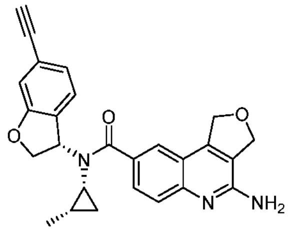 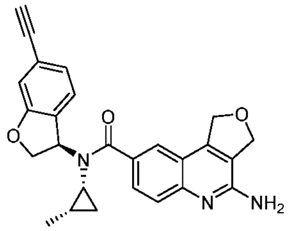

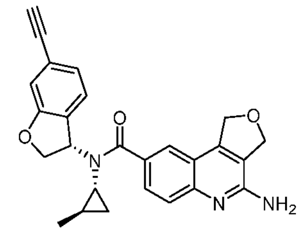 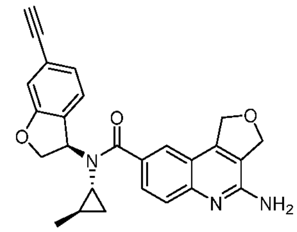 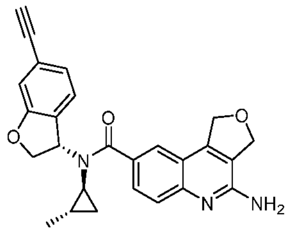 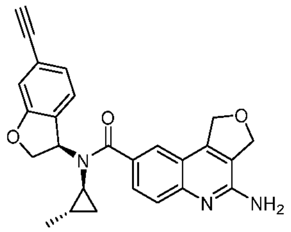

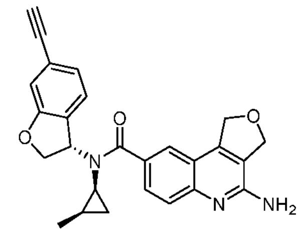 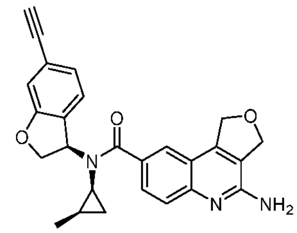 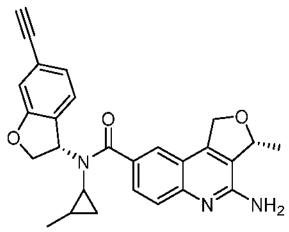 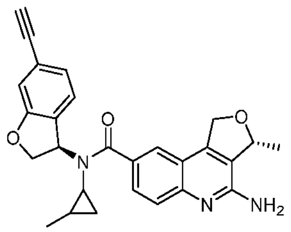

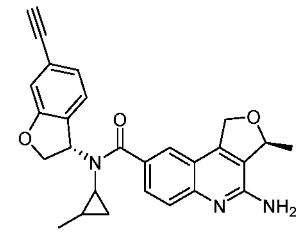 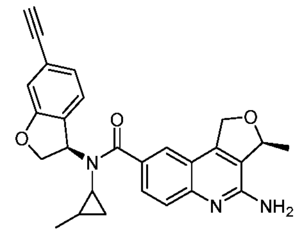 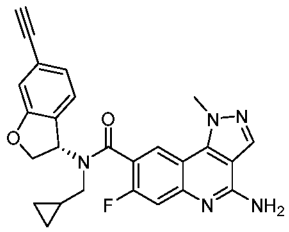 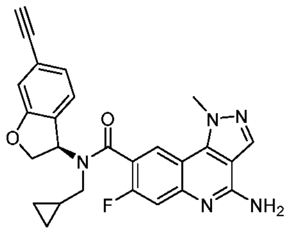

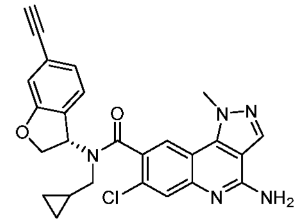
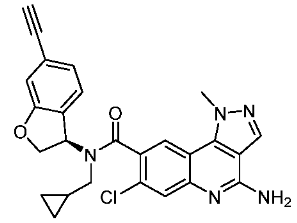
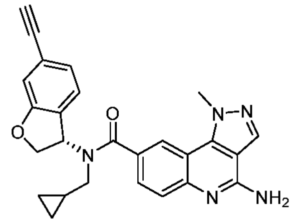
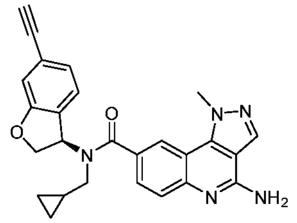
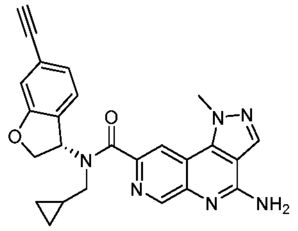
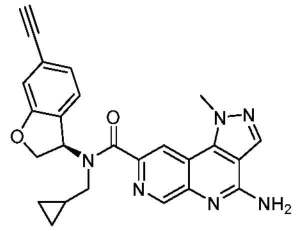
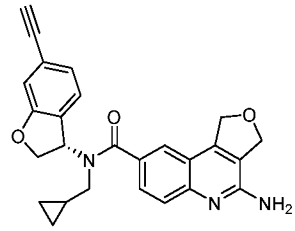
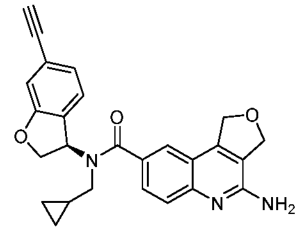
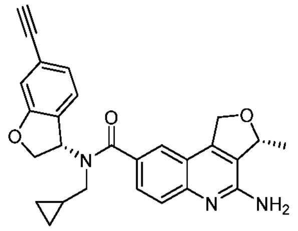
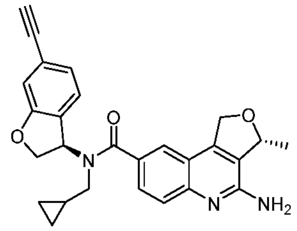
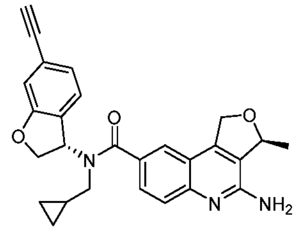
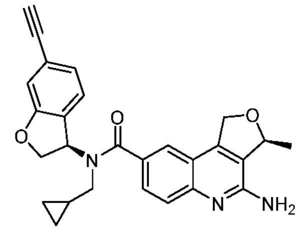
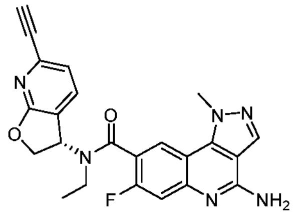
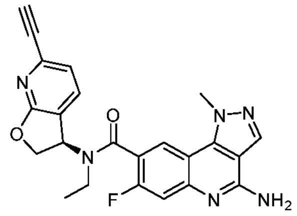
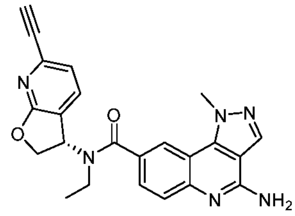
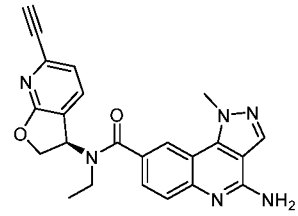
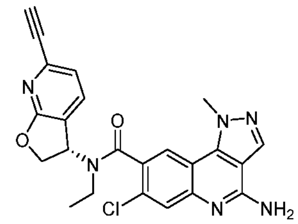
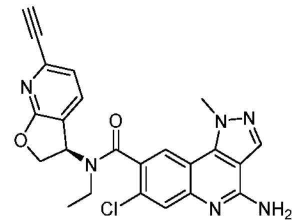
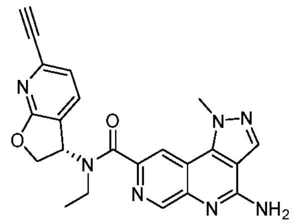
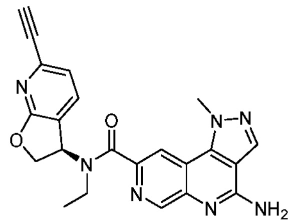
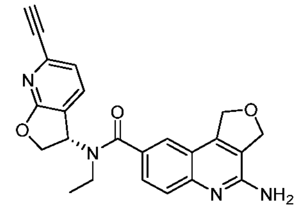
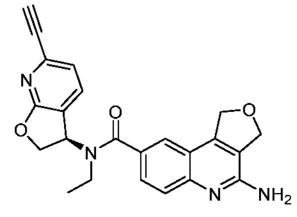
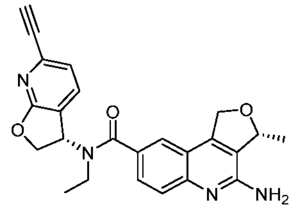
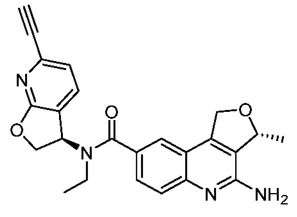
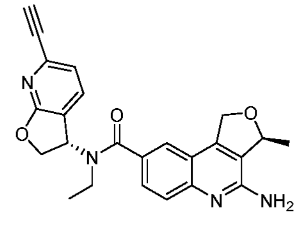
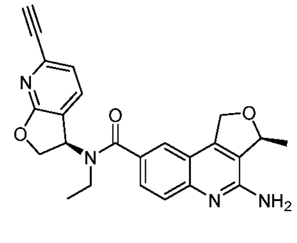
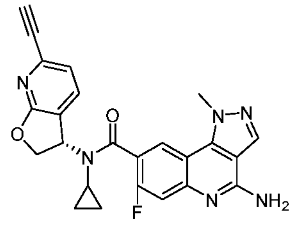
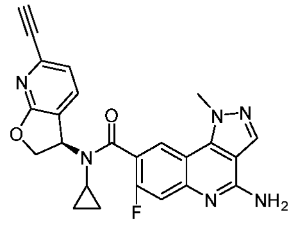

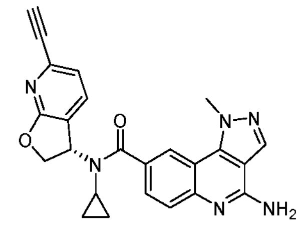
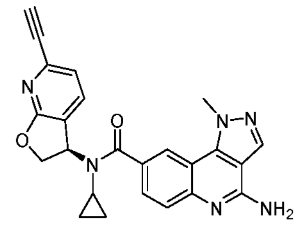
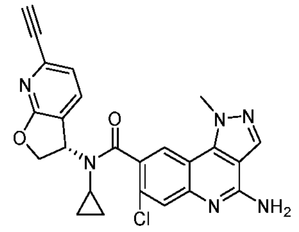
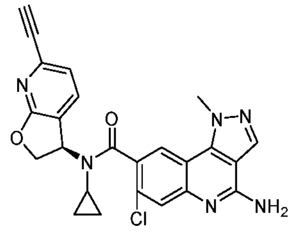
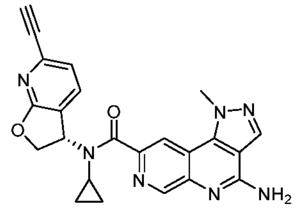
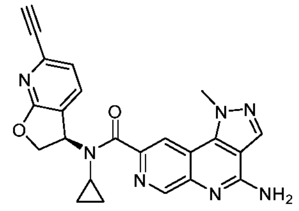
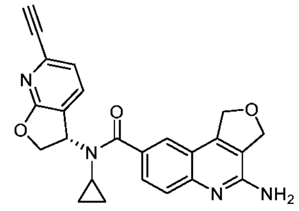
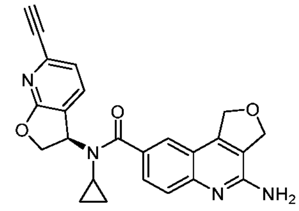
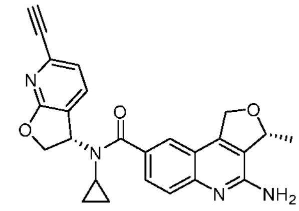
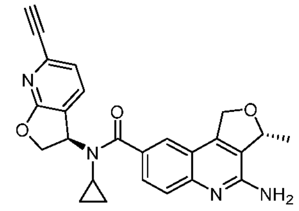
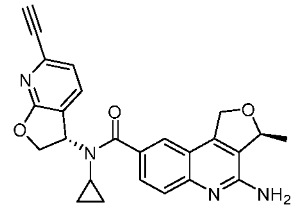
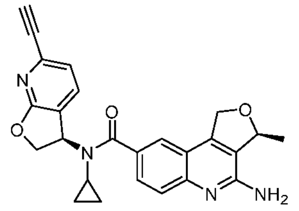
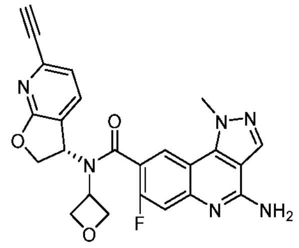
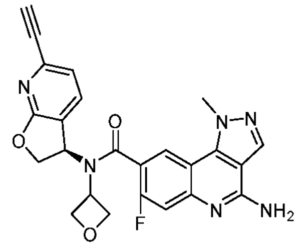
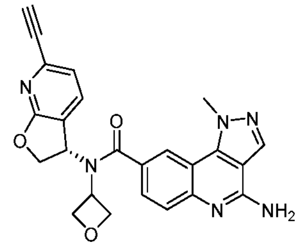
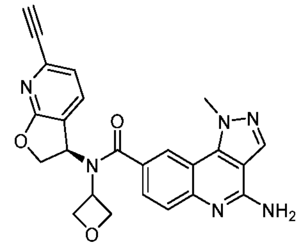
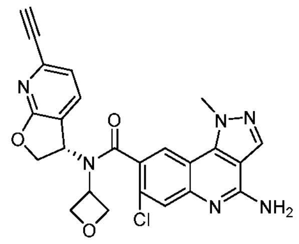
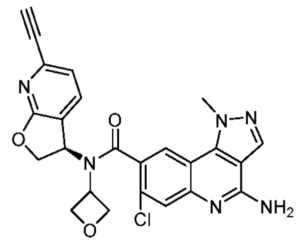
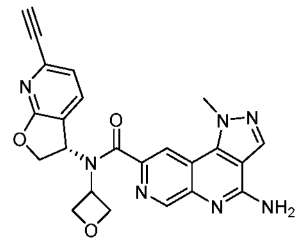
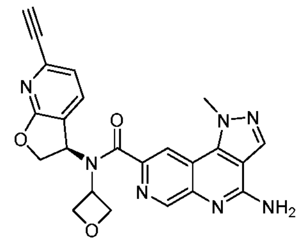
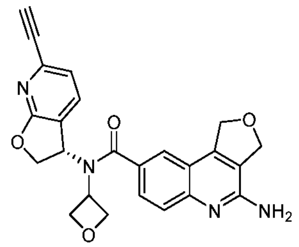
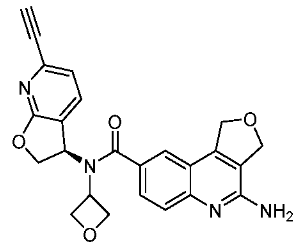
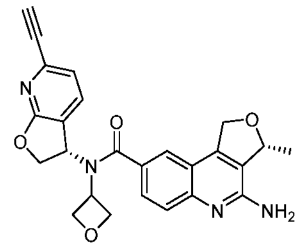
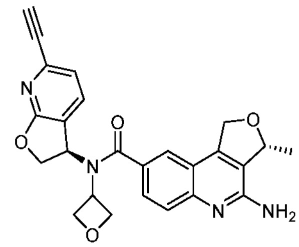

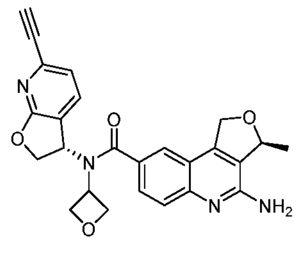
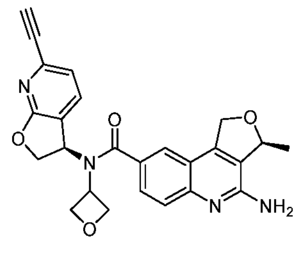
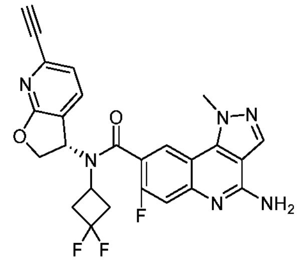
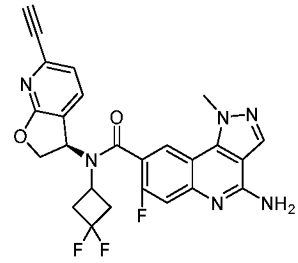

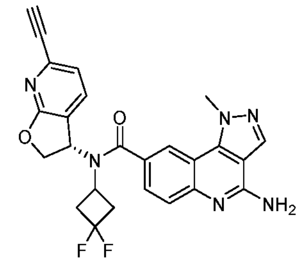
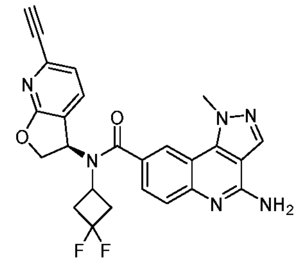
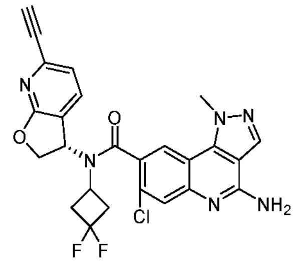
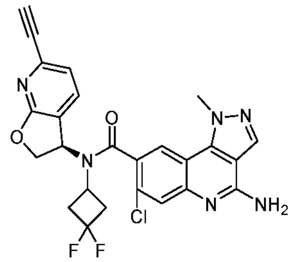

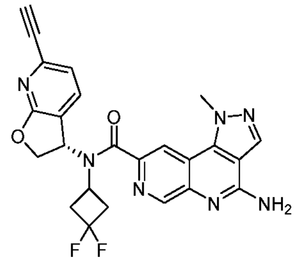
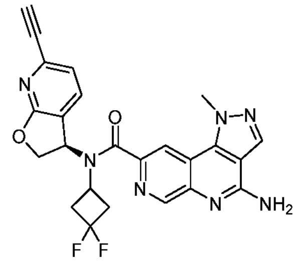
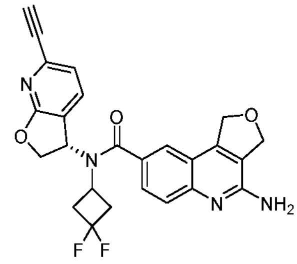
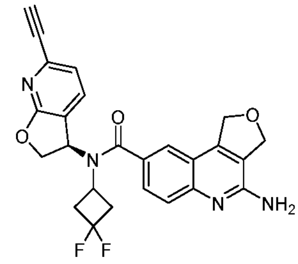

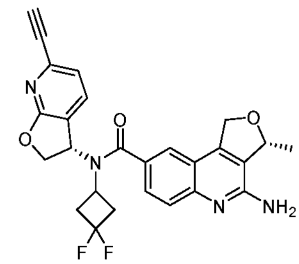
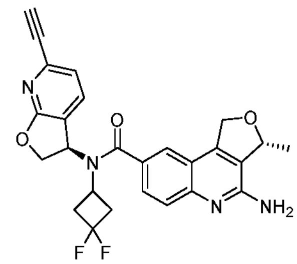
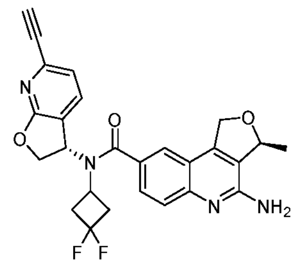
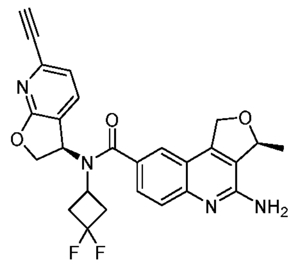

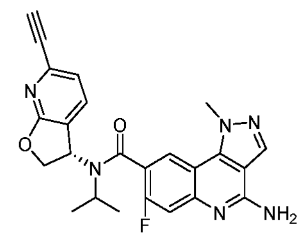
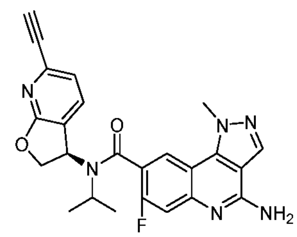
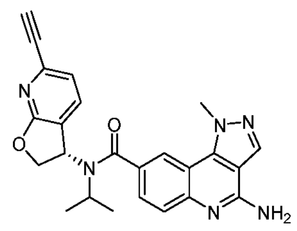
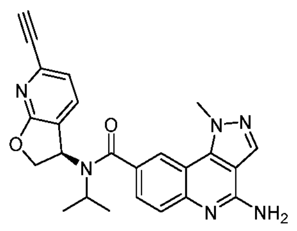

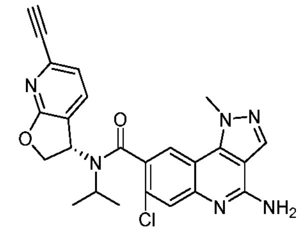
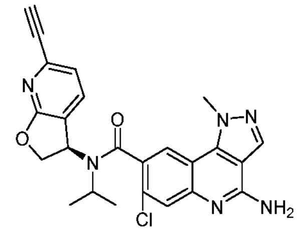
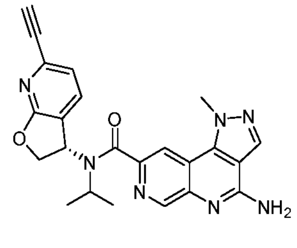
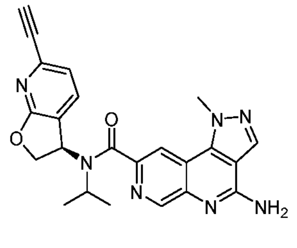

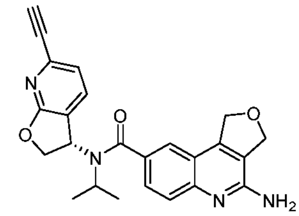 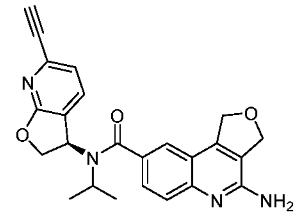 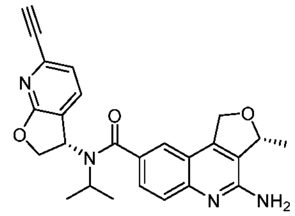 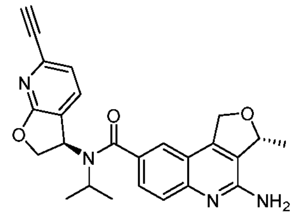

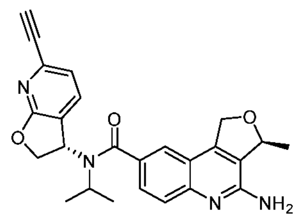 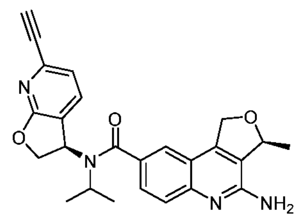 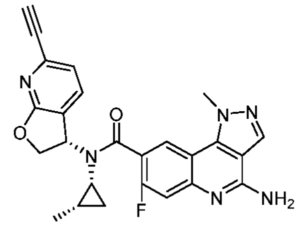 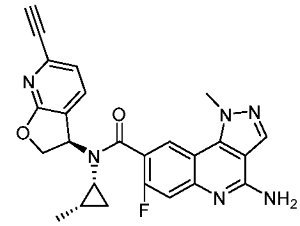

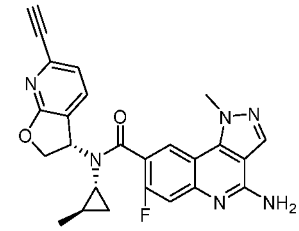 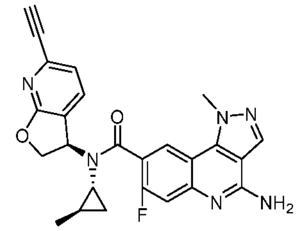 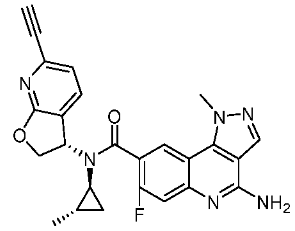 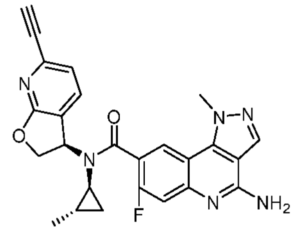

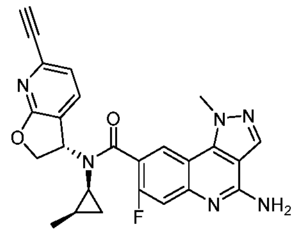 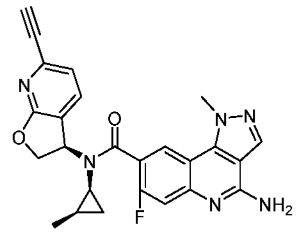 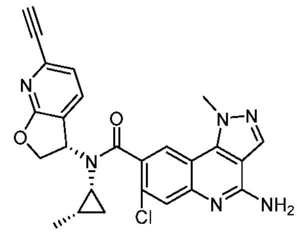 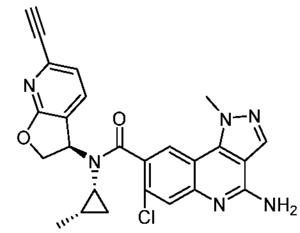

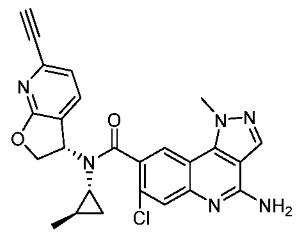 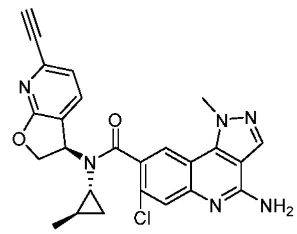 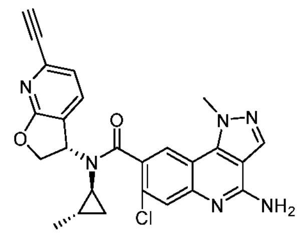 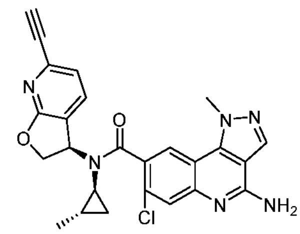

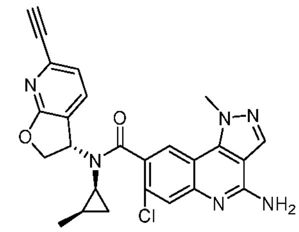 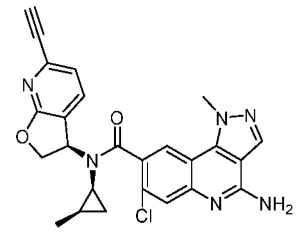 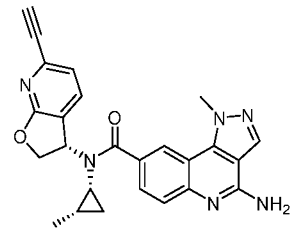 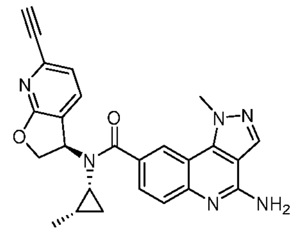

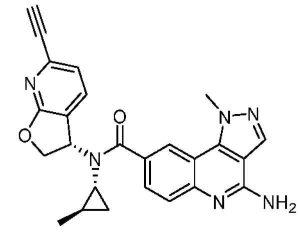
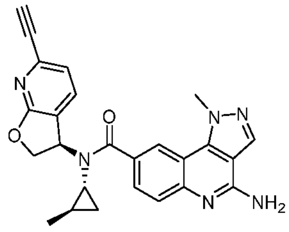
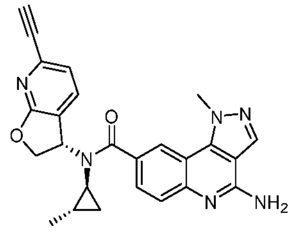
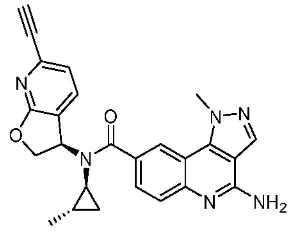
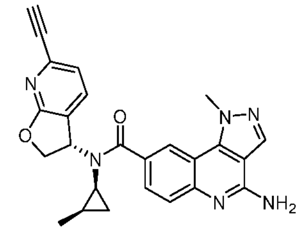
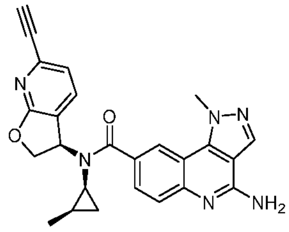
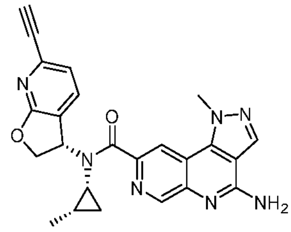
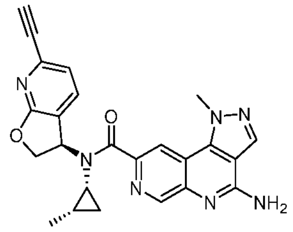
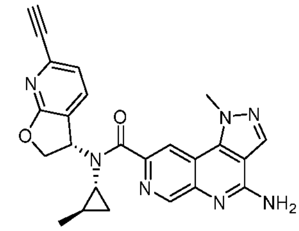
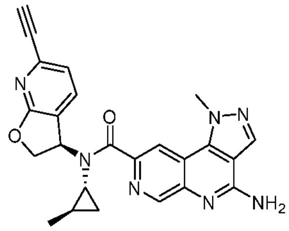
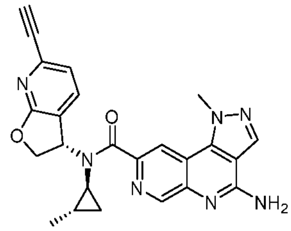
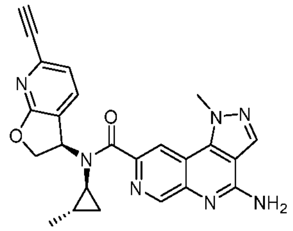
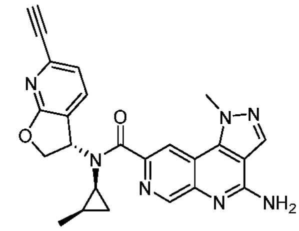
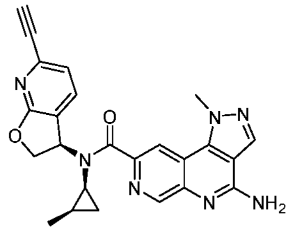
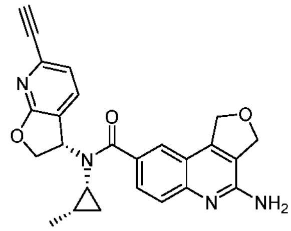
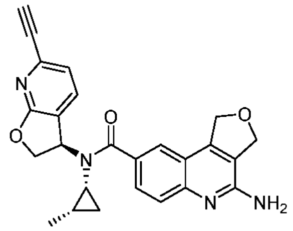
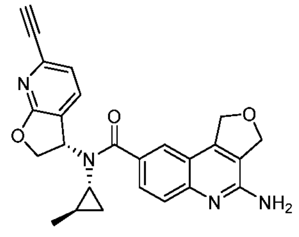
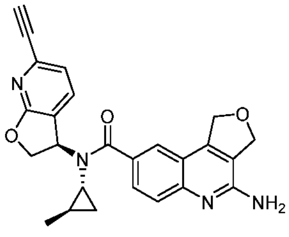
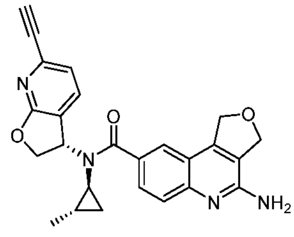
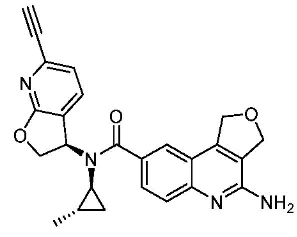
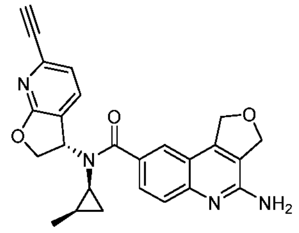
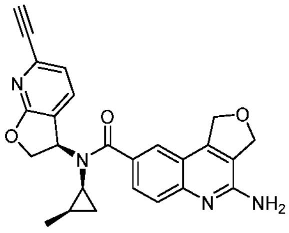
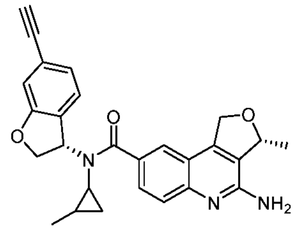
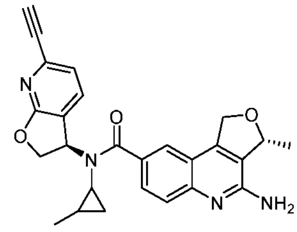

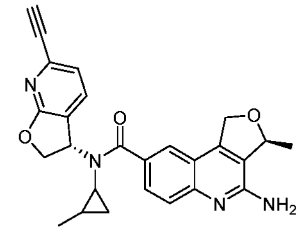
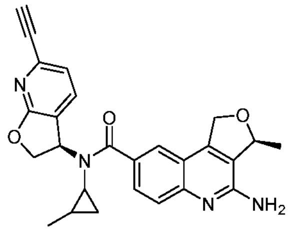
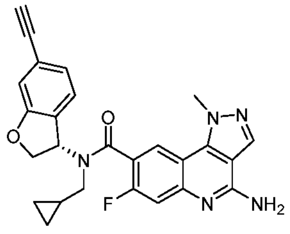
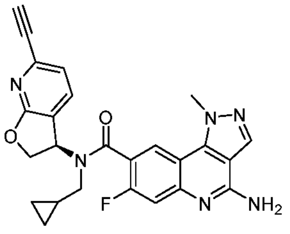
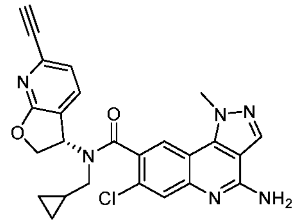
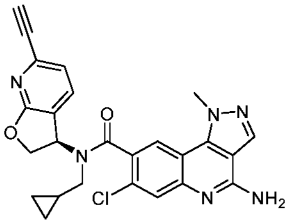
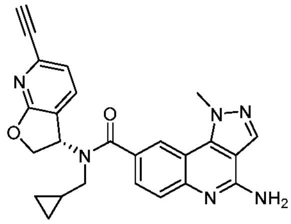
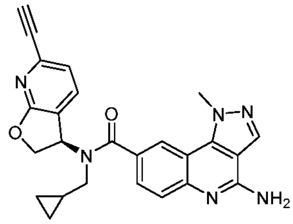
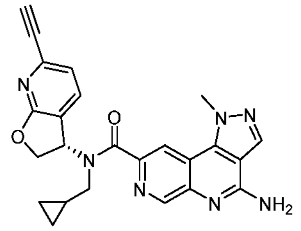
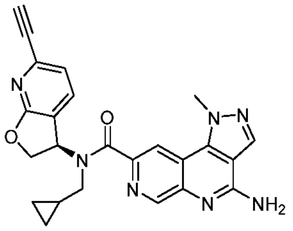
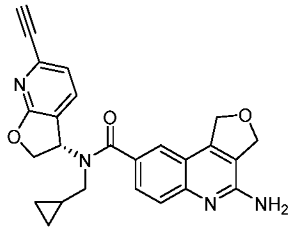
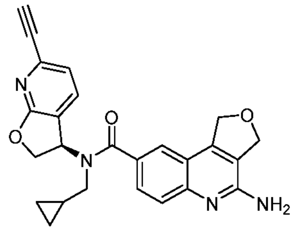
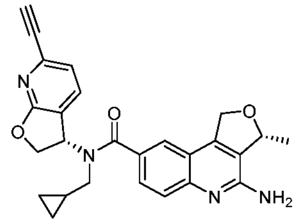
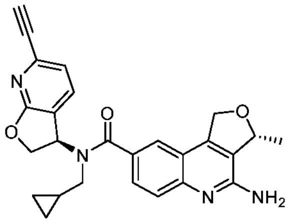
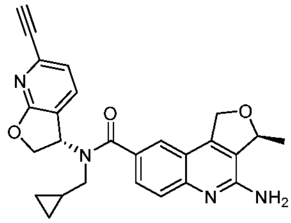
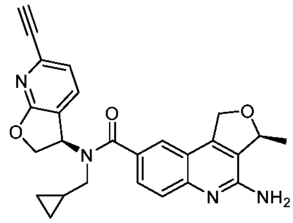
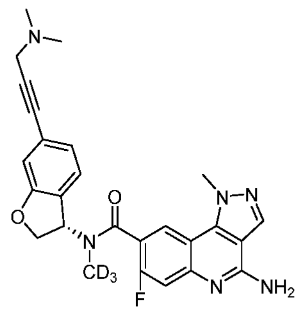
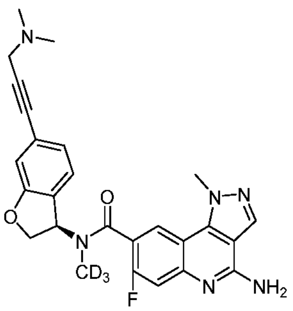
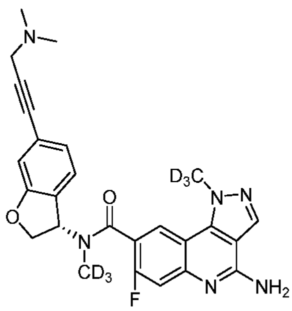
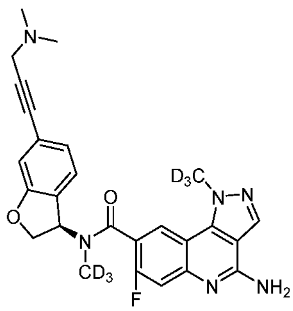
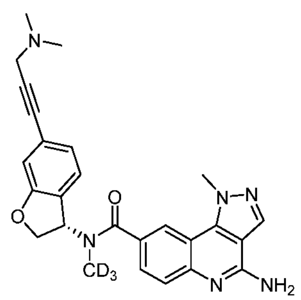
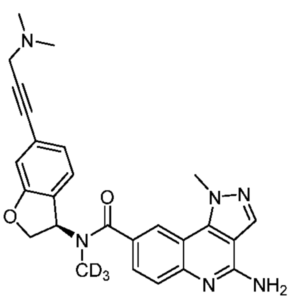
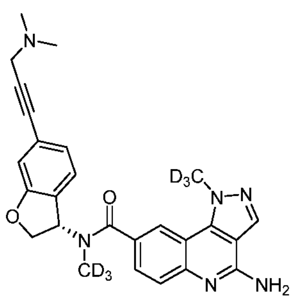
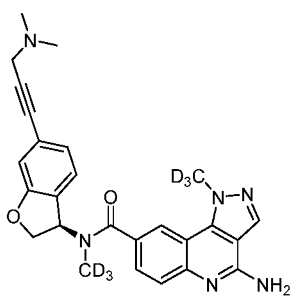

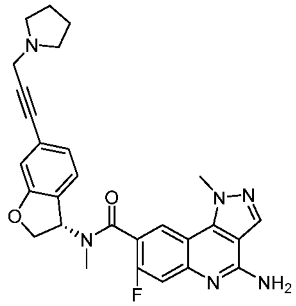
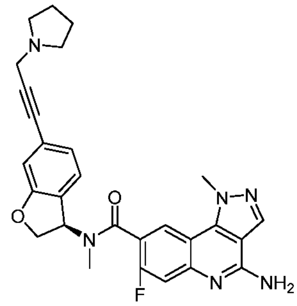
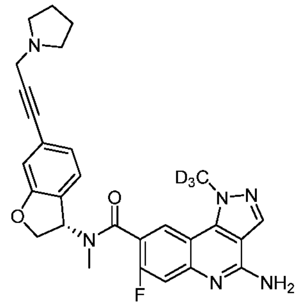
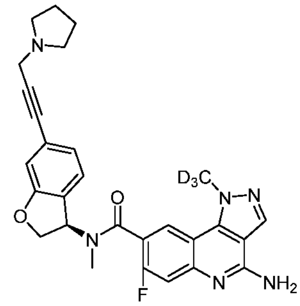
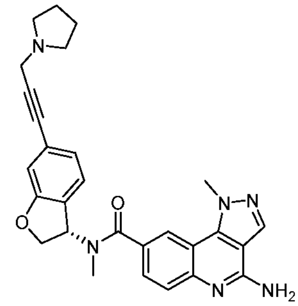
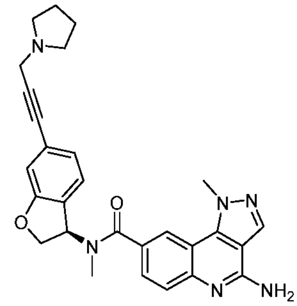
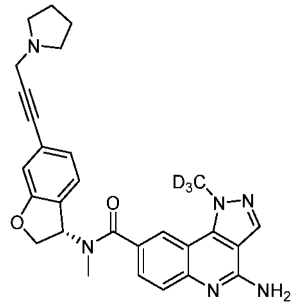
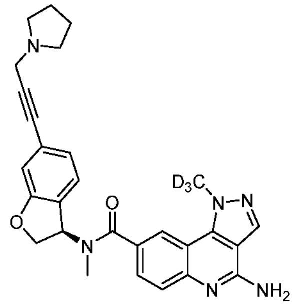
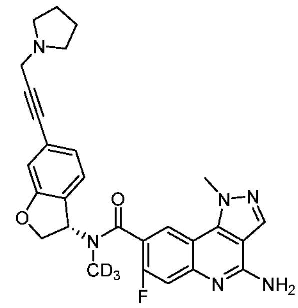
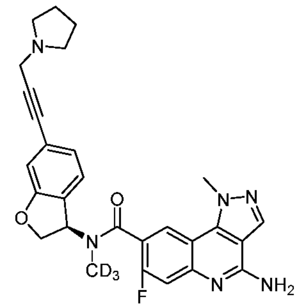
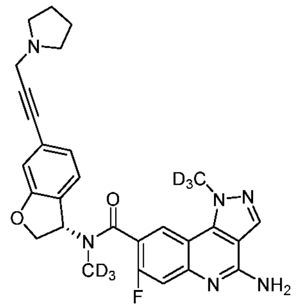
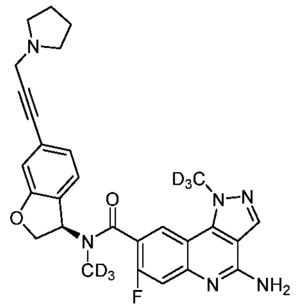
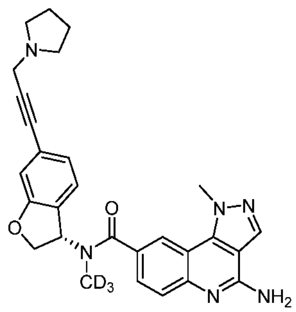
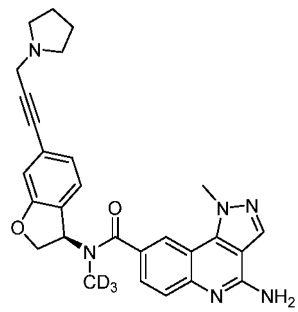
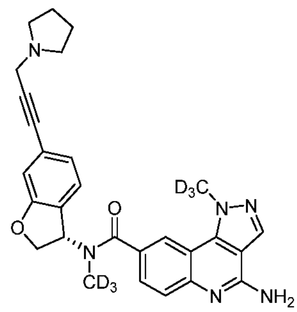
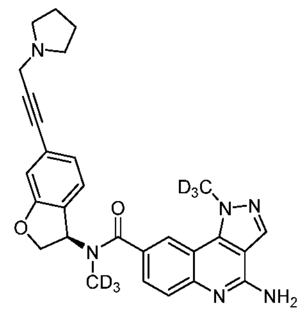
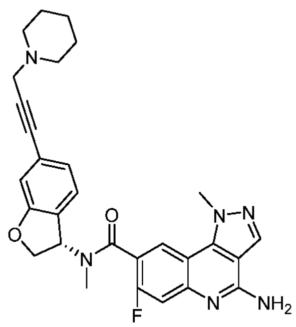
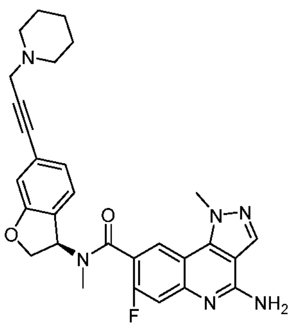
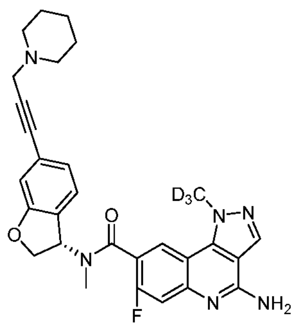
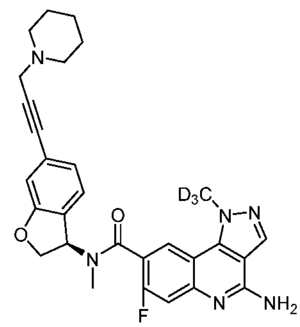

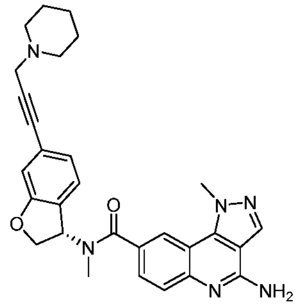
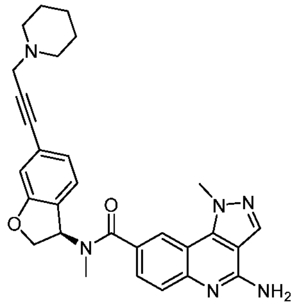
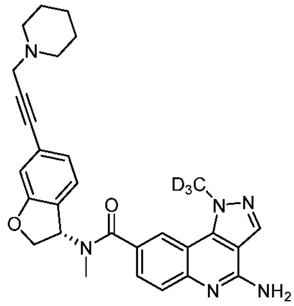
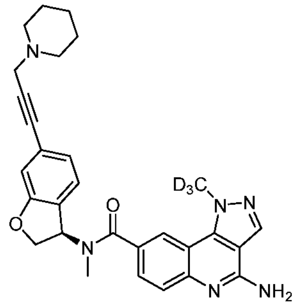
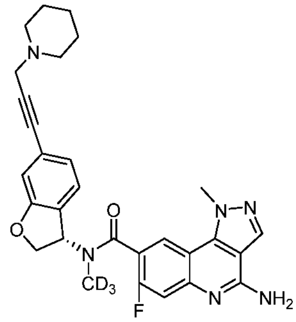
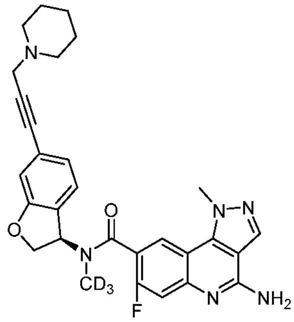
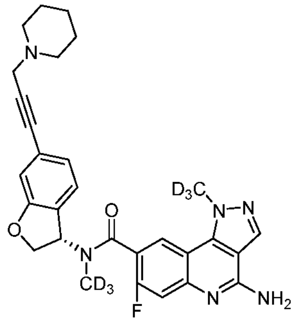
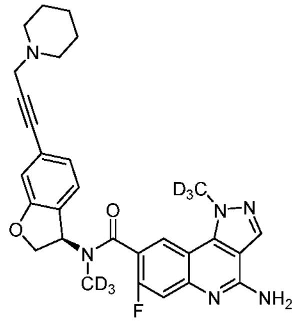
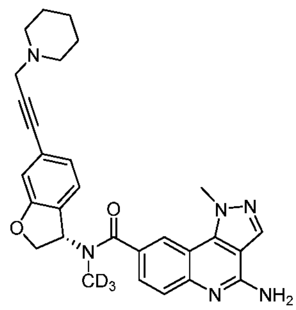
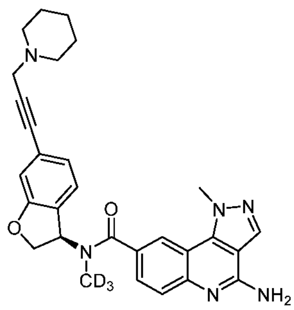
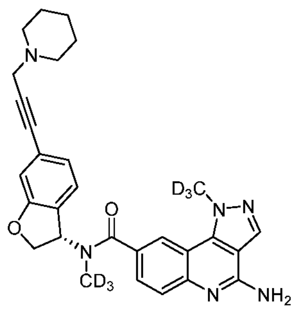
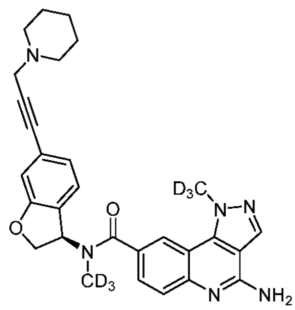
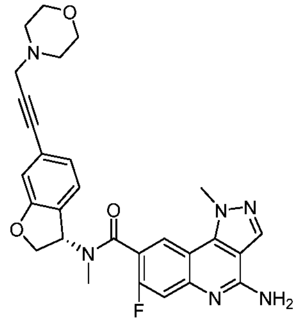
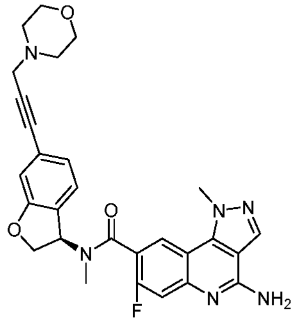
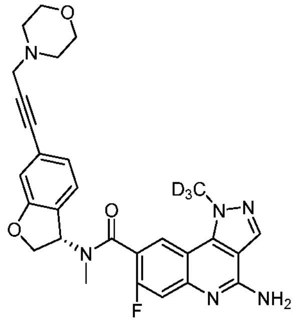
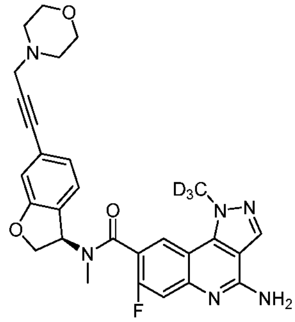
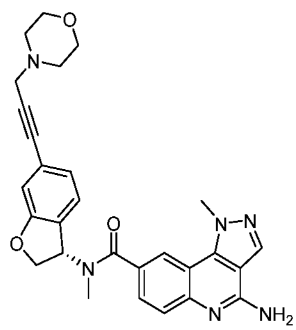
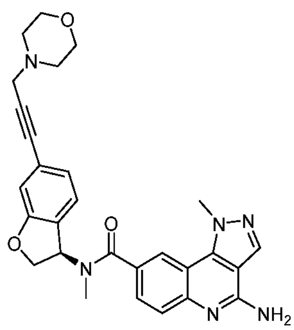
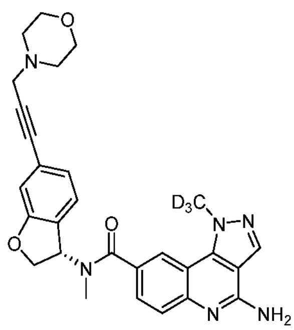
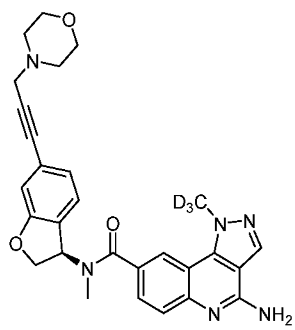

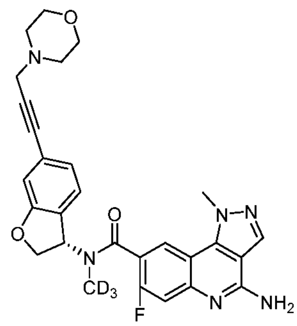
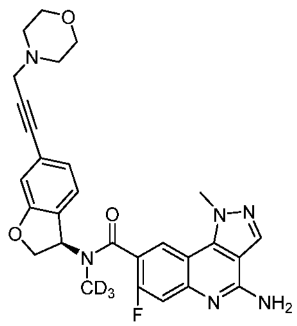
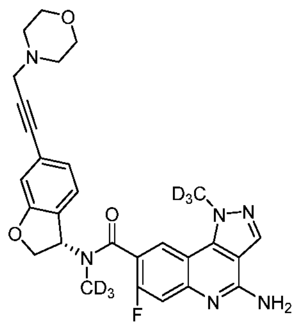
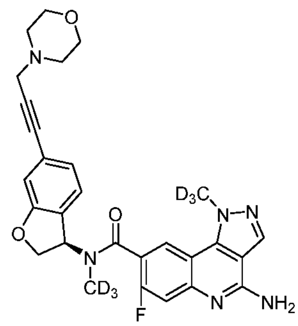

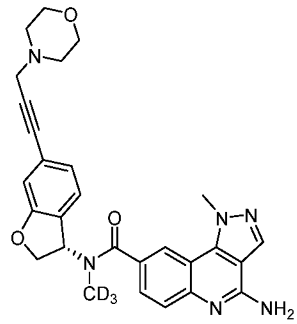
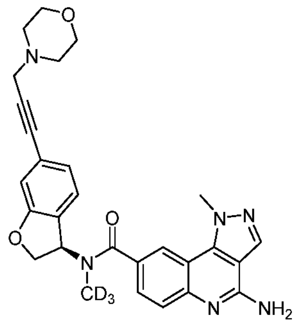
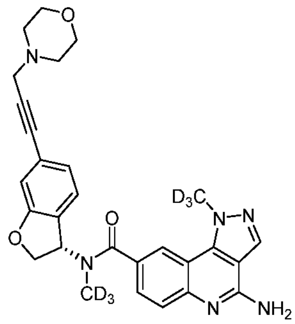
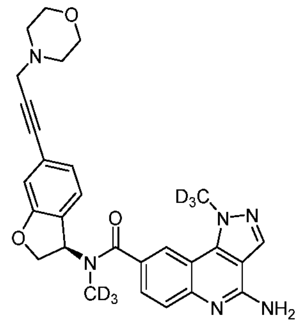

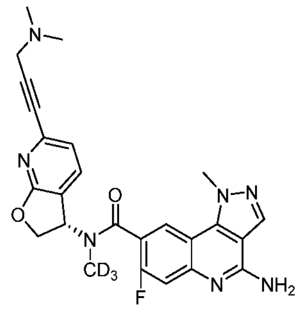
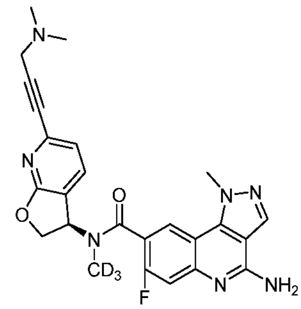
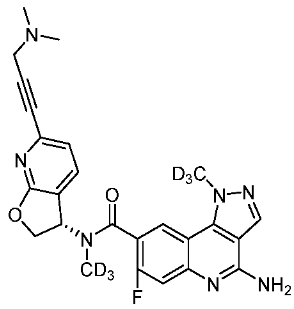
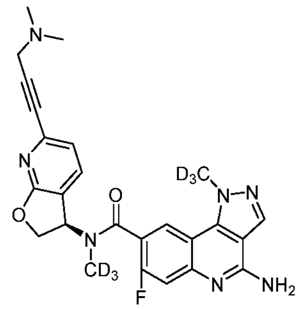

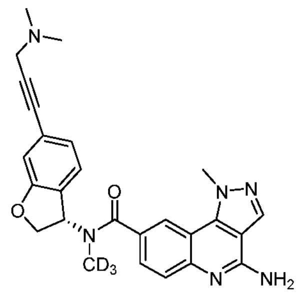
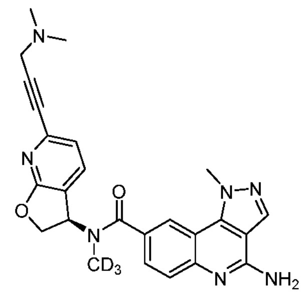
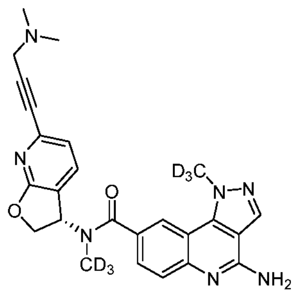
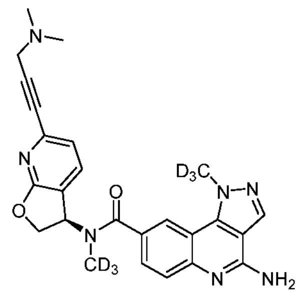

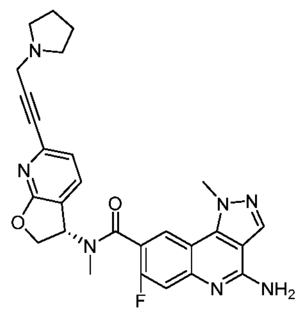
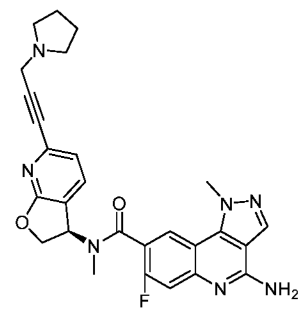
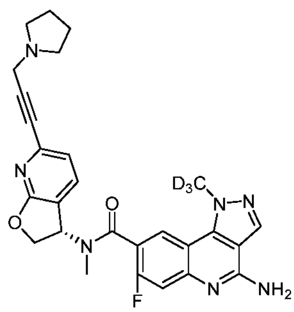
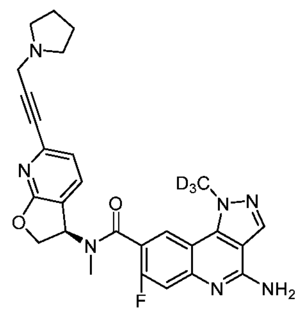

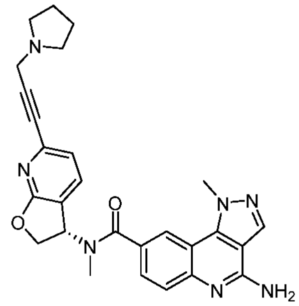
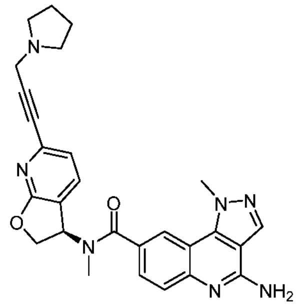
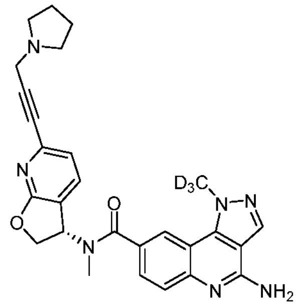
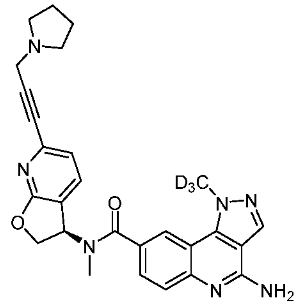

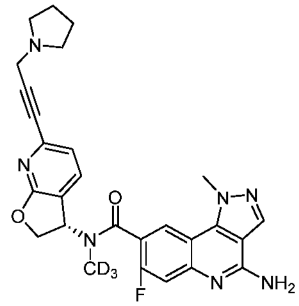
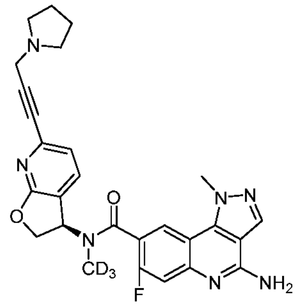
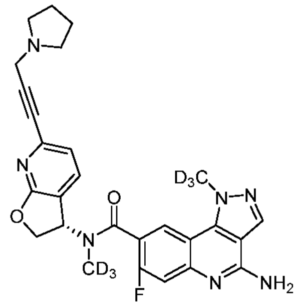
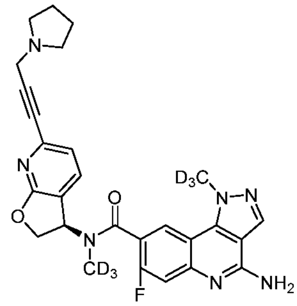

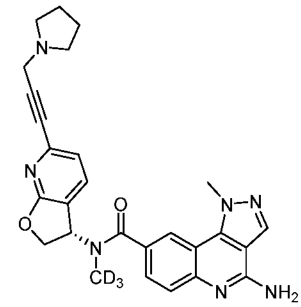
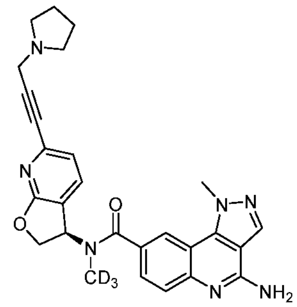
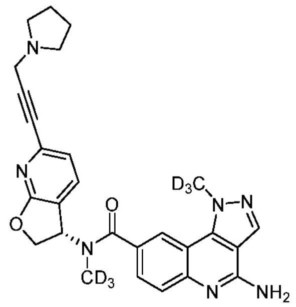
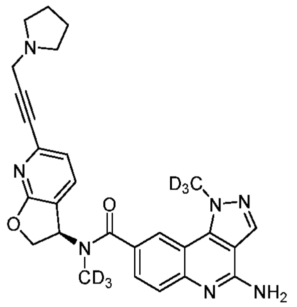

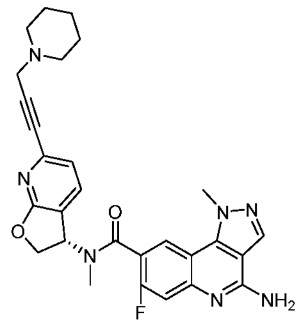
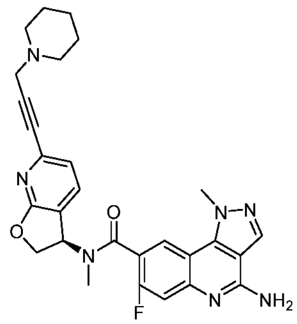
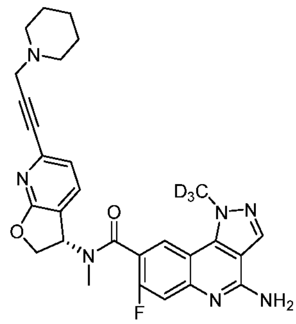
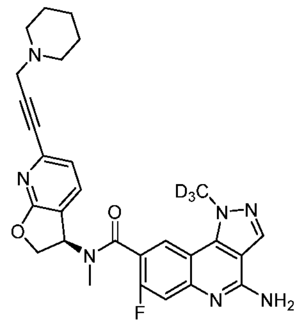

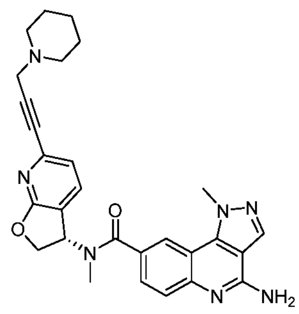
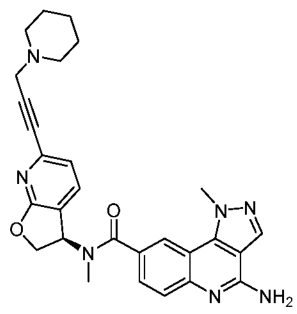
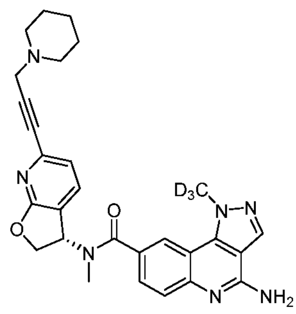
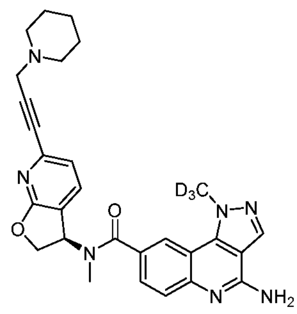

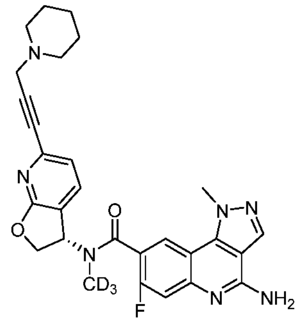
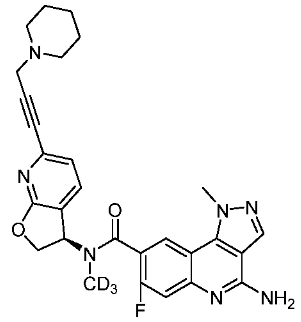
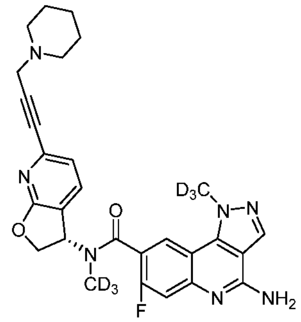
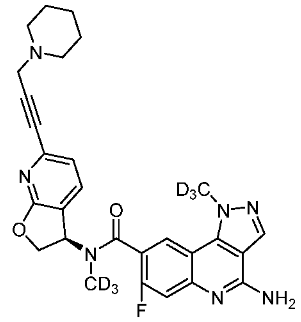

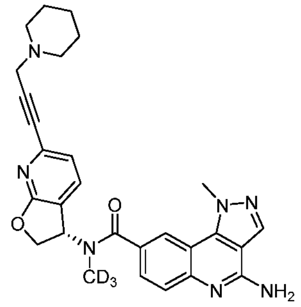
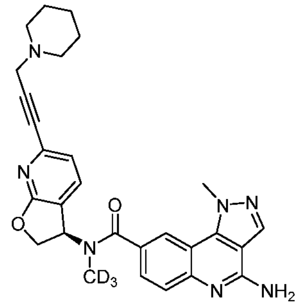
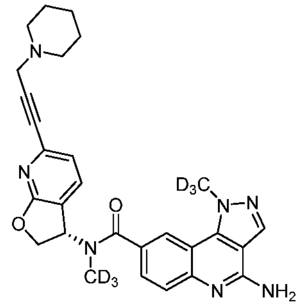
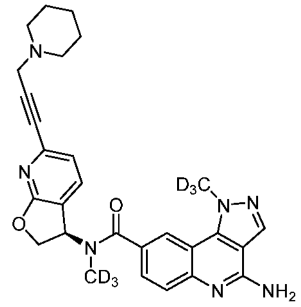

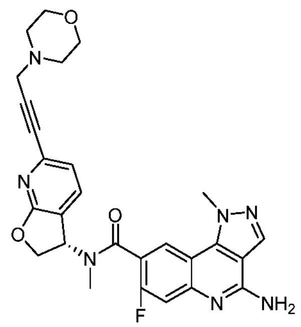
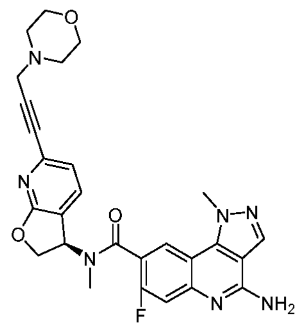
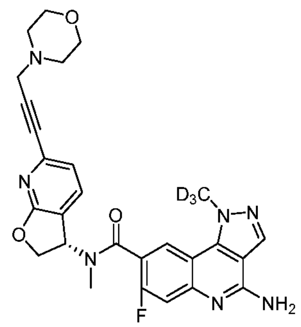
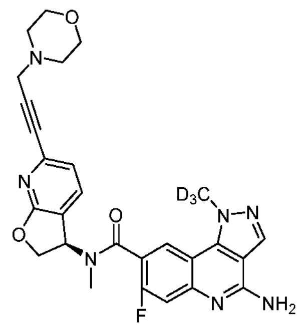

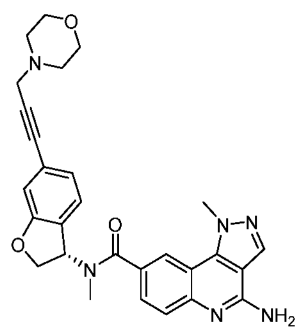
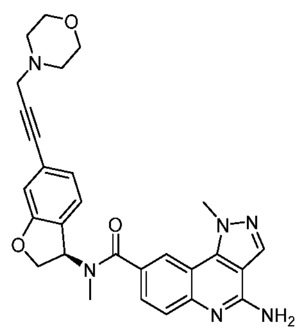
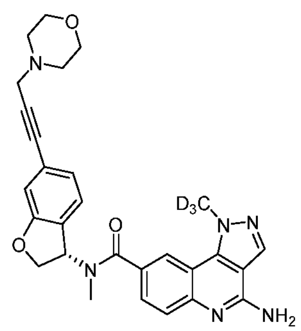
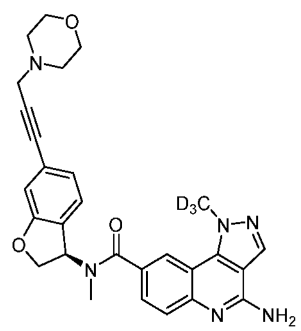

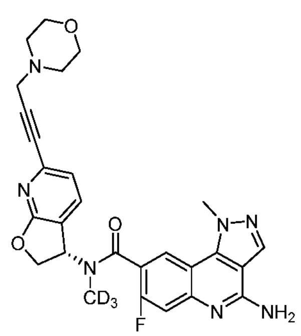
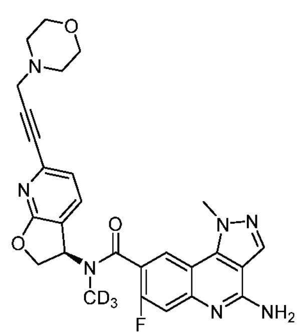
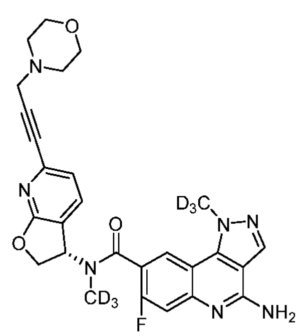
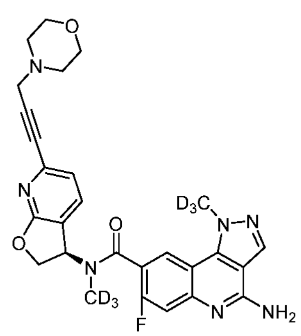

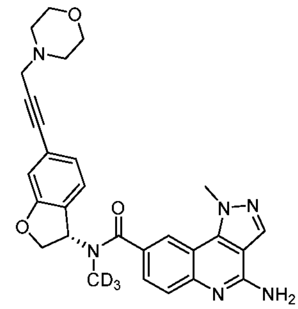 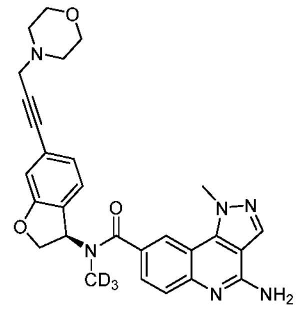 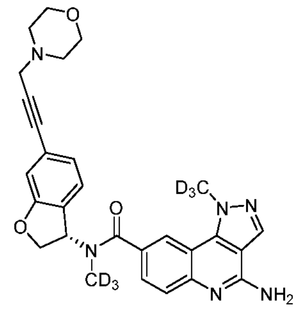 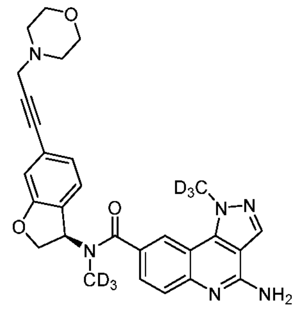

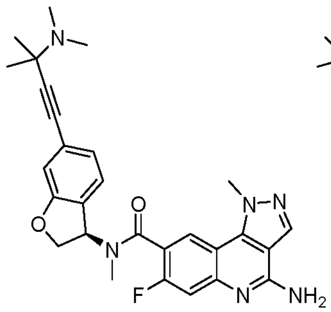 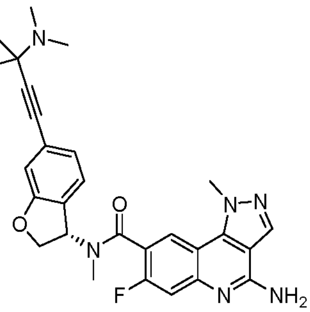.

In one example of the present disclosure, compound **1a** has a retention time of 2.214 min as analyzed by SFC detection conditions (chromatographic column: Chiralcel OD-3 150 × 4.6 mm I.D., 3 μm; mobile phase: phase A: carbon dioxide, phase B: ethanol (0.05% diethylamine); gradient: B%: 40%).

In one example of the present disclosure, compound **1b** has a retention time of 2.715 min as analyzed by SFC detection conditions (chromatographic column: Chiralcel OD-3 150 × 4.6 mm I.D., 3 μm; mobile phase: phase A: carbon dioxide, phase B: ethanol (0.05% diethylamine); gradient: B%: 40%).

In one example of the present disclosure, compound **2a** has a retention time of 1.797 min as analyzed by SFC detection conditions (chromatographic column: Chiralcel OJ-3 100 × 4.6 mm I.D., 3 μm; mobile phase: phase A: carbon dioxide, phase B: ethanol (0.05% diethylamine); gradient: B%: 40%).

In one example of the present disclosure, compound **2b** has a retention time of 3.929 min as analyzed by SFC detection conditions (chromatographic column: Chiralcel OJ-3 100 × 4.6 mm I.D., 3 μm; mobile phase: phase A: carbon dioxide, phase B: ethanol (0.05% diethylamine); gradient: B%: 40%).

In one example of the present disclosure, compound **3a** has a retention time of 5.283 min as analyzed by SFC detection conditions (chromatographic column: ChiralPak AD-3 150 × 4.6 mm I.D., 3 μm; mobile phase: phase A: carbon dioxide, phase B: ethanol (0.05% diethylamine); gradient: B%: 5% to 40% (0 to 4.5 min), 40% (4.5 to 6.5 min), 5% (6.5 to 8 min)); compound **3a** has a retention time of 5.471 min as analyzed by SFC detection conditions (chromatographic column: Chiralcel OD-3 150 × 4.6 mm I.D., 3 μm; mobile phase: phase A: carbon dioxide, phase B: methanol (0.05% diethylamine); gradient: 5% to 40% (0 to 2 min), 40% (2 to 4 min), 5% (4 to 6min)).

In one example of the present disclosure, compound **3b** has a retention time of 5.581 min as analyzed by SFC detection conditions (chromatographic column: ChiralPak AD-3 150 × 4.6 mm I.D., 3 μm; mobile phase: phase A: carbon dioxide, phase B: ethanol (0.05% diethylamine); gradient: B%: 5% to 40% (0 to 4.5 min), 40% (4.5 to 6.5 min), 5% (6.5 to 8 min)); compound **3b** has a retention time of 5.458 min as analyzed by SFC detection conditions (chromatographic column: Chiralcel OD-3 150 × 4.6 mm I.D., 3 μm; mobile phase: phase A: carbon dioxide, phase B: methanol (0.05% diethylamine); gradient: 5% to 40% (0 to 2 min), 40% (2 to 4 min), 5% (4 to 6min)).

In one example of the present disclosure, compound **3c** has a retention time of 2.029 min as analyzed by SFC detection conditions (chromatographic column: Chiralpak IC-3 100 × 4.6 mm I.D., 3 μm; mobile phase: phase A: carbon dioxide, phase B: ethanol (0.05% diethylamine); gradient: B%: 60%); compound **3c** has a retention time of 5.803 min as analyzed by SFC detection conditions (chromatographic column: Chiralcel OD-3 150 × 4.6 mm I.D., 3 μm; mobile phase: phase A: carbon dioxide, phase B: methanol (0.05% diethylamine); gradient: 5% to 40% (0 to 2 min), 40% (2 to 4 min), 5% (4 to 6min)).

In one example of the present disclosure, compound **3d** has a detection retention time of 2.714 min as analyzed by SFC detection conditions (chromatographic column: Chiralpak IC-3 100 × 4.6 mm I.D., 3 μm; mobile phase: phase A: carbon dioxide, phase B: ethanol (0.05% diethylamine); gradient: B%: 60%); compound **3d** has a retention time of 5.772 min as analyzed by SFC detection conditions (chromatographic column: Chiralcel OD-3 150 × 4.6 mm I.D., 3 μm; mobile phase: phase A: carbon dioxide, phase B: methanol (0.05% diethylamine); gradient: 5% to 40% (0 to 2 min), 40% (2 to 4 min), 5% (4 to 6min)).

In one example of the present disclosure, compound **4a** has a retention time of 2.298 min as analyzed by SFC detection conditions (chromatographic column: Chiralpak IC-3 100 × 4.6 mm I.D., 3 μm; mobile phase: phase A: carbon dioxide, phase B: ethanol (0.05% diethylamine); gradient: B%: 60%).

In one example of the present disclosure, compound **4b** has a retention time of 3.083 min as analyzed by SFC detection conditions (chromatographic column: Chiralpak IC-3 100 × 4.6 mm I.D., 3 μm; mobile phase: phase A: carbon dioxide, phase B: ethanol (0.05% diethylamine); gradient: B%: 60%).

In one example of the present disclosure, compound **5a** has a retention time of 3.058 min as analyzed by SFC detection conditions (chromatographic column: ChiralPak AD-3 50 × 4.6 mm I.D., 3 μm; mobile phase: phase A: carbon dioxide, phase B: ethanol (0.05% diethanolamine); gradient: B%: 40%).

In one example of the present disclosure, compound **5b** has a retention time of 3.649 min as analyzed by SFC detection conditions (chromatographic column: ChiralPak AD-3 50 × 4.6 mm I.D., 3 μm; mobile phase: phase A: carbon dioxide, phase B: ethanol (0.05% diethanolamine); gradient: B%: 40%).

In one example of the present disclosure, compound **6a** has a retention time of 2.347 min as analyzed by SFC detection conditions (chromatographic column: Chiralpak IG-3 50 × 4.6 mm I.D., 3 μm; mobile phase: phase A: carbon dioxide, phase B: isopropanol (0.05% diethylamine); gradient: B%: 5 to 40% (0 to 2 min), 40% (2 to 3.2 min), 5% (3.2 to 4 min)).

In one example of the present disclosure, compound **6b** has a retention time of 2.650 min as analyzed by SFC detection conditions (chromatographic column: Chiralpak IG-3 50 × 4.6 mm I.D., 3 μm; mobile phase: phase A: carbon dioxide, phase B: isopropanol (0.05% diethylamine); gradient: B%: 5 to 40% (0 to 2 min), 40% (2 to 3.2 min), 5% (3.2 to 4 min)).

In one example of the present disclosure, compound **7a** has a retention time of 2.008 min as analyzed by SFC detection conditions (chromatographic column: Chiralcel OJ-3 150 × 4.6 mm I.D., 3 μm; mobile phase: phase A: carbon dioxide, phase B: methanol (0.05% diethylamine); gradient: B%: 40%).

In one example of the present disclosure, compound **7b** has a retention time of 2.872 min as analyzed by SFC detection conditions (chromatographic column: Chiralcel OJ-3 150 × 4.6 mm I.D., 3 μm; mobile phase: phase A: carbon dioxide, phase B: methanol (0.05% diethylamine); gradient: B%: 40%).

In one example of the present disclosure, compound **8a** has a retention time of 1.152 min as analyzed by SFC detection conditions (chromatographic column: Chiralcel OD-3 150 × 4.6 mm I.D., 3 μm; mobile phase: phase A: carbon dioxide, phase B: ethanol (0.05% diethylamine); gradient: B%: 40%).

In one example of the present disclosure, compound **8b** has a retention time of 1.655 min as analyzed by SFC detection conditions (chromatographic column: Chiralcel OD-3 150 × 4.6 mm I.D., 3 μm; mobile phase: phase A: carbon dioxide, phase B: ethanol (0.05% diethylamine); gradient: B%: 40%).

In one example of the present disclosure, compound **9a** has a retention time of 3.426 min as analyzed by SFC detection conditions (chromatographic column: Chiralpak AS-3 150 × 4.6 mm I.D., 3 μm; mobile phase: phase A: carbon dioxide, phase B: ethanol (0.05% diethylamine); gradient: B%: 5% to 40% (5 to 4 min), 40% to 5% (4 to 4.2 min), 5% (4.2 to 6 min)).

In one example of the present disclosure, compound **9b** has a retention time of 3.698 min as analyzed by SFC detection conditions (chromatographic column: Chiralpak AS-3 150 × 4.6 mm I.D., 3 μm; mobile phase: phase A: carbon dioxide, phase B: ethanol (0.05% diethylamine); gradient: B%: 5% to 40% (5 to 4 min), 40% to 5% (4 to 4.2 min), 5% (4.2 to 6 min)).

In one example of the present disclosure, compound **9c** has a retention time of 2.281 min as analyzed by SFC detection conditions (chromatographic column: Chiralpak AD-3 150 × 4.6 mm I.D., 3 μm; mobile phase: phase A: carbon dioxide, phase B: isopropanol (0.05% diethylamine); gradient: B%: 40%).

In one example of the present disclosure, compound **9d** has a retention time of 2.599 min as analyzed by SFC detection conditions (chromatographic column: Chiralpak AD-3 150 × 4.6 mm I.D., 3 μm; mobile phase: phase A: carbon dioxide, phase B: isopropanol (0.05% diethylamine); gradient: B%: 40%).

The present disclosure also provides a use of the compound or the pharmaceutically acceptable salt thereof in the manufacture of a medicament for treating diseases related to PRMT5.

The present disclosure further provides the following synthesis methods:

Method 1 (intermediate):

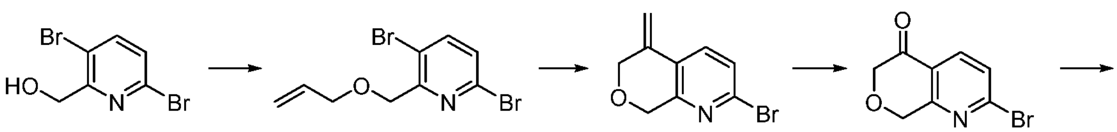

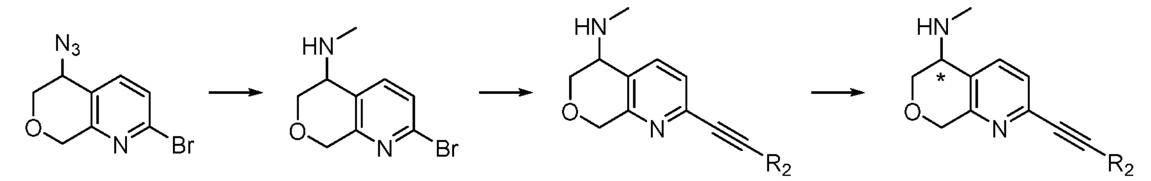

wherein $R_2$ is selected from H and $CF_3$;
the carbon atom with "*" is a chiral carbon atom, which exists in an (R) or (S) single enantiomer form or an (R) or (S) single enantiomer-rich form.

Method 2 (intermediate):

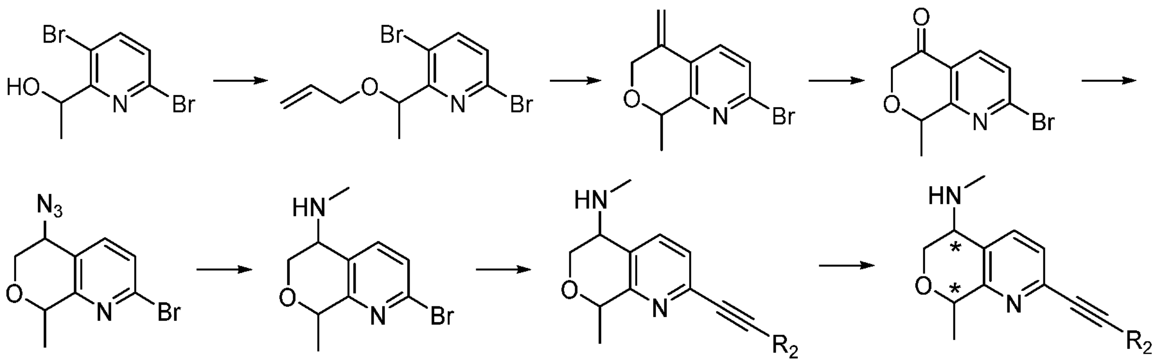

wherein $R_2$ is selected from H and $CF_3$;
the carbon atom with "*" is a chiral carbon atom, which exists in an (R) or (S) single enantiomer form or an (R) or (S) single enantiomer-rich form.

Method 3:

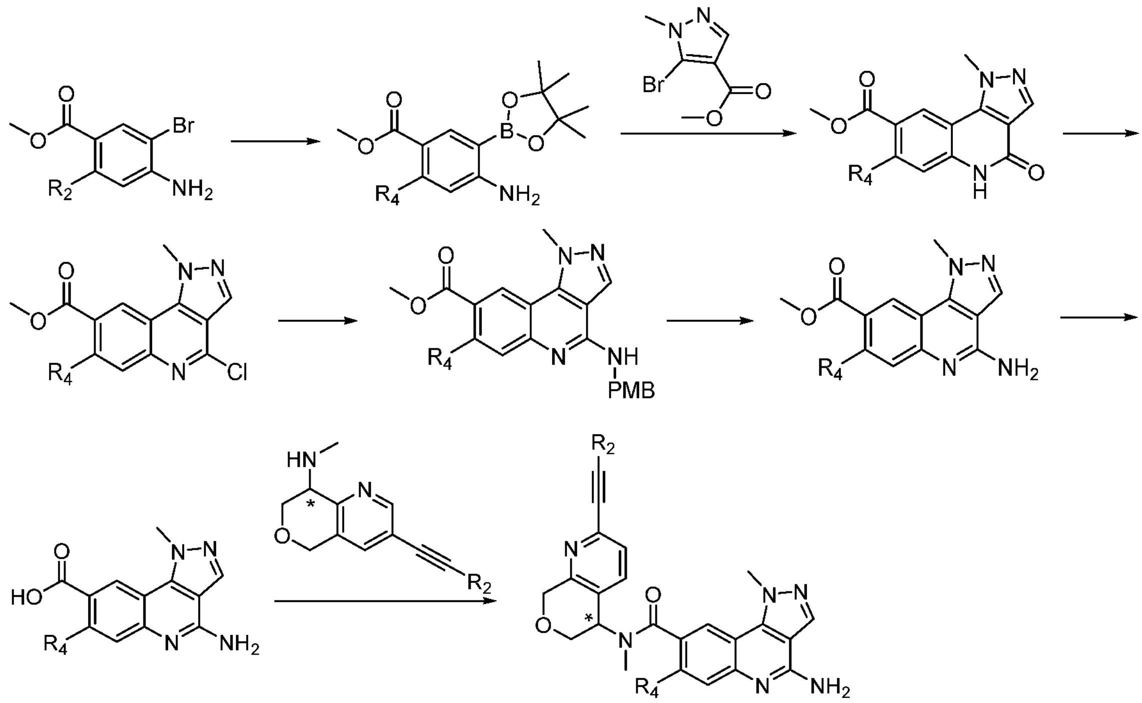

wherein $R_2$ is selected from H and $CF_3$;
$R_4$ is selected from H, F, Cl, Br, and $CH_3$;
the carbon atom with "*" is a chiral carbon atom, which exists in an (R) or (S) single enantiomer form or an (R) or (S) single enantiomer-rich form.

Method 4:

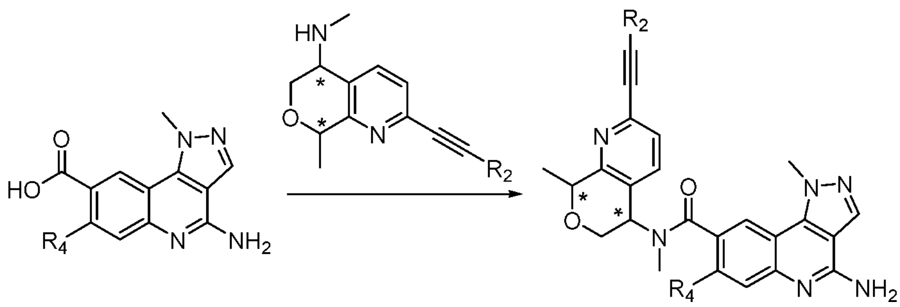

wherein $R_2$ is selected from H and $CF_3$;
$R_4$ is selected from H, F, Cl, Br, and $CH_3$;
the carbon atom with "*" is a chiral carbon atom, which exists in an (R) or (S) single enantiomer form or an (R) or (S) single enantiomer-rich form.

Method 5:

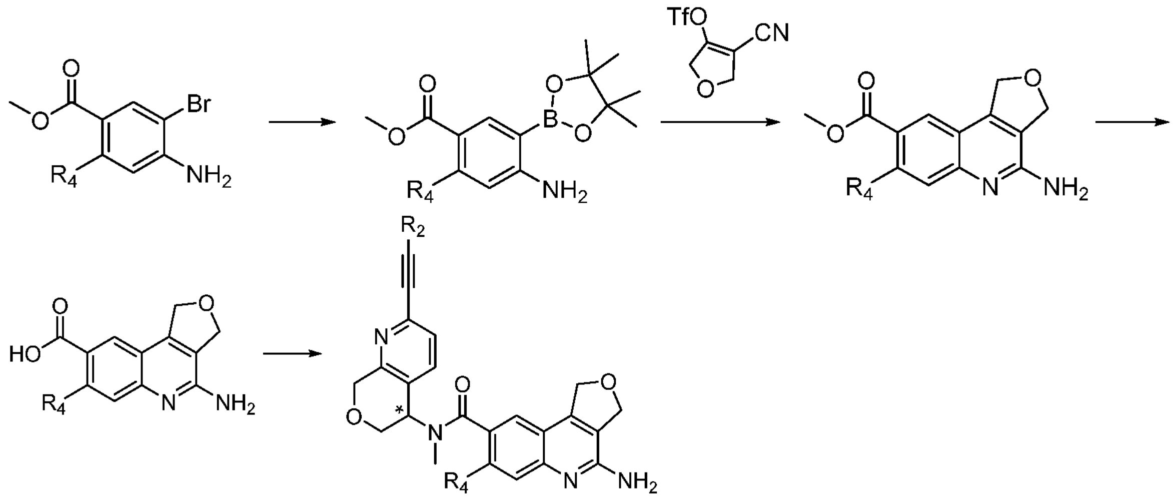


wherein $R_2$ is selected from H and $CF_3$;
$R_4$ is selected from H, F, Cl, Br, and $CH_3$;
the carbon atom with "*" is a chiral carbon atom, which exists in an (R) or (S) single enantiomer form or an (R) or (S) single enantiomer-rich form.

Method 6:

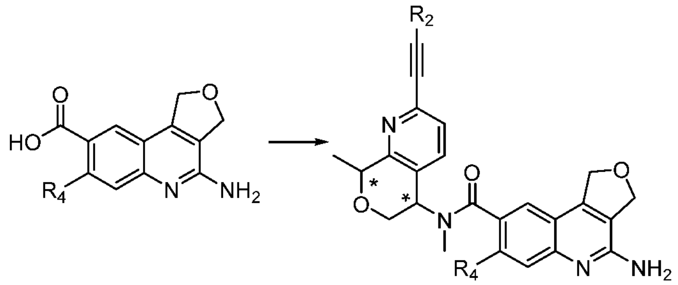

wherein $R_2$ is selected from H and $CF_3$;
$R_4$ is selected from H, F, Cl, Br, and $CH_3$;
the carbon atom with "*" is a chiral carbon atom, which exists in an (R) or (S) single enantiomer form or an (R) or (S) single enantiomer-rich form.

Method 7 (intermediate):

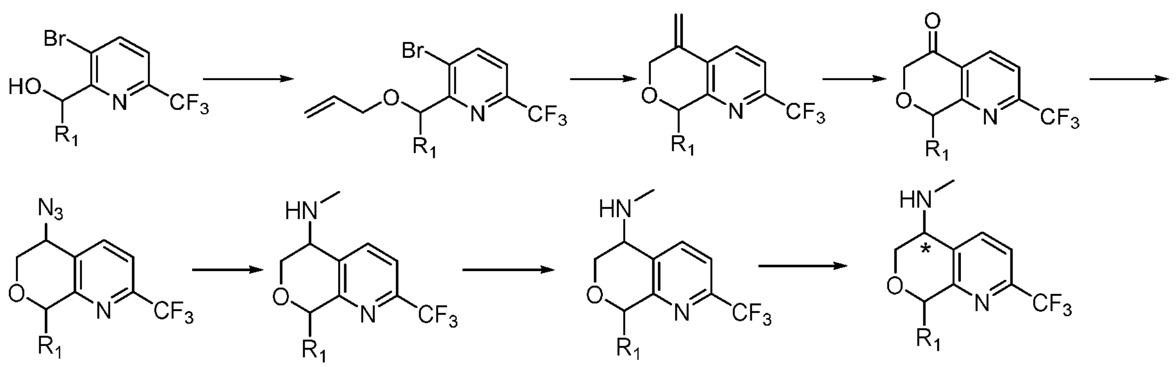

wherein $R_1$ is selected from H and $CH_3$;
the carbon atom with "*" is a chiral carbon atom, which exists in an (R) or (S) single enantiomer form or an (R) or (S) single enantiomer-rich form.

Method 8:

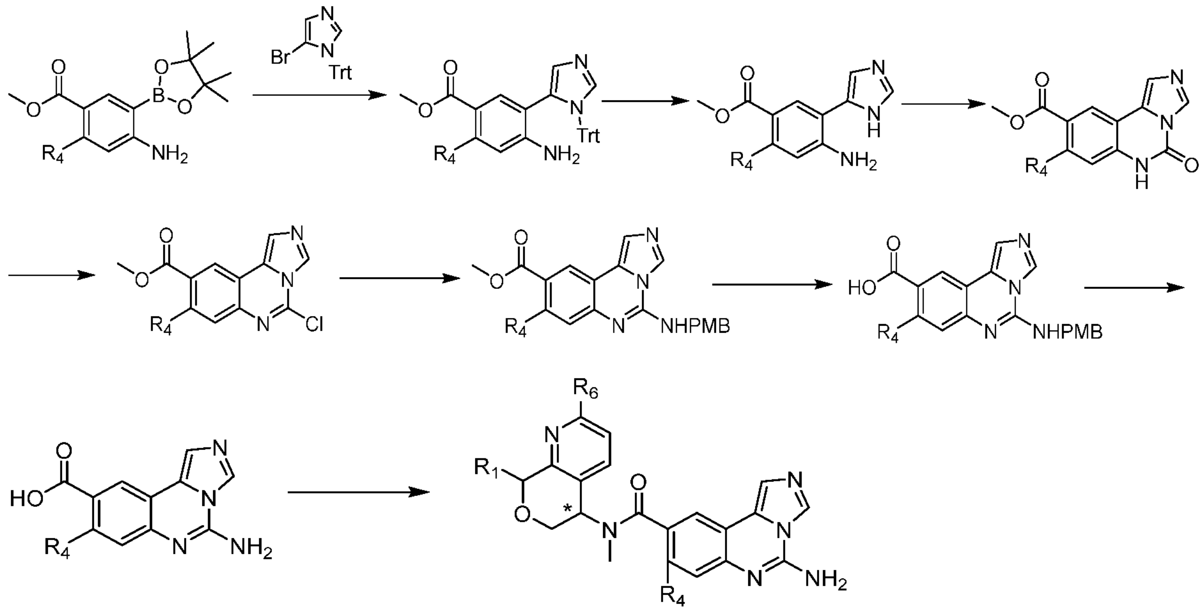

wherein

$R_1$ is selected from H and $CH_3$;
$R_6$ is selected from $CF_3$, and

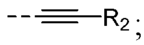

$R_4$ is selected from H, F, Cl, Br, and $CH_3$;
the carbon atom with "*" is a chiral carbon atom, which exists in an (R) or (S) single enantiomer form or an (R) or (S) single enantiomer-rich form.

Method 9:

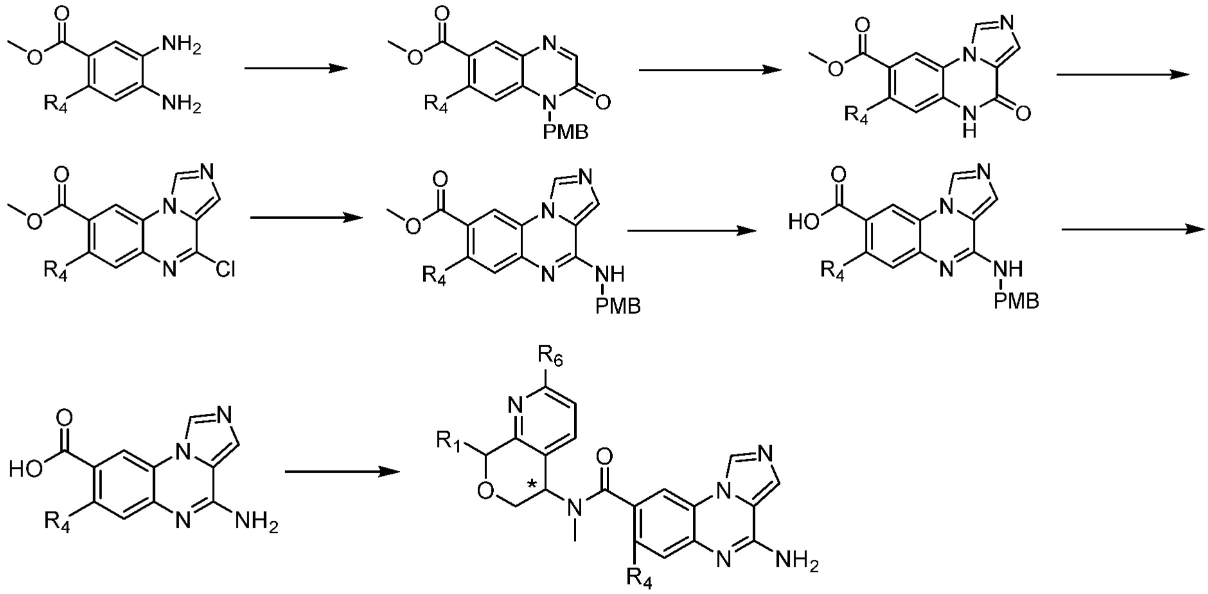

$R_1$ is selected from H and $CH_3$;
$R_6$ is selected from $CF_3$, and

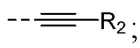

$R_4$ is selected from H, F, Cl, Br, and $CH_3$;
the carbon atom with "*" is a chiral carbon atom, which exists in an (R) or (S) single enantiomer form or an (R) or (S) single enantiomer-rich form.

Method 10:

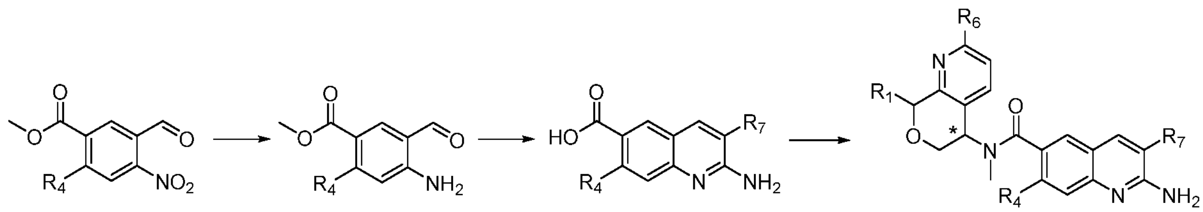

wherein $R_1$ is selected from H and $CH_3$;
$R_4$ is selected from H, F, Cl, Br, and $CH_3$;
$R_7$ is selected from $CH_3$, $CD_3$, and halogen;
the carbon atom with "*" is a chiral carbon atom, which exists in an (R) or (S) single enantiomer form or an (R) or (S) single enantiomer-rich form.

The present disclosure further provides the following test methods:

Test method 1: PRMT5 enzyme inhibitory activity assay

**Experimental purpose:** To test the inhibitory effect of small molecule compounds on PRMT5·MTA

**Experimental materials:** PRMT5/MEP50, peptide H4 (1-21), LANCE® Ultra Europium-anti-methyl-Histone H4 Arginine 3 (H4R3me) antibody, ULight streptavidin, LANCE detection buffer, SAM (S-adenosylmethionine), porcine skin collagen, and MTA (methylthioadenosine).

**Experimental methods:**

(1) Buffer preparation: Taking 10 mL as an example. 10 mM MTA: 10 mg of MTA was dissolved in 3296 μL of DMSO. The mixture was then aliquoted and stored at -80°C. The buffer was prepared on the day of the experiment, according to the schemes shown in Tables 1 and 2.

Table 1: Preparation scheme of buffer with MTA

| Component | Storage concentration | Final concentration | Addition volume |
|---|---|---|---|
| Bicine, pH 7.6 | 1000 mM | 20 mM | 200 μL |
| NaCl | 5000 mM | 25 mM | 50 μL |
| DTT | 1000 mM | 2 mM | 20 μL |
| Porcine skin collagen (gelatin) | 0.08% | 0.005% | 625 μL |
| MTA | 10 mM | 2.6 μM | 2.6 μL |
| Tween-20 | 1% | 0.01% | 100 μL |
| Deionized water | - | - | 9002.4 μL |

Table 2: Preparation scheme of buffer without MTA

| Component | Storage concentration | Final concentration | Addition volume |
|---|---|---|---|
| Bicine, pH 7.6 | 1000 mM | 20 mM | 200 μL |
| NaCl | 5000 mM | 25 mM | 50 μL |
| DTT | 1000 mM | 2 mM | 20 μL |
| Porcine skin collagen (gelatin) | 0.08% | 0.005% | 625 μL |
| Tween-20 | 1% | 0.01% | 100 μL |
| Deionized water | - | - | 9005.0 μL |

(2) Compound preparation

The compound was dissolved in DMSO to obtain a stock solution with a concentration of 10 mM. A gradient dilution was performed in a compound dilution plate to obtain four compound wells at the following concentrations: 1 mM, 37.037 μM, 1.3717 μM, and 0.0508 μM. These compounds at four concentrations were then transferred to a compound transfer plate with a transfer volume of 8 μL for each concentration. Additionally, DMSO was added to the empty wells of the compound transfer plate for later use. The Echo550 liquid handler was employed to perform serial dilutions and transfer the liquid to the experimental plate. Upon completion of the liquid transfer, the experimental plate was ready for use.

(3) Reaction

The enzyme solution and substrate mixture were prepared using the buffer, with PRMT5 at a concentration of 7.6 nM, peptide H4 (1-21) at a concentration of 0.32 μM, and SAM at a concentration of 2.6 μM.

5 μL of the PRMT5 solution per well was added to the compound wells and negative control wells of the experimental plate using an electronic multichannel pipette. An equal volume of buffer was added to the positive control wells. The experimental plate was centrifuged at 1000 rpm for one minute and then incubated at 25°C for 30 minutes in a temperature-controlled incubator. Subsequently, the substrate mixture was added to the positive control wells, negative control wells, and compound wells of the experimental plate using the same method. The plate was centrifuged and incubated at 25°C for 90 minutes.

(4) Detection and calculation

Principle: The experiment employed PerkinElmer's time-resolved fluorescence resonance energy transfer technology (LANCE® Ultra) for detection. During the reaction, two antibodies were added after PRMT methylated the substrate peptide H4 (1-21). The LANCE® Ultra Europium-anti-methyl-Histone H4 Arginine 3 (H4R3me) antibody acted as an energy donor and specifically bound to the methylation site on the peptide H4 (1-21), while ULight acted as an energy

acceptor and specifically bound to the biotin tag on the peptide H4 (1-21). When excited with a laser at a specific wavelength (the excitation wavelength in this experiment was 340 nm), the energy donor emitted light at a wavelength of 615 nm. If the spatial distance between the energy donor and the energy acceptor was close enough (i.e., both antibodies were simultaneously attached to the peptide H4 (1-21)), energy transfer occurred between the energy donor and the energy acceptor, causing the energy acceptor to emit light at a wavelength of 665 nm. The emitted lights at both 615 nm and 665 nm were detected using a plate reader, and the ratio of the 665 nm signal to the 615 nm signal was calculated. By plotting and calculation, the relevant parameters of the sample to be tested could be determined.

An antibody mixture was prepared using LANCE buffer, with a concentration of 4 nM for LANCE® Ultra Europium-anti-methyl-Histone H4 Arginine 3 (H4R3me) antibody and 53.3 nM for ULight. 10 μL of the detection solution per well was added to the positive control wells, negative control wells, and compound wells of the experimental plate using an electronic multichannel pipette. The plate was centrifuged and incubated at room temperature for 1 hour, and an Envision 2104 plate reader was used for reading. The inhibition rate of the compounds was calculated using interpolation. The inhibition rate was plotted using the four-parameter logistic curve and XLfit software to generate the inhibition curve of the compounds and calculate relevant parameters, including minimum inhibition rate, maximum inhibition rate, and $IC_{50}$. The formula for calculating the inhibition rate is follows:

$$\text{Single-well inhibition rate} = \left(1 - \frac{\text{Single-well signal value - Mean signal value of the positive control}}{\text{Mean signal value of the negative control - Mean signal value of the positive control}}\right) \times 100\%$$

## Technical effect

The compound of the present disclosure has a good binding effect with the PRMT5 MTA complex, exhibits significant inhibitory activity on the PRMT5 MTA enzyme, and exhibits significant anti-proliferative activity on LU99 cells; it has good inhibitory activity on MTAP-deficient HCT116 tumor cells, and weak inhibitory activity on wild-type HCT116 tumor cells, showing excellent selectivity; the *in vivo* PK results in mice show that the compound of the present disclosure exhibits a long half-life, a low clearance rate, a high drug exposure and oral bioavailability, demonstrating excellent *in vivo* pharmacokinetic properties; the compound of the present disclosure shows moderate metabolism in the human liver microsome test and has good metabolic stability; the compound of the present disclosure has a moderate ratio of free drug concentrations in the plasma of different species, good *in vitro* MDCKII-MDR1 monolayer cell permeability, weak inhibition of hERG potassium current, low risk of cardiotoxicity, and good drugability; the compound of the present disclosure also shows excellent *in vivo* anti-tumor efficacy and good maintenance of body weight in mice after administration.

## Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. When the compound of the present disclosure contains a relatively basic functional group, an acid addition salt can be obtained by contacting the compound with a sufficient amount of acid in a pure solution or a suitable inert solvent.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereoisomers, (*D*)-isomers, (*L*)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the

scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 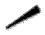 ) and a wedged dashed bond ( 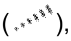 ), and the relative configuration of a stereogenic center is represented by a straight solid bond ( 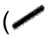 ) and a straight dashed bond ( 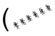 ), a wave line ( 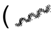 ) is used to represent a wedged solid bond ( 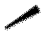 ) or a wedged dashed bond ( 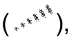 ), or the wave line ( 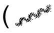 ) is used to represent a straight solid bond ( 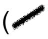 )or a straight dashed bond ( 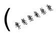 ).

Unless otherwise specified, the carbon atom with "*" is a chiral carbon atom, which exists in an (R) or (S) single enantiomer form or an (R) or (S) single enantiomer-rich form. For example,

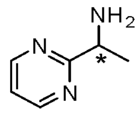

indicates that either

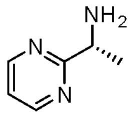 or 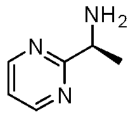 ,

or exists in an (R) or (S) single enantiomer-rich form.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

Unless otherwise specified, when a double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound and its substituent are represented by

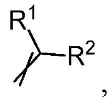

this refers to the (*Z*) isomer, (*E*) isomer or a mixture of two isomers of the compound.

The compound of the present disclosure may contain an unnatural proportion of atomic isotope at one or more than one atom that constitutes the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, *etc.* All isotopic variations of the compound of the present disclosure, whether radioactive or not, are encompassed within the scope of the present disclosure.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (*i.e.,* =O), it means two hydrogen atoms are substituted.

The term "optional" or "optionally" means that the subsequent event or condition may occur but not requisite, that the term includes the instance in which the event or condition occurs and the instance in which the event or condition does not occur.

The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to -2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X, the structure of A-X is actually A.

When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring. When the enumerative linking group does not indicate the direction for linking, the direction for linking is arbitrary, for example, the linking group L contained in

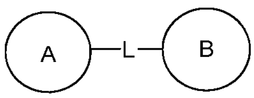

is -M-W-, then -M-W- can link ring A and ring B to form

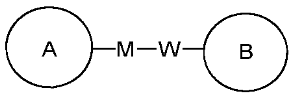

in the direction same as left-to-right reading order, and form

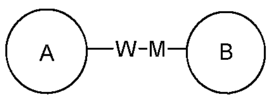

in the direction contrary to left-to-right reading order. A combination of the linking groups, substituents and/or variables thereof is allowed only when such combination can result in a stable compound.

Unless otherwise specified, when a group has one or more than one linkable site, any one or more than one site of the group can be linked to other groups through chemical bonds. When the linking site of the chemical bond is not positioned, and there is an H atom at the linkable site, then the number of H atoms at the site will decrease correspondingly

with the number of the chemical bonds linking thereto so as to meet the corresponding valence. The chemical bond between the site and other groups can be represented by a straight solid bond (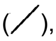), a straight dashed bond (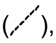), or a wavy line (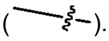). For example, the straight solid bond in $-OCH_3$ means that it is linked to other groups through the oxygen atom in the group; the straight dashed bond in

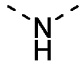

means that it is linked to other groups through the two ends of the nitrogen atom in the group; the wave lines in

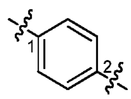

means that the phenyl group is linked to other groups through carbon atoms at position 1 and position 2;

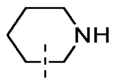

means that it can be linked to other groups through any linkable sites on the piperidinyl by one chemical bond, including at least four types of linkage, including

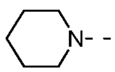 , 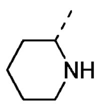 , 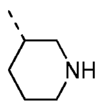 ,

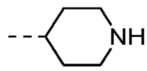 .

Even though the H atom is drawn on the -N-,

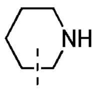

still includes the linkage of

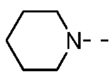 ,

merely when one chemical bond was connected, the H of this site will be reduced by one to the corresponding monovalent piperidinyl.

Unless otherwise specified, the term "halogen element" or "halogen" by itself or as part of another substituent refers to fluorine, chlorine, bromine, or iodine atom.

Unless otherwise specified, the term "$C_{1-3}$ alkyl" by itself or in combination with other terms refers to a linear or branched saturated hydrocarbon group consisting of 1 to 3 carbon atoms. The $C_{1-3}$ alkyl includes $C_{1-2}$ and $C_{2-3}$ alkyl, *etc.;* it can be monovalent (such as methyl), divalent (such as methylene), or multivalent (such as methine). Examples of $C_{1--3}$ alkyl include, but are not limited to, methyl (Me), ethyl (Et), propyl (including *n*-propyl and isopropyl), *etc.*

Unless otherwise specified, "$C_{2-4}$ alkenyl" by itself or in combination with other terms is used to mean a linear or branched hydrocarbon group consisting of 2 to 4 carbon atoms containing at least one carbon-carbon double bond, wherein the carbon-carbon double bond can be located at any position within the group. The $C_{2-4}$ alkenyl includes $C_{2-3}$, $C_4$, $C_3$, $C_2$ alkenyl, *etc.;* the $C_{2-4}$ alkenyl can be monovalent, divalent, or multivalent. Examples of $C_{2-4}$ alkenyl include, but are not limited to, vinyl, propenyl, butenyl, butadienyl, *etc.*

Unless otherwise specified, "$C_{2-4}$ alkynyl" by itself or in combination with other terms is used to mean a linear or

branched hydrocarbon group consisting of 2 to 4 carbon atoms containing at least one carbon-carbon triple bond, wherein the carbon-carbon triple bond can be located at any position within the group. The $C_{2-4}$ alkynyl includes $C_{2-3}$, $C_4$, $C_3$, $C_2$ alkynyl, *etc.* It can be monovalent, divalent, or multivalent. Examples of $C_{2-4}$ alkynyl include, but are not limited to, ethynyl, propynyl, butynyl, *etc.*

Unless otherwise specified, the term "$C_{1-3}$ alkoxy" by itself or in combination with other terms refers to an alkyl consisting of 1 to 3 carbon atoms that are connected to the rest of the molecule through an oxygen atom. The $C_{1-3}$ alkoxy includes $C_{1-2}$, $C_{2-3}$, $C_3$, $C_2$ alkoxy, *etc.* It can be monovalent, divalent, or multivalent. Examples of $C_{1--3}$ alkoxy include, but are not limited to, methoxy, ethoxy, propoxy (including *n*-propoxy and isopropoxy), *etc.*

Unless otherwise specified, the term "$C_{1-3}$ alkylamino" by itself or in combination with other terms refers to an alkyl consisting of 1 to 3 carbon atoms that are connected to the rest of the molecule through a nitrogen atom. The $C_{1-3}$ alkylamino includes $C_{1-2}$, $C_3$, $C_2$ alkylamino, etc. It can be monovalent, divalent, or multivalent. Examples of $C_{1-3}$ alkylamino include, but are not limited to, $-NHCH_3$, $-N(CH_3)_2$, $-NHCH_2CH_3$, $-N(CH_3)CH_2CH_3$, $-NHCH_2CH_2CH_3$, $-NHCH_2(CH_3)_2$, etc.

Unless otherwise specified, the term "$C_{3-6}$ cycloalkyl" by itself or in combination with other terms refers to a saturated cyclic hydrocarbon group consisting of 3 to 6 carbon atoms monocyclic. The $C_{3-6}$ cycloalkyl includes $C_{3-5}$, $C_{4-5}$, $C_{5-6}$ cycloalkyl, *etc.;* it can be monovalent, divalent, or multivalent. Examples of $C_{3-6}$ cycloalkyl include, but are not limited to, cyclopropyl, cyclobutyl, cyclopentyl, cyclohexyl, etc.

Unless otherwise specified, the term "$C_{4-6}$ cycloalkenyl" by itself or in combination with other terms refers to a partially unsaturated monocyclic hydrocarbon group consisting of 4 to 6 carbon atoms containing at least one carbon-carbon double bond. The $C_{4-6}$ cycloalkenyl includes $C_{4-5}$ or $C_{5-6}$ cycloalkenyl, etc.; it can be monovalent, divalent, or multivalent. Examples of $C_{4-6}$ cycloalkenyl include, but are not limited to, cyclobutenyl, cyclopentenyl, cyclopentadienyl, cyclohexenyl, cyclohexadienyl, etc.

Unless otherwise specified, the term "4- to 6-membered heterocycloalkyl" by itself or in combination with other terms refers to a saturated cyclic group consisting of 4 to 6 ring atoms, wherein 1, 2, 3, or 4 ring atoms are heteroatoms independently selected from O, S, and N, and the rest are carbon atoms, wherein carbon atoms are optionally oxidized (i.e., C(O)), nitrogen atoms are optionally quaternized, and nitrogen and sulfur heteroatoms can be optionally oxidized (i.e., NO and $S(O)_p$, p is 1 or 2). It is a monocyclic system. In addition, with regard to the "4- to 6-membered heterocycloalkyl", a heteroatom may occupy the connection position of the heterocycloalkyl with the rest of the molecule. The 4- to 6-membered heterocycloalkyl includes 5- to 6-membered, 4-membered, 5-membered, 6-membered heterocycloalkyl, etc. It can be monovalent, divalent, or multivalent. Examples of 4- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, hexahydropyridazinyl, etc.

Unless otherwise specified, the term "5- to 6-membered heterocycloalkenyl" by itself or in combination with other terms refers to a partially unsaturated monocyclic group consisting of 5 to 6 ring atoms containing at least one carbon-carbon double bond, wherein 1, 2, 3, or 4 ring atoms are heteroatoms independently selected from O, S, and N, and the rest are carbon atoms, wherein carbon atoms are optionally oxidized (i.e., C(O)), nitrogen atoms are optionally quaternized, and nitrogen and sulfur heteroatoms can be optionally oxidized (i.e., NO and $S(O)_p$, p is 1 or 2). In addition, with regard to the "5- to 6-membered heterocycloalkenyl", a heteroatom may occupy the connection position of the heterocycloalkenyl with the rest of the molecule. The 5- to 6-membered heterocycloalkenyl includes 5-membered and 6-membered heterocycloalkenyl. It can be monovalent, divalent, or multivalent. Examples of 5- to 6-membered heterocycloalkenyl include, but are not limited to

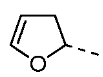, 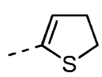, 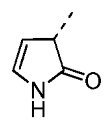,

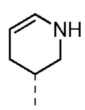, or 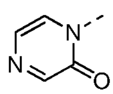.

Unless otherwise specified, the terms "5- to 6-membered heteroaryl ring" and "5- to 6- membered heteroaryl" are used interchangeably. The term "5- to 6-membered heteroaryl" refers to a monocyclic group consisting of 5 to 6 ring atoms with a conjugated $\pi$-electron system, in which 1, 2, 3, or 4 ring atoms are heteroatoms independently selected from O, S,

and N, and the rest are carbon atoms, Here, the nitrogen atom is optionally quaternized, and nitrogen and sulfur heteroatoms can be optionally oxidized (i.e., NO and $S(O)_p$, wherein p is 1 or 2). The 5- to 6-membered heteroaryl can be linked to the rest of the molecule through a heteroatom or a carbon atom. It can be monovalent, divalent, or multivalent. The 5- to 6-membered heteroaryl includes 5-membered and 6-membered heteroaryl. Examples of the 5- to 6-membered heteroaryl include, but are not limited to, pyrrolyl (including *N*-pyrrolyl, 2-pyrrolyl, and 3-pyrrolyl, etc.), pyrazolyl (including 2-pyrazolyl and 3-pyrazolyl, etc.), imidazolyl (including *N*-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (including 1*H*-1,2,3-triazolyl, 2*H*-1,2,3-triazolyl, 1*H*-1,2,4-triazolyl, and 4*H*-1,2,4-triazolyl, etc.), tetrazolyl, isoxazolyl (including 3-isoxazolyl, 4-isoxazolyl, and 5-isoxazolyl, etc.), thiazolyl (including 2-thiazolyl, 4-thiazolyl, and 5-thiazolyl, etc.), furyl (including 2-furyl and 3-furyl, etc.), thienyl (including 2-thienyll and 3-thienyl, etc.), pyridyl (including 2-pyridyl, 3-pyridyl, and 4-pyridyl, etc.), pyrazinyl, pyrimidinyl (including 2-pyrimidinyl, 4-pyrimidinyl, etc.).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The solvents used in the present disclosure are commercially available. The compounds of the present disclosure are named according to the conventional naming principles in the art or by ChemDraw® software, and the commercially available compounds use the supplier catalog names.

BRIEF DESCRIPTION OF THE DRAWINGS

FIG. 1. Diagram of the binding mode of compound A with PRMT5 MTA complex.
FIG. 2. Diagram of the binding mode of compound B with PRMT5 MTA complex.
FIG. 3. Diagram of the binding mode of compound C with PRMT5 MTA complex.
FIG. 4. Diagram of the binding mode of compound D with PRMT5 MTA complex.
FIG. 5. Diagram of the binding mode of compound E with PRMT5 MTA complex.
FIG. 6. Diagram of the binding mode of compound F with PRMT5 MTA complex.
FIG. 7. Diagram of the binding mode of compound G with PRMT5 MTA complex.
FIG. 8. Diagram of the binding mode of compound H with PRMT5 MTA complex.
FIG. 9. Body weight change curve of mice in an LU99 cell subcutaneous xenograft tumor model (compound **1a**).
FIG. 10. Body weight change curve of mice in the LU99 cell subcutaneous xenograft tumor model (compounds **3d and 4b**).
FIG. 11. Average tumor volume at different time points in the LU99 cell subcutaneous xenograft tumor model (compound **1a**).
FIG. 12. Average tumor volume at different time points in the LU99 cell subcutaneous xenograft tumor model (compounds **3d and 4b**).

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the specific examples of the present disclosure without departing from the spirit and scope of the present disclosure.

**Computing example 1**

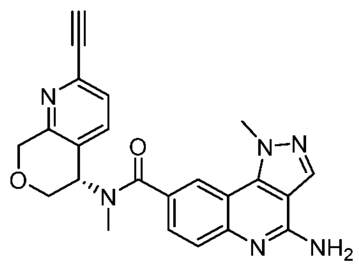

Compound A

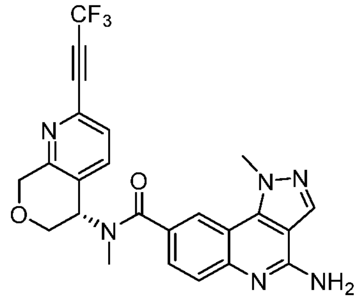

Compound B

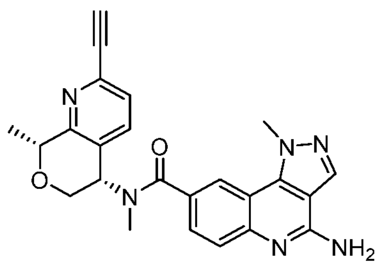

Compound C

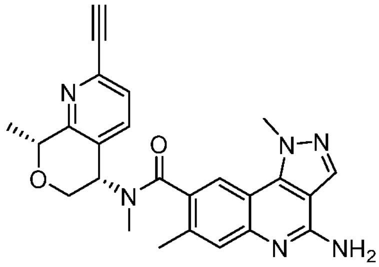

Compound D

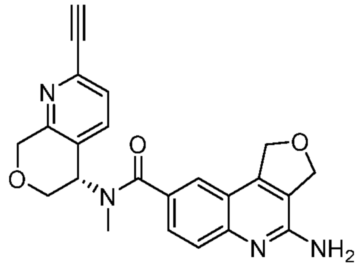

Compound E

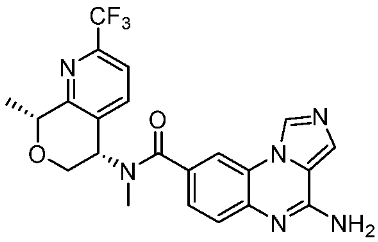

Compound F

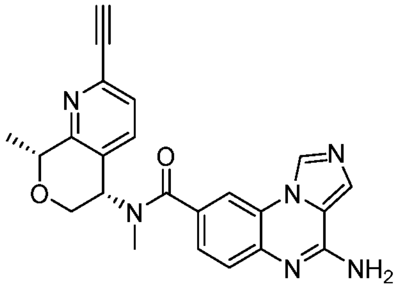

Compound G

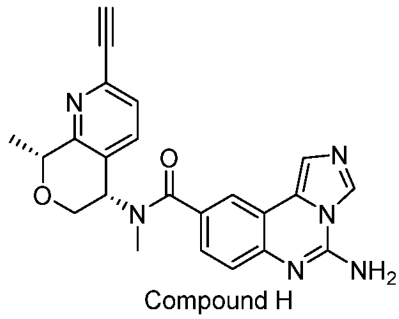

Compound H

The molecular docking process was conducted by using the Induced Fit Docking[1-2] default settings in Maestro (Schrödinger version 2022-1). The crystal structure of PRMT5 in the PDB database (PDB ID: 7S1S) was selected as the docking template. To prepare the protein, hydrogen atoms were added using the Protein Preparation Wizard module in Maestro[2], and energy minimization was performed using the OPLS4 force field. For ligand preparation, the three-dimensional structure of the molecule was generated using LigPrep[3], and energy minimization was performed using the OPLS4 force field. A box was automatically generated centered on Trp579 in 7S1S, in which the example compounds were placed during the molecular docking process. The interactions between the protein and the example compounds were analyzed, and then reasonable docking conformations were selected and saved based on the calculated IFD scores and binding modes. The binding modes of compounds A to H are as shown in FIGS. 1 to 8.

[1] Glide, Schrödinger, LLC, New York, NY, 2021.
[2] Prime, Schrödinger, LLC, New York, NY, 2021.
[3] Maestro, Schrödinger, LLC, New York, NY, 2022.
[4] LigPrep, Schrödinger, LLC, New York, NY, 2022.

Conclusion: The series of compounds of the present disclosure exhibit good binding to PRMT5 ·MTA complex.

**Reference Example 1 Synthesis of Intermediate A**

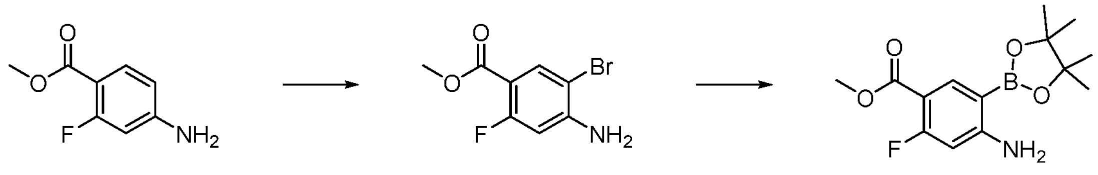

A-1 A-2 A-3

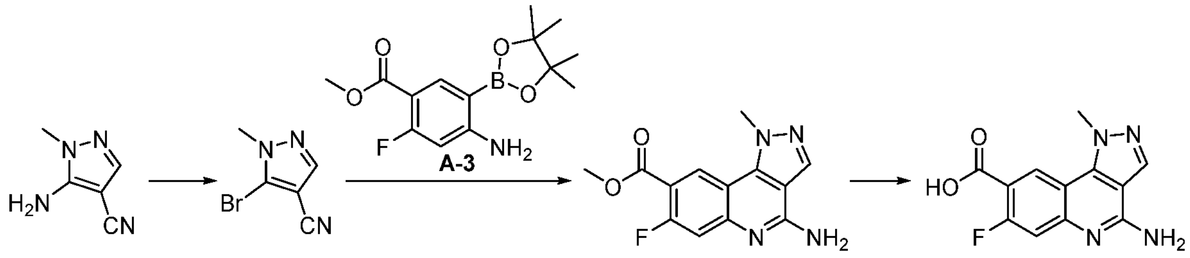

A-3

A-4 A-5 A-6 A

Step 1

At 0°C, N-bromosuccinimide (21.04 g, 118.24 mmol) was slowly added to a solution of compound **A-1** (20 g, 118.24 mmol) in acetonitrile (200 mL). The mixture was stirred at room temperature (25°C) for 16 hours. The reaction mixture was added with water (100 mL) and extracted with ethyl acetate (100 mL * 3). The organic phases were combined, washed with saturated sodium chloride aqueous solution (50 mL * 3), dried over anhydrous sodium sulfate, filtered, and then the filtrate was concentrated under reduced pressure. The crude product was subjected to column chromatography (silica gel, petroleum ether: ethyl acetate = 3:1) to obtain compound **A-2.** MS: m/z 248.0/250.0 $[M+H]^+$.

Step 2

At 0°C, potassium acetate (5.93 g, 60.47 mmol) and [1,1'-bis(diphenylphosphino)ferrocene]dichloropalladium(II) (737.47 mg, 1.01 mmol) were added to a solution of compound **A-2** (5.00 g, 20.16 mmol) and bis(pinacolato)diboron (6.14 g, 24.19 mmol) in dioxane (50 mL). The mixture was stirred at 100°C for 20 hours. The mixture was filtered and the filtrate was concentrated under reduced pressure. The crude product was subjected to column chromatography (silica gel, petroleum ether: ethyl acetate = 20:1) to obtain compound **A-3.** MS: *m*/*z* 296.2 $[M+H]^+$.

Step 3

At 0-5°C, a solution of hydrogen bromide (24.84 g, 122.82 mmol, 40% aqueous solution) and sodium nitrite (847.47 mg, 12.28 mmol) in water (15 mL) was slowly added to a solution of compound **A-4** (1 g, 8.19 mmol) in water (15 mL), and then cuprous bromide (1.88 g, 13.10 mmol) was added to the mixture. After the addition, the reaction mixture was stirred at 0-5°C for 3 hours, and then naturally warmed to 25°C and stirred for another 18 hours. The reaction mixture was cooled to 0-5°C, ice water (60 mL) was added to the reaction mixture and stirred at 0-5°C for 1 hour, and then the reaction mixture was filtered. The filter cake was washed with ice water (50 mL), and the filtrate was extracted with dichloromethane (50 mL * 3). The organic phases were combined and washed with saturated sodium chloride aqueous solution (30 mL * 3). The organic phase and the filter cake were combined and concentrated under reduced pressure to obtain compound **A-5,** which was directly used in the next step. MS: *m*/*z* 186.0/187.9 $[M+H]^+$.

Step 4

Potassium phosphate (667.55 mg, 4.83 mmol) and bis[di-tert-butyl-(4-dimethylaminophenyl)phosphine]dichloropalladium(II) (761.33 mg, 1.08 mmol) were added to a solution of compound **A-5** (1 g, 5.38 mmol) and compound **A-3** (1.59 g, 5.38 mmol) in dioxane (16 mL) and water (4 mL). The mixture was stirred at 90°C in an oil bath for 6 hours. The reaction mixture was filtered and the filtrate was concentrated under reduced pressure. The crude product was purified by column chromatography (silica gel, dichloromethane: methanol = 10:1) to obtain compound **A-6.** MS: m/z 275.0 $[M+H]^+$.

Step 5

Lithium hydroxide monohydrate (156.06 mg, 3.72 mmol) was added to a solution of compound **A-6** (510 mg, 1.86 mmol) in water (5 mL), methanol (5 mL) and tetrahydrofuran (5 mL). The mixture was stirred at room temperature (25°C) for

19 hours. 2 mol/L dilute hydrochloric acid was added to the reaction mixture to adjust the pH to 5-6. The mixture was stirred for 5 minutes, filtered, and the filter cake was washed with water (10 mL * 3) and then dried under reduced pressure. Compound **A** was obtained. MS: m/z 261.0 $[M+H]^+$. $^1H$ NMR (400 MHz, DMSO-$d_6$) δ = 8.76-8.69 (m, 1H), 8.27 (s, 1H), 7.50 (br s, 2H), 7.28-7.23 (m, 1H), 4.38 (s, 3H).

**Example 1**

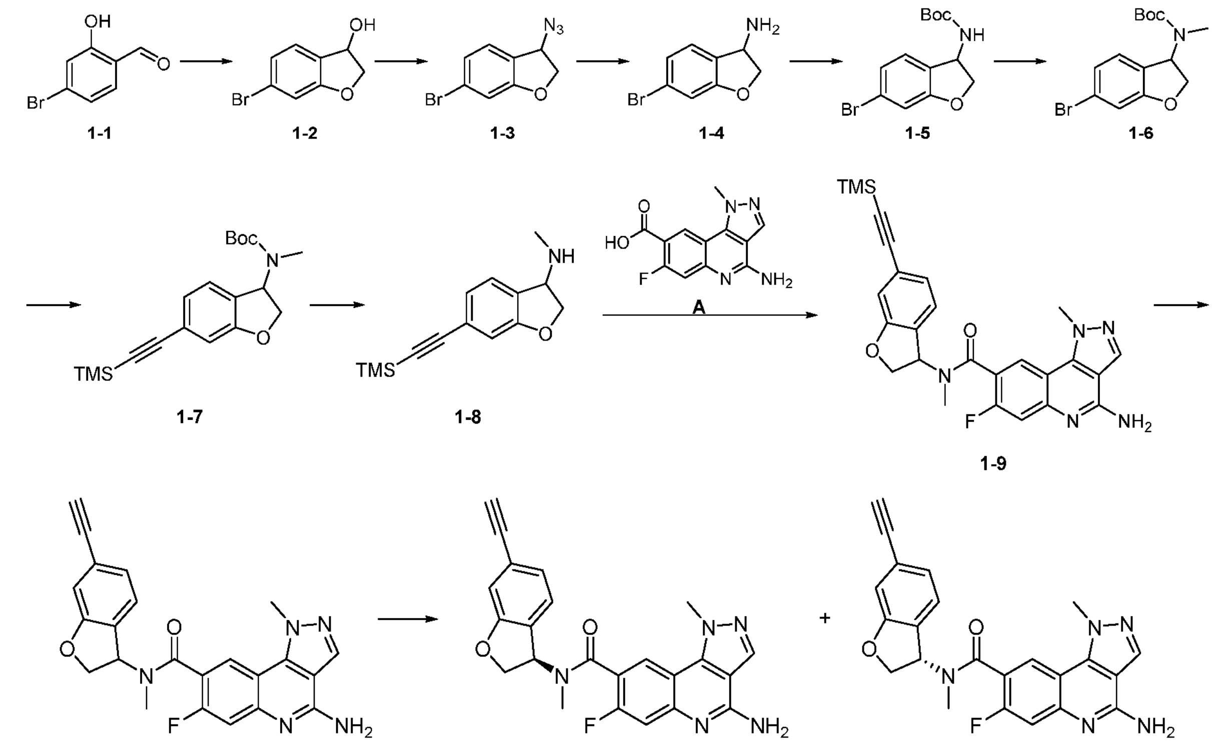

1-1 1-2 1-3 1-4 1-5 1-6

1-7 1-8 A 1-9

1 1a or 1b + 1a or 1b

Step 1

Compound **1-1** (4.5 g, 22.39 mmol) was dissolved in dimethyl sulfoxide (60 mL), then trimethylsulfoxonium iodide (5.91 g, 26.86 mmol) was added, and the mixture was cooled to 0°C. Potassium tert-butoxide (3.01 g, 26.86 mmol) was slowly added in batches. The reaction mixture was slowly warmed to 25°C under a nitrogen atmosphere and stirred for 2 hours. The reaction mixture was quenched by adding water (100 mL) and extracted with ethyl acetate (100 mL × 3). The organic phases were combined, washed with saturated brine (100 mL × 2), dried over anhydrous sodium sulfate, and filtered. The filtrate was concentrated under reduced pressure to obtain a crude product, which was purified by flash silica gel column (ethyl acetate/petroleum ether = 0 to 25%). Compound **1-2** was obtained. $^1H$ NMR (400 MHz, $CDCl_3$) δ ppm 7.28 (d, *J*=5.77 Hz, 1H), 7.09 (dd, *J*=7.91, 1.63 Hz, 1H), 7.06 (d, *J*=1.25 Hz, 1H), 5.33 (dd, *J*=6.53, 2.51 Hz, 1H), 4.55-4.61 (m, 1H), 4.44-4.49 (m, 1 H).

Step 2

Compound **1-2** (1.7 g, 7.91 mmol) was dissolved in toluene (27 mL). Under a nitrogen atmosphere at 0°C, diphenylphosphoryl azide (2.39 g, 8.70 mmol) was slowly added dropwise, and then a solution of 1.8-diazabicyclo[5.4.0]undec-7-ene (1.32 g, 8.70 mmol) in toluene (1 mL) was slowly added dropwise. The reaction mixture was slowly warmed to 25°C under a nitrogen atmosphere and stirred for 3.5 hours. The reaction mixture was diluted with ethyl acetate (80 mL). The organic phase was washed with saturated brine (40 mL × 2), dried over anhydrous sodium sulfate, filtered, and the filtrate was concentrated under reduced pressure to obtain a crude product, which was purified by flash silica gel column (ethyl acetate/petroleum ether = 0 to 2%). Compound **1-3** was obtained. $^1H$ NMR (400 MHz, DMSO-$d_6$) δ ppm 7.46 (d, *J*=8.03 Hz, 1H), 7.22 (d, *J*=1.51 Hz, 1H), 7.18 (dd, *J*=7.91, 1.63 Hz, 1H), 5.34 (dd, *J*=7.03, 2.26 Hz, 1H), 4.57-4.66 (m, 1H), 4.46-4.55 (m, 1 H).

Step 3

Compound **1-3** (1.03 g, 4.29 mmol) was dissolved in tetrahydrofuran (26 mL), then triphenylphosphine (1.69 g, 6.44 mmol) was added, and the reaction mixture was stirred at 25°C for 1 hour under a nitrogen atmosphere. A solution of potassium hydroxide (601.82 mg, 10.73 mmol) in water (6.5 mL) was added dropwise, and the reaction mixture was stirred at 25°C for 12 hours. The reaction mixture was diluted with ethyl acetate (60 mL), washed with saturated brine (30 mL × 2), dried over anhydrous sodium sulfate, filtered, and the filtrate was concentrated under reduced pressure to obtain a crude product. The crude product was purified by flash silica gel column (methanol/ethyl acetate = 0 to 3%) to obtain a crude product **1-4,** which was used directly in the next step.

Step 4

The crude product **1-4** (918 mg, 4.29 mmol) was dissolved in dichloromethane (15 mL) and cooled to 0°C. Triethylamine (867.91 mg, 8.58 mmol) was added under a nitrogen atmosphere, and a solution of di-*tert*-butyl dicarbonate (935.96 mg, 4.29 mmol) in dichloromethane (5 mL) was added dropwise. The reaction mixture was warmed to 25°C and stirred for 12 hours. The reaction mixture was diluted with dichloromethane (30 mL). The organic phase was washed with water (20 mL × 2), then washed with saturated brine (20 mL), dried over anhydrous sodium sulfate, filtered, and the filtrate was concentrated under reduced pressure to obtain a crude product, which was purified by flash silica gel column (ethyl acetate/petroleum ether = 0 to 15%) to obtain the compound **1-5.** $^1H$ NMR (400 MHz, $CDCl_3$) $\delta$ ppm 7.20 (d, *J*=8.03 Hz, 1H), 7.06 (dd, *J*=7.91, 1.63 Hz, 1H), 7.01 (d, *J=1.51* Hz, 1H), 5.31 (br s, 1H), 4.84 (br s, 1H), 4.65-4.73 (m, 1H), 4.36 (dd, *J*=10.04, 4.27 Hz, 1H), 1.47 (s, 9 H).

Step 5

Compound **1-5** (500 mg, 1.59 mmol) was dissolved in tetrahydrofuran (10 mL) and cooled to 0°C. Sodium hydride (127.31 mg, 3.18 mmol, purity: 60%) was added under a nitrogen atmosphere and stirred at 0°C for 2 hours. Iodomethane (451.78 mg, 3.18 mmol) was added dropwise, and the reaction mixture was slowly warmed to 25°C and stirred for 12 hours under a nitrogen atmosphere. The reaction mixture was quenched by adding water (10 mL) dropwise and extracted with ethyl acetate (10 mL × 3). The organic phases were combined, washed with saturated brine (10 mL), dried over anhydrous sodium sulfate, and filtered. The filtrate was concentrated under reduced pressure to obtain a crude product, which was purified by flash silica gel column (ethyl acetate/petroleum ether = 0 to 10%). Compound **1-6** was obtained. $^1H$ NMR (400 MHz, $CDCl_3$) $\delta$ ppm 7.04-7.14 (m, 2H), 7.01 (s, 1H), 5.56-6.16 (m, 1H), 4.63 (br t, *J*=9.54 Hz, 1H), 4.39 (br d, *J*=7.28 Hz, 1H), 2.55 (br s, 3H), 1.50 (s, 9 H).

Step 6

Compound **1-6** (500 mg, 1.52 mmol) and trimethylsilylacetylene (1.50 g, 15.23 mmol) were dissolved in triethylamine (5 mL), then dichlorobis(triphenylphosphine)palladium(II) (213.86 mg, 304.69 μmol) and copper(I) iodide (58.03 mg, 304.69 μmol) were added. The reaction mixture was stirred at 75°C for 3 hours under a nitrogen atmosphere. The reaction mixture was diluted with ethyl acetate (20 mL), filtered, and the filter cake was washed with ethyl acetate (10 mL × 2). The collected filtrate was washed with water (10 mL × 2), washed with saturated brine (10 mL), dried over anhydrous sodium sulfate, filtered, and the filtrate was concentrated under reduced pressure to obtain a crude product, which was purified by flash silica gel column (ethyl acetate/petroleum ether = 0 to 10%) to obtain compound 1-7. $^1H$ NMR (400 MHz, $CDCl_3$) $\delta$ ppm 7.16 (br d, *J*=7.03 Hz, 1H), 7.06 (dd, *J*=7.65, 1.13 Hz, 1H), 6.93 (s, 1H), 5.70-6.17 (m, 1H), 4.62 (br t, *J*=9.29 Hz, 1H), 4.38 (br s, 1H), 2.51 (br s, 3H), 1.50 (s, 9H), 0.25 (s, 9 H).

Step 7

Compound **1-7** (100 mg, 289.43 μmol) was dissolved in dichloromethane (1 mL), then trifluoroacetic acid (383.75 mg, 3.37 mmol) was added dropwise, and the reaction mixture was stirred at 25°C for 1 hour. The reaction mixture was concentrated under reduced pressure, diluted with dichloromethane (2 mL), and concentrated under reduced pressure to obtain the crude trifluoroacetate salt of **1-8,** which was used directly in the next step.

Step 8

The crude product **1-8** (70 mg, crude trifluoroacetate salt) was dissolved in *N,N*-dimethylacetamide (1 mL). *N,N-Diisopropylethylamine* (73.74 mg, 570.52 μmol) was added dropwise, and then intermediate **A** (74.23 mg, 285.26 μmol) and *O*-(7-azabenzotriazol-1-yl)-*N,N,N,N-tetramethyluronium* hexafluorophosphate (130.16 mg, 342.31 μmol) were

added, and the reaction mixture was stirred at 25°C for 12 hours. The reaction mixture was dropwise added with water (5 mL) and extracted with ethyl acetate (10 mL × 3). The organic phases were combined, washed with saturated brine (10 mL), dried over anhydrous sodium sulfate, filtered, and the filtrate was concentrated under reduced pressure to obtain a crude product **1-9.** MS: *m*/*z* 488.1 $[M+H]^+$.

Step 9

The crude product **1-9** (130 mg, 266.61 μmol) was dissolved in methanol (2 mL), then potassium carbonate (73.69 mg, 533.22 μmol) was added, and the reaction mixture was stirred at 25°C for 1 hour. The reaction mixture was diluted with *N,N*-dimethylformamide (1 mL), filtered, and the filtrate was collected. The crude product was purified by preparative high performance liquid chromatography (chromatographic column: Welch Xtimate C18 150 * 30 mm * 5 μm; mobile phase: [phase A: water (0.225% formic acid); phase B: acetonitrile]; gradient: B%: 8% to 48%) to obtain compound **1.** MS: *m*/*z* 416.0 $[M+H]^+$. $^1$H NMR (400 MHz, $CD_3OD$) δ ppm 8.41 (d, *J*=6.88 Hz, 1H), 8.32 (s, 1H), 7.42-7.55 (m, 1H), 7.26-7.41 (m, 1H), 7.04-7.18 (m, 1H), 6.88-6.99 (m, 1H), 5.59-6.55 (m, 1H), 4.76-4.84 (m, 1H), 4.66 (br dd, *J*=10.38, 3.50 Hz, 1H), 4.43-4.52 (m, 3H), 3.52 (d, *J*=3.25 Hz, 1H), 2.67-2.85 (m, 3 H).

Step 10

Compound **1** was purified by preparative SFC (separation conditions, chromatographic column: DAICEL CHIRALCEL OD-H (250 mm * 30 mm, 5 μm); mobile phase: [phase A: supercritical carbon dioxide, phase B: ethanol (0.1% ammonia water)]; gradient: B%: 40%) to obtain compounds **1a** and **1b**.

Compound **1a**: SFC analysis detection conditions: chromatographic column: Chiralcel OD-3 150 * 4.6 mm I.D., 3 μm; mobile phase: phase A: supercritical carbon dioxide, phase B: ethanol (0.05% diethylamine); gradient: B%: 40%, retention time: 2.214 min, ee = 94.56%. MS: *m*/*z* 416.3 $[M+H]^+$. $^1$H NMR (400 MHz, $CD_3OD$) δ ppm 8.24 (br d, *J*=7.28 Hz, 1H), 8.11 (d, *J*=2.26 Hz, 1H), 7.14-7.37 (m, 2H), 6.94-7.06 (m, 1H), 6.77-6.89 (m, 1H), 5.51-6.42 (m, 1H), 4.65-4.72 (m, 1H), 4.52-4.58 (m, 1H), 4.30-4.41 (m, 3H), 3.41 (d, *J*=3.51 Hz, 1H), 2.57-2.74 (m, 3 H).

Compound **1b:** SFC analysis detection conditions: chromatographic column: Chiralcel OD-3 150 * 4.6 mm I.D., 3 μm; mobile phase: phase A: supercritical carbon dioxide, phase B: ethanol (0.05% diethylamine); gradient: B%: 40%, retention time: 2.715 min, ee = 88.50%. MS: *m*/*z* 416.3 $[M+H]^+$. $^1$H NMR (400 MHz, $CD_3OD$) δ ppm 8.34 (d, *J*=7.28 Hz, 1H), 8.21 (d, *J*=2.76 Hz, 1H), 7.26-7.47 (m, 2H), 7.04-7.16 (m, 1H), 6.88-6.98 (m, 1H), 5.61-6.53 (m, 1H), 4.76-4.82 (m, 1H), 4.65 (dd, *J*=10.67, 3.64 Hz, 1H), 4.42-4.50 (m, 3H), 3.51 (d, *J*=4.02 Hz, 1H), 2.68-2.83 (m, 3 H).

**Example 2**

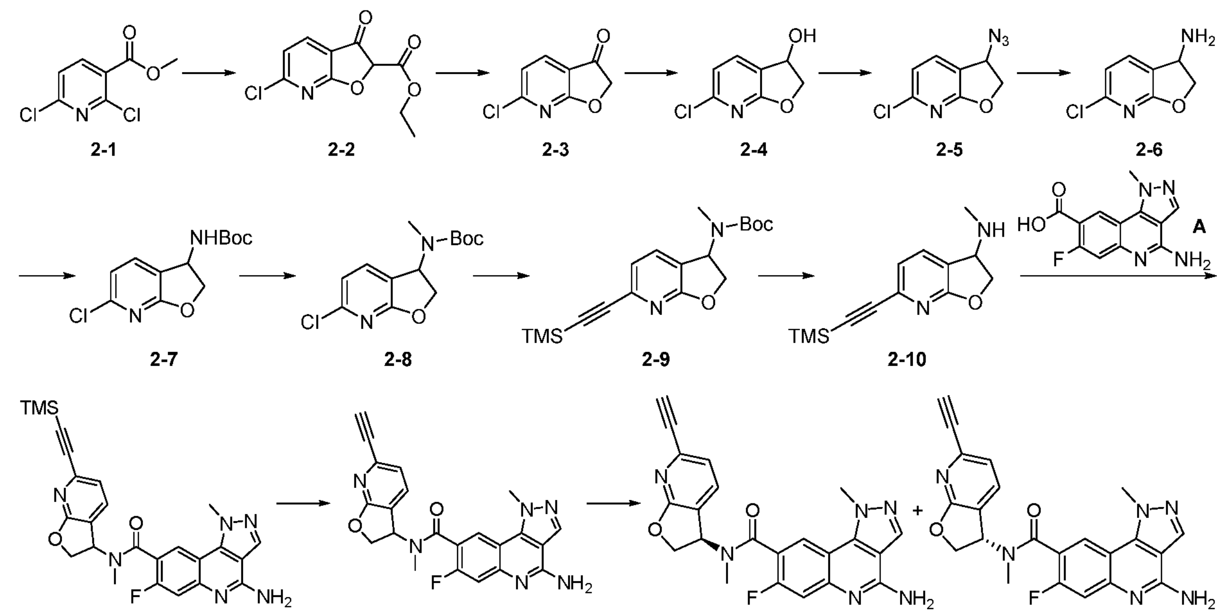

2-1 2-2 2-3 2-4 2-5 2-6

2-7 2-8 2-9 2-10 A

2-11 2 2a or 2b + 2b or 2a

Step 1

Sodium hydride (10.19 g, 254.82 mmol, purity: 60%) was dissolved in tetrahydrofuran (200 mL). Ethyl glycolate (12.73 g, 122.31 mmol) was added at 0°C, and then the mixture was stirred for 30 minutes. A solution of compound **2-1** (methyl 2,6-dichloronicotinate, 21 g, 101.93 mmol) in tetrahydrofuran (200 mL) was added. After the temperature was raised to 75°C, the reaction mixture was stirred for 2.5 hours. After the reaction was completed, ethanol was slowly added with stirring until no more hydrogen gas was released. The reaction mixture was then neutralized to neutral with dilute hydrochloric acid or acetic acid aqueous solution, and diluted with ethyl acetate (500 mL). The organic phase was washed with water (500 mL × 2) and saturated brine (500 mL × 2), dried over anhydrous sodium sulfate, filtered, and the filtrate was concentrated under reduced pressure to obtain compound **2-2,** which was directly used in the next step.

Step 2

Compound **2-2** (22 g, 91.42 mmol) was dissolved in dioxane (220 mL) and hydrochloric acid (58 mL), and the reaction mixture was stirred at 95°C for 12 hours. The reaction mixture was diluted with ethyl acetate (200 mL), and sodium bicarbonate was added to adjust the pH to 8. The organic phase was washed with water (200 mL × 2) and then with saturated brine (200 mL × 2), dried over anhydrous sodium sulfate, filtered, and the filtrate was concentrated under reduced pressure to obtain a crude product, which was purified by flash silica gel column (0-25% ethyl acetate/petroleum ether). Compound **2-3** was obtained. MS: *m*/*z* 169.8 $[M+H]^+$.

Step 3

Compound **2-3** (3 g, 17.69 mmol) was dissolved in ethanol (30 mL), then sodium borohydride (1.69 g, 44.67 mmol) was added in batches at 0°C, and the reaction mixture was warmed to 25°C and stirred for 2 hours. The reaction mixture was quenched with saturated ammonium chloride (100 mL) aqueous solution and diluted with ethyl acetate (100 mL). Diluted hydrochloric acid was added to adjust the pH to below 3. The organic phase was washed with water (100 mL × 2) and saturated brine (100 mL × 2), dried over anhydrous sodium sulfate, filtered, and the filtrate was concentrated under reduced pressure to obtain a crude product, which was purified by flash silica gel column (0-30% ethyl acetate/petroleum ether). Compound **2-4** was obtained. MS: *m*/*z* 171.8 $[M+H]^+$.

Step 4

Compound **2-4** (1 g, 5.83 mmol) was dissolved in toluene (10 mL). Diphenylphosphoryl azide (2.41 g, 8.74 mmol) and 1.8-diazabicyclo[5.4.0]undec-7-ene (1.33 g, 8.74 mmol) were added at 0°C. The reaction mixture was stirred for 2 hours under a nitrogen atmosphere, then warmed to 25°C and stirred for another 10 hours. The reaction mixture was diluted with ethyl acetate (100 mL). The organic phase was washed with water (100 mL × 2) and saturated brine (100 mL × 2), dried over anhydrous sodium sulfate, filtered, and the filtrate was concentrated under reduced pressure to obtain a crude product, which was purified by flash silica gel column (0-20% ethyl acetate/petroleum ether). Compound **2-5** was obtained. MS: *m*/*z* 196.8 $[M+H]^+$.

Step 5

Compound **2-5** (1.7 g, 8.65 mmol) was dissolved in tetrahydrofuran (20 mL), then triphenylphosphine (3.40 g, 12.97 mmol) was added, and the reaction mixture was stirred at 25°C for 1 hour under a nitrogen atmosphere. A solution of potassium hydroxide (1.21 g, 21.62 mmol) in water (5 mL) was added, and the reaction mixture was heated to 50°C and stirred for another 11 hours. The reaction mixture was diluted with ethyl acetate (100 mL). The organic phase was sequentially washed with water (100 mL × 2) and saturated brine (100 mL × 2), dried over anhydrous sodium sulfate. The filtrate was concentrated under reduced pressure to obtain compound **2-6,** and the crude product was directly used in the next step.

Step 6

Compound **2-6** (400 mg, 703.42 μmol) was dissolved in dichloromethane (4 mL), then triethylamine (106.77 mg, 1.06 mmol, 146.86 μL) and di-*tert*-butyl dicarbonate (161.20 mg, 738.59 μmol) were added, and the reaction mixture was stirred at 25°C for 12 hours. The reaction mixture was diluted with ethyl acetate (100 mL). The organic phase was sequentially washed with water (100 mL × 2) and saturated brine (100 mL × 2), dried over anhydrous sodium sulfate, filtered, and the filtrate was concentrated under reduced pressure to obtain a crude product, which was purified by flash silica gel column (0-20% ethyl acetate/petroleum ether). Compound **2-7** was obtained. MS: *m*/*z* 214.8 $[M-{}^tBu+H]^+$.

Step 7

Compound **2-7** (1.2 g, 4.43 mmol) was dissolved in tetrahydrofuran (12 mL). Sodium hydride (354.59 mg, 8.87 mmol, purity: 60%) was added at 0°C, and then the mixture was stirred for 1 hour. Methyl iodide (1.26 g, 8.87 mmol) was then added dropwise to the reaction mixture, and stirring was continued at 0°C for 30 minutes. The temperature was raised to 25°C, and the reaction was stirred for another 10.5 hours. Ethanol was slowly added to the reaction mixture with stirring until no more hydrogen gas was released. The pH of the mixture was then adjusted to 7 with dilute hydrochloric acid. Ethyl acetate (100 mL) was added for dilution. The organic phase was sequentially washed with water (100 mL × 2) and saturated brine (100 mL × 2), dried over anhydrous sodium sulfate, filtered, and the filtrate was concentrated under reduced pressure to obtain a crude product, which was purified by flash silica gel column (0-20% ethyl acetate/petroleum ether). Compound **2-8** was obtained. MS: *m/z* 228.8 [M-*t*Bu+H]$^+$.

Step 8

Compound **2-8** (150 mg, 526.80 μmol) and trimethylsilylacetylene (517.41 mg, 5.27 mmol) were dissolved in triethylamine (3 mL), then dichlorobis(triphenylphosphine)palladium(II) (147.90 mg, 210.72 μmol) and copper(I) iodide (50.16 mg, 263.40 μmol) were added. The reaction mixture was stirred at 75°C for 12 hours under a nitrogen atmosphere. The reaction mixture was diluted with water (10 mL) and extracted with ethyl acetate (10 mL × 2). The organic phases were combined, washed with saturated brine (10 mL), dried over anhydrous sodium sulfate, and filtered. The filtrate was concentrated under reduced pressure to obtain a crude product, which was purified by flash silica gel column (0-20% ethyl acetate/petroleum ether) to obtain compound **2-9.** MS: *m/z* 290.9 [M-*t*Bu+H]$^+$.

Step 9

Compound **2-9** (117 mg, 337.67 μmol) was dissolved in dichloromethane (2 mL), then trifluoroacetic acid (1.20 g, 10.50 mmol) was added, and the reaction mixture was stirred at 25°C for 2 hours. The reaction mixture was directly concentrated to obtain the crude trifluoroacetate salt of compound **2-10,** which was directly used in the next step.

Step 10

Compound **2-10** (65 mg, crude trifluoroacetate salt) was dissolved in *N,N*-dimethylacetamide (1.5 mL). Intermediate A (72.08 mg, 277.01 μmol), *N,N*-diisopropylethylamine (102.29 mg, 791.46 μmol), and *O*-(7-azabenzotriazol-1-yl)-*N,N,N,N*-tetramethyluronium hexafluorophosphate (130.41 mg, 342.97 μmol) were sequentially added, and the reaction mixture was stirred at 25°C for 12 hours. The reaction mixture was diluted with ethyl acetate (10 mL). The organic phase was washed with water (10 mL × 2) and saturated brine (10 mL × 2), dried over anhydrous sodium sulfate, and filtered. The filtrate was concentrated under reduced pressure to obtain compound **2-11,** which was directly used in the next step. MS: *m/z* 489.1 [M+H]$^+$.

Step 11

Compound **2-11** (110 mg, 225.14 μmol) was dissolved in methanol (3 mL), then potassium carbonate (62.23 mg, 450.28 μmol) was added, and the reaction mixture was stirred at 25°C for 3 hours. After the reaction was completed, the reaction mixture was diluted with ethyl acetate (20 mL). The organic phase was washed with water (20 mL × 2) and saturated brine (20 mL × 2), dried over anhydrous sodium sulfate, filtered, and the filtrate was concentrated under reduced pressure. The crude product was purified by preparative high performance liquid chromatography (chromatographic column: Welch Xtimate C18 150 × 30 mm × 5 μm; mobile phase: [phase A: water (0.225% formic acid); phase B: acetonitrile]; gradient: B%: 8% to 48%). Compound **2** was obtained.

Step 12

Compound **2** was purified by SFC (separation conditions, chromatographic column: DAICEL CHIRALCEL OJ (250 mm × 30 mm, 10 μm); mobile phase: [phase A: supercritical carbon dioxide, phase B: ethanol (0.1% ammonia water)]; gradient: B%: 55%) to obtain compounds **2a** and **2b.**

Compound **2a:** SFC detection conditions: chromatographic column: Chiralcel OJ-3 100 × 4.6 mm I.D., 3 μm; mobile phase: phase A: supercritical carbon dioxide, phase B: ethanol (0.05% diethylamine); gradient: B%: 40%, retention time: 1.797 min, ee = 100%. MS: *m/z* 439.2 [M+Na]$^+$. $^1$H NMR (400 MHz, $CD_3OD$) $\delta$ ppm 8.20-8.40 (m, 1H), 8.10 (s, 1H), 7.65-7.80 (m, 1H), 7.23-7.34 (m, 1H), 7.06-7.20 (m, 1H), 5.55-6.45 (m, 1H), 4.50-4.70 (m, 2H), 4.27-4.40 (m, 3H), 4.03-4.08 (m, 1H), 2.63-2.80 (m, 3H).

Compound **2b:** SFC detection conditions: chromatographic column: Chiralcel OJ-3 100 × 4.6 mm I.D., 3 μm; mobile phase: phase A: supercritical carbon dioxide, phase B: ethanol (0.05% diethylamine); gradient: B%: 40%, retention time: 3.929 min, ee = 99.30%. MS: *m/z* 439.4 $[M+Na]^+$. $^1H$ NMR (400 MHz, $CD_3OD$) $\delta$ ppm 8.28-8.45 (m, 1H), 8.20 (s, 1 H), 7.75-7.90 (m, 1H), 7.30-7.45 (m, 1H), 7.17-7.23 (m, 1H), 5.67-6.53 (m, 1H), 4.89-4.97 (m, 1H), 4.69-4.78 (m, 1H), 4.38-4.50 (m, 3H), 3.33-3.37 (m, 1H), 2.66-2.91 (m, 3H).

**Example 3**

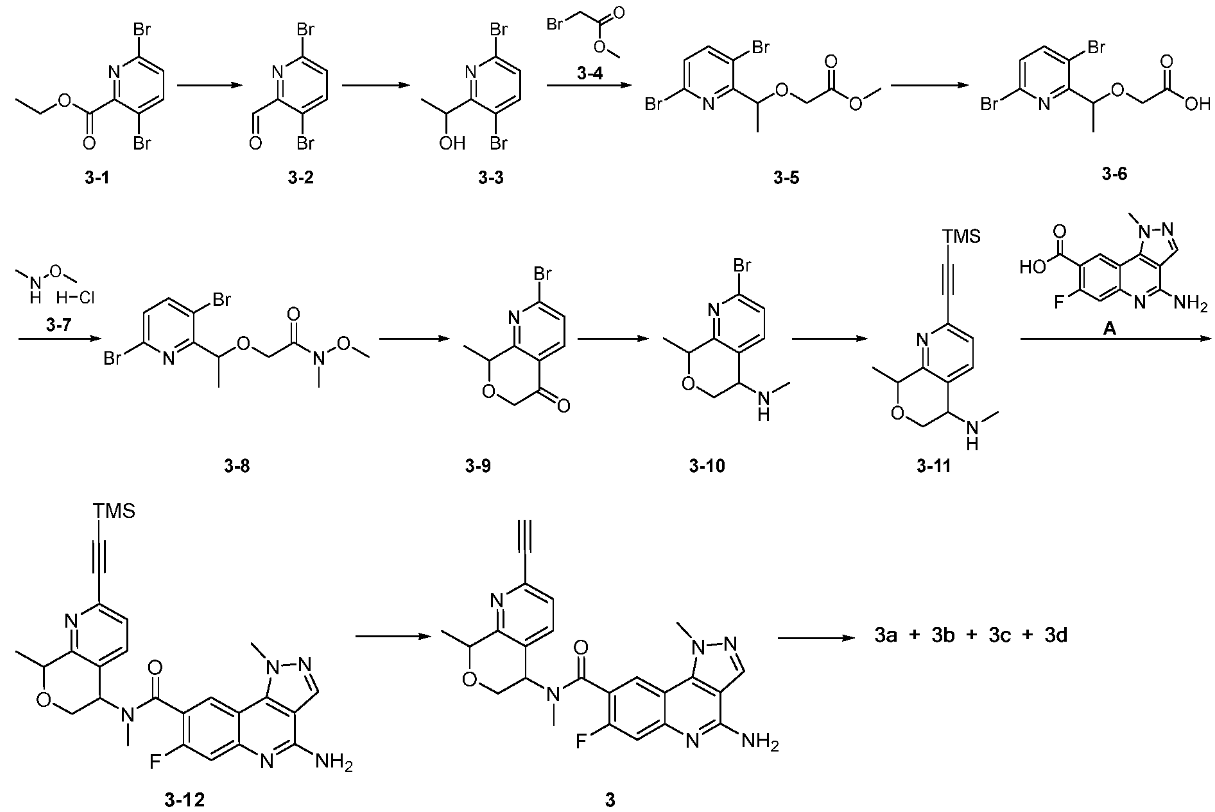


3-1, 3-2, 3-3, 3-5, 3-6, 3-8, 3-9, 3-10, 3-11, 3-12, 3

3a + 3b + 3c + 3d

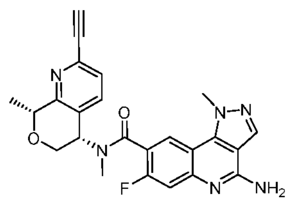

**3a or 3b or 3c or 3d**

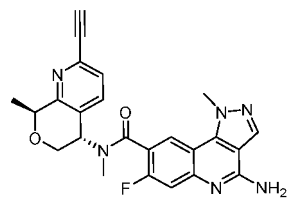

**3a or 3b or 3c or 3d**

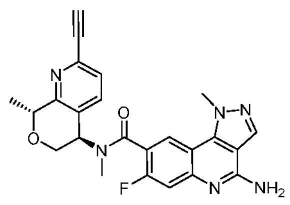

**3a or 3b or 3c or 3d**

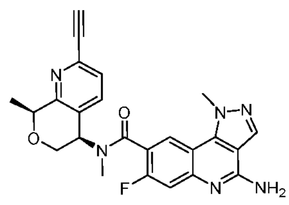

**3a or 3b or 3c or 3d**

Step 1

Compound **3-1** (9 g, 29.13 mmol) was dissolved in tetrahydrofuran (135 mL). Under a nitrogen atmosphere, a 1M toluene solution of diisobutylaluminum hydride (1 M, 43.70 mL) was added dropwise at -78°C. The reaction mixture was slowly stirred at -78°C for 3 hours under an $N_2$ atmosphere. At 0°C, the reaction mixture was slowly poured into a mixture of glacial acetic acid (22.5 mL) and water (112.5 mL). The temperature was then raised to 25°C, and the mixture was stirred for 1.5 hours, and cooled to 0°C. Saturated sodium bicarbonate aqueous solution was added dropwise to the reaction mixture to adjust the pH to 8, and the mixture was extracted with ethyl acetate (200 mL × 2). The phases were combined, washed with saturated brine (200 mL), dried over anhydrous sodium sulfate, filtered, and the filtrate was concentrated under reduced pressure to obtain a crude product, which was purified by flash silica gel column (0-10% ethyl acetate/dichloromethane). Compound **3-2** was obtained. $^1H$ NMR (400 MHz, $CDCl_3$) $\delta$ ppm 10.10 (s, 1H), 7.88 (d, *J*=8.28 Hz, 1H), 7.55 (d, *J*=8.53 Hz, 1 H).

Step 2

Compound **3-2** (3 g, 11.32 mmol) was dissolved in tetrahydrofuran (45 mL). Methylmagnesium bromide (3 M, 7.55 mL) was added dropwise at -78°C under a nitrogen atmosphere. The reaction mixture was stirred at -78°C for 0.5 hours, and then warmed to 25°C and stirred for 0.5 hours. Under a nitrogen atmosphere, the reaction mixture was quenched by slowly adding saturated ammonium chloride aqueous solution (30 mL) dropwise and extracted with ethyl acetate (50 mL × 2). The organic phases were combined, washed with saturated brine (30 mL), dried over anhydrous sodium sulfate, and filtered. The filtrate was concentrated under reduced pressure to obtain a crude product, which was purified by flash silica gel column (0-10% ethyl acetate/petroleum ether). Compound **3-3** was obtained. $^1H$ NMR (400 MHz, $CDCl_3$) $\delta$ ppm 7.69 (d, *J*=8.28 Hz, 1H), 7.30 (d, *J*=8.28 Hz, 1H), 5.10 (q, *J*=6.44 Hz, 1H), 1.47 (d, *J*=6.27 Hz, 3 H).

Step 3

Compound **3-3** (1.8 g, 6.41 mmol) and compound **3-4** (2.94 g, 19.22 mmol) were dissolved in tetrahydrofuran (72 mL), cooled to 0°C, and then sodium hydride (512.56 mg, 12.81 mmol, purity: 60%) was added in batches. The reaction mixture was stirred at 0°C for 4 hours under a nitrogen atmosphere, slowly warmed to 25°C and stirred for another 12 hours. The reaction mixture was cooled to 0°C, quenched by slowly adding saturated ammonium chloride aqueous solution (50 mL) dropwise, and extracted with ethyl acetate (50 mL × 3). The organic phases were combined, washed with saturated brine (50 mL × 2), dried over anhydrous sodium sulfate, and filtered. The filtrate was concentrated under reduced pressure to obtain a crude product, which was purified by flash silica gel column (0-15% ethyl acetate/petroleum ether). Compound **3-5** was obtained. MS: *m*/*z* 351.7, 353.7, 355.7 $[M+H]^+$.

Step 4

Compound **3-5** (1.5 g, 4.25 mmol) was dissolved in tetrahydrofuran (8 mL). Methanol (8 mL) and water (4 mL) were added, and then lithium hydroxide monohydrate (356.62 mg, 8.50 mmol) was added, and the reaction mixture was stirred at 25°C for 2 hours. The reaction mixture was concentrated under reduced pressure to remove tetrahydrofuran and methanol, and 1 M dilute hydrochloric acid was added dropwise to the aqueous phase to adjust the pH to 3-4. The mixture was extracted with ethyl acetate (30 mL × 2). The organic phases were combined, washed with saturated brine (20 mL), dried over anhydrous sodium sulfate, filtered, and the filtrate was concentrated under reduced pressure to obtain compound **3-6.** MS: *m*/*z* 337.7, 339.7, 341.7 $[M+H]^+$.

Step 5

Compound **3-6** (1.55 g, 4.57 mmol) and compound **3-7** (535.23 mg, 5.49 mmol) were dissolved in *N,N*-dimethylformamide (15 mL), and then *O*-(7-azabenzotriazol-1-yl)-*N,N,N',N'*-tetramethyluronium hexafluorophosphate (2.09 g, 5.49 mmol) and *N,N*-diisopropylethylamine (1.77 g, 13.72 mmol) were added. The reaction mixture was stirred at 25°C for 12 hours. The reaction mixture was added with water (20 mL) and extracted with ethyl acetate (30 mL × 2). The organic phases were combined, washed with water (20 mL × 2) and saturated brine (20 mL), dried over anhydrous sodium sulfate, filtered, and the filtrate was concentrated under reduced pressure to obtain a crude product, which was purified by flash silica gel column (0-50% ethyl acetate/petroleum ether). Compound **3-8** was obtained. MS: *m*/*z* 380.8, 382.8, 384.8 $[M+H]^+$.

Step 6

Compound **3-8** (700 mg, 1.83 mmol) was dissolved in tetrahydrofuran (23 mL), cooled to -78°C, then n-butyllithium (2.5 M, 879.47 μL) was added dropwise, and the reaction mixture was stirred at -78°C for 4 hours under a nitrogen atmosphere. The reaction mixture was slowly warmed to 0°C, quenched by slowly adding saturated ammonium chloride aqueous solution (10 mL) dropwise, and extracted with ethyl acetate (20 mL × 3). The organic phases were combined, washed with saturated brine (20 mL × 2), dried over anhydrous sodium sulfate, and filtered. The filtrate was concentrated under reduced pressure to obtain a crude product, which was purified by flash silica gel column (0-15% ethyl acetate/petroleum ether). Compound **3-9** was obtained. MS: *m*/*z* 241.7, 243.7 $[M+H]^+$.

Step 7

Compound **3-9** (250 mg, 1.03 mmol) was dissolved in methanol (2.5 mL). Methylamine tetrahydrofuran solution (1 M, 2.52 mL) and zinc chloride (211.15 mg, 1.55 mmol) were added, and the mixture was stirred at 25°C for 2 hours. Sodium cyanoborohydride (97.35 mg, 1.55 mmol) was then added, and the reaction mixture was stirred at 40°C for 40

hours under a nitrogen atmosphere. The reaction mixture was quenched by adding 10% sodium bicarbonate aqueous solution (20 mL) and then extracted with dichloromethane (20 mL × 4). The organic phases were combined, washed with saturated brine (20 mL), dried over anhydrous sodium sulfate, filtered, and the filtrate was concentrated under reduced pressure to obtain a crude product, which was purified by flash silica gel column (0-10% methanol/dichloromethane). Compound **3-10** was obtained. MS: *m*/*z* 256.9, 258.9 $[M+H]^+$.

Step 8

Compound **3-10** (170 mg, 661.15 μmol) and trimethylsilylacetylene (649.37 mg, 6.61 mmol) were dissolved in triethylamine (3.4 mL), then dichlorobis(triphenylphosphine)palladium(II) (92.81 mg, 132.23 μmol) and copper(I) iodide (25.18 mg, 132.23 μmol) were added. The reaction mixture was stirred at 75°C for 3 hours under a nitrogen atmosphere. The reaction mixture was diluted with ethyl acetate (10 mL), filtered, and the filter cake was washed with ethyl acetate (10 mL × 2). The filtrate was collected, and the organic phase was washed with water (10 mL × 2) and saturated brine (10 mL), dried over anhydrous sodium sulfate, filtered, and the filtrate was concentrated under reduced pressure to obtain a crude product, which was purified by flash silica gel column (0-10% methanol/dichloromethane). Compound **3-11** was obtained. MS: *m*/*z* 274.9 $[M+H]^+$.

Step 9

Compound **3-11** (140 mg, 510.14 μmol) and compound **A** (119.48 mg, 459.13 μmol) were dissolved in *N,N*-dimethylacetamide (2.8 mL), and then *O*-(7-azabenzotriazol-1-yl)-*N,N,N',N'*-tetramethyluronium hexafluorophosphate (232.77 mg, 612.17 μmol) and *N,N*-diisopropylethylamine (131.86 mg, 1.02 mmol) were added. The reaction mixture was stirred at 25°C for 12 hours. The reaction mixture was added with water (10 mL) and extracted with ethyl acetate (10 mL × 3). The organic phases were combined, washed with saturated brine (10 mL), dried over anhydrous sodium sulfate, filtered, and the filtrate was concentrated under reduced pressure to obtain a crude product, which was purified by flash silica gel column (0-10% methanol/dichloromethane). Compound **3-12** was obtained. MS: *m*/*z* 517.1 $[M+H]^+$.

Step 10

Compound **3-12** (280 mg, 541.96 μmol) was dissolved in methanol (3 mL), then potassium carbonate (149.80 mg, 1.08 mmol) was added, and the reaction mixture was stirred at 25°C for 2 hours. The reaction mixture was filtered, the filter cake was washed with methanol (1 mL), and the filtrate was collected. The filtrate was purified by preparative high performance liquid chromatography (chromatographic column: Welch Xtimate C18 150 × 30 mm × 5 μm; mobile phase: [phase A: water (0.225% formic acid); phase B: acetonitrile]; gradient: B%: 7% to 47%) to obtain compound **3.** MS: *m*/*z* 445.0 $[M+H]^+$.

Step 11

Compound **3** was purified by SFC (separation conditions, chromatographic column: DAICEL CHIRALCEL OD-H (250 mm × 30 mm, 5 μm); mobile phase: [phase A: supercritical carbon dioxide, phase B: methanol (0.1% ammonia water)]; gradient: B%: 40%) to obtain two crude compounds. The retention time of the first crude compound was 5.483 min and the retention time of the second crude compound was 5.820 min by SFC detection (chromatographic column: Chiralcel OD-3 150 × 4.6 mm I.D., 3 μm; mobile phase: phase A: supercritical carbon dioxide, phase B: methanol (0.05% diethylamine); gradient: B%: 5% to 40% (0 to 2 min), 40% (2 to 4 min), 5% (4 to 6min)).

The first crude compound was then purified by SFC (separation conditions, chromatographic column: DAICEL CHIRALPAK AD (250 mm × 30 mm, 10 μm); mobile phase: [phase A: supercritical carbon dioxide, phase B: ethanol (0.1% ammonia water)]; gradient: B%: 30%) to obtain compounds **3a** and **3b.**

Compound **3a:**

Detection under SFC detection condition I (chromatographic column: ChiralPak AD-3 150 × 4.6 mm I.D., 3 μm; mobile phase: phase A: supercritical carbon dioxide, phase B: ethanol (0.05% diethylamine); gradient: B%: 5% to 40% (0 to 4.5 min), 40% (4.5 to 6.5 min), 5% (6.5 to 8 min)) showed a retention time of 5.283 min. Further detection under SFC detection condition II (chromatographic column: Chiralcel OD-3 150 × 4.6 mm I.D., 3 μm; mobile phase: phase A: supercritical carbon dioxide, phase B: methanol (0.05% diethylamine); gradient: B%: 5% to 40% (0 to 2 min), 40% (2 to 4 min), 5% (4 to 6 min)) showed a retention time of 5.471 min, with a chiral purity of 91.55%. MS: *m*/*z* 445.0 $[M+H]^+$. $^1H$ NMR (400 MHz, $CD_3OD$) $\delta$ ppm 8.47 (d, *J*=6.75 Hz, 1H), 8.34-8.37 (m, 1H), 7.84 (d, *J*=7.88 Hz, 1H), 7.54 (d, *J*=8.13 Hz, 1H), 7.49 (d, *J*=10.88 Hz, 1H), 5.89 (br s, 1H), 4.76 (br d, *J*=6.13 Hz, 1H), 4.50 (s, 3H), 4.38 (br d, *J*=12.38 Hz, 1H), 4.17 (dd, *J*=12.82,

4.07 Hz, 1H), 3.75-3.79 (m, 1H), 2.87 (s, 3H), 1.63 (d, *J*=6.63 Hz, 3 H).

Compound **3b:**

Detection under SFC detection condition I (chromatographic column: ChiralPak AD-3 150 × 4.6 mm I.D., 3 μm; mobile phase: phase A: supercritical carbon dioxide, phase B: ethanol (0.05% diethylamine); gradient: B%: 5% to 40% (0 to 4.5 min), 40% (4.5 to 6.5 min), 5% (6.5 to 8 min)) showed a retention time of 5.581 min. Further detection under SFC detection condition II (chromatographic column: Chiralcel OD-3 150 × 4.6 mm I.D., 3 μm; mobile phase: phase A: supercritical carbon dioxide, phase B: methanol (0.05% diethylamine); gradient: B%: 5% to 40% (0 to 2 min), 40% (2 to 4 min), 5% (4 to 6 min)) showed a retention time of 5.458 min, with a chiral purity of 100%. MS: *m/z* 445.1 $[M+H]^+$. $^1H$ NMR (400 MHz, $CD_3OD$) $\delta$ ppm 8.39 (d, *J*=7.50 Hz, 1H), 8.20-8.24 (m, 1H), 7.79 (d, *J*=7.63 Hz, 1H), 7.51-7.56 (m, 1H), 7.37-7.43 (m, 1H), 5.92-6.06 (m, 1H), 4.81 (br s, 1H), 4.43-4.47 (m, 3H), 4.36 (dd, *J*=11.76, 5.63 Hz, 1H), 4.06 (dd, *J*=11.57, 7.07 Hz, 1H), 3.74-3.78 (m, 1H), 2.80-2.89 (m, 3H), 1.59 (d, *J*=6.75 Hz, 3 H).

The second crude compound was then purified by SFC (separation conditions, chromatographic column: DAICEL CHIRALPAK IC (250 mm × 30 mm, 10 μm); mobile phase: [phase A: supercritical carbon dioxide, phase B: ethanol (0.1% ammonia water)]; gradient: B%: 60%) to obtain compounds **3c** and **3d.**

Compound **3c:**

Detection under SFC detection condition III (chromatographic column: Chiralpak IC-3 100 × 4.6 mm I.D., 3 μm; mobile phase: phase A: supercritical carbon dioxide, phase B: ethanol (0.05% diethylamine); gradient: B%: 60%) showed a retention time of 2.029 min. Further detection under SFC detection condition IV (chromatographic column: Chiralcel OD-3 150 × 4.6 mm I.D., 3 μm; mobile phase: phase A: supercritical carbon dioxide, phase B: methanol (0.05% diethylamine); gradient: B%: 5% to 40% (0 to 2 min), 40% (2 to 4 min), 5% (4 to 6 min)) showed a retention time of 5.803 min, with a chiral purity of 93.45%. MS: *m/z* 445.0 $[M+H]^+$. $^1H$ NMR (400 MHz, $CD_3OD$) $\delta$ ppm 8.39 (d, *J*=7.38 Hz, 1H), 8.22 (s, 1H), 7.76-7.85 (m, 1H), 7.54 (br d, *J*=7.88 Hz, 1H), 7.35-7.44 (m, 1H), 6.00 (s, 1H), 4.81 (br s, 1H), 4.44-4.47 (m, 3H), 4.36 (dd, *J*=11.63, 5.88 Hz, 1H), 4.06 (br dd, *J*=11.69, 7.07 Hz, 1H), 3.74-3.78 (m, 1H), 2.79-2.89 (m, 3H), 1.53-1.69 (m, 3 H).

Compound **3d:**

Detection under SFC detection condition III (chromatographic column: Chiralpak IC-3 100 × 4.6 mm I.D., 3 μm; mobile phase: phase A: supercritical carbon dioxide, phase B: ethanol (0.05% diethylamine); gradient: B%: 60%) showed a retention time of 2.714 min. Further detection under SFC detection condition IV (chromatographic column: Chiralcel OD-3 150 × 4.6 mm I.D., 3 μm; mobile phase: phase A: supercritical carbon dioxide, phase B: methanol (0.05% diethylamine); gradient: B%: 5% to 40% (0 to 2 min), 40% (2 to 4 min), 5% (4 to 6min)) showed a retention time of 5.772 min, with a chiral purity of 98.56%. MS: *m/z* 445.0 $[M+H]^+$. $^1H$ NMR (400 MHz, $CD_3OD$) $\delta$ ppm 8.38 (br d, *J*=7.25 Hz, 1H), 8.22 (s, 1H), 7.84 (br d, *J*=7.63 Hz, 1H), 7.54 (br d, J=8.13 Hz, 1H), 7.37 (br d, *J*=11.88 Hz, 1H), 5.89 (br s, 1H), 4.76 (br d, *J*=6.00 Hz, 1H), 4.46 (s, 3H), 4.37 (br d, *J*=13.13 Hz, 1H), 4.17 (dd, *J*=12.94, 3.94 Hz, 1H), 3.74-3.78 (m, 1H), 2.88 (s, 3H), 1.63 (br d, *J*=6.50 Hz, 3 H).

**Example 4**

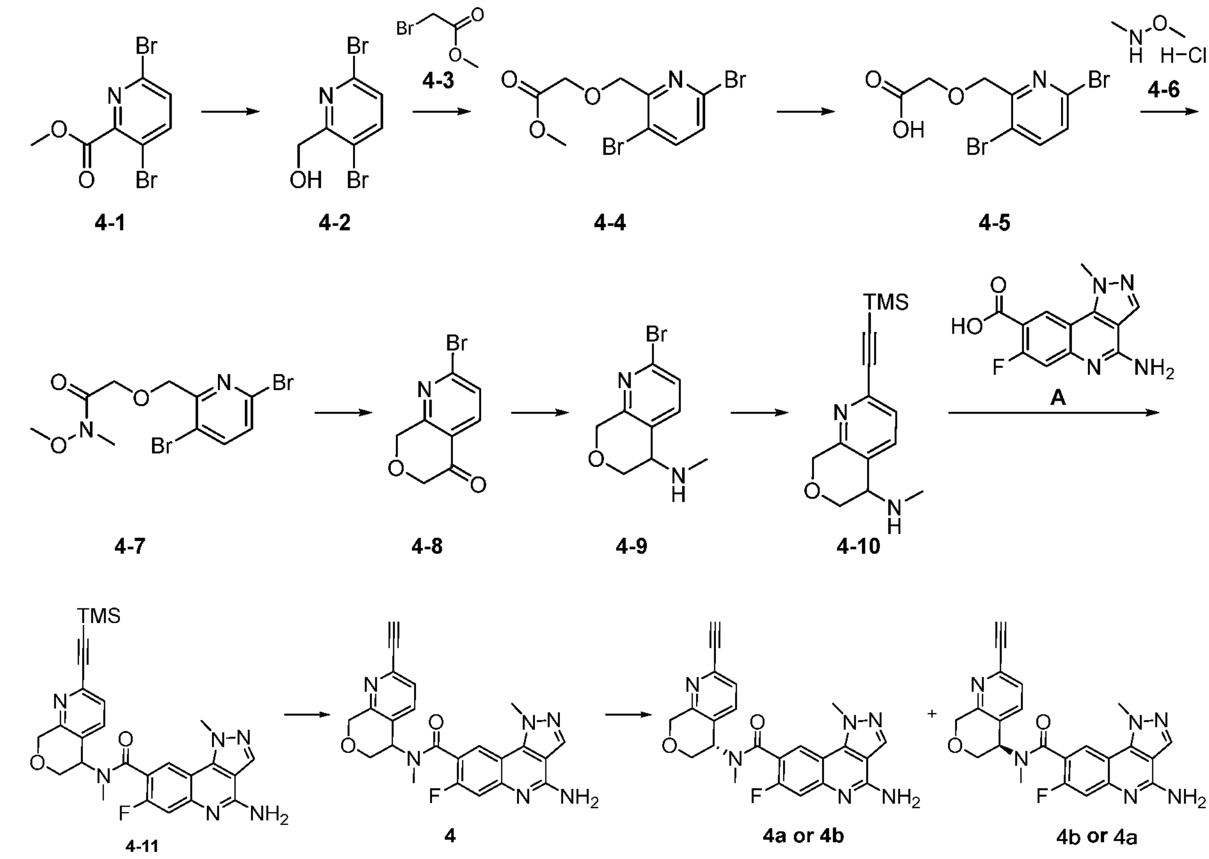


4-1 4-2 4-4 4-5

4-7 4-8 4-9 4-10

4-11 4 4a or 4b 4b or 4a

Step 1

Compound **4-1** (6.7 g, 22.72 mmol) was dissolved in ethanol (67 mL), then sodium borohydride (2.51 g, 66.35 mmol) was added in batches at 0°C under a nitrogen atmosphere, and the reaction mixture was warmed to 25°C under an $N_2$ atmosphere and stirred for 12 hours. Under an $N_2$ atmosphere, the reaction mixture was quenched by slowly adding saturated ammonium chloride aqueous solution (30 mL) dropwise and extracted with ethyl acetate (50 mL × 2). The organic phases were combined, washed with saturated brine (30 mL), dried over anhydrous sodium sulfate, and filtered. The filtrate was concentrated under reduced pressure to obtain a crude product, which was purified by flash silica gel column (0-10% ethyl acetate/petroleum ether). Compound **4-2** was obtained. MS: *m*/*z* 265.7, 267.7, 269.7 $[M+H]^+$.

Step 2

Compound **4-2** (3 g, 11.24 mmol) and compound **4-3** (5.16 g, 33.72 mmol) were dissolved in tetrahydrofuran (90 mL), cooled to 0°C, and then sodium hydride (899.15 mg, 22.48 mmol, purity: 60%) was added in batches. The reaction mixture was heated to 40°C under a nitrogen atmosphere and stirred for 36 hours. The reaction mixture was cooled to 0°C, quenched by slowly adding saturated ammonium chloride aqueous solution (50 mL) dropwise, and extracted with ethyl acetate (50 mL × 3). The organic phases were combined, washed with saturated brine (50 mL × 2), dried over anhydrous sodium sulfate, and filtered. The filtrate was concentrated under reduced pressure to obtain a crude product, which was purified by flash silica gel column (0-20% ethyl acetate/petroleum ether). Compound **4-4** was obtained. MS: *m*/*z* 337.8, 339.8, 341.8 $[M+H]^+$.

Step 3

Compound **4-4** (3.15 g, 9.29 mmol) was dissolved in tetrahydrofuran (16 mL). Methanol (16 mL) and water (8 mL) were added, and then lithium hydroxide monohydrate (779.83 mg, 18.59 mmol) was added, and the reaction mixture was stirred at 25°C for 2 hours. The reaction mixture was concentrated under reduced pressure to remove tetrahydrofuran and methanol, and 1 M dilute hydrochloric acid was added dropwise to the aqueous phase to adjust the pH to 3-4. The mixture was extracted with ethyl acetate (50 mL × 3). The organic phases were combined, washed with saturated brine (30 mL × 2), dried over anhydrous sodium sulfate, filtered, and the filtrate was concentrated under reduced pressure to obtain

compound **4-5.** MS: *m/z* 323.7, 325.7, 327.7 $[M+H]^+$.

Step 4

Compound **4-5** (3.02 g, 9.29 mmol) and compound **4-6** (1.09 mg, 11.15 mmol) were dissolved in *N,N*-dimethylformamide (30 mL), and then *O*-(7-azabenzotriazol-1-yl)-*N,N,N',N'*-tetramethyluronium hexafluorophosphate (4.24 g, 11.15 mmol) and *N,N*-diisopropylethylamine (3.60 g, 27.88 mmol) were added. The reaction mixture was stirred at 25°C for 12 hours. The reaction mixture was added with water (30 mL) and extracted with ethyl acetate (50 mL × 3). The organic phases were combined, washed with water (30 mL × 2) and saturated brine (30 mL), dried over anhydrous sodium sulfate, filtered, and the filtrate was concentrated under reduced pressure to obtain a crude product, which was purified by flash silica gel column (0-50% ethyl acetate/petroleum ether) to obtain compound **4-7.** MS: *m/z* 3660.8, 368.8, 370.8 $[M+H]^+$.

Step 5

Compound **4-7** (1.8 g, 4.89 mmol) was dissolved in tetrahydrofuran (54 mL), cooled to -78°C, then n-butyllithium (2.5 M, 2.35 mL) was added dropwise, and the reaction mixture was stirred at -78°C for 4 hours under a nitrogen atmosphere. The reaction mixture was slowly warmed to 0°C, quenched by slowly adding saturated ammonium chloride aqueous solution (20 mL) dropwise, and extracted with ethyl acetate (30 mL × 3). The organic phases were combined, washed with saturated brine (20 mL × 2), dried over anhydrous sodium sulfate, and filtered. The filtrate was concentrated under reduced pressure to obtain a crude product, which was purified by flash silica gel column (0-10% ethyl acetate/petroleum ether). Compound **4-8** was obtained. MS: *m/z* 228.0, 230.0 $[M+H]^+$.

Step 6

Compound **4-8** (450 mg, 1.97 mmol) was dissolved in methanol (4.5 mL). Methylamine tetrahydrofuran solution (1 M, 4.25 mL) and zinc chloride (403.4 mg, 2.96 mmol) were added, and the mixture was stirred at 25°C for 2 hours. Sodium cyanoborohydride (186.0 mg, 2.96 mmol) was then added, and the reaction mixture was stirred at 40°C for 12 hours under a nitrogen atmosphere. The reaction mixture was quenched by adding water (10 mL) and then extracted with dichloromethane (10 mL × 4). The organic phases were combined, washed with saturated brine (10 mL), dried over anhydrous sodium sulfate, filtered, and the filtrate was concentrated under reduced pressure to obtain a crude product, which was purified by flash silica gel column (0-10% methanol/dichloromethane). Compound **4-9** was obtained. MS: *m/z* 242.8, 244.8 $[M+H]^+$.

Step 7

Compound **4-9** (100 mg, 411.35 μmol) and trimethylsilylacetylene (404.0 mg, 4.11 mmol) were dissolved in triethylamine (2 mL), then dichlorobis(triphenylphosphine)palladium(II) (57.8 mg, 82.27 μmol) and copper(I) iodide (15.7 mg, 82.27 μmol) were added. The reaction mixture was stirred at 75°C for 3 hours under a nitrogen atmosphere. The reaction mixture was diluted with ethyl acetate (20 mL), filtered, and the filter cake was washed with ethyl acetate (10 mL × 2). The filtrate was collected, and the organic phase was washed with water (10 mL × 2) and saturated brine (10 mL), dried over anhydrous sodium sulfate, filtered, and the filtrate was concentrated under reduced pressure to obtain a crude product, which was purified by flash silica gel column (0-10% methanol/dichloromethane). Compound **4-10** was obtained. MS: *m/z* 260.9 $[M+H]^+$.

Step 8

Compound **4-10** (90 mg, 345.61 μmol) and compound **A** (89.9 mg, 345.61 μmol) were dissolved in *N,N*-dimethylacetamide (1 mL), and then *O*-(7-azabenzotriazol-1-yl)-*N,N,N',N'*-tetramethyluronium hexafluorophosphate (157.7 mg, 414.74 μmol) and *N,N*-diisopropylethylamine (89.3 mg, 691.23 μmol) were added. The reaction mixture was stirred at 25°C for 12 hours. The reaction mixture was added with water (5 mL) and extracted with ethyl acetate (10 mL × 4). The organic phases were combined, washed with water (10 mL × 2) and saturated brine (10 mL), dried over anhydrous sodium sulfate, filtered, and the filtrate was concentrated under reduced pressure to obtain a crude product, which was purified by flash silica gel column (0-3% methanol/dichloromethane). Compound **4-11** was obtained. MS: *m/z* 503.1 $[M+H]^+$.

Step 9

Compound **4-11** (85 mg, 169.12 μmol) was dissolved in methanol (1.7 mL), then potassium carbonate (46.8 mg, 338.23 μmol) was added, and the reaction mixture was stirred at 25°C for 1 hour. The reaction mixture was added with water (5 mL) and extracted with ethyl acetate (10 mL × 3). The organic phases were combined, washed with water (10 mL × 2) and saturated brine (10 mL × 2), dried over anhydrous sodium sulfate, filtered, and the filtrate was concentrated under reduced pressure to obtain compound **4.** MS: *m/z* 431.2 $[M+H]^+$.

Step 10

Compound **4** was purified by SFC (separation conditions, chromatographic column: DAICEL CHIRALPAK IC (250 mm × 30 mm, 10 μm); mobile phase: [phase A: supercritical carbon dioxide, phase B: ethanol (0.1% ammonia water)]; gradient: B%: 60%) to obtain compounds **4a** and **4b.**

Compound **4a:** SFC detection conditions: chromatographic column: Chiralpak IC-3 100 × 4.6 mm I.D., 3 μm; mobile phase: phase A: supercritical carbon dioxide, phase B: ethanol (0.05% diethylamine); gradient: B%: 60%, retention time: 2.298 min, ee = 98.98%. MS: *m/z* 431.0 $[M+H]^+$. $^1H$ NMR (400 MHz, DMSO-d6) $\delta$ ppm 8.28 - 8.39 (m, 2 H) 7.78 (br d, *J*=7.78 Hz, 1 H) 7.57 (br d, *J*=7.28 Hz, 3 H) 7.34 - 7.44 (m, 1 H) 5.84 (br s, 1 H) 4.64 - 4.87 (m, 2 H) 4.40 (br d, *J*=6.53 Hz, 3 H) 4.16 (br s, 2 H) 4.04 (br s, 1 H) 2.67 - 2.81 (m, 3 H).

Compound **4b:** SFC detection conditions: chromatographic column: Chiralpak IC-3 100 × 4.6 mm I.D., 3 μm; mobile phase: phase A: supercritical carbon dioxide, phase B: ethanol (0.05% diethylamine); gradient: B%: 60%, retention time: 3.083 min, ee = 98.78%. MS: *m/z* 431.0 $[M+H]^+$. $^1H$ NMR (400 MHz, DMSO-d6) $\delta$ ppm 8.24 - 8.38 (m, 2 H) 7.77 (br d, *J*=7.78 Hz, 1 H) 7.57 (br d, *J*=7.53 Hz, 1 H) 7.27 - 7.38 (m, 3 H) 5.84 (br s, 1 H) 4.63 - 4.90 (m, 2 H) 4.39 (br d, *J*=5.27 Hz, 3 H) 4.16 (br s, 2 H) 4.04 (br s, 1 H) 2.67 - 2.81 (m, 3 H).

## Example 5

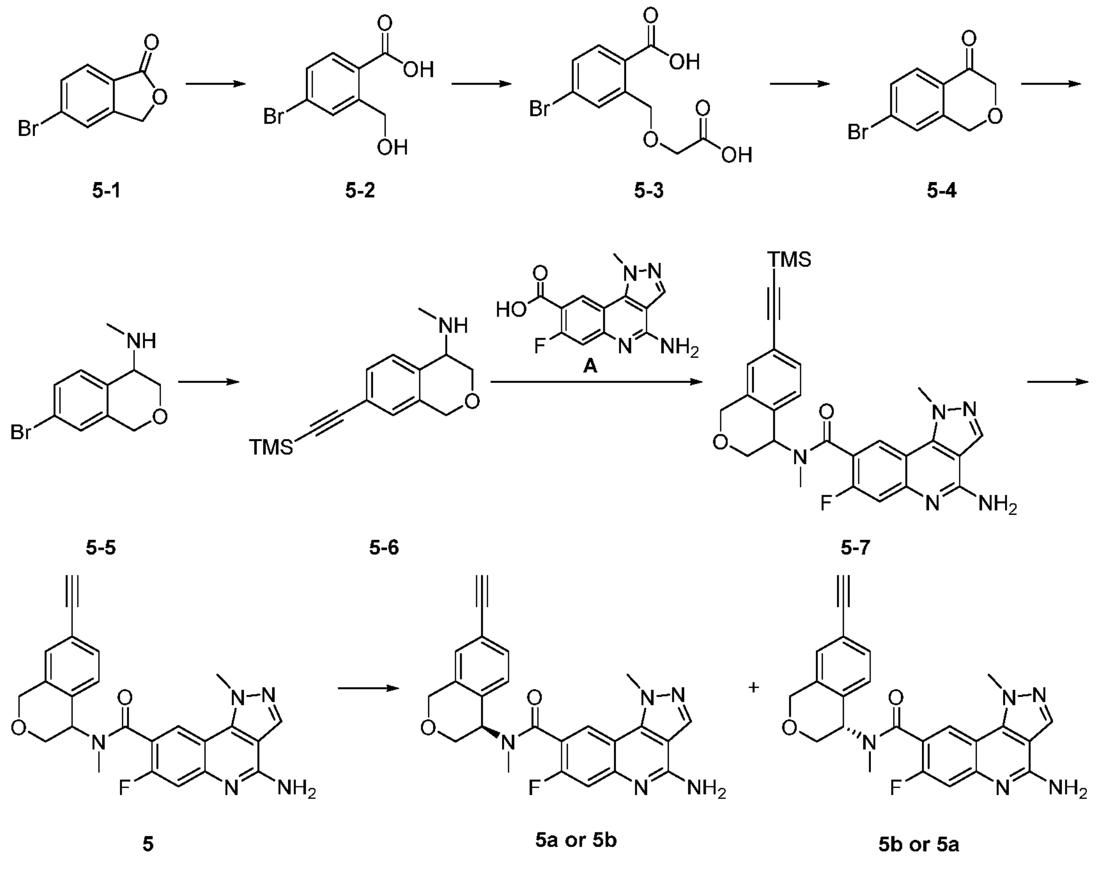

5-1 5-2 5-3 5-4

5-5 5-6 A 5-7

5 5a or 5b + 5b or 5a

Step 1

Compound **5-1** (5 g, 23.47 mmol) was dissolved in tetrahydrofuran (40 mL), methanol (20 mL), and water (20 mL), and then lithium hydroxide monohydrate (2.95 g, 70.41 mmol) was added at 25°C, and the reaction mixture was stirred at 25°C for 12 hours. The reaction mixture was concentrated, diluted with water (30 mL), and then adjusted to pH 3-4 by adding 1 N dilute hydrochloric acid. A solid precipitated, which was filtered, and the filter cake was dried to obtain compound **5-2.** The crude product was directly used in the next step.

Step 2

Compound **5-2** (5 g, 21.64 mmol) and bromoacetic acid (3.31 g, 23.81 mmol) were dissolved in tetrahydrofuran (200 mL). Sodium hydride (3.46 g, 86.56 mmol, purity: 60%) was then added in batches at 25°C, followed by the addition of sodium iodide (324.38 mg, 2.16 mmol). The reaction mixture was heated to 65°C and stirred for another 12 hours. The reaction mixture was cooled to room temperature, quenched by adding water (150 mL), and then washed with methyl *tert*-butyl ether (90 mL × 3). The pH of the aqueous phase was adjusted to 3-4 by adding 1N dilute hydrochloric acid, followed by extraction with ethyl acetate (180 mL × 3). The organic phases were combined, washed with saturated brine (180 mL), dried over anhydrous sodium sulfate, and filtered. The filtrate was concentrated to obtain compound **5-3** and the crude product was used directly in the next step.

Step 3

Compound **5-3** (2 g, 6.92 mmol) was dissolved in acetic anhydride (40 mL), then potassium acetate (2.92 g, 29.75 mmol) was added, and the reaction mixture was stirred at 140°C for 2 hours. The reaction mixture was concentrated, diluted with water (20 mL), and extracted with ethyl acetate (20 mL × 3). The organic phases were combined, washed with saturated brine (20 mL × 2), dried over anhydrous sodium sulfate, filtered, and the filtrate was concentrated to remove the solvent and then dissolved in ethanol (20 mL). Sodium hydroxide (1.11 g, 27.67 mmol) was added, and the reaction mixture was stirred at 40°C for 2 hours. The reaction mixture was directly concentrated, diluted with water (20 mL), and then extracted with dichloromethane (20 mL × 3). The organic phases were combined, washed with saturated brine (20 mL × 2), dried over anhydrous sodium sulfate, filtered, and the filtrate was concentrated to obtain a crude product, which was purified by silica gel column chromatography (0-10% ethyl acetate/petroleum ether) to obtain compound **5-4.** MS: *m*/*z* 226.8, 228.8 $[M+H]^+$. $^1$H NMR (400 MHz, $CD_3OD$) $\delta$ 7.87 (d, *J*=8.28 Hz, 1H), 7.62 (dd, *J*=1.76, 8.28 Hz, 1H), 7.58 (s, 1H), 4.89 (s, 2H), 4.34 (s, 2H).

Step 4

Compound **5-4** (500 mg, 2.20 mmol) was dissolved in methanol (5 mL). Methylamine (2.51 mL) and zinc chloride (450.21 mg, 3.30 mmol) were added, and the mixture was stirred for 0.5 hours. Sodium cyanoborohydride (207.58 mg, 3.30 mmol) was then added, and the reaction mixture was stirred at 20°C for another 48 hours. The reaction mixture was quenched by adding 10% sodium bicarbonate aqueous solution (10 mL), filtered, and then the filtrate was extracted with dichloromethane (20 mL × 3). The organic phases were combined, washed with saturated brine (20 mL × 2), dried over anhydrous sodium sulfate, filtered, and the filtrate was concentrated to obtain a crude product, which was purified by silica gel column chromatography (0-10% methanol/dichloromethane) to obtain compound **5-5.** MS: *m*/*z* 241.8, 243.8 $[M+H]^+$.

Step 5

Compound **5-5** (220 mg, 908.67 μmol) and trimethylsilylacetylene (89.25 mg, 908.67 μmol) were dissolved in triethylamine (2 mL). The mixture was purged with nitrogen three times, followed by the addition of copper(I) iodide (34.61 mg, 181.73 μmol) and dichlorobis(triphenylphosphine)palladium (127.56 mg, 181.73 μmol). The reaction mixture was heated to 75°C under a nitrogen atmosphere and stirred for another 2 hours. After the reaction was completed, the reaction mixture was directly concentrated to obtain a crude product, which was purified by silica gel column chromatography (0-100% ethyl acetate/petroleum ether) to obtain compound **5-6.**

Step 6

Compound **5-6** (50 mg, 192.74 μmol) and compound **A** (50.16 mg, 192.74 μmol) were dissolved in *N,N*-dimethylacetamide (1 mL), and then *O*-(7-azabenzotriazol-1-yl)-*N,N,N',N'*-tetramethyluronium hexafluorophosphate (87.94 mg, 231.29 μmol) and *N,N*-diisopropylethylamine (99.64 mg, 770.96 μmol) were added. The reaction mixture was stirred at 20°C for 12 hours. The reaction mixture was added with water (10 mL) and then extracted with

dichloromethane (10 mL × 3). The organic phases were combined, washed with saturated brine (10 mL × 2), dried over anhydrous sodium sulfate, filtered, and the filtrate was concentrated to obtain compound **5-7.** MS: *m/z* 502.1 [M+H]+.

Step 7

Compound **5-7** (50 mg, 99.68 μmol) was dissolved in methanol (1 mL), then potassium carbonate (41.33 mg, 299.03 μmol) was added, and the reaction mixture was stirred at 20°C for 1 hour. The reaction mixture was added with water (10 mL) and then extracted with dichloromethane (10 mL × 3). The organic phases were combined, washed with saturated brine (10 mL × 2), dried over anhydrous sodium sulfate, filtered, and the filtrate was concentrated to obtain a crude product. The crude product was purified by preparative high performance liquid chromatography (chromatographic column: Welch Xtimate C18 150 × 30 mm × 5 μm; mobile phase: [phase A: water (0.225% formic acid); phase B: acetonitrile]; gradient: B%: 0% to 40%) to obtain compound **5.** *MS: m/z* 430.0 [M+H]+.

Step 8

Compound **5** was purified by SFC (separation conditions, chromatographic column: DAICEL CHIRALPAK AD (250 mm × 30 mm, 10 μm); mobile phase: [phase A: supercritical carbon dioxide, phase B: ethanol (0.1% ammonia water)]; B%: 30%) to obtain compounds **5a** and **5b.**

Compound **5a:** SFC detection conditions: chromatographic column: ChiralPak AD-3 50 × 4.6 mm I.D., 3 μm; mobile phase: phase A: supercritical carbon dioxide, phase B: ethanol (0.05% diethylamine); gradient: B%: 40%, retention time: 3.058 min, ee = 100%. MS: m/z 430.1 [M+H]+. $^1$H NMR (400 MHz, $CD_3OD$) δ ppm8.36 (d, *J*=7.28 Hz, 1H), 8.20-8.21 (m, 1H), 7.20-7.46 (m, 4H), 5.86 (br s, 1H), 4.79-4.83 (m, 1H), 4.67-4.78 (m, 1H), 4.42-4.47 (m, 3H), 4.23-4.29 (m, 1H), 4.11-4.17 (m, 1H), 3.50-3.54 (m, 1H), 2.76-2.94 (m, 3H).

Compound **5b:** SFC detection conditions: chromatographic column: ChiralPak AD-3 50 × 4.6 mm I.D., 3 μm; mobile phase: phase A: supercritical carbon dioxide, phase B: ethanol (0.05% diethylamine); gradient: B%: 40%, retention time: 3.649 min, ee = 95.82%. MS: m/z 430.1 [M+H]+. $^1$H NMR (400 MHz, $CD_3OD$) δ ppm 8.36 (d, *J*=7.03 Hz, 1H), 8.20-8.25 (m, 1H), 7.21-7.45 (m, 4H), 5.86 (br s, 1H), 4.76-4.81 (m, 1H), 4.67-4.73 (m, 1H), 4.41-4.47 (m, 3H), 4.23-4.29 (m, 1H), 4.11-4.17 (m, 1H), 3.51-3.54 (m, 1H), 2.78-2.95 (m, 3H).

## Example 6

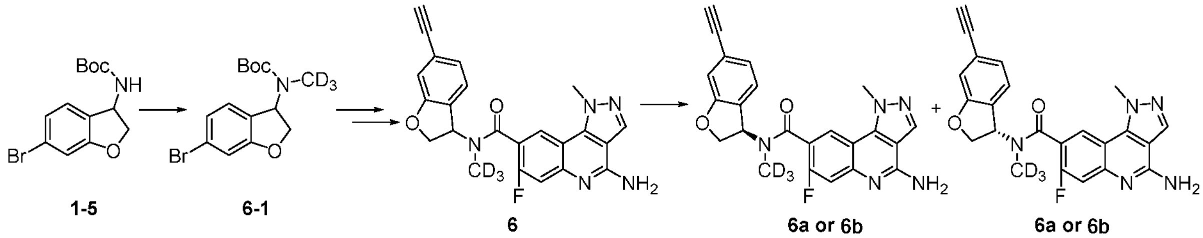

1-5 6-1 6 6a or 6b 6a or 6b

Step 1

Compound **1-5** (100 mg, 318.29 μmol) was dissolved in tetrahydrofuran (2 mL) and cooled to 0°C. Sodium hydride (25.46 mg, 636.59 μmol, purity: 60%) was added under a nitrogen atmosphere and stirred at 0°C for 2 hours. Deuterated iodomethane (92.28 mg, 636.59 μmol) was added dropwise, and the reaction mixture was slowly warmed to 25°C and stirred for 12 hours under a nitrogen atmosphere. The reaction mixture was added with water (10 mL) dropwise and extracted with ethyl acetate (10 mL × 3). The organic phases were combined, washed with saturated brine (10 mL), dried over anhydrous sodium sulfate, and filtered. The filtrate was concentrated under reduced pressure to obtain a crude product, which was purified by flash silica gel column (0-10% ethyl acetate/petroleum ether). Compound **6-1** was obtained.

Step 2

Using compound **6-1** as the starting material, the crude product of compound **6** was prepared with reference to the method in steps 6 to 9 of Example 1. The crude product was then purified by preparative high performance liquid chromatography (chromatographic column: Welch Xtimate C18 150 * 30 mm * 5 μm; mobile phase: [phase A: water (0.225% formic acid); phase B: acetonitrile]; gradient: B%: 8% to 48%) to obtain compound **6.** MS: *m/z* 419.0 [M+H]+.

Step 3

Compound **6** was purified by SFC (separation conditions, chromatographic column: DAICEL CHIRALPAK IG (250 mm × 30 mm, 10 μm); mobile phase: [phase A: supercritical carbon dioxide, phase B: isopropanol (0.1% ammonia water)]; B%: 50%) to obtain compounds **6a** and **6b.**

Compound **6a:** SFC detection conditions: chromatographic column: Chiralpak IG-3 50 × 4.6 mm I.D., 3 μm; mobile phase: phase A: supercritical carbon dioxide, phase B: isopropanol (0.05% diethylamine); gradient: B%: 5 to 40% (0 to 2 min), 40% (2 to 3.2 min), 5% (3.2 to 4 min), retention time: 2.347 min, ee = 99.82%. MS: m/z 419.0 $[M+H]^+$. $^1H$ NMR (400 MHz, $CD_3OD$) $\delta$ ppm 8.35 (d, J=7.25 Hz, 1 H) 8.21 (d, J=2.63 Hz, 1 H) 7.27 - 7.45 (m, 2 H) 7.05 - 7.15 (m, 1 H) 6.87 - 6.99 (m, 1 H) 5.61 - 6.52 (m, 1 H) 4.76 - 4.83 (m, 1 H) 4.65 (dd, J=10.44, 3.69 Hz, 1 H) 4.42 - 4.49 (m, 3 H) 3.51 (d, J=3.75 Hz, 1 H).

Compound 6b: SFC detection conditions: chromatographic column: Chiralpak IG-3 50 × 4.6 mm I.D., 3 μm; mobile phase: phase A: supercritical carbon dioxide, phase B: isopropanol (0.05% diethylamine); gradient: B%: 5 to 40% (0 to 2 min), 40% (2 to 3.2 min), 5% (3.2 to 4 min), retention time: 2.650 min, ee = 97.90%. MS: m/z 419.3 $[M+H]^+$. $^1H$ NMR (400 MHz, $CD_3OD$) $\delta$ ppm 8.34 (br d, J=7.25 Hz, 1 H) 8.21 (d, J=1.75 Hz, 1 H) 7.26 - 7.45 (m, 2 H) 7.04 - 7.16 (m, 1 H) 6.88 - 6.98 (m, 1 H) 5.60 - 6.52(m, 1 H) 4.75 - 4.84 (m, 1 H) 4.64 (br dd, J=10.32, 3.56 Hz, 1 H) 4.42 - 4.50 (m, 3 H) 3.51 (d, J=4.00 Hz, 1 H).

**Example 7**

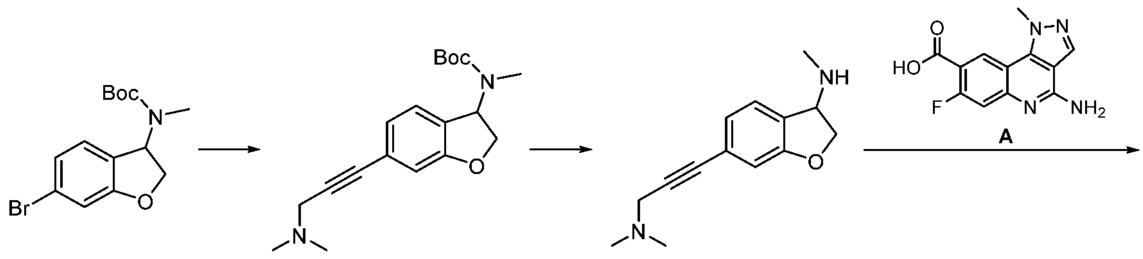


**1-6** **7-1** **7-2**

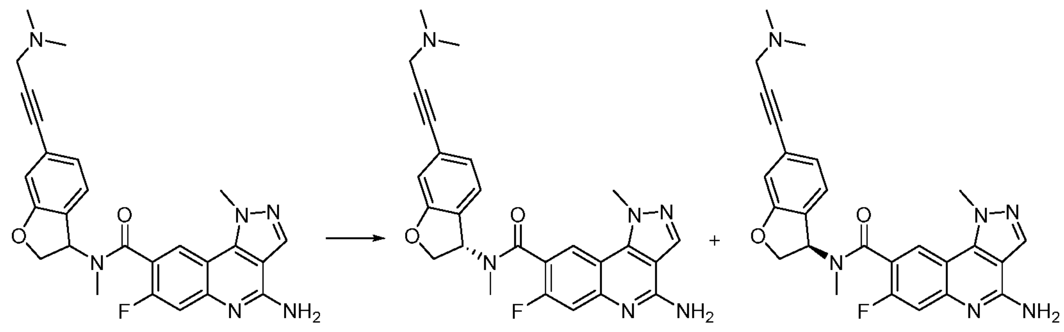

**7** **7a or 7b** **7b or 7a**

Step 1

Compound **1-6** (500 mg, 1.52 mmol) and 1-dimethylamino-2-propyne (1.27 g, 15.23 mmol) were dissolved in triethylamine (10 mL), then dichlorobis(triphenylphosphine)palladium(II) (213.9 mg, 304.69 μmol) and copper(I) iodide (58.0 mg, 304.69 μmol) were added. The reaction mixture was stirred at 75°C for 12 hours under a nitrogen atmosphere. The reaction mixture was diluted with ethyl acetate (20 mL), filtered, and the filter cake was washed with ethyl acetate (20 mL × 2). The filtrate was collected, and the organic phase was washed with water (20 mL × 2) and saturated brine (20 mL), dried over anhydrous sodium sulfate, filtered, and the filtrate was concentrated under reduced pressure to obtain a crude product, which was purified by flash silica gel column (0-70% ethyl acetate/petroleum ether). Compound **7-1** was obtained. MS: *m*/*z* 331.0 $[M+H]^+$.

Step 2

Compound **7-1** (200 mg, 605.29 μmol) was dissolved in a solution of hydrogen chloride in ethyl acetate (4 M, 2 mL). The reaction mixture was stirred at 25°C for 3 hours. The reaction mixture was directly concentrated under reduced pressure, diluted by adding ethyl acetate (1 mL), and concentrated under reduced pressure to obtain the crude hydrochloride of compound **7-2.** MS: *m/z* 230.9 $[M+H]^+$.

Step 3

Compound **7-2** (139 mg, crude hydrochloride) and compound **A** (172.8 mg, 663.90 μmol) were dissolved in *N,N*-dimethylacetamide (2.8 mL), and then *O*-(7-azabenzotriazol-1-yl)-*N,N,N',N'*-tetramethyluronium hexafluorophosphate (275.4 mg, 724.26 μmol) and *N,N*-diisopropylethylamine (195.0 mg, 1.51 mmol) were added. The reaction mixture was stirred at 25°C for 12 hours. The reaction mixture was directly concentrated under reduced pressure to obtain a crude product, which was purified by preparative high performance liquid chromatography (chromatographic column: Phenomenex C18 80 × 40 mm × 3 μm; mobile phase: [phase A: water (0.0125% ammonia water + 10 mM ammonium bicarbonate); phase B: acetonitrile]; gradient: B%: 41% to 71%) to obtain compound **7.** MS: *m/z* 473.0 $[M+H]^+$.

Step 4

Compound 7 was purified by SFC (separation conditions, chromatographic column: DAICEL CHIRALCEL OJ (250 mm × 30 mm, 10 μm); mobile phase: [phase A: supercritical carbon dioxide, phase B: methanol (0.1% ammonia water)]; B%: 40%) to obtain compounds **7a** and **7b.**

Compound **7a:** SFC detection conditions: chromatographic column: Chiralcel OJ-3 150 × 4.6 mm I.D., 3 μm; mobile phase: phase A: supercritical carbon dioxide, phase B: methanol (0.05% diethylamine); gradient: B%: 40%, retention time: 2.008 min, ee = 99.36%. MS: m/z 473.0 $[M+H]^+$. $^1$H NMR (400 MHz, $CD_3OD$) δ ppm 8.34 (br d, *J*=7.38 Hz, 1 H) 8.21 (s, 1 H) 7.23 - 7.46 (m, 2 H) 7.00 - 7.15 (m, 1 H) 6.83 - 6.96 (m, 1 H) 6.48 - 6.52 (m, 0.5 H) 5.58-5.62 (m, 0.5 H) 4.64 (br d, *J*=7.00 Hz, 2 H) 4.40 - 4.50 (m, 3 H) 3.48 (br d, *J*=5.75 Hz, 2 H) 2.67 - 2.85 (m, 3 H) 2.37 (br d, *J*=5.50 Hz, 6 H).

Compound **7b:** SFC detection conditions: chromatographic column: Chiralcel OJ-3 150 × 4.6 mm I.D., 3 μm; mobile phase: phase A: supercritical carbon dioxide, phase B: methanol (0.05% diethylamine); gradient: B%: 40%, retention time: 2.872 min, ee = 97.94%. MS: m/z 473.1 $[M+H]^+$. $^1$H NMR (400 MHz, $CD_3OD$) δ ppm 8.34 (br d, *J*=7.00 Hz, 1 H) 8.21 (s, 1 H) 7.25 - 7.44 (m, 2 H) 7.01 - 7.14 (m, 1 H) 6.84 - 6.96 (m, 1 H) 6.48 - 6.52 (m, 0.5 H) 5.58-5.62 (m, 0.5 H) 4.64 (br d, *J*=6.75 Hz, 2 H) 4.37 - 4.50 (m, 3 H) 3.50 (br d, *J*=6.50 Hz, 2 H) 2.67 - 2.84 (m, 3 H) 2.38 (br d, *J*=5.88 Hz, 6 H).

**Example 8**

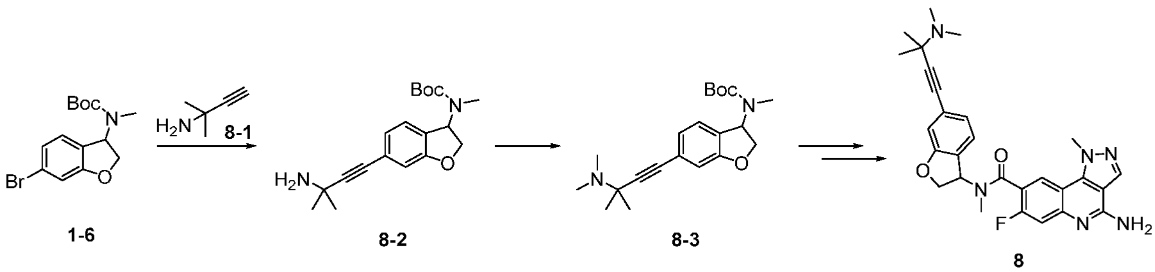


1-6 8-2 8-3 8

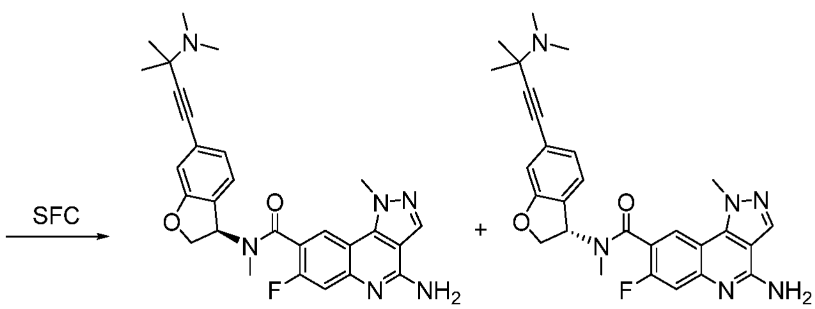


**8a or 8b** **8b or 8a**

Step 1

Compound **1-6** (1 g, 3.05 mmol) and compound **8-1** (2.53 g, 30.47 mmol) were dissolved in triethylamine (10 mL), then dichlorobis(triphenylphosphine)palladium(II) (320.79 mg, 457.04 μmol) and copper(I) iodide (87.04 mg, 457.04 μmol) were added. The reaction mixture was stirred at 75°C for 3 hours under a nitrogen atmosphere. The reaction mixture was diluted with ethyl acetate (20 mL), filtered, and the filter cake was washed with ethyl acetate (10 mL × 2). The collected filtrate was washed with water (10 mL × 2), washed with saturated brine (10 mL), dried over anhydrous sodium sulfate, filtered, and the filtrate was concentrated under reduced pressure to obtain a crude product, which was purified by flash silica gel column (0-20% ethyl acetate/petroleum ether) to obtain compound **8-2.** MS: *m*/*z* 315.1 $[M-NH_2+H]^+$.

Step 2

Under a nitrogen atmosphere, compound **8-2** (300 mg, 907.93 μmol) was dissolved in trifluoroethanol (6 mL), then paraformaldehyde (40.86 mg, 1.36 mmol) was added, and the mixture was stirred at 25°C for 1 hour. Sodium borohydride (51.52 mg, 1.36 mmol) was added, and the reaction mixture was stirred at 80°C for 6 hours. The reaction mixture was cooled to room temperature, quenched by adding water (5 mL) to the reaction mixture, extracted with ethyl acetate (10 mL × 3), washed with saturated brine, dried over anhydrous sodium sulfate, and concentrated to obtain a crude product, which was purified by preparative TLC (petroleum ether: ethyl acetate = 5:1) to obtain compound **8-3.** MS: *m*/*z* 359.2 $[M+H]^+$. $^1H$ NMR (400 MHz, $CDCl_3$) $\delta$ ppm 7.13 (br s, 1H), 7.00 (br dd, *J* = 0.9, 7.7 Hz, 1H), 6.91 - 6.84 (m, 1H), 6.13 - 5.70 (m, 1 H) 4.64 - 4.54 (m, 1H), 4.40 - 4.30 (m, 1H), 2.50 (br s, 3H), 2.34 (s, 6H), 1.48 (s, 9H), 1.44 (s, 6H).

Step 3

Using compound **8-3** as the starting material, compound **8** was prepared with reference to the method in steps 2 to 3 of Example 7. MS: *m*/*z* 501.2 $[M+H]^+$.

Step 4

Compound **8** was purified by SFC (separation conditions, chromatographic column: DAICEL CHIRALCEL OJ (250 mm × 30 mm, 10 μm); mobile phase: [phase A: supercritical carbon dioxide, phase B: methanol (0.1% ammonia water)]; B%: 35%) to obtain compounds **8a** and **8b.**

Compound **8a:** SFC detection conditions: chromatographic column: Chiralcel OD-3 150 × 4.6 mm I.D., 3 μm; mobile phase: phase A: supercritical carbon dioxide, phase B: ethanol (0.05% diethylamine); gradient: B%: 40%, retention time: 1.152 min, ee = 100%. MS: m/z 501.2 $[M+H]^+$. $^1H$ NMR (400 MHz, DMSO-$d_6$) 8.25 (s, 1H), 7.39 - 7.32 (m, 1H), 7.30 (br s, 2H), 7.07 - 6.96 (m, 1H), 6.88 (br d, *J* = 19.3 Hz, 1H), 6.40 - 6.44 (m, 0.5H), 5.58 - 5.50 (m, 0.5H), 4.62 (br s, 1H), 4.39 (br d, *J* = 10.3 Hz, 3H), 2.67 (s, 2H), 2.55 (s, 2H), 2.22 (br s, 6H), 1.35 (br s, 6H).

Compound **8b:** SFC detection conditions: chromatographic column: Chiralcel OD-3 150 × 4.6 mm I.D., 3 μm; mobile phase: phase A: supercritical carbon dioxide, phase B: ethanol (0.05% diethylamine); gradient: B%: 40%, retention time: 1.655 min, ee = 99.04%. MS: m/z 501.2 $[M+H]^+$. $^1H$ NMR (400 MHz, DMSO-$d_6$) $\delta$ ppm 8.26 (br s, 1H), 7.28-7.32 (m, 3H), 7.14 - 6.96 (m, 1H), 6.88 (br d, *J* = 19.3 Hz, 1H), 6.41 - 6.45 (m, 0.5H), 5.52 - 5.46 (m, 0.5H), 4.63 (br s, 1H), 4.39 (br d, *J* = 9.3 Hz, 3H), 2.67 (br s, 2H), 2.55 (br s, 2H), 2.22 (br s, 6H), 1.35 (br s, 6H).

**Example 9**

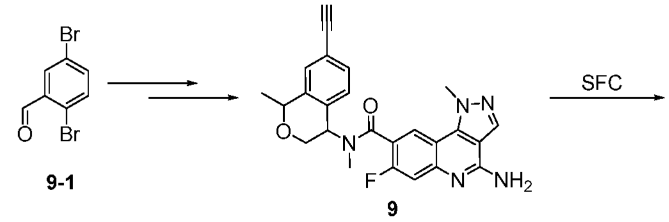


9-1

9

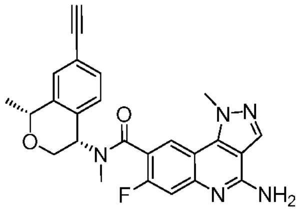

9a or 9b or 9c or 9d

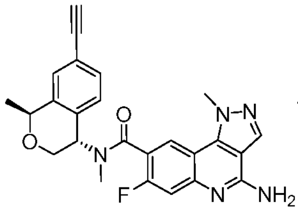

9a or 9b or 9c or 9d

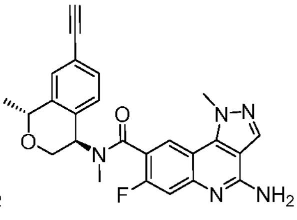

9a or 9b or 9c or 9d

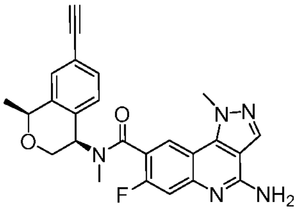

9a or 9b or 9c or 9d

Step 1

Using compound **9-1** as the starting material, the crude product of compound 9 was prepared with reference to the method in steps 2 to 10 of Example 3. The crude product was purified by preparative high performance liquid chromatography (chromatographic column: Welch Xtimate C18 150 × 25 mm × 5 μm; mobile phase: [phase A: water (0.0125% ammonia water + 10 mM ammonium bicarbonate); phase B: acetonitrile]; gradient: B%: 39% to 59%) to obtain compound **9.** MS: *m*/*z* 444.1 $[M+H]^+$.

Step 2

Compound **9** was purified by SFC (separation conditions, chromatographic column: DAICEL CHIRALPAK AD (250 mm × 30 mm, 10 μm); mobile phase: [phase A: supercritical carbon dioxide, phase B: isopropanol (0.1% ammonia water)]; gradient: B%: 35%) to obtain three crude compounds. The retention time of the first crude compound was 1.934 min, the retention time of the second crude compound was 2.278 min, and the retention time of the second crude compound was 2.596 min by SFC detection (chromatographic column: Chiralpak AD-3 150 × 4.6 mm I.D., 3 μm; mobile phase: phase A: supercritical carbon dioxide, phase B: isopropanol (0.05% diethylamine); gradient: B%: 40%).

The first crude compound was then purified by SFC (separation conditions, chromatographic column: ChiralPak IH (250 mm × 30 mm, 10 μm); mobile phase: [phase A: supercritical carbon dioxide, phase B: ethanol (0.1% ammonia water)]; gradient: B%: 30%) to obtain compounds **9a** and **9b.**

The second crude compound was then purified by SFC (separation conditions, chromatographic column: DAICEL CHIRALCEL OD (250 mm × 30 mm, 10 μm); mobile phase: [phase A: supercritical carbon dioxide, phase B: methanol (0.1% ammonia water)]; gradient: B%: 40%) to obtain compound **9c.**

The third crude compound was then purified by SFC (separation conditions, chromatographic column: DAICEL CHIRALPAK AD (250 mm × 30 mm, 10 μm); mobile phase: [phase A: supercritical carbon dioxide, phase B: isopropanol (0.1% ammonia water)]; gradient: B%: 35%) to obtain compound **9d.**

Compound **9a:**

SFC detection condition: chromatographic column: Chiralpak AS-3 150 × 4.6 mm I.D., 3 μm; mobile phase: phase A: supercritical carbon dioxide, phase B: ethanol (0.05% diethylamine); gradient: B%: 5% to 40% (5 to 4 min), 40% to 5% (4 to 4.2 min), 5% (4.2 to 6 min), with a retention time of 3.426 min and a chiral purity of 100%. MS: m/z 444.1 $[M+H]^+$. $^1H$ NMR (400 MHz, $CD_3OD$) $\delta$ ppm 8.35 (d, *J* = 7.3 Hz, 1H), 8.22 - 8.16 (m, 1H), 7.47 - 7.27 (m, 4H), 5.95 (s, 1H), 5.03 - 4.95 (m, 1H), 4.85 - 4.79 (m, 2H), 4.60 (br s, 1H), 4.47 - 4.38 (m, 3H), 3.99 (dd, *J* = 7.0, 11.5 Hz, 1H), 3.52 (s, 1H), 2.87 (s, 1H), 1.57 - 1.37 (m, 3H).

Compound **9b:**

SFC detection condition: chromatographic column: Chiralpak AS-3 150 × 4.6 mm I.D., 3 μm; mobile phase: phase A: supercritical carbon dioxide, phase B: ethanol (0.05% diethylamine); gradient: B%: 5% to 40% (5 to 4 min), 40% to 5% (4 to 4.2 min), 5% (4.2 to 6 min), with a retention time of 3.698 min and a chiral purity of 99.27%. MS: m/z 444.1 $[M+H]^+$. $^1H$ NMR (400 MHz, $CD_3OD$) $\delta$ ppm 8.37 (d, *J* = 7.3 Hz, 1H), 8.23 (s, 1H), 7.51 - 7.34 (m, 4H), 5.80 (d, *J* = 3.3 Hz, 1H),

4.82 - 4.74 (m, 1H), 4.61 (br s, 2H), 4.47 (s, 3H), 4.41 - 4.35 (m, 1H), 4.13 (dd, *J* = 4.0, 12.5 Hz, 1H), 3.58 - 3.52 (m, 1H), 3.00 (s, 1H), 1.60 (d, *J* = 6.5 Hz, 3H).

Compound **9c:**

SFC detection condition: chromatographic column: Chiralpak AD-3 150 × 4.6 mm I.D., 3 μm; mobile phase: phase A: supercritical carbon dioxide, phase B: isopropanol (0.05% diethylamine); gradient: B%: 40%, with a retention time of 2.281 min and a chiral purity of 99.83%. MS: m/z 444.1 $[M+H]^+$. $^1H$ NMR (400 MHz, $CD_3OD$) δ ppm 8.35 (d, *J* = 7.3 Hz, 1H), 8.21 - 8.14 (m, 1H), 7.45 - 7.27 (m, 4H), 5.94 (t, *J* = 6.1 Hz, 1H), 5.00 - 4.92 (m, 1H), 4.44 (s, 2H), 4.40 (s, 1H), 4.32 (dd, *J* = 5.5, 11.8 Hz, 1H), 3.98 (br dd, *J* = 6.9, 11.7 Hz, 1H), 3.52 (s, 1H), 2.89 - 2.71 (m, 3H), 1.57 - 1.37 (m, 3H).

Compound **9d:**

SFC detection condition: chromatographic column: Chiralpak AD-3 150 × 4.6 mm I.D., 3 μm; mobile phase: phase A: supercritical carbon dioxide, phase B: isopropanol (0.05% diethylamine); gradient: B%: 40%, with a retention time of 2.599 min and a chiral purity of 99.29%. MS: m/z 444.1 $[M+H]^+$. $^1H$ NMR (400 MHz, $CD_3OD$) δ ppm 8.37 (d, *J* = 7.0 Hz, 1H), 8.22 (s, 1H), 7.48 - 7.33 (m, 4H), 5.78 (d, *J* = 2.5 Hz, 1H), 4.59 (s, 3H), 4.46 (s, 3H), 4.36 (d, *J* = 12.3 Hz, 1H), 4.12 (dd, *J* = 4.1, 12.7 Hz, 1H), 3.56 - 3.51 (m, 1H), 2.99 (s, 1H), 1.58 (d, *J* = 6.3 Hz, 3H).

## Bioassay data

### Test example 1: LU99 cell proliferation inhibition assay

Experimental purpose: To test the antiproliferative activity of compounds against LU99 cells

Experimental materials: 1640 medium, penicillin/streptomycin antibiotics were purchased from Gibco, and fetal bovine serum was purchased from Biosera. CellTiter-Glo (chemiluminescent cell viability assay reagent) was purchased from Promega. LU99 cell line was purchased from JCRB, and Envision multimode microplate reader (PerkinElmer).

Experimental operation:

1) Cell culture: LU99 cells were seeded into an ultra-low attachment 96-well black-walled plate, with 80 μL of cell suspension per well, containing 1000 LU99 cells. The cell plate was incubated overnight in a $CO_2$ incubator.
2) Administration: The test compound was 5-fold diluted with DMSO across eight concentrations using a pipette, ranging from 2 mM to 25.6 nM, and tested in duplicate. 78 μL of medium was added to an intermediate plate. Subsequently, 2 μL of the serially diluted compound per well was transferred to the corresponding wells of the intermediate plate and mixed well. 20 μL of the mixture per well was then transferred to the cell plate. The concentration of the compound transferred into the cell plate ranged from 10 μM to 0.128 nM. The cell plate was incubated for 6 days in a $CO_2$ incubator. An additional cell plate was prepared to read the signal value on the day of compound addition, which served as the maximum value (Max in the following equation) for data analysis.
3) Cell proliferation and viability detection: 100 μL of chemiluminescent cell viability assay reagent was added to each well of the cell plate and incubated at room temperature for 30 minutes to stabilize the luminescent signal. A multimode microplate reader was used for reading.
4) Data analysis: Using the equation (Sample - Min) / (Max - Min) * 100% to convert the raw data into inhibition rate, the $IC_{50}$ value can be obtained by curve fitting with four parameters (obtained by "log (inhibitor) vs. response -- Variable slope" mode in GraphPad Prism).

Experimental results: The experimental results are shown in Table 3.

Table 3: Results of the antiproliferative activity of the compounds of the present disclosure against LU99 cells

| Compound | Antiproliferative activity $IC_{50}$ (nM) in LU99 cell | Compound | Antiproliferative activity $IC_{50}$ (nM) in LU99 cell |
|---|---|---|---|
| Compound **1a** | 2.9 | Compound **6b** | 5.1 |
| Compound **2a** | 11.7 | Compound **7a** | 0.9 |

(continued)

| Compound | Antiproliferative activity $IC_{50}$ (nM) in LU99 cell | Compound | Antiproliferative activity $IC_{50}$ (nM) in LU99 cell |
|---|---|---|---|
| Compound **3d** | 1.9 | Compound **8a** | 16.1 |
| Compound **4b** | 3.1 | Compound **9d** | 2.3 |
| Compound **5b** | 4.4 | / | / |
| Conclusion: The compounds of the present disclosure exhibit significant antiproliferative activity against LU99 cells. | | | |

**Test example 2: Pharmacokinetic evaluation in mice**

Experimental methods:

The test compound was mixed with 5% DMSO/10% polyethylene glycol-15 hydroxystearate/85% water, vortexed, and sonicated to prepare a clear solution with a concentration of 0.2 mg/mL. The solution was filtered through a microporous membrane and set aside for late use. Balb/c male mice weighing 18 to 20 grams were selected. The candidate compound solution was administered intravenously at a dose of 1 mg/kg. The candidate compound solution was administered orally at a dose of 2 mg/kg. Whole blood was collected for a certain period of time and prepared into plasma. The drug concentration was analyzed by the LC-MS/MS method, and the pharmacokinetic parameters were calculated using Phoenix WinNonlin software (Pharsight, USA).

Definitions of each parameter: IV: Intravenous injection; PO: Oral administration; $C_0$: The plasma concentration immediately after intravenous injection; $C_{max}$: The maximum plasma concentration observed after administration; $T_{max}$: The time required to reach the peak drug concentration after administration; $T_{1/2}$: The time required for the plasma concentration to decrease by half; $V_{dss}$: Apparent volume of distribution, which refers to a proportionality constant describing the ratio of the amount of drug *in vivo* to the plasma concentration when the drug reaches dynamic equilibrium *in vivo*. Cl: Clearance, which refers to the apparent volume of distribution of a drug cleared from the body per unit of time; $T_{last}$: The time of the last detection point; $AUC_{0-last}$: Area under the drug-time curve, representing the area enclosed by the plasma concentration curve and the time axis; F: A measure of the rate and extent to which a drug is absorbed into the blood circulation, serving as an important indicator for evaluating the extent of drug absorption. The experimental results are shown in Table 4.

Table 4: PK test results of compounds of the present disclosure in plasma

| Parameters | | $C_0$ (nM) | $C_{max}$ (nM) | $T_{max}$ (h) | $T_{1/2}$ (h) | $V_{dss}$ (L/kg) | Cl (mL/min/kg) | $T_{last}$ (h) | $AUC_{0-last}$ (nM.h) | F (%) |
|---|---|---|---|---|---|---|---|---|---|---|
| Compound **1a** | IV | 4322 | -- | -- | 1.91 | 0.80 | 5.3 | 8 | 7333 | -- |
| | PO | -- | 1652 | 1.0 | 2.20 | -- | -- | 24 | 9521 | 64.9 |
| Compound **2a** | IV | 4158 | -- | -- | 0.84 | 0.77 | 17.8 | -- | 2296 | -- |
| | PO | -- | 1078 | 0.5 | 1.56 | -- | -- | 8 | 2481 | 54.0 |
| Compound **3d** | IV | 2544 | -- | -- | 1.13 | 1.31 | 25.9 | 8 | 1444 | -- |
| | PO | -- | 750 | 0.8 | 3.09 | -- | -- | -- | 2359 | 83.1 |
| Compound **4b** | IV | 1354 | -- | -- | 0.80 | 2.11 | 39.3 | 6 | 979 | -- |
| | PO | -- | 618 | 0.5 | 2.97 | -- | -- | -- | 2168 | 112.0 |
| Compound **7a** | IV | 1002 | -- | -- | 4.94 | 6.04 | 17.9 | 24 | 1948 | -- |
| | PO | -- | 215 | 2.0 | 6.01 | -- | -- | 24 | 1648 | 43.3 |
| "--" means not tested or data not available.<br>Conclusion: The compound of the present disclosure exhibit long half-life, low clearance, high drug exposure and oral bioavailability with superior *in vivo* pharmacokinetic properties. | | | | | | | | | | |

**Test example 3: Enzyme inhibitory activity assay**

Experimental purpose: To test the inhibitory effect of the compounds of the present disclosure on PRMT5·MTA.

Experimental materials: PRMT5/MEP50, peptide H4 (1-21), LANCE® Ultra Europium-anti-methyl-Histone H4 Arginine 3 (H4R3me) antibody, ULight streptavidin, LANCE detection buffer, SAM (S-adenosylmethionine), porcine skin collagen, and MTA (methylthioadenosine).

Experimental methods:

(1) Buffer preparation: Taking 10 mL as an example. 10 mM MTA: 10 mg of MTA was dissolved in 3296 μL of DMSO. The mixture was then aliquoted and stored at -80°C. The buffer was prepared on the day of the experiment, according to the preparation scheme shown in Table 5.

Table 5: Preparation scheme of buffer

| Component | Storage concentration | Final concentration | Addition volume |
|---|---|---|---|
| Bicine, pH 7.6 | 1000 mM | 20 mM | 200 μL |
| NaCl | 5000 mM | 25 mM | 50 μL |
| DTT | 1000 mM | 2 mM | 20 μL |
| Porcine skin collagen (gelatin) | 0.08% | 0.005% | 625 μL |
| MTA | 10 mM | 2.6 μM | 2.6 μL |
| Tween-20 | 1% | 0.01% | 100 μL |
| Deionized water | - | - | 9002.4 μL |

(1) Compound preparation

The compound was dissolved in DMSO to obtain a stock solution with a concentration of 10 mM. A gradient dilution was performed in a compound dilution plate to obtain four compound wells at the following concentrations: 1 mM, 37.037 μM, 1.3717 μM, and 0.0508 μM. These compounds at four concentrations were then transferred to a compound transfer plate with a transfer volume of 8 μL for each concentration. Additionally, DMSO was added to the empty wells of the compound transfer plate for later use. The Echo550 liquid handler was employed to perform serial dilutions and transfer the liquid to the experimental plate. Upon completion of the liquid transfer, the experimental plate was ready for use.

(2) Reaction

The enzyme solution and substrate mixture were prepared using the buffer, with PRMT5 at a concentration of 7.6 nM, peptide H4 (1-21) at a concentration of 0.32 μM, and SAM at a concentration of 1.3 μM.

5 μL of the PRMT5 solution per well was added to the compound wells and negative control wells of the experimental plate using an electronic multichannel pipette. An equal volume of buffer was added to the positive control wells. The experimental plate was centrifuged at 1000 rpm for 30 seconds gently mixed on an orbital plate shaker for 30 seconds, and then incubated at 25°C for 30 minutes in a temperature-controlled incubator. Subsequently, the substrate mixture was added to the positive control wells, negative control wells, and compound wells of the experimental plate using the same method. The plate was centrifuged and incubated at 25°C for 90 minutes.

(4) Detection and calculation

Principle: The experiment employed PerkinElmer's time-resolved fluorescence resonance energy transfer technology (LANCE® Ultra) for detection. During the reaction, two antibodies were added after PRMT methylated the substrate peptide H4 (1-21). The LANCE® Ultra Europium-anti-methyl-Histone H4 Arginine 3 (H4R3me) antibody acted as an energy donor and specifically bound to the methylation site on the peptide H4 (1-21), while ULight acted as an energy acceptor and specifically bound to the biotin tag on the peptide H4 (1-21). When excited with a laser at a specific wavelength (the excitation wavelength in this experiment was 340 nm), the energy donor emitted light at a wavelength of 615 nm. If the spatial distance between the energy donor and the energy acceptor was close enough (i.e., both antibodies were simultaneously attached to the peptide H4 (1-21)), energy transfer occurred between the energy donor and the

energy acceptor, causing the energy acceptor to emit light at a wavelength of 665 nm. The emitted lights at both 615 nm and 665 nm were detected using a plate reader, and the ratio of the 665 nm signal to the 615 nm signal was calculated. By plotting and calculation, the relevant parameters of the sample to be tested could be determined.

An antibody mixture was prepared using LANCE buffer, with a concentration of 4 nM for LANCE® Ultra Europium-anti-methyl-Histone H4 Arginine 3 (H4R3me) antibody and 53.3 nM for ULight. 10 μL of the detection solution per well was added to the positive control wells, negative control wells, and compound wells of the experimental plate using an electronic multichannel pipette. The plate was centrifuged and incubated at room temperature for 1 hour, and an Envision 2104 plate reader was used for reading. The inhibition rate of the compounds was calculated using interpolation. The inhibition rate was plotted using the four-parameter logistic curve and XLfit software to generate the inhibition curve of the compounds and calculate relevant parameters, including minimum inhibition rate, maximum inhibition rate, and $IC_{50}$. The formula for calculating the inhibition rate is follows:

$$\text{Single-well inhibition rate} = \left(1 - \frac{\text{Single-well signal value - Mean signal value of the positive control}}{\text{Mean signal value of the negative control - Mean signal value of the positive control}}\right) \times 100\%$$

Experimental results: The experimental results are shown in Table 6.

Table 6: Inhibitory activity results of compounds of the present disclosure on PRMT5·MTA

| Compound | PRMT5-MTA inhibitory activity $IC_{50}$ (nM) |
|---|---|
| Compound **1a** | 1.8 |
| Compound **3d** | 2.2 |
| Compound **4b** | 2.2 |
| Conclusion: The compounds of the present disclosure exhibit significant inhibitory activity against PRMT5·MTA. | |

**Test example 4: *In vitro* detection of SDMA levels in HCT116 WT/MTAP KO cells**

Experimental materials:

RPMI-1640 medium was purchased from GIbco, penicillin/streptomycin antibiotics were purchased from Wisent, and fetal bovine serum was purchased from Biosera. Protein primary antibody and fluorescent secondary antibody were purchased from Cell Signaling Technology. HCT116 WT cell line was purchased from Nanjing Cobioer Biosciences Co., Ltd. The HCT116 MTAP KO cells were purchased from Wuhan Heyan Biomedical Technology Co., Ltd. The 4% paraformaldehyde (PFA) fixative solution, Triton X-100, etc. were purchased from Beyotime Biotechnology. Envision multilabel plate reader (PerkinElmer).

Experimental methods:

Cells were seeded into a 96-well black-walled plate, with 80 μL of cell suspension per well, containing 2000 cells. The cell plate was incubated overnight in a $CO_2$ incubator.

The test compound was serially diluted 5-fold across eight concentrations using a pipette, ranging from 2 mM to 25.6 nM, and tested in duplicate. 78 μL of medium was added to an intermediate plate. Subsequently, 2 μL of the serially diluted compound per well was transferred to the corresponding wells of the intermediate plate and mixed well. 20 μL of the mixture per well was then transferred to the cell plate. The concentration of the compound transferred into the cell plate ranged from 10 μM to 0.128 nM. The cell plate was placed in a $CO_2$ incubator and cultured for 4 days, and then the cells were removed, and the medium was discarded. The cells were fixed with 4% paraformaldehyde fixative solution for 30 minutes, washed three times with PBS (phosphate buffered saline), permeabilized with 0.1% Triton for 20 minutes, washed three times with PBS, blocked with 0.1% BSA (bovine serum albumin) solution at room temperature for 1 hour, incubated with protein primary antibody (anti-SDMA or anti-β-actin, diluted 1:1000) at 4°C overnight, washed 3 times with PBST (phosphate buffered saline + 0.1% Tween 20), incubated with fluorescent secondary antibody (FITC or Cy5 labeled, diluted 2:1000) at room temperature for 2 hours. After washing three times with PBST, the plate was read using an Envision plate reader (FITC and Cy5).

Data analysis:

The fluorescence intensity was measured to calculate the FITC/Cy5 ratio for each sample well and the inhibition rate (%) under compound treatment. The calculation formulas are as follows:

$$\text{FITC / Cy5} = (\text{FITCsample - FITCblank}) / (\text{FITCsample - FITCblank})$$

$$\text{inhibition\%} = [1 - (\text{FITC / Cy5comp.}) / (\text{FITC / Cy5DMSO})] * 100$$

The $IC_{50}$ values were then determined by four-parameter curve fitting (obtained using the "log(inhibitor) vs. response -- Variable slope" mode in GraphPad Prism). The experimental results are shown in Table 7.

Table 7: Results of *in vitro* SDMA level screening experiment for compounds of the present disclosure

| **Compound No.** | **SDMA $IC_{50}$ (nM)** | |
|---|---|---|
| | **HCT116 WT** | **HCT116 MTAP KO** |
| Compound **1a** | 53.1 | 0.2 |
| Compound **3d** | 10.7 | 1.2 |
| Compound **4b** | 44.0 | 0.1 |
| Compound **7a** | 93.5 | 0.1 |
| Conclusion: The compounds of the present disclosure can significantly inhibit the SDMA level in MTAP-deficient tumor cells, while the inhibitory activity on the SDMA level in MTAP-non-deficient cells is weak, showing excellent selectivity. | | |

**Test example *5: In vitro* liver microsomal metabolic stability assay of compounds**

Experimental materials:

Liver microsomes: purchased from Corning or Xenotech and stored in a -80°C freezer. Reduced nicotinamide adenine dinucleotide phosphate (NADPH), supplier: Chem-Impex International, Cat. No.: 00616. Control compounds: Testosterone, Diclofenac, Propafenone.

Experimental steps:

(1) Preparation of working solution

Stock solution: 10 mM DMSO solution. Preparation of working concentration: The stock solution was diluted to 100 μM using 100% acetonitrile (organic phase content: 99% acetonitrile, 1% DMSO).

(2) Experimental steps

Two 96-well incubation plates were prepared and named as T60 incubation plate and NCF60 incubation plate.

445 μL of microsomal working solution (liver microsomal protein concentration of 0.56 mg/mL) was added to both the T60 incubation plate and the NCF60 incubation plate. The two incubation plates were pre-incubated in a 37°C water bath for approximately 10 minutes.

After pre-incubation, 5 μL of the test compound or control compound working solution was added to both the T60 incubation plate and the NCF60 incubation plate and mixed well. 50 μL of potassium phosphate buffer per well was added to the NCF60 incubation plate to initiate the reaction. 180 μL of termination solution (acetonitrile solution containing 200 ng/mL tolbutamide and 200 ng/mL labetalol) and 6 μL of NADPH regeneration system working solution were added to a T0 termination plate. 54 μL of sample was then removed from the T60 incubation plate and added to the T0 termination plate to generate T0 samples. 44 μL of NADPH regeneration system working solution per well was added to the T60 incubation plate to initiate the reaction. Only 54 μL of microsomal working solution, 6 μL of NADPH regeneration system working solution, and 180 μL of termination solution were added to the blank plate. Therefore, in the samples containing the test compounds or control compounds, the final concentrations of the compounds, testosterone, diclofenac, and propafenone in the reaction were 1 μM, the concentration of liver microsomes was 0.5 mg/mL, and the final concentrations of DMSO and

acetonitrile in the reaction system were 0.01% (v/v) and 0.99% (v/v), respectively.

After incubating for an appropriate period (*e.g.,* 5, 15, 30, 45, and 60 minutes), 180 μL of termination solution (acetonitrile solution containing 200 ng/mL tolbutamide and 200 ng/mL labetalol) was added to the sample wells of each termination plate, and then 60 μL of sample was removed from the T60 incubation plate to terminate the reaction.

All sample plates were shaken well and centrifuged at 3220 × g for 20 minutes. 80 μL of supernatant from each well was then diluted to 240 μL with pure water for liquid chromatography-tandem mass spectrometry analysis. The experimental results are shown in Table 8.

Table 8: Liver microsomal stability test results of the compound of the present disclosure

| Compound | Human liver microsomal stability $CL_{int}$ (mL/min/kg) |
|---|---|
| Compound **1a** | 17.6 |
| Compound **2a** | 9.2 |
| Compound **3d** | 28.6 |
| Compound **4b** | 14.3 |
| Compound **5b** | 48.0 |
| Conclusion: The compounds of the present disclosure exhibit moderate metabolism in the human liver microsome test and have good metabolic stability. | |

**Test example 6: Plasma protein binding rate assay of compounds**

Experimental purpose: To evaluate the protein binding rate of the compounds of the present disclosure in the plasma of CD-1 mouse, SD rat, beagle dogs, and human using equilibrium dialysis.

Experimental scheme:

Blank plasma (796 μL) from humans, beagle dogs, SD rats, and CD-1 mice (purchased from BioreclamationIVT) was taken, and the working solution of the test compound or the working solution of warfarin was added to achieve a final concentration of 2 μM for both the test compound and warfarin in the plasma samples. The samples were thoroughly mixed. The final concentration of the organic phase DMSO was 0.5%. 50 μL of the test compound and warfarin plasma samples were transferred to the sample receiving plate, and the corresponding volume of blank plasma or buffer was immediately added to ensure a final volume of 100 μL per well, with a plasma-to-dialysis buffer volume ratio of 1:1. A stop solution was then added to these samples, which served as $T_0$ samples for recovery and stability determination. The test compound and warfarin plasma samples were added to the administration side of each dialysis well, and blank dialysis buffer was added to the corresponding receiving side of the dialysis well. The dialysis plate was then sealed with a gas-permeable membrane and placed in a humidified 5% $CO_2$ incubator, incubated with shaking at 37°C and 100 rpm for 4 hours. After dialysis, 50 μL of the dialyzed buffer samples and dialyzed plasma samples were transferred to a new sample receiving plate. The corresponding volume of blank plasma or buffer was added to the samples to ensure a final volume of 100 μL per well, with a plasma-to-dialysis buffer volume ratio of 1:1. All samples were subjected to protein precipitation followed by LC/MS/MS analysis. The unbound fraction of the compound was calculated using the following formula: PPB_Unbound (%) = $100 * F_C / T_C$, where $F_C$ is the concentration of the compound in the buffer side of the dialysis plate; $T_C$ is the concentration of the compound in the plasma side of the dialysis plate; and $T_0$ is the concentration of the compound in the plasma sample at time zero. The experimental results are shown in Table 9. Herein, H stands for human, R stands for rat, M stands for mouse, and D stands for dog.

Table 9: Results of plasma protein binding rate of the compounds of the present disclosure

| Compound | PPB_unbound(%) |
|---|---|
| Compound **1a** | 0.6(M), 0.5(R), 1.5(D), 1.4(H) |
| Compound **2a** | 7.8(M), 10.4(H) |
| Compound **3d** | 3.4(M), 6.9(R), 4.3(D), 5.8(H) |
| Compound **4b** | 10.3(M), 13.5(R), 17.2(D), 7.7(H) |
| Compound **5b** | 0.9(M), 1.4(H) |

(continued)

| Compound | PPB_unbound(%) |
|---|---|
| Compound **7a** | 3.5(M), 3.4(H) |
| Conclusion: The compounds of the present disclosure exhibit moderate proportions of free drug concentration in plasma across different species, demonstrating good druggability. | |

**Test example 7: Antitumor activity test of the compound in a BALB/c nude mouse subcutaneous xenograft tumor model**

Experimental purpose: To evaluate the tumor inhibitory effect of the compounds of the present disclosure in a subcutaneous xenograft tumor model of human large cell lung cancer LU99.

Experimental methods:

Female BALB/c nude mice were subcutaneously inoculated with the human large cell lung cancer LU99 cell line. After inoculation, the mice were randomly divided into groups based on body weight and tumor volume, with six animals per group.

Dosing regimen 1: Dosing began when the tumor volume reached 150-190 $mm^3$ after inoculation. The vehicle was 5% DMSO/10% Solutol/85% double-distilled water. Control group: The vehicle was administered orally (p.o.) once daily (QD) at a dose of 0.1 mL/10 g; Treatment group: The test compound was dissolved in the vehicle and administered orally once daily at doses of 7.5 mg/kg or 15 mg/kg.

Dosing regimen 2: Dosing began when the tumor volume reached 110-150 $mm^3$ after inoculation. The vehicle was 5% DMSO/10% Solutol/85% double-distilled water. Control group: The vehicle was administered orally (p.o.) once daily (QD) at a dose of 0.1 mL/10 g; Treatment group: The test compound was dissolved in the vehicle and administered orally once daily at doses of 5 mg/kg, 15 mg/kg, or 50 mg/kg.

After the experimental grouping, the mice were weighed twice weekly, and the tumor diameters were measured using a vernier caliper. The tumor volume was calculated using the formula: tumor volume = long diameter × wide diameter$^2$ / 2. The tumor growth inhibition rate (TGI) was then calculated using the formula: TGI (%) = $[1 - (T_i - T_0) / (C_i - C_0)] \times 100$, where $T_i$ is the average tumor volume of a given treatment group on a given day, $T_0$ is the average tumor volume of the treatment group at the start of administration, $C_i$ is the average tumor volume of the control group on a given day (the same day as $T_i$), and $C_0$ is the average tumor volume of the control group at the start of administration.

On day 19 after experimental administration, the mice were euthanized and sampled.

Experimental results:

The body weight change curves of mice in each group are shown in FIGS. 9 to 10, and the average tumor volumes of each group at different time points are shown in FIGS. 11 to 12. The TGI was calculated based on the average tumor volume on day 19 after administration. The specific experimental results are shown in Table 10.

Table 10: Evaluation of tumor inhibitory efficacy of the compounds of the present disclosure on subcutaneous xenograft tumor model of LU99 cell

| **Group** | | **Average tumor volume on day 19 after administration ($mm^3$)** | **TGI (%)** |
|---|---|---|---|
| Dosing regimen 1 | Control group | 2137 | / |
| | Treatment group (compound **1a**, 7.5 mpk) | 435 | 87 |
| | Treatment group (compound **1a**, 15 mpk) | 178 | 100 |

(continued)

| Group | | Average tumor volume on day 19 after administration (mm$^3$) | TGI (%) |
|---|---|---|---|
| Dosing regimen 2 | Control group | 2158 | / |
| | Treatment group (compound **3d,** 5 mpk) | 772 | 68 |
| | Treatment group (compound **3d,** 15 mpk) | 264 | 93 |
| | Treatment group (compound **3d,** 50 mpk) | 91 | 102 |
| | Treatment group (compound **4b,** 5 mpk) | 453 | 84 |
| | Treatment group (compound **4b,** 15 mpk) | 345 | 89 |
| | Treatment group (compound **4b,** 50 mpk) | 72 | 103 |

Experimental conclusion: The compounds of the present disclosure exhibit excellent anti-tumor efficacy *in vivo,* and the body weight of mice is well maintained after administration.

**Test example 8: *In vitro* MDCKII-MDR1 monolayer cell permeability test**

Experimental purpose: The permeability and efflux ratio of the compound of the present disclosure were evaluated using the MDCKII-MDR1 monolayer cell test system to determine the potential of the compound to cross the blood-brain barrier and the potential to be effluxed by the P-GP transporter.

Experimental methods:

MDR1-MDCKII cells (sourced from the Netherlands Cancer Institute) were seeded onto 96-well plates (from Corning) at a cell density of $2.5 \times 10^5$ cells/mL. The cells were cultured for 4-7 days to form confluent cell monolayers. Hank's balanced salt buffer containing 10 mM 4-(2-hydroxyethyl)piperazine-1-ethanesulfonic acid (pH 7.40 $\pm$ 0.05) was used as the transport buffer. The bidirectional transport of the test compound was assessed at a concentration of 50 $\mu$M, with the DMSO concentration in the incubation system kept below 1%. After adding the samples, the cell plates were incubated at 37 $\pm$ 1°C, under 5% $CO_2$ and saturated humidity, for 150 minutes. All samples were subjected to quantitative analysis using the LC-MS/MS method. The apparent permeability coefficient ($P_{app}$, cm/s) and efflux ratio were calculated using the following formula.

The apparent permeability coefficient ($P_{app}$, cm/s) was calculated using the following formula: $P_{app} = (dC_r / d_t) \times V_r / (A \times C_0)$, where $dC_r/d_t$ represents the cumulative concentration of the compound on the receiving side over unit time ($\mu$M/s); $V_r$ is the volume of the solution on the receiving side (with the solution volumes on the apical and basolateral sides being 0.075 mL and 0.250 mL, respectively); A is the relative surface area of the cell monolayer (0.0804 cm$^2$); and $C_0$ is the initial concentration of the test compound (nM) or the peak area ratio of the reference compound on the administration side. The efflux ratio was calculated using the following formula: Efflux Ratio = $P_{app}(BA) / P_{app}(AB)$. The experimental results are shown in Table 11.

Table 11: Results of MDCKII-MDR1 cell permeability test

| Compound | Apparent permeability coefficient (A to B, $10^{-6}$ cm/s) | Efflux ratio |
|---|---|---|
| Compound **1a** | 6.38 | 1.63 |
| Compound **3d** | 9.39 | 2.11 |
| Compound **4b** | 8.15 | 2.84 |
| Compound **5b** | 4.46 | 4.02 |
| Compound **7a** | 6.04 | 2.29 |
| Compound **8a** | 3.51 | 0.96 |
| Compound **9d** | 3.10 | 0.93 |

Conclusion: The compounds of the present disclosure have good permeability in the permeability assay in MDCKII-MDR1 monolayer cells (highly expressing human p-gp).

**Test example 9: hERG Potassium channel inhibition assay**

Experimental purpose: To detect the effects of the compounds of the present disclosure on the hERG potassium ion channel using the electrophysiological manual whole-cell patch-clamp method.

Experimental materials:

Extracellular solution composition (mM): 140 sodium chloride, 5 potassium chloride, 1 calcium chloride, 1.25 magnesium chloride, 10 HEPES, and 10 glucose, with the pH adjusted to 7.4 using sodium hydroxide; Intracellular solution composition (mM): 140 potassium chloride, 1 magnesium chloride, 1 calcium chloride, 10 EGTA, and 10 HEPES, with the pH adjusted to 7.2 using potassium hydroxide; HEPES: 4-(2-Hydroxyethyl)piperazine-1-ethanesulfonic acid, *N*-(2-hydroxyethyl)piperazine-N'-(2-ethanesulfonic acid); EGTA: Ethylene glycol-bis(2-aminoethyl ether)-*N,N,N',N'*-tetraacetic acid.

Experimental methods:

(1) Cell culture

CHO cells stably expressing hERG were cultured in 35 mm cell culture dishes at a seeding density of 300,000 cells per dish and incubated at 37°C with 5% $CO_2$. The cells were passaged every 48 hours at a ratio of 1:5. The medium composition: 90% F12, 10% fetal bovine serum, 100 μg/mL G418, and 100 μg/mL Hygromycin B. On the day of the experiment, the cell culture was removed, and the cells were rinsed once with extracellular solution. Then, 0.25% Trypsin-EDTA solution was added, and the cells were digested at room temperature for 3-5 minutes. The digestion solution was aspirated, and the cells were resuspended in extracellular solution before being transferred to laboratory dishes for electrophysiological recordings and later use.

(2) Preparation of test samples

The test compound was prepared as a 15.00 mM stock solution using DMSO. On the test day, the stock solution of the test compound was serially diluted 3-fold using DMSO. That is, 10 μL of the stock solution of the test compound was added to 20 μL of DMSO, resulting in intermediate concentrations of the test compound serially diluted in DMSO to 5.00, 1.67, 0.56, 0.19, 0.062, and 0.021 mM, respectively. Subsequently, 10 μL of the intermediate concentration of the test compound was added to 4990 μL of extracellular solution, diluted 500-fold to obtain the final concentrations required for testing. The highest test concentration was 10.00 μM, with subsequent concentrations of 10.00, 3.33, 1.11, 0.37, 0.12, and 0.041 μM.

Preparation of positive control cisapride: A 150.00 μM cisapride stock solution was serially diluted 3-fold using 100% DMSO. That is, 10 μL of the 150.00 μM cisapride stock solution was added to 20 μL of DMSO, resulting in five intermediate concentrations of cisapride serially diluted in DMSO to 150.00, 50.00, 16.67, 5.56, and 1.85 μM, respectively. Subsequently, 10 μL of the intermediate concentration of cisapride was added to 4990 μL of extracellular solution, diluted 500-fold to obtain the final concentrations required for testing. The highest test concentration was 300.00 nM, followed by 300.00, 100.00, 33.33, 11.11, and 3.70 nM for a total of 5 concentrations. The final test concentrations contained no more than 0.2% DMSO, a level that did not affect the hERG potassium channel current.

(3) Electrophysiological recording process

CHO cells stably expressing the hERG potassium channel were used to record hERG potassium channel currents at room temperature using the whole-cell patch-clamp technique. The glass microelectrode prepared by pulling a glass electrode blank (BF150-86-10, Sutter) using a puller. After filling the electrode with internal solution, the tip resistance was approximately 2-5 MΩ. The glass microelectrodes were inserted into the amplifier probe and connected to the Axopatch 200B patch-clamp amplifier (Molecular Devices). The clamp voltage and data recording were controlled and recorded by computer using pClamp 10 software, with a sampling frequency of 10 kHz and a filter frequency of 2 kHz. After establishing whole-cell recordings, the cells were clamped at -100 mV, and the step voltage to induce the hERG potassium current (*I* hERG) was a 2-second depolarizing voltage from -100 mV to +20 mV, followed by repolarization to -50 mV for 1 second before returning to -100 mV. This voltage stimulus was applied every 5 seconds to detect the peak of hERG potassium current at -50 mV during repolarization. Once the hERG potassium current stabilized (after 1 minute), drug administration began. The test compound concentration was continuously administered by gravity starting from the lowest test concentration, with each concentration being applied for at least 1 minute until reaching inhibition steady-state or for a maximum of 3 minutes. Each concentration of the test compound was tested in at least 3 cells ($n \geq 3$), while each

concentration of the positive control was tested in at least 2 cells ($n \geq 2$).

(4) Data processing

Data analysis and processing were performed using pClamp 10, GraphPad Prism 8, and Excel software. The inhibition of hERG potassium current (peak hERG tail current induced at -50 mV) at different test compound concentrations was calculated using the following formula:

$$\text{Inhibition\%} = [1 - (I / I\text{o})] \times 100\%$$

where Inhibition% represents the percentage of inhibition of the hERG potassium current by the test compound, and *I* and *Io* represent the amplitudes of the hERG potassium current after and before drug administration, respectively.

The $IC_{50}$ of the test compound was calculated using GraphPad Prism 8 software by fitting the following equation:

$$Y = \text{Bottom} + (\text{Top - Bottom}) / (1 + 10\text{^}((\text{LogIC}_{50} - X)) \times \text{HillSlope}))$$

where X is the logarithmic value of the test compound detection concentration, Y is the percentage of inhibition at the corresponding concentration, Bottom and Top are the minimum and maximum percentage of inhibition, respectively, and HillSlope is the slope of the curve.

The hERG $IC_{50}$ values of the compounds of the present disclosure are shown in Table 12.

Table 12: hERG $IC_{50}$ values of the compounds of the present disclosure

| Test sample | hERG $IC_{50}$ (μM) |
|---|---|
| Compound **3d** | 17.4 |
| Compound **4b** | 24.5 |
| **[0626]** Conclusion: The compounds of the present disclosure have weak inhibition on the hERG potassium current and a low risk of cardiotoxicity. | |

**Claims**

**1.** A compound of formula (III) or a pharmaceutically acceptable salt thereof,

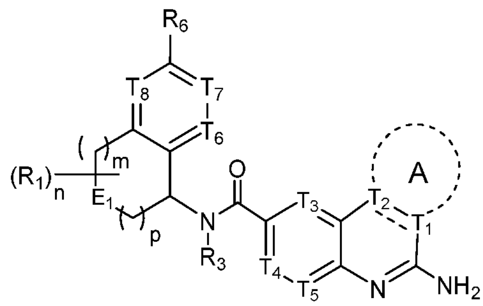

(III)

,

wherein

$E_1$ is selected from $CH_2$, NH, and O;
the structural moiety

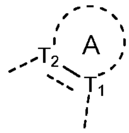

is selected from

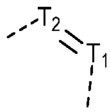 , 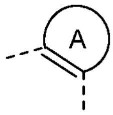 , 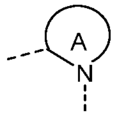 ,

and

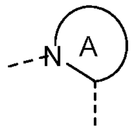 ;

each $R_1$ is independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_a$;

$R_6$ is selected from $C_{2-4}$ alkynyl and $C_{2-4}$ alkenyl, and the $C_{2-4}$ alkynyl and $C_{2-4}$ alkenyl are each independently and optionally substituted by 1, 2, or 3 $R_b$;

alternatively, $R_6$ is selected from halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_b$, in which case the structural moiety

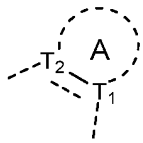

is selected from

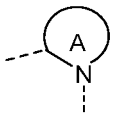 and 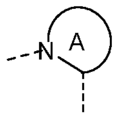 ;

ring A is selected from 5- to 6-membered heteroaryl, $C_{4-6}$ cycloalkenyl, or 5- to 6-membered heterocycloalkenyl, and each 5- to 6-membered heteroaryl, $C_{4-6}$ cycloalkenyl, or 5-to 6-membered heterocycloalkenyl is independently and optionally substituted by 1, 2, or 3 $R_c$;

$T_1$ and $T_2$ are each independently selected from $CR_7$ and N;

$T_3$, $T_4$, and $T_5$ are each independently selected from $CR_4$ and N;

$T_6$, $T_7$, and $T_8$ are each independently selected from $CR_5$ and N;

$R_3$ is selected from H, $C_{1-3}$ alkyl, $C_{3-6}$ cycloalkyl, 4- to 6-membered heterocycloalkyl, -$C_{1-3}$ alkyl-$C_{3-6}$ cycloalkyl, and -$C_{1-3}$ alkyl-4- to 6-membered heterocycloalkyl, and the $C_{1-3}$ alkyl, $C_{3-6}$ cycloalkyl, 4- to 6-membered heterocycloalkyl, -$C_{1-3}$ alkyl-$C_{3-6}$ cycloalkyl, and -$C_{1-3}$ alkyl-4- to 6-membered heterocycloalkyl are each independently and optionally substituted by 1, 2, or 3 $R_e$;

$R_4$, $R_5$, and $R_7$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 $R_d$;

$R_a$, $R_c$, $R_d$, and $R_e$ are each independently selected from H, D, halogen, OH, $NH_2$, $CH_3$, and $CD_3$;

each $R_b$ is independently selected from H, D, halogen, OH, $NH_2$, $C_{1-3}$ alkyl, $C_{1-3}$ alkoxy, $C_{1-3}$ alkylamino, and 4- to 6-membered heterocycloalkyl, and the $C_{1-3}$ alkyl, $C_{1-3}$ alkoxy, and $C_{1-3}$ alkylamino are each independently and optionally substituted by 1, 2, or 3 R;

each R is independently selected from H, D, halogen, OH, $NH_2$, $CH_3$, $CF_3$, and $CD_3$;

n is selected from 0, 1, 2, and 3;

m is selected from 0, 1, and 2;
p is selected from 0, 1, and 2, and m and p are not simultaneously 0;
"hetero" in the 4- to 6-membered heterocycloalkyl, 5- to 6-membered heteroaryl, or 5- to 6-membered heterocycloalkenyl represents 1, 2, 3, or 4 heteroatoms or heteroatom groups independently selected from -O-, -NH-, -S-, and N.

**2.** The compound or the pharmaceutically acceptable salt thereof according to claim 1, wherein each $R_b$ is independently selected from H, D, F, Cl, OH, $NH_2$, $CH_3$, $CH_2CH_3$, $OCH_3$, $OCH_2CH_3$, $NHCH_3$, $NHCH_2CH_3$, $N(CH_3)_2$, pyrrolidinyl, piperidinyl, piperazinyl, and morpholinyl, and the $CH_3$, $CH_2CH_3$, $OCH_3$, $OCH_2CH_3$, $NHCH_3$, $NHCH_2CH_3$, $N(CH_3)_2$, pyrrolidinyl, piperidinyl, piperazinyl, and morpholinyl are each independently and optionally substituted by 1, 2, or 3 R; alternatively, each $R_b$ is independently selected from H, D, F, Cl, OH, $NH_2$, $CH_3$, $CD_3$, $CF_3$, $-C(CH_3)_2OH$, $N(CH_3)_2$,

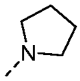, 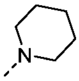, and 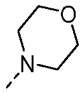.

**3.** The compound or the pharmaceutically acceptable salt thereof according to claim 1, wherein $R_1$ is selected from H, F, Cl, Br, I, $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$, and the $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$ are each independently and optionally substituted by 1, 2, or 3 $R_a$; alternatively, $R_1$ is selected from F and $CH_3$.

**4.** The compound or the pharmaceutically acceptable salt thereof according to claim 1, wherein $R_3$ is selected from H, $CH_3$, $CH_2CH_3$, $CH_2CH_2CH_3$, $CH(CH_3)_2$, cyclopropyl, cyclobutyl, oxetanyl, azetidinyl, $-CH_2$-cyclopropyl, $-CH_2CH_2$-cyclopropyl, and $-CH_2$-oxetanyl, and the $CH_3$, $CH_2CH_3$, $CH_2CH_2CH_3$, $CH(CH_3)_2$, cyclopropyl, cyclobutyl, oxetanyl, azetidinyl, $-CH_2$-cyclopropyl, $-CH_2CH_2$-cyclopropyl, and $-CH_2$-oxetanyl are each independently and optionally substituted by 1, 2, or 3 $R_e$; alternatively, $R_3$ is selected from H, $CH_3$, $CF_3$, $CD_3$, $CH_2CH_3$, $CH(CH_3)_2$, cyclopropyl, cyclobutyl,

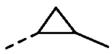, 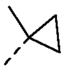, 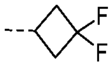, 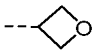,

and

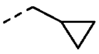.

**5.** The compound or the pharmaceutically acceptable salt thereof according to claim 1, wherein each $R_4$ is independently selected from H, F, Cl, Br, I, $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$, and the $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$ are each independently and optionally substituted by 1, 2, or 3 $R_d$; alternatively, each $R_4$ is independently selected from H, F, Cl, Br, and $CH_3$.

**6.** The compound or the pharmaceutically acceptable salt thereof according to claim 1, wherein each $R_5$ is independently selected from H, F, Cl, Br, I, $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$, and the $CH_3$, $CH_2CH_3$, $CH_2CH_2CH_3$, $CH(CH_3)_2$, $OCH_3$, $OCH_2CH_3$, $OCH_2CH_2CH_3$, and $OCH(CH_3)_2$ are each independently and optionally substituted by 1, 2, or 3 $R_d$; alternatively, each $R_5$ is independently selected from H, F, Cl, Br, and $CH_3$.

**7.** The compound or the pharmaceutically acceptable salt thereof according to claim 1 or 3, wherein $R_6$ is selected from ethynyl, propynyl, vinyl, and propenyl, and the ethynyl, propynyl, vinyl, and propenyl are each independently and optionally substituted by 1, 2, or 3 $R_b$; alternatively, $R_6$ is selected from

, 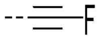, 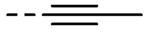, 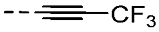,

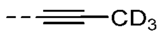, 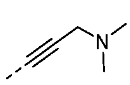, 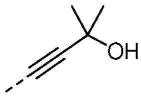, 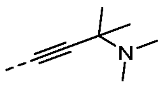, 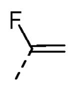, 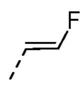, 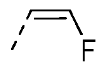, 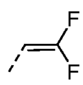,

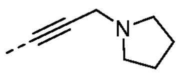, 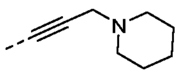, and 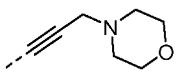.

**8.** The compound or the pharmaceutically acceptable salt thereof according to claim 1, wherein the structural moiety

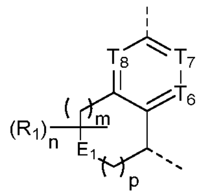

is selected from

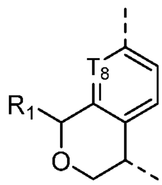 and 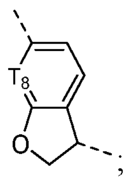;

alternatively, the structural moiety

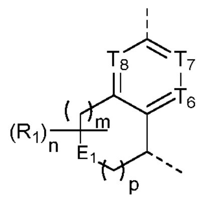

is selected from

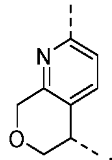, 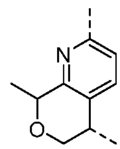,

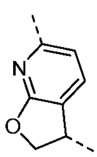, 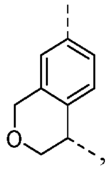, 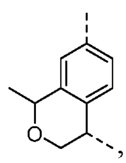, and 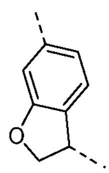.

**9.** The compound or the pharmaceutically acceptable salt thereof according to claim 1, wherein ring A is selected from pyridyl, pyrimidinyl, pyrazinyl, pyridazinyl, imidazolyl, pyrazolyl, pyrrolyl, thiazolyl, thienyl, furanyl, oxazolyl, triazolyl, cyclobutenyl, cyclopentenyl, cyclohexenyl, oxacyclopentenyl, oxacyclohexenyl, azacyclopentenyl, and azacyclohexenyl, and the pyridyl, pyrimidinyl, pyrazinyl, pyridazinyl, imidazolyl, pyrazolyl, pyrrolyl, thiazolyl, thienyl, furanyl, oxazolyl, triazolyl, cyclobutenyl, cyclopentenyl, cyclohexenyl, oxacyclopentenyl, oxacyclohexenyl, azacyclopentenyl, and azacyclohexenyl are each independently and optionally substituted by 1, 2, or 3 $R_c$.

**10.** The compound or the pharmaceutically acceptable salt thereof according to claim 1, wherein the structural moiety

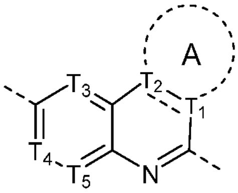

is selected from

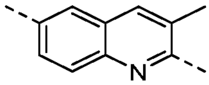 ,

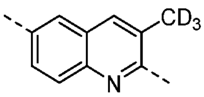 , 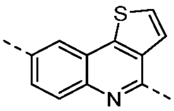 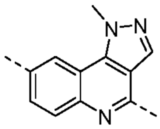 , 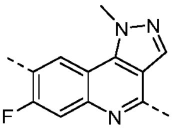 , 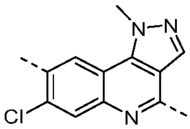 , 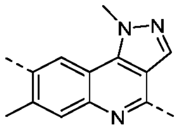 ,

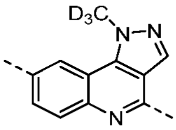 , 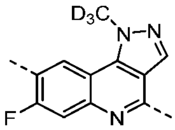 , 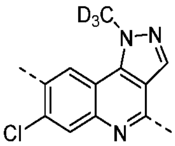 , 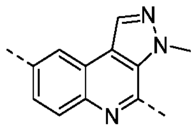 , 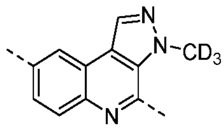 ,

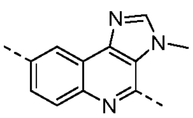 , 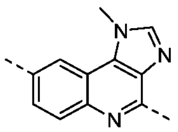 , 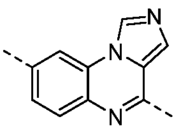 , 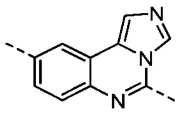 , 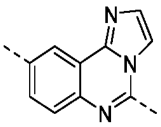 , 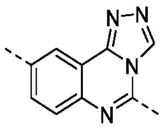 ,

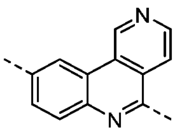 , 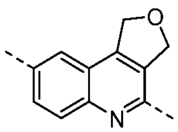 , 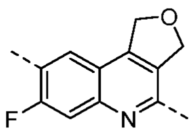 , 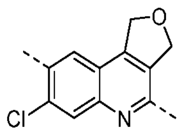 , 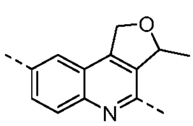 ,

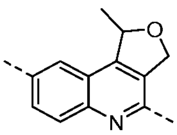 , 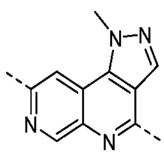 , and 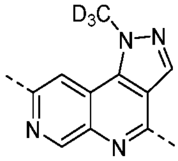 .

**11.** The compound or the pharmaceutically acceptable salt thereof according to any one of claims 1 to 10, selected from:

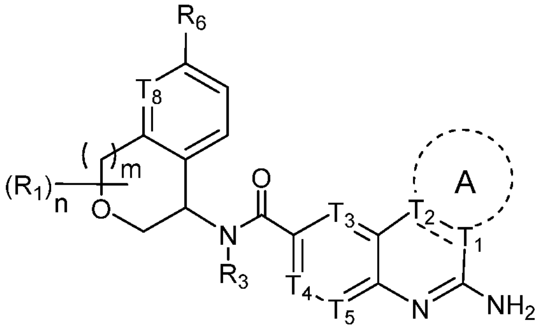

(P)

,

wherein

the structural moiety

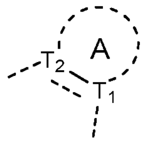

is selected from

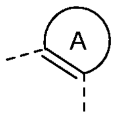

, 

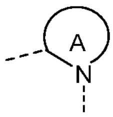

, and 

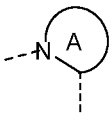

;

ring A is selected from 5- to 6-membered heteroaryl or 5- to 6-membered heterocycloalkenyl, and each 5- to 6-membered heteroaryl or 5- to 6-membered heterocycloalkenyl is independently and optionally substituted by 1, 2, or 3 $R_e$;
$T_3$, $T_4$, and $T_5$ are each independently selected from $CR_4$;
each $T_8$ is independently selected from CH and N;
each $R_1$ is independently selected from H, halogen, and $C_{1-3}$ alkyl, and the $C_{1-3}$ alkyl is optionally substituted by 1, 2, or 3 $R_a$;
$R_3$ is selected from H and $C_{1-3}$ alkyl, and the $C_{1-3}$ alkyl is optionally substituted by 1, 2, or 3 $R_e$;
$R_4$ is selected from H, halogen, and $C_{1-3}$ alkyl, and the $C_{1-3}$ alkyl is optionally substituted by 1, 2, or 3 $R_d$;
$R_6$ is selected from $C_{2-4}$ alkynyl and $C_{2-4}$ alkenyl, and the $C_{2-4}$ alkynyl and $C_{2-4}$ alkenyl are each independently and optionally substituted by 1, 2, or 3 $R_b$;
$R_a$, $R_c$, $R_d$, and $R_e$ are each independently selected from H, D, halogen, OH, $NH_2$, $CH_3$, and $CD_3$;
each $R_b$ is independently selected from H, D, halogen, $C_{1-3}$ alkyl, $C_{1-3}$ alkylamino, and 4-to 6-membered heterocycloalkyl, and the $C_{1-3}$ alkyl, $C_{1-3}$ alkoxy, and $C_{1-3}$ alkylamino are each independently and optionally substituted by 1, 2, or 3 R;
each R is independently selected from H, D, halogen, OH, $NH_2$, $CH_3$, $CF_3$, and $CD_3$;
n is selected from 0, 1, 2, and 3;
m is selected from 0 and 1.

**12.** The compound or the pharmaceutically acceptable salt thereof according to any one of claims 1 to 10, selected from:

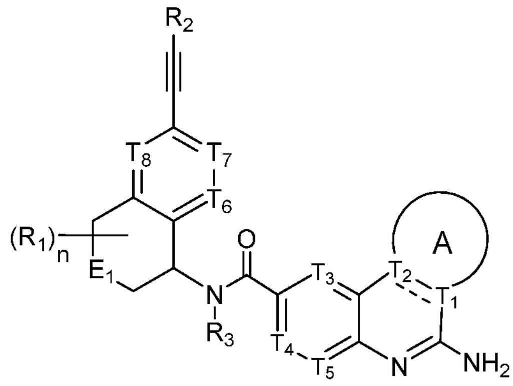

(I) ,

wherein

each $R_1$ is independently selected from H, halogen, and $C_{1-3}$ alkyl, and the $C_{1-3}$ alkyl is optionally substituted by 1, 2, or 3 $R_a$;
$R_2$ is selected from H, halogen, and $C_{1-3}$ alkyl, and the $C_{1-3}$ alkyl is optionally substituted by 1, 2, or 3 $R_b$;
$E_1$ is selected from O;
the structural moiety

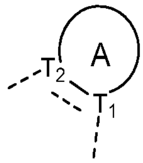

is selected from

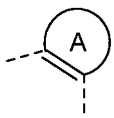 , 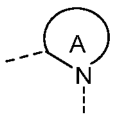 , and 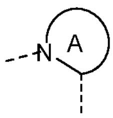 ;

ring A is selected from 5- to 6-membered heteroaryl and 5- to 6-membered heterocycloalkenyl, and the 5- to 6-membered heteroaryl and 5- to 6-membered heterocycloalkenyl are each independently and optionally substituted by 1, 2, or 3 $R_c$;
$T_3$, $T_4$, and $T_5$ are each independently selected from $CR_4$;
$T_6$ and $T_7$ are each independently CH;
$T_8$ is selected from CH and N;
$R_3$ is selected from H and $C_{1-3}$ alkyl, and the $C_{1-3}$ alkyl is optionally substituted by 1, 2, or 3 $R_e$;
$R_4$ is selected from H, halogen, and $C_{1-3}$ alkyl, and the $C_{1-3}$ alkyl is optionally substituted by 1, 2, or 3 $R_d$;
$R_a$, $R_b$, $R_c$, $R_d$, and $R_e$ are each independently selected from H, halogen, OH, $NH_2$, $CH_3$, and $CD_3$;
n is selected from 0, 1, 2, and 3;
"hetero" in the 5- to 6-membered heteroaryl or 5- to 6-membered heterocycloalkenyl represents 1, 2, 3, or 4 heteroatoms or heteroatom groups independently selected from -O-, - NH-, -S-, and N.

**13.** A compound as shown below or a pharmaceutically acceptable salt thereof,

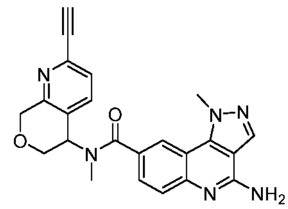 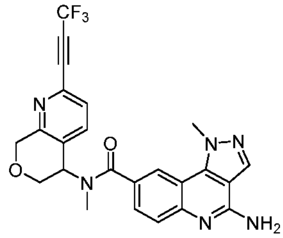 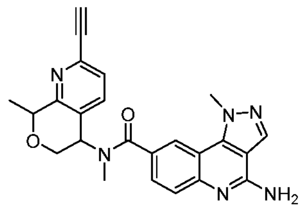 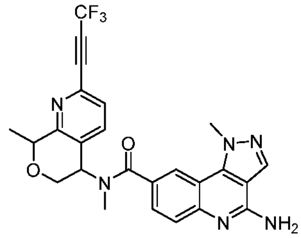

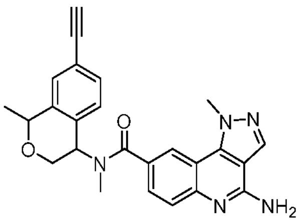 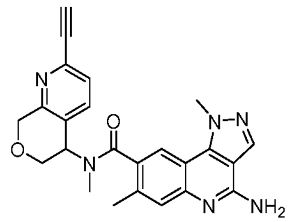 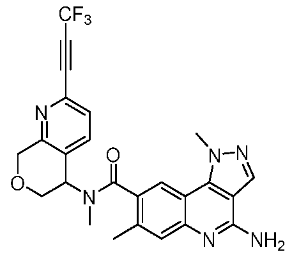 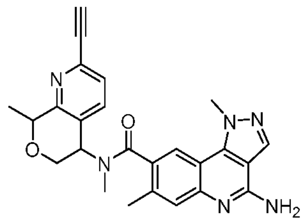

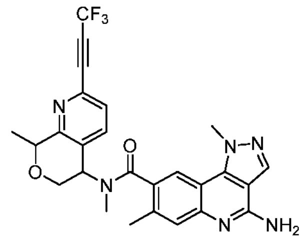 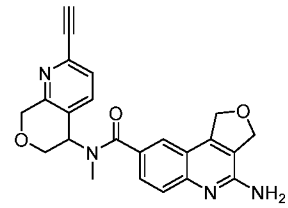 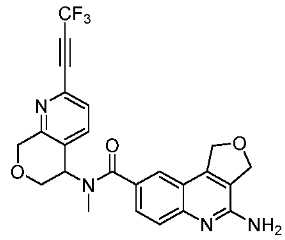 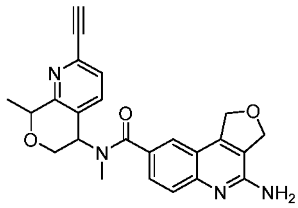

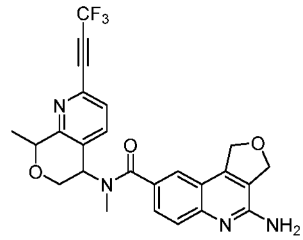 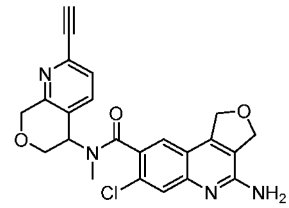 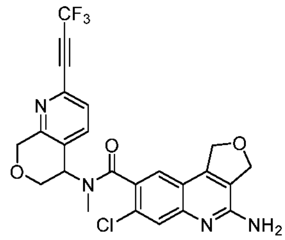 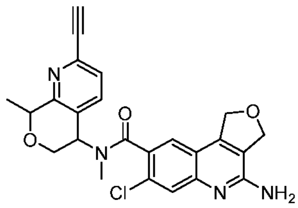

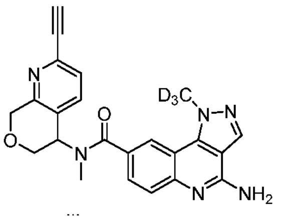 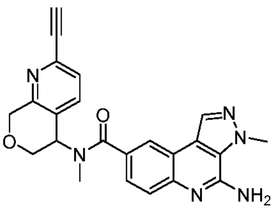 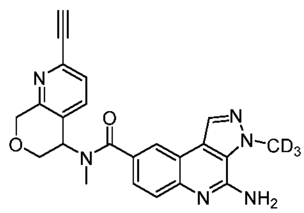 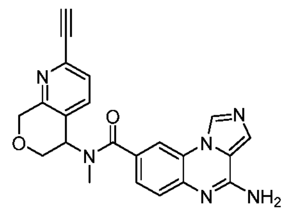

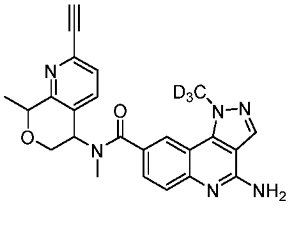 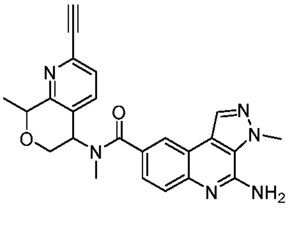 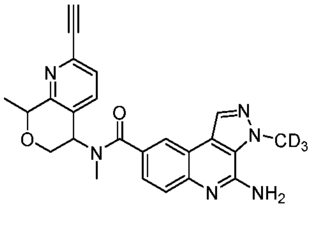 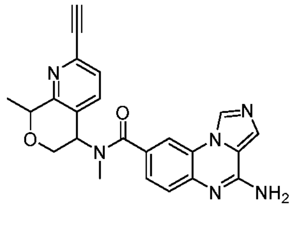

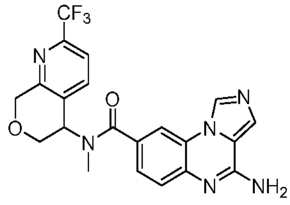 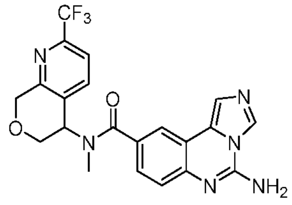 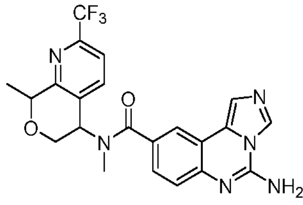 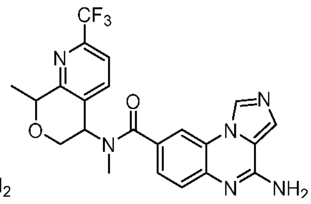

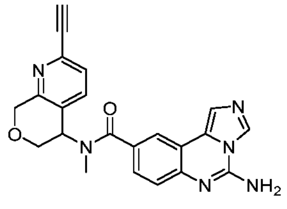 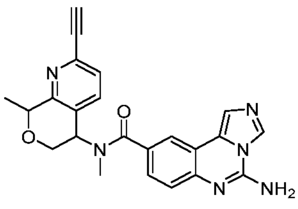 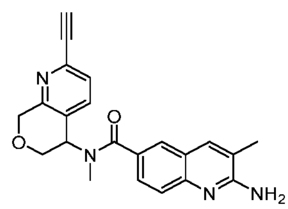 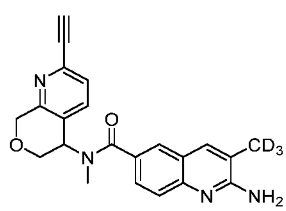

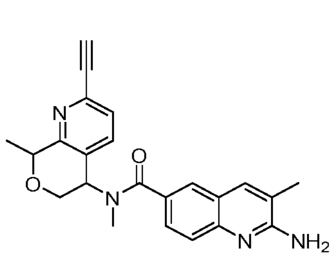 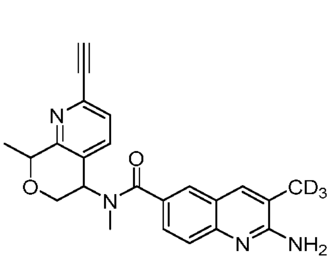 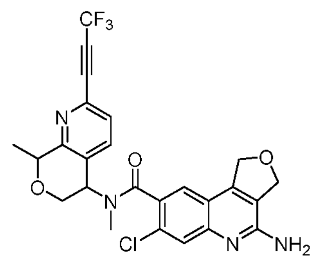

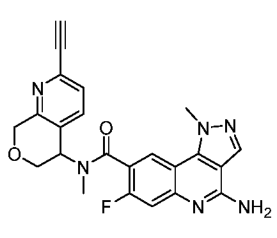 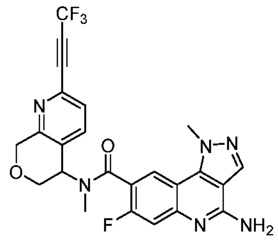 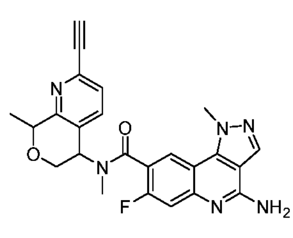 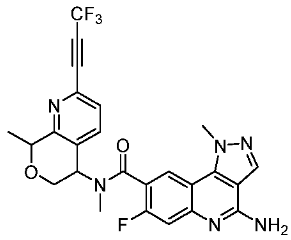

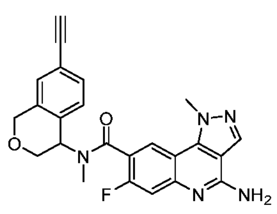 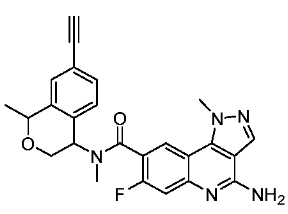 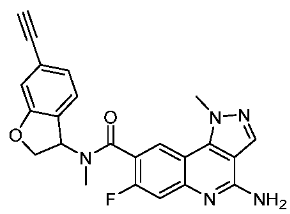 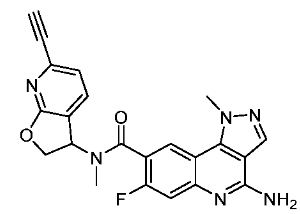

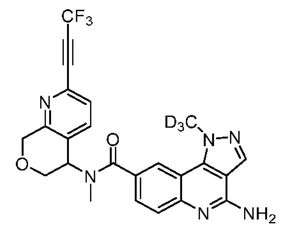 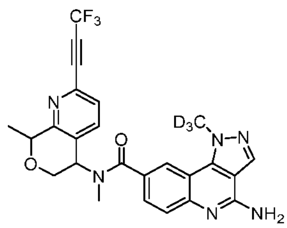 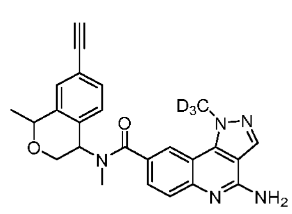 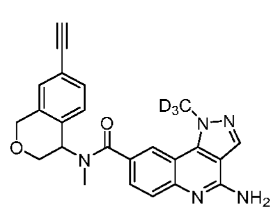

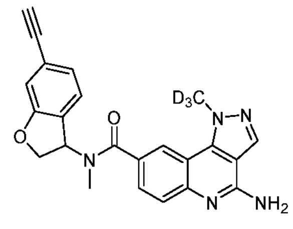
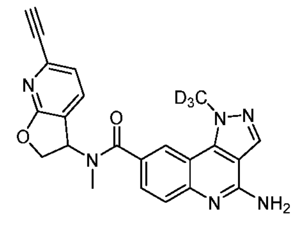
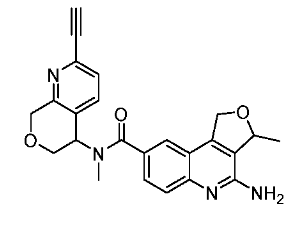
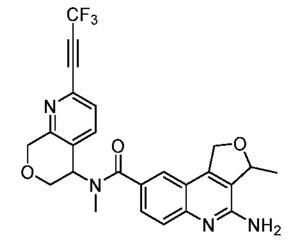

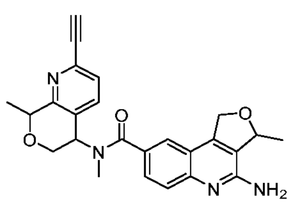
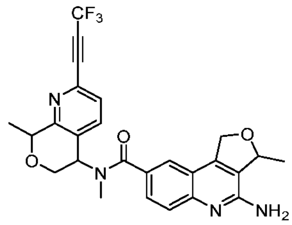
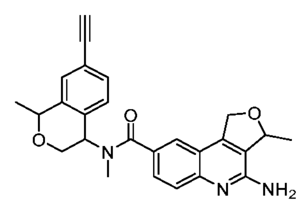
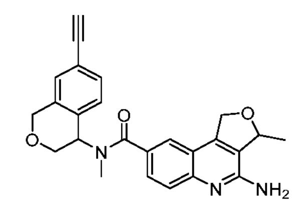

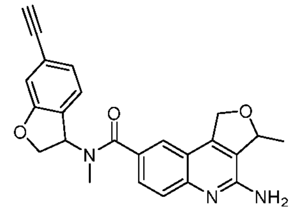
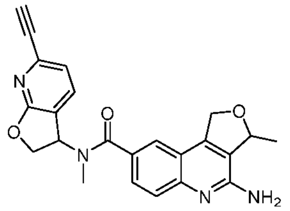
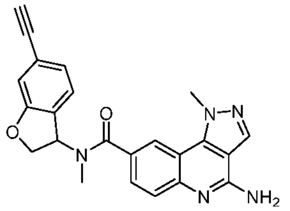
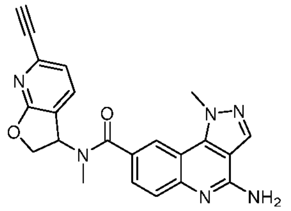

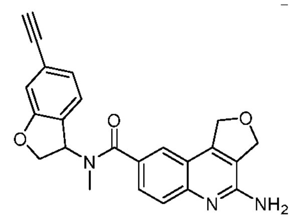
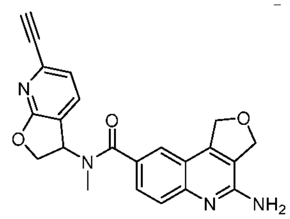
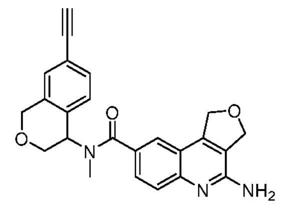
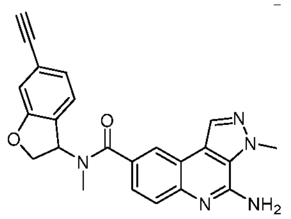

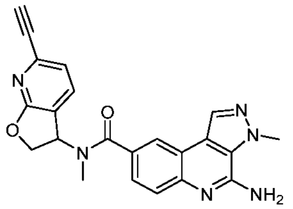
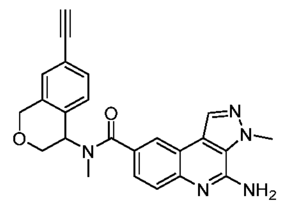
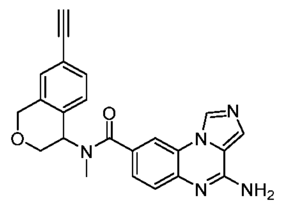
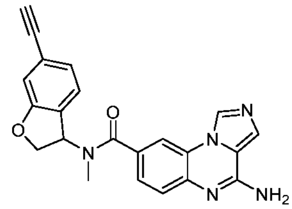

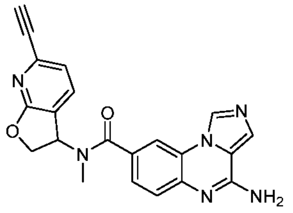
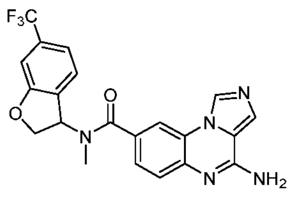
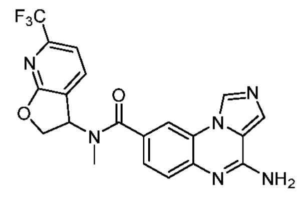
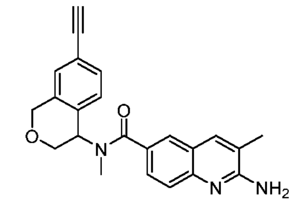

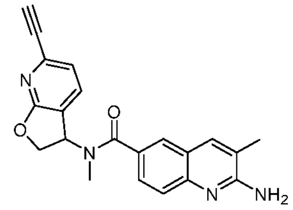
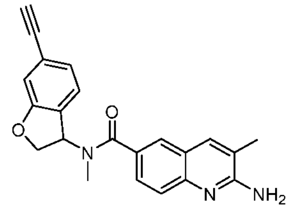
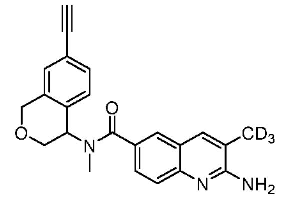
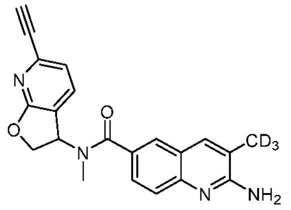

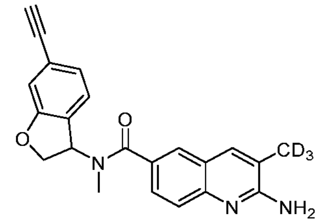

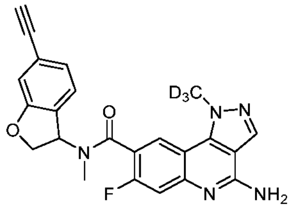

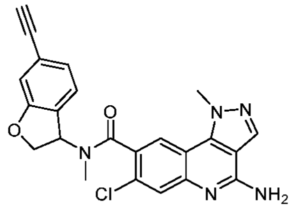

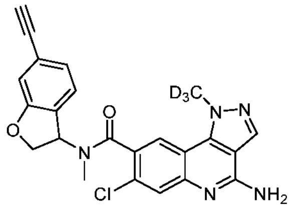

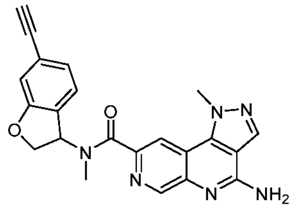

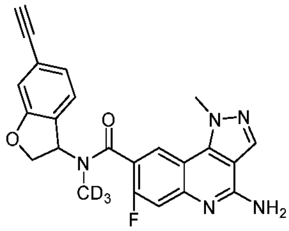

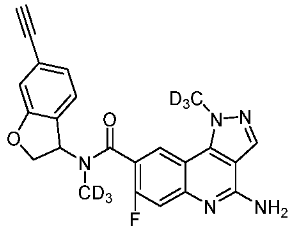

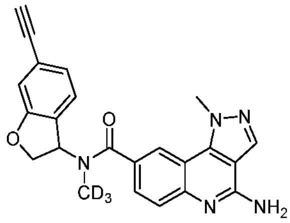

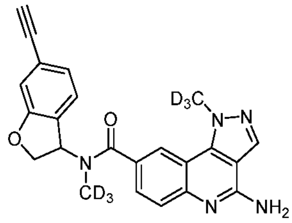

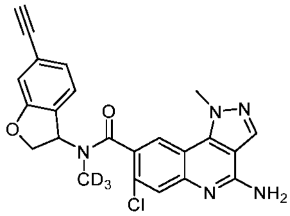

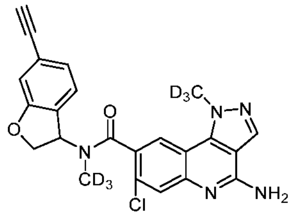

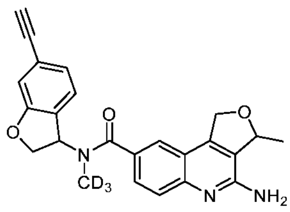

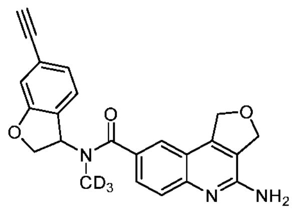

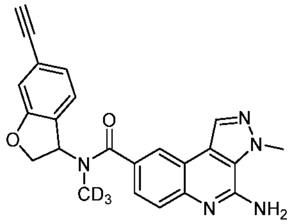

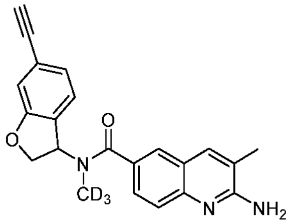

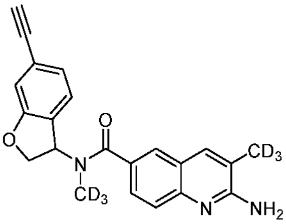

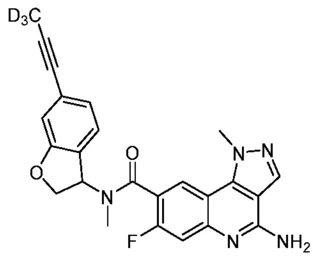

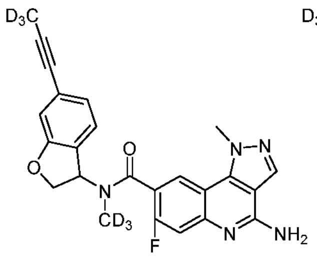

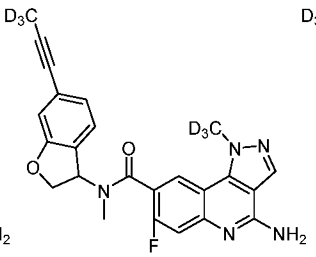

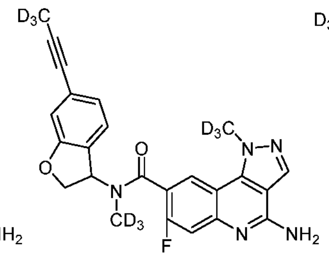

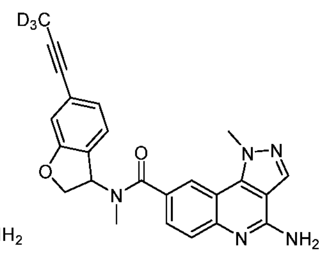

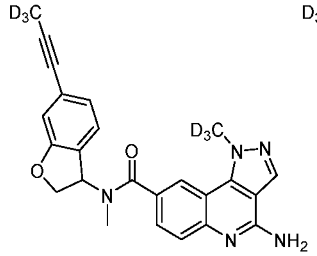 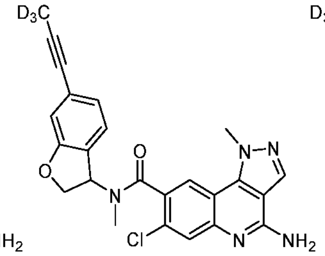 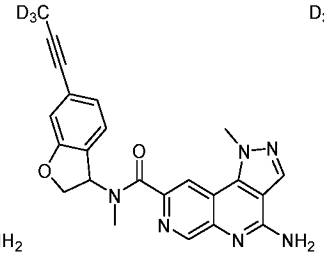 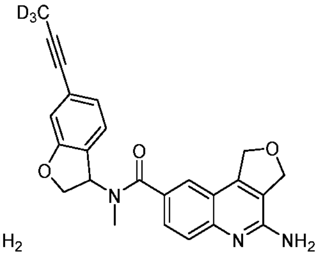

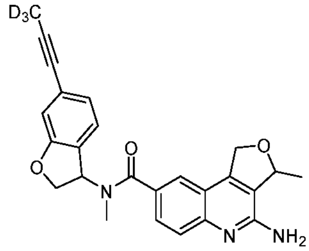 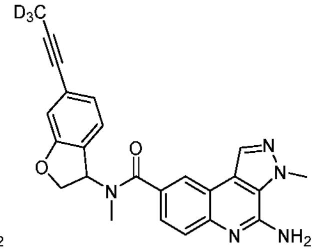 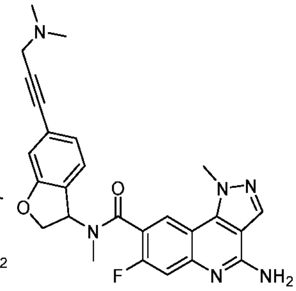 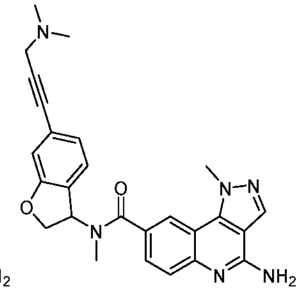

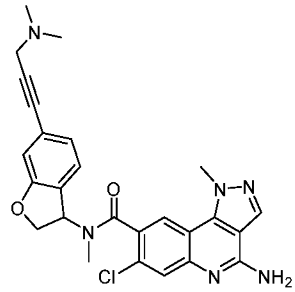 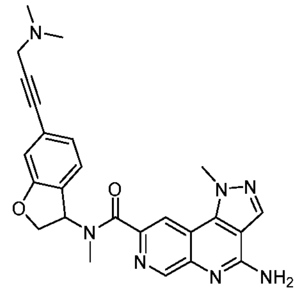 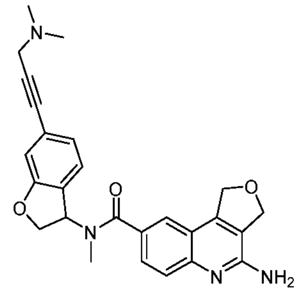 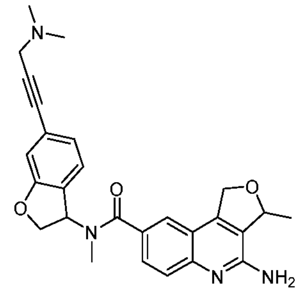

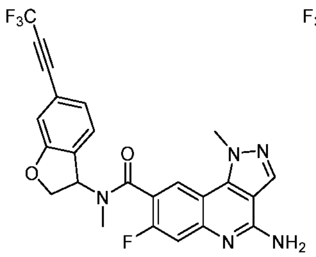 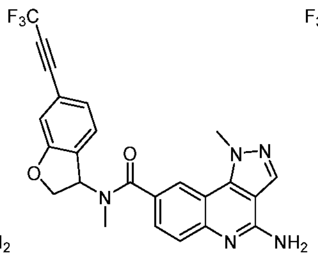 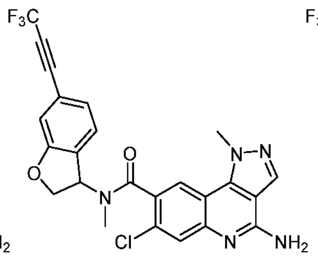 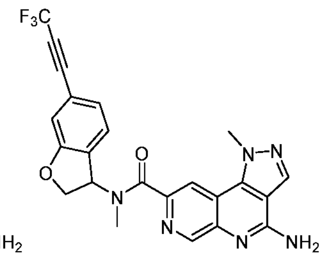

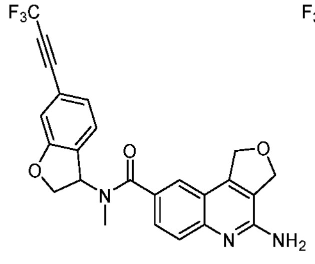 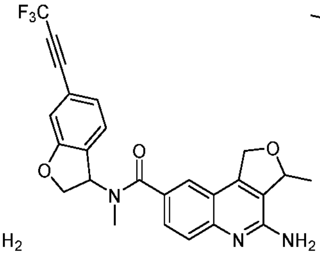 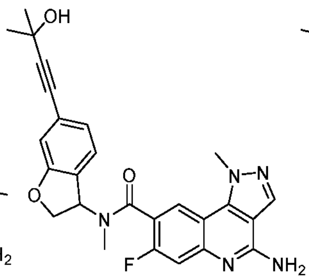 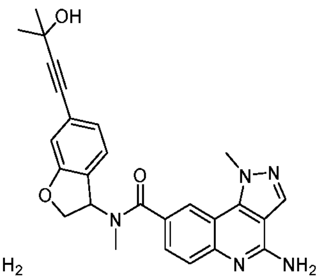

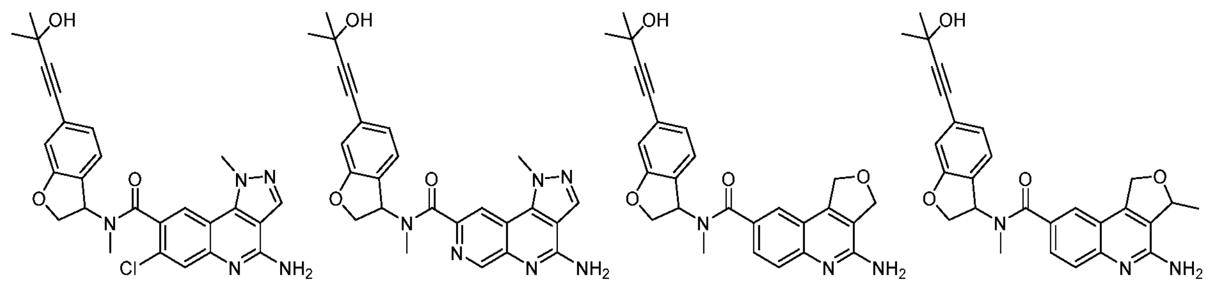

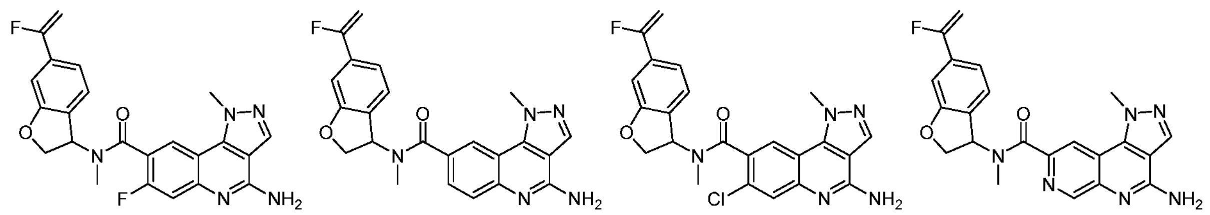

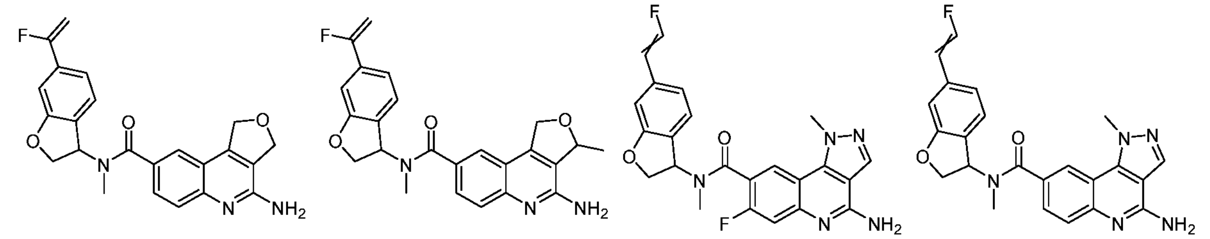

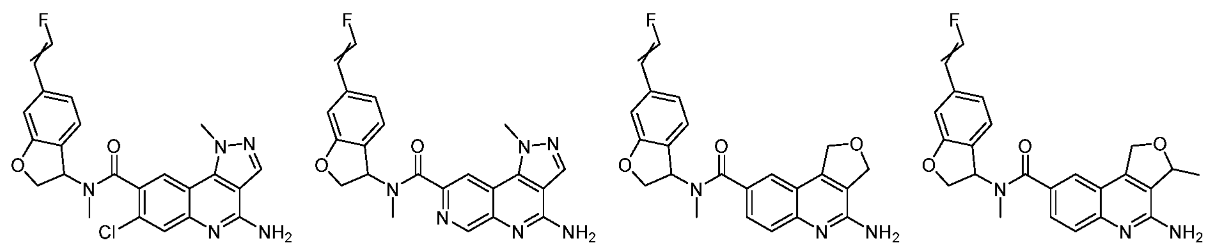

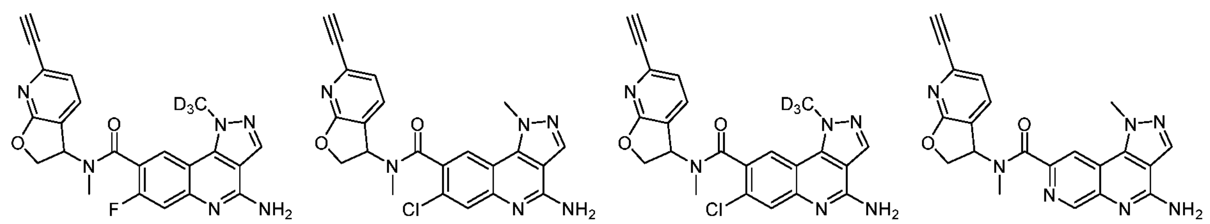

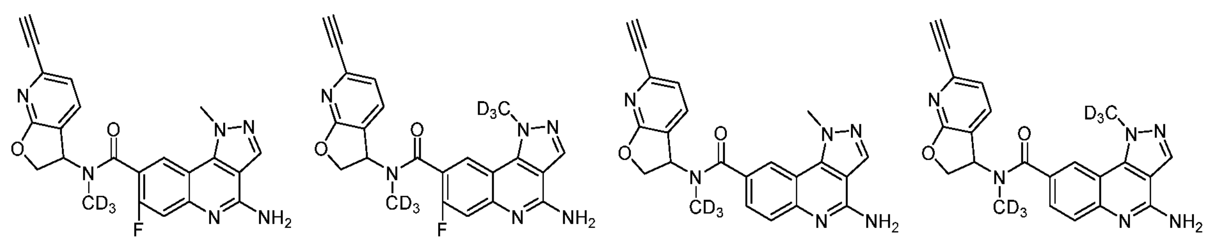

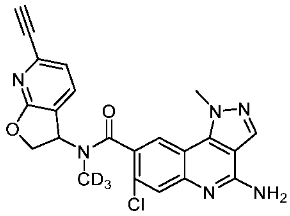
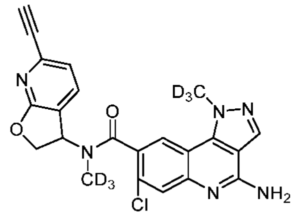
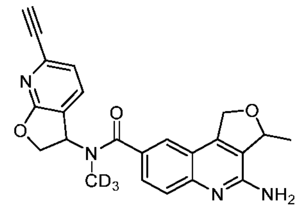
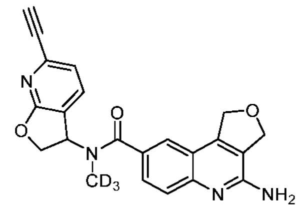
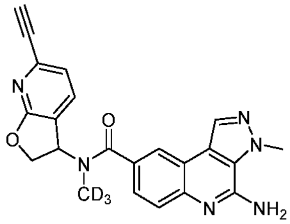
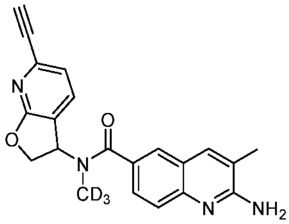
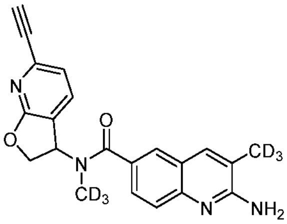
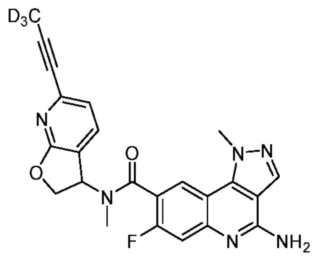
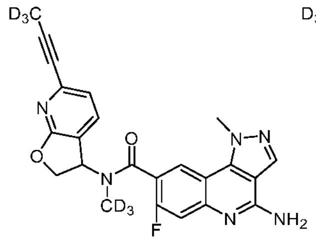
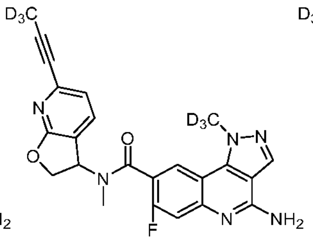
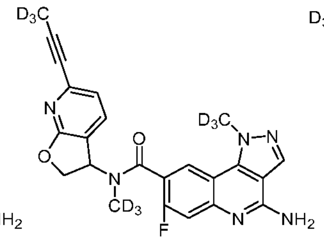
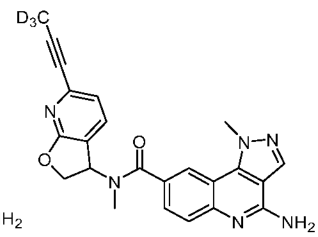
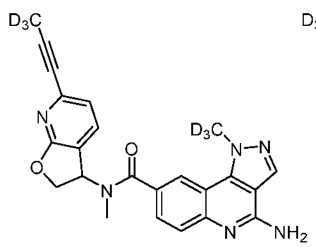
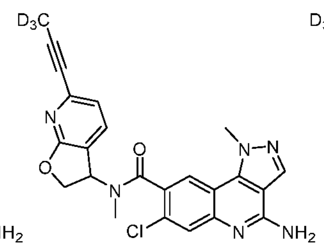
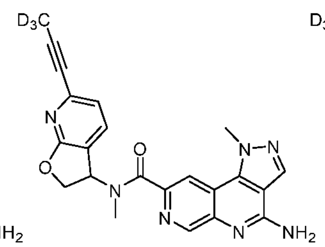
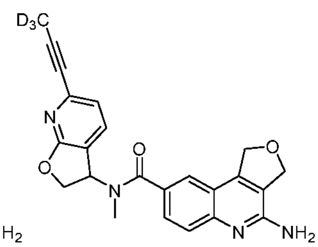
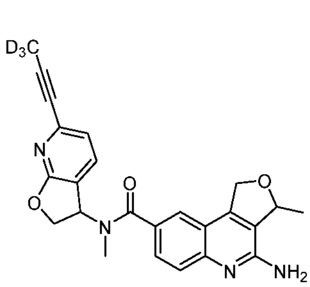
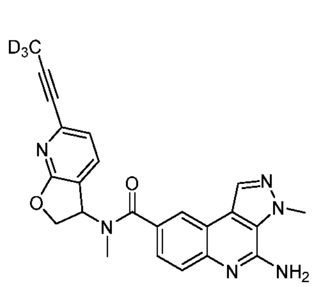
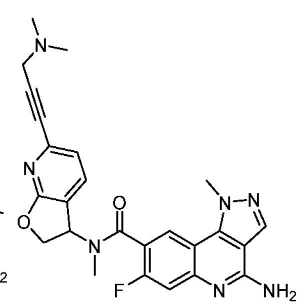
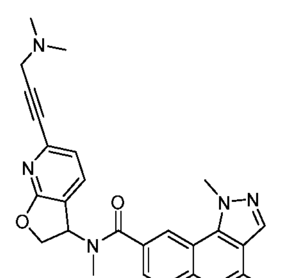
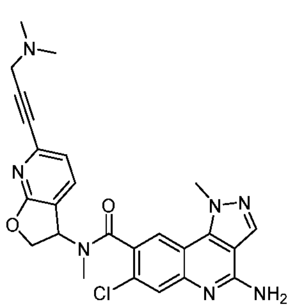
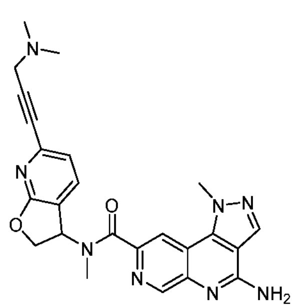
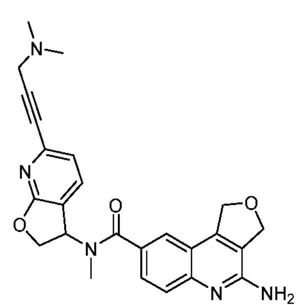
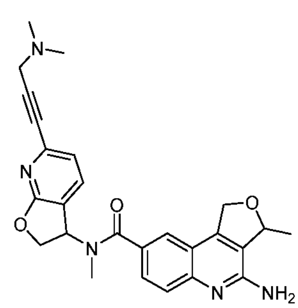

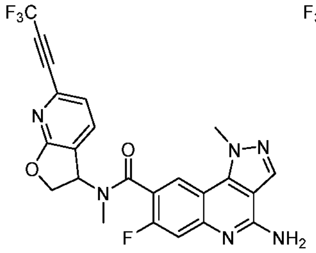
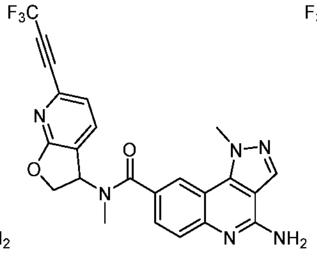
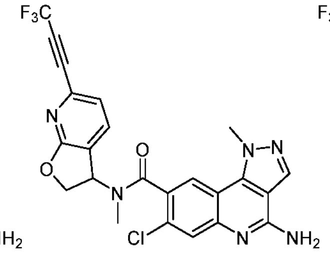
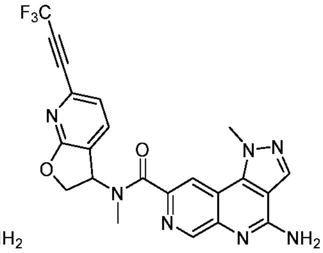
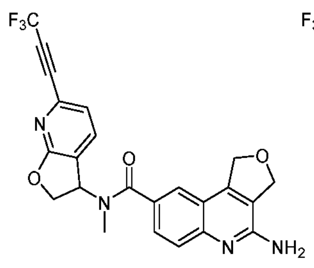
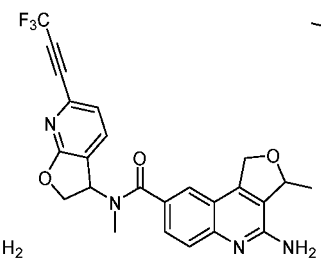
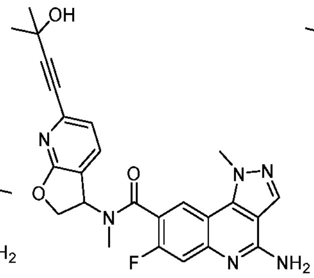
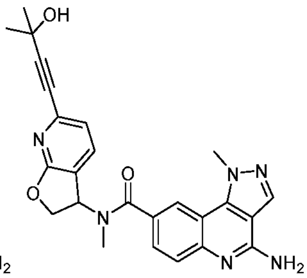
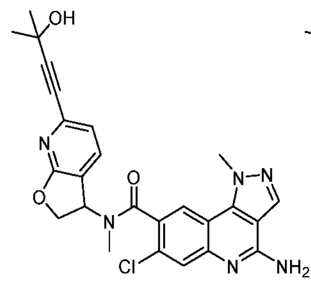
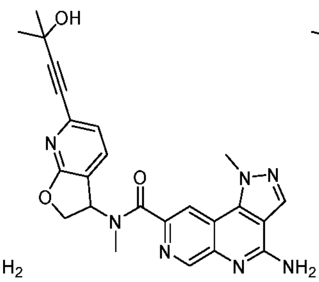
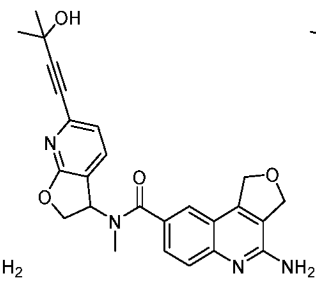
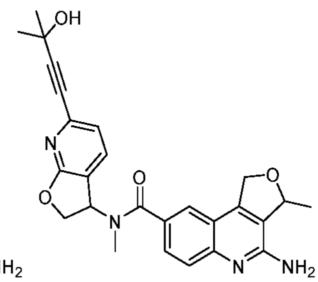
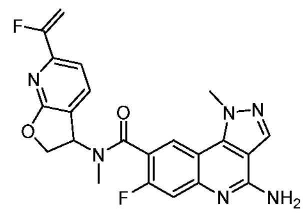
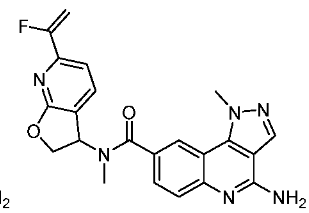
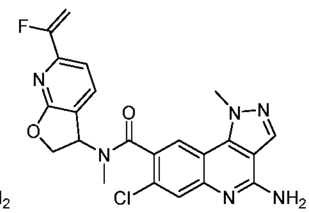
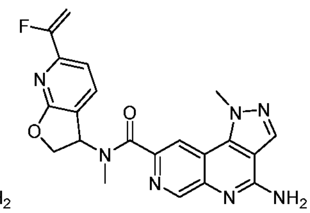
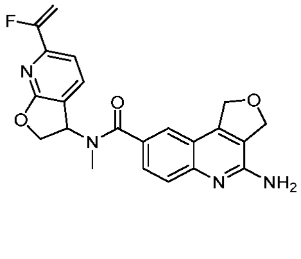
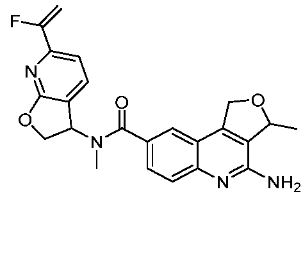
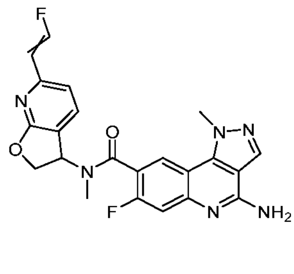
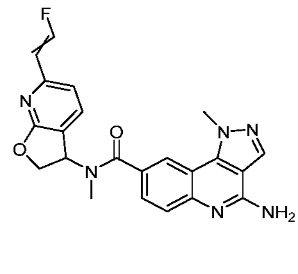
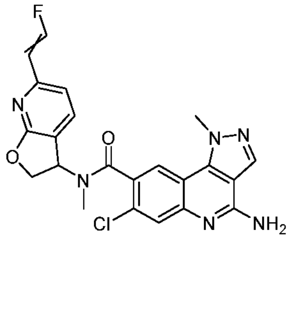
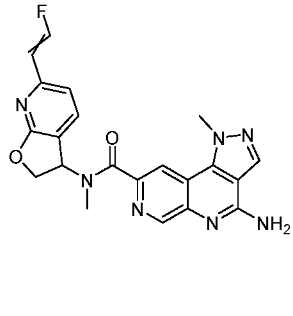
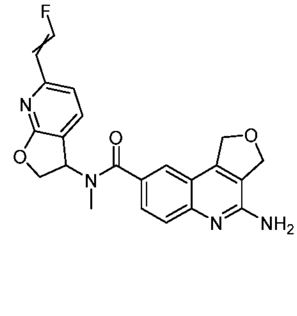
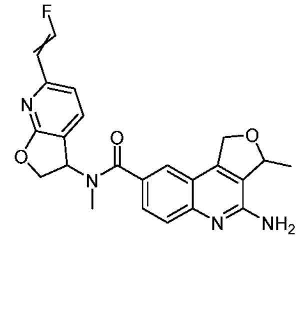

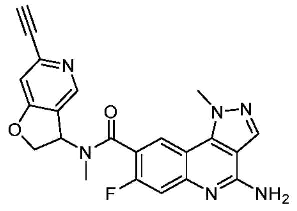
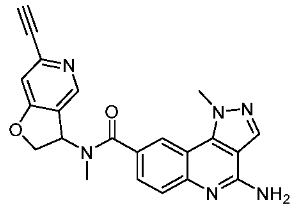
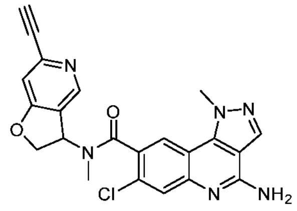
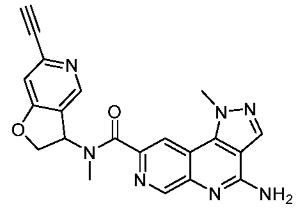
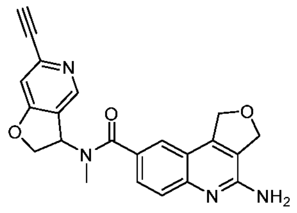
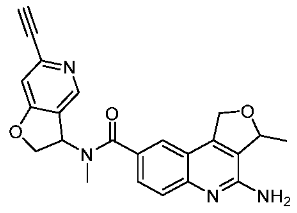
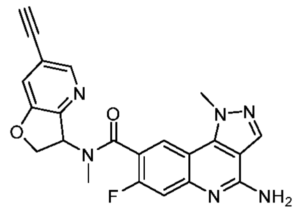
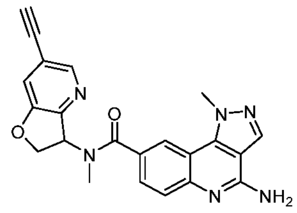
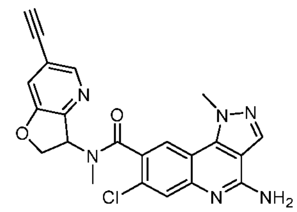
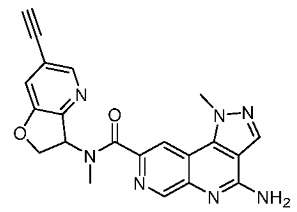
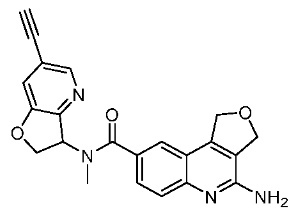
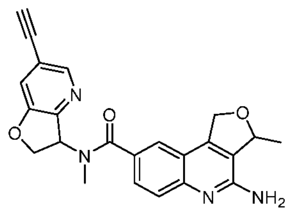
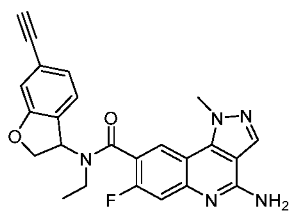
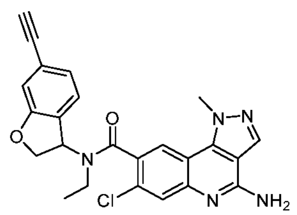
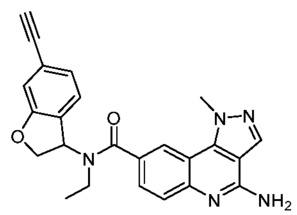
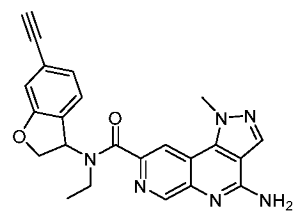
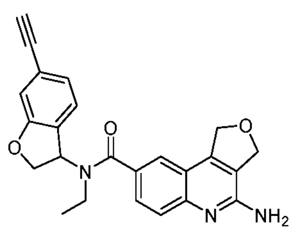
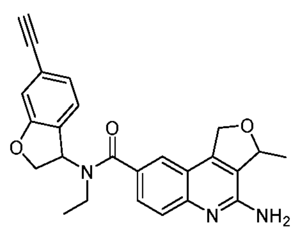
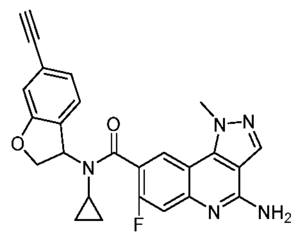
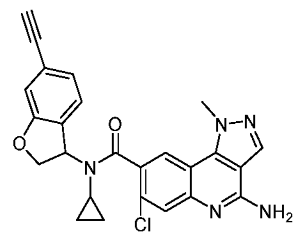
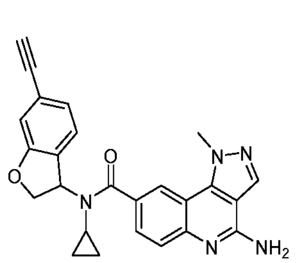
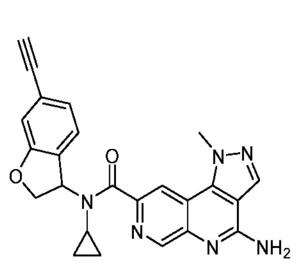
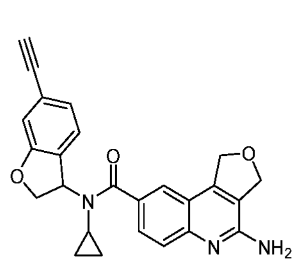
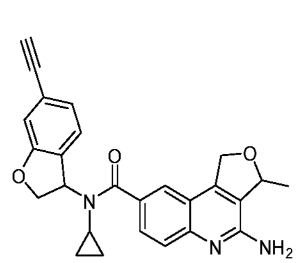
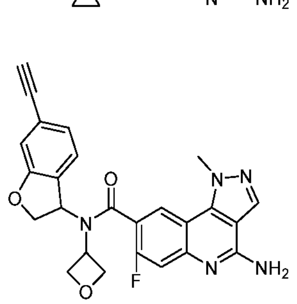
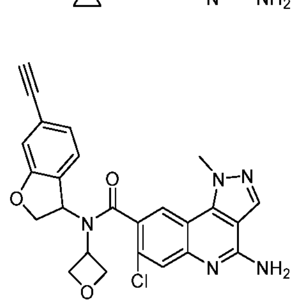
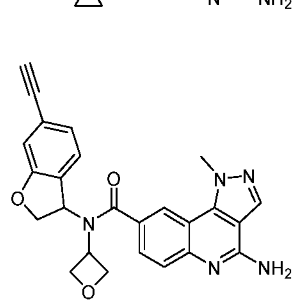
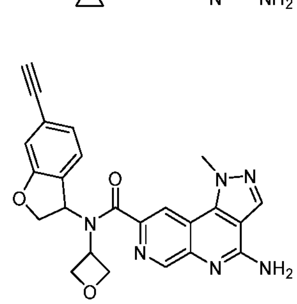

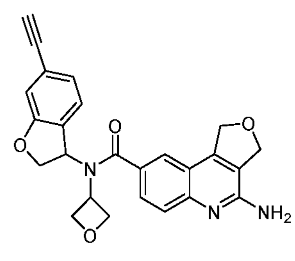
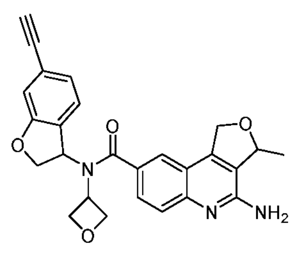
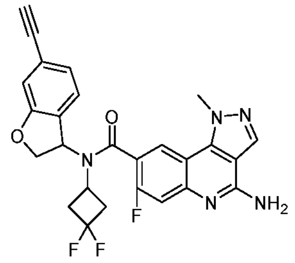
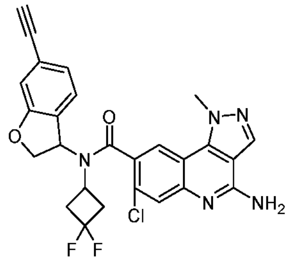
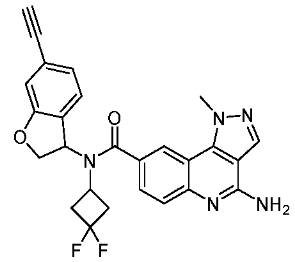
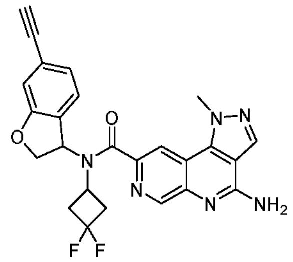
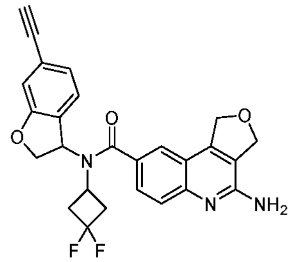
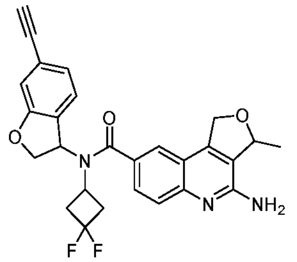
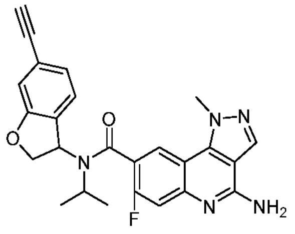
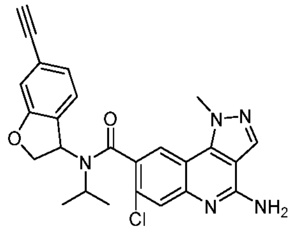
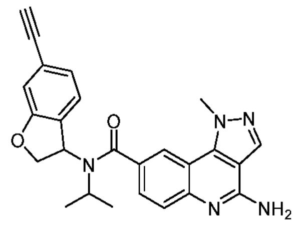
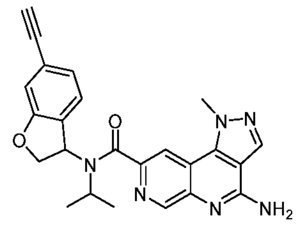
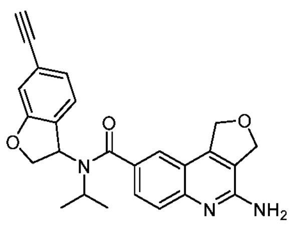
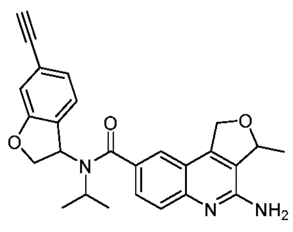
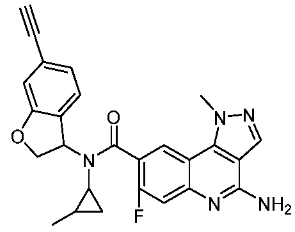
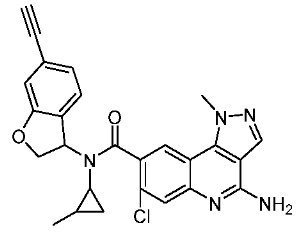
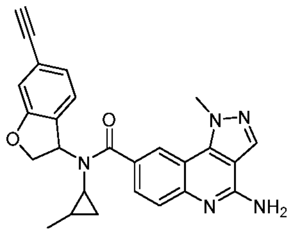
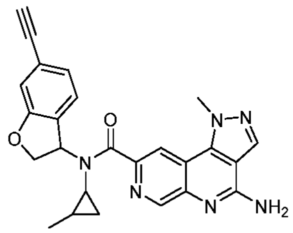
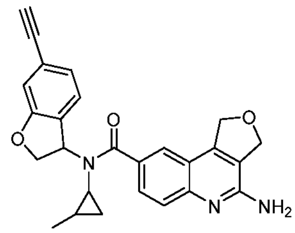
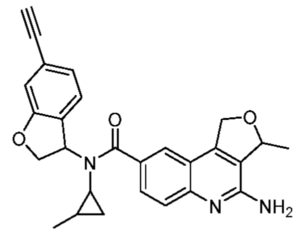
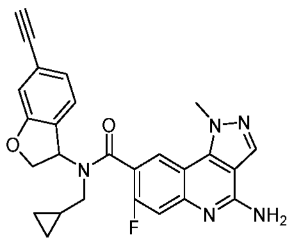
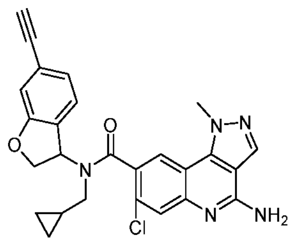
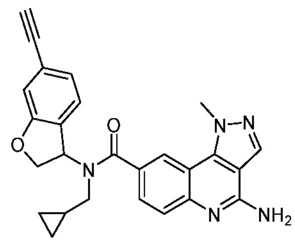
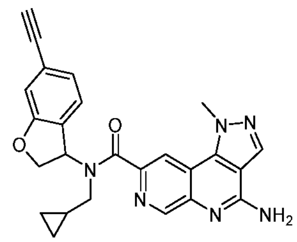

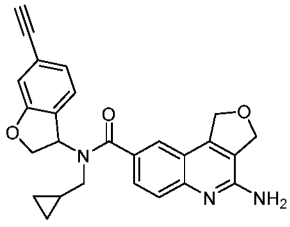
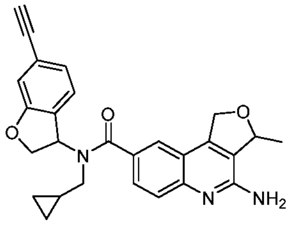
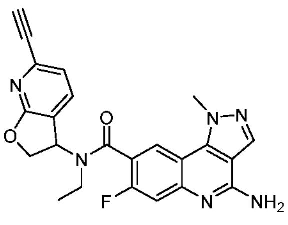
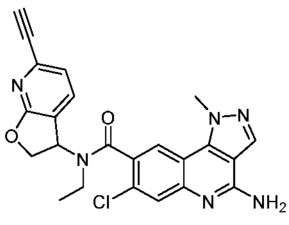

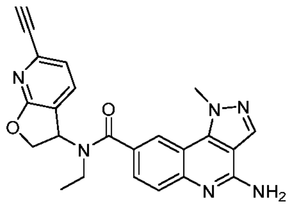
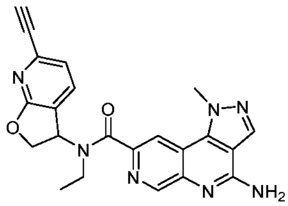
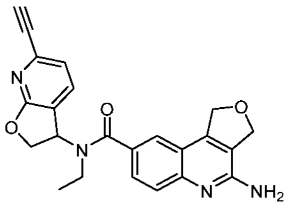
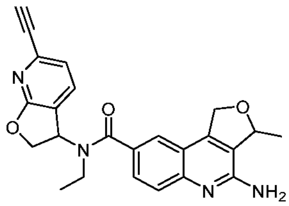

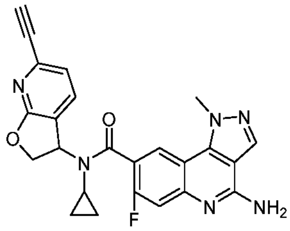
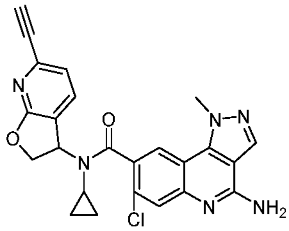
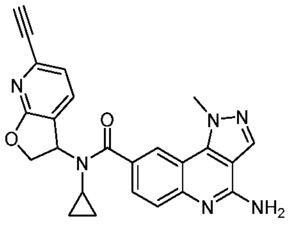
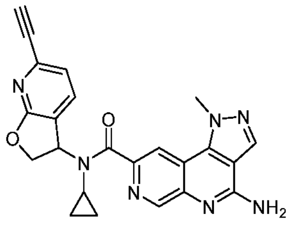

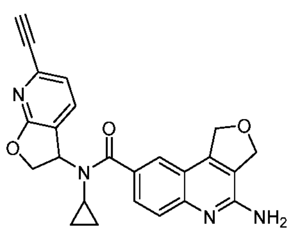
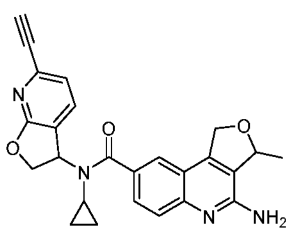
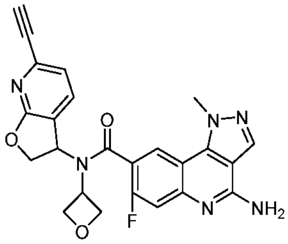
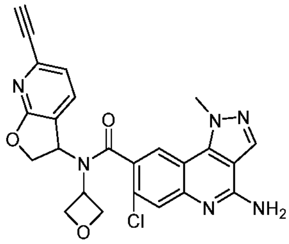

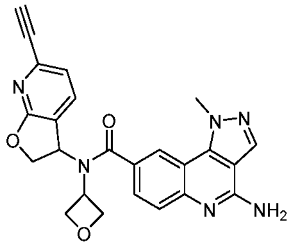
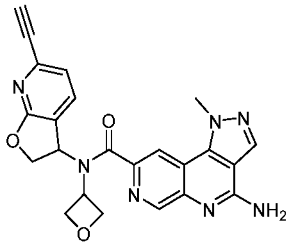
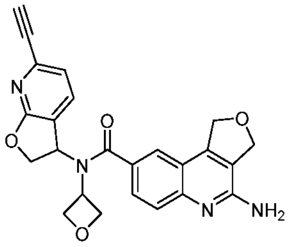
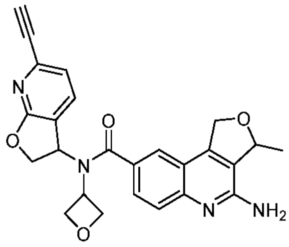

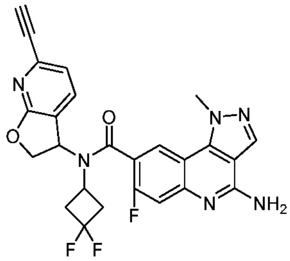
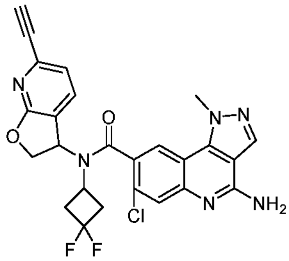
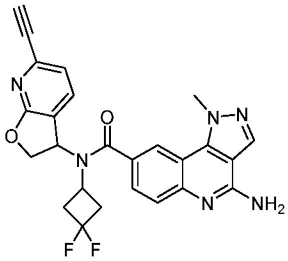
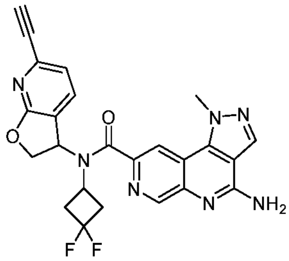

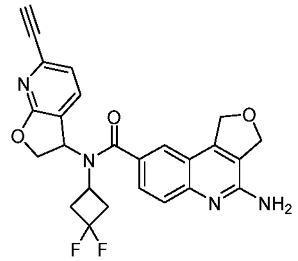
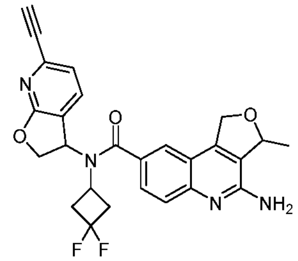
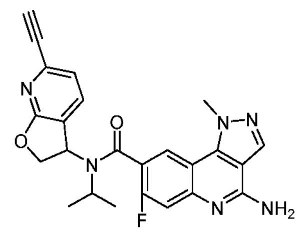
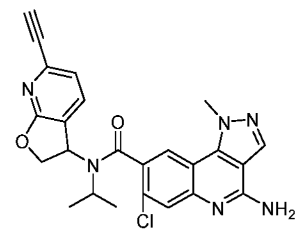
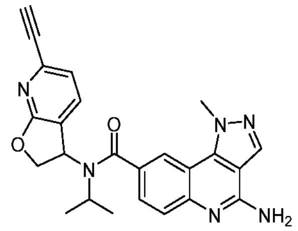
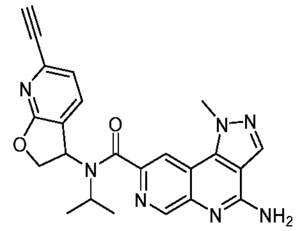
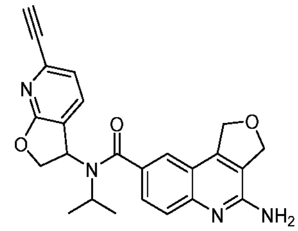
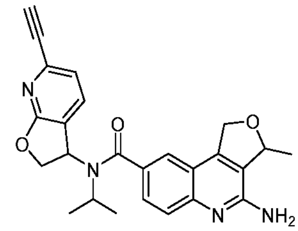
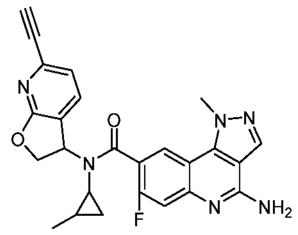
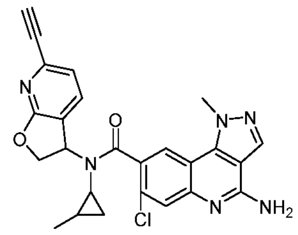
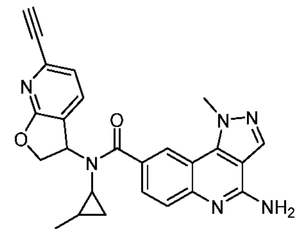
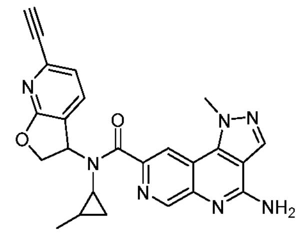
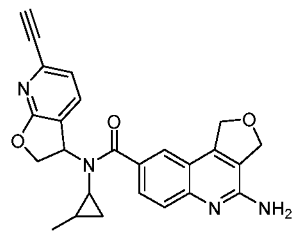
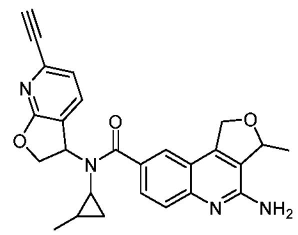
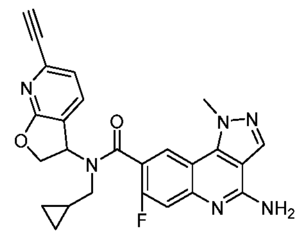
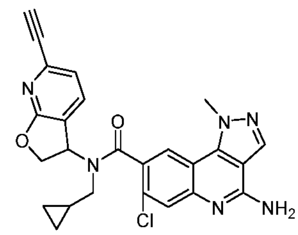
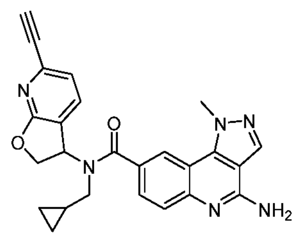
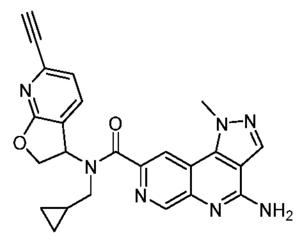
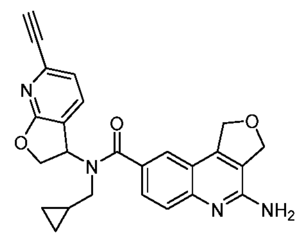
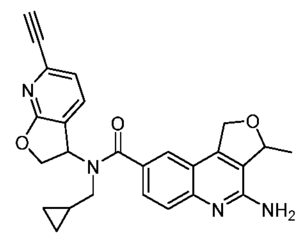
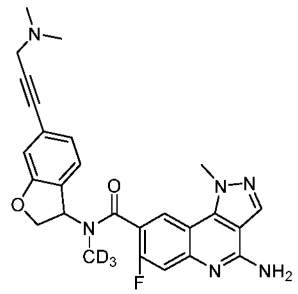
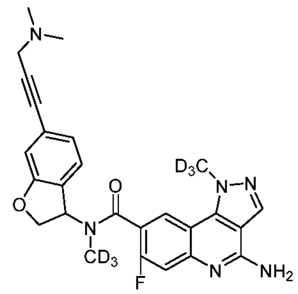
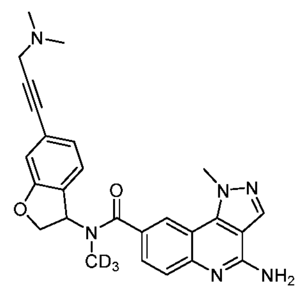
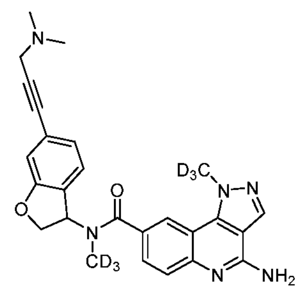

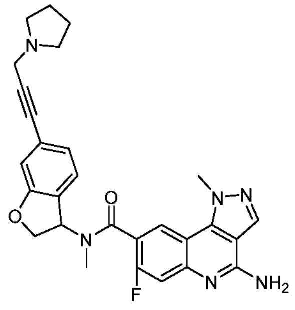 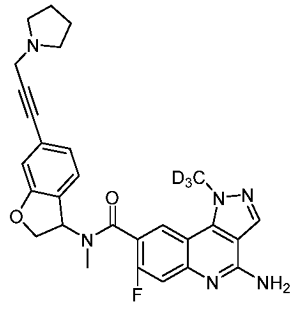 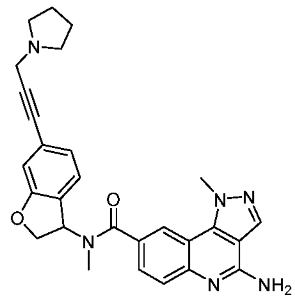 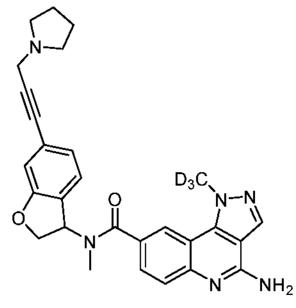

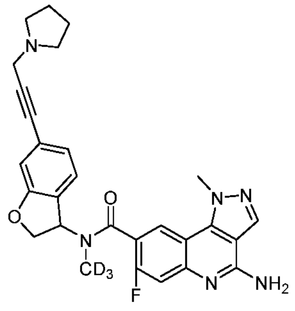 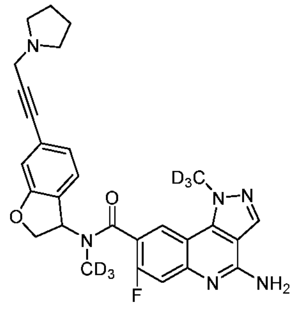 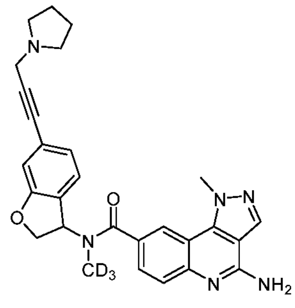 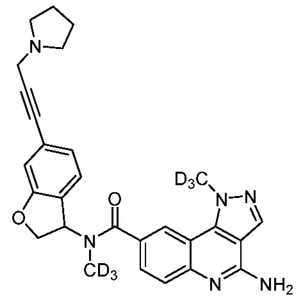

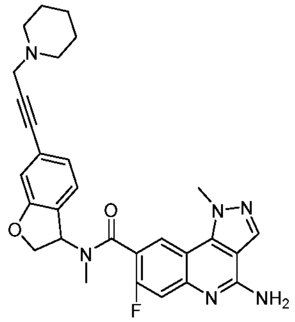 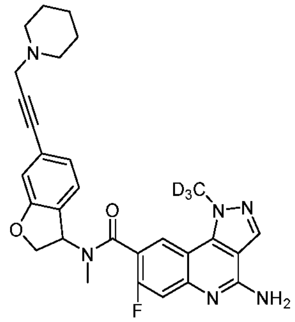 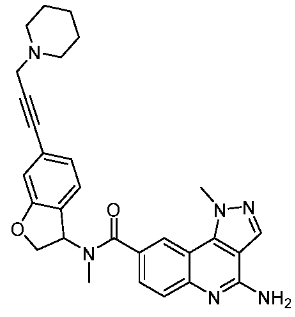 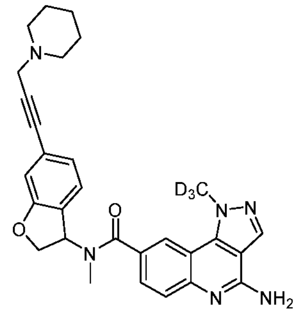

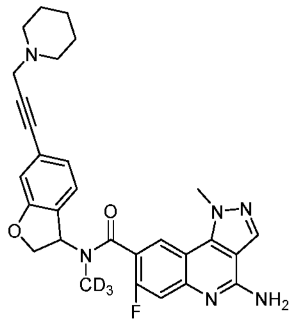 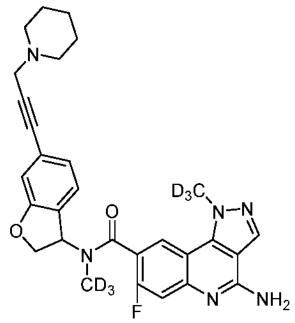 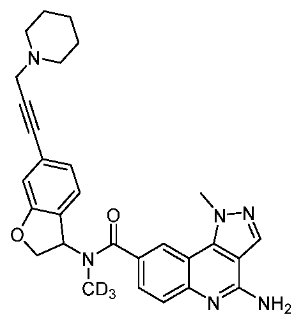 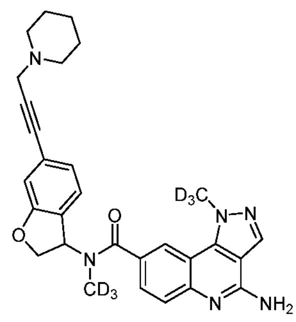

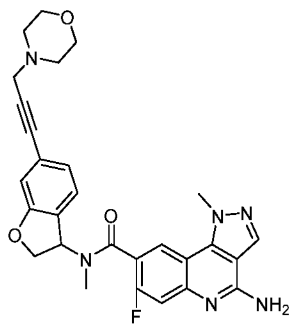 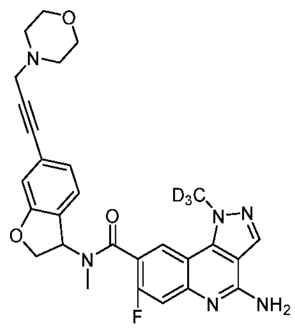 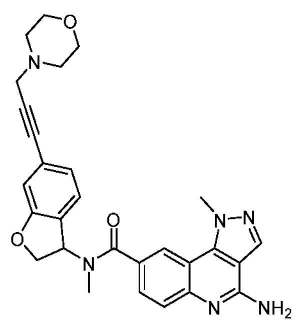 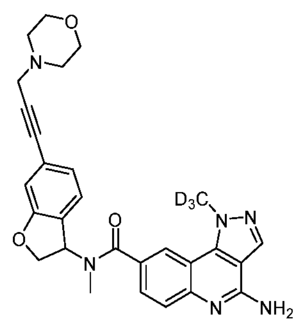

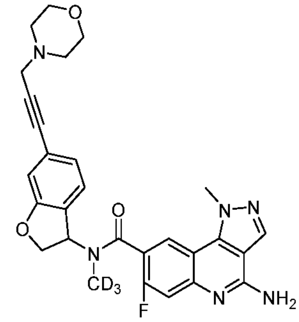
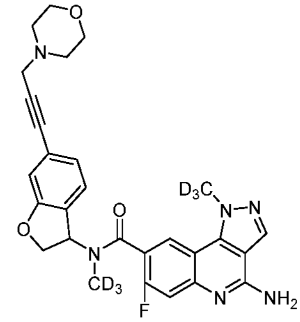
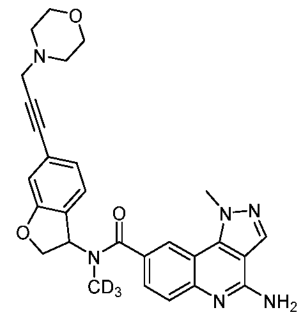
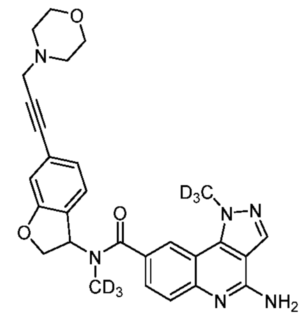

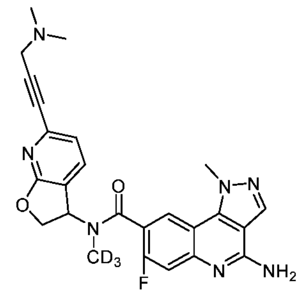
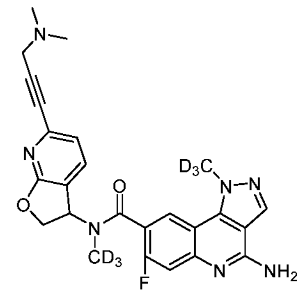
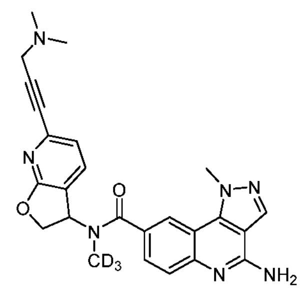
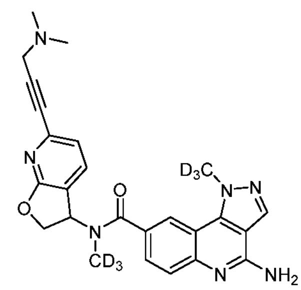

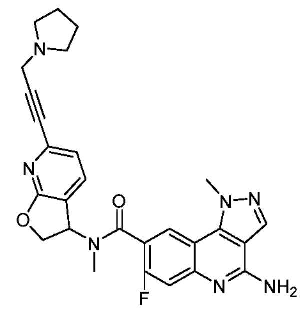
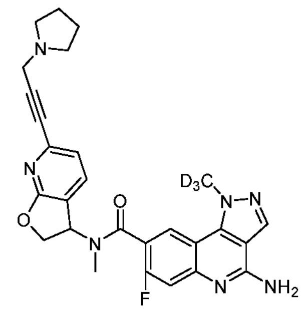
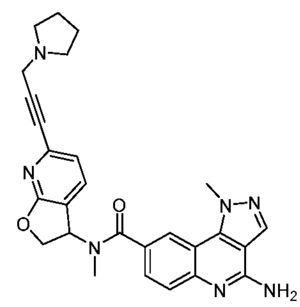
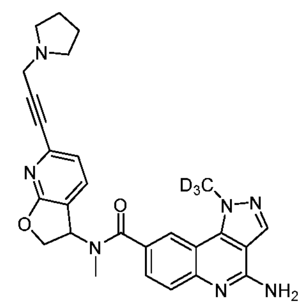

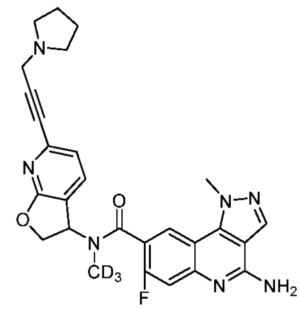
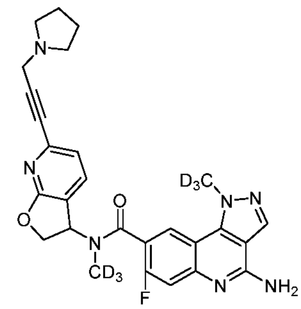
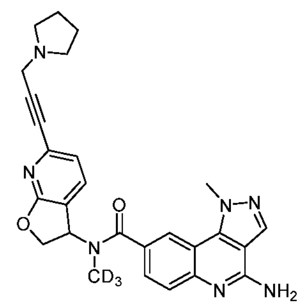
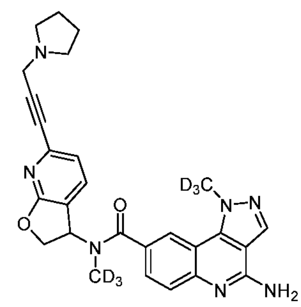

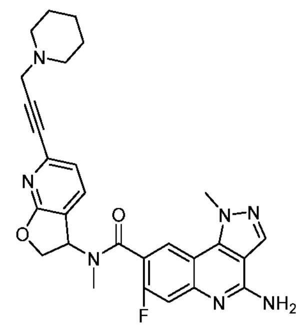
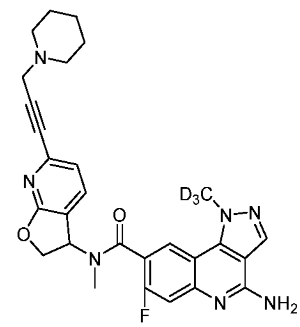
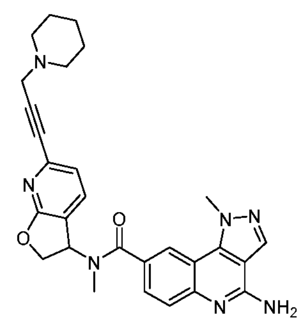
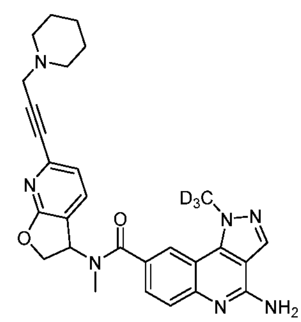

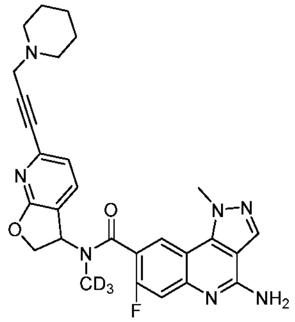
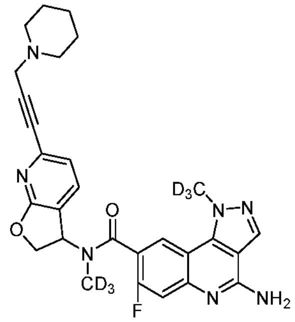
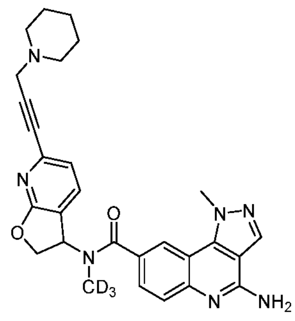
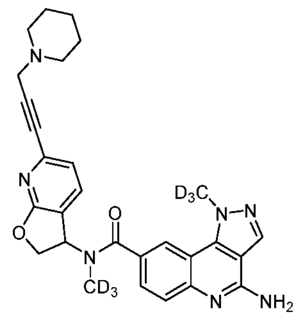

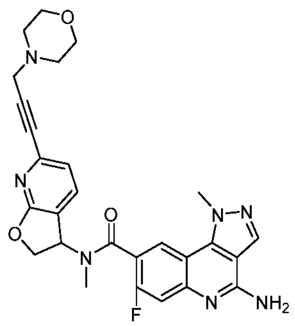
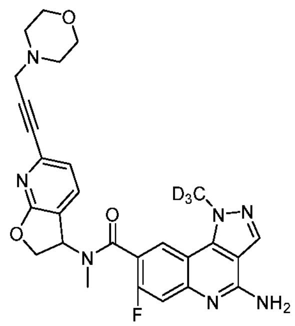
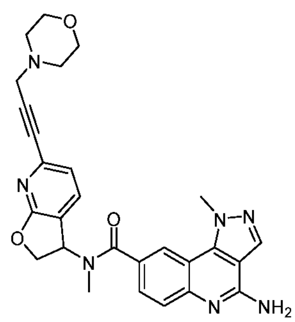
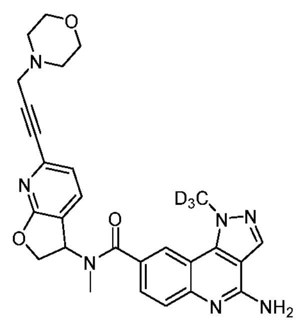

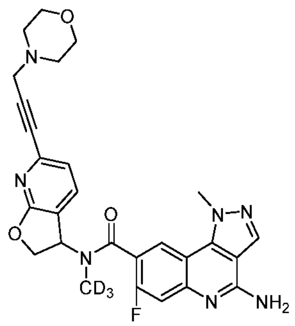
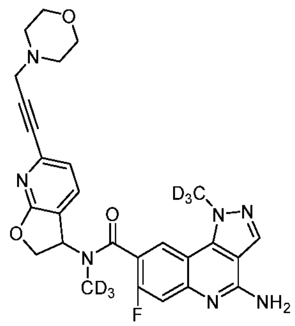
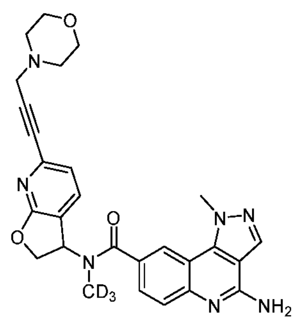
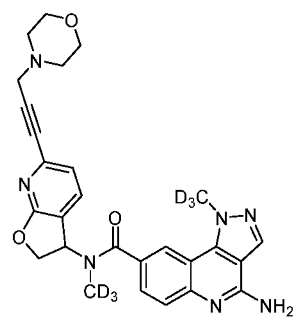

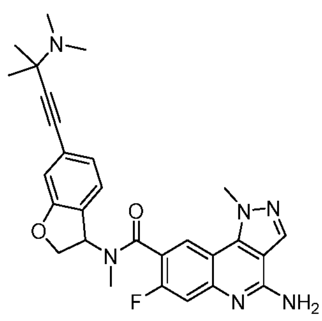.

**14.** The compound or the pharmaceutically acceptable salt thereof according to claim 13, selected from:

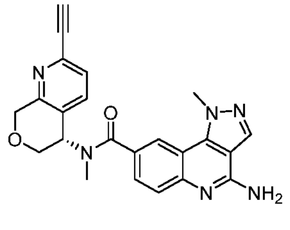
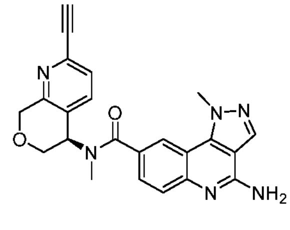
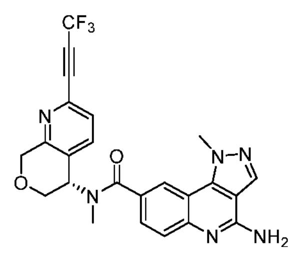
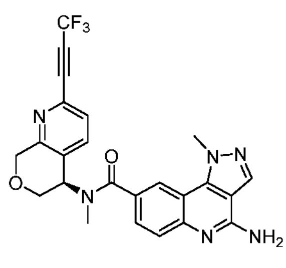

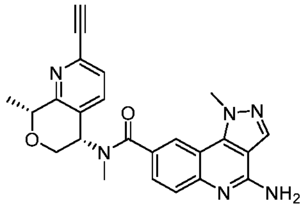 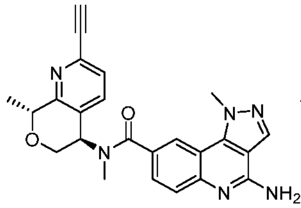 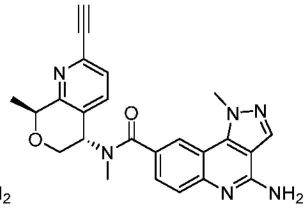 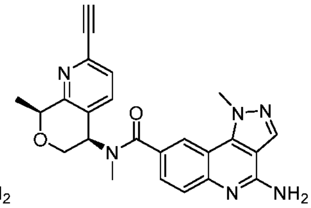

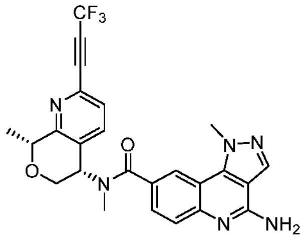 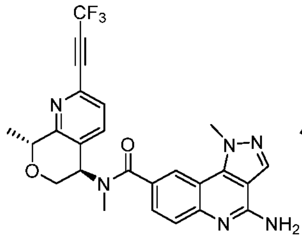 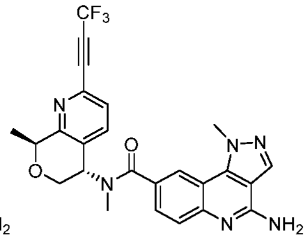 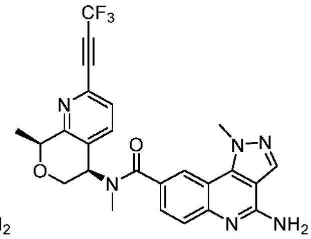

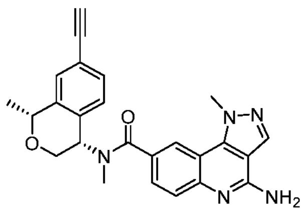 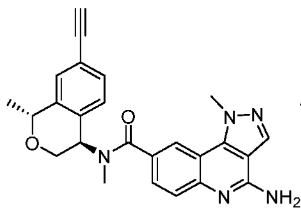 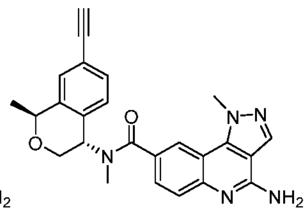 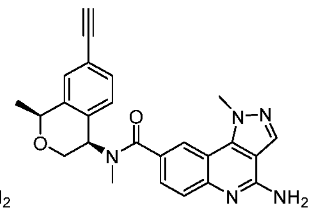

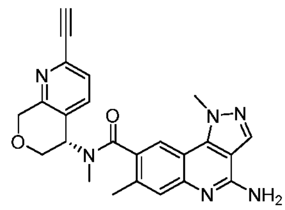 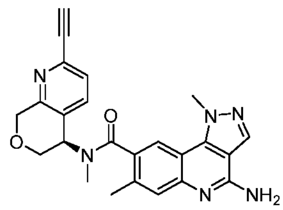 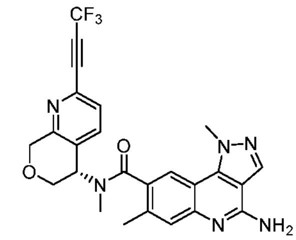 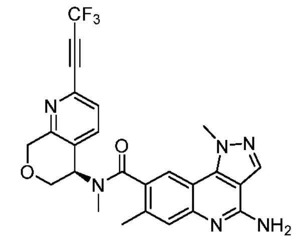

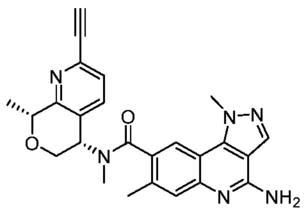 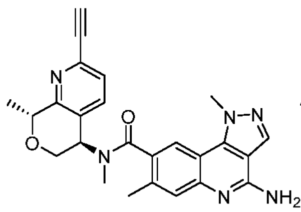 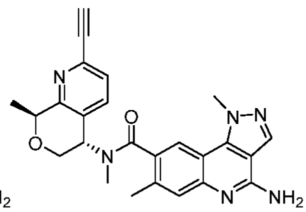 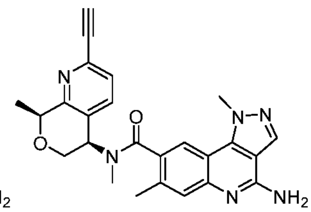

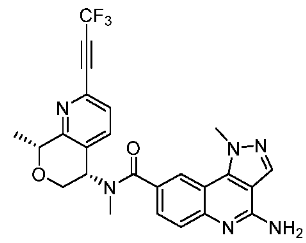 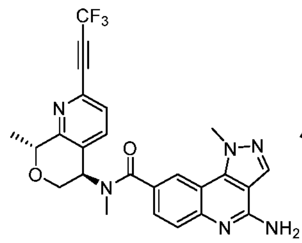 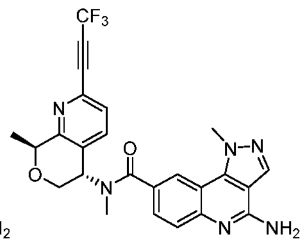 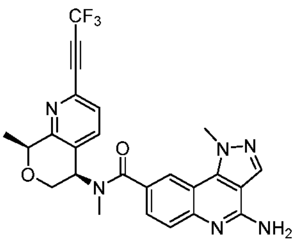

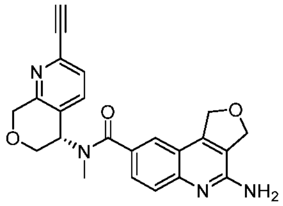
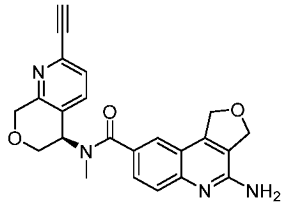
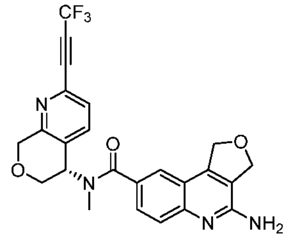
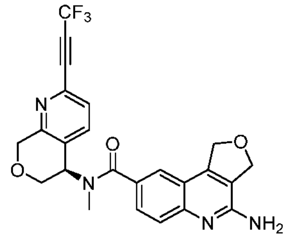
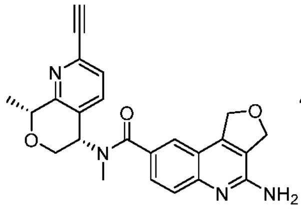
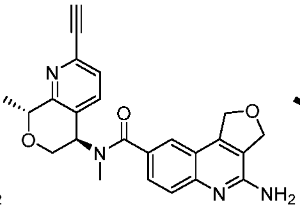
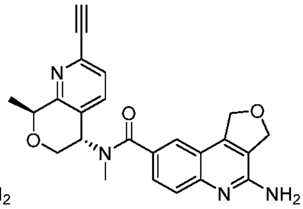
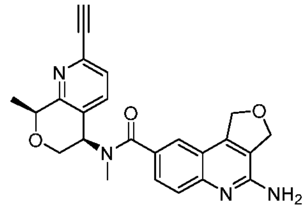
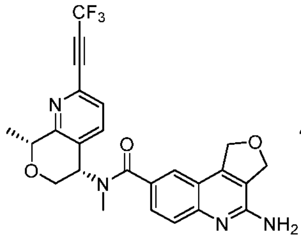
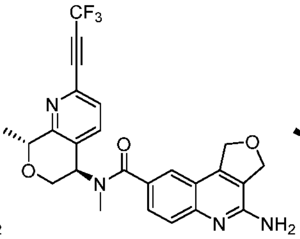
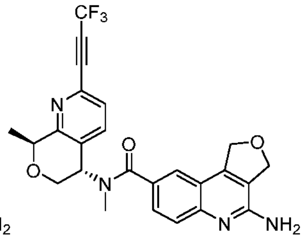
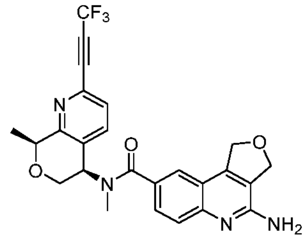
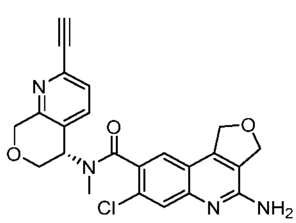
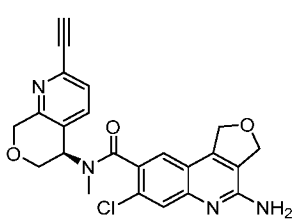
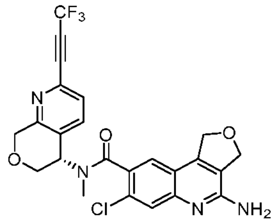
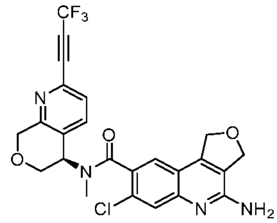
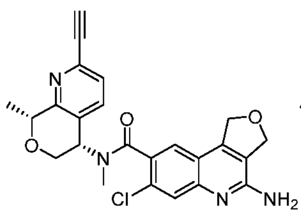
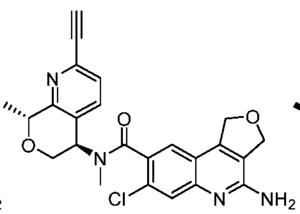
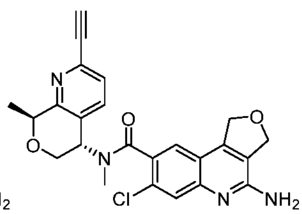
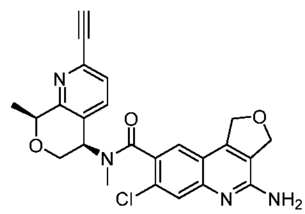
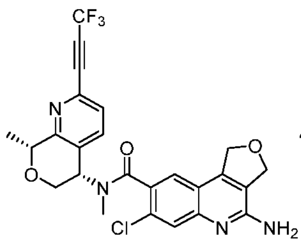
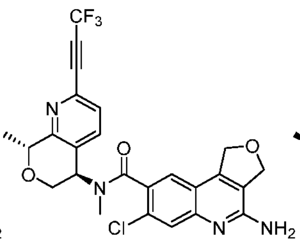
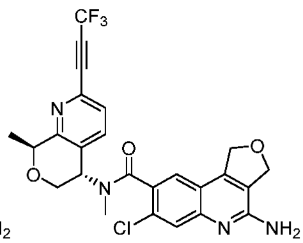
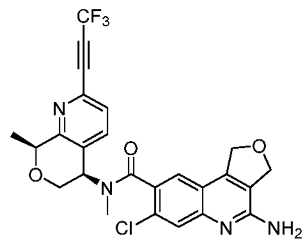

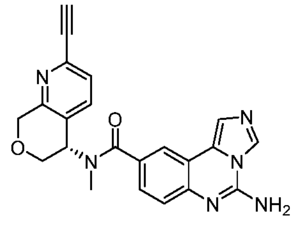
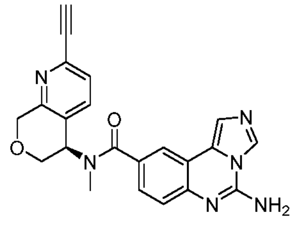
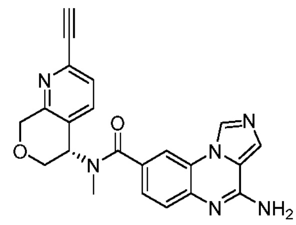
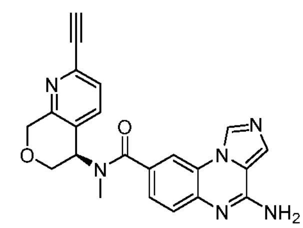

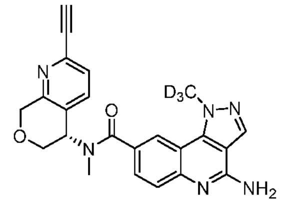
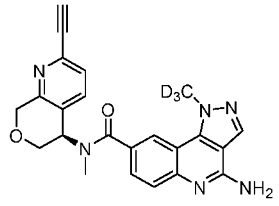
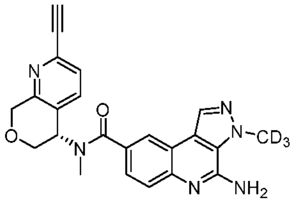
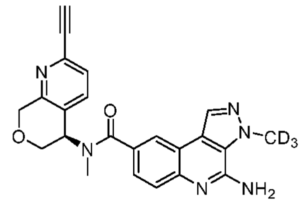

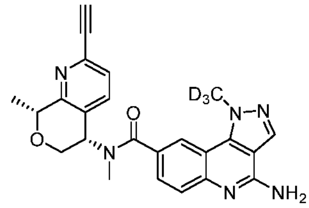
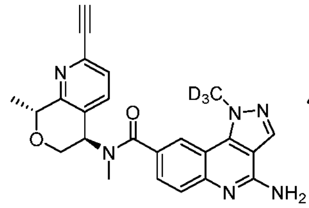
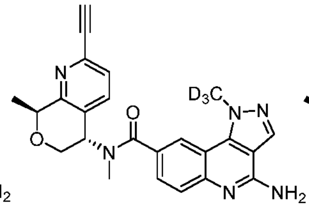
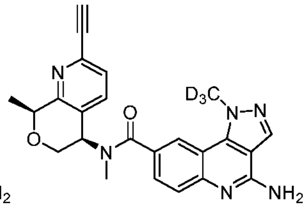

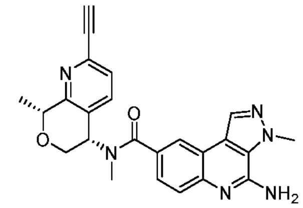
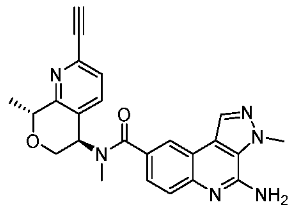
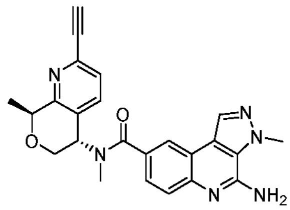
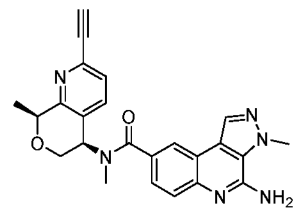

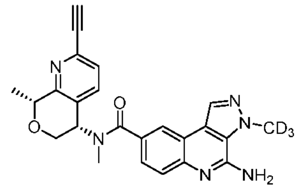
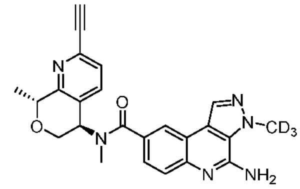
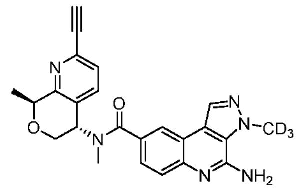
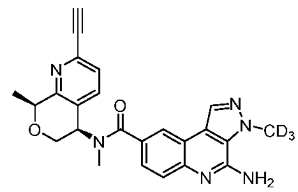

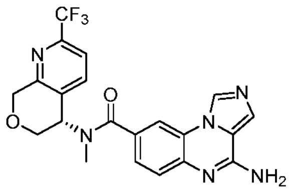
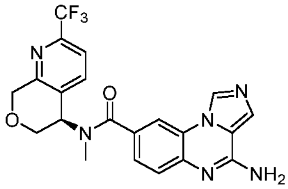
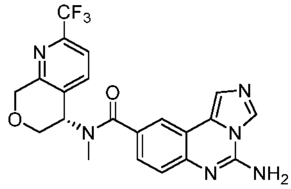
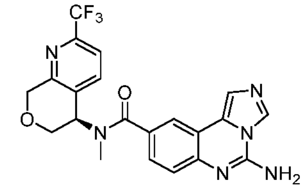

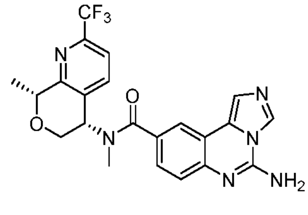
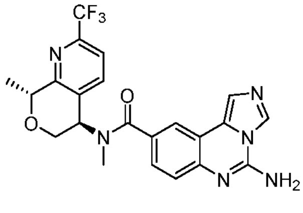
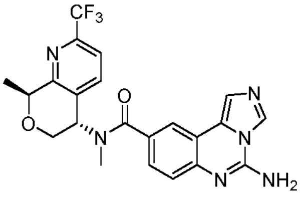
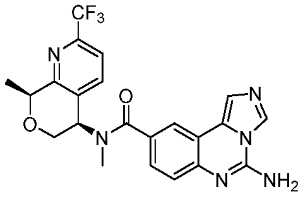

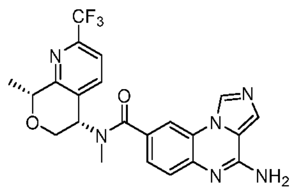
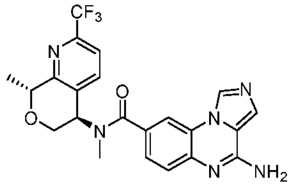
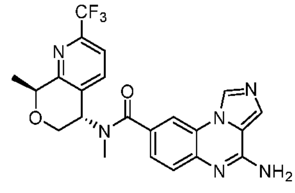
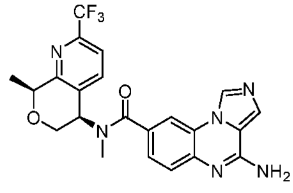

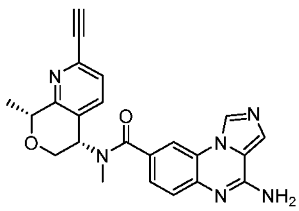
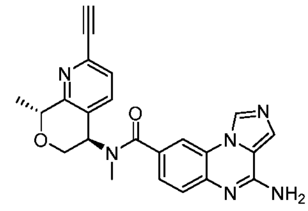
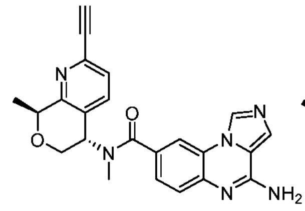
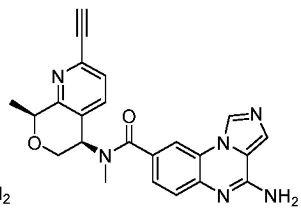

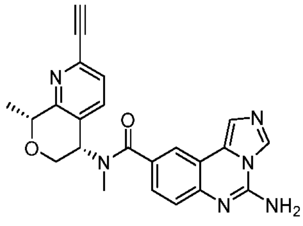
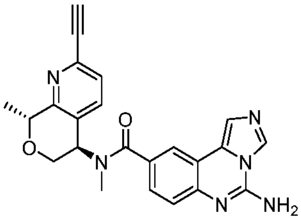
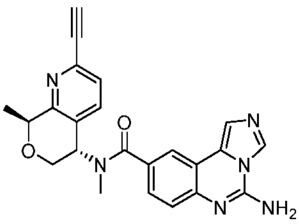
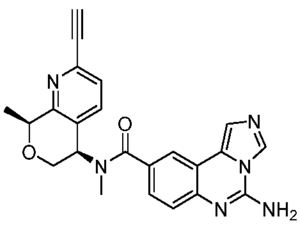

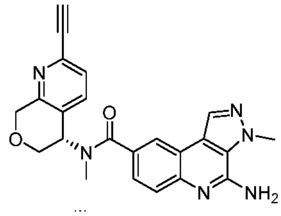
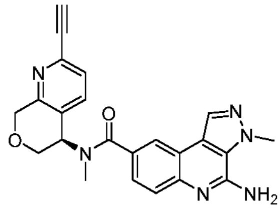
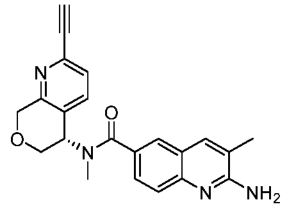
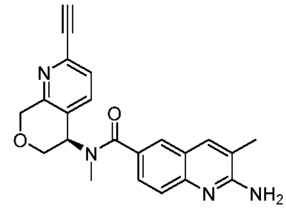

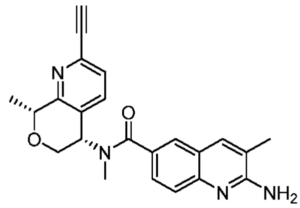
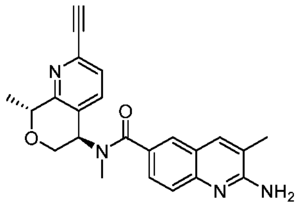
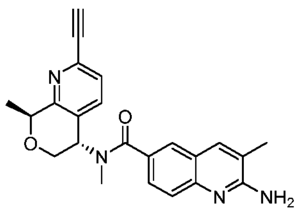
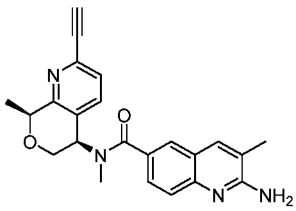

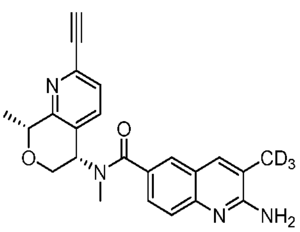
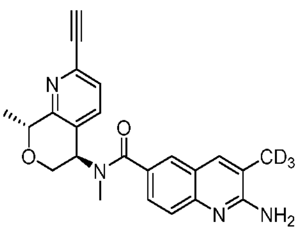
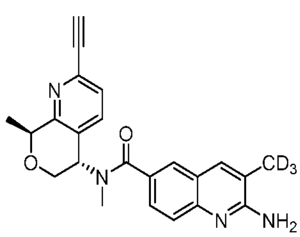
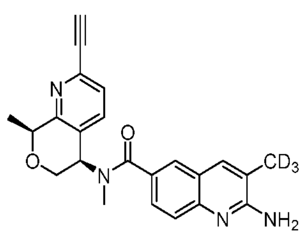

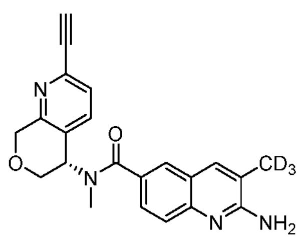
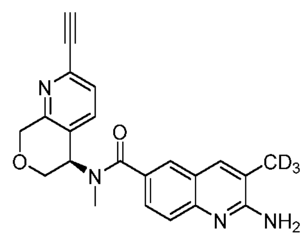

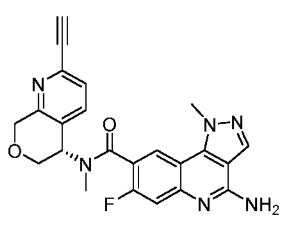
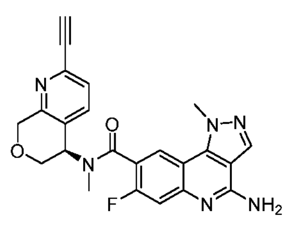
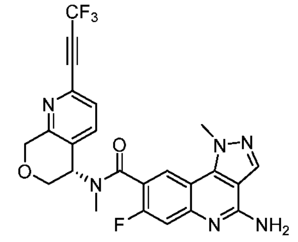
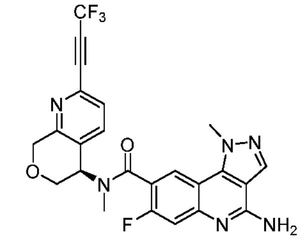
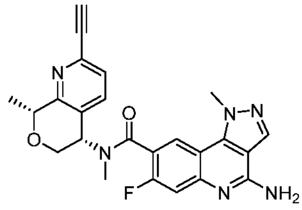
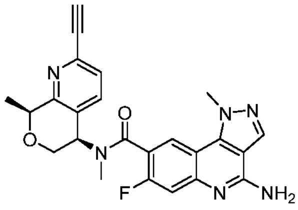
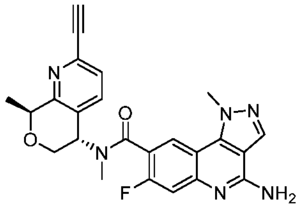
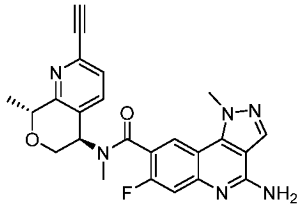
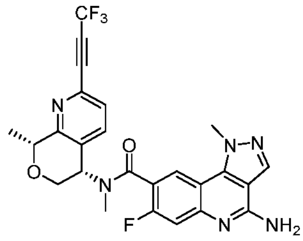
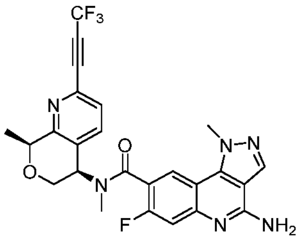
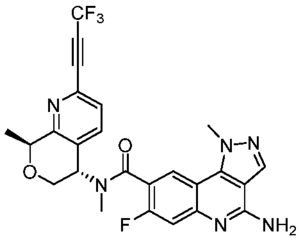
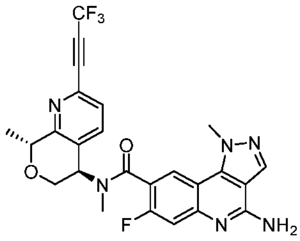
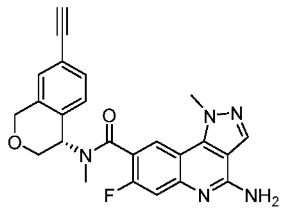
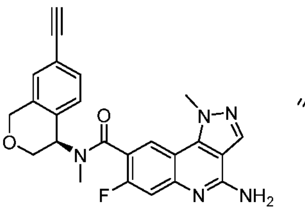
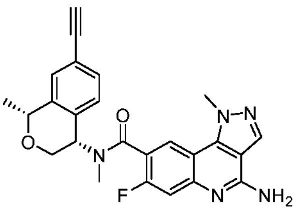
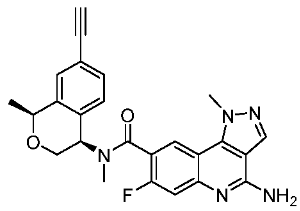
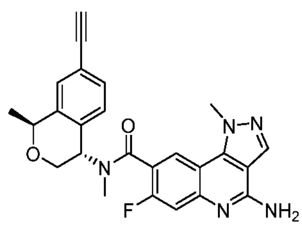
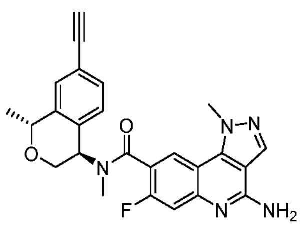
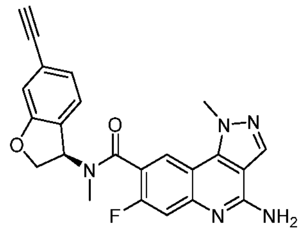
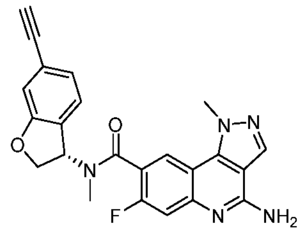
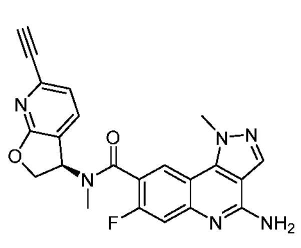
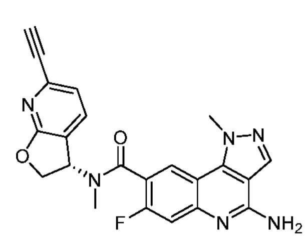
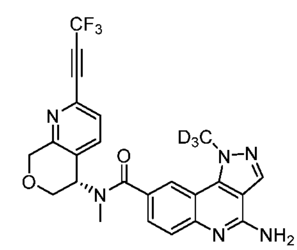
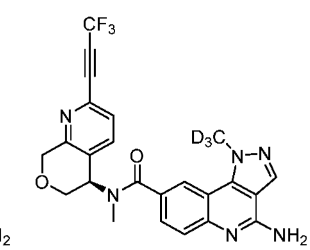

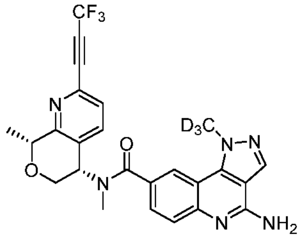
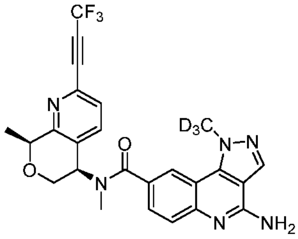
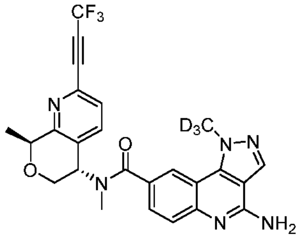
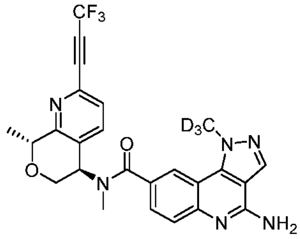

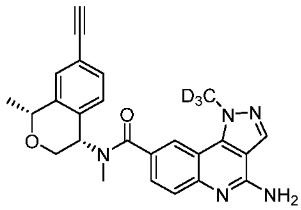
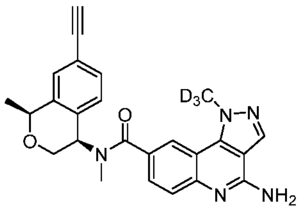
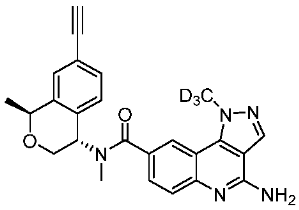
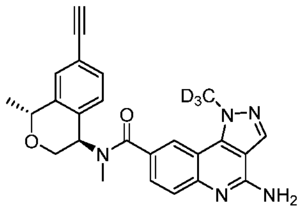

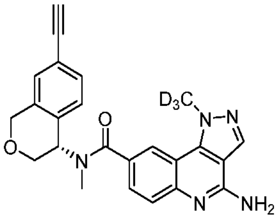
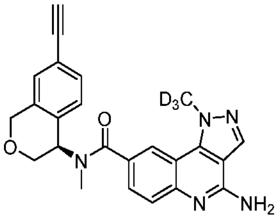
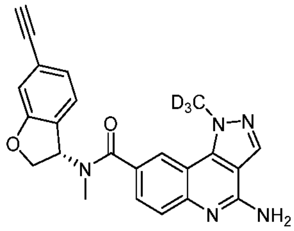
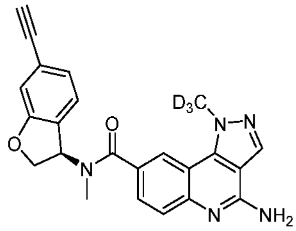

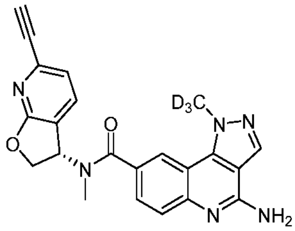
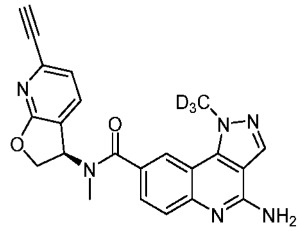
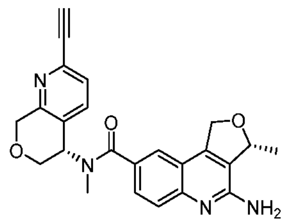
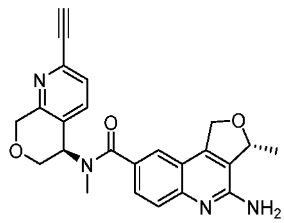

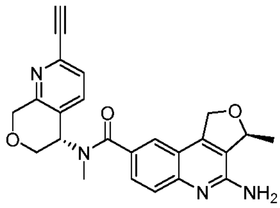
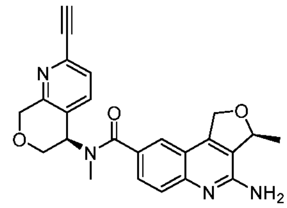
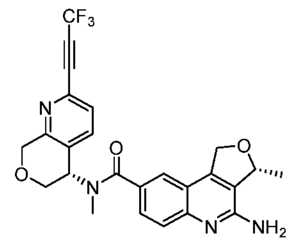
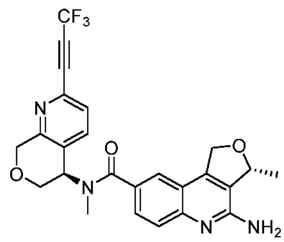

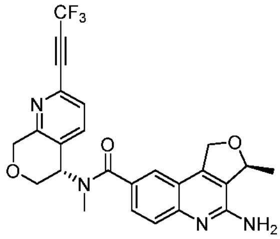
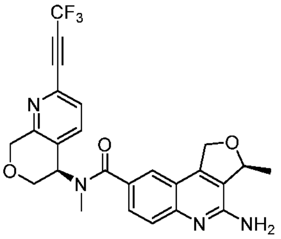
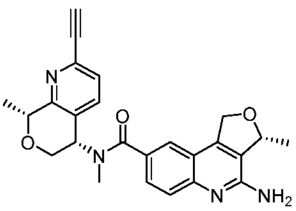
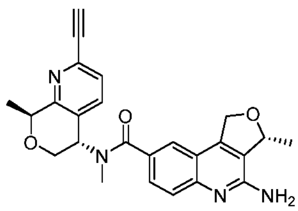

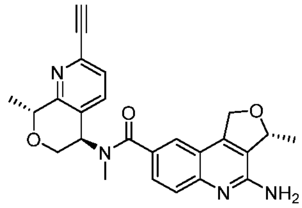 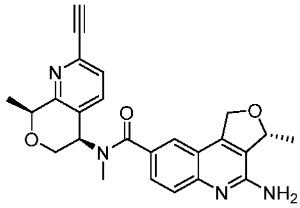 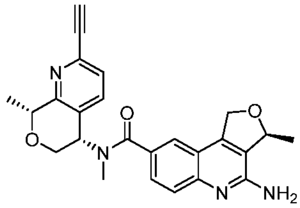 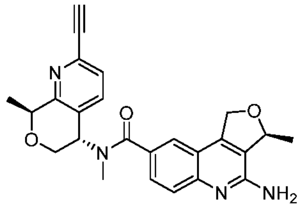

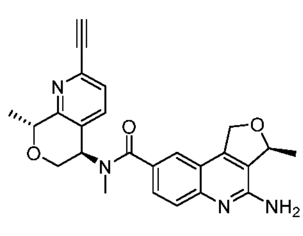 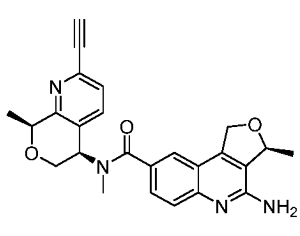 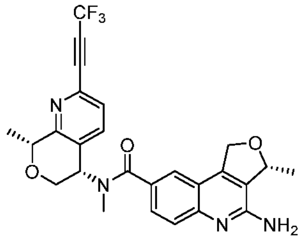 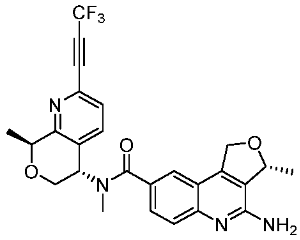

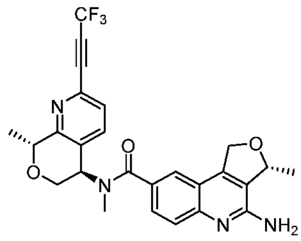 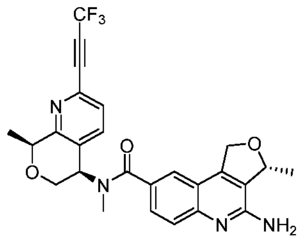 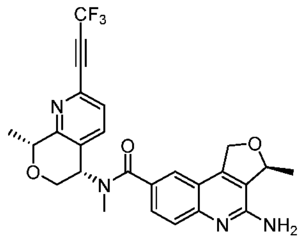 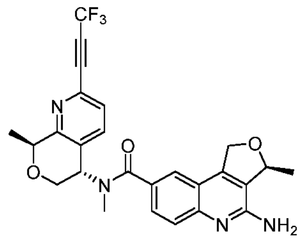

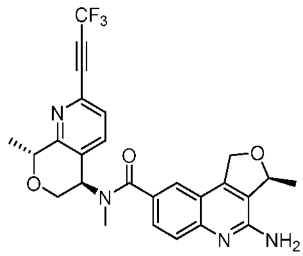 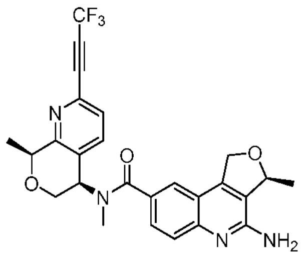 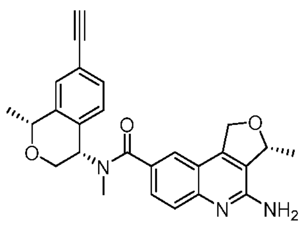 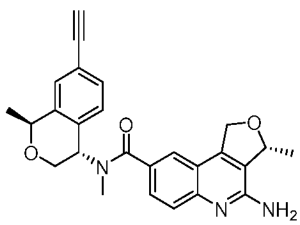

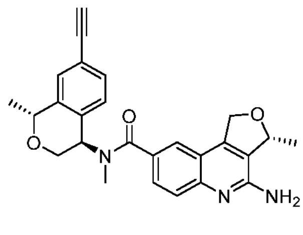 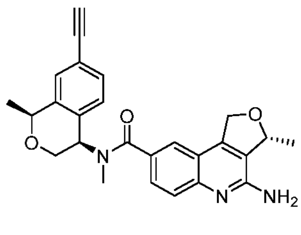 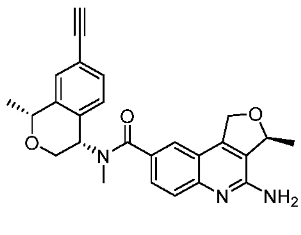 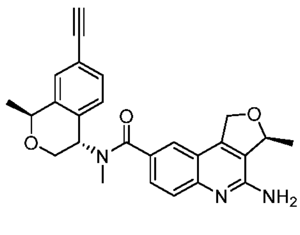

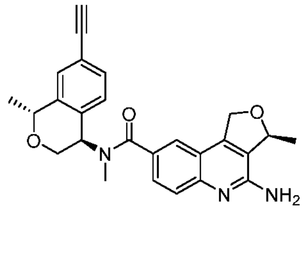 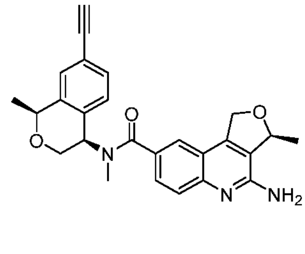 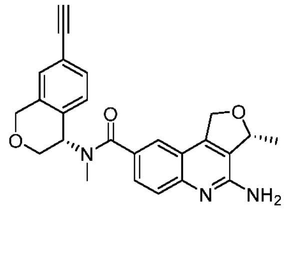 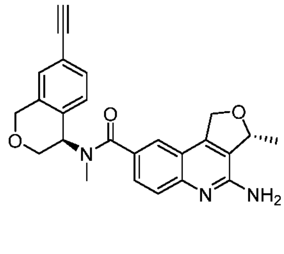

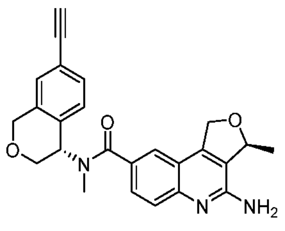
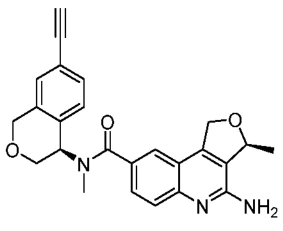
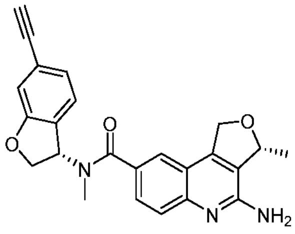
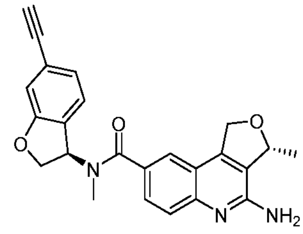

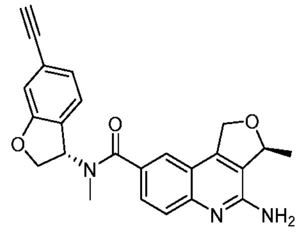
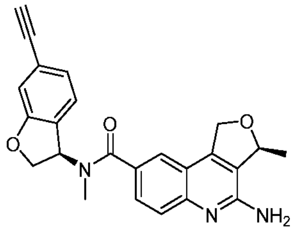
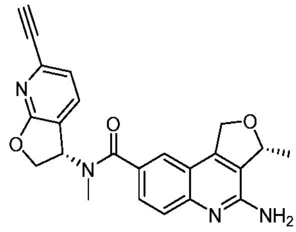
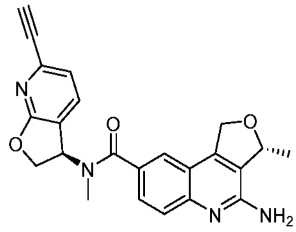

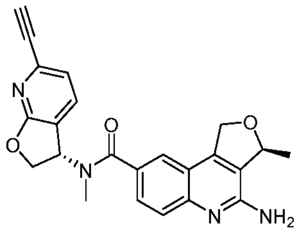
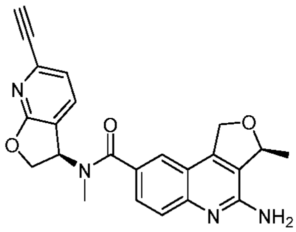
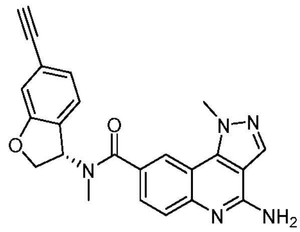
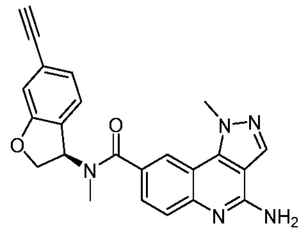

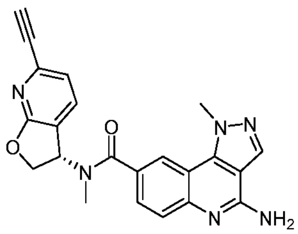
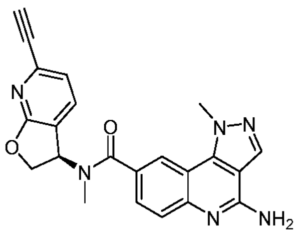
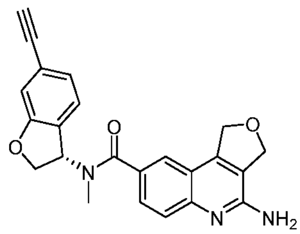
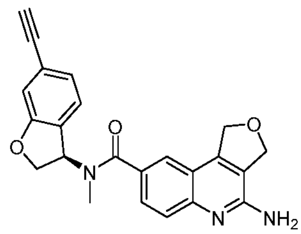

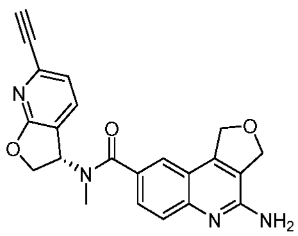
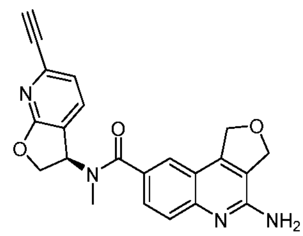
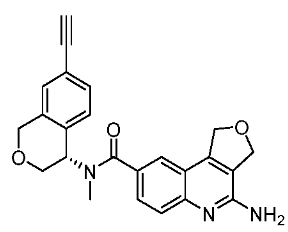
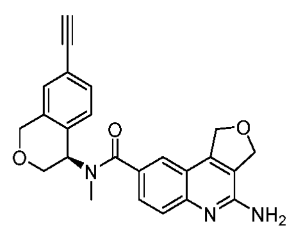

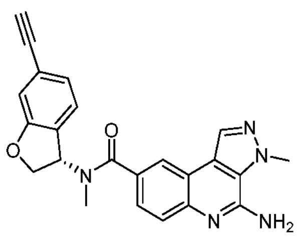
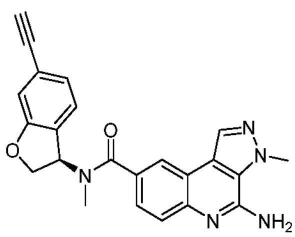
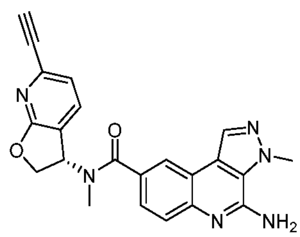
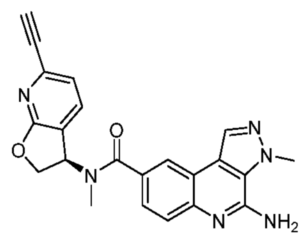

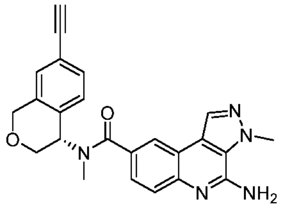 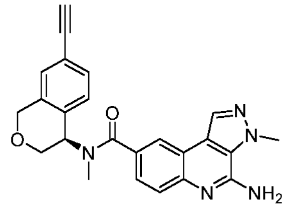 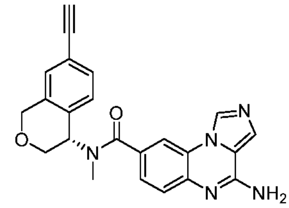 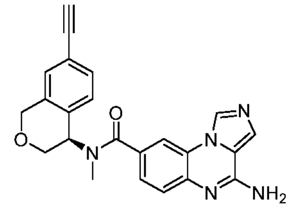

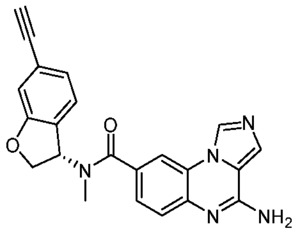 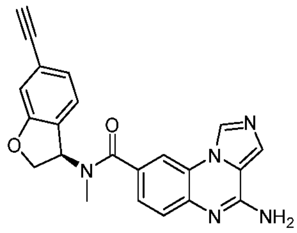 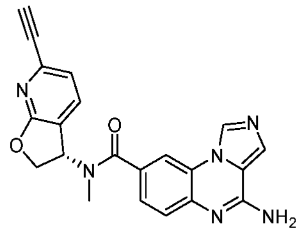 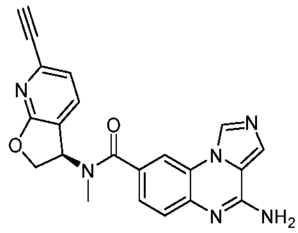

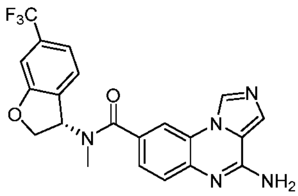 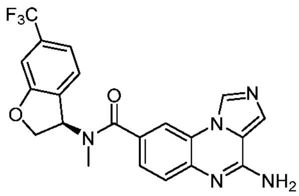 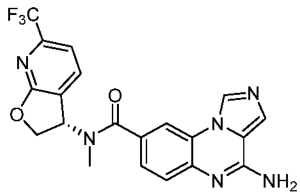 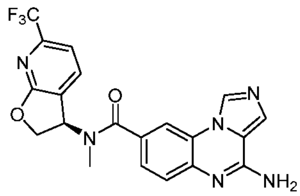

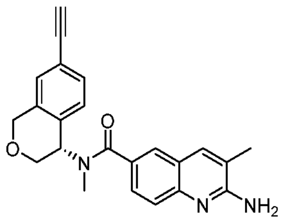 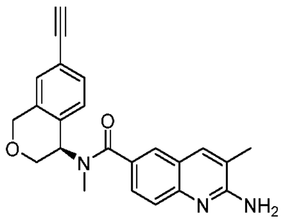 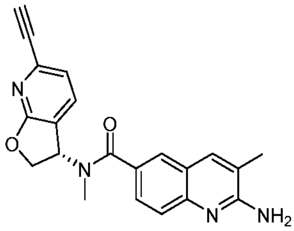 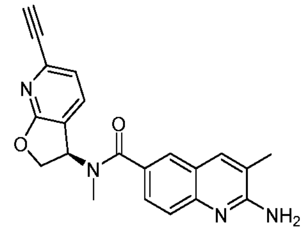

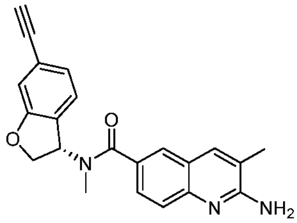 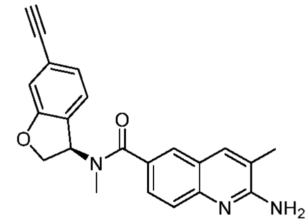 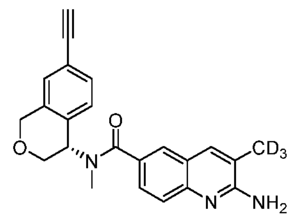 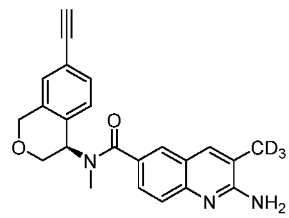

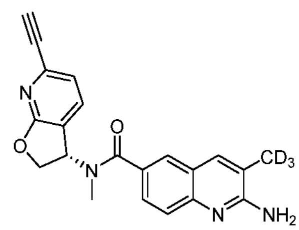 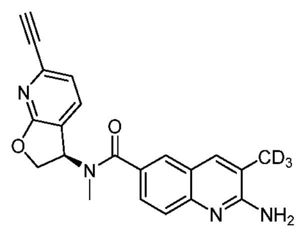 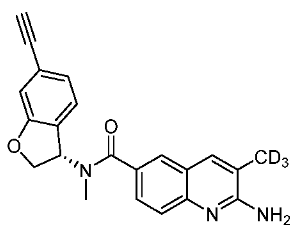 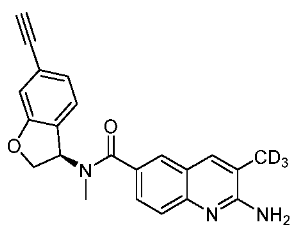

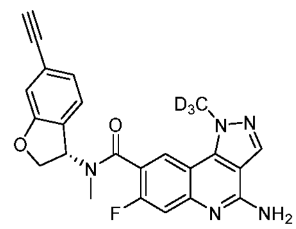 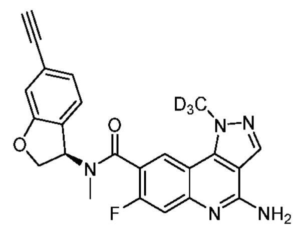 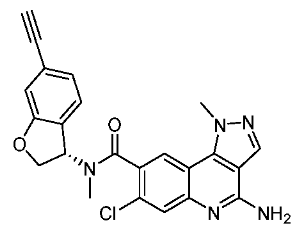 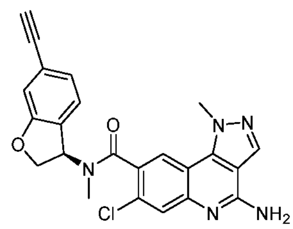

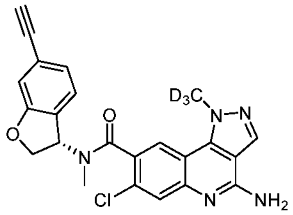
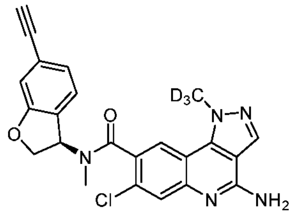
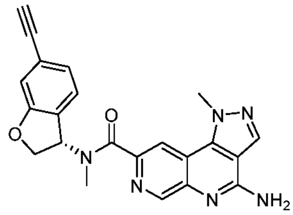
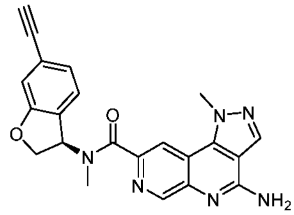
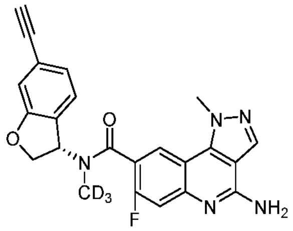
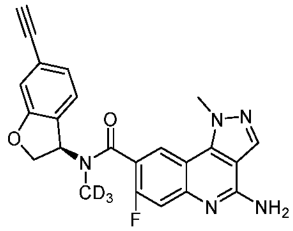
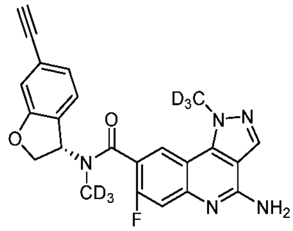
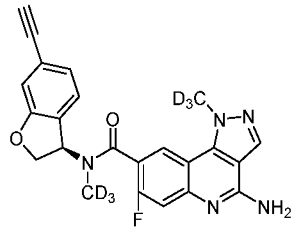
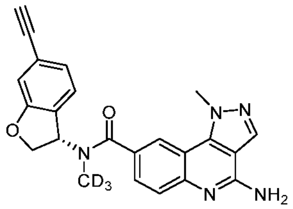
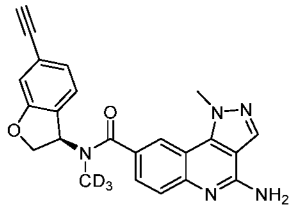
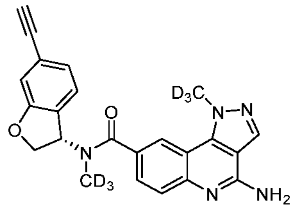
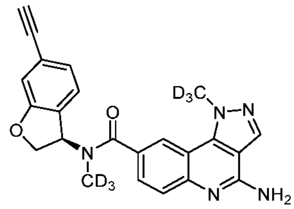
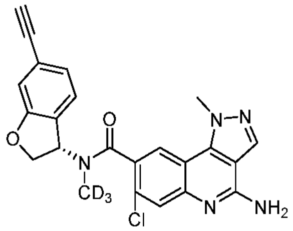
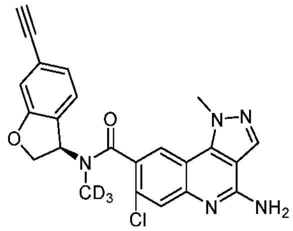
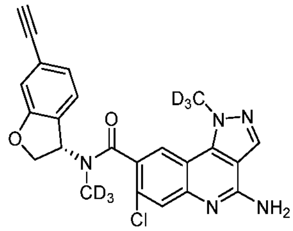
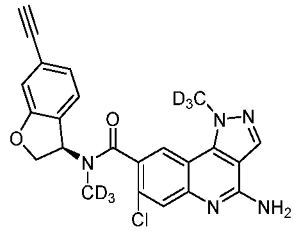
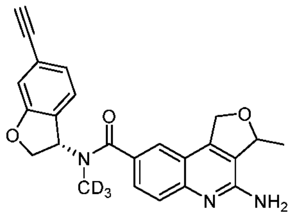
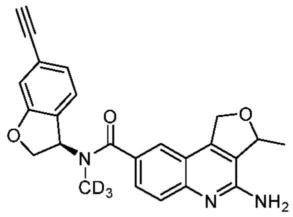
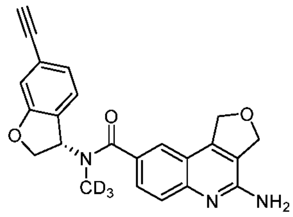
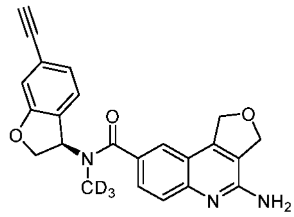
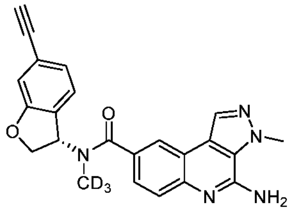
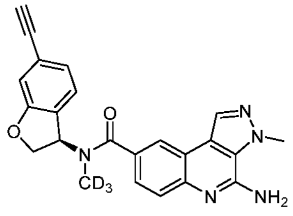
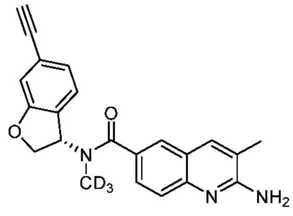
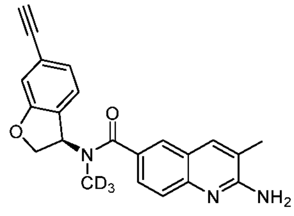

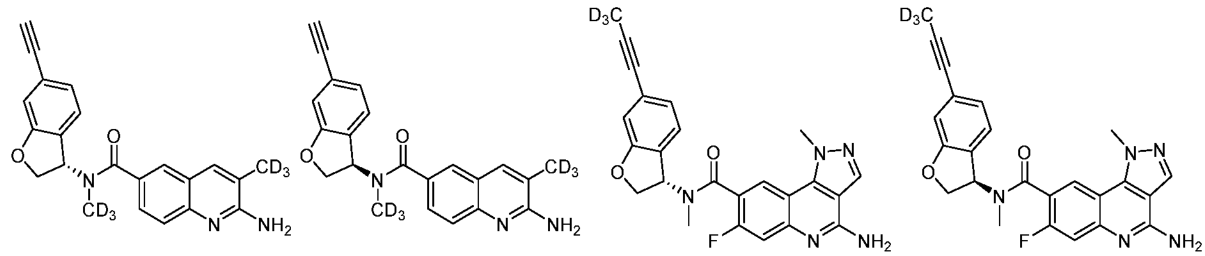

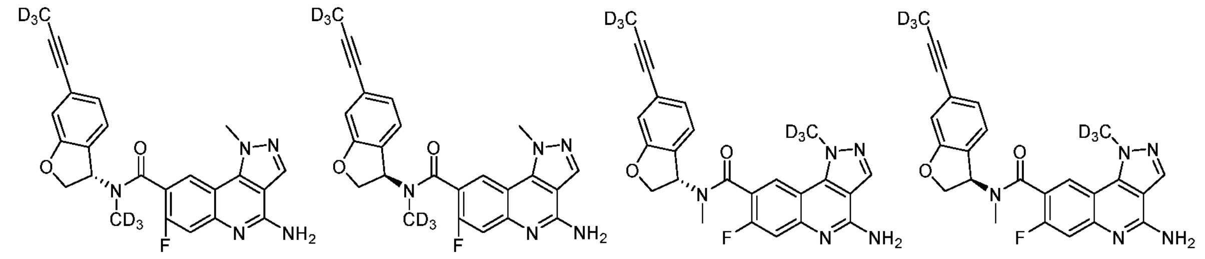

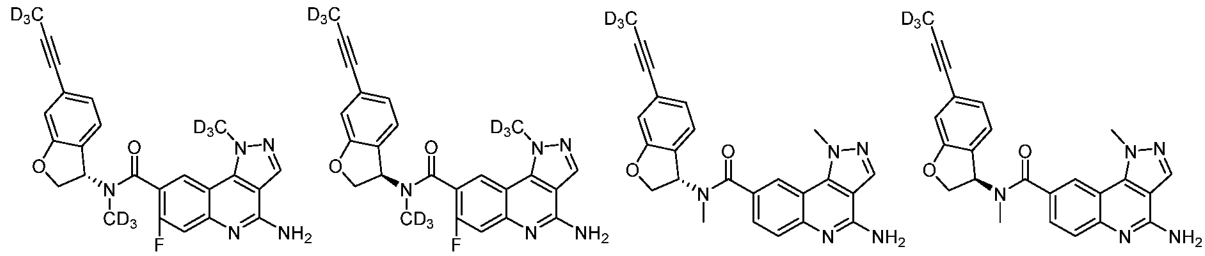

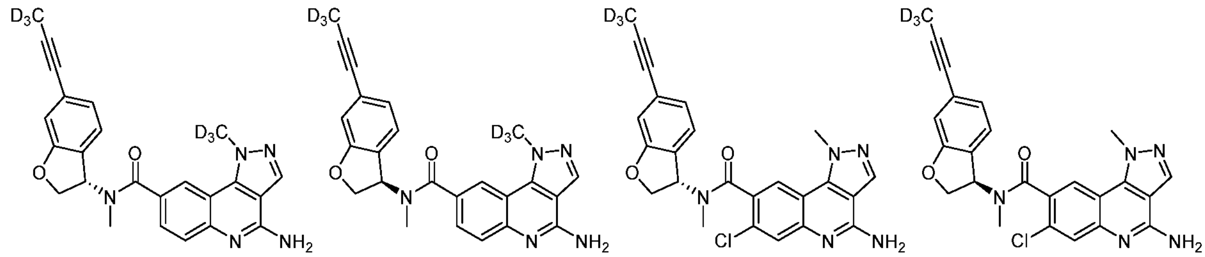

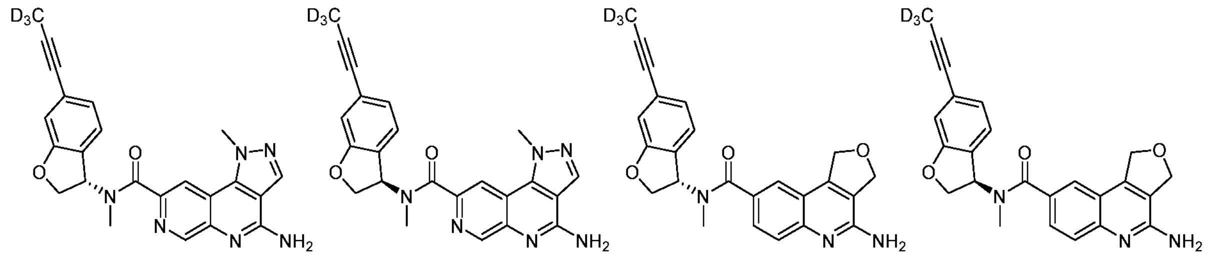

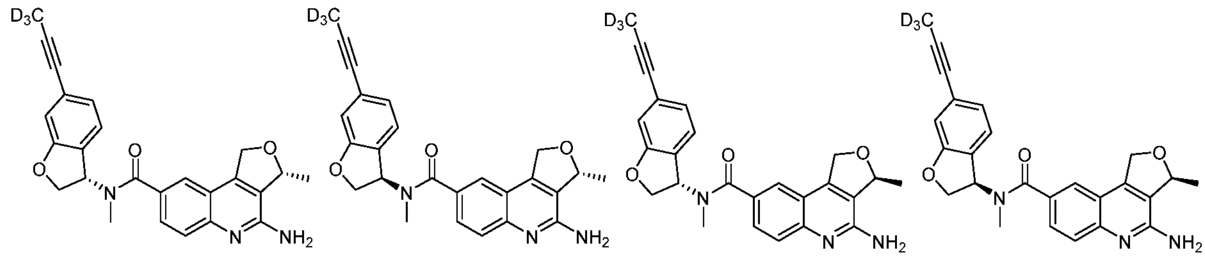

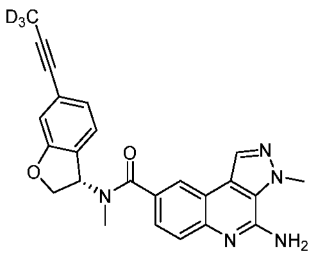
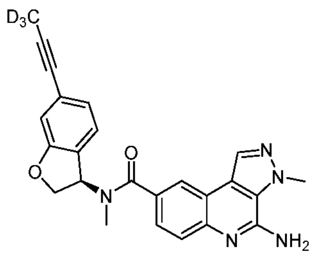
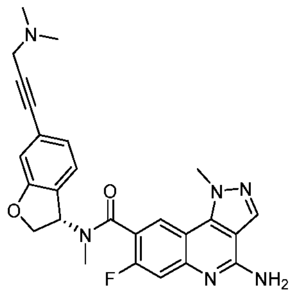
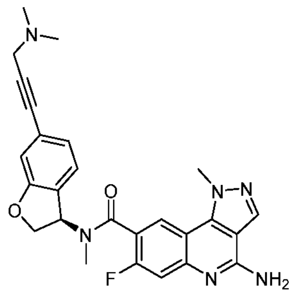

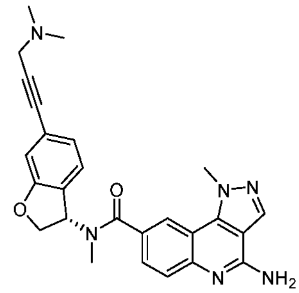
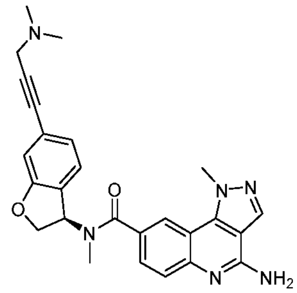
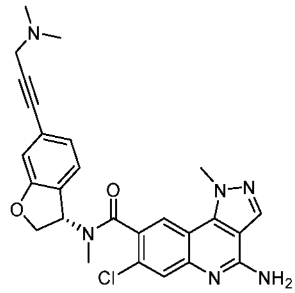
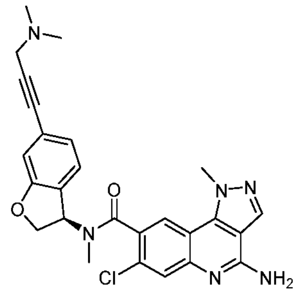

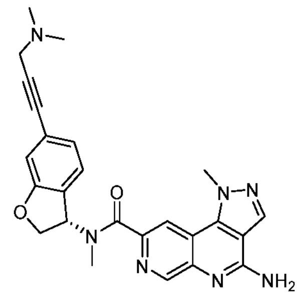
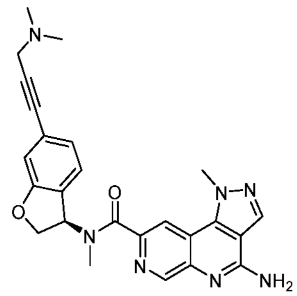
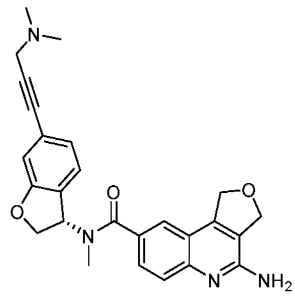
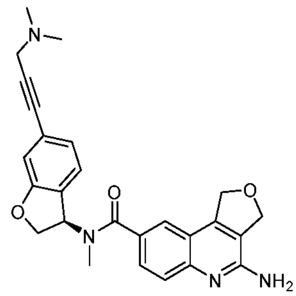

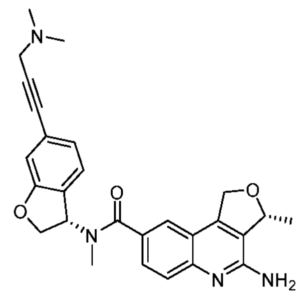
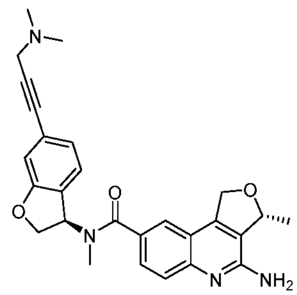
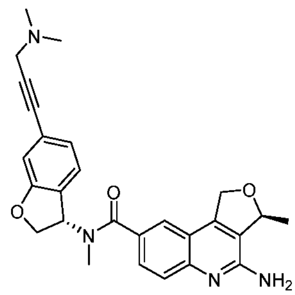
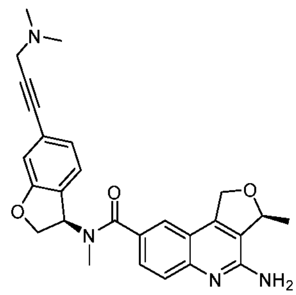

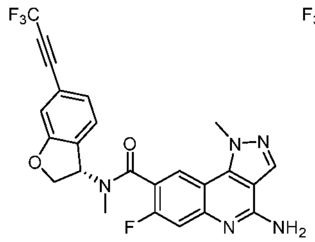
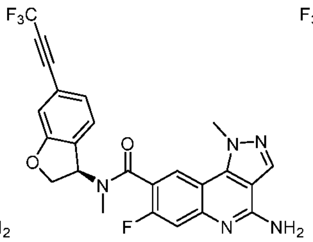
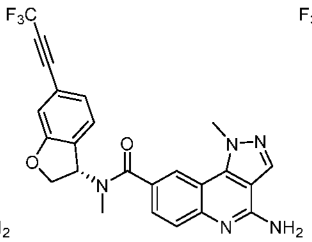
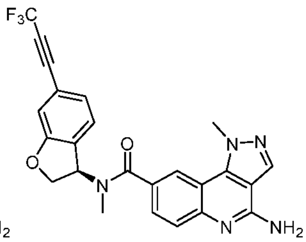

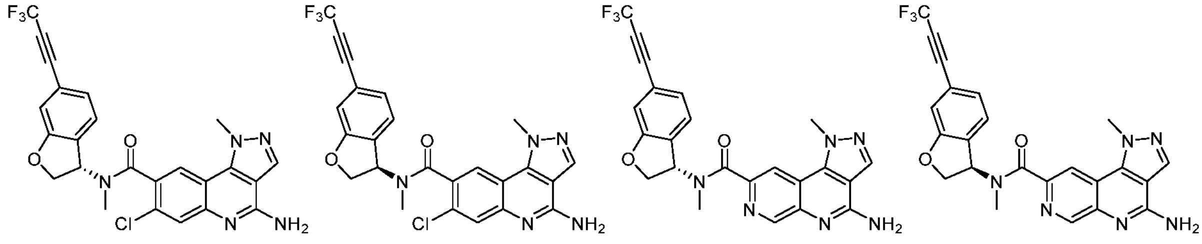

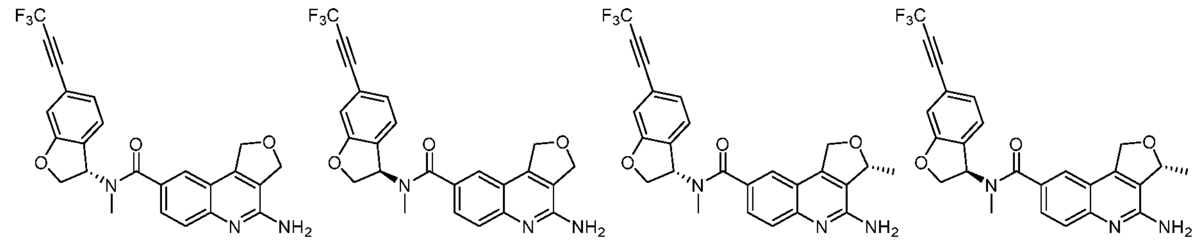

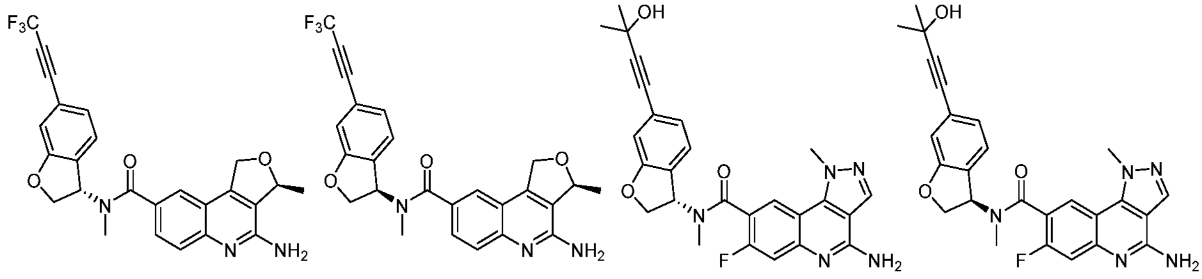

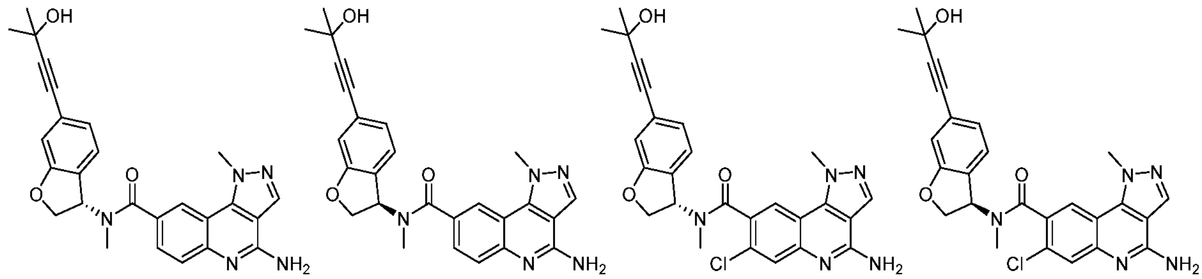

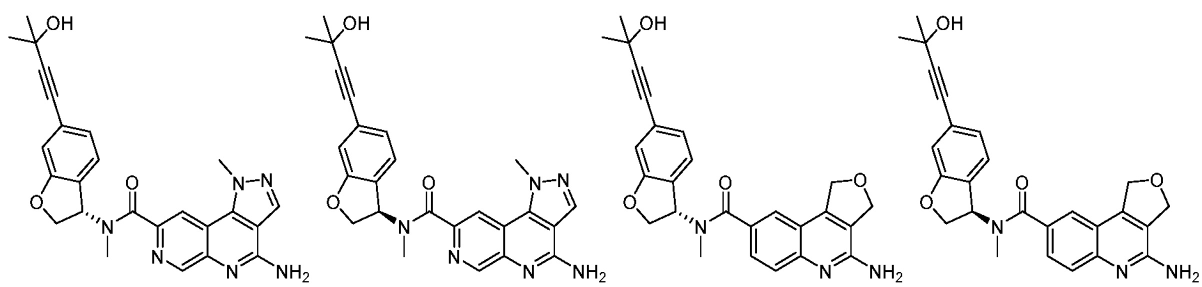

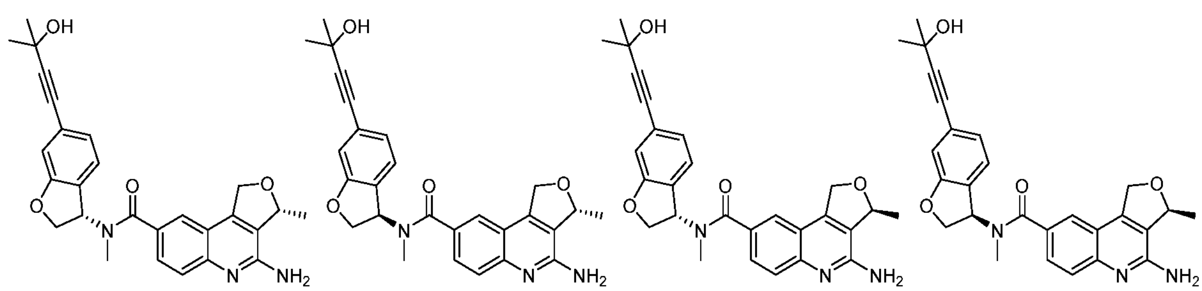

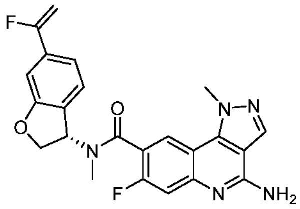
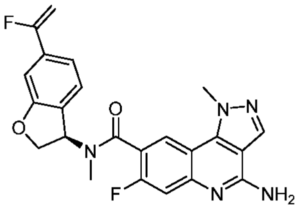
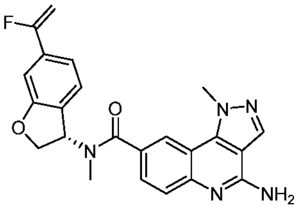
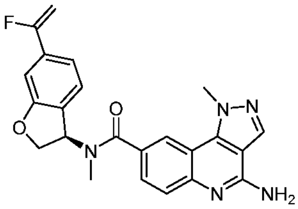
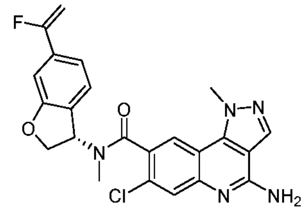
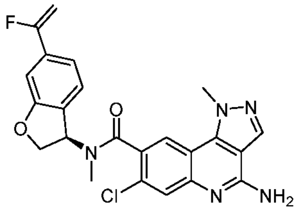
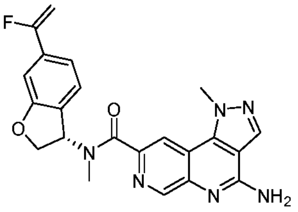
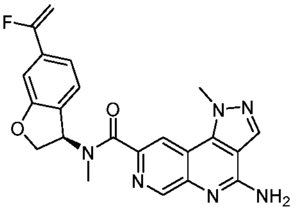
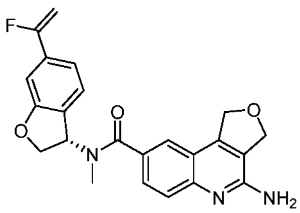
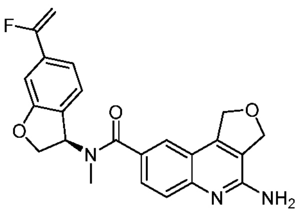
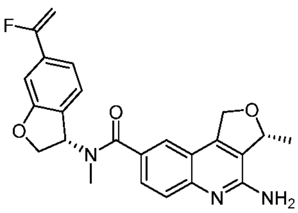
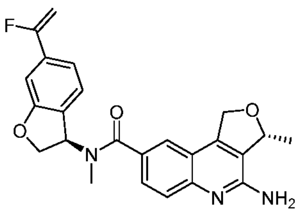
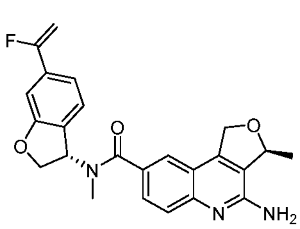
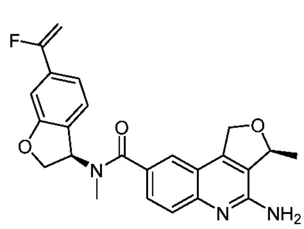
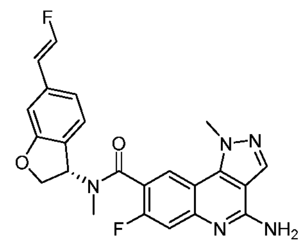
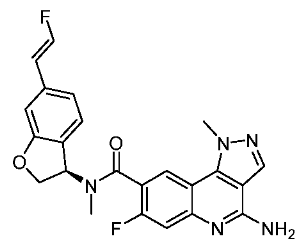
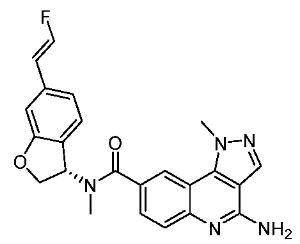
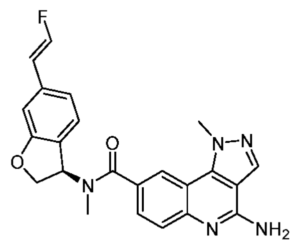
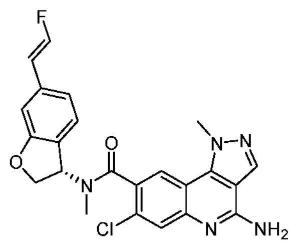
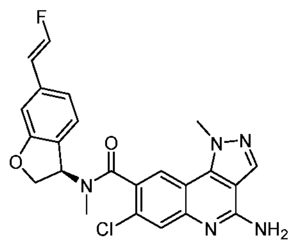
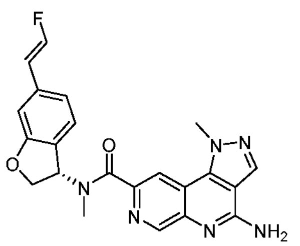
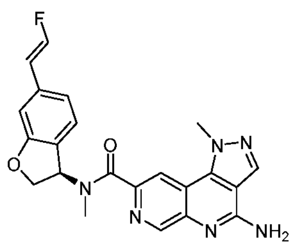
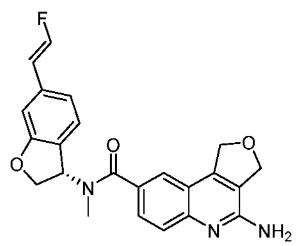
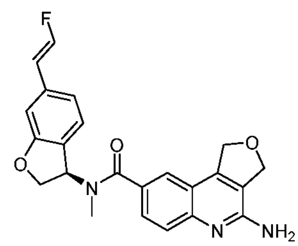

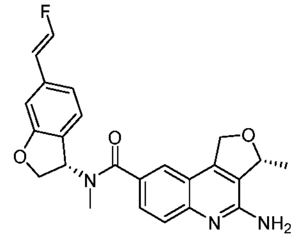 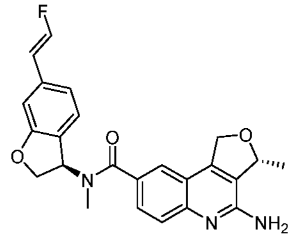 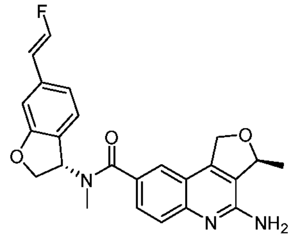 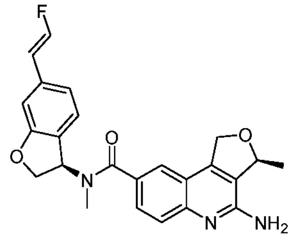

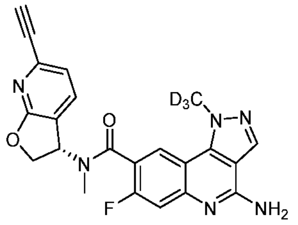 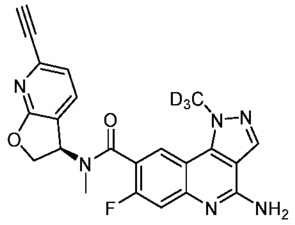 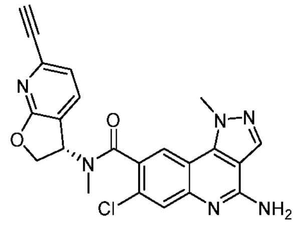 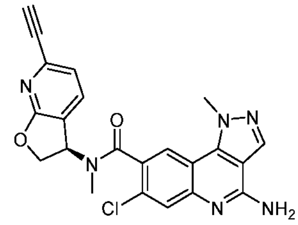

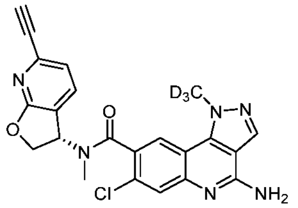 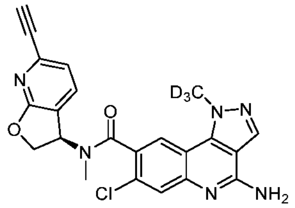 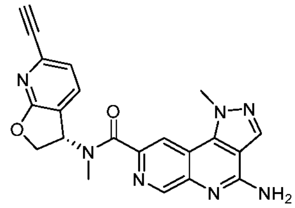 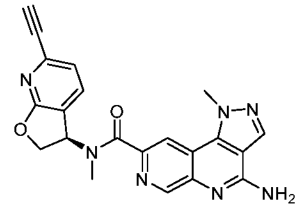

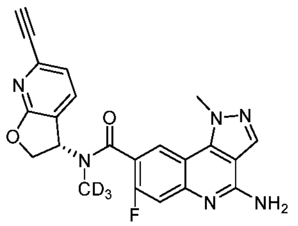 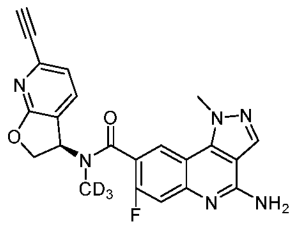 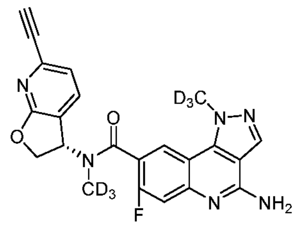 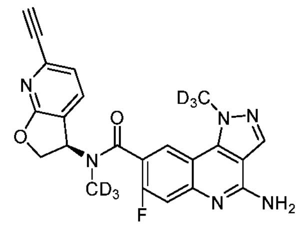

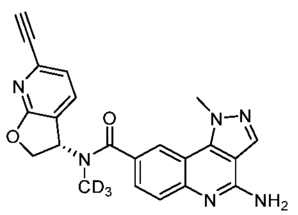 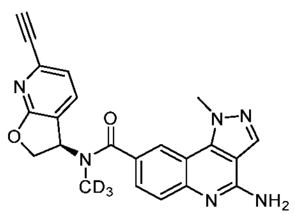 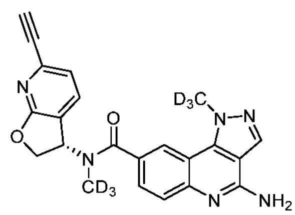 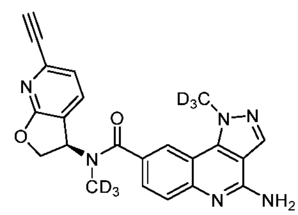

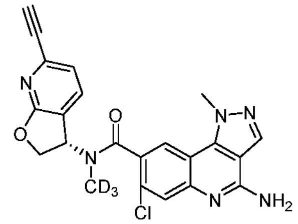 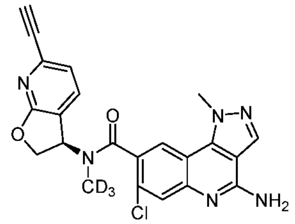 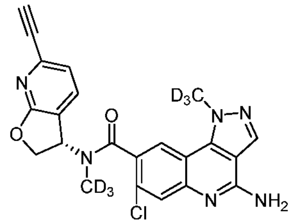 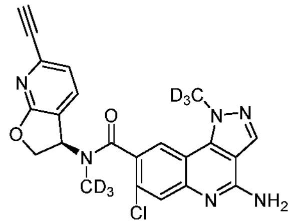

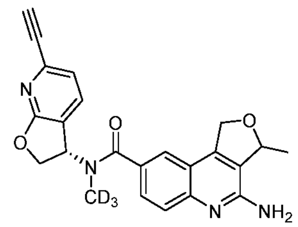 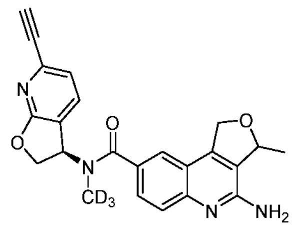 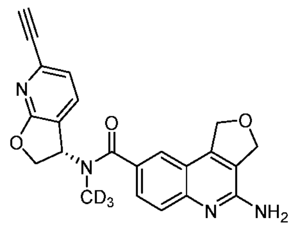 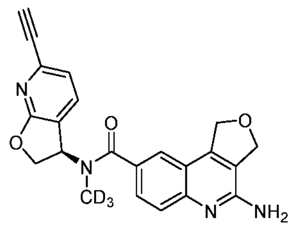

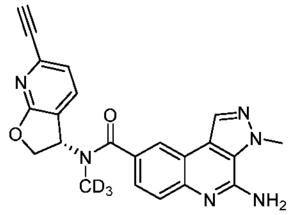 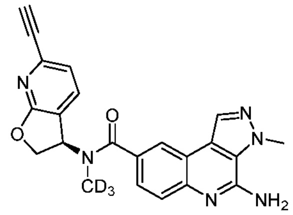 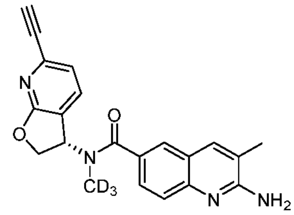 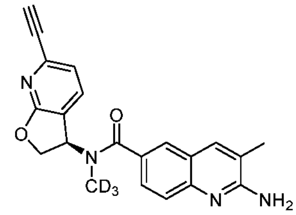

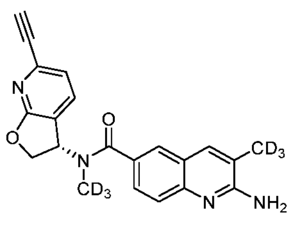 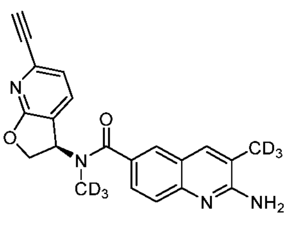 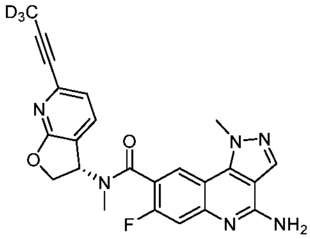 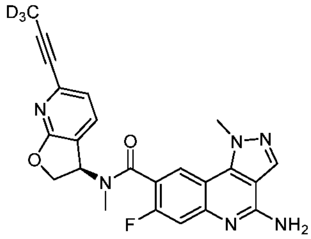

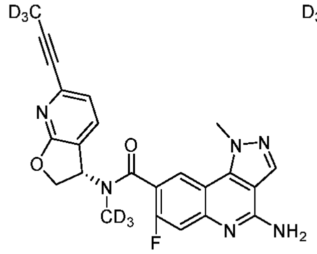 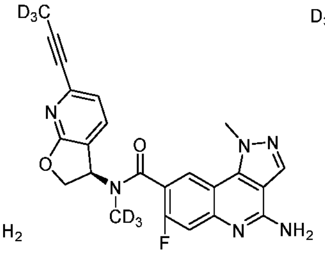 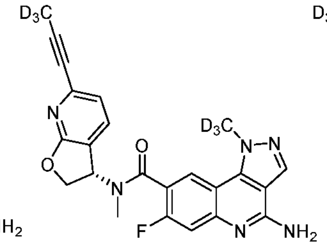 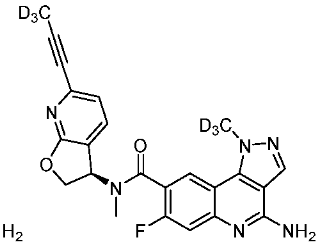

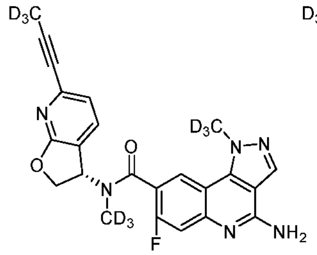 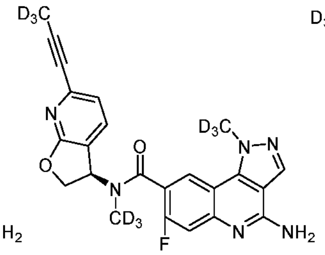 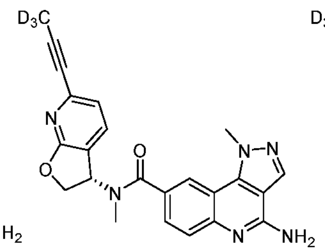 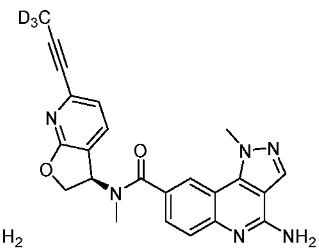

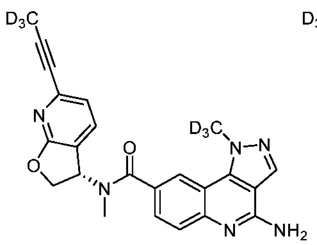 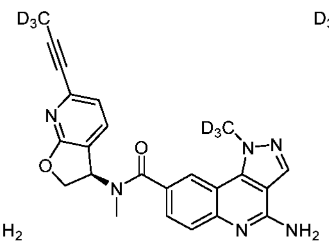 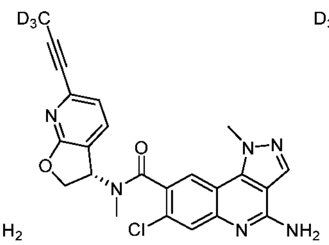 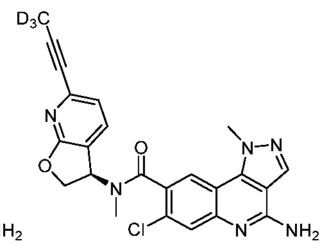

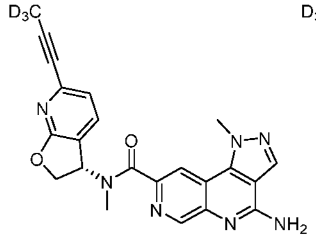 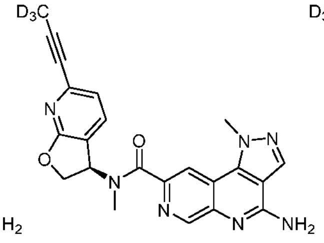 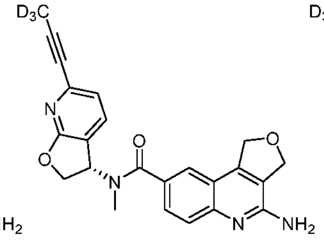 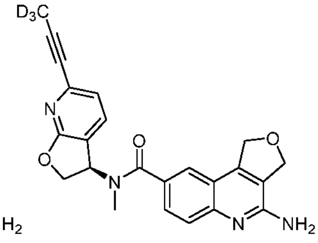

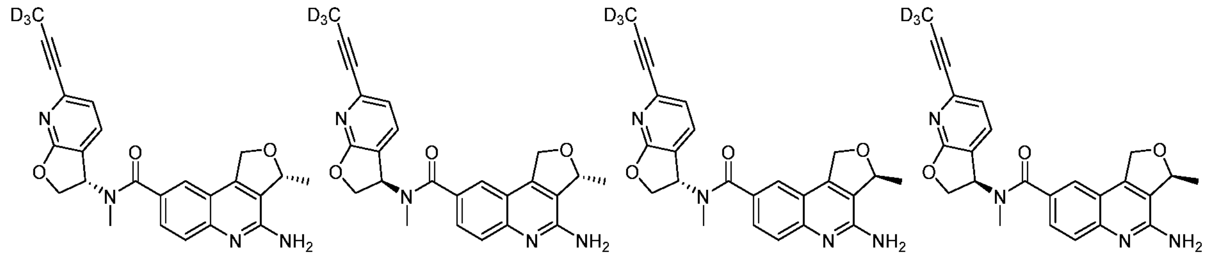

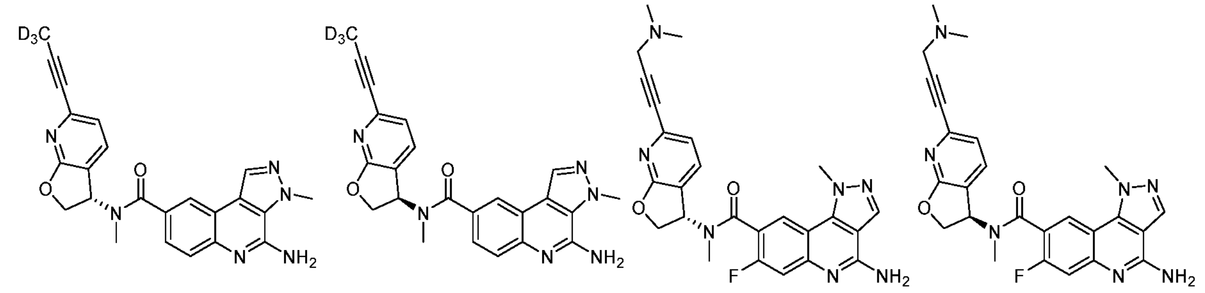

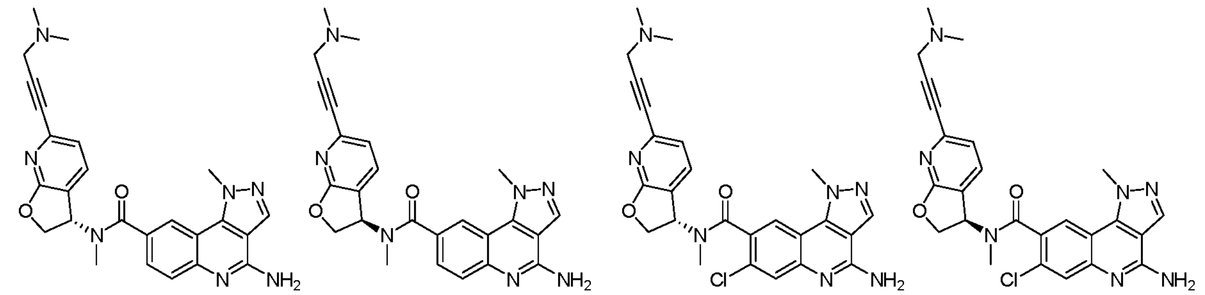

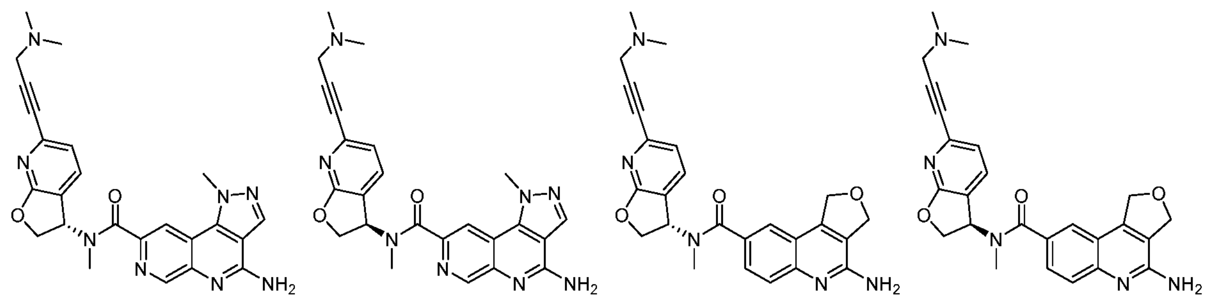

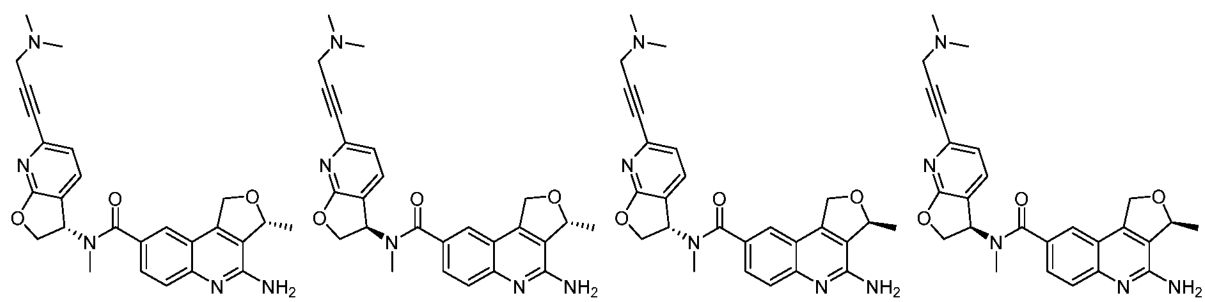

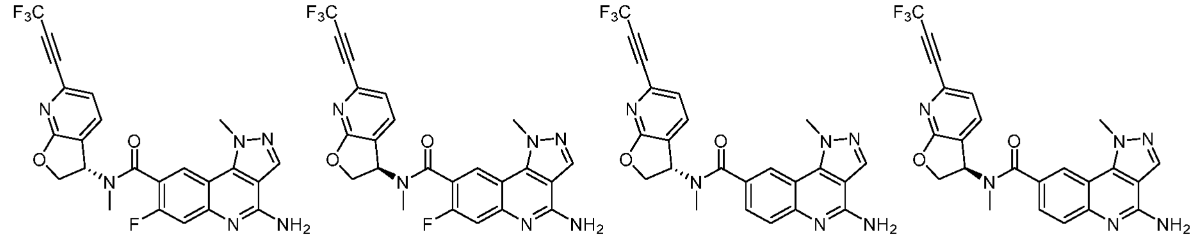

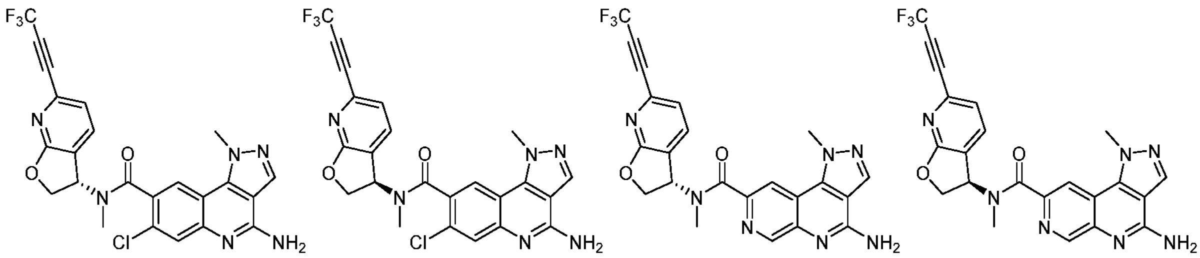

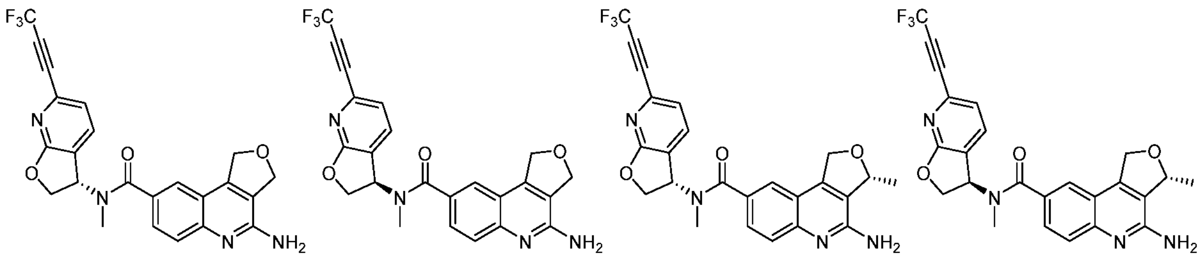

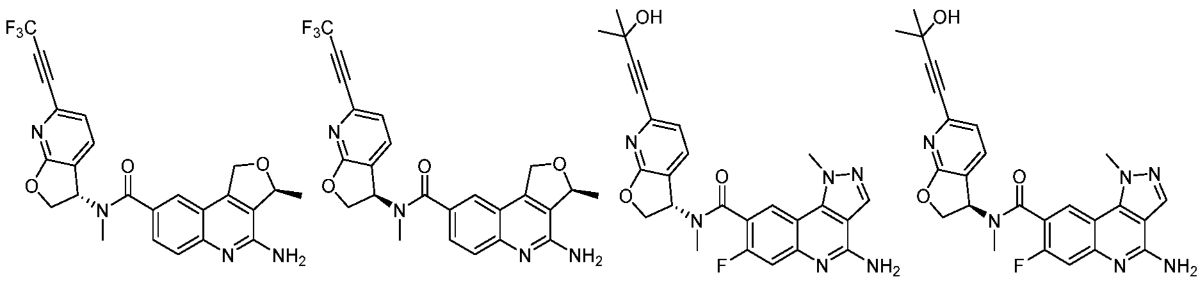

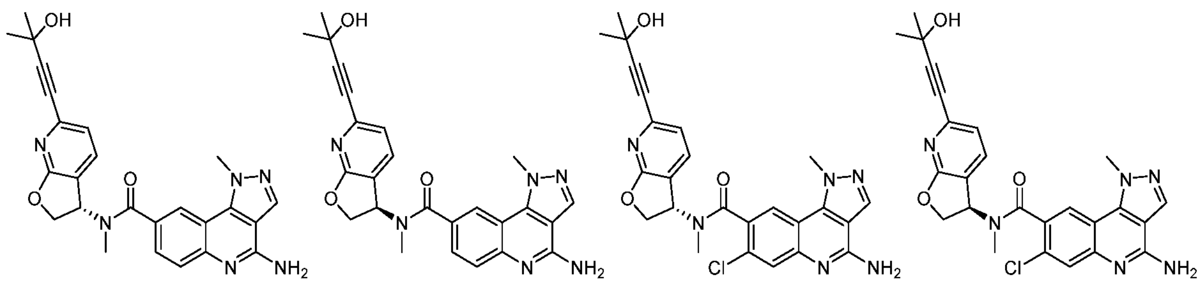

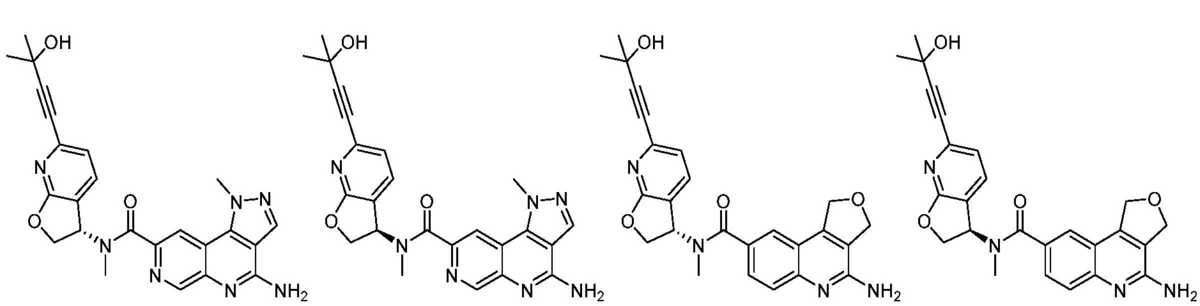

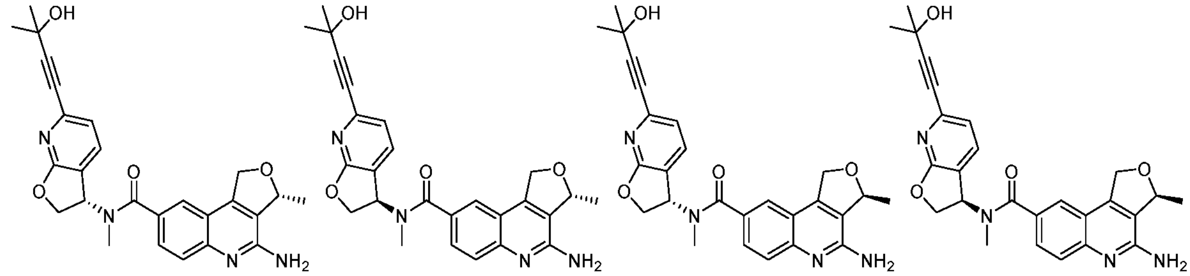

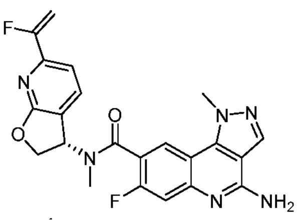
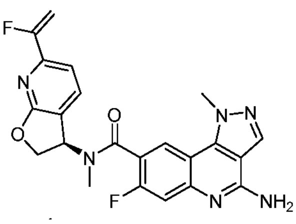
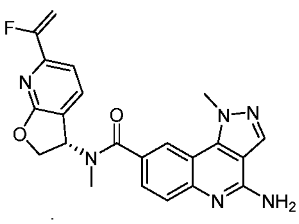
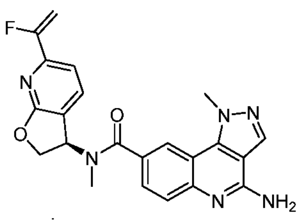

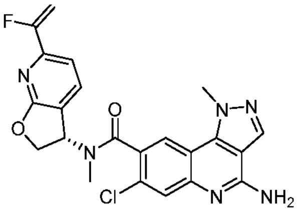
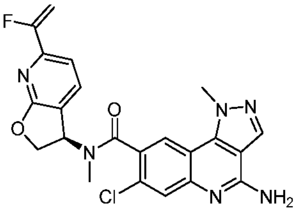
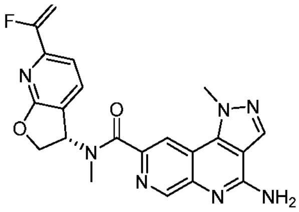
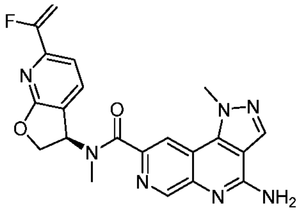

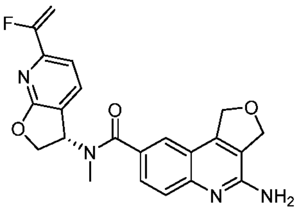
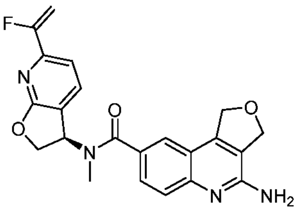
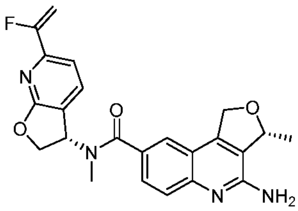
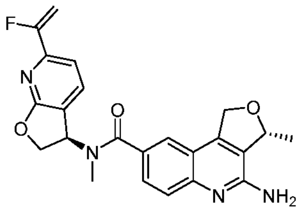

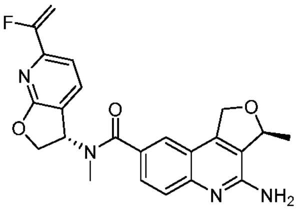
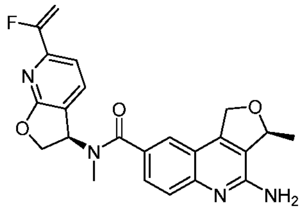
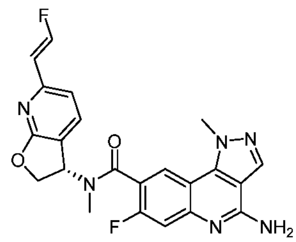
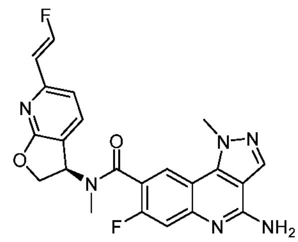

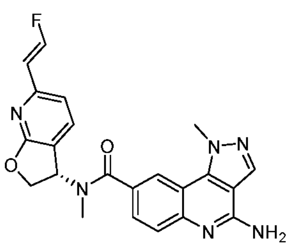
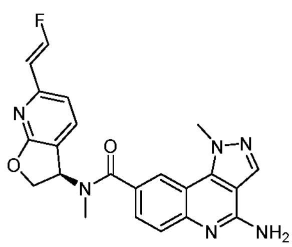
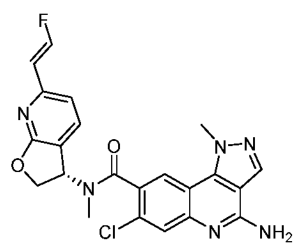
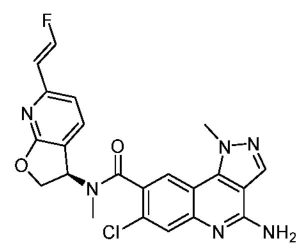

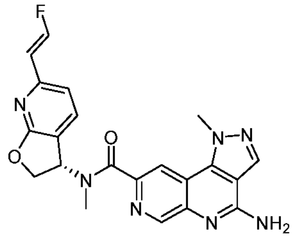
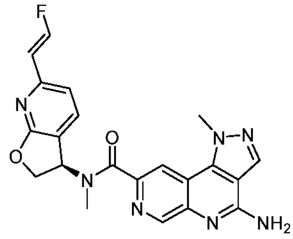
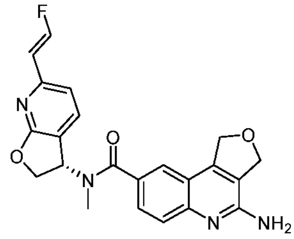
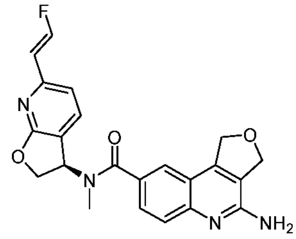
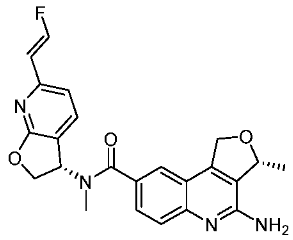
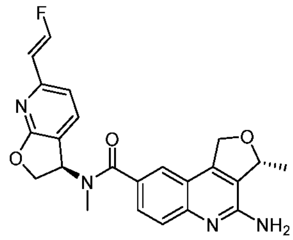
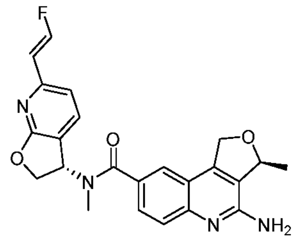
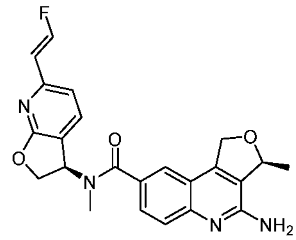
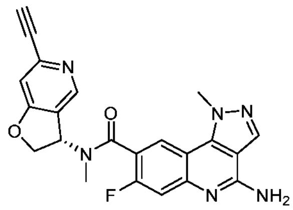
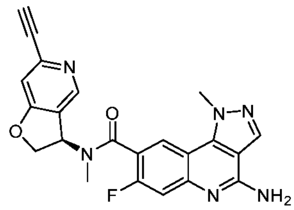
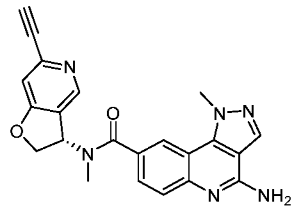
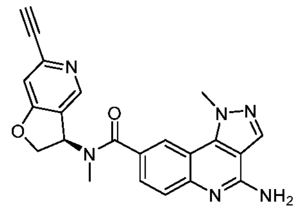
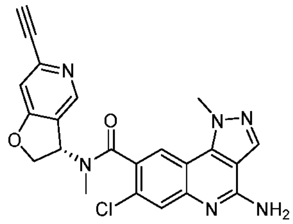
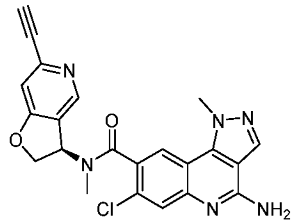
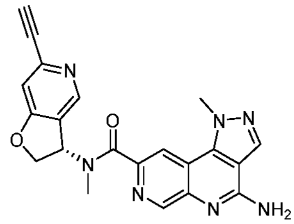
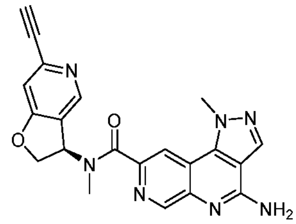
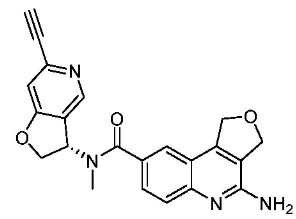
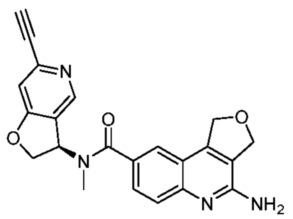
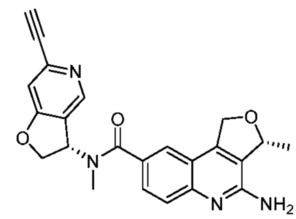
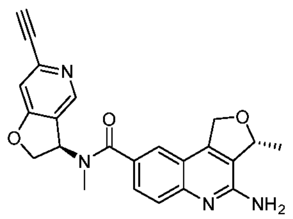
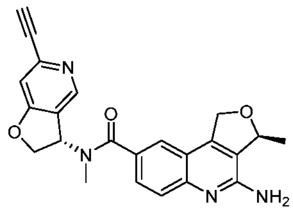
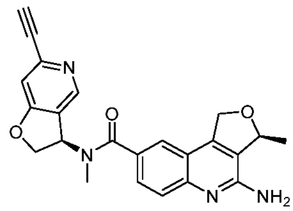
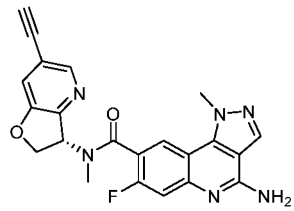
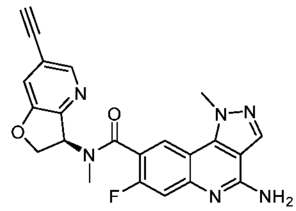
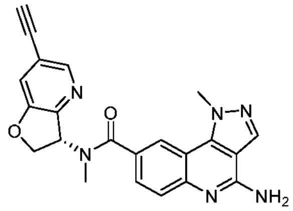
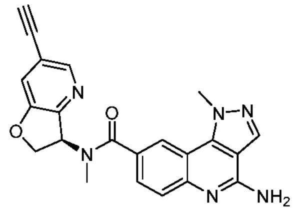
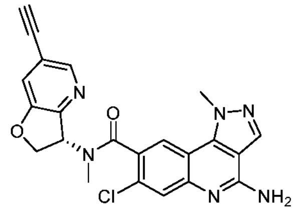
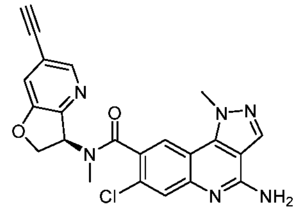

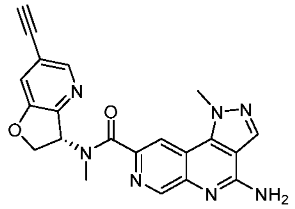 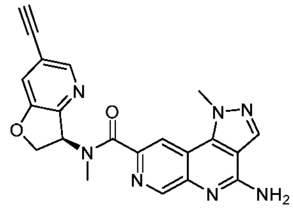 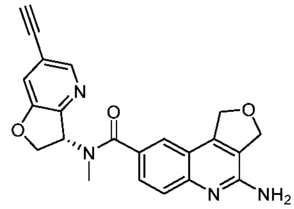 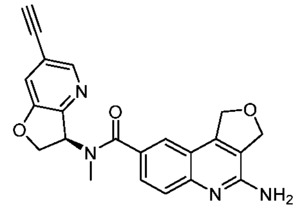

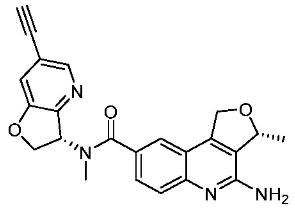 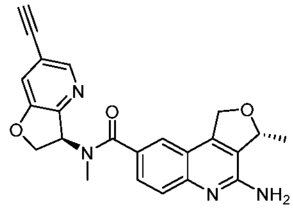 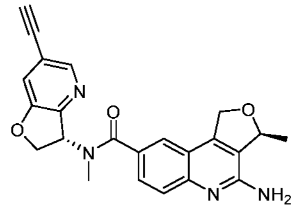 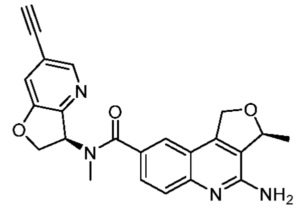

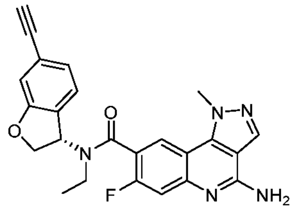 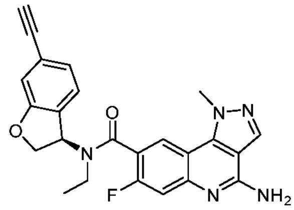 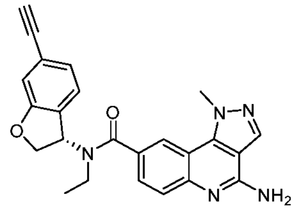 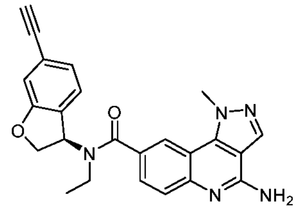

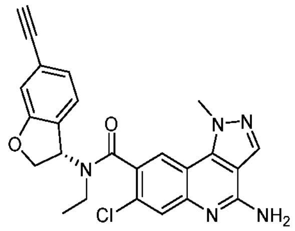 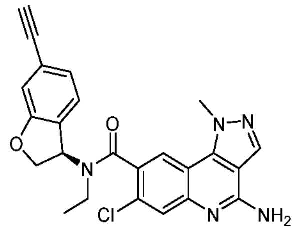 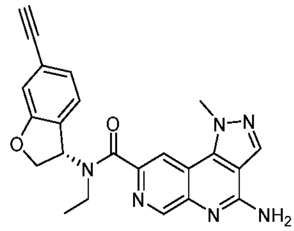 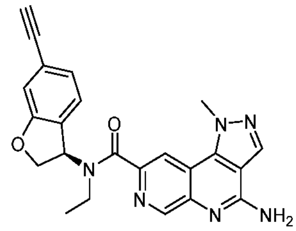

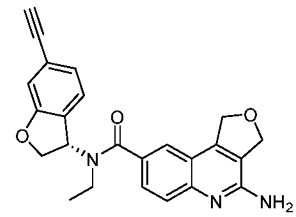 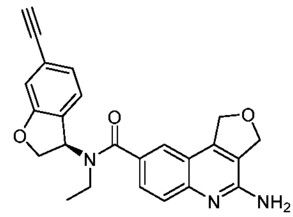 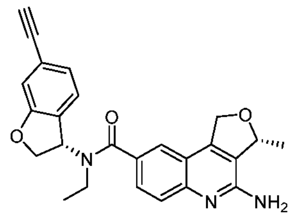 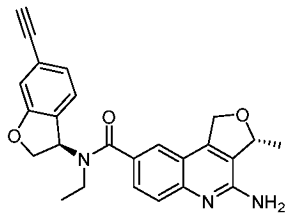

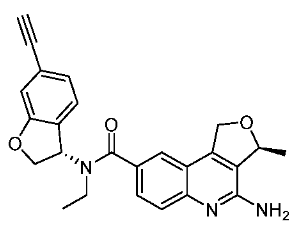 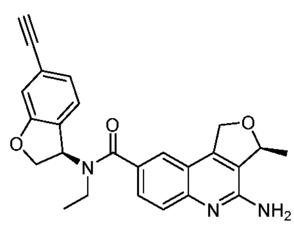 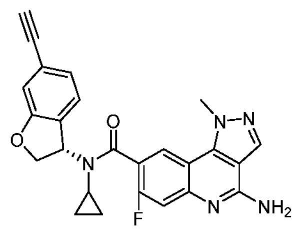 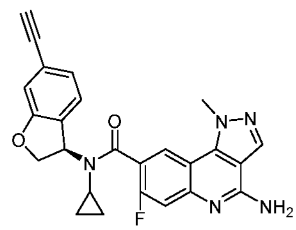

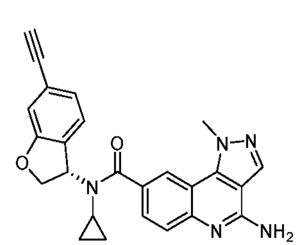 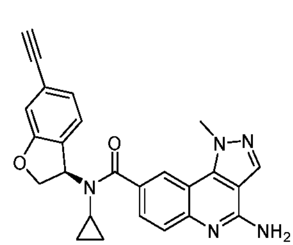 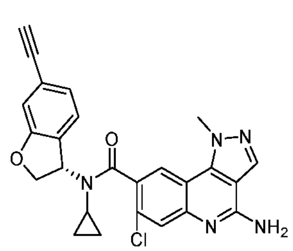 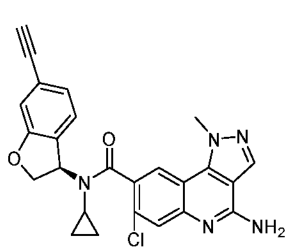

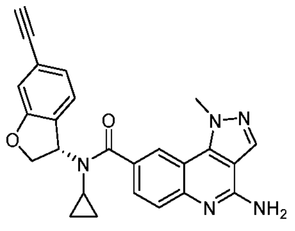
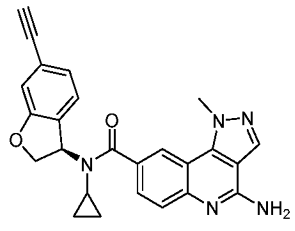
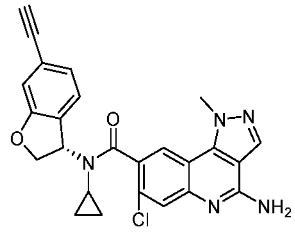
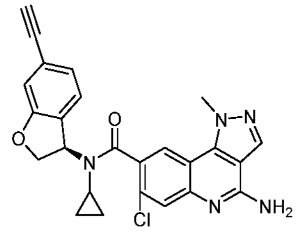
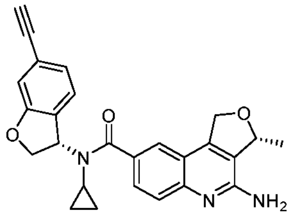
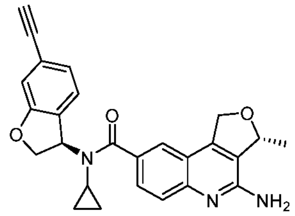
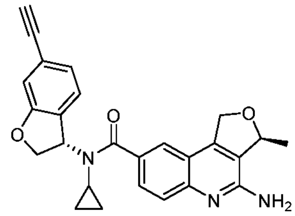
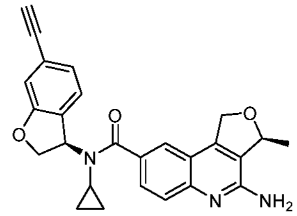
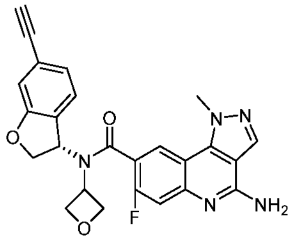
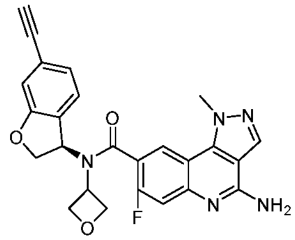
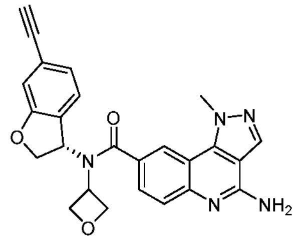
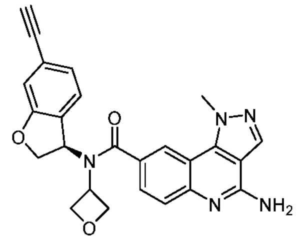
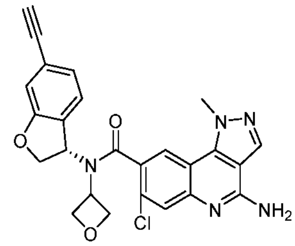
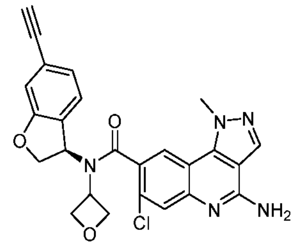
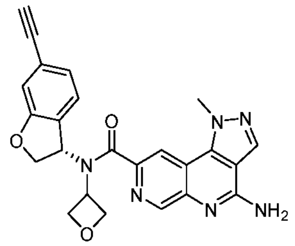
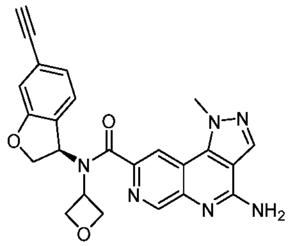
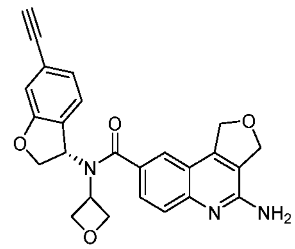
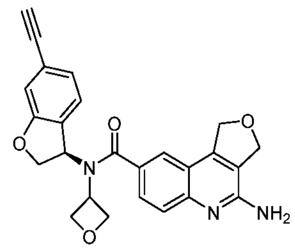
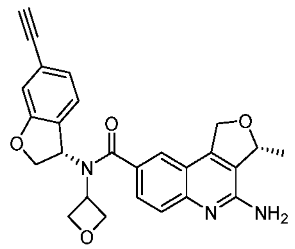
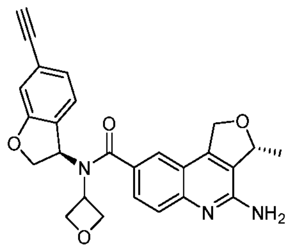
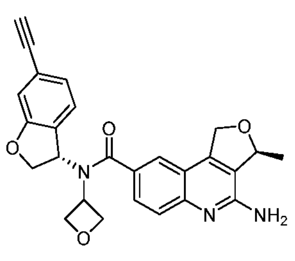
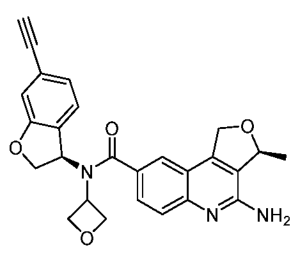
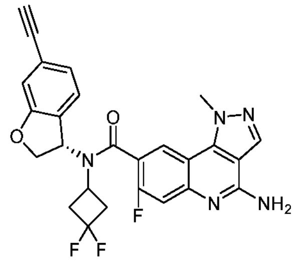
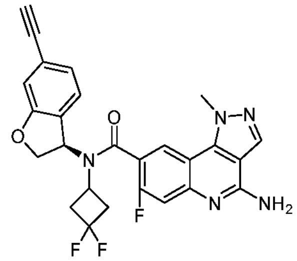

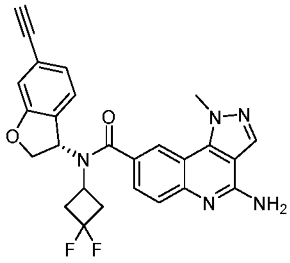 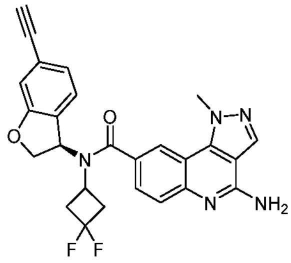 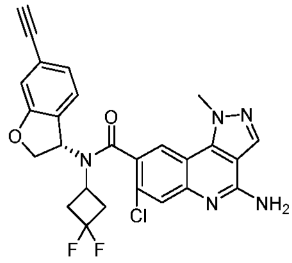 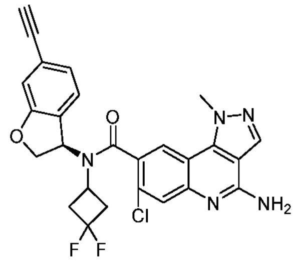

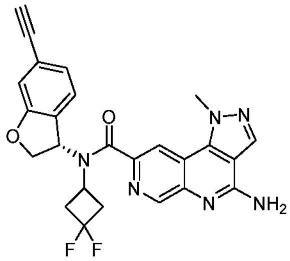 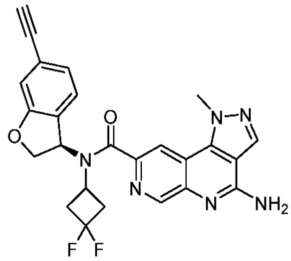 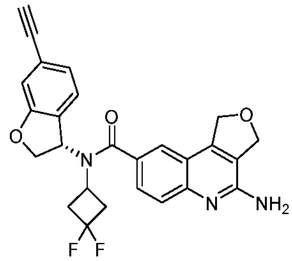 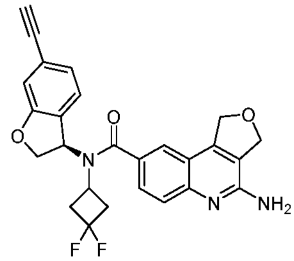

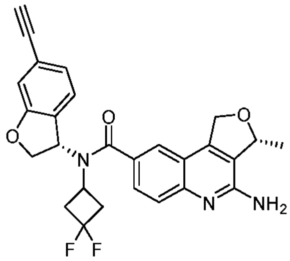 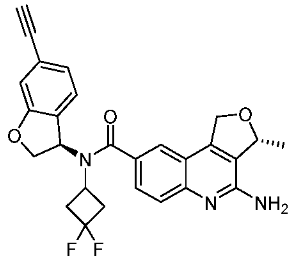 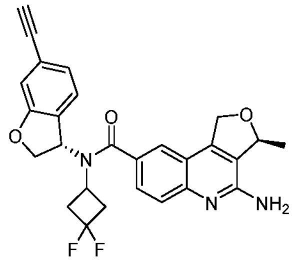 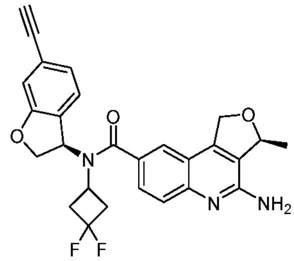

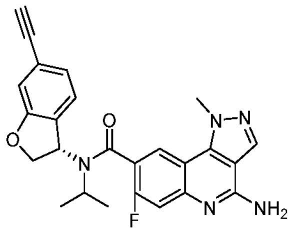 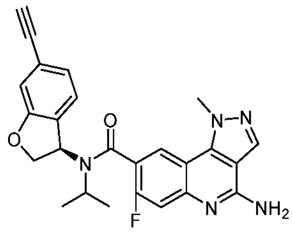 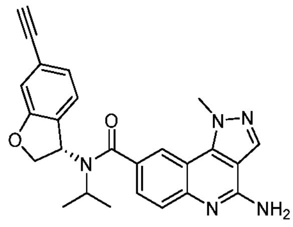 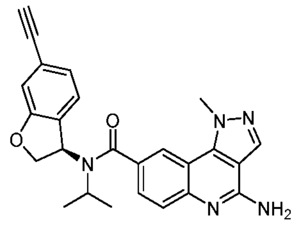

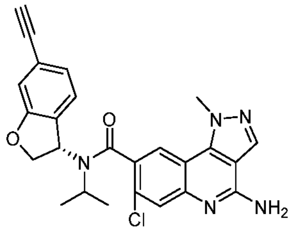 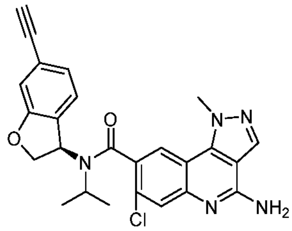 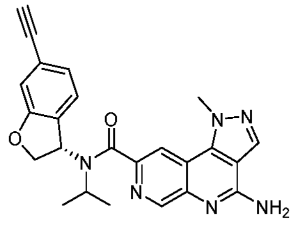 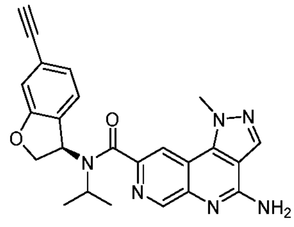

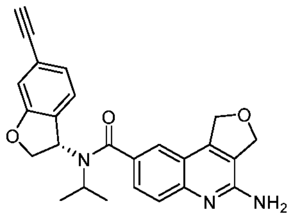 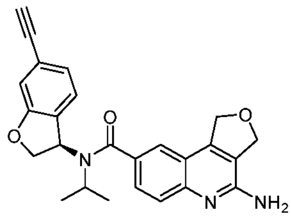 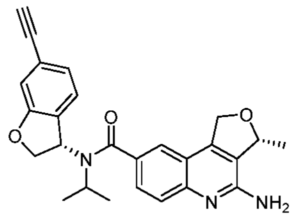 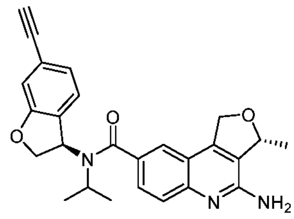

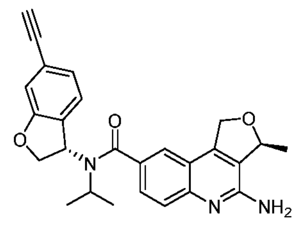
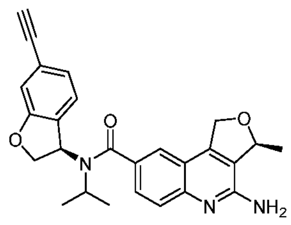
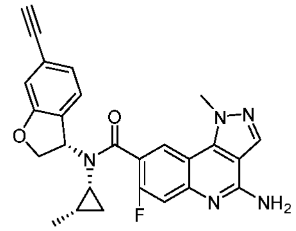
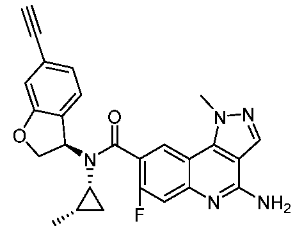

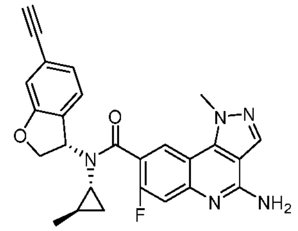
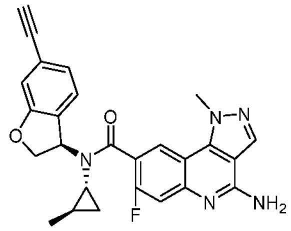
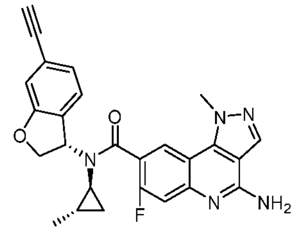
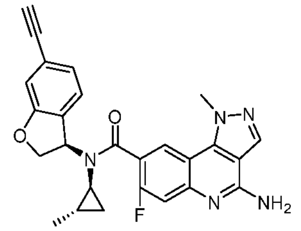

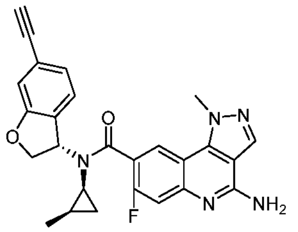
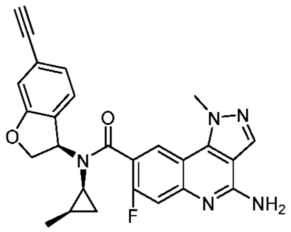
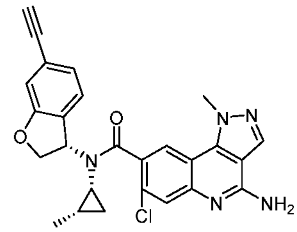
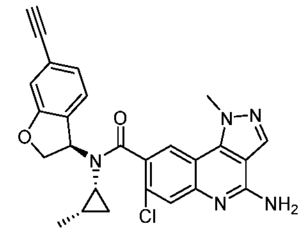

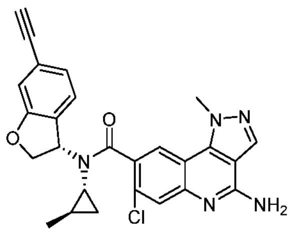
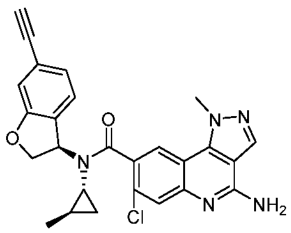
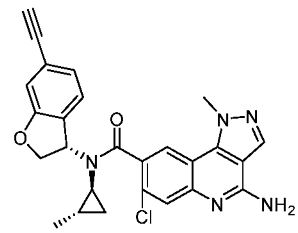
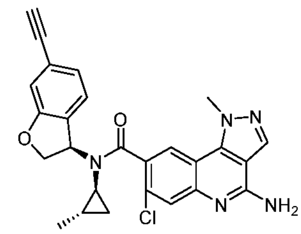

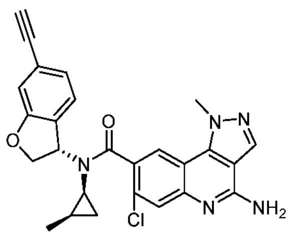
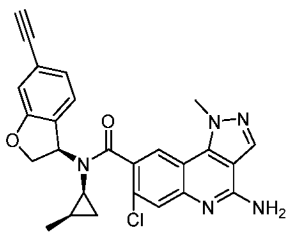
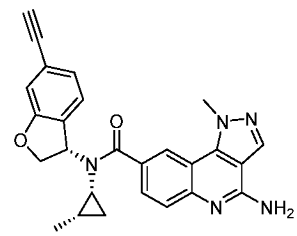
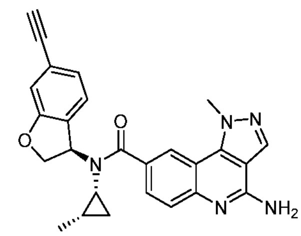

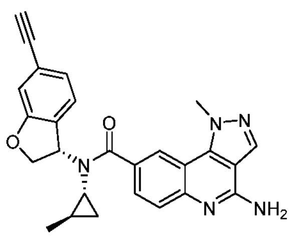
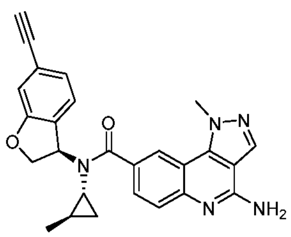
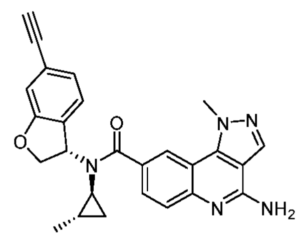
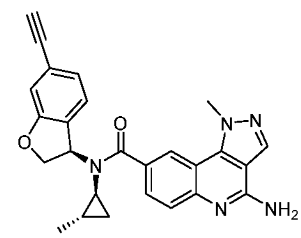

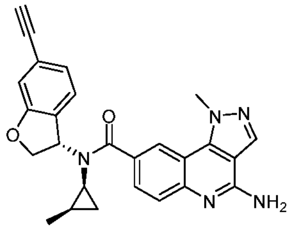
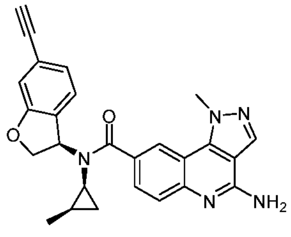
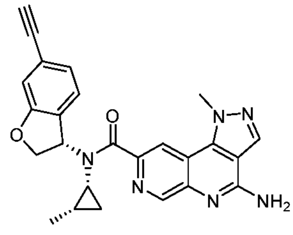
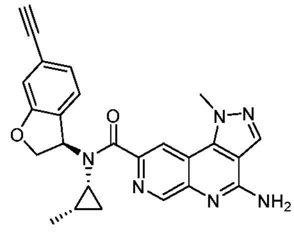

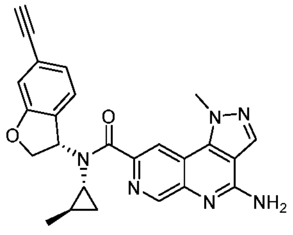
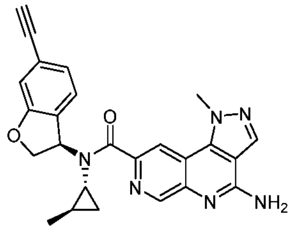
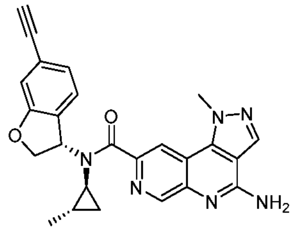
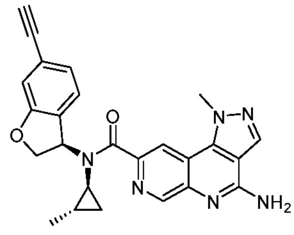

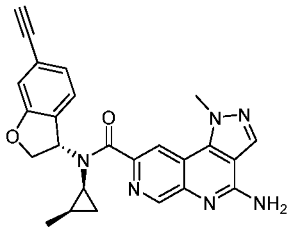
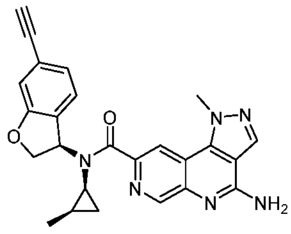
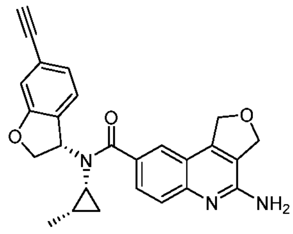
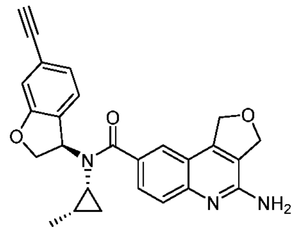

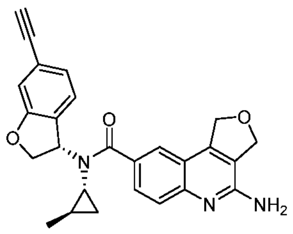
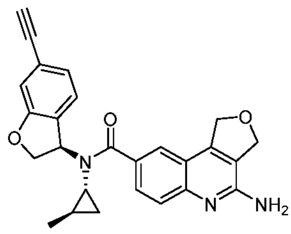
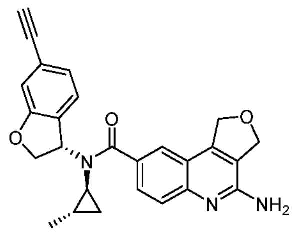
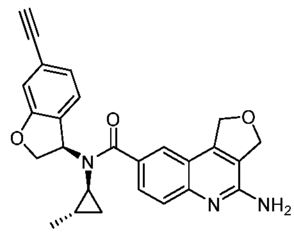

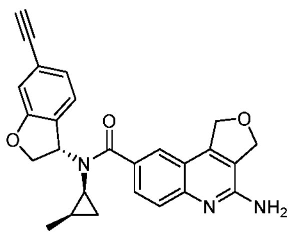
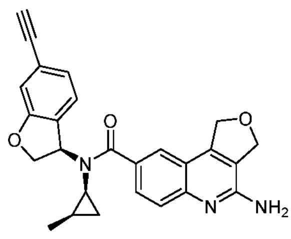
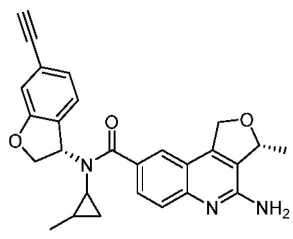
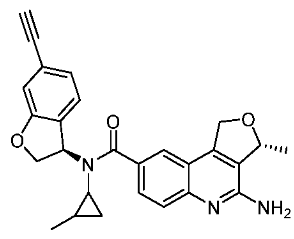

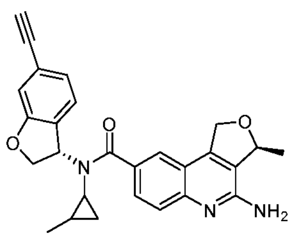
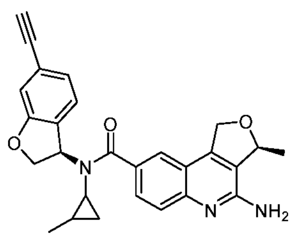
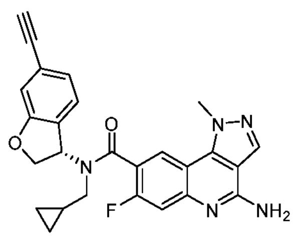
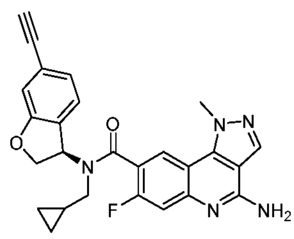

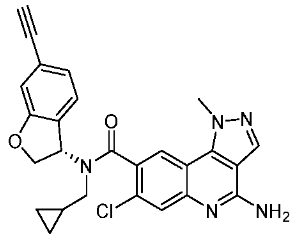 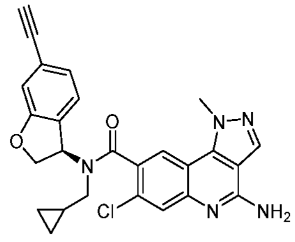 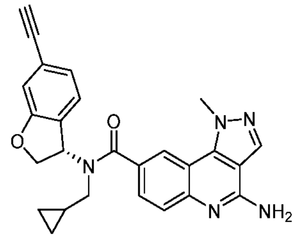 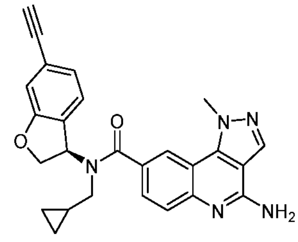

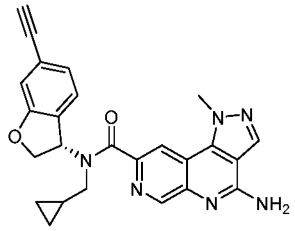 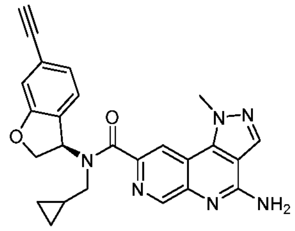 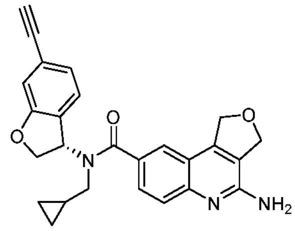 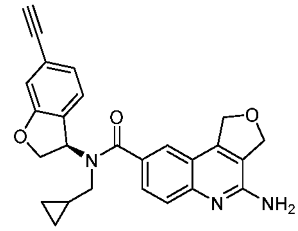

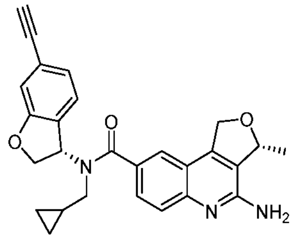 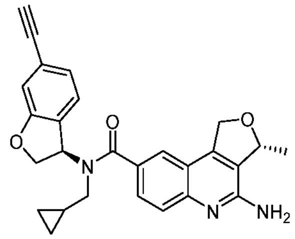 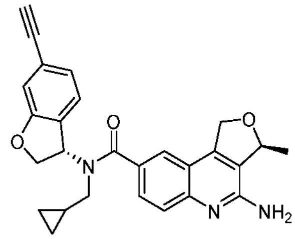 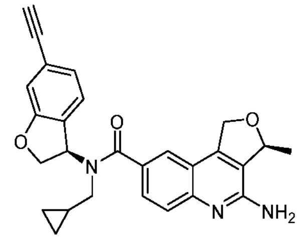

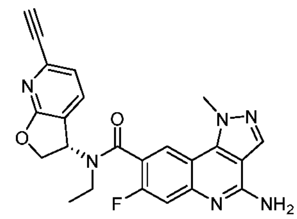 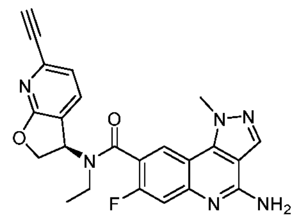 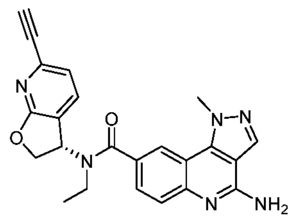 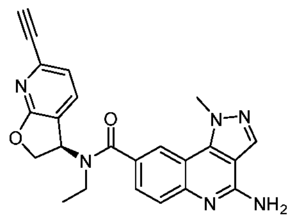

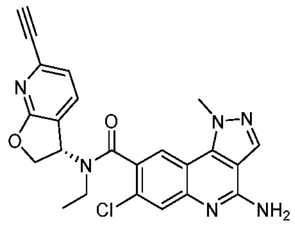 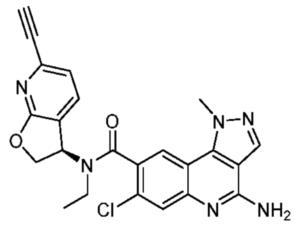 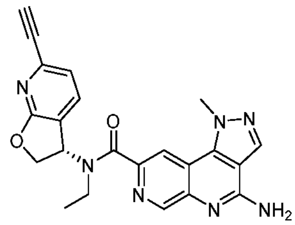 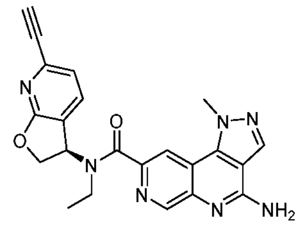

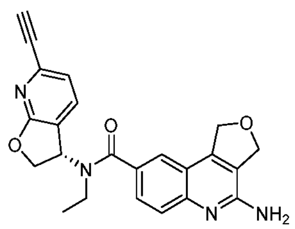 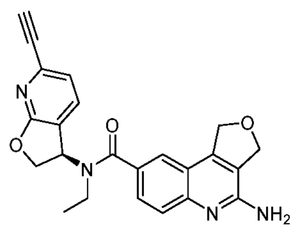 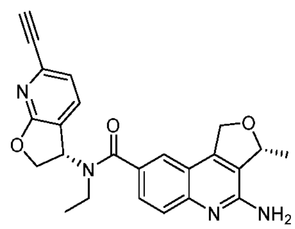 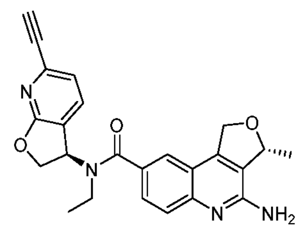

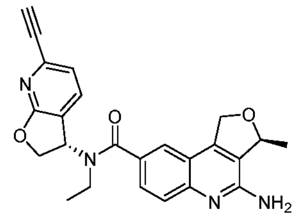
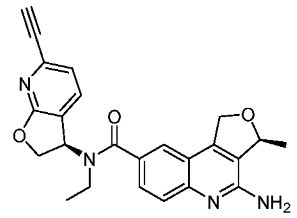
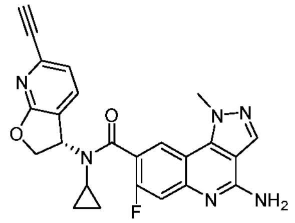
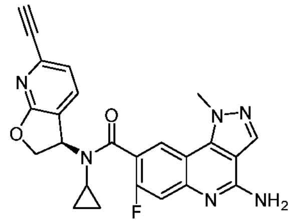
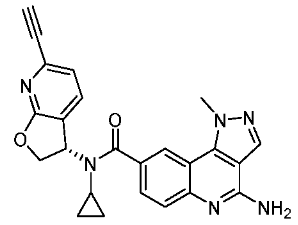
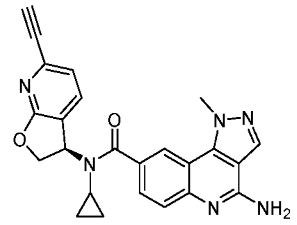
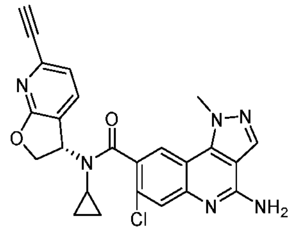
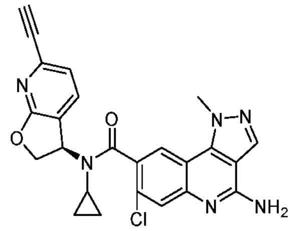
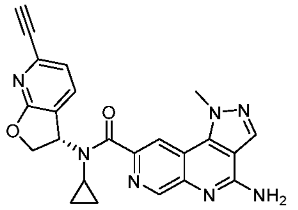
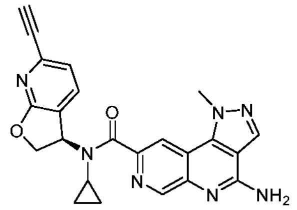
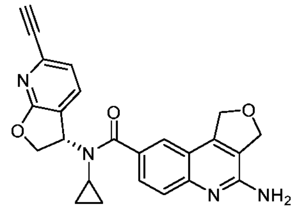
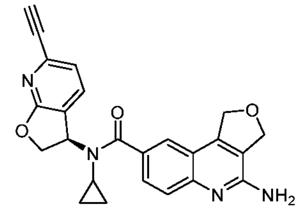
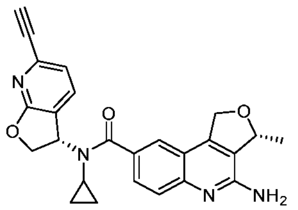
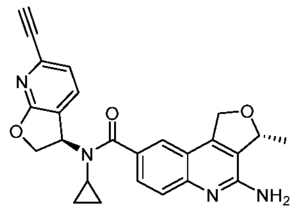
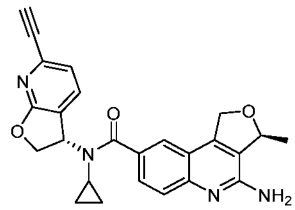
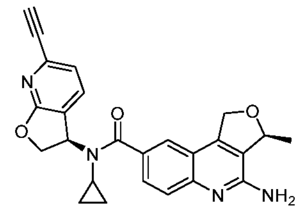
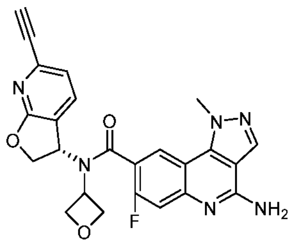
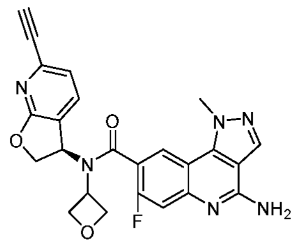
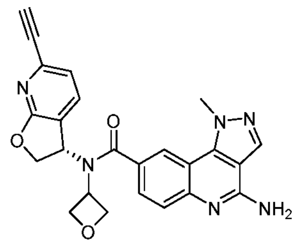
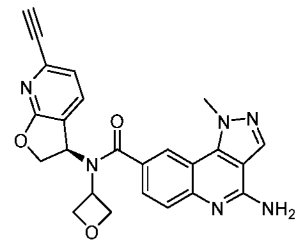
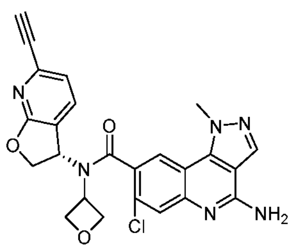
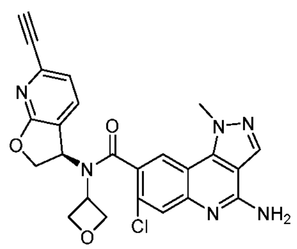
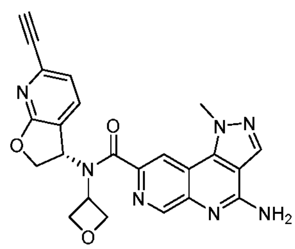
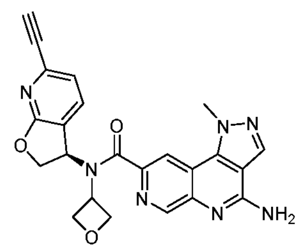

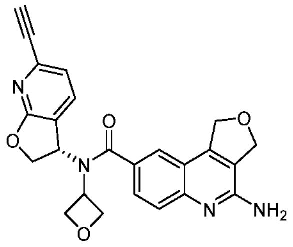 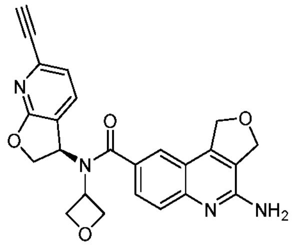 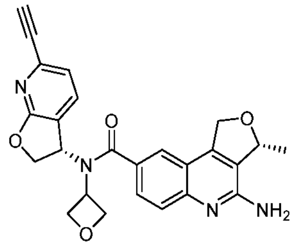 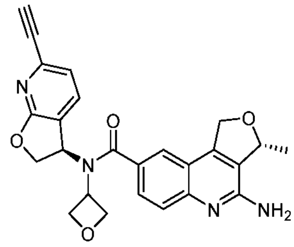

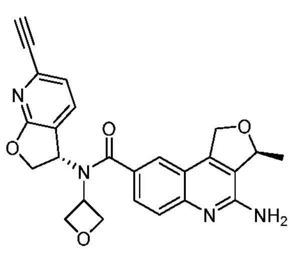 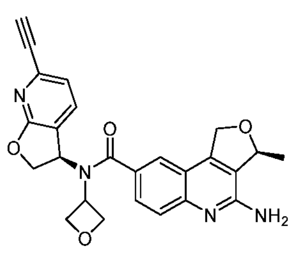 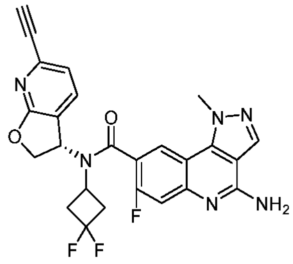 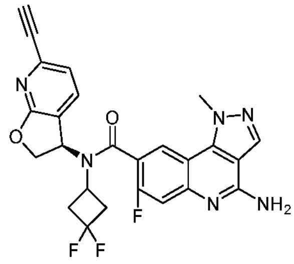

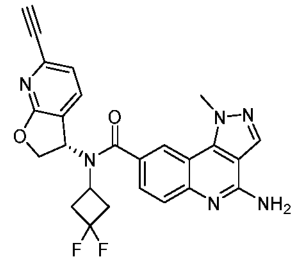 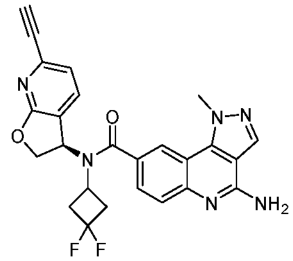 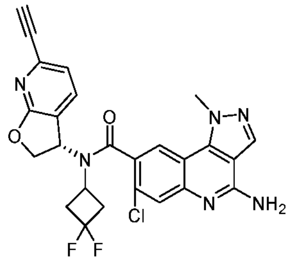 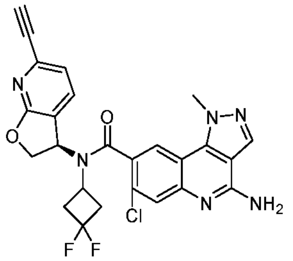

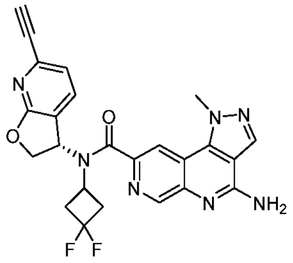 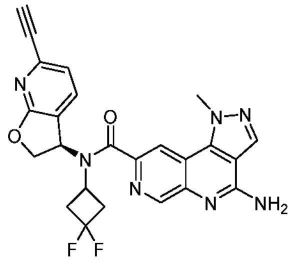 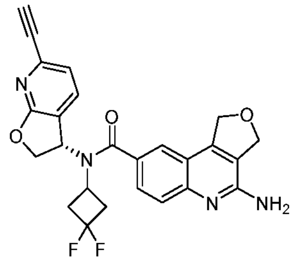 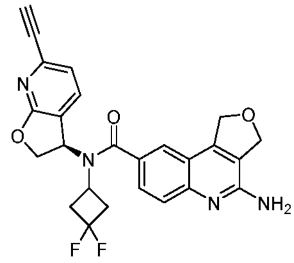

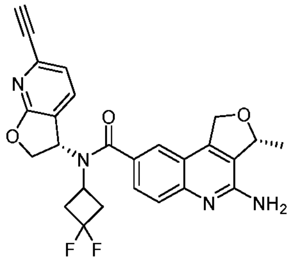 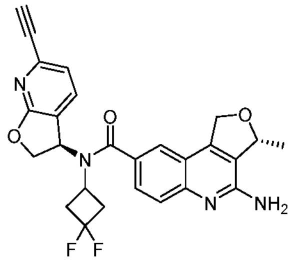 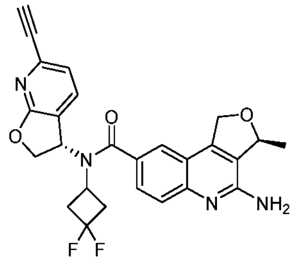 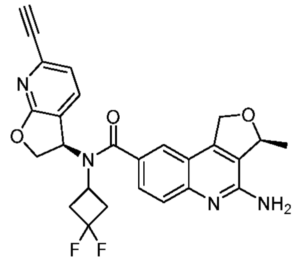

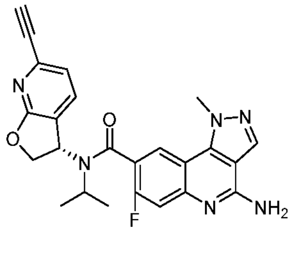 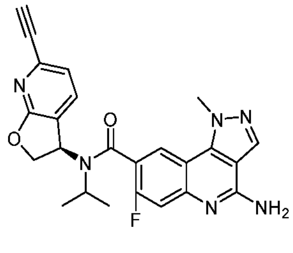 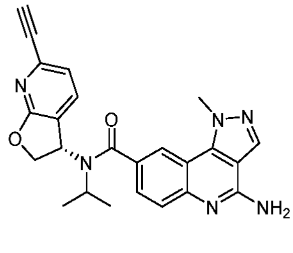 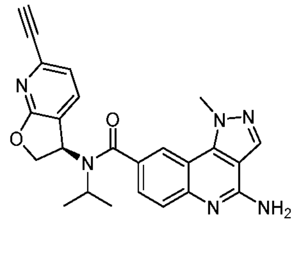

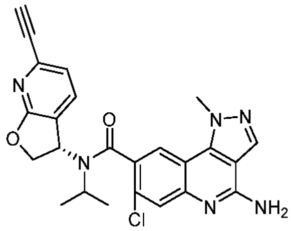
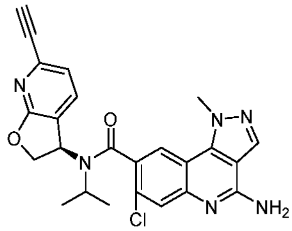
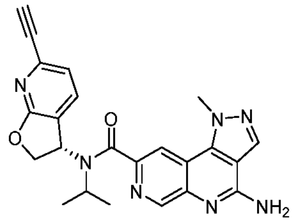
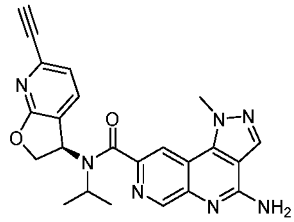

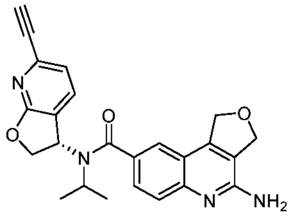
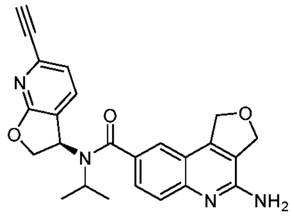
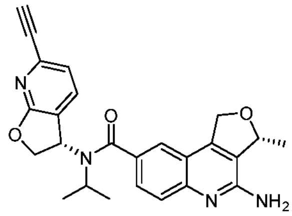
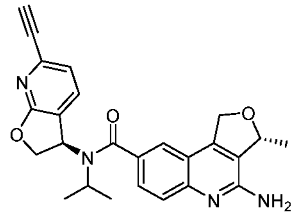

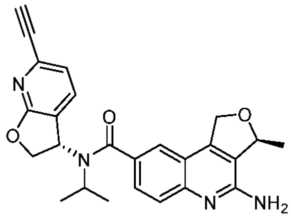
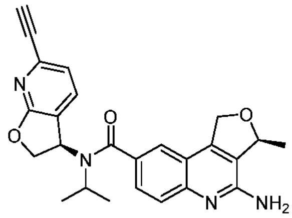
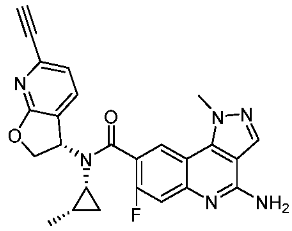
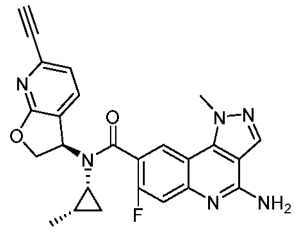

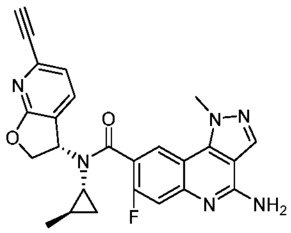
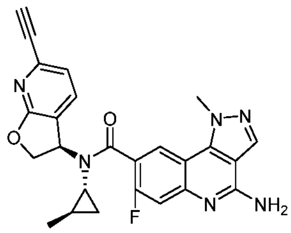
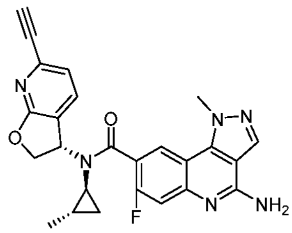
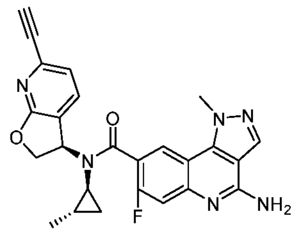

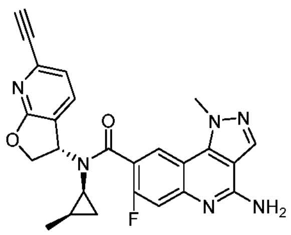
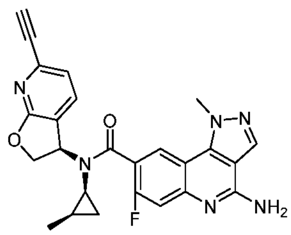
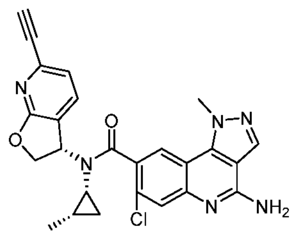
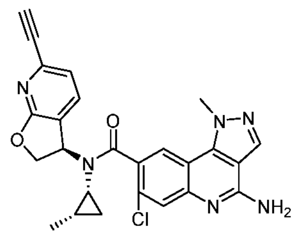

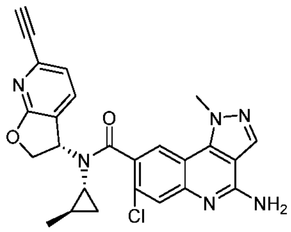
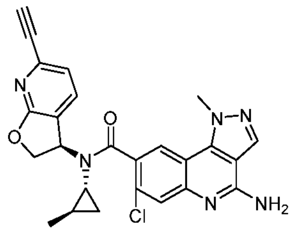
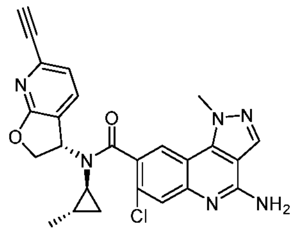
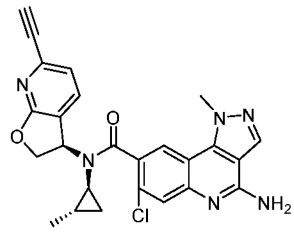

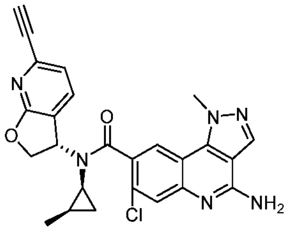
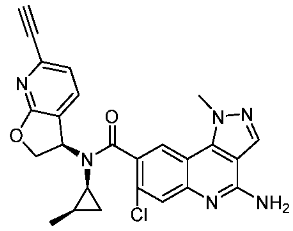
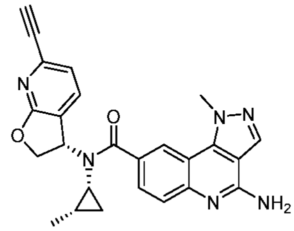
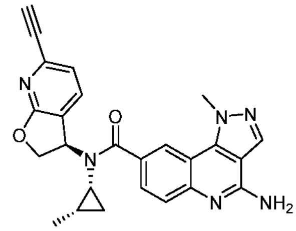
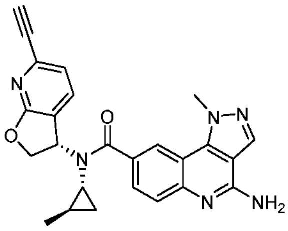
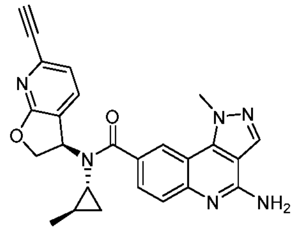
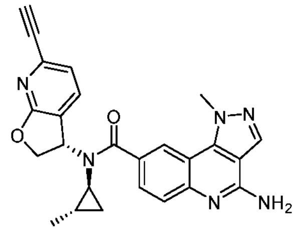
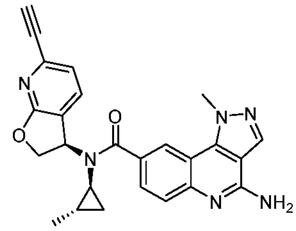
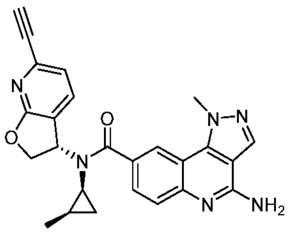
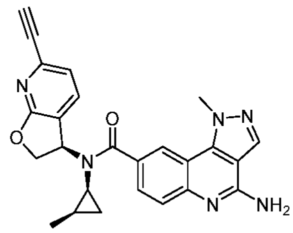
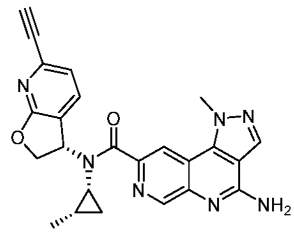
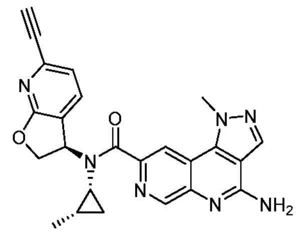
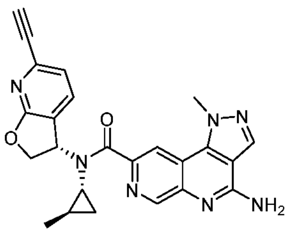
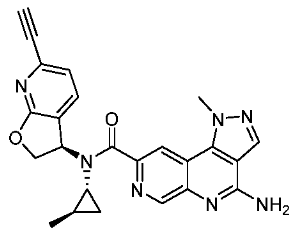
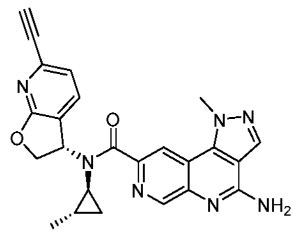
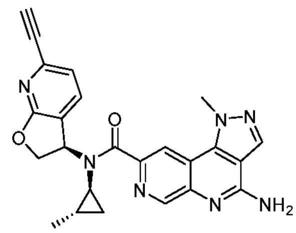
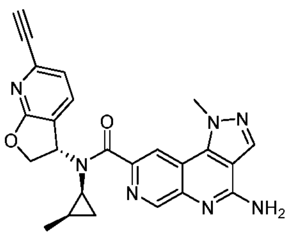
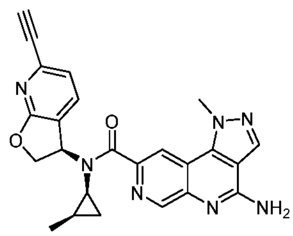
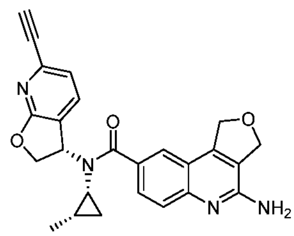
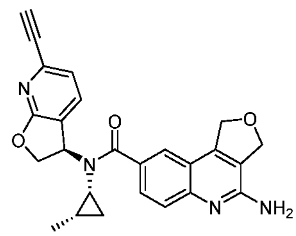
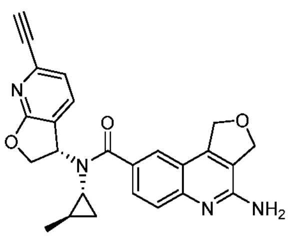
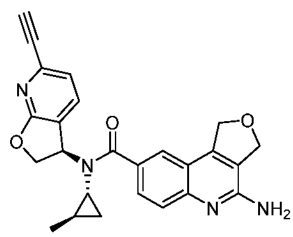
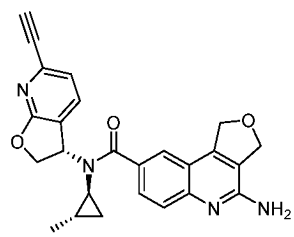
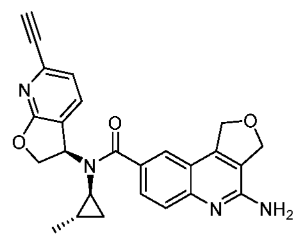

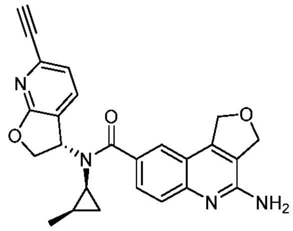
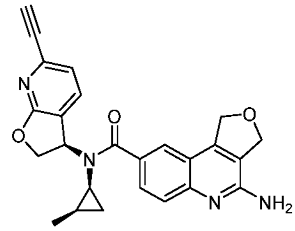
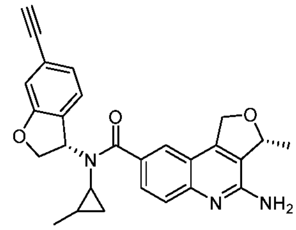
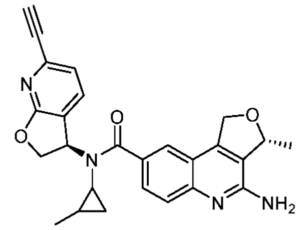
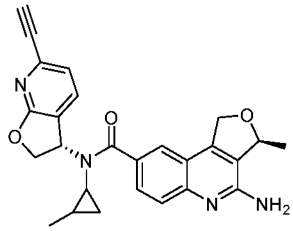
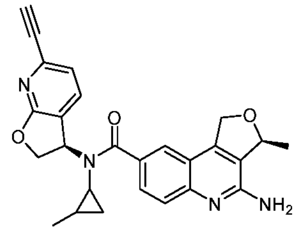
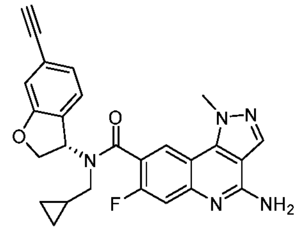
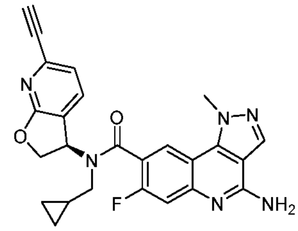
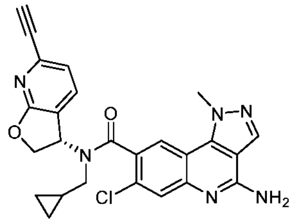
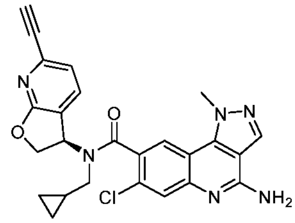
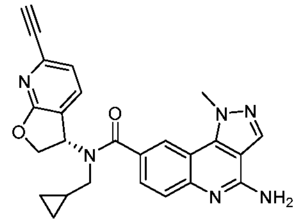
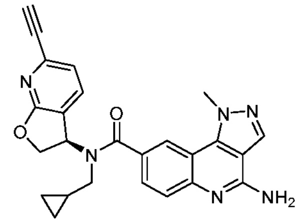
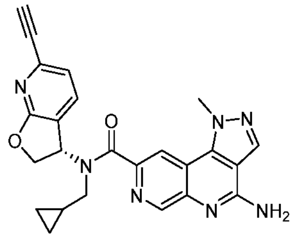
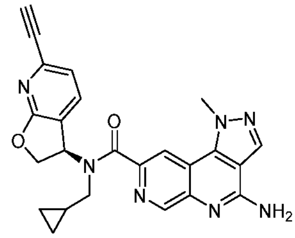
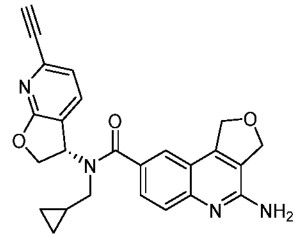
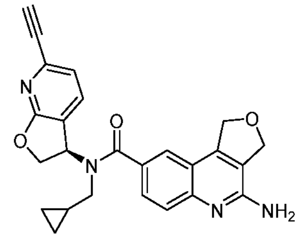
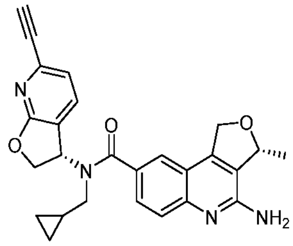
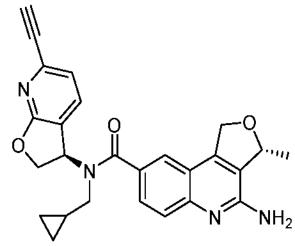
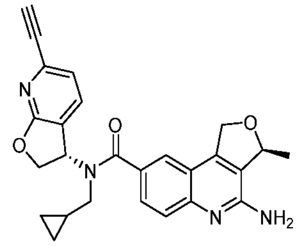
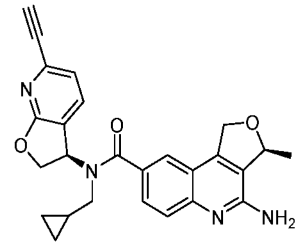
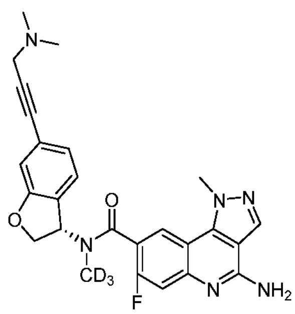
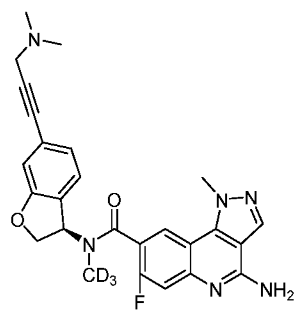
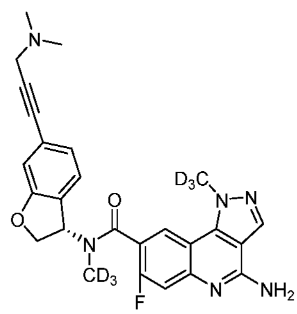
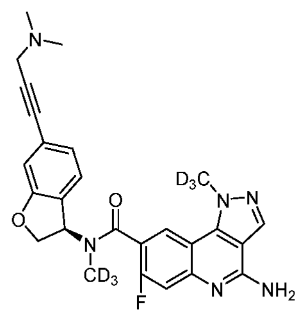

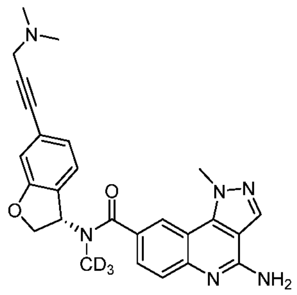
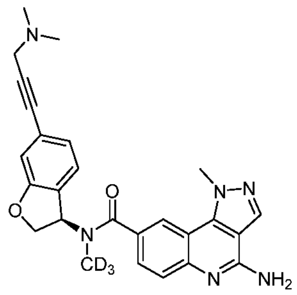
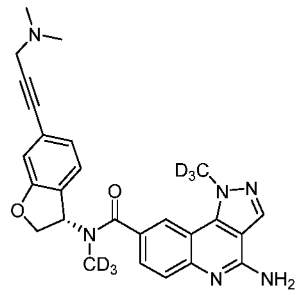
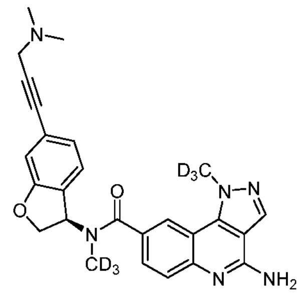
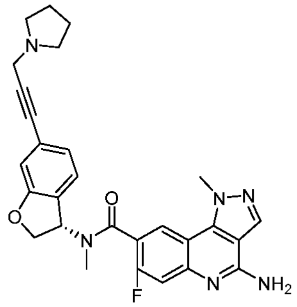
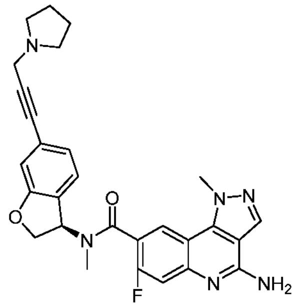
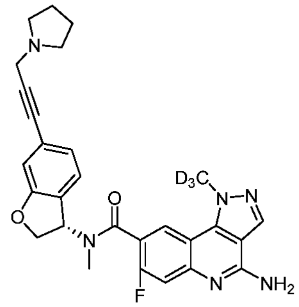
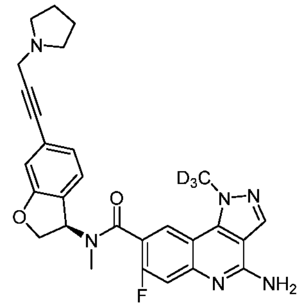
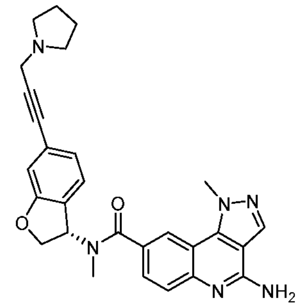
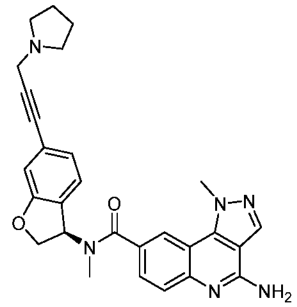
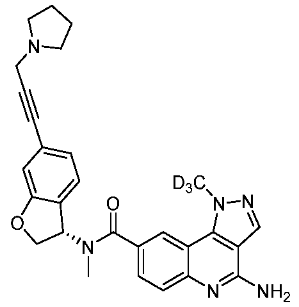
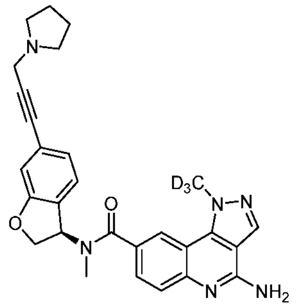
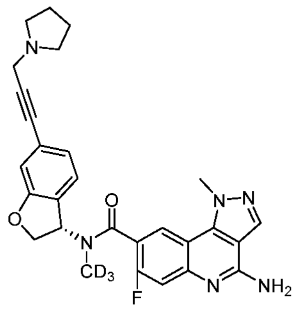
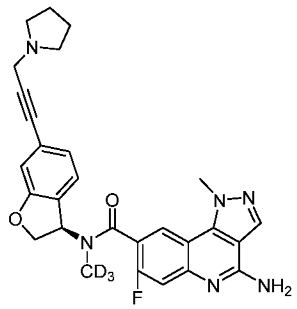
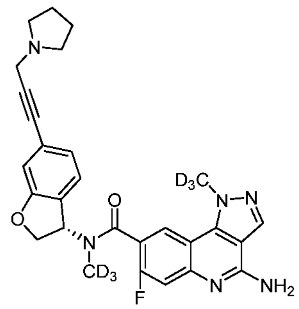
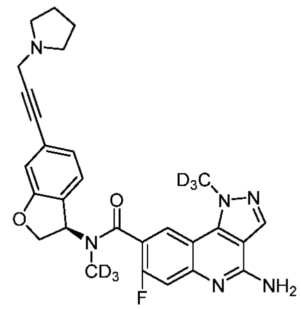
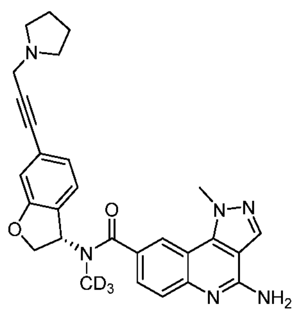
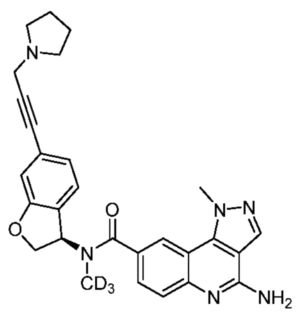
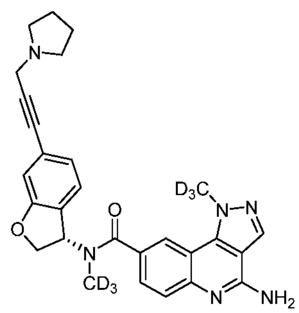
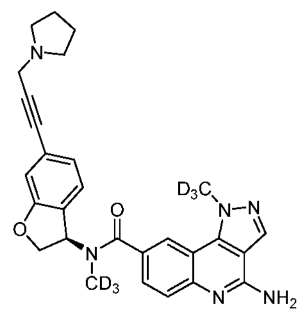

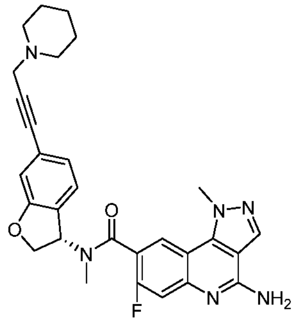
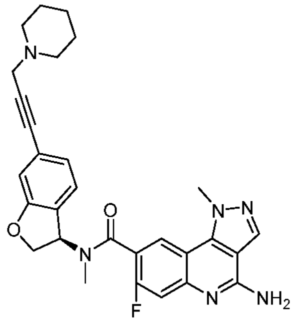
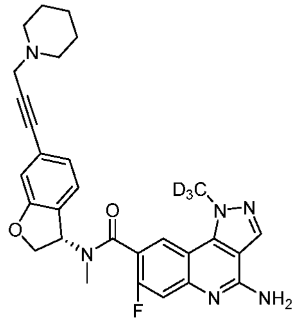
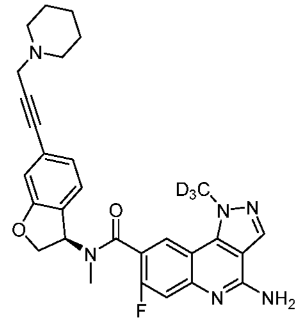

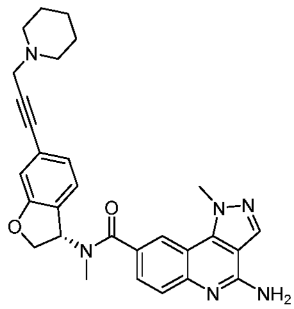
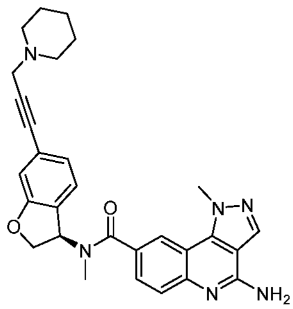
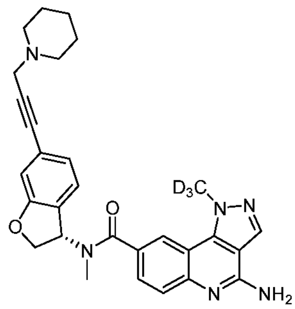
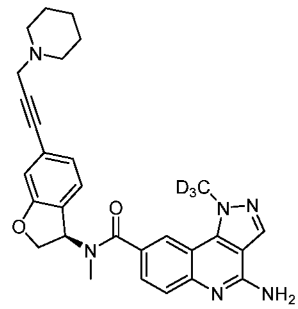

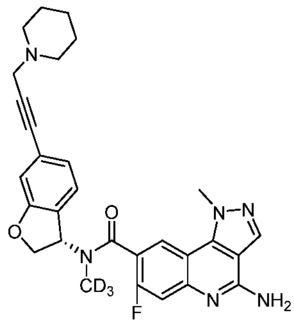
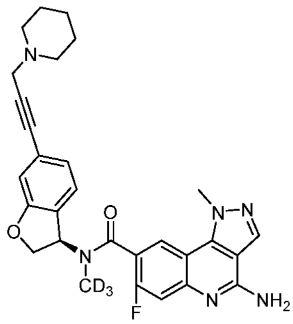
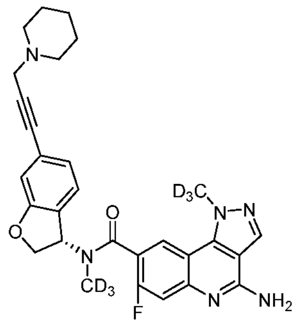
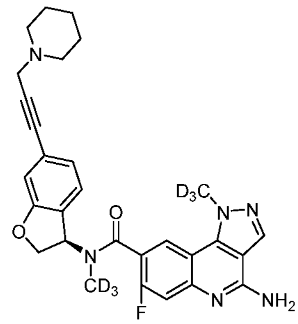

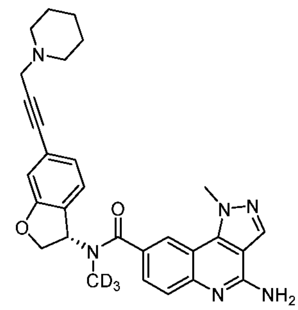
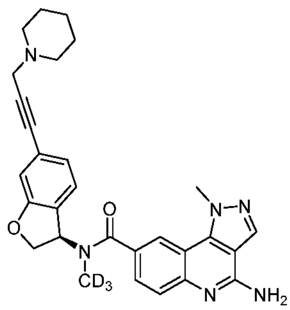
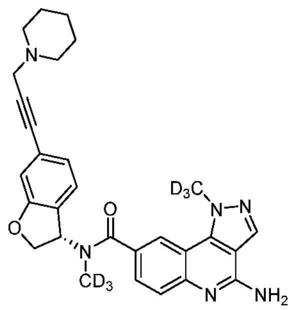
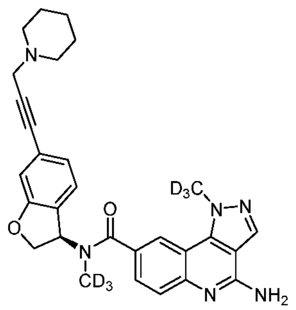

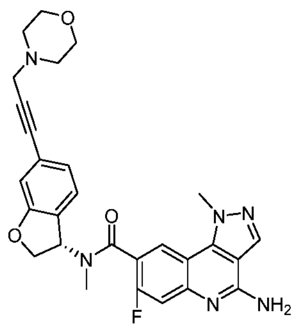
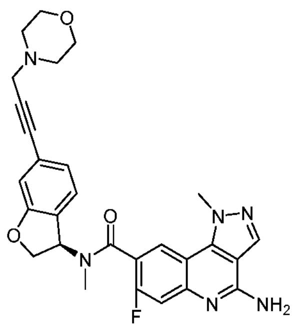
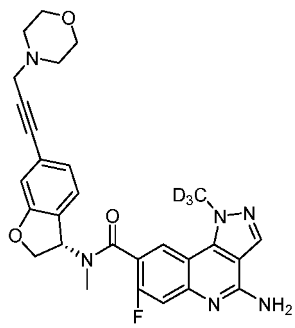
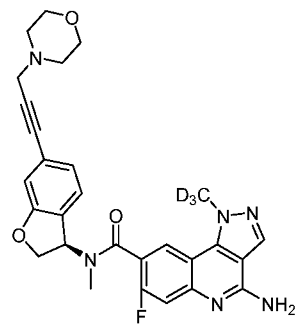

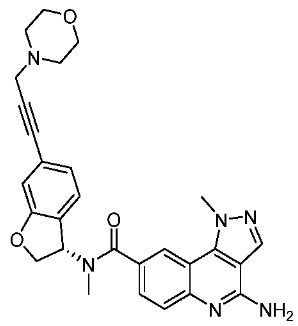
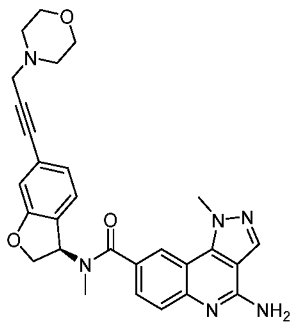
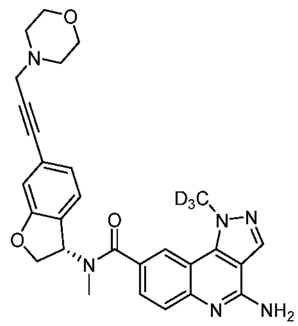
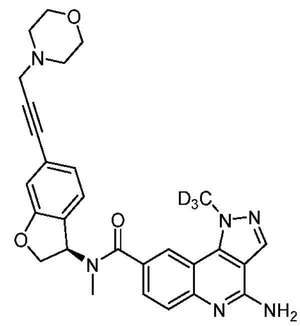
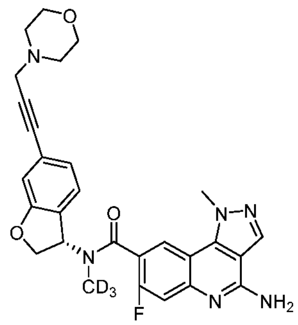
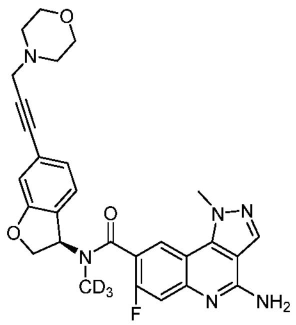
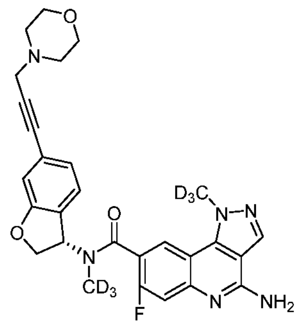
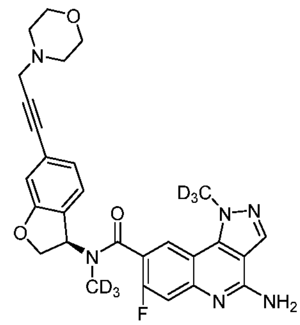
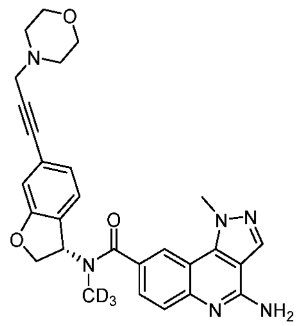
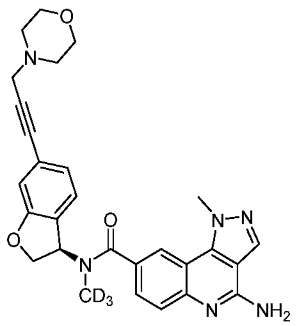
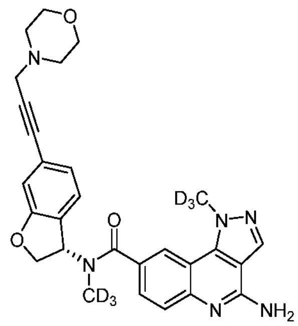
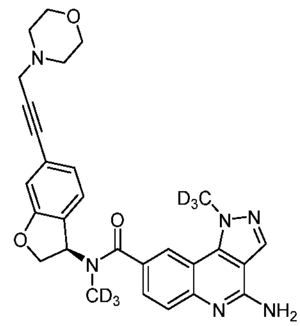
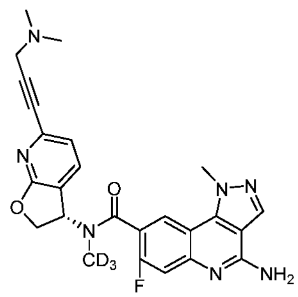
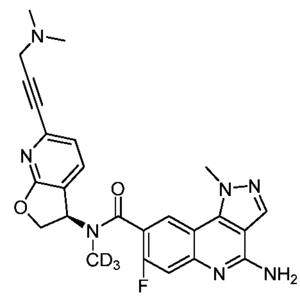
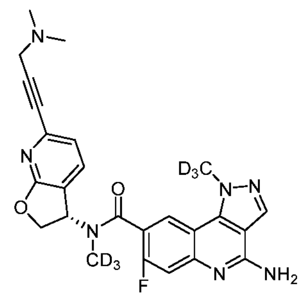
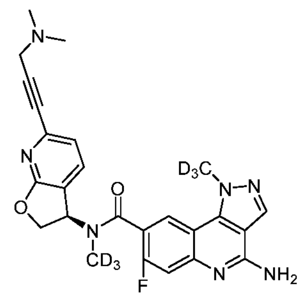
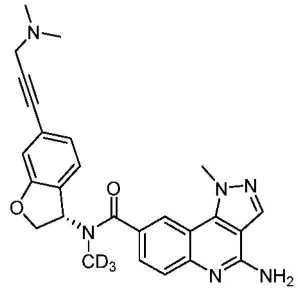
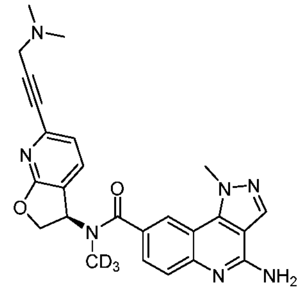
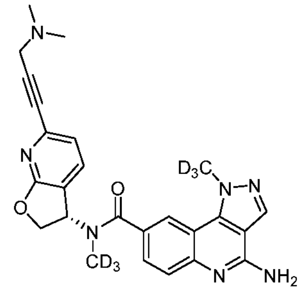
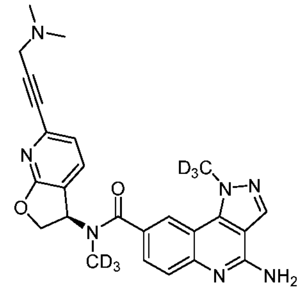

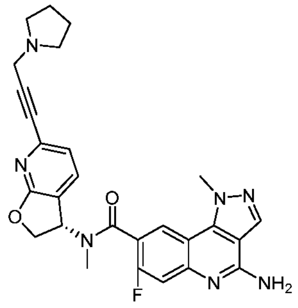
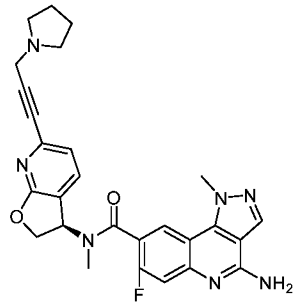
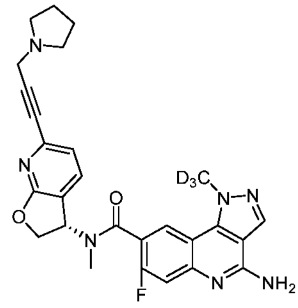
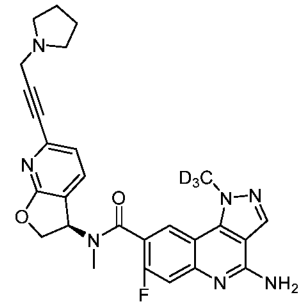
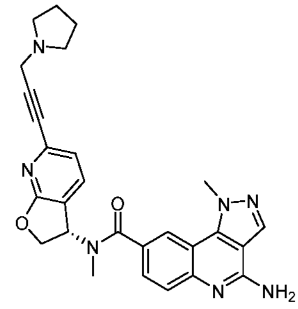
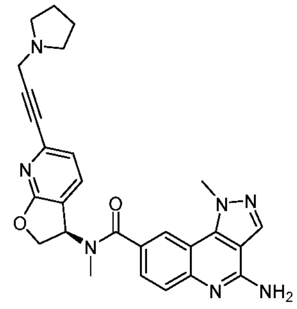
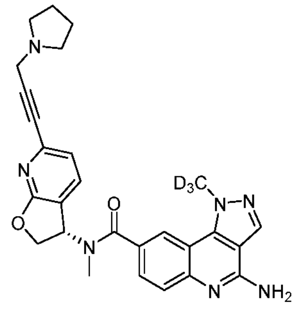
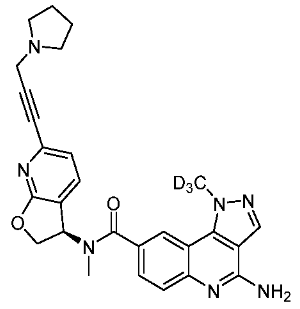
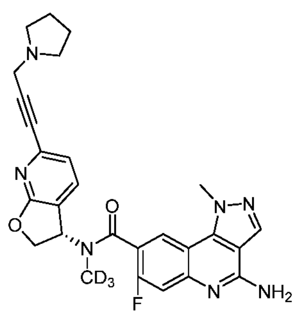
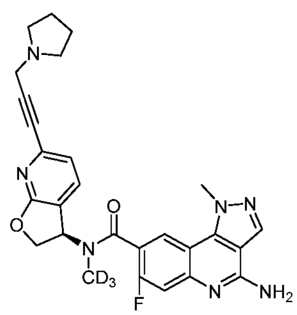
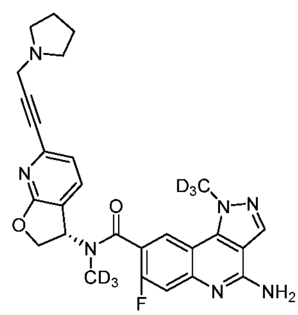
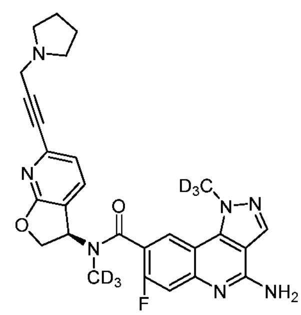
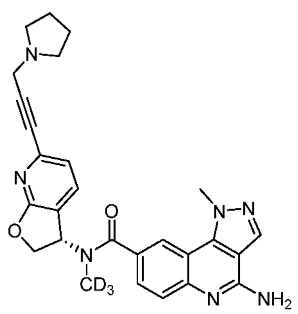
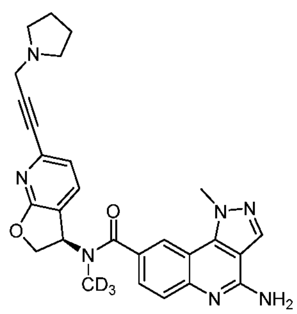
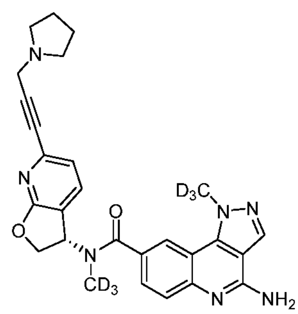
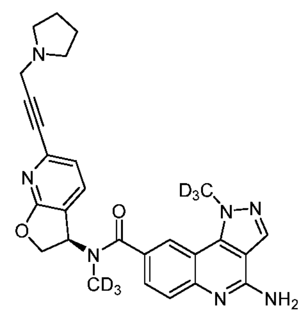
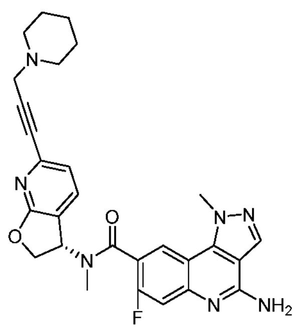
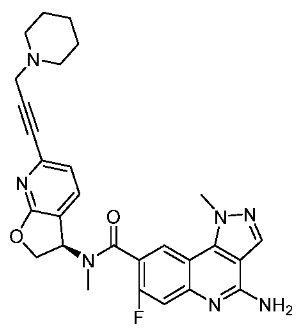
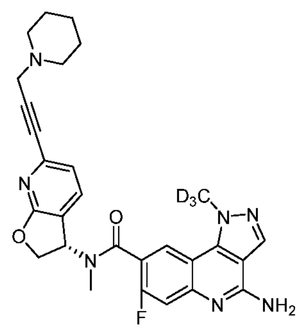
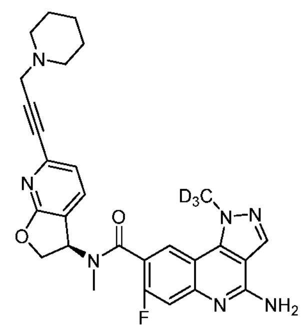

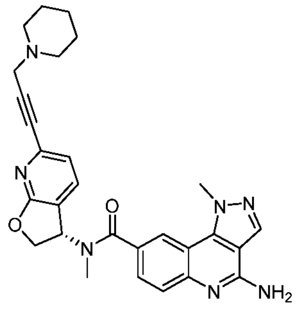
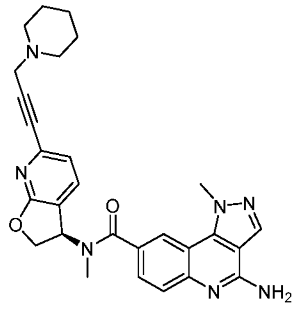
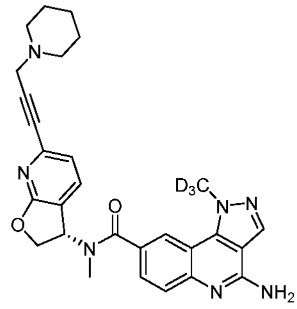
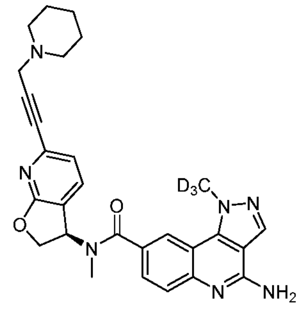

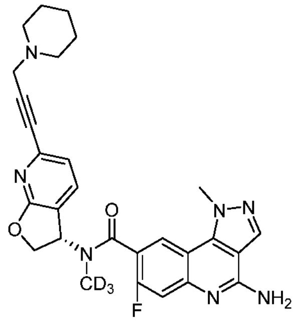
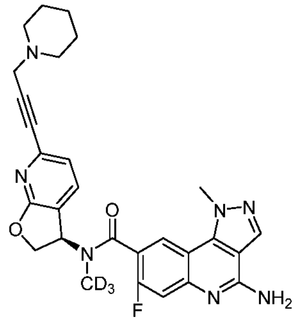
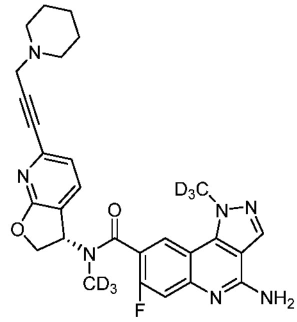
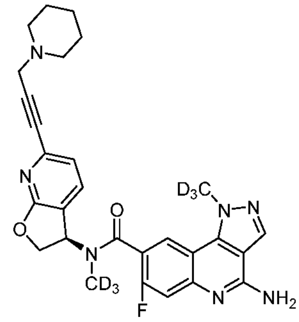

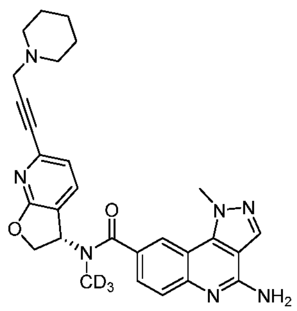
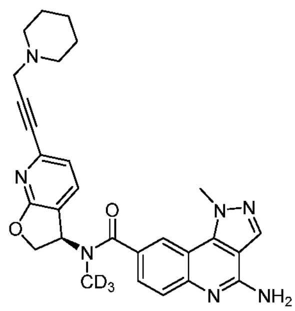
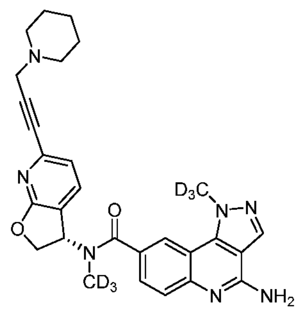
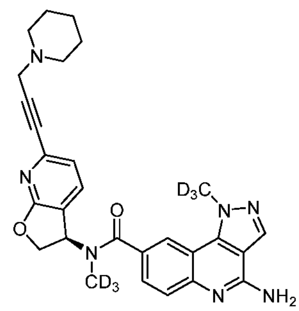

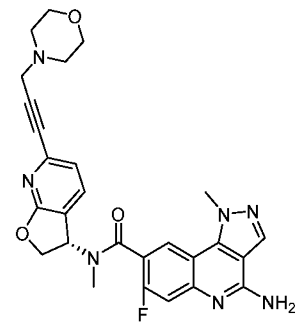
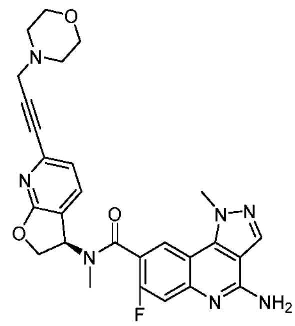
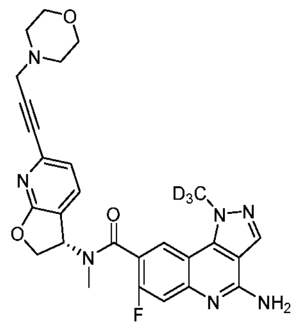
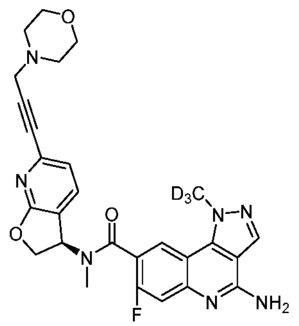

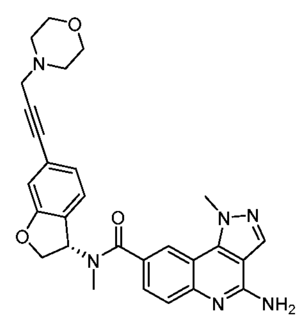
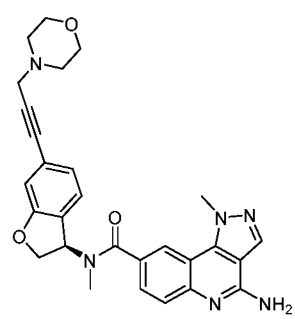
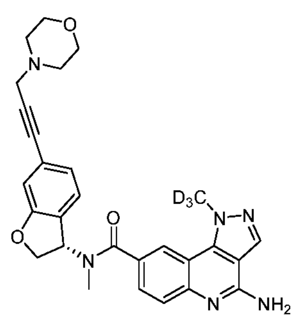
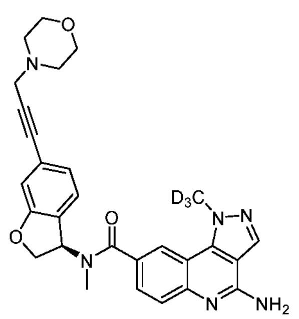

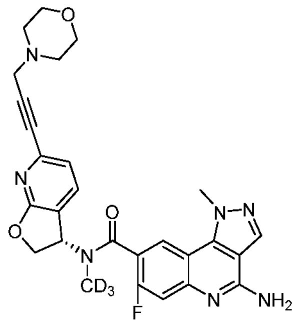 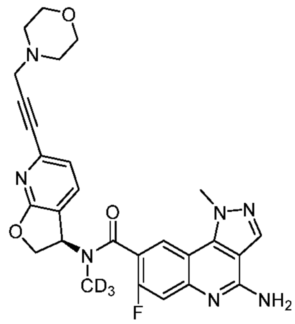 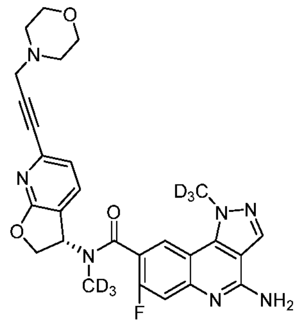 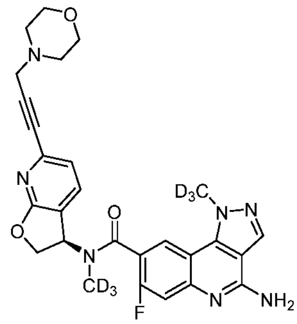

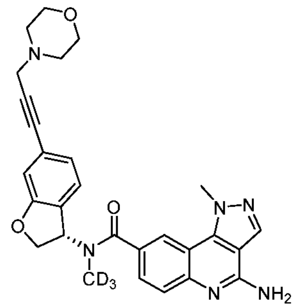 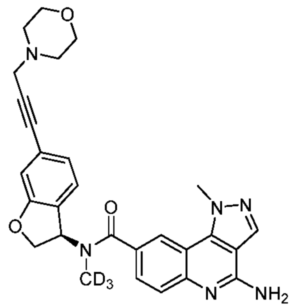 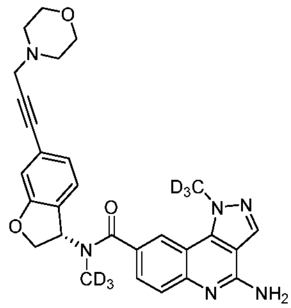 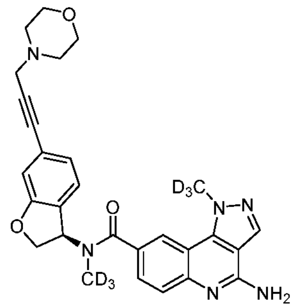

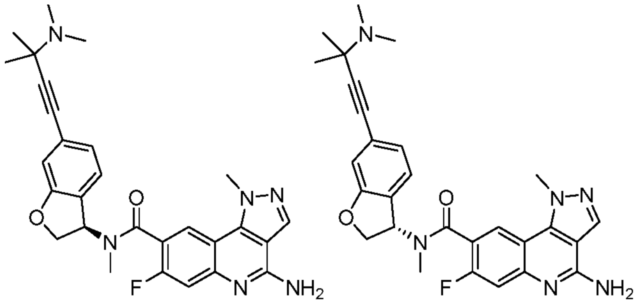.

**15.** A use of the compound or the pharmaceutically acceptable salt thereof according to any one of claims 1 to 14 in the manufacture of a medicament for treating diseases related to PRMT5.

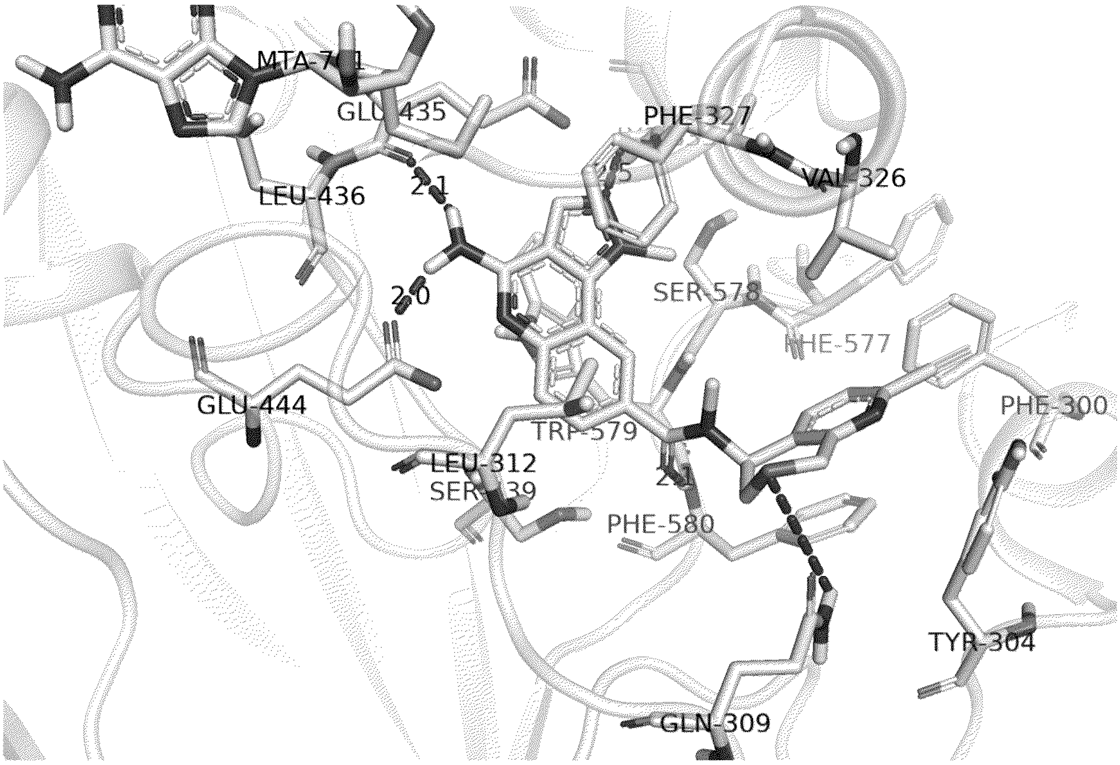
PHE-327
VAL-326
LEU-436
2.1
2.0
SER-578
GLU-444
TRP-579
PHE-300
LEU-312
PHE-580
TYR-304
GLN-309

FIG. 1

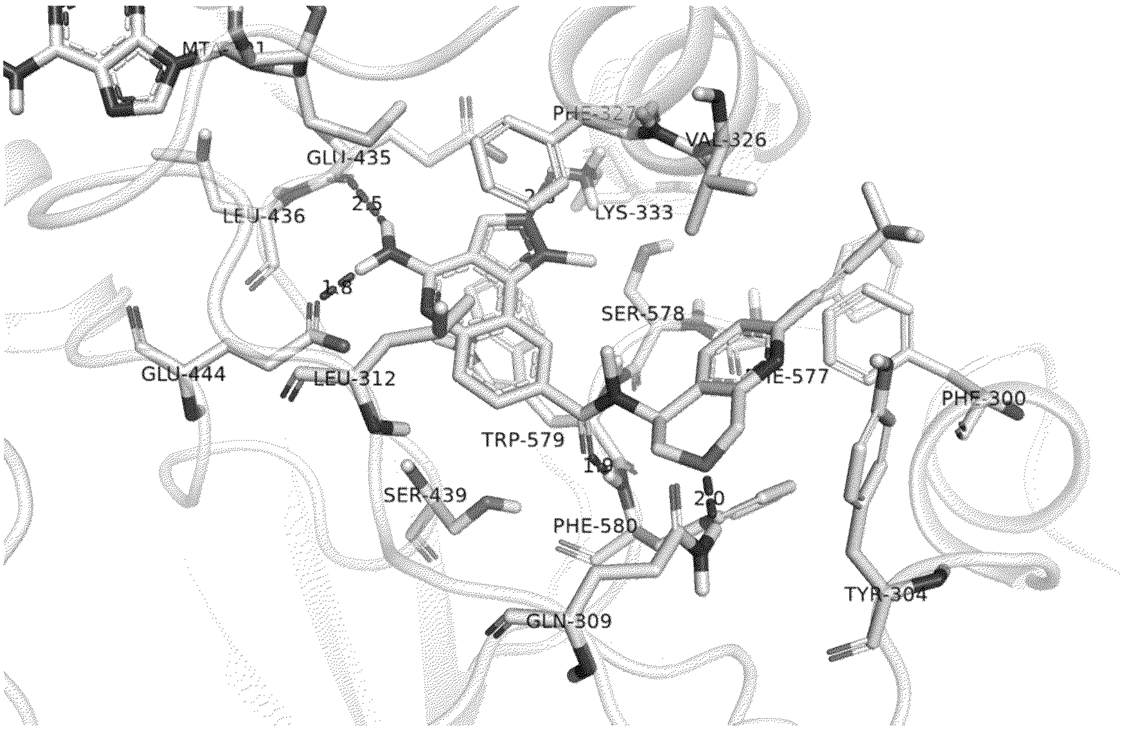
VAL-326
GLU-435
2.5
LYS-333
1.8
SER-578
GLU-444
LEU-312
TRP-579
1.9
SER-439
2.0
PHE-580
TYR-304
GLN-309

FIG. 2

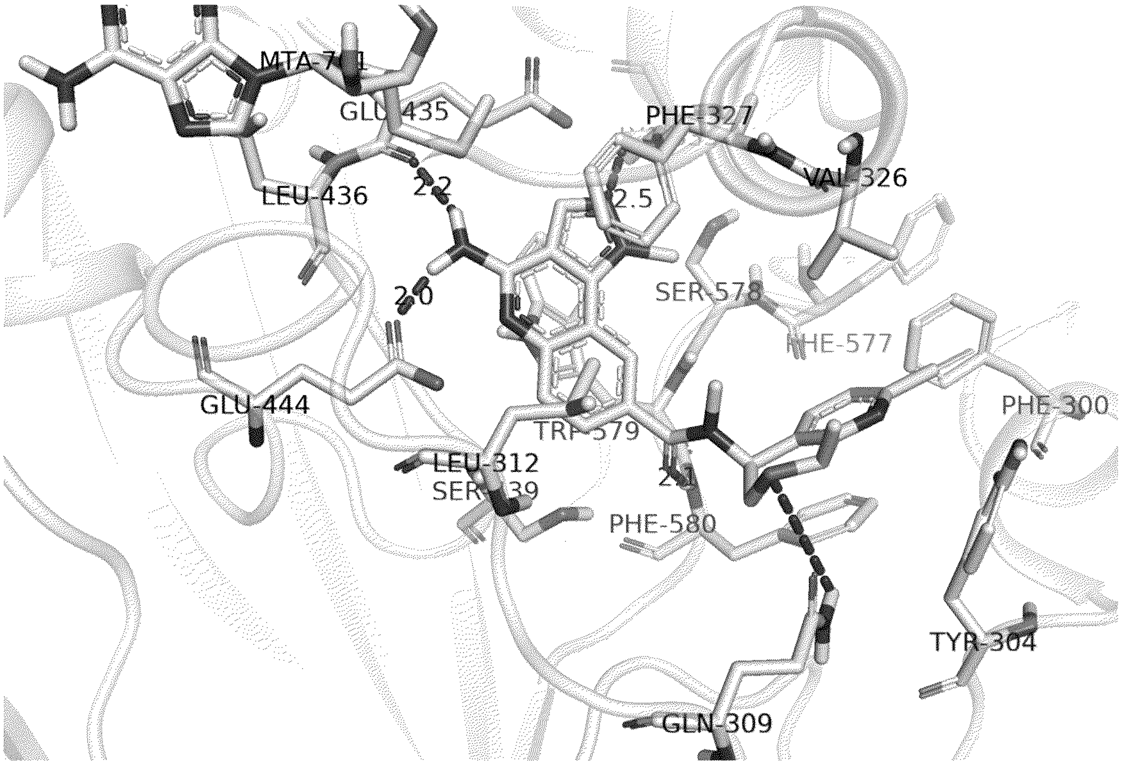
MTA-701
GLU-435
PHE-327
VAL-326
LEU-436
2.2
2.5
2.0
SER-578
PHE-577
GLU-444
PHE-300
TRP-579
LEU-312
SER-439
2.1
PHE-580
TYR-304
GLN-309

FIG. 3

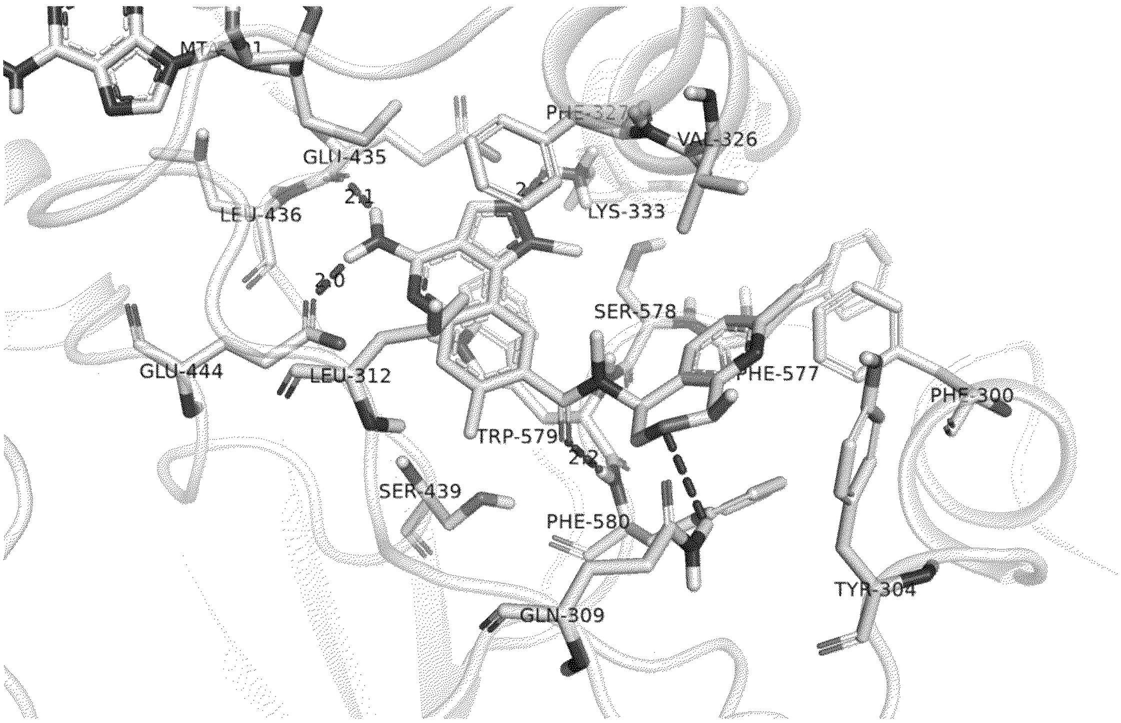
MTA-701
PHE-327
VAL-326
GLU-435
2.1
LEU-436
LYS-333
2.0
SER-578
GLU-444
LEU-312
PHE-577
PHE-300
TRP-579
2.2
SER-439
PHE-580
TYR-304
GLN-309

FIG. 4

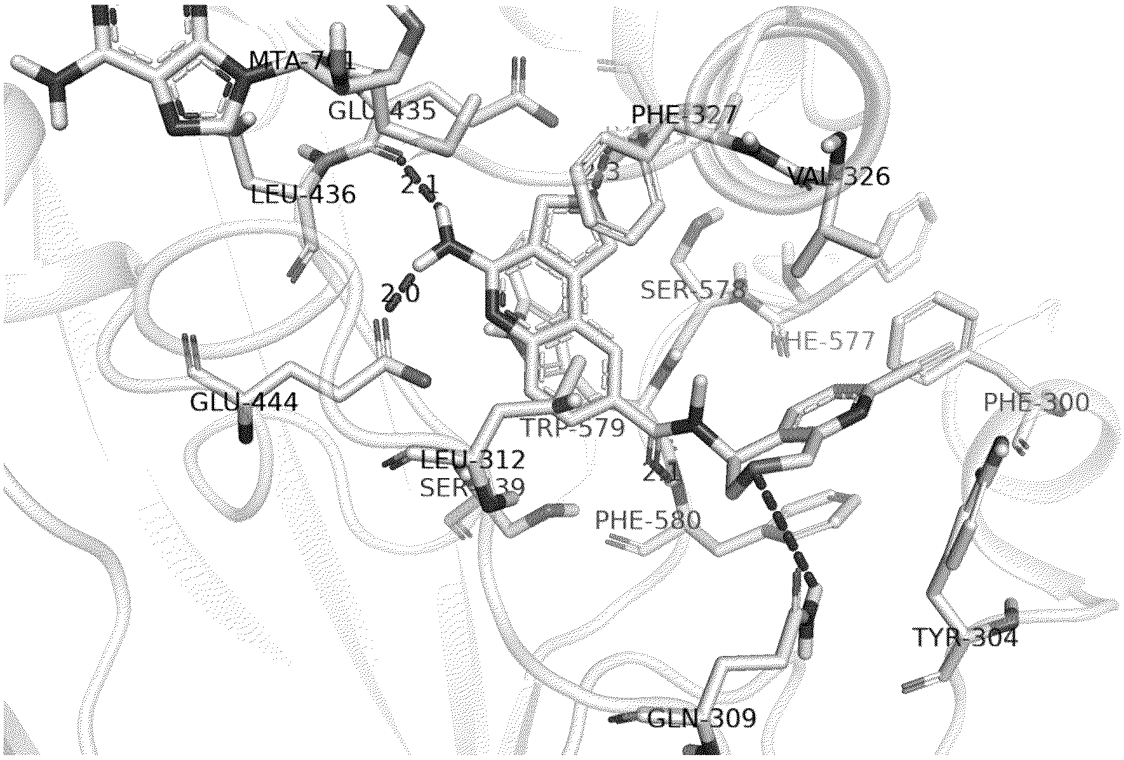
MTA-701
GLU-435
PHE-327
VAL-326
LEU-436
2.1
2.0
SER-578
PHE-577
GLU-444
PHE-300
TRP-579
LEU-312
SER-439
2.1
PHE-580
TYR-304
GLN-309

FIG. 5

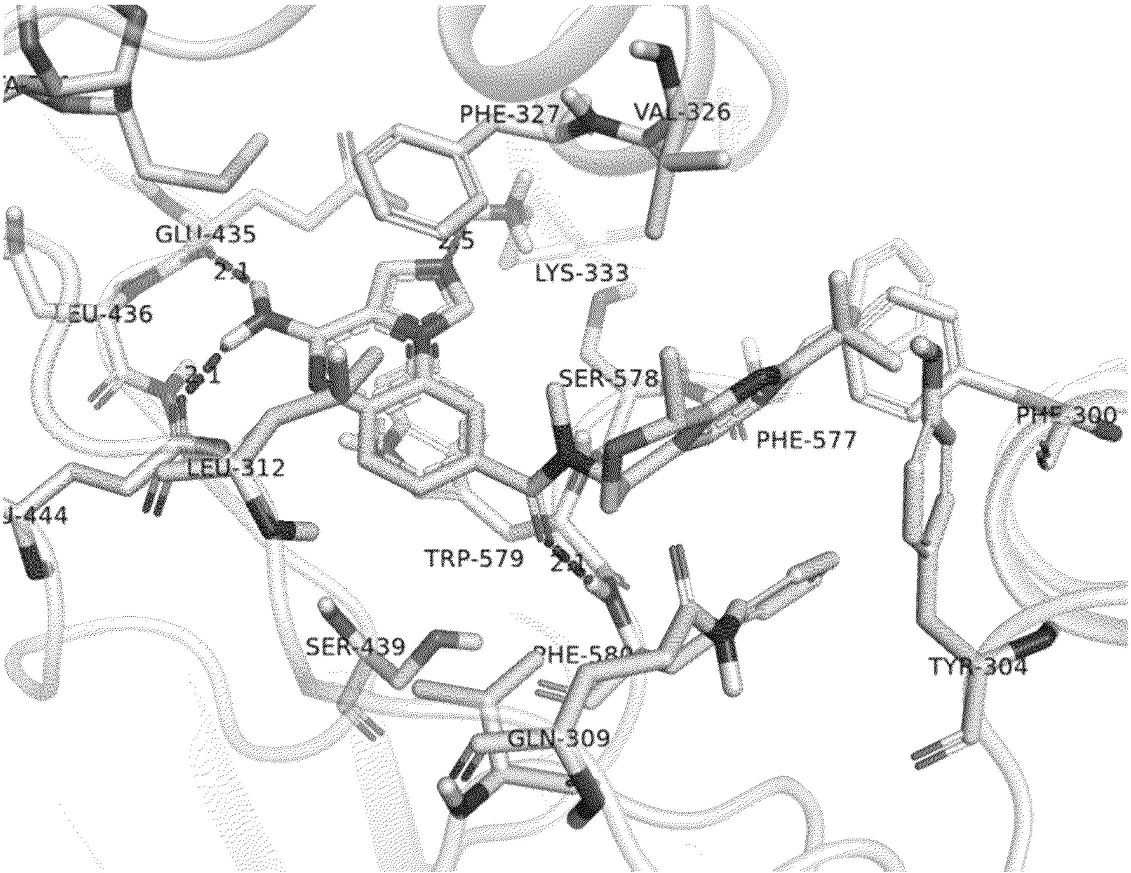
PHE-327
VAL-326
GLU-435
2.5
2.1
LYS-333
LEU-436
2.1
SER-578
PHE-577
PHE-300
LEU-312
TRP-579
2.1
SER-439
TYR-304
GLN-309

FIG. 6

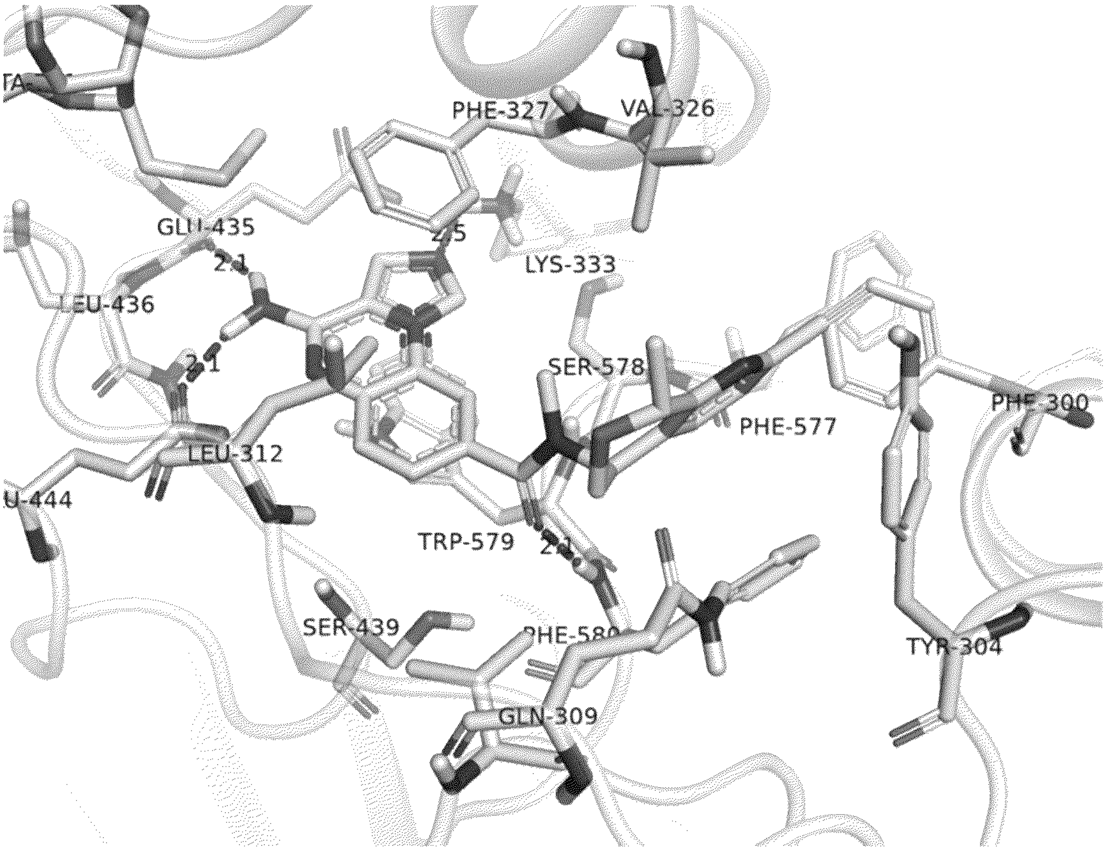
PHE-327
VAL-326
GLU-435
2.1
2.5
LYS-333
LEU-436
2.1
SER-578
PHE-577
PHE-300
LEU-312
TRP-579
2.1
SER-439
PHE-580
TYR-304
GLN-309

FIG. 7

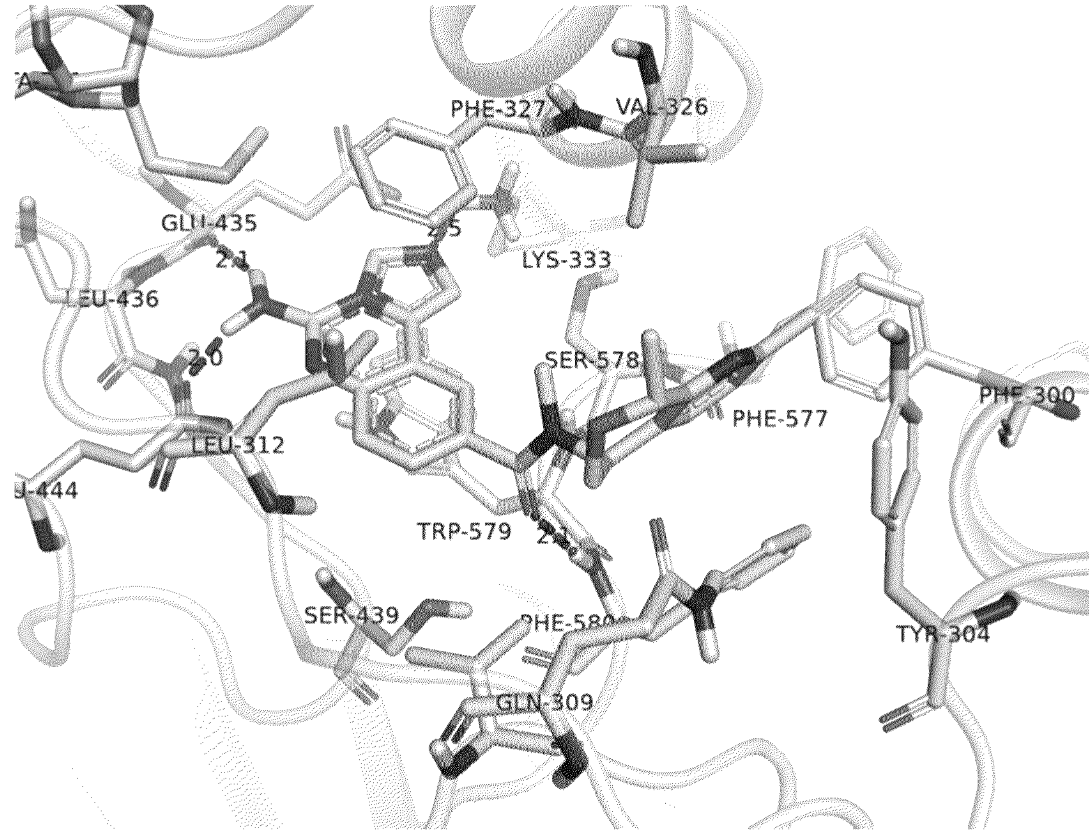
PHE-327
VAL-326
GLU-435
2.1
2.5
LYS-333
LEU-436
2.0
SER-578
PHE-577
PHE-300
LEU-312
TRP-579
2.1
SER-439
PHE-580
TYR-304
GLN-309

FIG. 8

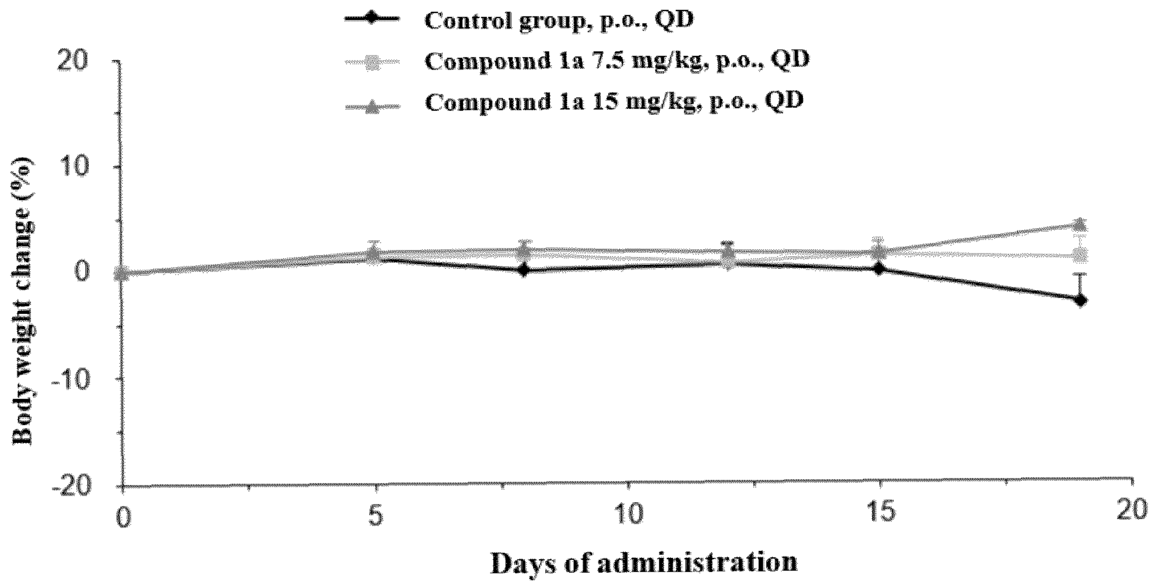
Control group, p.o., QD
Compound 1a 7.5 mg/kg, p.o., QD
Compound 1a 15 mg/kg, p.o., QD
Body weight change (%)
20
10
0
-10
-20
0
5
10
15
20
Days of administration

FIG. 9

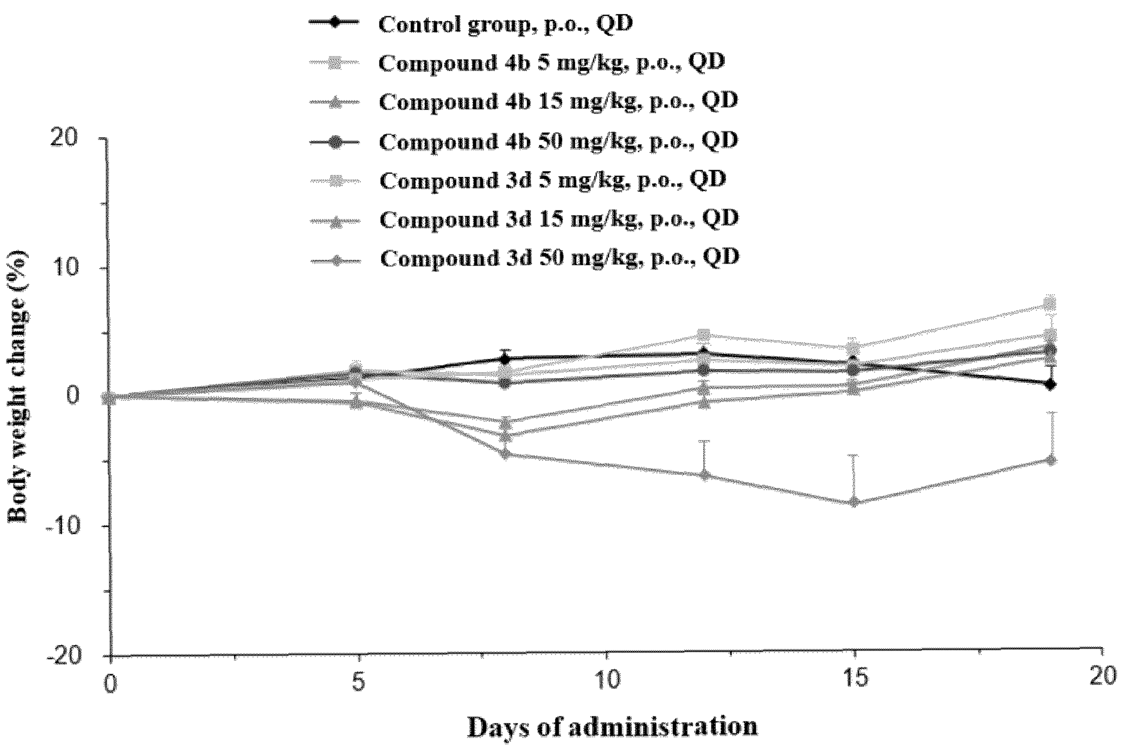
Control group, p.o., QD
Compound 4b 5 mg/kg, p.o., QD
Compound 4b 15 mg/kg, p.o., QD
Compound 4b 50 mg/kg, p.o., QD
Compound 3d 5 mg/kg, p.o., QD
Compound 3d 15 mg/kg, p.o., QD
Compound 3d 50 mg/kg, p.o., QD
Body weight change (%)
20
10
0
-10
-20
0
5
10
15
20
Days of administration

FIG. 10

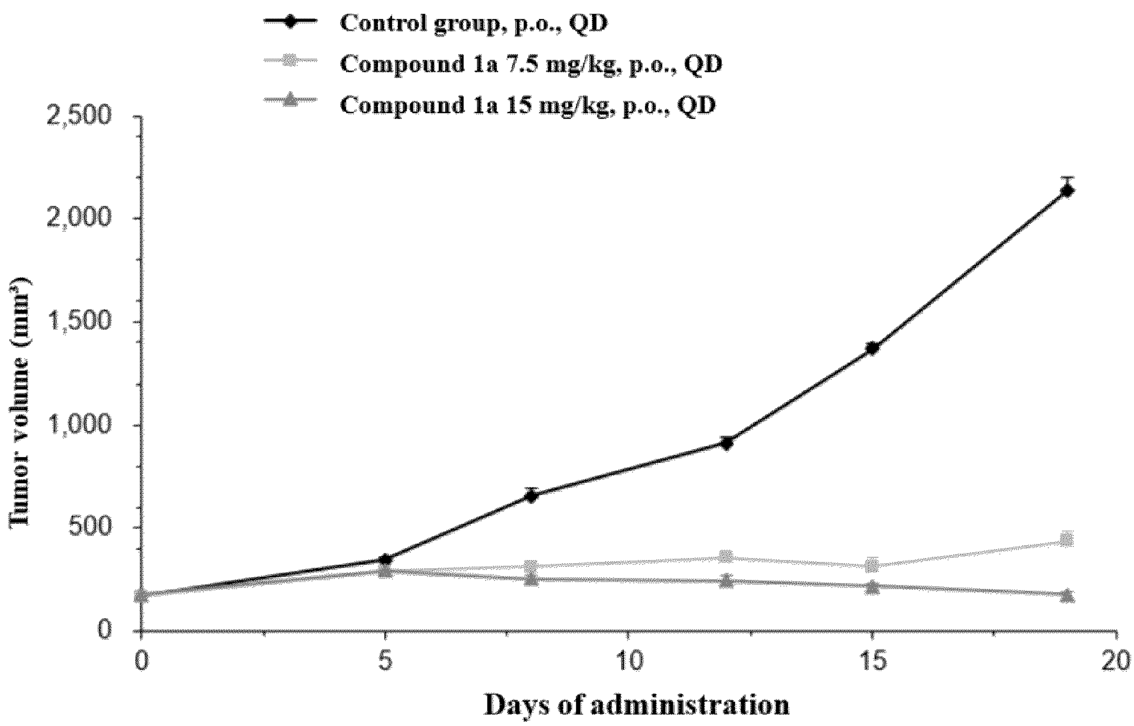
Control group, p.o., QD
Compound 1a 7.5 mg/kg, p.o., QD
Compound 1a 15 mg/kg, p.o., QD
Tumor volume (mm³)
2,500
2,000
1,500
1,000
500
0
0
5
10
15
20
Days of administration

FIG. 11

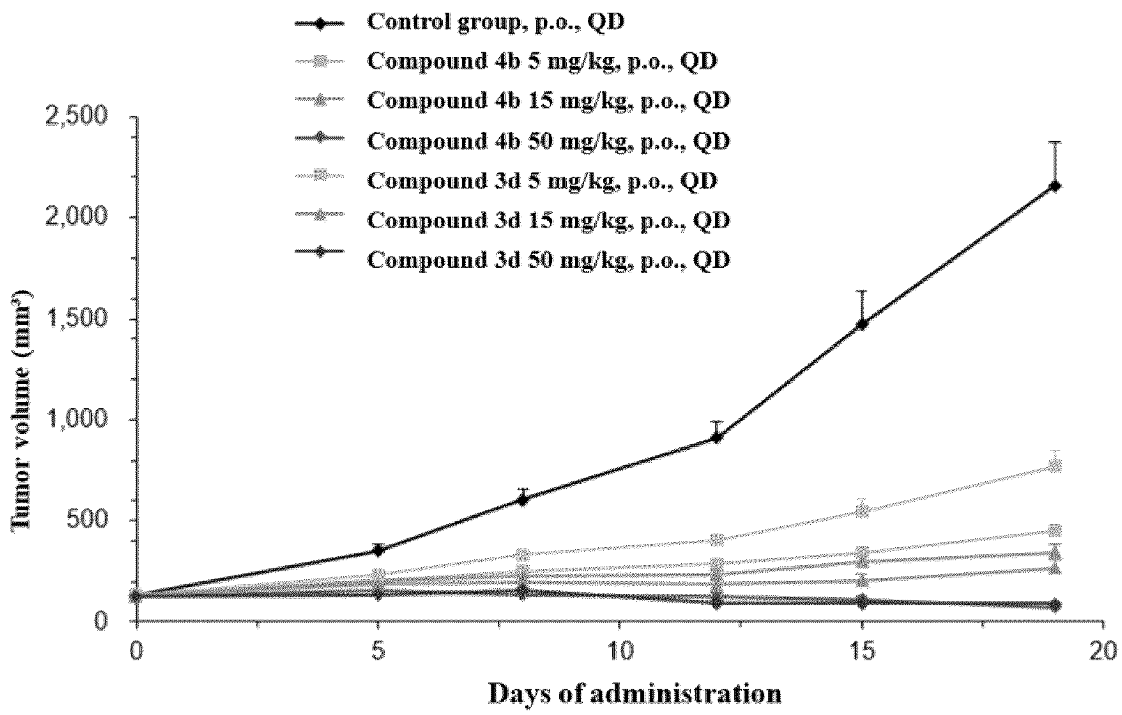
Control group, p.o., QD
Compound 4b 5 mg/kg, p.o., QD
Compound 4b 15 mg/kg, p.o., QD
Compound 4b 50 mg/kg, p.o., QD
Compound 3d 5 mg/kg, p.o., QD
Compound 3d 15 mg/kg, p.o., QD
Compound 3d 50 mg/kg, p.o., QD
Tumor volume (mm³)
2,500
2,000
1,500
1,000
500
0
0
5
10
15
20
Days of administration

FIG. 12

# INTERNATIONAL SEARCH REPORT

International application No.
**PCT/CN2023/112666**

**A. CLASSIFICATION OF SUBJECT MATTER**

C07D519/00(2006.01)i；C07D405/12(2006.01)i；A61K31/437(2006.01)i；A61K31/4709(2006.01)i；A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, CNKI, ENTXTC, DWPI, STN: 南京明德新药研发有限公司, 酰胺, 杂环, 蛋白质精氨酸甲基转移酶, amid+, heterocycl+, PRMT5, inhibit+, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| A | WO 2022169948 A1 (AMGEN INC.) 11 August 2022 (2022-08-11)<br>see abstract, and claim 1, and description, embodiment | 1-15 |
| A | WO 2021163344 A1 (AMGEN INC.) 19 August 2021 (2021-08-19)<br>see abstract, and claim 1, and description, embodiment | 1-15 |
| A | WO 2022132914 A1 (AMGEN INC.) 23 June 2022 (2022-06-23)<br>see abstract, and claim 1, and description, embodiment | 1-15 |

☐ Further documents are listed in the continuation of Box C.　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 October 2023** | **20 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/112666**

| Patent document cited in search report | Publication date (day/month/year) | Patent family member(s) | Publication date (day/month/year) |
|---|---|---|---|
| WO 2022169948 A1 | 11 August 2022 | CA 3210332 A1 | 11 August 2022 |
| | | AU 2022217791 A1 | 17 August 2023 |
| WO 2021163344 A1 | 19 August 2021 | AU 2021219730 A1 | 25 August 2022 |
| | | JP 2023513580 A | 31 March 2023 |
| | | EP 4103558 A1 | 21 December 2022 |
| | | US 2023159510 A1 | 25 May 2023 |
| | | CA 3170321 A1 | 19 August 2021 |
| WO 2022132914 A1 | 23 June 2022 | AR 124369 A1 | 22 March 2023 |
| | | KR 20230121820 A | 21 August 2023 |
| | | CO 2023008167 A2 | 21 July 2023 |
| | | US 2022194955 A1 | 23 June 2022 |
| | | TW 202233183 A | 01 September 2022 |
| | | CA 3204823 A1 | 23 June 2022 |
| | | UY 39565 A | 30 June 2022 |
| | | IL 303451 A | 01 August 2023 |
| | | AU 2021400942 A1 | 06 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2022109954611
- CN 2022110677997
- CN 2022116596067
- CN 2023101673027